(19) Europäisches Patentamt / European Patent Office / Office européen des brevets

(11) **EP 4 355 369 B1**

(12) **EUROPEAN PATENT SPECIFICATION**


(45) Date of publication and mention of the grant of the patent:
**18.06.2025 Bulletin 2025/25**

(21) Application number: **22711105.1**

(22) Date of filing: **08.03.2022**

(51) International Patent Classification (IPC):
***A61K 47/54*** (2017.01) ***A61K 47/55*** (2017.01)
***A61P 35/00*** (2006.01)

(52) Cooperative Patent Classification (CPC):
**A61K 47/549; A61K 47/55; A61K 47/554**

(86) International application number:
**PCT/NL2022/050127**

(87) International publication number:
**WO 2022/265493 (22.12.2022 Gazette 2022/51)**


(54) **CONJUGATE OF SAPONIN, OLIGONUCLEOTIDE AND GALNAC**

KONJUGAT AUS SAPONIN, OLIGONUKLEOTID UND GALNAC

CONJUGUÉ DE SAPONINE, D'OLIGONUCLÉOTIDE ET DE GALNAC


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2021 PCT/NL2021/050384**
**09.09.2021 PCT/NL2021/050549**

(43) Date of publication of application:
**24.04.2024 Bulletin 2024/17**



(73) Proprietor: **Sapreme Technologies B.V.**
**3584 CM Utrecht (NL)**

(72) Inventors:
- **POSTEL, Ruben**
**3584 CM Utrecht (NL)**
- **HERMANS, Guy**
**9820 Merelbeke (BE)**
- **VAN DE LANGEMHEEN, Helmus**
**3881 CL Putten (NL)**
- **ASADIAN BIRJAND, Mazdak**
**12165 Berlin (DE)**

(74) Representative: **Nederlandsch Octrooibureau**
**P.O. Box 29720**
**2502 LS The Hague (NL)**



(56) References cited:
**WO-A1-2011/054811** **WO-A1-2015/071388**
**WO-A1-2020/126627** **WO-A1-2022/055351**
**WO-A2-2020/126620**

- **SABINE SEWING ET AL: "GalNAc Conjugation Attenuates the Cytotoxicity of Antisense Oligonucleotide Drugs in Renal Tubular Cells", MOLECULAR THERAPY: NUCLEIC ACIDS., vol. 14, 1 March 2019 (2019-03-01), US, pages 67 - 79, XP055762603, ISSN: 2162-2531, DOI: 10.1016/j.omtn.2018.11.005**
- **XU RAN ET AL: "On the origins of triterpenoid skeletal diversity", PHYTOCHEMISTRY, ELSEVIER, AMSTERDAM , NL, vol. 65, no. 3, 9 May 2017 (2017-05-09), pages 261 - 291, XP085002429, ISSN: 0031-9422, DOI: 10.1016/J.PHYTOCHEM.2003.11.014**
- **AARON D. SPRINGER ET AL: "GalNAc-siRNA Conjugates: Leading the Way for Delivery of RNAi Therapeutics", NUCLEIC ACID THERAPEUTICS, vol. 28, no. 3, 1 June 2018 (2018-06-01), US, pages 109 - 118, XP055555952, ISSN: 2159-3337, DOI: 10.1089/nat.2018.0736**
- **BACHRAN CHRISTOPHER ET AL: "Saponins in Tumor Therapy", MINI-REVIEWS IN MEDICINAL CHEMISTRY, vol. 8, 1 August 2008 (2008-08-01), pages 575 - 584, XP055775950**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 4 355 369 B1

- **HENDRIK FUCHS ET AL: "Glycosylated Triterpenoids as Endosomal Escape Enhancers in Targeted Tumor Therapies", BIOMEDICINES, vol. 5, no. 2, 29 March 2017 (2017-03-29), pages 14, XP055429235, DOI: 10.3390/ biomedicines5020014**

Remarks:

The complete document including Reference Table(s) and the Sequence Listing(s) can be downloaded from the EPO website

**Description**

TECHNOLOGICAL FIELD

The invention relates to an oligonucleotide conjugate comprising a saponin covalently linked to a ligand for ASGPR (ASGPR ligand) and further comprising a covalently linked oligonucleotide, i.e. a (saponin, oligonucleotide, ASGPR ligand) conjugate comprising a saponin covalently linked to a ligand for ASGPR, the ligand comprising at least one GalNAc, wherein the saponin-oligonucleotide-ASGPR ligand conjugate further comprises a covalently bound oligonucleotide such as an siRNA or an antisense oligonucleotide (referred to as oligonucleotide conjugate of the invention). Furthermore, the invention relates to a pharmaceutical composition of the invention comprising the saponin-oligonucleotide-ASGPR ligand conjugate, for use as a medicament. The invention also relates to a pharmaceutical composition of the invention, for use in the treatment or prophylaxis of a disease or health problem in which an expression product is involved of for example genes: HSP27, apoB, TTR, PCSK9, TMPRSS6, ALAS1, AT3, GO, CC5, X gene of HBV, S gene of HBV, AAT, miR-122, hepatitis B virus HbsAg, LDHA, CEBPA and LDH, and for use in the treatment or prophylaxis of for example a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, α1-antitrypsin deficiency, β-thalassaemia, or an auto-immune disease. The invention also relates to a method for producing an oligonucleotide conjugate of the invention. Finally, the invention relates to an *in vitro* or ex *vivo* method for transferring the the saponin-oligonucleotide-ASGPR ligand conjugate of the invention (also referred to as oligonucleotide conjugate of the invention) from outside a cell to inside said cell.

BACKGROUND OF THE INVENTION

The ability to inhibit a protein's function, whether in humans or in pathogens, is an integral part of the discovery of new drugs. Conventional small molecule drugs as well as antibodies are strongly limited to a subset of the available targets. Small molecules bind principally to cofactor sites or transmitter sites, effectively sites that are designed to accommodate small molecules. Antibodies can bind a greater variety of proteins but are commonly limited to extracellular targets.

Oligonucleotide therapy is a relatively young and fast-developing field which aims to overcome many of the issues encountered with small-molecule drugs or antibody drugs by directly manipulating the genetic transcription and translation pathways. The potency and versatility of oligonucleotides, in particular the prospect of suppressing genes encoding proteins that are 'undruggable' by classical small molecule drugs, makes them attractive drug candidates. The first oligonucleotide drugs were based on antisense technology, whereby single-stranded nucleic acid molecules would bind with sequence specificity to their complementary mRNA target, thus triggering degradation of the duplex by the RNase H system.

In 1998, Andrew Fire and Craig Mello published a seminal paper identifying double-stranded RNAs (dsRNAs) as the causative agents for post-transcriptional gene silencing (PTGS) in *Caenorhabditis elegans,* a phenomenon they termed RNA interference (RNAi). The discovery of RNAi explained puzzling observations of gene silencing in plants and fungi and kicked off a revolution in biology that eventually showed non-coding RNAs to be central regulators of gene expression in multicellular organisms. Shortly thereafter it was discovered that small dsRNAs (typically 15-30 base-pairs (bp)) can catalytically induce RNAi silencing in mammalian cells without eliciting nonspecific interferon responses. Targeting the RNAi pathway through small dsRNAs such as small interfering RNAs (siRNAs) and short hairpin RNA (shRNA) has several theoretical advantages over antisense being notably more efficient (catalytic) and giving longer inhibition of gene expression. This could translate into lower doses and lower cost together with less frequent dosing. Lower exposures could also mean fewer toxicity problems for siRNA.

However, before siRNA reaches its target *in vivo,* it faces a number of significant barriers that block its pathway to the RNA-Induced Silencing Complex (RISC) machinery. Upon entering the bloodstream, siRNA is vulnerable to degradation by endogenous nucleases, and renal excretion due to its small size and highly anionic character. In addition, before reaching its target cell, the siRNA must navigate the tight endothelial junctions of the blood vessels and diffuse through the extracellular matrix. Due to its numerous negative charges, siRNA does not readily bind to or cross the cell membrane, and once inside cells, it must escape from endosomes to interact with its intracellular protein targets.

Hence, the success of oligonucleotide (such as siRNA) therapy strongly depends on an effective delivery of the drug from outside a cell into the cytosol. Consequently, much attention is directed at developing delivery systems which improve oligonucleotide (such as siRNA and antisense oligonucleotides) delivery. Aside from viral delivery, the main methods employed to enhance siRNA (or other oligonucleotides) delivery to the cell employ liposomes, cell-penetrating peptides (CPPs) and their mimics, or nanoparticles (Gooding, Matt, et al. Chemical biology & drug design 80.6 (2012): 787-809). Safety concerns, such as the possibility of insertion mutagenesis and immunogenesis, are considered to limit

the future of viral approaches.

Liposomes are the most commonly used delivery vector for oligonucleotides such as siRNA, where the oligonucleotide is encapsulated within a lipid bilayer. Several problems are encountered with liposomal oligonucleotide delivery, such as oxygen radical-mediated toxicity (typical for of cationic liposomes), cell toxicity, effects on gene regulation and inflammatory responses. Furthermore, *in vivo* delivery using lipids seems to predominantly target the liver and spleen.

Cell-penetrating peptides (CCP), also called protein transduction domains, are short peptides (usually <30 amino-acid residues long) which have the unusual property of being able to cross the cell membrane. Through covalent linking to the oligonucleotide or through the formation of non-covalent complexes with the oligonucleotide, a CPP may enhance cellular uptake of the oligonucleotide. The field of CPP mediated oligonucleotide delivery is extremely complex since it combines the challenges posed by both oligonucleotide technology and peptide technology, two fields which are far from mature, in a single drug. Aside from the oligonucleotide-related issues, typical challenges encountered for CPPs are related to the (lack of) *in vivo* stability of the peptide chain, often requiring the use of non-natural peptide derivatives which may be complex to synthesize and/or exhibit reduced cell-penetrating activity.

Despite the enhanced cellular delivery, overcoming entrapment by endosomes is one of the major challenges to the design of efficient CPPs and other transport systems (Gooding *et al*).

Nanoparticles and nano-carriers are nanoscale oligonucleotide delivery systems typically comprised of a polymer, biological stabilizers and cell-targeting ligands complexed with the oligonucleotide. An exemplary siRNA nano-carrier system is known under the name siRNA Dynamic Poly-Conjugates. This system is based around an amphipathic polymer linked to polyethylene glycol (PEG) as a biological stabilizer and a hepatocyte-targeting ligand wherein the siRNA is covalently bound to the polymer by a disulfide bond. The targeting moieties and PEG moieties are attached to the polymer via maleamate linkages, forming negatively charged nanoparticles which do not bind to serum proteins. Following internalization by endocytosis, the maleamate bonds are readily hydrolysed on acidification of the endosome, exposing the cationic amine groups of the polymer and inducing endosomal escape via a proton sponge effect. Nano-carriers are also known in the form of cationic polymers such as polyethylene-imines (PEIs) (sometimes combined with cyclodextrins) which can form electrostatic complexes with oligonucleotides such as siRNA, affording protection from degradation and aiding internalisation. However, toxicity at higher concentrations resulting from membrane disruption and apoptosis induction may limit the applications of cationic polymers as therapeutic delivery agent. Aside from complex preparation of the nanoparticle and nano-carriers, their long-term stability is a major barrier to commercially viable use.

Oligonucleotides can be used to modulate gene expression via a range of processes including RNAi, target degradation by RNase H-mediated cleavage, splicing modulation, non-coding RNA inhibition, gene activation and programmed gene editing. Oligonucleotides are nucleic acid polymers with the potential to treat or manage a wide range of diseases. Although the majority of oligonucleotide therapeutics have focused on gene silencing, other strategies are being pursued, including splice modulation and gene activation, expanding the range of possible targets beyond what is generally accessible to conventional pharmaceutical modalities.

Examples of pharmaceutical oligonucleotides are single-stranded antisense oligonucleotides and double-stranded short interfering RNAs (siRNAs) which share a fundamental principle: an oligonucleotide binds a target RNA through Watson-Crick base pairing, and the resulting duplex directs degradation of the target messenger RNA (mRNA). In the cytoplasm (in the case of siRNAs) or in the nucleus (in the case of antisense oligonucleotides), the oligonucleotides can modulate the expression of the cognate RNA. Antisense oligonucleotides (ASOs) are small (~18-30 nucleotides), synthetic, single-stranded nucleic acid polymers of diverse chemistries, which can be employed to modulate gene expression via various mechanisms. ASOs can be subdivided into two major categories: RNase H competent and steric block. Steric block oligonucleotides are ASOs that are designed to bind to target transcripts with high affinity but do not induce target transcript degradation as they lack RNase H competence. Steric block oligonucleotides can mask specific sequences within a target transcript and thereby interfere with transcript RNA-RNA and/or RNA-protein interactions. SiRNA molecules are the effector molecules of RNAi and classically consist of a characteristic 19 + 2mer structure (that is, a duplex of two 21-nucleotide RNA molecules with 19 complementary bases and terminal 2-nucleotide 3' overhangs). One of the strands of the siRNA (the guide or antisense strand) is complementary to a target transcript, whereas the other strand is designated the passenger or sense strand. SiRNAs act to guide the Argonaute 2 protein (AGO2), as part of the RNA-induced silencing complex (RISC), to complementary target transcripts. Complete complementarity between the siRNA and the target transcript results in cleavage (that is, slicer activity) of the target opposite position 10-11 of the guide strand, catalysed by AGO2, leading to gene silencing. SiRNA approaches include Dicer substrate siRNAs, small internally segmented siRNAs, self-delivering siRNAs (asymmetric and hydrophobic), single-stranded siRNAs and divalent siRNAs. Steric block ASOs competitively inhibit miRNAs via direct binding to the small RNA species within the RISC complex. Such ASOs are known as anti-miRNA oligonucleotides, anti-miRs or antagomirs. The first anti-miRNA drug to enter clinical trials was miravirsen (SPC3649), which is an ASO designed to treat chronic hepatitis C virus (HCV) infection via targeting of the liver-specific miRNA miR-122. Other classes of oligonucleotide therapeutics modulate RNA function by binding to splice sites on pre-mRNAs, which results - for example - in the skipping of mutation-containing exons in diseases such as muscular dystrophy.

Further examples of oligonucleotides for clinical applications are microRNAs (miRNAs) which are endogenous RNAi triggers that have been implicated in e.g. cancer, cell cycle progression, infectious disease, immunity, diabetes, metabolism, myogenesis and muscular dystrophy. MiRNA hairpins embedded within long primary miRNA transcripts are sequentially processed by two RNase III family enzymes, DICER1 (Dicer) and DROSHA, which liberate the hairpin and then cleave the loop sequence, respectively. The resulting duplex RNA (analogous to an siRNA) is loaded into an Argonaute protein (for example, AGO2) and one strand discarded to generate the mature, single-stranded miRNA species. As with siRNAs, miRNAs guide RISC to target sequences where they initiate gene silencing. In contrast with siRNAs, miRNAs typically bind with partial complementarity and induce silencing via slicer-independent mechanisms.

Therapeutic oligonucleotides are generally 15 to 30 nucleotides in length and are designed to be complementary to a specific region of a messenger RNA (mRNA) encoding a disease-related protein or a regulatory RNA. After parenteral administration, the oligonucleotide enters a cell and binds to any complementary RNA. Once the oligonucleotide drug has bound to its complementary mRNA or pre-mRNA, a series of events ensues. The outcomes depend partly on the nature of the targeted sequence and include destruction of the mRNA by means of enzymatic cleavage (which is helpful when the mRNA is mutated and encodes a pathogenic protein), a change in the pre-mRNA splicing pattern (which is helpful when the "default" splicing pattern produces a pathogenic product), or a change in the function of a regulatory RNA.

An example of an oligonucleotide-comprising drug molecule is an siRNA-GalNAc conjugate, in which the siRNA portion targets the PCSK9 enzyme. This enzyme binds and degrades the low-density lipoprotein receptor (when bound to low-density lipoprotein) and is a target in the treatment of cardiovascular disease. A further example is a GalNAc conjugate comprising antisense oligonucleotide against apolipoprotein(a), which is expressed in the liver. Also, the gene *DMD* has been the target of oligonucleotide-based drug molecules. Duchenne's muscular dystrophy is a uniformly fatal disease caused by mutations in *DMD,* the gene encoding dystrophin. Spinal muscle atrophy is an autosomal recessive disease caused by mutations in *SMN1* that result in loss of SMN1 protein function. A target for oligonucleotide-based therapy is gene *SMN2.*

Oligonucleotide drugs that use sequence-driven cleavage mechanisms to reduce levels of a disease-related mRNA and its protein product are for example mipomersen and inclisiran, which use cleavage mechanisms to modify cholesterol disposition. The two drugs each target a different gene product, each of which is important in hypercholesterolemia. The common factor in each mechanism - as a result of the hybridization of each oligonucleotide drug to the target - is the activation of endogenous enzymes which results in cleavage of the targeted mRNA at the site of hybridization. Mipomersen is a single-stranded oligonucleotide with a sequence that is complementary to a portion of the RNA encoding apolipoprotein B (apoB), a component of low-density lipoprotein (LDL) cholesterol that is produced in the liver. When mipomersen hybridizes to the pre-mRNA for apolipoprotein B, the presence of the DNA-RNA heteroduplex attracts and activates RNase H which cleaves the mRNA in the heteroduplex. Cleavage renders the apolipoprotein B mRNA inactive, thereby reducing the amount of apolipoprotein B that is produced. As a result, the export of very low-density lipoprotein cholesterol from the liver is reduced and, ultimately, circulating levels of LDL cholesterol are diminished. Inclisiran induces cleavage of the mRNA encoding proprotein convertase subtilisin-kexin type 9 (PCSK9), an enzyme that negatively regulates levels of the LDL receptor (LDLR). Persons with naturally occurring genetic variants that reduce the activity of PCSK9 have increased LDLR levels, reduced LDL cholesterol levels, and reduced cardiovascular risk as compared with persons who do not have these variants. Inclisiran cleaves and inactivates PCSK9 mRNA, which has the effect of decreasing levels of PCSK9 and therefore increasing both, LDLR levels and the clearance of LDL cholesterol, and reducing circulating levels of LDL cholesterol. Inclisiran is a double-stranded, small interfering RNA (siRNA). One of the RNA strands of inclisiran is complementary to a portion of PCSK9 mRNA. Once inclisiran enters the cell, the complementary strand (or guide strand) is loaded into the RNA-induced silencing complex (RISC), a protein complex that displays the strand to the intracellular milieu. Once a near-perfect complementary sequence (within an mRNA molecule) hybridizes with part of the guide strand, an enzyme that is part of RISC cleaves the mRNA. The mRNA cleavage products cannot be translated, and PCSK9 protein levels are thereby reduced.

Oligonucleotide-based drugs that trigger RNase H-mediated and RISC-mediated cleavage (inotersen and patisiran, respectively) are being used in the treatment of transthyretin (TTR) amyloidosis. In this form of amyloidosis, mutations in *TTR* induce misfolding of the protein product, which results in the formation of amyloid deposits in multiple tissues, including peripheral neurons and the heart.

FDA-approved oligonucleotide therapeutics targeting the liver are mipomersen (targeting gene *apoB for treating* homozygous familial hypercholesterolaemia), Defibrotide (hepatic veno-occlusive disease), patisiran *TTR* (hereditary transthyretin amyloidosis, polyneuropathy)), inotersen *TTR* (hereditary transthyretin amyloidosis, polyneuropathy)), and givosiran. Givosiran is a GalNAc conjugate for treating acute hepatic porphyria; the target gene is ALAS1. Oligonucleotide drug candidates under clinical development, targeting genes in the liver, are for example miravirsen targeting *miR-122* (hepatitis C infection), RG-101 targeting *miR-122* (hepatitis C infection), AB-729 targeting hepatitis B virus HBsAg (hepatitis B infection), ARO-AAT targeting AAT ($\alpha$1-antitrypsin deficiency), SLN124 targeting gene TMPRSS6 ($\beta$-thalassaemia), DCR-PHXC targeting *LDHA* (primary hyperoxaluria) and MTL-CEPBA targeting *CEBPA* (hepatocellular carcinoma).

Despite considerable progress, two major hurdles stand in the way of widespread application of oligonucleotide therapeutics: drug safety and delivery. The inability to deliver the oligonucleotide drug candidate to the organs and cells that are expressing the disease related RNAs has proved to be the greatest obstacle to the successful development of oligonucleotide therapeutics. Oligonucleotides are considerably larger than traditional small-molecule drug candidates. Their size, coupled with their highly anionic nature, makes it difficult for them to diffuse across cell membranes to reach the cytoplasmic and nuclear compartments. Oligonucleotides are typically large, hydrophilic poly-anions (single-stranded ASOs are ~4-10 kDa, double-stranded siRNAs are ~14 kDa), properties that mean they do not readily pass through the plasma membrane. In addition, difficulties arise when considering sufficient release and delivery of the oligonucleotides in the cytosol and/or nucleus, when escape of the oligonucleotide from the endolysosomal system before lysosomal degradation or re-export via exocytosis is considered in order to be able to arrive at the correct intracellular site of action. The current oligonucleotide cell delivery strategies are inefficient, leading to renal excretion of the drug or delivery of the majority of the drug to cells and tissues that are not of therapeutic interest. Despite the improved oligonucleotide (such as siRNA) delivery to the cell which may be achieved by some of the aforementioned delivery systems, endosomal escape remains a major barrier to the application of oligonucleotide-based therapeutics such as RNAi-based therapeutics. Only a minuscule portion of endocytosed oligonucleotide escapes into the cytoplasmic space where it can exert its intended function, while the vast majority remains trapped in endocytic compartments and is inactive. Recent literature suggests a passive siRNA escape rate of <0.01% (Setten, Ryan L., *et al.,* Nature Reviews Drug Discovery 18.6 (2019): 421-446). Furthermore, it has been shown that the capacity of cells to functionally internalize oligonucleotides to produce target effect (e.g., knockdown) appears to be independent of the extent to which cells internalize bulk oligonucleotides. These observations have led to the hypothesis of separate 'productive' and 'non-productive' free-uptake pathways, although the molecular mechanisms distinguishing these pathways remain obscure (Setten, Ryan L., John J. Rossi, and Si-ping Han. Nature Reviews Drug Discovery 18.6 (2019): 421-446).

All in all, there remains a significant need for improved delivery systems of oligonucleotides which result in an increased potency (e.g., target knockdown), less toxicity and/or less off-target effects, regardless of the underlying mechanism (improved endosomal escape, increased 'productive' pathway uptake, or another mechanism).

An emerging strategy entails the conjugation of antisense oligonucleotides (ASOs) to receptor ligands in order to increase oligonucleotide potency and distribution to selected tissues. For instance, conjugation of tri-antennary N-acetylgalactosamine (GalNAc) to oligonucleotide therapeutics yields 10-30-fold increased potency in isolated hepatocytes, as well as in liver *in vivo.* GalNAc is found on damaged glycoproteins that have lost terminal sialic acid residues from their pendant oligosaccharides. The liver functions to clear these proteins from the systemic circulation by expressing trimeric asialoglycoprotein receptors (ASGPRs) at very high levels (order of $10^5$-$10^6$ per cell) on the surface of hepatocytes. ASGPRs bind specifically to GalNAc at neutral pH for endocytosis of circulating macromolecules from the blood and release GalNAc at acidic pH (~5-6) for cargo drop-off in the early endosome. Freed ASGPRs are then recycled back to the cell surface for reuse. Approximately one-third of RNAi drugs currently in clinical trials are single-molecule, chemically modified RNAi triggers conjugated to multivalent GalNAc ligands targeting the ASGPRs. The suitable physiology of the liver, the unique properties of ASGPRs, the non-toxic nature of the GalNAc ligand and the simplicity of GalNAc-siRNA conjugates make this an attractive approach for systemic RNAi delivery to hepatocytes.

By the targeted delivery of oligonucleotide therapeutics (oligonucleotide bioconjugates) to hepatocytes through asialoglycoprotein receptor (ASGPR)-mediated uptake, the interactions between the conjugate and its corresponding cell surface receptor protein are promoted, leading to subsequent internalization by receptor-mediated endocytosis. The interaction of bioconjugates with cell type-associated receptors thereby enables targeted delivery to specific tissues, or cell types within a tissue. The ASGPR binds and internalizes tri-antennary N-acetylgalactosamine (GalNAc) conjugated with for example a therapeutic antisense oligonucleotide, which are internalized and then released inside the cell, where they can hybridize to their cognate pre-messenger RNA (mRNA) and induce cleavage of the RNA-DNA heteroduplex, or for example conjugated with a small interfering RNA (siRNA) directed against a disease-related gene. After release of the siRNA from the endosomal or lysosomal compartment, the now-cytoplasmic siRNA can load into the RNA-induced silencing complex (RISC). The loaded RISC can then scan all expressed RNAs for sequence complementarity. When a complementary sequence is detected by hybridization, an enzyme that is part of the RISC cleaves the targeted mRNA, thereby reducing the expression of the disease-related protein. RNAi denotes RNA interference. This receptor-mediated uptake allows for dosing that is lower than that required for the therapeutic delivery of unconjugated oligonucleotides. Both single-stranded and double-stranded oligonucleotides can be delivered to hepatocytes with the use of GalNAc conjugates. For example, drugs for the treatment of diseases such as haemophilia A and haemophilia B are developed based on oligonucleotide-GalNAc conjugates, as well as siRNA-GalNAc conjugate inclisiran targeting gene *PCSK9* and siRNA-GalNAc conjugate givosiran targeting gene *ALAS1.* WO 2020/126627 discloses a conjugate of the claimed and exemplified saponin and a targeting molecule such as an antibody and an oligonucleotide. In the examples the saponin SO1861 was conjugated to an antibody (cetuximab, antibody recognizing EGFR) and an oligonucleotide against Hsp27 (example 5).

Despite technological advances, achieving efficient oligonucleotide delivery remains a major translational

limitation. For oligonucleotide-based drug platforms, approaches aiming at improving oligonucleotide delivery, such as chemical modification of oligonucleotides, bio-conjugation and the use of nano-carriers for delivery of oligonucleotides, are used, but the delivery challenge still needs improvement. There still exists a need for improved delivery systems which further increase the potency, decrease the toxicity and/or reduce off-target effects of ASGPR targeted oligonucleotide systems (e.g. GalNAc-oligonucleotide conjugates).

SUMMARY

An aspect of the invention relates to an oligonucleotide conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of $(GalNAc)_3$Tris, and further covalently linked to an oligonucleotide, wherein the at least one saponin is selected from monodesmosidic triterpenoid saponins and bidesmosidic triterpenoid saponins as defined in the claims. The invention is defined by the claims. Any subject-matter falling outside the scope of the claims is not part of the invention and provided for comparison or reference purposes only.

An embodiment is the oligonucleotide conjugate of the invention comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably three GalNAc moieties, more preferably the ligand for ASGPR comprises or consists of $(GalNAc)_3$Tris, and further covalently linked to an oligonucleotide, wherein the at least one saponin is a monodesmosidic or bidesmosidic penta-cyclic triterpene saponin of the 12,13-dehydrooleanane type, preferably with an aldehyde function in position C-23 of the aglycone core structure of the saponin, wherein the oligonucleotide conjugate comprises 1-16 saponin moieties, preferably 1-8 saponin moieties, more preferably 1 saponin moiety, 4 saponin moieties or 8 saponin moieties.

An embodiment is the oligonucleotide conjugate of the invention, wherein the at least one GalNAc moiety, preferably three GalNAc moieties, the at least one saponin, preferably 1-16 saponin moieties, more preferably 1-8 saponin moieties such as 1, 4 or 8 saponin moieties, and the oligonucleotide are covalently bound via a tri-functional linker, preferably with each of the GalNAc moiety or moieties, the saponin or saponin moieties and the oligonucleotide covalently bound to a separate arm of the tri-functional linker.

An embodiment is the oligonucleotide conjugate of the invention, comprising one saponin moiety, or 4 saponin moieties, preferably 4 saponin moieties covalently bound to a dendron, preferably a G2 dendron such as for example N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide), or 8 saponin moieties, preferably 8 saponin moieties covalently bound to a dendron, preferably a G3 dendron such as for example (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide.

An embodiment is the oligonucleotide conjugate of the invention, wherein the at least one saponin moiety is linked via a hydrazone bond or via a semicarbazone bond. The at least one saponin is preferably covalently bound in the oligonucleotide conjugate via a semicarbazone bond. Such a hydrazone bond or semicarbazone bond (with a linker) is for example formed involving the aldehyde group at the C-23 atom of the quillaic acid aglycone core structure of the saponin or of the gypsogenin aglycone core structure of the saponin.

Preferred is the oligonucleotide conjugate of the invention, wherein the oligonucleotide comprised by said conjugate is defined as a nucleic acid that is no longer than 150 nt, preferably wherein the oligonucleotide has a size of 5 - 150 nt, preferably being 8 - 100 nt, most preferably being 10 - 50 nt.

An aspect of the invention relates to a pharmaceutical composition comprising the oligonucleotide conjugate of the invention, and optionally a pharmaceutically acceptable excipient and/or optionally a pharmaceutically acceptable diluent.

An aspect of the invention relates to the oligonucleotide conjugate of the invention or to a pharmaceutical composition comprising the oligonucleotide conjugate of the invention, for use as a medicament.

An aspect of the invention relates to a pharmaceutical composition comprising the oligonucleotide conjugate of the invention, for use in the treatment or prophylaxis of a disease or health problem in which an expression product is involved of any one or more of genes: *HSP27, apoB, TTR, PCSK9, TMPRSS6, ALAS1, AT3,* GO, *CC5,* X gene of HBV, S gene of HBV, *AAT,* miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3,* GO, CC5, Xgene of HBV, S gene of HBV, *AAT,* miR-122, hepatitis B virus HbsAg, *LDHA, CEBPA* and LDH.

An aspect of the invention relates to a pharmaceutical composition comprising the oligonucleotide conjugate of the invention, for use in the treatment or prophylaxis of a disease or health problem in which an expression product is involved of any one or more of genes: *HSP27, apoB, TTR, PCSK9, TMPRSS6, ALAS1, AAT,* miR-122, hepatitis B virus

HbsAg, *LDHA* and *CEBPA,* and/or for use in the treatment or prophylaxis of a disease or health problem which involves any one or more of genes: *HSP27, apoB, TTR, PCSK9, TMPRSS6, ALAS1, AAT,* miR-122, hepatitis B virus HbsAg, *LDHA* and *CEBPA.*

An aspect of the invention relates to a pharmaceutical composition comprising the oligonucleotide conjugate of the invention, for use in the treatment or prophylaxis of a disease or health problem in which an expression product is involved of any one or more of genes: *HSP27* and *apoB,* preferably *apoB,* and/or for use in the treatment or prophylaxis of a disease or health problem which involves any one or more of genes: *HSP27* and *apoB,* preferably *apoB.*

An aspect of the invention relates to a pharmaceutical composition comprising the oligonucleotide conjugate of the invention, for use in the treatment or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, $\alpha$1-antitrypsin deficiency, $\beta$-thalassaemia, or an auto-immune disease.

An aspect of the invention relates to a pharmaceutical composition comprising the oligonucleotide conjugate of the invention, for use in the treatment or prophylaxis of a cancer such as endometrial carcinoma, breast cancer, lung cancer or hepatocellular carcinoma, and/or a cardiovascular disease such as hypercholesterolemia, preferably hypercholesterolemia.

An aspect of the invention relates to an *in vitro* or ex *vivo* method for transferring the oligonucleotide conjugate of the invention from outside a cell to inside said cell, preferably for subsequently transferring the oligonucleotide comprised by the oligonucleotide conjugate of the invention into the cytosol and/or into the nucleus of said cell, comprising the steps of:

a) providing a cell which expresses ASGPR, preferably ASGPR1, on its surface, the cell preferably selected from a liver cell, a virally infected mammalian cell and a mammalian tumor cell, wherein preferably said cell is a human cell;
b) providing the oligonucleotide conjugate of the invention for transferring into the cell provided in step a);
c) contacting the cell of step a) *in vitro* or ex *vivo* with the oligonucleotide conjugate of step b), preferably in a liquid medium, therewith effecting the transfer of the oligonucleotide conjugate from outside the cell into said cell, and optionally and preferably therewith subsequently effecting the transfer of the oligonucleotide comprised by the oligonucleotide conjugate into the cytosol and/or nucleus of said cell.

An aspect of the invention relates to an *in vitro* or ex *vivo* method for transferring the oligonucleotide conjugate of the invention from outside a cell to inside said cell, preferably subsequently transferring the oligonucleotide comprised by said oligonucleotide conjugate into the cytosol of said cell, comprising the steps of:

a) providing a cell which expresses ASGPR on its surface, the cell preferably selected from a liver cell, a virally infected cell and a tumor cell, and providing the oligonucleotide conjugate of the invention for transferring into the provided cell;
b) contacting the cell of step a) *in vitro* or *ex vivo* with the oligonucleotide conjugate of step a), therewith effecting the transfer of the oligonucleotide conjugate from outside the cell into said cell, and preferably therewith subsequently effecting the transfer of the oligonucleotide comprised by the oligonucleotide conjugate into the cytosol of said cell.

Preferably, the cell is a human cell. Preferably, the cell is a liver cell.

An aspect of the invention relates to a saponin conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of $(GalNAc)_3$Tris, wherein the at least one saponin is selected from monodesmosidic triterpenoid saponins and bidesmosidic triterpenoid saponins.

An aspect of the invention relates to a pharmaceutical combination comprising:

- a first pharmaceutical composition comprising the saponin conjugate of the invention and optionally comprising a pharmaceutically acceptable excipient and/or a pharmaceutically acceptable diluent; and
- a second pharmaceutical composition comprising a second conjugate of an effector molecule and a ligand for ASGPR, wherein the ligand for ASGPR preferably comprises at least one GalNAc moiety, preferably three or four GalNAc moieties, more preferably the ligand for ASGPR comprises or consists of $(GalNAc)_3$Tris, or a third conjugate of an effector molecule and a binding molecule comprising a binding site for a cell-surface molecule, and optionally comprising a pharmaceutically acceptable excipient and/or pharmaceutically acceptable diluent.

An aspect of the invention relates to a pharmaceutical composition comprising:

- the saponin conjugate of the invention;

- a second conjugate of an effector molecule and a ligand for ASGPR wherein the ligand for ASGPR preferably comprises at least one GalNAc moiety, preferably three or four GalNAc moieties, more preferably the ligand for ASGPR comprises or consists of $(GalNAc)_3$Tris, or a third conjugate of an effector molecule and a binding molecule comprising a binding site for a cell-surface molecule, and optionally comprising a pharmaceutically acceptable excipient and/or pharmaceutically acceptable diluent.

An aspect of the invention relates to a pharmaceutical combination of the invention comprising the saponin conjugate of the invention or to a pharmaceutical composition of the invention comprising the saponin conjugate of the invention, for use as a medicament.

An aspect of the invention relates to a pharmaceutical combination of the invention comprising the saponin conjugate of the invention or a pharmaceutical composition of the invention comprising the saponin conjugate of the invention, for use in the treatment or prophylaxis of a disease or health problem in which an expression product is involved of any one or more of genes: *HSP27, apoB, TTR, PCSK9, TMPRSS6, ALAS1, AT3,* GO, *CC5,* X gene of HBV, S gene of HBV, *AAT,* miR-122, hepatitis B virus HbsAg, *LDHA, CEBPA* and *LDH.*

An aspect of the invention relates to an *in vitro* or ex *vivo* method for transferring the second conjugate or the third conjugate of the invention from outside a cell to inside said cell, preferably subsequently transferring the effector molecule comprised by the second conjugate or the third conjugate of the invention into the cytosol of said cell, comprising the steps of:

a) providing a cell which expresses ASGPR on its surface, and, when the third conjugate is to be transferred into the cell, which expresses the cell-surface molecule for which the third conjugate comprises a binding molecule for binding to said cell-surface molecule, the cell preferably selected from a liver cell, a virally infected cell and a tumor cell;
b) providing the second conjugate or the third conjugate of any one of the invention for transferring into the cell provided in step a);
c) providing the saponin conjugate of the invention;
d) contacting the cell of step a) *in vitro* or ex *vivo* with the second conjugate or the third conjugate of step b) and the saponin conjugate of step c), therewith effecting the transfer of the second conjugate or the third conjugate from outside the cell into said cell, and preferably therewith subsequently effecting the transfer of the second conjugate or the third conjugate into the cytosol of said cell, or preferably therewith subsequently effecting the transfer of at least the effector molecule comprised by the second conjugate or the third conjugate into the cytosol of said cell.

An aspect of the invention relates to a method for providing the oligonucleotide conjugate of the invention, comprising the steps of:

a) providing at least one saponin moiety comprising a covalently bound first linker, wherein the first linker comprises at least one first reactive group for covalent binding to a second reactive group on a second linker or to a seventh reactive group on a seventh linker;
b) providing an oligonucleotide comprising a covalently bound third linker, wherein the third linker comprises a third reactive group for covalent binding to a fourth reactive group on a fourth linker or to an eighth reactive group on the seventh linker;
c) providing at least one GalNAc moiety comprising a covalently bound fifth linker, wherein the fifth linker comprises a fifth reactive group for covalent binding to a sixth reactive group on a sixth linker or to a ninth reactive group on the seventh linker; and
either
(d1) linking the first linker to the second linker through formation of a covalent bond between the first reactive group and the second reactive group, linking the third linker to the fourth linker through formation of a covalent bond between the third reactive group and the fourth reactive group, linking the fifth linker to the sixth linker through formation of a covalent bond between the fifth reactive group and the sixth reactive group, and covalently linking the second linker, fourth linker and sixth linker together, therewith providing the oligonucleotide, or(d2) linking the first linker to the seventh linker through formation of a covalent bond between the first reactive group and the seventh reactive group, linking the third linker to the seventh linker through formation of a covalent bond between the third reactive group and the eighth reactive group, linking the fifth linker to the seventh linker through formation of a covalent bond between the fifth reactive group and the ninth reactive group, therewith providing the oligonucleotide conjugate.

An embodiment is the method for providing the oligonucleotide conjugate of the invention, wherein the seventh linker is a tri-functional linker, such as the tri-functional linker represented by formula (XXI):

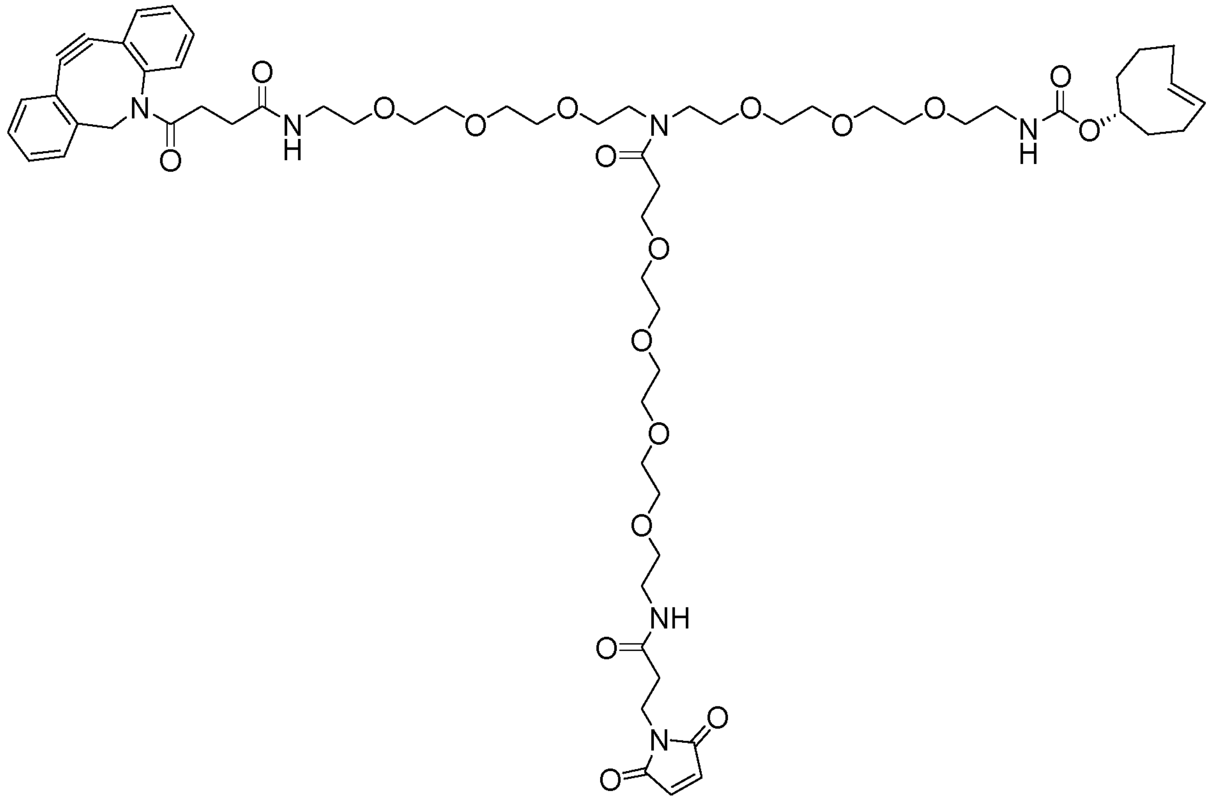

(XXI).

Preferred is the method for providing the oligonucleotide conjugate of the invention, wherein the at least one saponin moiety are 1-16 saponin moieties, preferably 1-8 saponin moieties, such as 1, 4 or 8 saponin moieties.

Preferred is the method for providing the oligonucleotide conjugate of the invention, wherein the saponin is SO1861, SO1832, QS-21, or any functional derivative thereof, preferably SO1861 or SO1832.

Preferred is the method for providing the oligonucleotide conjugate of the invention, wherein the saponin moiety or the saponin moieties is/are covalently linked via a hydrazone bond or a semicarbazone bond.

Preferred is the method for providing the oligonucleotide conjugate of the invention, wherein the at least one GalNAc moiety are 1-4 GalNAc moieties, preferably 1 or 3 GalNAc moieties.

An embodiment is the oligonucleotide conjugate of the invention, wherein the saponin comprised by the oligonucleotide conjugate is isolated from a plant. Preferably, the saponin is isolated from a part of a plant, such as the root, or from a part of a tree, such as the bark. Preferably, the saponin is isolated from roots derived from a plant.

DEFINITIONS

The term "GalNAc" has its regular scientific meaning and here refers to N-acetylgalactosamine and to the IUPAC name thereof: 2-(acetylamino)-2-deoxy-D-galactose.

The term "$(GalNAc)_3$Tris" has its regular scientific meaning in for example the field of siRNA-based therapy, and here refers to a moiety comprising three GalNAc units each separately covalently bound to the hydroxyl groups of tris(hydroxymethyl)aminomethane (Tris) (IUPAC name: 2-amino-2-(hydroxymethyl) propane-1,3-diol), preferably via at least one linker. $(GalNAc)_3$Tris can exist as a free amine comprising molecule or may be further functionalized via the remaining amine binding site, for example to form the $(GalNAc)_3$Tris-moiety comprising conjugates described herein.

The term "oligonucleotide" has its regular scientific meaning and here refers to a string of two or more nucleotides, i.e. an oligonucleotides is a short oligomer composed of ribonucleotides or deoxyribonucleotides. Examples are RNA and DNA, and any modified RNA or DNA, such as a string of nucleic acids comprising a nucleotide analogue such as a bridged nucleic acid (BNA), also known as locked nucleic acid (LNA) or a 2'-O,4'-C-aminoethylene or a 2'-O,4'-C-aminomethylene bridged nucleic acid ($BNA^{NC}$), wherein the nucleotide is a ribonucleotide or a deoxyribonucleotide.

As used herein, the terms "nucleic acid", "oligonucleotide" and "polynucleotide" are synonymous to one another and are to be construed as encompassing any polymeric molecule made of units, wherein a unit comprises a nucleobase (or simply "base" e.g. being a canonical nucleobase like adenine (A), cytosine (C), guanine (G), thymine (T), or uracil (U), or any known non-canonical, modified, or synthetic nucleobase like 5-methylcytosine, 5-hydroxymethylcytosine, xanthine,

hypoxanthine, 7-methylguanine; 5,6-dihydrouracil *etc.)* or a functional equivalent thereof, which renders said polymeric molecule capable of engaging in hydrogen bond-based nucleobase pairing (such as Watson-Crick base pairing) under appropriate hybridisation conditions with naturally-occurring nucleic acids such as deoxyribonucleic acid (DNA) or ribonucleic acid (RNA), which naturally-occurring nucleic acids are to be understood being polymeric molecules made of units being nucleotides.

Hence, from a chemistry perspective, the term nucleic acid under the present definition can be construed as encompassing polymeric molecules that chemically are DNA or RNA, as well as polymeric molecules that are nucleic acid analogues, also known as xeno nucleic acids (XNA) or artificial nucleic acids, which are polymeric molecules wherein one or more (or all) of the units are modified nucleotides or are functional equivalents of nucleotides. Nucleic acid analogues are well known in the art and due to various properties, such as improved specificity and/or affinity, higher binding strength to their target and/or increased stability *in vivo,* they are extensively used in research and medicine. Typical examples of nucleic acid analogues include but are not limited to locked nucleic acid (LNA) (that is also known as bridged nucleic acid (BNA)), phosphorodiamidate morpholino oligomer (PMO also known as Morpholino), peptide nucleic acid (PNA), glycol nucleic acid (GNA), threose nucleic acid (TNA), hexitol nucleic acid (HNA), 2'-deoxy-2'-fluoroarabinonucleic acid (FANA or FNA), 2'-deoxy-2'-fluororibonucleic acid (2'-F RNA or FRNA); altritol nucleic acids (ANA), cyclohexene nucleic acids (CeNA) *etc.*

In accordance with the canon, length of a nucleic acid is expressed herein the number of units from which a single strand of a nucleic acid is build. Because each unit corresponds to exactly one nucleobase capable of engaging in one base pairing event, the length is frequently expressed in so called "base pairs" or "bp" regardless of whether the nucleic acid in question is a single stranded (ss) or double stranded (ds) nucleic acid. In naturally occurring nucleic acids 1 bp corresponds to 1 nucleotide, abbreviated to 1 nt. For example, a single stranded nucleic acid made of 1000 nucleotides (or a double stranded nucleic acid made of two complementary strands each of which is made of 1000 nucleotides) is described as having a length of 1000 base pairs or 1000 bp, which length can also be expressed as 1000 nt or as 1 kilobase that is abbreviated to 1 kb. 2 kilobases or 2 kb are equal to the length of 2000 base pair which equates 2000 nucleotides of a single stranded RNA or DNA. To avoid confusion however, in view of the fact the nucleic acids as defined herein may comprise or consist of units not only chemically being nucleotides but also being functional equivalents thereof, the length of nucleic acids will preferentially be expressed herein in "bp" or "kb" rather than in the equally common in the art denotation "nt".

In advantageous embodiments, the nucleic acid as disclosed herein is no longer than 1 kb, preferably no longer than 500 bp, most preferably no longer than 250 bp.

In particularly advantageous embodiments, the nucleic acid is an oligonucleotide (or simply an oligo) defined as nucleic acid being no longer than 100 bp, *i.e.* in accordance with the above provided definition, being any polymeric molecule made of no more than 100 units, wherein each unit comprises a nucleobase or a functional equivalent thereof, which renders said oligonucleotide capable of engaging in hydrogen bond-based nucleobase pairing under appropriate hybridisation conditions with DNA or RNA. Within the ambit of said definition, it will immediately be appreciated that the disclosed herein oligonucleotides can comprise or consist of units not only being nucleotides but also being synthetic equivalents thereof. In other words, from a chemistry perspective, as used herein the term oligonucleotide will be construed as possibly comprising or consisting of RNA, DNA, or a nucleic acid **analogue** such as but not limited to LNA (BNA), PMO (Morpholino), PNA, GNA, TNA, HNA, FANA, FRNA, ANA, CeNA and/or the like.

The term "RNAi-mediated gene-targeting" has its regular scientific meaning and here refers to the *in vivo, ex vivo* or *in vitro* approach of influencing functioning of the gene in a cell by transferring into said cell an oligonucleotide, such as a small double-stranded RNA molecule, that targets mRNA involved in transcription of the gene: for example, small double-stranded RNA molecules are capable of efficiently triggering RNAi silencing of specific genes.

The term "bridged nucleic acid", or "BNA" in short, or "locked nucleic acid" or "LNA" in short or 2'-O,4'-C-aminoethylene or 2'-O,4'-C-aminomethylene bridged nucleic acid ($BNA^{NC}$), has its regular scientific meaning and here refers to a modified RNA nucleotide. A BNA is also referred to as 'constrained RNA molecule' or 'inaccessible RNA molecule'. A BNA monomer can contain a five-membered, six-membered or even a seven-membered bridged structure with a "fixed" $C_3$'-endo sugar puckering. The bridge is synthetically incorporated at the 2', 4'-position of the ribose to afford a 2', 4'-BNA monomer. A BNA monomer can be incorporated into an oligonucleotide polymeric structure using standard phosphoramidite chemistry known in the art. A BNA is a structurally rigid oligonucleotide with increased binding affinity and stability. The term "BNA" also refers to $BNA^{NC}$ or 2',4'-$BNA^{NC}$ (2'-O,4'-aminoethylene bridged nucleic acid) and has its regular scientific meaning and here also refers to an oligonucleotide that contains one or more nucleotide building blocks with a six-member bridged structure with an N-O linkage, and with an (N-H) or (N-Me) residue.

The term antisense oligonucleotide has its regular scientific meaning and may be indicated in short in the description as "AON" or "ASO".

The term "BNA-based antisense oligonucleotide", or in short "BNA AON", has its regular scientific meaning and here refers to a string of antisense nucleotides wherein at least one of said nucleotides is a BNA.

The term "proteinaceous" has its regular scientific meaning and here refers to a molecule that is protein-like,

meaning that the molecule possesses, to some degree, the physicochemical properties characteristic of a protein, is of protein, relating to protein, containing protein, pertaining to protein, consisting of protein, resembling protein, or being a protein. The term "proteinaceous" as used in for example 'proteinaceous molecule' refers to the presence of at least a part of the molecule that resembles or is a protein, wherein 'protein' is to be understood to include a chain of amino-acid residues at least two residues long, thus including a peptide, a polypeptide and a protein and an assembly of proteins or protein domains. In the proteinaceous molecule, the at least two amino-acid residues are for example linked via (an) amide bond(s), such as (a) peptide bond(s). In the proteinaceous molecule, the amino-acid residues are natural amino-acid residues and/or artificial amino-acid residues such as modified natural amino-acid residues. In a preferred embodiment, a proteinaceous molecule is a molecule comprising at least two amino-acid residues, preferably between two and about 2.000 amino-acid residues. In one embodiment, a proteinaceous molecule is a molecule comprising from 2 to 20 (typical for a peptide) amino acids. In one embodiment, a proteinaceous molecule is a molecule comprising from 21 to 1.000 (typical for a polypeptide, a protein, a protein domain, such as an antibody, a Fab, an scFv, one or multiple Vh domains, a ligand for a receptor such as EGF) amino acids. Preferably, the amino-acid residues are (typically) linked via (a) peptide bond(s). According to the invention, said amino-acid residues are or comprise (modified) (non-)natural amino acid residues.

The term "effector molecule", or "effector moiety" when referring to the effector molecule as part of e.g. a covalent conjugate, has its regular scientific meaning and here refers to a molecule that can selectively bind to for example any one or more of the target molecules: a protein, a peptide, a carbohydrate, a saccharide such as a glycan, a (phospho)lipid, a nucleic acid such as DNA, RNA, an enzyme, and regulates the biological activity of such one or more target molecule(s). The effector molecule is for example a molecule selected from any one or more of a small molecule such as a drug molecule, a toxin such as a protein toxin, an oligonucleotide such as a BNA, a xeno nucleic acid or an siRNA, an enzyme, a peptide, a protein, or any combination thereof. Thus, for example, an effector molecule or an effector moiety is a molecule or moiety selected from any one or more of a small molecule such as a drug molecule, a toxin such as a protein toxin, an oligonucleotide such as a BNA, a xeno nucleic acid or an siRNA, an enzyme, a peptide, a protein, or any combination thereof, that can selectively bind to any one or more of the target molecules: a protein, a peptide, a carbohydrate, a saccharide such as a glycan, a (phospho)lipid, a nucleic acid such as DNA, RNA, an enzyme, and that upon binding to the target molecule regulates the biological activity of such one or more target molecule(s). Typically, an effector molecule can exert a biological effect inside a cell such as a mammalian cell such as a human cell, such as in the cytosol of said cell. Typical effector molecules are thus drug molecules, plasmid DNA, toxins such as toxins comprised by antibody-drug conjugates (ADCs), oligonucleotides such as siRNA, BNA, nucleic acids comprised by an antibody-oligonucleotide conjugate (AOC). For example, an effector molecule is a molecule which can act as a ligand that can increase or decrease (intracellular) enzyme activity, gene expression, or cell signalling. The effector moiety is not a saponin or a saponin derivative on which the conjugate of the invention is based. The effector moiety is not the conjugate of the invention.

The term "health problem" has its regular scientific meaning and here refers to any condition of the body of a subject such as a human patient, or of a part, organ, muscle, vein, artery, skin, limb, blood, cell, *etc.* thereof, that is suboptimal when compared to the condition of that body or part thereof in a healthy subject, therewith hampering the proper functioning and/or well-being of the subject, e.g. impairs normal functioning of a human body.

The term "GalNAc-decorated oligonucleotide drug" has its regular scientific meaning and here refers to an oligonucleotide for interfering in transcription of a gene, wherein one or more GalNAc units are coupled to the oligonucleotide, e.g. via at least one linker.

The term "locked nucleic acid", in short "LNA", which is a bridged nucleic acid, has its regular scientific meaning and here refers to an oligonucleotide that contains one or more nucleotide building blocks in which an additional methylene bridge fixes the ribose moiety either in the C3'-endo conformation (beta-D-LNA) or C2'-endo (alpha-L-LNA) conformation, as is known in the art.

The term "chemically modified, metabolically stable siRNA" has its regular scientific meaning and here refers to an siRNA molecule comprising chemical modifications compared to the RNA oligonucleotide consisting of naturally occurring nucleotides, such that the modified siRNA molecule is more resistant towards metabolic degradation, digestion, enzymatic lysis, *etc., i.e.* more stable.

The term "saponin" has its regular scientific meaning and here refers to a group of amphipatic glycosides which comprise one or more hydrophilic glycone moieties combined with a lipophilic aglycone core which is a sapogenin. The saponin may be naturally occurring or synthetic (i.e. non-naturally occurring). The term "saponin" includes naturally-occurring saponins, derivatives of naturally-occurring saponins as well as saponins synthesized *de novo* through chemical and/or biotechnological synthesis routes.

The term "saponin derivative" (also known as "modified saponin") has its regular scientific meaning and here refers to a compound corresponding to a naturally-occurring saponin which has been derivatised by one or more chemical modifications, such as the oxidation of a functional group, the reduction of a functional group and/or the formation of a covalent bond with another molecule. Preferred modifications include derivatisation of an aldehyde group of the aglycone core; of a carboxyl group of a saccharide chain or of an acetoxy group of a saccharide chain. Typically, the saponin

derivative does not have a natural counterpart, i.e. the saponin derivative is not produced naturally by e.g. plants or trees. The term "saponin derivative" includes derivatives obtained by derivatisation of naturally-occurring saponins as well as derivatives synthesized *de novo* through chemical and/or biotechnological synthesis routes resulting in a compound corresponding to a naturally-occurring saponin which has been derivatised by one or more chemical modifications.

The term "aglycone core structure" has its regular scientific meaning and here refers to the aglycone core of a saponin without the one or two carbohydrate antenna or saccharide chains (glycans) bound thereto. For example, quillaic acid is the aglycone glycoside core structure for SO1861, QS-7, and QS-21.

The term "saccharide chain" has its regular scientific meaning and here refers to any of a glycan, a carbohydrate antenna, a single saccharide moiety (monosaccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The terms "SO1861" and "SO1862" refer to the same saponin of *Saponaria officinalis,* though in deprotonated form or api form, respectively. The molecular mass is 1862 Dalton as this mass is the formal mass including a proton at the glucuronic acid. At neutral pH, the molecule is deprotonated. When measuring the mass using mass spectrometry in negative ion mode, the measured mass is 1861 Dalton. Similarly, the terms "SO1831" and "SO1832" refer to the same saponin of *Saponaria officinalis,* though in deprotonated form or api form, respectively. The molecular mass is 1832 Dalton as this mass is the formal mass including a proton at the glucuronic acid. At neutral pH, the molecule is deprotonated. When measuring the mass using mass spectrometry in negative ion mode, the measured mass is 1831 Dalton.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

The term "antibody-drug conjugate" or "ADC" has its regular scientific meaning and here refers to any conjugate of an antibody such as an IgG, a Fab, an scFv, an immunoglobulin, an immunoglobulin fragment, one or multiple Vh domains, *etc.,* and any molecule that can exert a therapeutic effect when contacted with cells of a subject such as a human patient, such as an active pharmaceutical ingredient, a toxin, an oligonucleotide, an enzyme, a small molecule drug compound, *etc.*

The term "antibody-oligonucleotide conjugate" or "AOC" has its regular scientific meaning and here refers to any conjugate of an antibody such as an IgG, a Fab, an scFv, an immunoglobulin, an immunoglobulin fragment, one or multiple Vh domains, *etc.,* with any oligonucleotide molecule that can exert a therapeutic effect when contacted with cells of a subject such as a human patient, such as an oligonucleotide selected from a natural or synthetic string of nucleic acids encompassing DNA, modified DNA, RNA, modified RNA, synthetic nucleic acids, presented as a single-stranded molecule or a double-stranded molecule, such as a BNA, an allele-specific oligonucleotide (ASO), a short or small interfering RNA (siRNA; silencing RNA), an anti-sense DNA, anti-sense RNA, *etc.*

The term "conjugate" has its regular scientific meaning and here refers to at least a first molecule that is covalently bound to at least a second molecule, therewith forming a covalently coupled assembly comprising or consisting of the first molecule and the second molecule. Typical conjugates are $(GalNAc)_3$Tris - siRNA, an ADC, an AOC, and SO1861-EMCH (EMCH linked to the aldehyde group of the aglycone glycoside core structure of the saponin).

The term "moiety" has its regular scientific meaning and here refers to a molecule that is bound, linked, conjugated to a further molecule, linker, assembly of molecules, *etc.,* and therewith forming part of a larger molecule, conjugate, assembly of molecules. Typically, a moiety is a first molecule that is covalently bound to a second molecule, involving one or more chemical groups initially present on the first molecule and present on the second molecules. For example, saporin is a typical effector molecule. As part of an antibody-drug conjugate, the saporin is a typical effector moiety in the ADC. As part of an antibody-oligonucleotide conjugate, a BNA or an siRNA is a typical effector moiety in the AOC.

The term 'S' as used such as in an antibody-saponin conjugate or construct comprising a linker, represents 'stable linker' which remains intact in the endosome and in the cytosol.

The term 'L' as used such as in an antibody-saponin conjugate or construct comprising a linker, represents 'labile linker' which is cleaved under slightly acid conditions in the endosome.

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments of the invention described herein can operate in combination and cooperation, unless specified otherwise.

Furthermore, the various embodiments, although referred to as "preferred" or "e.g." or "for example" or "in particular" and the like are to be construed as exemplary manners in which the invention may be implemented rather than

as limiting the scope of the invention.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the elements or components are present, unless the context clearly requires that there is one and only one of the elements or components. The indefinite article "a" or "an" thus usually means "at least one".

The term "DAR" normally stands for *Drug Antibody Ratio* and refers to the average *drug* to *antibody ratio* for a given preparation of *antibody drug* conjugate (ADC) and here refers to a ratio of the number of bound SO1861 moieties, or SPT001, or saponin moieties in general, or bound payload, e.g. an AON such as an ApoB BNA, with respect to the conjugate molecule.

## BRIEF DESCRIPTION OF THE DRAWINGS

**Figure 1A-C:** Synthesis of trivalent-GalNAc.
**Figure 2A-B:** Synthesis of SO1861-DBCO.
**Figure 3:** Synthesis of monovalent-GalNAc-SO1861.
**Figure 4:** Synthesis of trivalent-GalNAc-SO1861.
**Figure 5A-C:** Synthesis of monovalent-GalNAc-BNA.
**Figure 6A-B:** Synthesis of trivalent-GalNAc-BNA.
**Figure 7:** Synthesis of trivalent-GalNAc-BNA.
**Figure 8A-G:** Synthesis of trivalent GalNAc.
**Figure 9A and 9B:** General toxicity (MTS) of GalNAc-SO1861, SO1861-EMCH, and trivalent-GalNAc-SO1861 on HepG2 (A) and Huh7 (B) cell lines. The legend displayed next to Figure 9B also applies for Figure 9A.
**Figure 10A and 10 B:** Cell killing assay (MTS) HepG2 (A) and Huh7 (B) cell lines. The legend displayed next to Figure 10B also applies for Figure 10A.
**Figure 11A and 11B:** Gene expression analysis on HepG2 (A) and Huh7 (B) cell lines.
**Figure 12A and 12B:** Gene expression analysis on HepG2 (A) and Huh7 (B) cell lines. The legend displayed next to Figure 12B also applies for Figure 12A.
**Figure 13A and 13B:** Cell viability assay on HepG2 (A) and Huh7 (B) cell lines. The legend displayed next to Figure 13B also applies for Figure 13A.
**Figure 14A and 14B:** Gene expression analysis on HepG2 (A) and Huh7 (B) cell lines. The legend displayed next to Figure 14B also applies for Figure 14A.
**Figure 15A and 15B:** Cell viability assay on HepG2 (A) and Huh7 (B) cell lines. The legend displayed next to Figure 15B also applies for Figure 15A.
**Figure 16A and 16B:** Gene expression analysis on HepG2 (A) and Huh7 (B) cell lines. The legend displayed next to Figure 16B also applies for Figure 16A.
**Figure 16C and 16D:** Cell viability assay on HepG2 (C) and Huh7 (D) cell lines. The legend displayed next to Figure 16D also applies for Figure 16C.
**Figure 17A and 17B:** Gene expression analysis on HepG2 (A) and Huh7 (B) cell lines. The legend displayed next to Figure 17B also applies for Figure 17A.
**Figure 18A:** Relative cell viability of primary human hepatocytes (compared with untreated cells ('Untr'), which is set to 100%) upon contacting the cells with a concentration series of trivalent GalNAc conjugated with SO1861 ($(GN)_3$-SPT), the combination of 10 pM monoclonal antibody anti-CD71 conjugated with saporin (CD71-SPRN) and a concentration series of SO1861 bound though EMCH to the aglycone aldehyde group (SPT-EMCH), the combination of 10 pM CD71-SPRN and a concentration series of $(GN)_3$-SPT, and the combination of 10 pM CD71-SPRN and a concentration series of $(GN)_3$ conjugated with a dendron with four SO1861 moieties covalently bound thereto (DAR = 4, *i.e.* the ratio $(GN)_3$ : dendron is 1:1 and the ratio dendron : SO1861 is 1:4) ($(GN)_3$-dSPT4).

**Figure 18B:** Relative *apoB* expression in primary human hepatocytes (compared to untreated cells ('Untr'), which are set to 100%) upon contacting the cells with a concentration series of trivalent GalNAc conjugated with antisense oligonucleotide targeting *apoB* mRNA ($(GalNAc)_3$-ApoB), the combination of 300 nM trivalent GalNAc conjugated with saponin SO1861 ($(GalNAc)_3$-SPT001) and a concentration series of $(GalNAc)_3$-ApoB, or the conjugate of trivalent GalNAc conjugated with a dendron with four SO1861 moieties covalently bound thereto (DAR = 4) and conjugated with antisense oligonucleotide targeting *apoB* ($(GalNAc)_3$-$dSPT_4$-ApoB). SO1861 is also referred to as 'SPT001' and as 'dSPT', throughout the description and claims.

**Figure 19A and 19B:** Cell viability of primary human hepatocytes (A) and Huh7 hepatocyte cell line (B) (both compared with untreated cells ('Untr'), which are set to 100%), upon contacting the cells with a concentration series of trivalent GalNAc conjugated with SO1861 (Trivalent GalNAc-SPT001), the combination of 10 pM monoclonal antibody anti-CD71 conjugated with saporin (CD71-saporin) and a concentration series of covalent conjugate Trivalent GalNAc-SPT001.

**Figure 19C:** Cartoon of covalent Trivalent GalNAc-SPT001 conjugate (DAR = 1 for the saponin SO1861). 'S' is SPT001 SO1861 in the cartoon.

**Figure 19D:** Cartoon of anti-CD71 antibody - saporin conjugate (OKT-9), wherein 'T' is the toxin saporin covalently linked to the antibody heavy chain.

**Figure 19E:** Cartoon of covalent Trivalent $(GalNAc)_3$-dSPT4 (or $GN_3$-dSPT4) conjugate (DAR = 4 for the saponin SO1861). 'S' is SPT001 SO1861 in the cartoon; dSPT4 describes a dendron with four SO1861 molecules, conjugated as described elsewhere.

**Figure 20A and B:** Relative *apoB* expression in primary human hepatocytes (A) and Huh7 hepatocyte cell line (B), upon contacting the cells with a concentration series of trivalent GalNAc conjugated with antisense oligonucleotide for silencing ApoB mRNA and ApoB protein expression, i.e. targeting *apoB* (TrivalentGalNAc-ApoBBNA), the combination of 300 nM trivalent GalNAc conjugated with SO1861 (TrivalentGalNAc-SPT001) (DAR = 1 with regard to bound SPT001 (saponin SO1861)) and a concentration series of TrivalentGalNAc-ApoB BNA, or a concentration series of trivalent GalNAc conjugated with four SO1861 moieties covalently bound thereto (DAR = 4) and conjugated with antisense oligonucleotide for silencing ApoB mRNA and ApoB protein expression, i.e. targeting *apoB* (TrivalentGalNAc-ApoBBNA-SPT001; here four SO1861 molecules were conjugated with a single dendron moiety to the GalNAc / oligonucleotide conjugate).

**Figure 20C:** Cartoon of covalent TrivalentGalNAc-ApoBBNA conjugate. 'A' is antisense oligonucleotide ApoB (ApoBBNA) in the cartoon.

**Figure 20D:** Cartoon of TrivalentGalNAc-ApoBBNA-SPT001. 'S' is SPT001, also referred to as SO1861, in the cartoon; 'E' is the effector moiety antisense oligonucleotide targeting *apoB (ApoB* BNA) in the cartoon.

**Figure 20E:** Cartoon of $(GalNAc)_3$-dSPT4-ApoB conjugated with a dendron with four SO1861 moieties covalently bound thereto (DAR = 4). 'S' is SPT001, also referred to as SO1861, in the cartoon; 'E' is the effector moiety antisense oligonucleotide targeting *apoB (ApoB* BNA) in the cartoon.

**Figure 21A and B:** Absolute apoB protein expression in primary human hepatocytes (A) and in the Huh7 hepatocyte cell line (B), under influence of contacting the cells with a concentration series of TrivalentGalNAc-ApoBBNA, the combination of 300 nM TrivalentGalNAc-SPT001 and a concentration series of TrivalentGalNAc-ApoBBNA, or with a concentration series of TrivalentGalNAc-ApoBBNA-SPT001; here four SO1861 molecules were conjugated with a single dendron moiety to the GalNAc/ oligonucleotide conjugate (therefore also named (GalNAc)3-dSPT4-ApoB).

**Figure 22A-C:** Synthesis of a saponin-oligonucleotide-ASGPR ligand conjugate, i.e. TrivalentGalNAc-*ApoB* BNA-SPT001, also referred to as $(GalNAc)_3$-SO1861-ApoB.

**Figure 23A:** Synthesis of SO1861-OEG-NHS (SPT001-L-NHS, molecule 12, wherein 'L' refers to a labile, cleavable linker).

**Figure 23B:** Synthesis of dendron$(SPT001)_4$-$NH_2$ (molecule 15 from molecules 13 and 14).

**Figure 23C:** Synthesis dendron$(SPT001)_4$-azide (molecule 19 from molecules 15 and 18).

**Figure 23D-E:** Synthesis dendron$(SPT001)_4$-trivalent GalNAc (molecule 24 from molecules 19 and 23). The molecule 21 shown in Fig. 23D is the Intermediate 7 shown in Fig. 8G (as is indicated by the Symbol

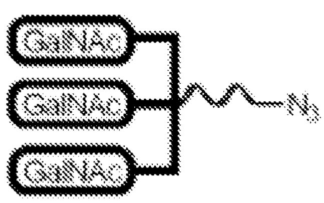

).

**Figure 24A-B:** Synthesis of DBCO-TCO-trivalent GalNAc (molecule 29) from molecule 27 and molecule 28

(trifunctional linker), wherein molecule 27 is GalNAc-thioacetate obtained from the reaction between molecule 22, the formic salt of trivalent GalNAc-amine, and molecule 26 (4-nitrophenyl 3-(acetylthio)propanate.

**Figure 24C:** Synthesis of DBCO-L-ApoB BNA oligo-trivalent GalNAc (molecule 31) by conjugating methyltetrazine-L-ApoB BNA oligo (molecule 30) to DBCO-TCO-trivalent GalNAc (molecule 29).

**Figure 24D:** Synthesis of dendron(-L-SO1861)$_4$-L-ApoB BNA oligo-trivalent GalNAc (molecule 34; 'Trivalent GalNAc-ApoB BNA-SPT001 conjugate') by conjugating molecule 31 (DBCO-L-ApoB BNA oligo-trivalent GalNAc) and molecule 19 (dendron(SPT001)$_4$-azide).

**Figure 25:** *ApoB* expression analysis in liver tissue of C57BL/6J mice.

**Figure 26:** Serum *apoB* protein analysis in C57BL/6J mice. Levels for 0.1 mg/kg ApoB#02 (196 hrs), 0.01 mg/kg (GalNAc)$_3$-ApoB + 5 mg/kg (GalNAc)$_3$-SO1861 (196 hrs and 336 hrs) and 0.1 mg/kg (GalNAc)$_3$-ApoB + 5 mg/kg (GalNAc)$_3$-SO1861 (196 hrs) are not reported.

**Figure 27:** Serum LDL-cholesterol (LDL-C) analysis in C57BL/6J mice.

**Figure 28:** Serum ALT analysis in C57BL/6J mice.

**Figure 29A-C:** *ApoB* expression analysis and cell viability assay on primary human hepatocytes, HepG2 and Huh7 cells for ApoB#02 conjugates.

**Figure 30:** *ApoB* expression analysis and cell viability assay on primary human hepatocytes for ApoB#02 conjugates.

**Figure 31:** *ApoB* expression analysis in liver tissue of C57BL/6J mice.

**Figure 32:** Serum *ApoB* protein analysis in C57BL/6J mice.

**Figure 33:** Serum LDL-cholesterol (LDL-C) analysis in C57BL/6J mice.

**Figure 34:** Serum ALT analysis in C57BL/6J mice.

**Figure 35A-D:** Trifunctional linker-(L-hydrazone-SO1861)-(L-BNA oligo)-(Trivalent-GalNAc) synthesis.

**Figure 36A-E:** Trifunctional linker-(dendron(-L-hydrazone-SO1861)4)-(L-BNA oligo)-(Trivalent-GalNAc) synthesis. Note that the drawing of Figure 36 E encompasses two sheets, labelled Fig. 36 E and Fig. 36 E (continued), which two sheets together display the conjugation of molecule 21 with molecule 19, therewith forming conjugate molecule 22.

**Figure 37A-C:** Trifunctional linker-(dendron(-L-hydrazone-SO1861)8)-(BNA oligo)-(Trivalent-GalNAc) synthesis. Note that the drawing of Figure 37 B encompasses two sheets, labelled Fig. 37 B and Fig. 37 B (continued), which two sheets together display the conjugation of molecule 24 with molecule 18, therewith forming conjugate molecule 25 (intermediate 12). Note that the drawing of Figure 37 C encompasses two sheets, labelled Fig. 37 C and Fig. 37 C (continued), which two sheets together display the conjugation of molecule 25 with molecule 21, therewith forming conjugate molecule 26.

**Figure 38A-C:** Trifunctional linker-(L-semicarbazone-SO1861)- (BNA oligo) - (Trivalent-GalNAc) synthesis.

**Figure 39A-F:** Trifunctional linker - (dendron(L-semicarbazone-SO1861)4) - (L-BNA oligo) - (Trivalent-GalNAc) synthesis. Note that the drawing of Figure 39 E encompasses two sheets, labelled Fig. 39 E and Fig. 39 E (continued), which two sheets together display the conjugation of molecule 40 with molecule 20, therewith forming conjugate intermediate 24.

**Figure 40A-D:** Trifunctional linker - (dendron(L-semicarbazone-SO1861)8) - (L-BNA oligo) - (Trivalent-GalNAc) synthesis. Note that the drawing of Figure 40 A encompasses two sheets, labelled Fig. 40 A and Fig. 40 A (continued), which two sheets together display the conjugation of molecule 43 with molecule 37, therewith forming conjugate (molecule 44) intermediate 25. Note that the drawing of Figure 40 B encompasses two sheets, labelled Fig. 40 B and Fig. 40 B (continued), which two sheets together display the conjugation of molecule 44 with molecule 18, therewith forming conjugate molecule 45 (intermediate 26). Note that the drawing of Figure 40 C encompasses two sheets, labelled Fig. 40 C and Fig. 40 C (continued), which two sheets together display the conjugation of molecule 45 with molecule 20, therewith forming conjugate molecule 46 (intermediate 27).

**Figure 41:** Trivalent linker-(L-SPT001)-(blocked TCO)-(trivalent GalNAc) synthesis synthesis.

**Figure 42A-B:** Trivalent linker-(blocked DBCO)-(L-BNA oligo)-(trivalent GalNAc) synthesis.

**Figure 43A-B:** Overview of conjugates obtainable with applying TFL (trifunctional linker) methodology. General scheme for the synthesis of a family of oligonucleotide conjugates 'CONJUGATE C1' of the invention by covalently conjugating GalNAc, a saponin, here SO1861, and an oligonucleotide, here a BNA, to the separate arms of a trifunctional linker (A). Family of conjugates CONJUGATE C1 synthesized according to the general scheme of (A), with indicated options R1 for the conjugated saponin and indicated options R2 for the conjugated BNA (B). The drawing of Figure 43A and B together displays the conjugation of Trifunctional linker labelled molecule 4 with Targeting ligand labelled molecule 41, Effector labelled molecule 12, and one of the Enhancers labelled molecule 3, 19, 25, 28, 40 and 45 or one of the Blocking agents for controls labelled molecule 5, 12a and 50, therewith forming a conjugate with the general conjugate structure 'CONJUGATE C1' as depicted in Figure 43 B, comprising group R1 and group R2, wherein R1 is any one of the groups R1 depicted below the conjugate CONJUGATE C1 and R2 in CONJUGATE C1 is any one of the groups R2 depicted below the conjugate CONJUGATE C1 in Figure 43 B.

DETAILED DESCRIPTION

The invention is defined by the claims. Any subject-matter falling outside the scope of the claims is not part of the invention and provided for comparison or reference purposes only.

A first aspect of the invention relates to a saponin conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of $(GalNAc)_3$Tris, wherein the at least one saponin is selected from monodesmosidic triterpenoid saponins and bidesmosidic triterpenoid saponins. Preferably, the ASGPR is ASGPR1. An aspect of the invention relates to an oligonucleotide conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of $(GalNAc)_3$Tris, and further covalently linked to an oligonucleotide, wherein the at least one saponin is selected from monodesmosidic triterpenoid saponins and bidesmosidic triterpenoid saponins. Typically, the ASGPR is ASGPR1. The triterpene saponin is typically and preferably a triterpenoid saponin of the 12,13-dehydrooleanane type such as a triterpenoid saponin or a bidesmosidic triterpene saponin belonging to the type of a 12,13-dehydrooleanane with an aldehyde function in position C-23. Preferred are the mono-desmosidic or bi-desmosidic penta-cyclic triterpene saponins of the 12,13-dehydrooleanane type, preferably with an aldehyde function in position C-23. Preferred is the oligonucleotide conjugate of the invention, wherein the oligonucleotide comprised by said conjugate is defined as a nucleic acid that is no longer than 150 nt, preferably wherein the oligonucleotide has a size of 5 - 150 nt, preferably being 8 - 100 nt, most preferably being 10 - 50 nt.

Surprisingly, the inventors established that the potency of a gene silencing oligonucleotide (ASO, AON, siRNA, BNA) is enhanced with at least a factor 10, that is to say at least a factor 100 or even a factor over 1000, when the mRNA expression and/or the protein expression relating to the target gene targeted by the therapeutic oligonucleotide, is considered, when the therapeutic oligonucleotide is contacted with a mammalian cell bearing ASGPR such as a human cell, such as a human liver cell, in the presence of a saponin such as SO1861 which is covalently conjugated with a ligand for the liver-cell specific receptor ASGPR1, such as trivalent-GalNAc ($GN_3$) (i.e. a conjugate comprising three GalNAc moieties). Combining the therapeutic oligonucleotide such as an siRNA or an ASO relating to treating a cancer (for example by silencing HSP27 expression) or relating to treating a too high blood plasma level of (LDL-)cholesterol, by targeting the *apoB* gene, with a saponin, e.g. provided as a GalNAc-comprising conjugate separate from the oligonucleotide, or e.g. provided as a saponin moiety covalently linked to a conjugate of an ASGPR ligand and the oligonucleotide, widens the therapeutic window for the therapeutic oligonucleotides. An effective gene silencing (any relevant extent of silencing) is achieved at lower oligonucleotide dose in the presence of an ASGPR1 targeting ligand comprising covalently linked saponin, when compared to the gene silencing obtained in the absence of (ASGPR1 targeting) saponin when contacting a target cell with the oligonucleotide coupled to a ligand for ASGPR1. That is to say, the inventors established near-complete (defined as at least 95% or higher) RNA expression inhibition, when receptor bearing cells, *in vitro* or *in vivo,* are contacted with the oligonucleotide conjugate of the invention. In addition, the inventors also achieved (near-)complete inhibition of protein expression, which, for example, for apoB protein subsequently results in (near-)complete clearance of LDL-cholesterol in serum. This surprisingly strong extent and efficacy of the gene silencing under influence of the oligonucleotide conjugate of the invention cannot be achieved when the saponin is not part of a conjugate comprising the payload (effector moiety, here an oligonucleotide) and a ligand for ASGPR, here the ASGPR1, such as $GN_3$. The improved potency of the gene silencing after contacting target (liver) cells that express the ASGPR1 with saponin-comprising conjugate of the invention is apparent when mRNA expression is assessed and/or when protein expression relating to the target gene is assessed, and indirectly when LDL-cholesterol level is assessed, since an LDL particle comprises one copy of the apoB protein (more specifically, the apoB100 protein), expression of which is silenced upon contacting cells with the oligonucleotide conjugate of the invention. An improved gene silencing is achieved at a fixed oligonucleotide dose in the presence of an ASGPR1 targeting ligand comprising covalently linked saponin, when compared to the gene silencing efficacy obtained in the absence of saponin when contacting a target cell with the same dose of oligonucleotide coupled to a ligand for ASGPR1. The inventors found that combining a conjugate of a therapeutic oligonucleotide and a ligand for ASGPR1 such as $(GalNAc)_3$ (also referred to as $GN_3$ or as $(GN)_3$) with a conjugate of a saponin and a ligand for ASGPR1 such as $(GalNAc)_3$ resulted in the surprisingly high improvement of the gene silencing efficacy, when the combination is contacted with ASGPR1 bearing cells such as human liver cells compared with the efficacy obtained in the absence of the saponin conjugate. The inventors also found that covalently coupling a saponin to a therapeutic oligonucleotide and a ligand for ASGPR1 such as $(GalNAc)_3$ resulted in the surprisingly high improvement of the gene silencing efficacy, when the combination is contacted with ASGPR1 bearing cells such as human liver cells, compared with the efficacy obtained in the absence of the saponin in the GalNAc-oligonucleotide conjugate.

Saponins of the triterpene glycoside type have the capacity to stimulate the delivery of effector molecules from outside a target cell to inside said cell, and subsequently into the cytosol of said cell. Saponins facilitate for example receptor-mediated uptake of certain effector molecules, such as a protein toxin comprised in an ADC. Endocytosis of the

toxin, as part of the ADC, delivers the toxin to the endosome and/or the lysosome of the target cell. Without wishing to be bound by any theory, subsequent release of the ADC, or at least of the toxin, out of the endosome or lysosome and into the cell cytosol, is stimulated or mediated by the triterpenoid saponin applied in the current invention. When a target cell is contacted with the toxin at a toxin dose that is too low for exerting an intracellular effect, co-administration of the toxin, such as part of an ADC, and a free saponin, such that the target cell is contacted with both the ADC and the saponin, can result in efficient toxin-mediated killing of the target cell, at the same toxin dose that is ineffective when contacted with the cell in the absence of the saponin. Thus, under influence of the free saponin, the potency of the toxin is improved. Still, the toxin dose required for efficient and sufficient target cell killing can be accompanied by undesired off-target effects, for example when the tumor cell receptor targeted by the ADC is not a truly (exclusively) tumor-cell specific receptor, but also expressed in *e.g.* healthy cells, elsewhere in a patient's body, or on cells that are present in the same organ where the target cells reside. Furthermore, the required dose of the free saponin needed to reach a sufficient extent of toxin activation (delivery of the toxin into the target-cell cytosol; the saponin threshold to mediate on-target delivery), may be at a level inducing undesired off-target effects. Since the free saponin is administered systemically, and non-targeted when the target (tumor) cell to be treated with the toxin is considered, a relatively high saponin dose is to be administered to the patient in need of the toxin treatment, in order to reach the local saponin dose at the level of the target cells. Moreover, one of the further problems and shortcomings encountered when the free saponin is co-administered with e.g. an ADC, is the difficulty to optimize the synchronization of the activity of the ADC + SPT001 (free saponin) at the right time and place in the target cell in the patient. The inventors now provide a 1-component conjugate solution which provides a solution for this problem.

The inventors now surprisingly found that the saponin dose required for potentiating the effect of a toxin on tumor cells can be lowered by a factor of 10, or even more by about a factor of 100 to 1000, when the saponin is conjugated with a cell-targeting moiety such as a receptor ligand (e.g., EGF or a cytokine), an antibody, or a binding fragment or domain thereof. For tumor cells, nowadays a series of cell-surface receptors are known for their specific expression on the surface of such aberrant cells. Saponins are successfully coupled by the inventors to antibodies and ligands for binding to receptors typically enriched on tumor cells such as trastuzumab, cetuximab, *etc.* Such targeted saponin conjugates indeed are able to enhance e.g. the cytotoxic effect of a toxin inside a target cell, requiring an at least 10-fold, such as a 100-fold to 1000-fold lower effective dose of the conjugate compared to the dose required for the same saponin in free form, i.e., not provided with a tumor cell-targeting moiety.

The inventors now further invented that target-cell specific delivery and subsequent endocytosis of the (targeted) saponin is also possible for non-aberrant cells or aberrant cells not related to e.g. cancer (tumor cells), auto-immune disease, virally infected cells, *etc.* That is to say, surprisingly, the inventors found that the ASGPR provides a suitable target receptor on cells bearing this receptor, such as liver cells, for entry of saponins provided with a ligand for ASGPR, in particular ASGPR1 (see Figure 3 for an example: saponin SO1861 (also referred to as 'SPT001') conjugated with a single GalNAc moiety). Known ligands such as monovalent GalNAc and trivalent GalNAc $(GN)_3$ are successfully coupled to one or a multitude (2, 4, 8, *etc.)* of saponin molecules, providing saponin conjugates comprising the ASGPR ligand and comprising at least one saponin moiety, preferably a bidesmosidic pentacyclic triterpene saponin of the 12,13-dehydrooleanane type with an aldehyde function in position C-23.

Contacting cells that express the ASGPR such as ASGPR1, such as liver cells, with the saponin conjugates comprising the ligand for the ASGPR (see for example Figure 4 and Figure 19C), results surprisingly in uptake of the saponin, as evident when such cells are co-targeted with e.g. conjugates comprising a gene-silencing nucleic acid and a binding molecule for a target cell surface molecule, e.g. CD71, ASGPR, resulting in improved saponin-dependent gene silencing in the target cell, or conjugates comprising such a binding molecule for the target cell and a toxin, resulting in saponin-dependent cytotoxicity. Contacting cells that express the ASGPR such as ASGPR1, such as liver cells, with the saponin conjugates comprising the ligand for the ASGPR, results surprisingly in uptake of the saponin, as evident when such cells are contacted with such a saponin conjugate comprising the saponin, the ligand for ASGPR1, e.g. GalNAc or $(GN)_3$, and further comprising an oligonucleotide for silencing expression of an mRNA and/or for silencing protein expression in the cell, i.e. oligonucleotide conjugates of the invention comprising at least one saponin, a ligand for ASGPR1 and an oligonucleotide, covalently linked together, such as via a trifunctional linker. Providing the saponin with a ligand for ASGPR results in effects in the target cell, exerted by a co-administered effector molecule such as a targeted oligonucleotide or toxin (e.g., anti-CD71 antibody or ASGPR ligand coupled to a nucleic acid or to a toxin), or exerted by the oligonucleotide that is also conjugated with the saponin and the ligand for ASGPR1 into the oligonucleotide conjugate of the invention (See also Figure 22A-C and Figure 24D). Such effects are apparent to a much lower extent, if at all, when the target cells are contacted with the effector molecule only, in the absence of targeted saponin, such as the free oligonucleotide or the oligonucleotide conjugated with e.g., trivalent GalNAc. Thus, the saponin conjugates of the invention and the oligonucleotide conjugates of the invention improve the potency of effector molecules such as oligonucleotides when a target cell comprising ASGPR at its surface is considered, and such target cell is contacted with both the saponin-ASGPR ligand of the invention and the (targeted) effector molecule, or with the oligonucleotide conjugate comprising saponin for enhancing endosomal escape of the effector moiety from the endosome and/or lysosome into the cytosol and/or ultimately into the nucleus of the target cell, GalNAc as the ligand for ASGPR1 and

an oligonucleotide as the effector moiety. Herewith, the therapeutic window of the effector molecule is improved: higher therapeutic effect at the same dose, under influence of co-administration of the saponin conjugate or under influence of the oligonucleotide conjugate; or similar therapeutic effect at lower dose, under influence of co-administration of the saponin conjugate or under influence of the oligonucleotide conjugate wherein the oligonucleotide is the effector moiety. The inventors also found that at least a 10-fold, for example about a 100-fold to 1000-fold lower dose of the saponin conjugate or of the oligonucleotide conjugate is required to reach a certain level of therapeutic effect in the target ASGPR expressing cell, such as a liver cell, induced by the co-administered ((ASGPR1) targeted) effector molecule such as an ASO, an siRNA, a BNA, a toxin such as a protein toxin, or induced by the oligonucleotide comprised as the effector moiety in the oligonucleotide conjugate, when compared to the saponin dose required for reaching the similar effect, when the saponin is not provided with a ligand for ASGPR. The inventors also found that at least a 10-fold, for example about a 100-fold to 1000-fold lower dose of an oligonucleotide is required to reach a certain level of therapeutic effect in the target ASGPR expressing cell, such as a liver cell, induced by an ((ASGPR1) targeted) oligonucleotide as the effector molecule such as an ASO, an siRNA, a BNA, that is administered to cells, when at least one saponin moiety such as an SO1861 moiety or SO1832 moiety is covalently linked to the oligonucleotide conjugate comprising at least one GalNAc, such as trivalent GalNAc.

Thus, surprisingly, the inventors now provide for improved therapeutic methods using the ASGPR as a target receptor for saponin-mediated delivery of an effector molecule such as a therapeutic oligonucleotide in the cytosol of ASGPR expressing cells, resulting in similar therapeutic effects induced by the effector molecule at lower doses than possible in the absence of the saponin conjugate of the invention or in the absence of covalently linked saponin in the oligonucleotide conjugate. Thus, the present invention provides for improved therapeutic use of the stimulatory effect of saponins, when the delivery of an effector molecule into the cytosol or nucleus of, e.g., liver cells is considered, by providing the saponin - ASGPR ligand conjugate of the invention and by providing the oligonucleotide conjugate of the invention (*e.g.,* saponin, oligonucleotide, $(GalNAc)_3$ covalently linked together in a single conjugate). Advantages reached by the inventors are amongst others: lower dose required for the effector molecule such as an oligonucleotide (siRNA or ASO) by co-administration of the ((ASGPR1) targeted) effector molecule with saponin or by administration of the oligonucleotide conjugate comprising the GalNAc moiety/moieties for targeting ASGPR1, comprising the oligonucleotide (e.g. siRNA or ASO for silencing mRNA expression relating to a selected gene and/or for silencing protein expression by targeting a selected gene) and comprising the at least one saponin for stimulating endosomal escape of the oligonucleotide once the oligonucleotide conjugate is endocytosed upon binding of the ASGPR1 ligand in the oligonucleotide conjugate to the cell ASGPR1 and delivered to the endosome/lysosome of the ASGPR1 bearing cell such as a liver cell, and lower dose required for the saponin by providing the saponin as a covalent conjugate of a ligand for ASGPR, such as trivalent GalNAc, and one or more saponin moieties (saponin conjugates of the invention), and an effector moiety such as a therapeutic oligonucleotide (e.g., an siRNA or an ASO) (oligonucleotide conjugate of the invention). Surprisingly, the ligand for ASGPR can be the same in the saponin conjugate and in the targeted effector molecule conjugate, resulting in efficient effector molecule-mediated effects in the target cell, under stimulatory influence of the saponin. That is to say, the saponin conjugate and the effector molecule conjugate, both comprising covalently linked ligand for ASGPR such as trivalent GalNAc, can be co-administered to target cells, such as liver cells exposing the ASGPR at their surface, such that the desired therapeutic effect of the effector molecule is efficiently obtained while both conjugates target the same receptor and use the same route for endocytosis into the cell. This surprising finding is highly beneficial for liver-cell targeting therapies such as therapies involving cytosolic delivery of a nucleic acid (ASO, BNA, siRNA, to list a few), since liver cells do express the ASGPR, which ASGPR is specific for liver cells, e.g. ASGPR1, whereas further liver cell receptors specific enough for use in liver cell targeting therapy are virtually absent.

The current invention thus opens new ways to improve liver-cell directed therapies wherein effector molecules such as ASO and siRNA must be intracellularly delivered and located for their mode of action. Targeted saponin provided as the saponin conjugates of the invention and the oligonucleotide conjugates of the invention widen the therapeutic window of such effector molecules (oligonucleotides such as siRNA, ASO), and at the same time, by providing the saponin with a liver cell-targeting binding molecule, also the therapeutic window of the saponin is improved, according to the invention. Thus, the inventors achieved efficient oligonucleotide delivery, therewith providing a solution to the major translational limitation that is apparent in the field of therapeutic oligonucleotides. For oligonucleotide-based drug platforms, the inventors provide a solution resulting in improving oligonucleotide delivery, based on the bio-conjugation of the invention for improved delivery of oligonucleotides. The improved delivery system of the invention results in increased potency of the oligonucleotide, that may aid in decreasing the toxicity and/or may aid in reducing off-target effects of ASGPR targeted oligonucleotide systems (*e.g.,* GalNAc-oligonucleotide conjugates).

The current inventors managed to synthesize conjugates of non-proteinaceous nature which are composed of two or three biologically active small molecules, for example when compared to the molecular size (molecular weight) of antibodies applied in (antibody-drug) conjugates based on antibodies such as IgG, which conjugates of the invention are still biologically active when the biological activity of each of the two or three small molecules comprised by the conjugates is considered: saponin conjugate of the invention and oligonucleotide conjugate of the invention. The conjugates of the

invention are relatively small, though the two or three small molecules that are covalently conjugated together in a single molecule are still able to exert the biological activity related to the nature of each of the small molecules. That is to say, the saponin enhances endosomal and lysosomal escape of oligonucleotides that are taken up by a cell. That is to say, ASGPR bearing cells take up the GalNAc comprising saponin conjugate and oligonucleotide conjugate of the invention. That is to say, gene silencing effects are apparent when ASGPR bearing cells are contacted with the oligonucleotide conjugate of the invention. The saponin- and GalNAc-comprising conjugate of the invention provides endosomal escape enhancing activity towards oligonucleotides when co-administered to the same cells with an oligonucleotide- and GalNAc-comprising conjugate. Both, the saponin and the oligonucleotide, are taken up by those cells, mediated by binding of GalNAc to the ASGPR on the target cells. The oligonucleotide that is delivered into the cytosol to a higher extent under influence of the saponin in the endosome/lysosome exerts its intracellular biological activity expressed as silencing of the gene targeted by the oligonucleotide (for example targeting the genes HSP27 and apoB). The oligonucleotide conjugate of the invention provides endosomal escape enhancing activity towards oligonucleotides comprised by the oligonucleotide conjugate. Both the saponin and the oligonucleotide are taken up by those cells as part of the oligonucleotide conjugate of the invention, mediated by binding of GalNAc to the ASGPR on the target cells. The oligonucleotide that is delivered into the cytosol to a higher extent under influence of the saponin in the endosome/lysosome exerts its intracellular biological activity expressed as silencing of the gene targeted by the oligonucleotide (for example targeting the genes *HSP27* or *apoB).* Thus, although relatively small molecules are conjugated together into a single conjugate, each of at least one saponin (such as one, four, eight saponin moieties), a GalNAc moiety or cluster of GalNAc moieties (e.g. $(GN)_3$), and an oligonucleotide, is still biologically active compared to the biological activity measured when cells are contacted with e.g. free saponin (e.g. in combination with a molecule that exerts its activity in the cytosol), GalNAc-oligonucleotide, free oligonucleotide. Although the saponin, the GalNAc and the oligonucleotide are relatively small molecules, conjugating these molecules together does not hamper their biological activity.

*Saponin conjugate - introduction*

The present invention provides a conjugate of a saponin and a ligand for asialoglycoprotein receptor (ASGPR), referred to herein as "saponin conjugate". The ASGPR ligand comprises at least one N-acetylgalactosamine (GalNAc) moiety. In accordance with preferred embodiments of the present invention each GalNAc moiety is bound to the remainder of the ASGPR ligand or - in case the ASGPR ligand consists of a single GalNAc moiety - to the saponin moiety, via a covalent bond to the oxygen on position "1" as indicated in formula (I):

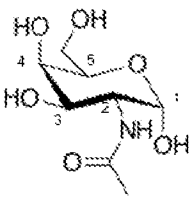

(I)

As shown in formula $(II)_s$, the ASGPR ligand consists of a single GalNAc moiety bound to the saponin moiety S, preferably via a saponin moiety linker $L_s$.

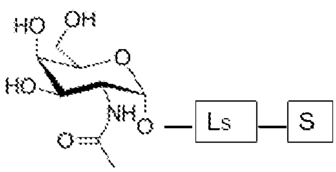

$(II)_s$

The ASGPR ligand may comprise more than one GalNAc moiety, such as 2, 3, 4, 5 or 6 GalNAc moieties, preferably 3 or 4 GalNAc moieties, more preferably 3 GalNAc moities. In such case, it is preferred that the GalNAc moieties are each separately covalently bound via the oxygen on position "1" to a central bridging moiety B, which effectively forms a bridge between the GalNAc moieties and the saponin moiety, preferably via a saponin moiety linker $L_s$. The GalNAc moieties may be directly bound to the bridging moiety B as shown in formula $(III)_s$:

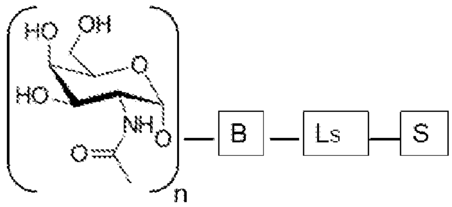

(III)s

wherein n is an integer larger than or equal to 2, $L_s$ is a saponin moiety linker and S is the saponin moiety. More preferably, the GalNAc moieties are bound to the bridging moiety B via GalNAc linkers $L_{GAL}$ as shown in formula $(IV)_S$:

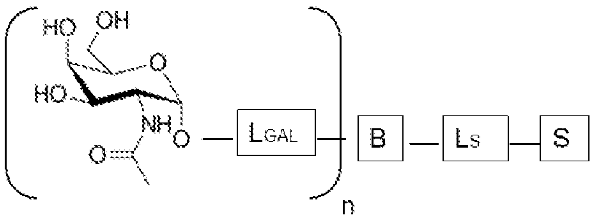

(IV)s

wherein n is an integer larger than or equal to 2, $L_S$ is a saponin moiety linker and S is the saponin moiety. While each occurrence of $L_{GAL}$ may be independently chosen, the skilled person will appreciate that the synthesis of the saponin conjugate is simplified in case each occurrence of $L_{GAL}$ represents the same moiety.

*Saponin conjugate - Linker* $L_S$

The saponin moiety linker $L_S$ represents any chemical moiety suitable for covalently binding a saponin to GalNAc as in formula $(II)_S$ or to the bridging moiety B as in formula $(III)_S$ and $(IV)_S$. The identity and size of the linker is not particularly limited and covalently binding a saponin to GalNAc as in formula $(II)_S$ or to the bridging moiety B as in formula $(III)_S$ and $(IV)_S$ may be effected by means of a 'regular' chemical functional group (*e.g.* an ester bond) as well as through "click-chemistry" type linkers which typically have a long chain length, resulting in a linker $E_S$ comprising *e.g.* more than 10 or more than 20 carbon atoms. Suitable linkers $L_S$ and associated coupling reactions for binding molecules to each other are described in the handbook Hermanson, Greg T. Bioconjugate techniques. Academic press, 2013.

As will be understood by the skilled person, the saponin moiety linker $L_S$ will typically be the result of a coupling reaction (e.g., "click-chemistry" type) between at least a first precursor $L_{S1}$ covalently bound to GalNAc or the bridging moiety B and a second precursor $L_{S2}$ covalently bound to the saponin moiety. This principle is illustrated in the following reaction scheme for the compound of formula $(II)_S$:

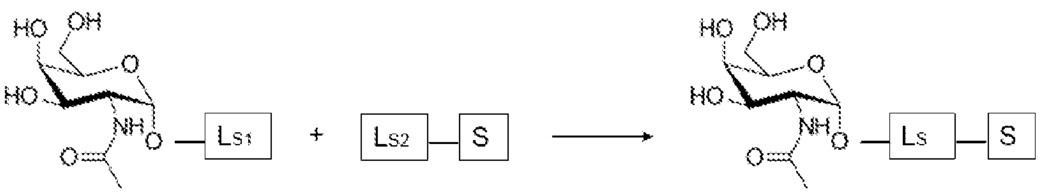

(V)s (VI)s (II)s

wherein $L_S$ is a saponin moiety linker, $L_{S1}$ is a precursor of the saponin moiety linker $L_S$ which is covalently bound to GalNAc or to the bridging moiety B and $L_{S2}$ is a precursor of the saponin moiety linker $L_S$ which is covalently bound to the saponin moiety and S is the saponin moiety.

The corresponding reaction scheme for the compound of formula $(III)_S$ is as follows:

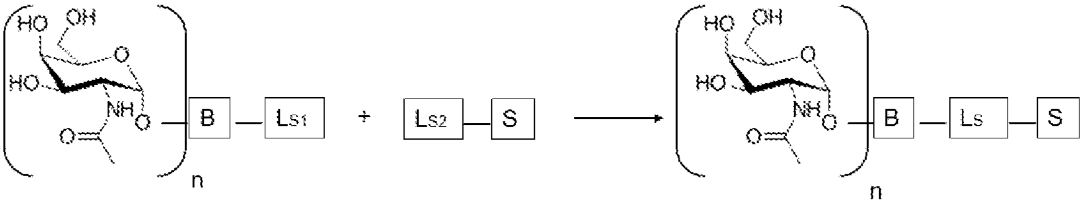

(VII)s (VI)s (III)s

wherein $L_S$ is a saponin moiety linker, $L_{S1}$ is a precursor of the saponin moiety linker $L_S$ which is covalently bound to GalNAc and $L_{S2}$ is a precursor of the saponin moiety linker $L_S$ which is covalently bound to the saponin moiety, n is an integer larger than or equal to 2, B is a bridging moiety and S is the saponin moiety.

The corresponding reaction scheme for the compound of formula $(IV)_S$ is as follows:

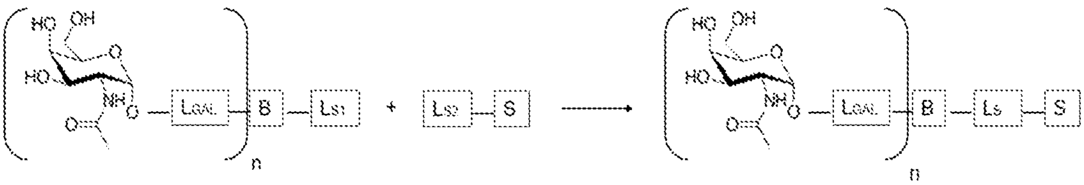

(VIII)s (VI)s (IV)s

wherein $L_S$ is a saponin moiety linker, $L_{S1}$ is a precursor of the saponin moiety linker $L_S$ which is covalently bound to GalNAc and $L_{S2}$ is a precursor of the saponin moiety linker $L_S$ which is covalently bound to the saponin moiety, n is an integer larger than or equal to 2, B is a bridging moiety, S is the saponin moiety and $L_{GAL}$ is a GalNAc linker.

The saponin moiety linker $L_S$ can be the result of a coupling reaction between at least a first precursor $L_{S1}$ covalently bound to GalNAc or the bridging moiety B and a second precursor $L_{S2}$ covalently bound to the saponin moiety, wherein the coupling reaction is for example an azide-alkyne cycloaddition, a thiol maleimide coupling, a Staudinger reaction, a nucleophilic ring-opening of strained heterocyclic electrophiles (such as aziridines, epoxides, cyclic sulphates, aziridinium ions, episulfonium ions), a carbonyl reaction of the non-aldol type (such as urea, thiourea, hydrazone, oxime ether, amide or aromatic heterocycle formation) or an addition to a carbon-carbon double bond (such as epoxidation, aziridination, dihydroxylation, sulfentyl halide addition, nitrosyl halide addition or micheal addition), preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling.

In accordance with some embodiments of the invention, the saponin moiety linker $L_S$ comprises a semicarbazone and/or a hydrazone and/or a 1, 2, 3-triazole, preferably a hydrazone and a 1, 2, 3-triazole or at least a semicarbazone. Whenever reference is made to a 1, 2, 3-triazole in the context of the linkers of the present application, this preferably means a 1*H*-1*,* 2, 3-triazole.

Such a hydrazone is *e.g.* the result of a coupling between a terminal hydrazide and an aldehyde containing compound (such as an aldehyde containing saponin). This hydrazide/aldehyde coupling is a known 'click'-chemistry tool in the field of bio-conjugation and is for example described in Hermanson, Greg T. Bioconjugate techniques. Academic press, 2013. Such a semicarbazone is e.g. the result of a coupling between a terminal semicarbazide and an aldehyde containing compound (such as an aldehyde containing saponin).

Such a 1, 2, 3-triazole is *e.g.* the result of a coupling between an azide and an alkyne containing compound. This azide/alkyne coupling is a commonly known 'click'-chemistry tool in the field of bio-conjugation and is for example described in Hermanson, Greg T. Bioconjugate techniques. Academic press, 2013.

Consequently, it is preferred that the saponin moiety linker $L_S$ is the result of a coupling reaction between a first precursor $L_{S1}$ covalently bound to GalNAc or the bridging moiety B, the first precursor $L_{S1}$ comprising an azide; and a second precursor $L_{S2}$ covalently bound to the saponin moiety, the second precursor $L_{S2}$ comprising an alkyne and preferably comprising a hydrazone resulting from a hydrazide/aldehyde coupling to an aldehyde of the saponin moiety or preferably comprising a semicarbazone resulting from a semicarbazide/aldehyde coupling to an aldehyde of the saponin moiety.

Embodiments of the structure for the precursor $L_{S1}$ present in the compounds of formula $(V)_S$ are the following azides:

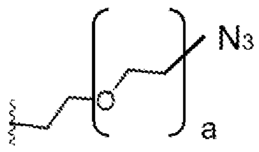

(XVII)

wherein a represents an integer larger than or equal to 0, preferably a represents an integer selected from 1, 2, and 3, more preferably a represents 2.

Embodiments of the structure for the precursor $L_{S1}$ present in the compounds of formula $(VII)_S$ or $(VIII)_S$ are the following azides:

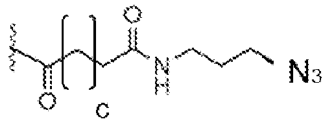

(XVIII)

wherein c represents an integer larger than or equal to 0, preferably c represents an integer in the range of 5-15, more preferably c represents 9.

Embodiments of the structure for the precursor $L_{S2}$ present in the compounds of formula $(VI)_S$, are the following hydrazones:

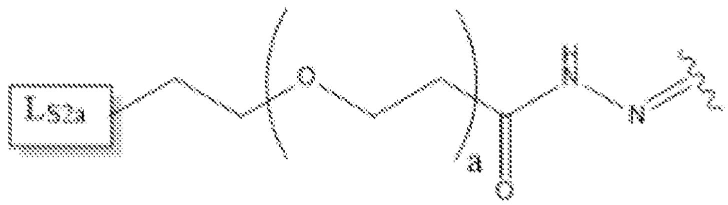

(XIX)

wherein a represents an integer larger than or equal to 0, preferably a represents an integer in the range of 2-6, more preferably a represents 4, and wherein $L_{S2a}$ represents an alkyne-containing moiety. As will be understood by the skilled person in light of the present disclosure, the hydrazone depicted in formula (XIX) results from a reaction of a hydrazide with an aldehyde of the saponin moiety.

$L_{S2a}$ preferably comprises less than 20 carbon atoms, more preferably $L_{S2a}$ represents a moiety according to formula (XX):

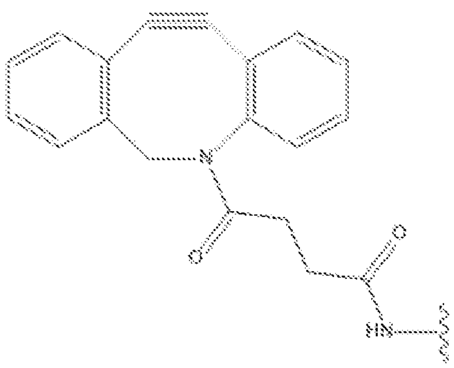

(XX)

Hence, in some embodiments the saponin conjugate is a compound of formula $(II)_S$, $(III)_S$ or $(IV)_S$ as described herein, wherein the saponin moiety linker $L_S$ is the result of a coupling reaction between a compound of formula $(V)_S$, $(VII)_S$ or $(VIII)_S$ with a compound of formula $(VI)_S$, wherein the first precursor $L_{S1}$ is an azide, for example an azide of formula (XVII) or formula (XVIII), and wherein the second precursor $L_{S2}$ is a compound of formula (XIX) comprising an alkyne-containing moiety $L_{S2a}$, for example the alkyne of formula (XX) and a hydrazone resulting from a reaction of a hydrazide with an aldehyde of the saponin moiety.

*Saponin conjugate - saponin*

An aspect of the invention relates to a saponin conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably three GalNAc moieties (also referred to as tri-GalNAc and $(GN)_3$), more preferably the ligand for ASGPR comprises or consists of $(GalNAc)_3$Tris, wherein the at least one saponin is selected from monodesmosidic triterpenoid saponins and bidesmosidic triterpenoid saponins.

An aspect of the invention relates to an oligonucleotide conjugate comprising at least one saponin, preferably 1-32, more preferably 1-16, most preferably 1-8 saponin moieties, such as 1, 4 or 8 saponin moieties, covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably three GalNAc moieties, more preferably the ligand for ASGPR comprises or consists of $(GalNAc)_3$Tris, and further covalently linked to an oligonucleotide, wherein the at least one saponin is selected from monodesmosidic triterpenoid saponins and bidesmosidic triterpenoid saponins. The saponin is selected for its endosomal escape enhancing activity when the intracellular effect of a molecule such as a proteinaceous toxin (e.g., saporin, dianthin) or a gene-silencing oligonucleotide such as HSP27 BNA is assessed when contacted with a target cell in the presence or absence of the saponin. For the saponins applied in the conjugates of the invention, typically, presence of the saponin in the endosome of a cell increases the biological activity (e.g., gene silencing, toxicity) in the cytosol of said cell with at least a factor 10 when a toxin or oligonucleotide is initially co-located with the saponin in the endosome.

An embodiment is the saponin conjugate of the invention or the oligonucleotide conjugate of the invention, wherein the saponin is a pentacyclic triterpene saponin of the 12,13-dehydrooleanane type, preferably with an aldehyde function in position C-23 of the aglycone core structure of the saponin.

An embodiment is the saponin conjugate of the invention or the oligonucleotide conjugate of the invention, wherein the saponin is a monodesmosidic or bidesmosidic pentacyclic triterpene saponin of the 12,13-dehydrooleanane type, preferably with an aldehyde function in position C-23 of the aglycone core structure of the saponin.

An embodiment is the saponin conjugate of the invention or the oligonucleotide conjugate of the invention, wherein the saponin comprises an aglycone core structure selected from the group consisting of:

2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
quillaic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en;
gypsogenic acid,
and
derivatives thereof,

preferably the saponin comprises an aglycone core structure selected from quillaic acid and gypsogenin or derivatives thereof, more preferably the saponin aglycone core structure is quillaic acid or a derivative thereof. Such preferred aglycones (or a derivative thereof) comprise an aldehyde group at the C-23 atom or a derivative thereof as described herein elsewhere. Without wishing to be bound by any theory, such an aldehyde group contributes to the endosomal escape enhancing activity of saponins of the triterpene glycoside type, comprising an aglycone selected from Group C, especially quillaic acid and gypsogenin.

An embodiment is the saponin conjugate of the invention or the oligonucleotide conjugate of the invention, wherein the at least one saponin comprises a first saccharide chain, which is bound to the $C_3$ atom or the $C_{28}$ atom of the

aglycone core structure of the at least one saponin, preferably to the $C_3$ atom, and/or wherein the at least one saponin comprises a second saccharide chain, which is bound to the $C_{28}$ atom of the aglycone core structure of the at least one saponin, preferably, the saponin comprises the first and second saccharide chain. Thus, when the saponin comprised by the saponin conjugate of the invention or the oligonucleotide conjugate of the invention bears two glycans (saccharide chains), the first saccharide chain is bound at position $C_3$ of the aglycone core structure and the second saccharide chain is bound at position $C_{28}$ of the aglycone core structure.

An embodiment is the saponin conjugate of the invention or the oligonucleotide conjugate of the invention, wherein

- the saponin comprises a saccharide chain bound to the aglycone core structure, which is selected from group A:

  GlcA-,
  Glc-,
  Gal-,
  Rha-(1→2)-Ara-,
  Gal-(1→2)-[Xyl-(1→3)]-GlcA-,
  Glc-(1→2)-[Glc-(1→4)]-GlcA-,
  Glc-(1→2)-Ara-(1→3)-[Gal-(1→2)]-GlcA-,
  Xyl-(1→2)-Ara-(1→3)-[Gal-(1→2)]-GlcA-,
  Glc-(1→3)-Gal-(1→2)-[Xyl-(1→3)]-Glc-(1→4)-Gal-,
  Rha-(1→2)-Gal-(1→3)-[Glc-(1→2)]-GlcA-,
  Ara-(1→4)-Rha-(1→2)-Glc-(1→2)-Rha-(1→2)-GlcA-,
  Ara-(1→4)-Fuc-(1→2)-Glc-(1→2)-Rha-(1→2)-GlcA-,
  Ara-(1→4)-Rha-(1→2)-Gal-(1→2)-Rha-(1→2)-GlcA-,
  Ara-(1→4)-Fuc-(1→2)-Gal-(1→2)-Rha-(1→2)-GlcA-,
  Ara-(1→4)-Rha-(1→2)-Glc-(1→2)-Fuc-(1→2)-GlcA-,
  Ara-(1→4)-Fuc-(1→2)-Glc-(1→2)-Fuc-(1→2)-GlcA-,
  Ara-(1→4)-Rha-(1→2)-Gal-(1→2)-Fuc-(1→2)-GlcA-,
  Ara-(1→4)-Fuc-(1→2)-Gal-(1→2)-Fuc-(1→2)-GlcA-,
  Xyl-(1→4)-Rha-(1→2)-Glc-(1→2)-Rha-(1→2)-GlcA-,
  Xyl-(1→4)-Fuc-(1→2)-Glc-(1→2)-Rha-(1→2)-GlcA-,
  Xyl-(1→4)-Rha-(1→2)-Gal-(1→2)-Rha-(1→2)-GlcA-,
  Xyl-(1→4)-Fuc-(1→2)-Gal-(1→2)-Rha-(1→2)-GlcA-,
  Xyl-(1→4)-Rha-(1→2)-Glc-(1→2)-Fuc-(1→2)-GlcA-,
  Xyl-(1→4)-Fuc-(1→2)-Glc-(1→2)-Fuc-(1→2)-GlcA-,
  Xyl-(1→4)-Rha-(1→2)-Gal-(1→2)-Fuc-(1→2)-GlcA-,
  Xyl-(1→4)-Fuc-(1→2)-Gal-(1→2)-Fuc-(1→2)-GlcA-, and
  derivatives thereof,

  or
- the saponin comprises a saccharide chain bound to the aglycone core structure, which is selected from group B:

  Glc-,
  Gal-,
  Rha-(1→2)-[Xyl-(1→4)]-Rha-,
  Rha-(1→2)-[Ara-(1→3)-Xyl-(1→4)]-Rha-,
  Ara-,
  Xyl-,
  Xyl-(1→4)-Rha-(1→2)-[R1-(→4)]-Fuc- wherein R1 is 4E-Methoxycinnamic acid,
  Xyl-(1→4)-Rha-(1→2)-[R2-(→4)]-Fuc- wherein R2 is 4Z-Methoxycinnamic acid,
  Xyl-(1→4)-[Gal-(1→3)]-Rha-(1→2)-4-OAc-Fuc-,
  Xyl-(1→4)-[Glc-(1→3)]-Rha-(1→2)-3,4-di-OAc-Fuc-,
  Xyl-(1→4)-[Glc-(1→3)]-Rha-(1→2)-[R3-(→4)]-3-OAc-Fuc- wherein R3 is 4E-Methoxycinnamic acid,
  Glc-(1→3)-Xyl-(1→4)-[Glc-(1→3)]-Rha-(1→2)-4-OAc-Fuc-,
  Glc-(1→3)-Xyl-(1→4)-Rha-(1→2)-4-OAc-Fuc-,
  (Ara- or Xyl-)(1→3)-(Ara- or Xyl-)(1→4)-(Rha- or Fuc-)(1--->2)-[4-OAc-(Rha- or Fuc-)(1→4)]-(Rha- or Fuc-),
  Xyl-(1→3)-Xyl-(1→4)-Rha-(1→2)-[Qui-(1→4)]-Fuc-,
  Api-(1→3)-Xyl-(1→4)-[Glc-(1→3)]-Rha-(1→2)-Fuc-,

Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1→4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
Glc-(1→3)-[Glc-(1→6)]-Gal-, and
derivatives thereof,

or

- the saponin is a bidesmosidic triterpene glycoside (a bidesmosidic triterpenoid saponin, preferably a pentacyclic saponin, more preferably a saponin of the 12,13-dehydrooleanane type, preferably with an aldehyde function in position C-23 of the aglycone core structure of the saponin) comprising a first saccharide chain selected from the group A bound to the aglycone core structure and comprising a second saccharide chain selected from the group B bound to the aglycone core structure.

Thus, when the saponin comprised by the saponin conjugate of the invention or the oligonucleotide conjugate of the invention bears two glycans (saccharide chains), the first saccharide chain is bound at position $C_3$ of the aglycone core structure and the second saccharide chain is bound at position $C_{28}$ of the aglycone core structure.

An embodiment is the saponin conjugate of the invention or the oligonucleotide conjugate of the invention, wherein the saponin's aglycone core structure is selected from any one or more of quillaic acid, gypsogenin, and a derivative thereof, preferably wherein the saponin's aglycone core structure is quillaic acid or gypsogenin, more preferably quillaic acid.

An embodiment is the saponin conjugate of the invention or the oligonucleotide conjugate of the invention, wherein the saponin is at least a bidesmosidic saponin comprising a first saccharide chain that comprises a terminal glucuronic acid residue and comprises a second saccharide chain that comprises at least four sugar residues in a branched configuration; preferably wherein the first saccharide chain is Gal-(1→2)-[Xyl-(1→3)]-GlcA and/or wherein the branched second saccharide chain of at least four sugar residues comprises a terminal fucose residue and/or a terminal rhamnose residue.

An embodiment is the saponin conjugate of the invention or the oligonucleotide conjugate of the invention, wherein the saponin comprises the first saccharide chain at position C-3 of the saponin's aglycone core structure and/or the second saccharide chain at position C-28 of the saponin's aglycone core structure; preferably wherein the first saccharide chain is a carbohydrate substituent at the C-3beta-OH group of the saponin's aglycone core structure and/or wherein the second saccharide chain is a carbohydrate substituent at the C-28-OH group of the saponin's aglycone core structure.

Without wishing to be bound by any theory, presence of an aldehyde group or a derivative thereof in the aglycone core structure (here, also referred to as 'aglycone') is beneficial for the capacity of the saponin to stimulate and/or potentiate the endosomal escape of (effector) molecules when such a saponin co-localizes in a cell, in the endosome of said cell, with these (effector) molecules such as oligonucleotides. Therefore, saponin conjugates of the invention or the oligonucleotide conjugates of the invention comprising saponin which has an aglycone with an aldehyde group is preferred. In quillaic acid and in gypsogenin the aldehyde group is at the $C_{23}$ atom of the aglycone.

An embodiment is the saponin conjugate of the invention or the oligonucleotide conjugate of the invention, wherein the saponin is selected from the group consisting of: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin Ia, Teaseed saponin I, Teaseedsaponin J, Assamsaponin F, Digitonin, Primula acid 1 and AS64R, stereoisomers thereof, derivatives thereof, and combinations thereof, preferably the saponin is selected from the group consisting of QS-21, a QS-21 derivative, SO1861, a SO1861 derivative, SO1832, a SO1832 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative. More preferred, the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative, SO1861 and combinations thereof, most preferably the saponin is a SO1861 derivative or SO1861, or a SO1832 derivative or SO1832. Such derivatives of the saponins are endosomal escape enhancing derivatives, to the same or similar extent as their non-derivatized natural counterparts.

A preferred embodiment is the oligonucleotide conjugate of the invention and/or the saponin conjugate of the invention, wherein the saponin comprised by the conjugate(s) is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Gypsophila elegans* saponin mixture, or a saponin isolated from *Gypsophila elegans;*
- Saponinum album saponin mixture, or a saponin isolated from Saponinum album;
- *Saponaria officinalis* saponin mixture, or a saponin isolated from *Saponaria officinalis;* and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or

c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, 10 SO1832, SO1861, SO1862, SO1904, QS-7, QS-7 api, QS-17, QS-18, QS-21 A-apio, QS-21 A-xylo, QS-21 B-apio and QS-21 B-xylo; or

d) a saponin comprising a 12, 13-dehydrooleanane type aglycone core structure without an aldehyde group at the C-23 position of the aglycone, selected from list D:
Aescin Ia, aescinate, alpha-Hederin, AMA-1, AMR, AS6.2, AS64R, Assamsaponin F, dipsacoside B, esculentoside A, macranthoidin A, NP-005236, NP-012672, Primula acid 1, saikosaponin A, saikosaponin D, Teaseed saponin I and Teaseedsaponin J,

preferably, the saponin is any one or more of a saponin selected from list A, B or C, more preferably, a saponin

selected from list B or C,
even more preferably, a saponin selected from list C.

Preferred is the oligonucleotide conjugate of the invention and/or the saponin conjugate of the invention, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* Saponarioside B, SO1542, SO1584, SO1658, SO1674, SO1700, SO1730, SO1772, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

A more particular embodiment is the oligonucleotide conjugate of the invention and/or the saponin conjugate of the invention, wherein the saponin is a saponin isolated from *Saponaria officinalis,* preferably wherein the saponin is any one or more of Saponarioside B, SO1542, SO1584, SO1658, SO1674, SO1700, SO1730, SO1772, SO1832, SO1861, SO1862 and SO1904; more preferably wherein the saponin is any one or more of SO1542, SO1584, SO1658, SO1674, SO1700, SO1730, SO1772, SO1832, SO1861, SO1862 and SO1904; even more preferably wherein the saponin is any one or more of SO1832, SO1861 and SO1862; even more preferably wherein the saponin is SO1832 and SO1861; most preferably being SO1861.

Such saponins of the triterpene glycoside type are capable of enhancing the endosomal escape of (effector) molecules such as oligonucleotides comprised by the oligonucleotide conjugate of the invention, that are present in the endosome (or lysosome) of a cell, when the saponin co-localizes with such (effector) molecule inside the cell. The inventors established that the endosomal escape enhancing activity of these saponins is at least about 5 times more potent, that is to say at least about 10 times more potent, such as 10 - 1000 times more potent or about 100 to 1000 times more potent when the saponin is contacted with a cell when the saponin is comprised by the saponin conjugate of the invention or when the saponin is comprised by the oligonucleotide conjugate of the invention. The free saponin is capable of stimulating the delivery of (effector) molecules in the cytosol of cells, when such cells are contacted with the (effector) molecules and the saponin, at 100-1000 times higher saponin concentration, compared to the concentration of the same saponin which is comprised by the saponin conjugate of the invention or which is comprised by the oligonucleotide conjugate of the invention, required to achieve the same extent of delivery of the (effector) molecule, such as an oligonucleotide selected from an ASO or an siRNA, from outside the cell to inside the endosome and finally in the cytosol and/or in the nucleus of said cell. Saponins which display such endosomal escape enhancing activity are listed in Table A1, as well as saponins with high structural similarity with saponins for which the ability to potentiate the cytosolic delivery of (effector) molecules has been established. When the saponin is part of the saponin conjugate of the invention or is part of the oligonucleotide conjugate of the invention, the targeted delivery of the saponin upon binding of the ligand, such as a single GalNAc moiety or a cluster of three covalently coupled GalNAc moieties, for ASGPR, such as ASGPR1, to the cell-surface binding site on the target cell, preferably a liver cell, on said cell, and after endocytosis, into the endosome of said cell, is thus at least 5-10 times more effective (potent), such as about 100 to 1000 times more effective (potent) compared to contacting the same cell with free, untargeted saponin which is not provided with a ligand for ASGPR for binding to ASGPR of a target cell.

Typically, for the saponin conjugate of the invention and for the oligonucleotide conjugate of the invention, the covalently bound saponin in the conjugate is cleaved off (released) from the conjugate once the conjugate is taken up by the ASGPR bearing cell and transported into the endosome. In the endosome, the chemical conditions and/or pH are such that the covalent bond between the saponin(s) and the remainder of the conjugate is broken and the saponin is released in free form in the endosome. It is preferred that the cleaved-off saponin has its natural chemical structure. For example, when the aldehyde group of a saponin is implied in covalent coupling of the saponin to the GalNAc moiety/moieties (typically via a linker) and/or to the oligonucleotide (typically via a linker), it is preferred that the saponin that is released from the conjugate in the endosome again comprises this aldehyde group, formed under influence of the de-coupling chemistry. Without wishing to be bound by any theory, it is believed that the saponin in its free form (preferably with a freely accessible aldehyde group, preferably with an aldehyde function in position C-23 of the aglycone core structure of the saponin) exerts optimal endosomal escape enhancing activity in the endosome. Saponin is for example cleaved off from conjugates in which the saponin is bound via a hydrazone bond or a semicarbazone bond, under influence of the acidic pH in the endosome of cells such as mammalian cells such as human cells, e.g. tumor cells, liver cells. Therefore, it is preferred that the saponin is covalently bound in the conjugates of the invention via a cleavable bond, preferably a cleavable covalent bond that is cleaved inside the endosome of a cell, such as a bond that is cleaved due to the acidic conditions in the endosome.

| TABLE A1. Saponins displaying (late) endosomal/lysosomal escape enhancing activity, and with an aglycone core of the 12,13-dehydrooleanane type[4)] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| NP-017777 | Gypsogenin | Gal-(1→2)-[Xyl-(1→3)]-GlcA- | Xyl-(1→4)-Rha-(1→2)-[R-(→4)]-Fuc-(R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc-(R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc-Fuc- |
| NP-018110[c], NP-017772[d] | Gypsogenin | Gal-(1→2)-[Xyl-(1→3)]-GlcA- | Xyl-(1→4)-[Glc-(1→3)]-Rha-(1→2)-3,4-di-OAc-Fuc- |
| NP-018109 | Gypsogenin | Gal-(1→2)-[Xyl-(1→3)]-GlcA- | Xyl-(1→4)-[Glc-(1→3)]-Rha-(1→2)-[R-(→4)]-3-OAc-Fuc-(R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1→4)]-Rha/Fuc- |
| SA1641[a], AE X55[b] | Gypsogenin | Gal-(1→2)-[Xyl-(1→3)]-GlcA- | Xyl-(1→3)-Xyl-(1→4)-Rha-(1→2)-[Qui-(1→4)]-Fuc- |
| SO1658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| gypsoside A[6)] | Gypsogenin | Gal-(1→4)-Glc (1→4)-[Ara-(1→3)]--GlcA- | Xyl-(1→3)-Fuc-(1→4)-[Xyl-(1→3)-Xyl-(1→3)]-Rha- |
| phytolaccagenin | Gypsogenin | absent | absent |
| | | | |
| Gypsophila saponin 1 (Gyp1) | Quillaic acid | Gal-(1→2)-[Xyl-(1→3)]-GlcA- | Glc-(1→3)-[Xyl-(1→4)]-Rha-(1→2)-Fuc- |
| NP-017674 | Quillaic acid | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc- |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |

(continued)

| ***TABLE A1. Saponins displaying (late) endosomal/lysosomal escape enhancing activity, and with an aglycone core of the 12,13-dehydrooleanane type[4)]*** | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017706 | Quillaic acid | Gal-(1→2)-[Xyl-(1→3)]-GlcA- | Api-(1→3)-Xyl-(1→4)-Rha-(1→2)-[Rha-(1→3)]-4-OAc-Fuc- |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc--Rha-(1→3)]-Fuc- |
| SA1657 | Quillaic acid | Gal-(1→2)-[Xyl-(1→3)]-GlcA- | Xyl-(1→3)-Xyl-(1→4)-Rha-(1→2)-[Qui-(1→4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | Gal-(1→2)-[Xyl-(1→3)]-GlcA- | Glc-(1→3)-[Xyl-(1→4)]-Rha-(1→2)-Fuc- |
| SO1584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1700[3)] | Quillaic acid | Gal-(1→2)-[Xyl-(1→3)]-GlcA- | Xyl-(1→4)-Rha-(1→2)-[Xyl-(1→3)-4-OAc-Qui-(1→4)]-Fuc- |

(continued)

| TABLE A1. Saponins displaying (late) endosomal/lysosomal escape enhancing activity, and with an aglycone core of the 12,13-dehydrooleanane type[4)] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| Saponarioside B[1)] | Quillaic acid | Gal-(1→2)-[Xyl-(1→3)]-GlcA- | Xyl-(1→3)-Xyl-(1→4)-Rha-(1→2)-[4-OAc-Qui-(1→4)]-Fuc- |
| SO1730[3)] | Quillaic acid | Gal-(1→2)-[Xyl-(1→3)]-GlcA- | Glc-(1→3)-Xyl-(1→4)-Rha-(1→2)-[-4-OAc-Qui-(1→4)]-Fuc- |
| SO1772[3)] | Quillaic acid | Gal-(1→2)-[Xyl-(1→3)]-GlcA- | 6-OAc-Glc-(1→3)-[Xyl-(1→4)]-Rha-(1→2)-[4-OAc-Qui-(1→4)]-Fuc- |
| SO1832[1)] (protonated SO1831) = Saponarioside A | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| SO1861 (deprotonated SO1862) | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| SO1862 (protonated SO1861), also referred to as *Sapofectosid*[5)] | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| SO1904[3)] | Quillaic acid | Gal-(1→2)-[Xyl-(1→3)]-GlcA- | 6-OAc-Glc-(1→3)-[Xyl-(1→4)]-Rha-(1→2)-[Xyl-(1→3)-4-OAc-Qui-(1→4)]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |

(continued)

| *TABLE A1. Saponins displaying (late) endosomal/lysosomal escape enhancing activity, and with an aglycone core of the 12,13-dehydrooleanane type[4)]* | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 | Quillaic acid | Gal-(1→2)-[Xyl-(1→3)]-GlcA- | Combination of the carbohydrate chains depicted for QS-21 A-apio, A-xylo, B-apio, B-xylo, for this position at the aglycone (see also the structure depicted as (Scheme Q)) |
| Agrostemmoside E (AG1856, AG2.8)[2)] | Quillaic acid | Gal-(1→2)-[Xyl-(1→3)]-GlcA- | [4,6-di-OAc-Glc-(1→3)]-[Xyl-(1→4)]-Rha-(1→2)-[3,4-di-OAc-Qui-(1→4)]-Fuc- |
| | | | |
| **Saponin Name** | **Aglycone core without an aldehyde group at the C-23 position** | **Carbohydrate substituent at the C-3beta-OH group** | **Carbohydrate substituent at the C-28-OH group** |
| NP-005236 | 2alpha-Hydroxyoleanolic acid | GlcA- | Glc/Gal- |
| AMA-1 | 16alpha-Hydroxyoleanolic acid | Glc- | Rha-(1→2)-[Xyl-(1→4)]-Rha- |
| AMR | 16alpha-Hydroxyoleanolic acid | Glc- | Rha-(1→2)-[Ara-(1→3)-Xyl-(1→4)]-Rha- |
| alpha-Hederin | Hederagenin (23-Hydroxyoleanolic acid) | Rha-(1→2)-Ara- | Not present |

(continued)

| Saponin Name | Aglycone core without an aldehyde group at the C-23 position | Carbohydrate substituent at the C-3beta-OH group | Carbohydrate substituent at the C-28-OH group |
|---|---|---|---|
| NP-012672 | 16alpha,23-Dihydroxyoleanolic acid | Ara/Xyl-(1→4)-Rha/-Fuc-(1→2)-Glc/-Gal-(1→2)-Rha/-Fuc-(1→2)-GlcA- | Ara/Xyl- |
| beta-Aescin (described: Aescin Ia) | Protoaescigenin-21 (2-methylbut-2-enoate)-22-acetat | Glc-(1→2)-[Glc-(1→4)]-GlcA- | Not present |
| aescinate | Aglycone core without an aldehyde group at the C-23 position | present | Not present |
| dipsacoside B | Aglycone core without an aldehyde group at the C-23 position | present | present |
| esculentoside A | Aglycone core without an aldehyde group at the C-23 position | present | Not present |
| Teaseed saponin I | 23-Oxo-barringtogenol C - 21,22-bis(2-methylbut-2-enoate) | Glc-(1→2)-Ara-(1→3)-[Gal-(1→2)]-GlcA- | Not present |
| Teaseedsaponin J | 23-Oxo-barringtogenol C - 21,22-bis(2-methylbut-2-enoate) | Xyl-(1→2)-Ara-(1→3)-[Gal-(1→2)]-GlcA- | Not present |
| Assamsaponin F | 23-Oxo-barringtogenol C - 21 (2-methylbut-2-enoate)-16,22-diacetat | Glc-(1→2)-Ara-(1→3)-[Gal-(1→2)]-GlcA- | Not present |
| Primula acid 1 | 3,16,28-Trihydroxyoleanan-12-en | Rha-(1→2)-Gal-(1→3)-[Glc-(1→2)]-GlcA- | Not present |
| AS64R | Gypsogenic acid | absent | Glc-(1→3)-[Glc-(1→6)]-Gal- |
| Macranthoidin A | Aglycone core without an aldehyde group at the C-23 position | present | present |
| saikosaponin A | Aglycone core without an aldehyde group at the C-23 position | present | absent |
| saikosaponin D | Aglycone core without an aldehyde group at the C-23 position | present | absent |
| | | | |
| | | **Carbohydrate substituent at the C-23-OH group** | |
| AS6.2 | Gypsogenic acid | Gal- | Glc-(1→3)-[Glc-(1→6)]-Gal- |

a, b: Different names refer to different isolates of the same structure

c, d: Different names refer to different isolates of the same structure

1) Jia et al., Major Triterpenoid Saponins from Saponaria officinalis, J. Nat. Prod. 1998, 61, 11, 1368-1373, Publication Date: September 19, 1998, https://doi.org/10.1021/np980167u

2) The structure of Agrostemmoside E (also referred to as AG1856 or AG2.8) is given in Fig. 4 of J. Clochard *et al,* A new acetylated triterpene saponin from Agrostemma githago L. modulates gene delivery efficiently and shows a high cellular tolerance, International Journal of Pharmaceutics, Volume 589, 15 November 2020, 119822.

(continued)

| Saponin Name | Aglycone core without an aldehyde group at the C-23 position | Carbohydrate substituent at the C-3beta-OH group | Carbohydrate substituent at the C-28-OH group |
|---|---|---|---|
| 3) Structures of SO1700, SO1730, SO1772, SO1904 are given in Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224)<br>4) See for example:<br>- thesis by Dr Stefan Böttger (2013): Untersuchungen zur synergistischen Zytotoxizität zwischen Saponinen und Ribosomen inaktivierenden Proteinen Typ I, and<br>- Sama et al., Structure-Activity Relationship of Transfection-Modulating Saponins - A Pursuit for the Optimal Gene Trafficker, Planta Med. Volume 85, pp. 513-518, 2019 (doi:10.1055/a-0863-4795) and<br>- Fuchs et al., Glycosylated Triterpenoids as Endosomal Escape Enhancers in Targeted Tumor Therapies, Biomedicine, Volume 5, issue 14, 2017 (doi:10.3390/biomedicines5020014).<br>5) Sama et al., Sapofectosid - Ensuring non-toxic and effective DNA and RNA delivery, International Journal of Pharmaceutics, Volume 534, Issues 1-2, 20 December 2017, Pages 195-205 (dx.doi.org/10.1016/j.ijpharm.2017.10.016) & Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224).<br>6) See for example: doi:10.1016/s0040-4039(01)90658-6, Tetrahedron Letters No. 8, pp. 477-482, 1963 and pubchem.ncbi.nlm.nih.gov/compound/Gipsoside | | | |

It is preferred that the saponin of the saponin conjugate and/or the saponin of the oligonucleotide conjugate of the invention is a saponin derived from a plant. The saponin comprised by the saponin conjugate of the invention and/or the saponin comprised by the oligonucleotide conjugate of the invention is preferably a saponin isolated from a plant. The term 'plant' is to be understood to include plants and trees. For example, the saponin is isolated from the root of a plant or from the bark of a tree. For example, the saponin is isolated from the root of a plant. Examples of such plants from which the saponin is derived (isolated) are *Quillaja saponaria, Gypsophila paniculate L., Saponaria officinalis* such as *Saponaria officinalis* L. and *Gypsophila elegans* such as *Gypsophila elegans* M. Bieb. Preferably, the saponin conjugate and/or the oligonucleotide conjugate comprise a single type of saponin, preferably a single type of saponin derived from plant material such as the roots of a plant, such as SO1861, SO1862 or SO1832 from *Saponaria officinalis* (e.g., *Saponaria officinalis* L., preferably *Saponaria officinalis L.)* (roots) or such as a QS-21, a QS-7 or a QS-17 from *Quillaja saponaria* (roots).

Suitable sources for isolating saponins according to the invention, i.e. those that display endosomal escape enhancing activity, are *Quillaja saponaria, Gypsophila paniculate L., Saponaria officinalis* and *Gypsophila elegans,* and Quillaja bark. Saponin suitable for the saponin derivatives of the invention and for the saponin conjugate of the invention are thus for example:

*Quillaja saponaria* saponin, saponin isolated from *Quillaja saponaria,* for example Quil-A, QS-17-api, QS-17-xyl, QS-21, QS-21A, QS-21B, QS-7-xyl,
Saponinum album, saponin isolated from Saponinum album.
*Saponaria officinalis* saponin, saponin isolated from *Saponaria officinalis* (preferred),
Quillaja bark saponin, saponin isolated from Quillaja bark saponin, for example Quil-A, QS-17-api, QS-17-xyl, QS-21, QS-21A, QS-21B, QS-7-xyl.

In addition, apart from QS-21, also the individual saponins present in QS-21 are suitable saponins for the saponin

conjugate of the invention, i.e. the saponins depicted as the saponins of SCHEME Q:

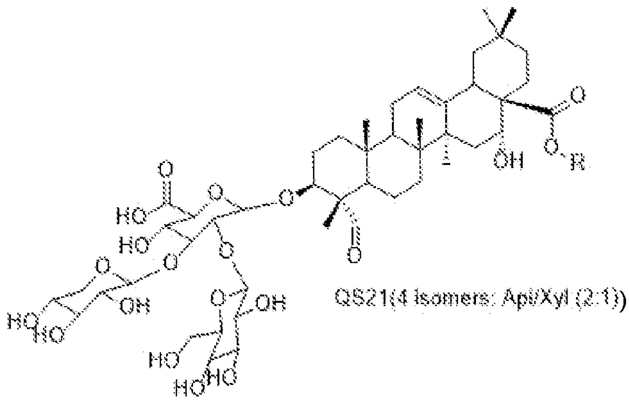

QS21(4 isomers: Api/Xyl (2:1))

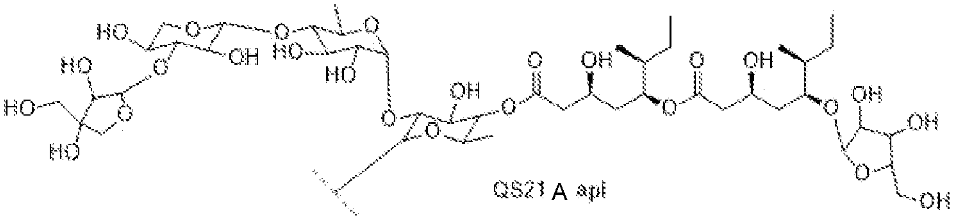

QS21 A api

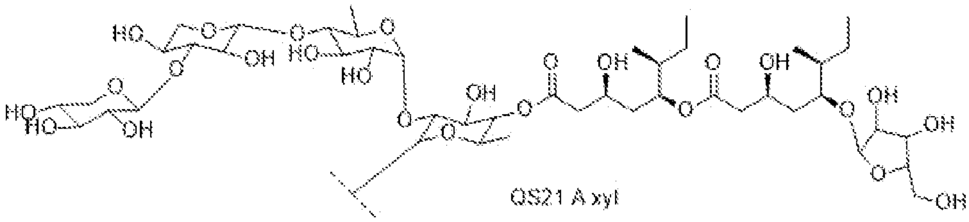

QS21 A xyl

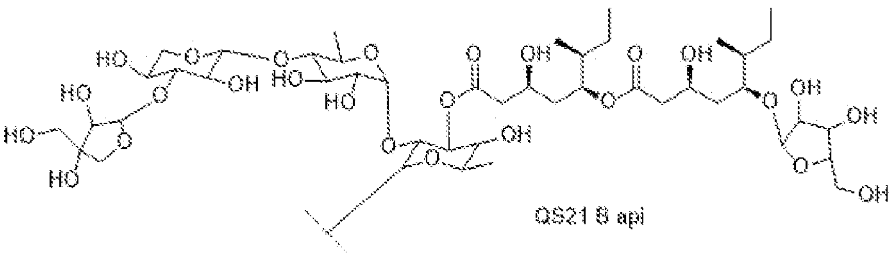

QS21 B api

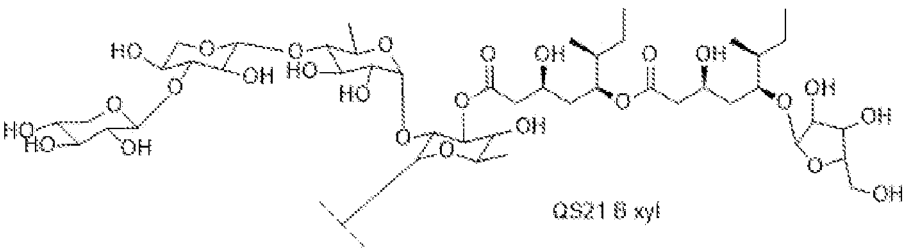

QS21 B xyl

**(Scheme Q)**

Thus, for all embodiments of the invention, the saponin, preferably a single type of saponin, is preferably a saponin isolated from plant material such as derived from the roots of a plant.

An embodiment is the oligonucleotide conjugate of the invention, wherein the saponin comprised by the oligonucleotide conjugate is isolated from a plant. Preferably, the saponin is isolated from a part of a plant, such as the root, or from a part of a tree, such as the bark. Preferably, the saponin is isolated from roots derived from a plant.

In embodiments the saponin conjugate or the oligonucleotide conjugate comprises at least one saponin, wherein the saponin is a derivative wherein

i. the aglycone core structure of the saponin comprises an aldehyde group which has been derivatised;
ii. a saccharide chain of the saponin, preferably the saccharide chain selected from group A comprises a carboxyl group which has been derivatised;
iii. a saccharide chain of the saponin, preferably the saccharide chain selected from group B comprises an acetoxy (Me(CO)O-) group which has been derivatised; or
iv. any combination of derivatisations (i), (ii) and/or (iii) is present.

Exemplary derivatisations (i) for an aldehyde group of the aglycone core structure include reduction to an alcohol, such as a primary alcohol; transformation into a hydrazone (functional group) or semicarbazone (functional group); transformation into a hemiacetal; transformation into an acetal; oxidation to a carboxyl; transformation into an imine; transformation into an oxime; transformation into a β-hydroxy ketone; or transformation into an enone, preferably reduction to an alcohol, such as a primary alcohol; or transformation into a hydrazone (functional group) or into a semicarbazone (functional group). The derivatised saponin of the conjugate of the invention still exerts its endosomal escape enhancing activity, once present in the endosome of cells that take up the saponin conjugate or the oligonucleotide conjugate of the invention.

In embodiments the saponin is a derivative wherein the aglycone core structure comprises an aldehyde group which has been derivatised by:

- reduction to an alcohol, such as a primary alcohol; or
- transformation into a hydrazone of formula $R^bHC=NNHR^a$; preferably into a hydrazone of formula $R^bHC=NNH(CO)R^a$

wherein $R^a$ is group comprising less than 20 carbon atoms, preferably less than 10 carbon atoms and $R^b$ is the remainder of the saponin. Preferably, $R^a$ is an N-alkylated maleimide. In particular embodiments, the aldehyde group has been transformation into a hydrazone bond through reaction with N-ε-maleimidocaproic acid hydrazide (EMCH); N-[β-maleimidopropionic acid] hydrazide (BMPH); or N-[κ-maleimidoundecanoic acid] hydrazide (KMUH).

Exemplary derivatisations (ii) for a carboxyl group of a saccharide chain include: reduction to an alcohol, such as a primary alcohol; transformation into an amide; transformation into an ester; transformation into an amine, such as a primary or secondary amine; or decarboxylation, preferably reduction to an alcohol, such as a primary alcohol; transformation into an amide; or transformation into an ester; more preferably transformation into an amide.

Preferably, the carboxyl group which is derivatised is part of a glucuronic acid moiety and, preferably, the saccharide chain is selected from group A.

In embodiments the saponin is a derivative wherein a saccharide chain, preferably a saccharide chain selected from group A, comprises a carboxyl group, preferably a carboxyl group of a glucuronic acid moiety, which has been derivatised by:

- reduction to an alcohol, such as a primary alcohol;
- transformation into an amide of formula $R^aNH(CO)R^b$; or
- transformation into an ester of formula $R^aO(CO)R^b$

wherein $R^a$ is group comprising less than 20 carbon atoms, preferably less than 10 carbon atoms and $R^b$ is the remainder of the saponin. Preferably, $R^a$ is an N-alkylated maleimide or a diol. In particular embodiments, the carboxyl group has been derivatised by transformation into an amide bond through reaction with 2-amino-2-methyl-1,3-propanediol (AMPD) or N-(2-aminoethyl)maleimide (AEM).

Exemplary derivatisations (iii) for an acetoxy group of a saccharide chain include: transformation into an alcohol, such as a secondary alcohol, by deacetylation, optionally followed by transformation of the resulting alcohol into an ether; ester or ketone; preferably transformation into an alcohol, such as a secondary alcohol, by deacetylation.

Preferably, the acetoxy group which is derivatised is part of a saccharide chain selected from group B.

In embodiments the saponin is a derivative wherein a saccharide chain, preferably a saccharide chain selected from group B, comprises an acetoxy group which has been derivatised by:

- transformation into an alcohol, such as a secondary alcohol, by deacetylation; or
- transformation into an alcohol, such as a secondary alcohol, by deacetylation followed by transformation of the resulting alcohol into an ether of formula $R^aOR^b$, an ester of formula $R^a(CO)OR^b$, or a ketone of formula $R^a(CO)R^b$

wherein $R^a$ is group comprising less than 20 carbon atoms, preferably less than 10 carbon atoms and $R^b$ is the remainder of the saponin. Preferably, $R^a$ is an N-alkylated maleimide or a diol.

Hence, in embodiments the saponin is a derivative wherein:

ii. the aglycone core structure comprises an aldehyde group which has been derivatised by:

- reduction to an alcohol;
- transformation into a hydrazone bond through reaction with N-ε-maleimidocaproic acid hydrazide (EMCH);
- transformation into a hydrazone bond through reaction with N-[β-maleimidopropionic acid] hydrazide (BMPH); or
- transformation into a hydrazone bond through reaction with N-[κ-maleimidoundecanoic acid] hydrazide (KMUH);

iii. the saccharide chain selected from group A comprises a carboxyl group, preferably a carboxyl group of a glucuronic acid moiety, which has been derivatised by transformation into an amide bond through reaction with 2-amino-2-methyl-1,3-propanediol (AMPD) or *N-(2*-aminoethyl)maleimide (AEM);
iv. the saccharide chain selected from group B comprises an acetoxy group (Me(CO)O-) which has been derivatised by transformation into a hydroxyl group (HO-); or
v. any combination of derivatisations (i), (ii) and/or (iii) is present.

An embodiment is the saponin conjugate of the invention or the oligonucleotide conjugate of the invention, wherein the saponin is a saponin derivative wherein

i. the saponin derivative comprises an aglycone core structure comprising an aldehyde group which has been derivatised;
ii. the saponin derivative comprises a saccharide chain, preferably a saccharide chain selected from group A, the saccharide chain comprising a carboxyl group which has been derivatised;
iii. the saponin derivative comprises a saccharide chain, preferably a saccharide chain selected from group B, the saccharide chain comprising an acetoxy (Me(CO)O-) group which has been derivatised; or
iv. the saponin derivative comprises any combination of derivatisations i., ii. and iii., preferably any combination of two derivatisations of derivatisations i., ii. and iii.

An embodiment is the saponin conjugate of the invention or the oligonucleotide conjugate of the invention, wherein the saponin is any one or more of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SO1832, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative. Most preferably the saponin is selected from the group consisting of a QS-21 derivative, a SO1861 derivative, SO1861 and combinations thereof, even more preferably the saponin is a SO1861 derivative or SO1861, or a SO1832 derivative or SO1832.

An embodiment is the saponin conjugate of the invention or the oligonucleotide conjugate of the invention, wherein the saponin is a saponin derivative of the quillaic acid saponin or the gypsogenin saponin according to the invention and is represented by Molecule 1:

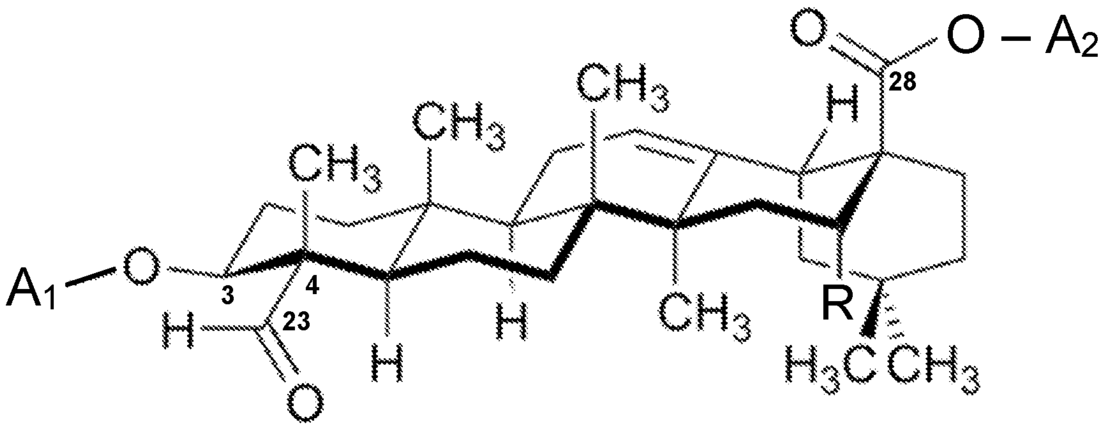

(Molecule 1)

wherein

$A_1$ represents hydrogen, a monosaccharide or a linear or branched oligosaccharide, preferably $A_1$ represents a saccharide chain selected from group A, more preferably $A_1$ represents a saccharide chain selected from group A and $A_1$ comprises or consists of a glucuronic acid moiety;
$A_2$ represents hydrogen, a monosaccharide or a linear or branched oligosaccharide, preferably $A_2$ represents a saccharide chain selected from group B, more preferably $A_2$ represents a saccharide chain selected from group B and $A_2$ comprises at least one acetoxy (Me(CO)O-) group, such as one, two, three or four acetoxy groups,

wherein at least one of $A_1$ and $A_2$ is not hydrogen, preferably both $A_1$ and $A_2$ are an oligosaccharide chain;
and R is hydrogen in gypsogenin or hydroxyl in quillaic acid;
wherein the saponin derivative corresponds to the saponin represented by Molecule 1 wherein at least one, preferably one or two, more preferably one, of the following derivatisations is present:

i. the aldehyde group at position $C_{23}$ of the quillaic acid or gypsogenin has been derivatised;
ii. the carboxyl group of a glucuronic acid moiety of $A_1$, when $A_1$ represents a saccharide chain selected from group A and $A_1$ comprises or consists of a glucuronic acid moiety, has been derivatised; and
iii. one or more, preferably all, of acetoxy group(s) of one saccharide moiety or of two or more saccharide moieties of $A_2$, when $A_2$ represents a saccharide chain selected from group B and $A_2$ comprises at least one acetoxy group, has/have been derivatised.

An embodiment is the saponin conjugate of the invention or the oligonucleotide conjugate of the invention, wherein $A_1$ represents a saccharide chain selected from group A and comprises or consists of a glucuronic acid moiety and wherein the carboxyl group of a glucuronic acid moiety of $A_1$ has been derivatised and/or wherein $A_2$ represents a saccharide chain selected from group B and $A_2$ comprises at least one acetoxy group and wherein at least one acetoxy group of $A_2$ has been derivatised.
An embodiment is the saponin conjugate of the invention or the oligonucleotide conjugate of the invention, wherein the saponin represented by Molecule 1 is a bidesmosidic triterpene saponin.
An embodiment is the saponin conjugate of the invention or the oligonucleotide conjugate of the invention, wherein the saponin derivative corresponds to the saponin represented by Molecule 1 wherein at least one, preferably one or two, more preferably one, of the following derivatisations is present:

i. the aldehyde group at position $C_{23}$ of the quillaic acid or gypsogenin has been derivatised by;

- reduction to an alcohol;
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
- transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or

- transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;

ii. the carboxyl group of a glucuronic acid moiety of $A_1$, when $A_1$ represents a saccharide chain selected from group A and $A_1$ comprises or consists of a glucuronic acid moiety, has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of $A_2$, when $A_2$ represents a saccharide chain selected from group B and $A_2$ comprises at least one acetoxy group, has/have been derivatised by transformation into a hydroxyl group (HO-) by deacetylation.

An embodiment is the saponin conjugate of the invention or the oligonucleotide conjugate of the invention, wherein $A_1$ is Gal-(1→2)-[Xyl-(1→3)]-GlcA and/or $A_2$ is Glc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)-alpha-L-rhamnopyranosyl-(1→2)-[beta-D-xylopyranosyl-(1→3)-4OAc-beta-D-quinovopyranosyl-(1→4)]-beta-D-fucopyranoside, more preferably the saponin is any one or more of: SO1861, SO1832, GE1741, SA1641 and QS-21, or a derivative thereof, most preferably SO1861 or a derivative thereof, or an SO1832 derivative or SO1832.
An embodiment is the saponin conjugate of the invention or the oligonucleotide conjugate of the invention, wherein the saponin is a saponin derivative wherein

i. the saponin derivative comprises an aglycone core structure comprising an aldehyde group which has been derivatised by:

- reduction to an alcohol;
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
- transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;

ii. the saponin derivative comprises a saccharide chain, preferably a saccharide chain selected from group A, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu- AMPD or a SO1861-Glu-AMPD or a saponin-Glu-AEM such as a QS-21-Glu-AEM or a SO1861-Glu-AEM;
iii. the saponin derivative comprises a saccharide chain, preferably a saccharide chain selected from group B, the saccharide chain comprising an acetoxy (Me(CO)O-) group which has been derivatised by transformation into a hydroxyl group (HO-) by deacetylation; or
iv. the saponin derivative comprises any combination of derivatisations i., ii. and iii., preferably any combination of two derivatisations of derivatisations i., ii. and iii.;

preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thio-ether bond with mercaptoethanol.
An embodiment is the saponin conjugate of the invention or the oligonucleotide conjugate of the invention, wherein the saponin is a saponin derivative wherein

i. the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH;
ii. the saponin derivative comprises a saccharide chain, preferably a saccharide chain selected from group A, the

saccharide chain comprising a carboxyl group, preferably a carboxyl group of a glucuronic acid moiety which has been derivatised by transformation into an amide bond through reaction with N-(2-aminoethyl)maleimide (AEM), therewith providing a saponin-Glu-AEM such as a QS-21-Glu-AEM or a SO1861-Glu-AEM; or

iii. the saponin derivative comprises a combination of derivatisations i. and ii.

An embodiment is the saponin conjugate of the invention or the oligonucleotide conjugate of the invention, wherein the saponin derivative comprises an aglycone core structure wherein the aglycone core structure comprises an aldehyde group and wherein the saponin derivative comprises a saccharide chain, preferably a saccharide chain selected from group A, the saccharide chain comprising a carboxyl group, preferably a carboxyl group of a glucuronic acid moiety, which glucuronic acid moiety has been derivatised by transformation into an amide bond through reaction with N-(2-aminoethyl)maleimide (AEM).

An embodiment is the saponin conjugate of the invention or the oligonucleotide conjugate of the invention, wherein the saponin derivative is represented by Molecule 2:

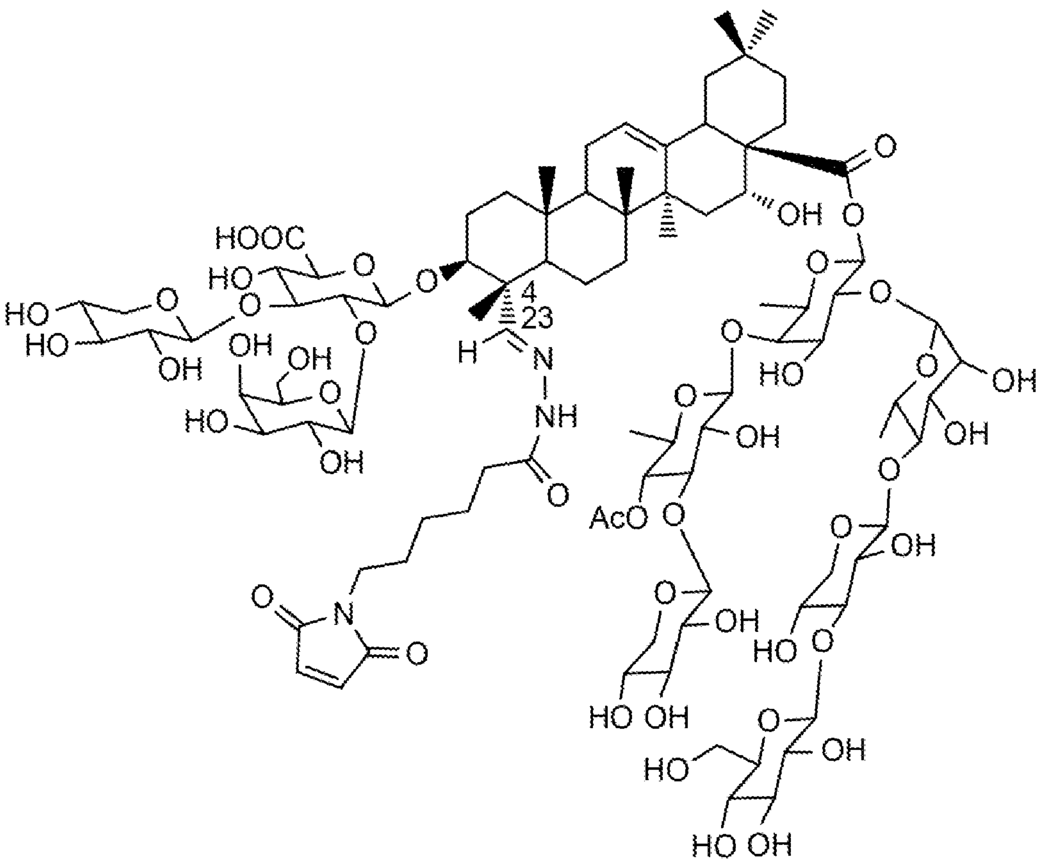

(Molecule 2)

or wherein the saponin derivative is represented by Molecule 3:

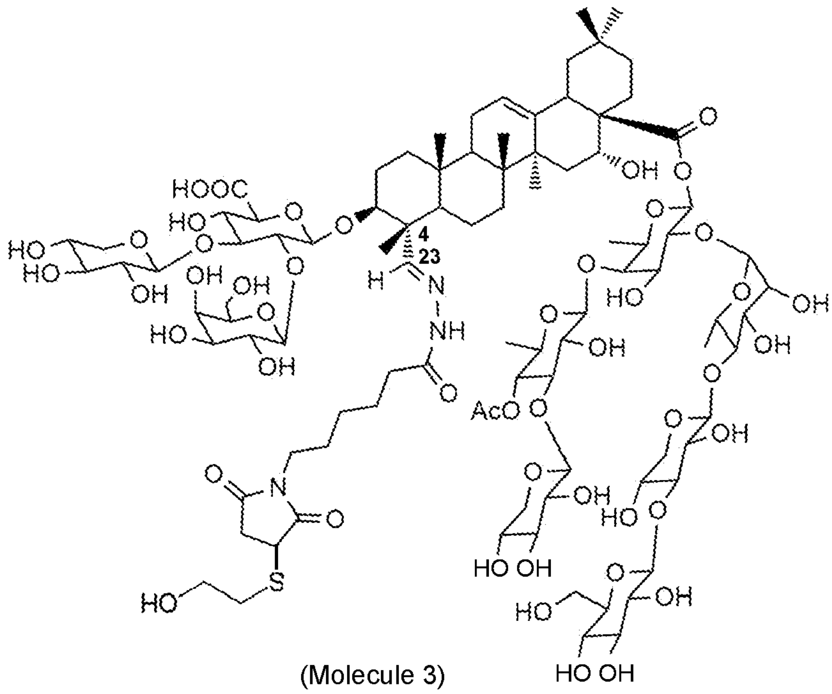

(Molecule 3)

An embodiment is the saponin conjugate of the invention or the oligonucleotide conjugate of the invention, wherein the at least one saponin and the ligand for ASGPR are covalently linked directly or via at least one linker. An embodiment is the oligonucleotide conjugate of the invention, wherein the at least one saponin and the ligand for ASGPR are covalently linked directly or via at least one linker, and/or wherein the at least one saponin and the oligonucleotide are covalently linked directly or via at least one linker, and/or wherein the ligand for ASGPR and the oligonucleotide are covalently linked directly or via at least one linker, preferably, the at least one saponin, the ligand for ASGPR and the oligonucleotide are linked via at least one linker.

An embodiment is the saponin conjugate of the invention, wherein the GalNAc moiety is bound to the saponin S, preferably via a saponin linker $L_s$, as represented in formula $(II)_S$:

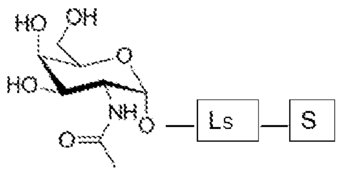

(II)s

An embodiment is the saponin conjugate of the invention, wherein the GalNAc moieties are each separately covalently bound via the oxygen on position "1" of the GalNAc moiety to a central bridging moiety B, which effectively forms a bridge between the GalNAc moieties and the saponin moiety, preferably via a saponin moiety linker $L_S$, as shown in formula $(III)_S$:

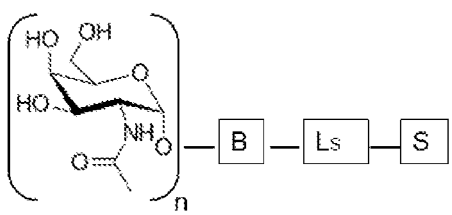

(III)s

wherein n is an integer larger than or equal to 2, preferably n is 3, $L_S$ is a saponin moiety linker and S is the saponin moiety.

An embodiment is the saponin conjugate of the invention, wherein the GalNAc moieties are bound to the bridging moiety B via GalNAc linkers $L_{GAL}$ as shown in formula $(IV)_S$:

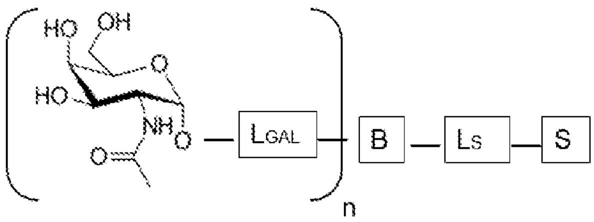

(IV)s

wherein n is an integer larger than or equal to 2, preferably n is 3, $L_S$ is a saponin moiety linker and S is the saponin moiety.

An embodiment is the saponin conjugate of the invention, wherein the saponin moiety linker Ls represents any chemical moiety suitable for covalently binding a saponin to GalNAc as in formula $(II)_S$ or to the bridging moiety B as in formula $(III)_S$ and $(IV)_S$.

An embodiment is the saponin conjugate of the invention, wherein the saponin moiety linker $L_S$ is the result of a coupling reaction between at least a first precursor $L_{S1}$ covalently bound to GalNAc or the bridging moiety B and a second precursor $L_{S2}$ covalently bound to the saponin moiety, wherein $L_{S1}$ is a precursor of the saponin moiety linker $L_S$ which is covalently bound to GalNAc or to the bridging moiety B and $L_{S2}$ is a precursor of the saponin moiety linker $L_S$ which is covalently bound to the saponin moiety.

An embodiment is the saponin conjugate of the invention, wherein the saponin moiety linker $L_S$ is the result of a coupling reaction between at least a first precursor $L_{S1}$ covalently bound to GalNAc or the bridging moiety B and a second precursor $L_{S2}$ covalently bound to the saponin moiety, wherein the coupling reaction is selected from the group consisting of an azide-alkyne cycloaddition, a thiol maleimide coupling, a Staudinger reaction, a nucleophilic ring-opening of strained heterocyclic electrophiles, a carbonyl reaction of the non-aldol type and an addition to a carbon-carbon double bond, preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling.

An embodiment is the saponin conjugate of the invention, wherein the saponin moiety linker $L_S$ is the result of a coupling reaction between a first precursor $L_{S1}$ covalently bound to GalNAc or the bridging moiety B, the first precursor $L_{S1}$ comprising an azide; and a second precursor $L_{S2}$ covalently bound to the saponin moiety, the second precursor $L_{S2}$ comprising an alkyne and preferably comprising a hydrazone resulting from a hydrazide/aldehyde coupling to an aldehyde of the saponin moiety.

An embodiment is the saponin conjugate of the invention, wherein the structure for the precursor $L_{S1}$ is the following azide of formula (XVII):

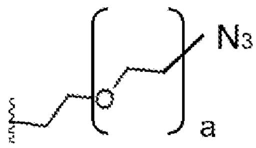

(XVII)

wherein a represents an integer larger than or equal to 0, preferably a represents an integer selected from 1, 2, and 3, more preferably a represents 2, or

wherein the structure for the precursor $L_{S1}$ comprises the following azide of formula (XVIII):

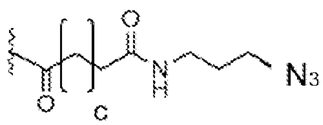

(XVIII)

wherein c represents an integer larger than or equal to 0, preferably c represents an integer in the range of 5-15, more preferably c represents 9.

An embodiment is the saponin conjugate of the invention, wherein the structure for the precursor $L_{S2}$ comprises the following hydrazone with formula (XIX):

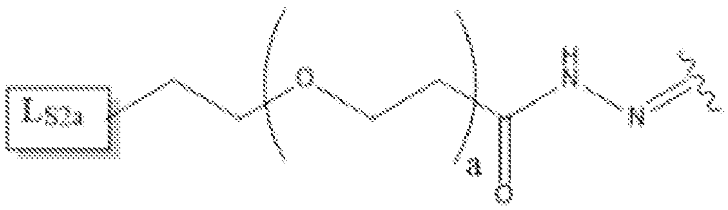

(XIX)

wherein a represents an integer larger than or equal to 0, preferably a represents an integer in the range of 2-6, more preferably a represents 4, and wherein $L_{S2a}$ represents an alkyne-containing moiety.

An embodiment is the saponin conjugate of the invention, wherein $L_{S2a}$ comprises less than 20 carbon atoms, preferably $L_{S2a}$ represents a moiety according to formula (XX):

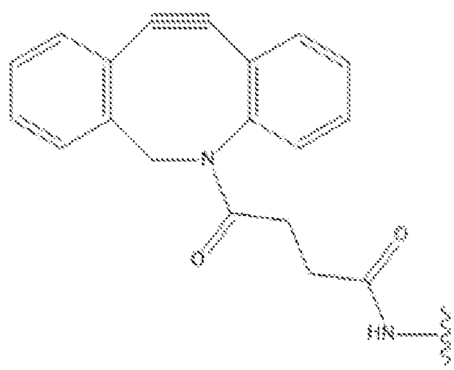

(XX).

An embodiment is the saponin conjugate of the invention, wherein the bridging moiety is a compound of formula (XV):

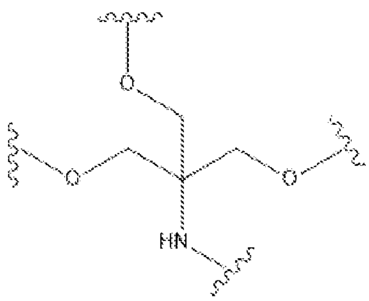

(XV)

wherein the oxygen atoms of the compound of formula (XV) are bound to GalNAc or to the GalNAc linkers $L_{GAL}$, and the nitrogen atom of the compound of formula (XV) is bound to the saponin moiety linker $L_S$.

An embodiment is the saponin conjugate of the invention, wherein $L_{GAL}$ represents any chemical moiety suitable for covalently binding GalNAc to the bridging moiety B.

An embodiment is the saponin conjugate of the invention, wherein $L_{GAL}$ comprises 2-25 carbon atoms, preferably 7-15 carbon atoms, more preferably 11 carbon atoms and wherein $L_{GAL}$ comprises at least one, preferably two amide moieties.

An embodiment is the saponin conjugate of the invention, wherein $L_{GAL}$ is a compound according to formula (XVI):

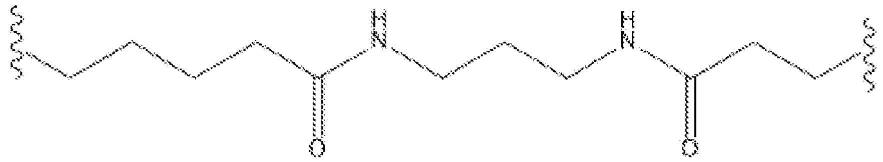

(XVI).

An embodiment is the oligonucleotide conjugate of the invention, wherein the at least one GalNAc moiety, the at least one saponin and the oligonucleotide are covalently bound via a tri-functional linker, preferably with each of the GalNAc moiety, the saponin and the oligonucleotide covalently bound to a separate arm of the tri-functional linker. A preferred oligonucleotide conjugate of the invention is an oligonucleotide conjugate, wherein the at least one GalNAc moiety, preferably three GalNAc moieties, at least one saponin, preferably 1-16 saponin moieties, more preferably 1-8 saponin moieties such as 1, 4 or 8 saponin moieties, and the oligonucleotide are covalently bound via a trifunctional linker, preferably with each of the GalNAc moiety/moieties, the saponin/saponin moieties and the oligonucleotide covalently bound to a separate arm of the trifunctional linker. An example of a trifunctional linker that is suitable for incorporation into an oligonucleotide conjugate of the invention is the trifunctional linker represented by formula (XXI):

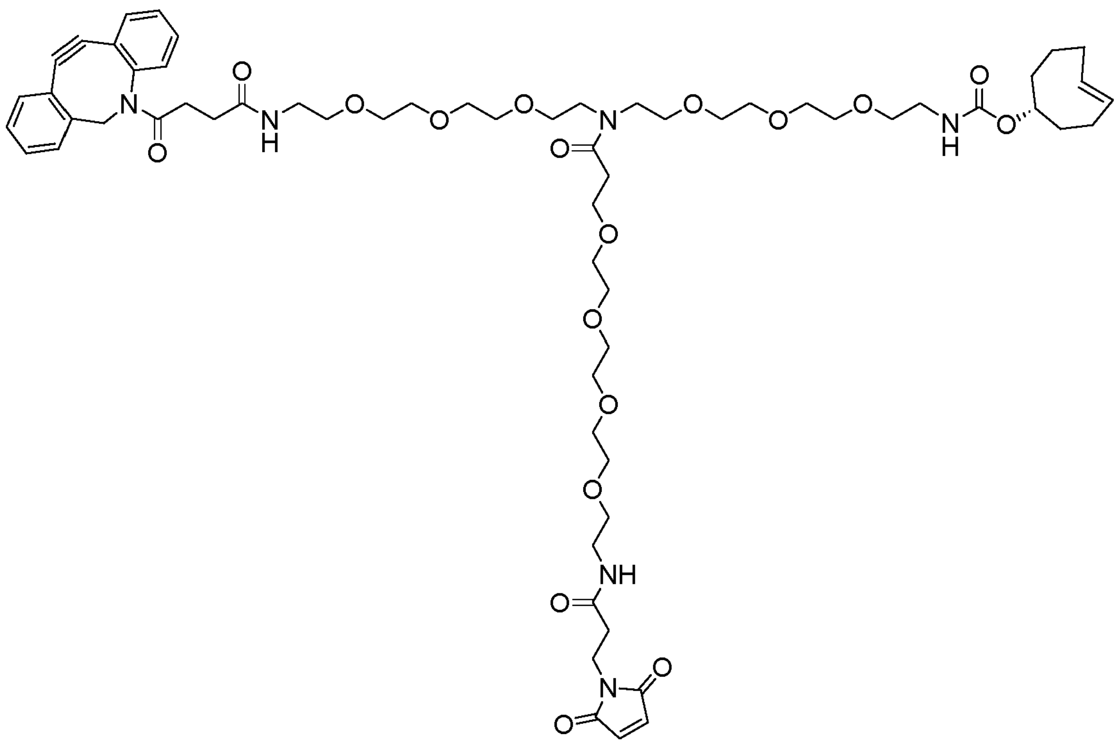

(XXI)

An embodiment is the oligonucleotide conjugate of the invention wherein a trifunctional linker, such as the trifunctional linker represented by formula (XXI), is covalently bound to one or more saponin moieties via a first arm of the linker, preferably 1-16 saponin moieties, more preferably 1-8 saponin moieties such as 1, 4 or 8 saponin moieties, covalently bound to at least one GalNAc moiety, preferably 1-4 GalNAc moieties, more preferably 3 GalNAc moieties, via a second arm of the linker, and covalently bound to an oligonucleotide, preferably an AON such as a BNA or an siRNA, via a third arm of the trifunctional linker.

An embodiment is the saponin conjugate of the invention or the oligonucleotide conjugate of the invention, wherein the ligand for ASGPR is $(GalNAc)_3$Tris represented by Molecule (DD3) or Molecule (DD4):

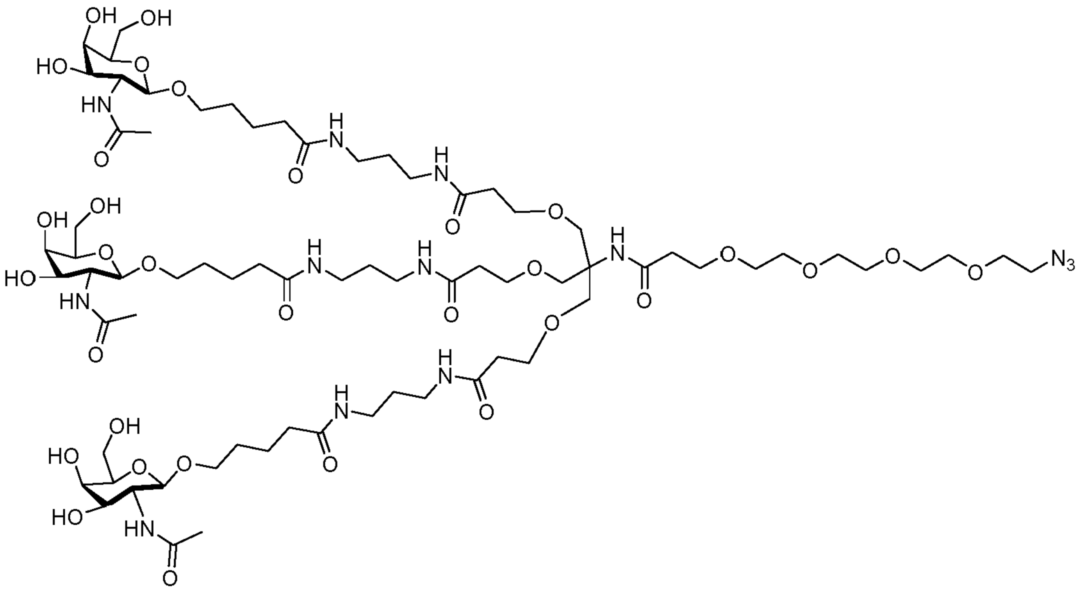

(DD3),

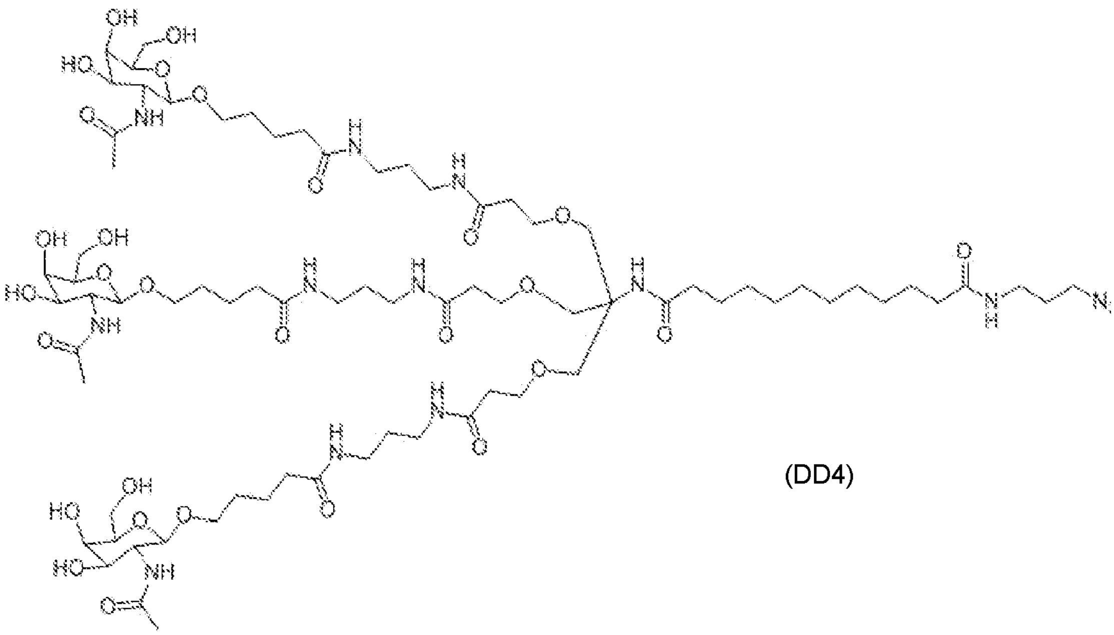

(DD4)

, or wherein the ligand for ASGPR is mono-GalNAc represented by Molecule II':

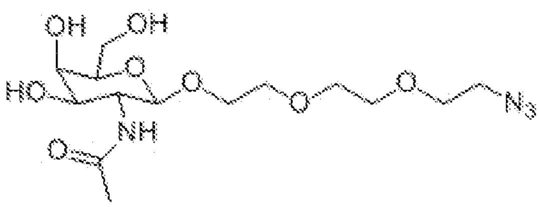

(Molecule II')

.

An embodiment is the saponin conjugate of the invention, wherein the at least one saponin is covalently bound to Molecule (DD3) or Moelcule (DD4) or Molecule II' of the invention via a linker represented by Molecule III':

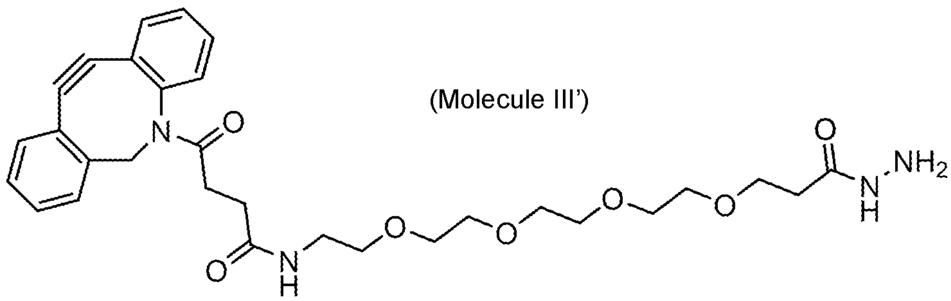

(Molecule III')

, wherein the hydrazide moiety of Molecule III' formed a covalent hydrazone bond with an aldehyde group in the saponin, and wherein the dibenzocyclooctyne group formed a covalent bond with the azide group of Molecule (DD3) ir Molecule (DD4) or Molecule II'.

An embodiment is the oligonucleotide conjugate of the invention, wherein the at least one saponin is covalently bound to the ligand for ASGPR via at least one cleavable linker, and/or wherein the at least one saponin is covalently bound to the oligonucleotide via at least one cleavable linker.

An embodiment is the saponin conjugate of the invention or the oligonucleotide conjugate of the invention, wherein the at least one saponin is covalently bound to the ligand for ASGPR via at least one cleavable linker.

An embodiment is the saponin conjugate of the invention or the oligonucleotide conjugate of the invention, wherein the cleavable linker is subject to cleavage under acidic conditions, reductive conditions, enzymatic conditions and/or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulfide bond.

An embodiment is the saponin conjugate of the invention or the oligonucleotide conjugate of the invention, wherein the cleavable linker is subject to cleavage *in vivo* under acidic conditions such as for example present in endosomes and/or lysosomes of mammalian cells, preferably human cells, preferably the cleavable linker is subject to cleavage *in vivo* at pH 4.0 - 6.5, and more preferably at pH $\leq$ 5.5.

An embodiment is the saponin conjugate of the invention or the oligonucleotide conjugate of the invention, wherein the conjugate comprises 1, 2, 3, 4, 5, 6, 8, 10, 16, 32, 64, 128 or 1-100 saponin moieties, or any number of saponin moieties therein between, such as 7, 9, 12 saponin moieties, and preferably 1, 4 or 8 saponin moieties. Preferred is the saponin SO1861 or SO1832, or a functional derivative thereof, and for example also QS-21 is a suitable saponin, or a functional derivative thereof.

An embodiment is the saponin conjugate of the invention or the oligonucleotide conjugate of the invention, wherein the conjugate comprises 1 saponin moiety. An embodiment is the saponin conjugate of the invention or the oligonucleotide conjugate of the invention, wherein the conjugate comprises 4 or 8 saponin moieties.

One of the many benefits of the conjugates of the invention and the application of the saponin conjugate and the oligonucleotide conjugate of the invention is the possibility to choose and select the number of saponin moieties in the conjugate that is suitable for achieving improved intracellular oligonucleotide activity (such as gene silencing activity), when compared to the activity of the oligonucleotide when taken up by a cell that is not co-contacted with saponin(s) together with the oligonucleotide. The inventors demonstrate (see the Examples section) that incorporation of a single saponin moiety in the conjugates of the invention already suffices for achieving improved intracellular oligonucleotide activity as measured as gene silencing. In further examples it is demonstrated that gene silencing is further improved by for example including 4 or 8 saponin moieties in the conjugate of the invention. The saponin conjugate and the oligonucleotide conjugate provide the freedom to couple any number between 1-64 or even more, such as 128 saponin moieties to the GalNAc moiety or moieties, preferably 3 GalNAc moieties, for the saponin conjugate, or to the GalNAc moiety/moieties and the oligonucleotide, for the oligonucleotide conjugate. As said, the inventors surprisingly found that combining the saponin and the GalNAc, or combining the saponin, the GalNAc and the oligonucleotide, does not hamper biological activity of any of the components in the conjugate. Moreover, increasing the number of saponin moieties in the conjugate can improve the oligonucleotide activity. Without wishing to be bound by any theory, the increased number of saponin moieties increases the endosomal escape enhancing activity of the saponin once taken up by a cell and transferred into the endosome. The administered oligonucleotide, as part of the oligonucleotide conjugate or separate as a GalNAc-oligonucleotide conjugate, that is present in the endosome together with the saponin, is, as a consequence of improved saponin-mediated endosomal escape activity, more efficiently transferred from the endosome into the cytosol, resulting in increased gene silencing activity. For example, the possibility to incorporate one or more saponin moieties in the conjugate allows for tailormade design of the conjugate when optimal gene silencing with a selected oligonucleotide is considered. Oligonucleotides, which transfer relatively inefficiently from the endosome to the cytosol, for example with an efficiency of 1-2%, benefit from

the presence of a single saponin moiety in the conjugate (1:1 ratio in the oligonucleotide conjugate), whereas oligonucleotides which are released into the cytosol from the endosome to a lower extent, may increasingly be transferred into the cytosol by applying more than a single saponin moiety in the conjugate, such as 2-100 moieties, 2-64, 2-34, 2-16, 4-12, 4-8 moieties. A suitable method for incorporating more than one saponin moiety is for example the incorporation of a dendron in the conjugate of the invention, such as a G2, G3 or G4 dendron, for example for binding 4 or 8 or 16 saponin moieties together in a conjugate. In the Examples section, examples of improved gene silencing (extent of silencing at a selected timepoint, duration of silencing of a target gene) are provided for, for example, oligonucleotide conjugates comprising a single saponin moiety, 4 saponin moieties and 8 saponin moieties. The inventors established that by contacting a cell with the oligonucleotide conjugate of the invention, the extent of gene silencing at a certain time point is improved and that also the duration in time of the gene silencing is extended, when compared to gene silencing in cells which are contacted with the oligonucleotide in the absence of saponin.

For the oligonucleotide conjugate of the invention, it is suitable (and preferred) that the conjugate comprises 1 or 3 GalNAc moieties, preferably 3 GalNAc moieties.

According to the invention, the saponin is typically one or more moieties of SO1861 or SO1832 (which is also referred to as SO1831), such as 1-32 moieties of SO1861 or SO1832, preferably 1, 2, 3, 4, 5, 6, 7, 8, 9, 10, 11, 12, 13, 14, 15 or 16 moieties, more preferably 1, 2, 4, 8, 12 or 16 moieties, most preferably 1, 4 or 8 moieties, such as 1, 4 or 8 SO1861 moieties or 1, 4 or 8 SO1832 moieties. The inventors established that SO1832 is about equally active as SO1861 when endosomal escape enhancing activity towards an oligonucleotide (or protein toxin) in the endosome is considered. As outlined here above, the number of saponin moieties can be optimized for the conjugates of the invention, when the extent and duration of gene silencing upon contacting a cell with an oligonucleotide is considered. For the oligonucleotide conjugate of the invention, this also applies to the number of GalNAc moieties in the conjugates of the invention. Typically, optimal gene silencing (extent and/or duration) is established when 1 or 3 GalNAc moieties are comprised by the conjugates of the invention.

The saponin SO1832 consists of the quillaic acid aglycone core with the carbohydrate substituent Gal-(1→2)-[Xyl-(1→3)]-GlcA- at the C-3beta-OH group and with the carbohydrate substituent Xyl-(1→3)-Xyl-(1→4)-Rha-(1→2)-[Xyl-(1→3)-4-OAc-Qui-(1→4)]-Fuc- at the C-28-OH group (See also Table A1). The chemical formula is $C_{82}H_{128}O_{45}$ and the exact mass is 1832,77 Dalton. The saponin structure of SO1832 is according to molecule (SO1832):

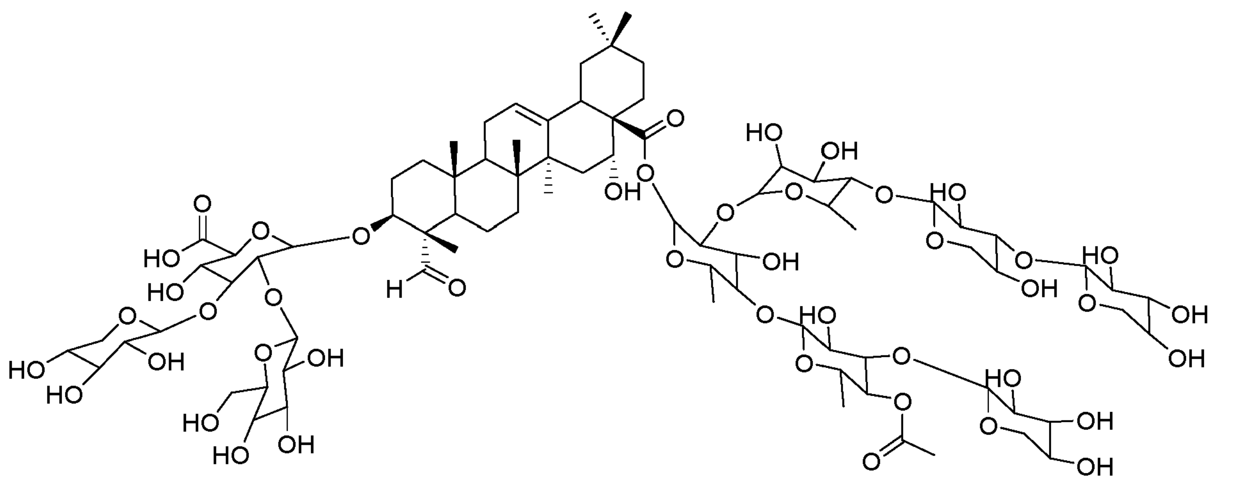

(SO1832).

The skilled person will understand that if the saponin comprised in the saponin conjugate of the present invention is a compound of formula $(II)_S$, $(III)_s$ or $(IV)_S$ as described herein wherein the saponin moiety linker $L_S$ is the result of a coupling reaction between a first precursor $L_{S1}$ covalently bound to GalNAc or the bridging moiety and a second precursor $L_{S2}$ covalently bound to the saponin moiety, the second precursor $L_{S2}$ comprising a hydrazone resulting from a hydrazide/aldehyde coupling to an aldehyde of the saponin moiety or comprising a semicarbazone functional group resulting from a semicarbazide/aldehyde coupling to an aldehyde of the saponin moiety, as described herein, this means that the aldehyde group is already occupied by the linker, such that suitable saponin derivatives are these wherein saponin is derivatised by (ii) a carboxyl group of a saccharide chain as described herein and/or (iii) an acetoxy group of a saccharide chain as described herein.

*Effector molecule conjugate - introduction*

Certain pharmaceutical combinations and certain pharmaceutical compositions of the present invention comprise a second conjugate of an effector molecule and a ligand for asialoglycoprotein receptor (ASGPR), referred to herein as "effector molecule conjugate". When the effector molecule is bound to the remainder of the effector molecule conjugate, it is referred to herein as an "effector moiety". The ASGPR ligand comprises at least one *N*-acetylgalactosamine (GalNAc) moiety and preferably three GalNAc moieties $(GN)_3$. In accordance with preferred embodiments of the present invention, each GalNAc moiety is bound to the remainder of the ASGPR ligand or - in case ASGPR ligand consists of a single GalNAc moiety - to the effector moiety, via a covalent bond to the oxygen on position "1" as indicated in formula (I):

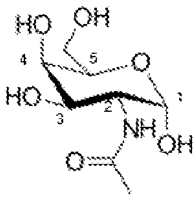

(I).

As shown in formula $(II)_E$, the ASGPR ligand consists of a single GalNAc moiety bound to the effector moiety E, preferably via an effector moiety linker $L_E$.

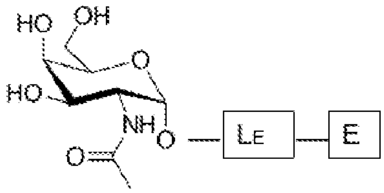

$(II)_E$

The ASGPR ligand may comprise more than one GalNAc moiety, such as 2, 3, 4, 5 or 6 GalNAc moieties, preferably 3 or 4 GalNAc moieties, most preferably three moieties. In such case, it is preferred that the GalNAc moieties are each separately covalently bound via the oxygen on position "1" to a central bridging moiety B, which effectively forms a bridge between the GalNAc moieties and the effector moiety, preferably via an effector moiety linker $L_E$. The GalNAc moieties may be directly bound to the bridging moiety B as shown in formula $(III)_E$:

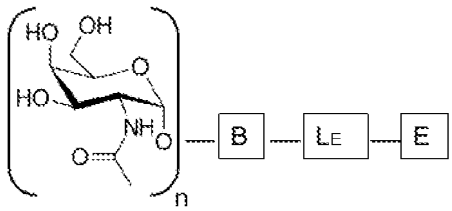

$(III)_E$

wherein n is an integer larger than or equal to 2, $L_E$ is an effector moiety linker and E is the effector moiety. More preferably, the GalNAc moieties are bound to the bridging moiety B via GalNAc linkers $L_{GAL}$ as shown in formula $(IV)_E$:

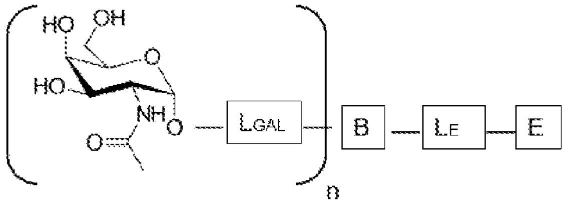

$(IV)_E$

wherein n is an integer larger than or equal to 2, $L_E$ is an effector moiety linker and E is the effector moiety. While each occurrence of $L_{GAL}$ may be independently chosen, the skilled person will appreciate that the synthesis of the conjugate is simplified in case each occurrence of $L_{GAL}$ represents the same moiety.

*Effector molecule conjugate - Linker $L_E$*

The effector moiety linker $L_E$ represents any chemical moiety suitable for covalently binding an effector moiety to GalNAc as in formula $(II)_E$ or to the bridging moiety B as in formula $(III)_E$ and $(IV)_E$. The identity and size of the linker is not particularly limited and covalently binding an effector molecule to GalNAc as in formula $(II)_E$ or to the bridging moiety B as in formula $(III)_E$ and $(IV)_E$ may be effected by means of a 'regular' chemical functional group (*e.g.*, an ether bond) as well as through "click-chemistry" type linkers which typically have a long chain length, resulting in a linker $E_L$ comprising *e.g.* more than 10 or more than 20 carbon atoms. Suitable effector moiety linkers $L_E$ and associated coupling reactions are described in the handbook Hermanson, Greg T. *Bioconjugate techniques.* Academic press, 2013.

As will be understood by the skilled person, the effector moiety linker $L_E$ will typically be the result of a coupling reaction (*e.g.*, "click-chemistry" type) between at least a first precursor $L_{E1}$ covalently bound to GalNAc or the bridging moiety B and a second precursor $L_{E2}$ covalently bound to the effector moiety. This principle is illustrated in the following reaction scheme for the compound of formula (II):

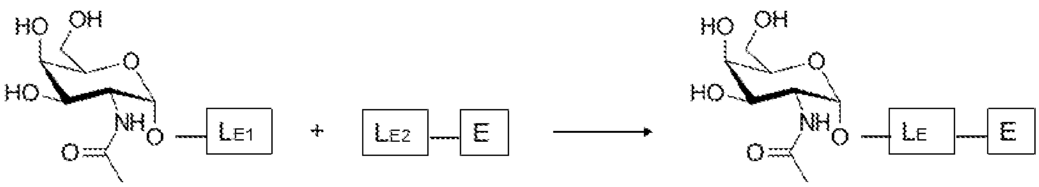

$(V)_E$ $(VI)_E$ $(II)_E$

wherein $L_E$ is an effector moiety linker, $L_{E1}$ is a precursor of the effector moiety linker $L_E$ which is covalently bound to GalNAc and $L_{E2}$ is a precursor of the effector moiety linker $L_E$ which is covalently bound to the effector moiety and E is the effector moiety.

The corresponding reaction scheme for the compound of formula (III) is as follows:

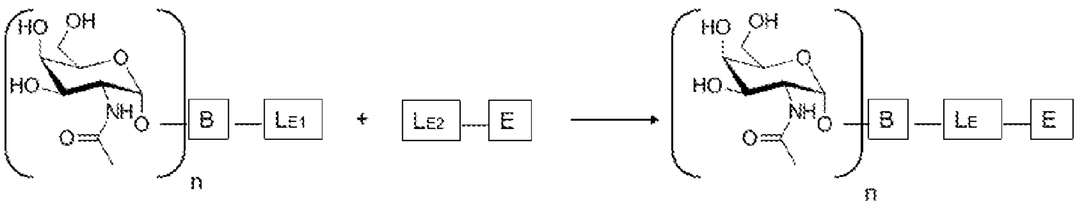

$(VII)_E$ $(VI)_E$ $(III)_E$

wherein $L_E$ is an effector moiety linker, $L_{E1}$ is a precursor of the effector moiety linker $L_E$ which is covalently bound to GalNAc and $L_{E2}$ is a precursor of the effector moiety linker $L_E$ which is covalently bound to the effector moiety, n is an integer larger than or equal to 2 and E is the effector moiety.

The corresponding reaction scheme for the compound of formula $(IV)_E$ is as follows:

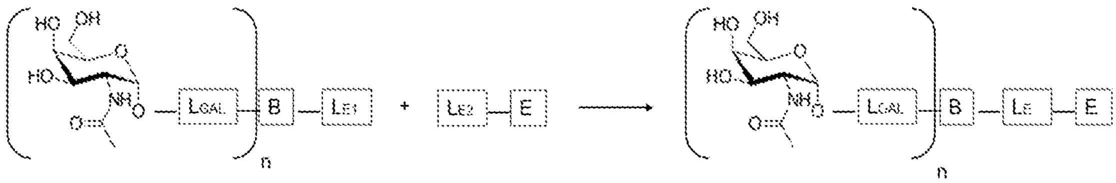

(VIII)E (VI)E (IV)E

wherein $L_E$ is an effector moiety linker, $L_{E1}$ is a precursor of the effector moiety linker $L_E$ which is covalently bound to GalNAc and $L_{E2}$ is a precursor of the effector moiety linker $L_E$ which is covalently bound to the effector moiety, n is an integer larger than or equal to 2, E is the effector moiety, and $L_{GAL}$ is a GalNAc linker and B is a bridging moiety.

The effector moiety linker $L_E$ can be the result of a coupling reaction between at least a first precursor $L_{E1}$ covalently bound to GalNAc or the bridging moiety and a second precursor $L_{E2}$ covalently bound to the effector moiety, wherein the coupling reaction is for example an azide-alkyne cycloaddition, a thiol maleimide coupling, a Staudinger reaction, a nucleophilic ring-opening of strained heterocyclic electrophiles (such as aziridines, epoxides, cyclic sulphates, aziridinium ions, episulfonium ions), a carbonyl reaction of the non-aldol type (such as urea, thiourea, hydrazone, oxime ether, amide or aromatic heterocycle formation) or an addition to a carbon-carbon double bond (such as epoxidation, aziridination, dihydroxylation, sulfentyl halide addition, nitrosyl halide addition or Michael addition), preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling.

In accordance with some embodiments of the invention, the effector moiety linker $L_E$ comprises a succinimide thio-ether moiety. Such a succinimide thio-ether is *e.g.* the result of a thiol maleimide coupling between an N-substituted maleimide and a thiol or sulfhydryl containing compound. This thiol maleimide coupling is a commonly known 'click'-chemistry tool in the field of bio-conjugation and is for example described in Hermanson, Greg T. *Bioconjugate techniques.* Academic press, 2013 page 289. Consequently, it is preferred that the effector moiety linker $L_E$ is the result of a coupling reaction between a first precursor $L_{E1}$ covalently bound to GalNAc or the bridging moiety, the first precursor $L_{E1}$ comprising an N-substituted maleimide; and a second precursor $L_{E2}$ covalently bound to the effector moiety, the second precursor $L_{E2}$ comprising a thiol or a precursor thereof. A suitable thiol precursor is a disulfide, which may be cleaved (*e.g., in-situ*) via reduction to the corresponding thiol.

A preferred structure for the precursor $L_{E1}$ present in the compounds of formula $(V)_E$, $(VII)_E$ or $(VIII)_E$ is the following terminal N-substituted maleimide:

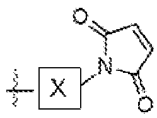

(IX)

wherein X represents any linker suitable for covalently bonding the terminal N-substituted maleimide to GalNAc or the bridging moiety B. As will be understood by the skilled person, X may be the result of a coupling reaction between a first moiety covalently bound to GalNAc or the bridging moiety and a second moiety covalently bound to the maleimide. X can comprise a hydrazone and/or a 1, 2, 3-triazole. Whenever reference is made to a 1, 2, 3-triazole in the context of the linkers of the present application, this preferably means a 1*H*-1, 2, 3-triazole.

Embodiments of the structure for the precursor $L_{E1}$ present in the compounds of formula $(V)_E$, $(VII)_E$ or $(VIII)_E$ are the following terminal N-substituted maleimides:

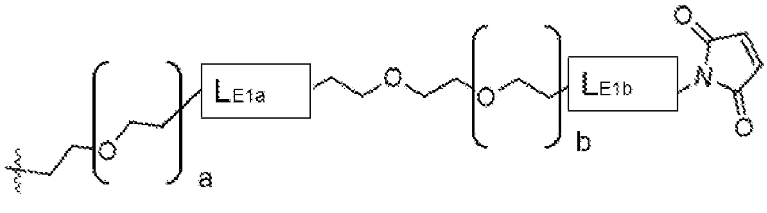

(X)

wherein a and b each independently represent an integer larger than or equal to 0, preferably a and b each independently represent an integer selected from 0, 1, 2, and 3, more preferably a and b represent 2, and wherein $L_{E1a}$ represents a hydrazone and/or a 1, 2, 3-triazole, preferably a 1, 2, 3-triazole and wherein $L_{E1b}$ represents a hydrazone and/or a 1, 2, 3-triazole, preferably a hydrazone;

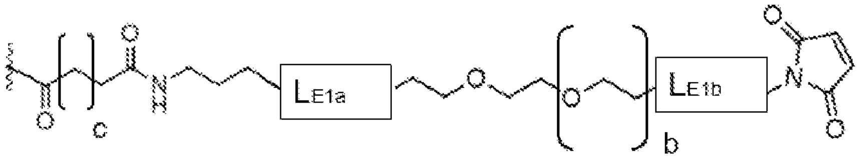

(XI)

wherein b and c each independently represent an integer larger than or equal to 0, preferably b represents an integer selected from 0, 1, 2, and 3 and c represents an integer in the range of 5-15, more preferably b represents 2, and c represents 9, wherein $L_{E1a}$ represents a hydrazone and/or a 1, 2, 3-triazole, preferably a 1, 2, 3-triazole and wherein $L_{E1b}$ represents a hydrazone and/or a 1, 2, 3-triazole, preferably a hydrazone;
and

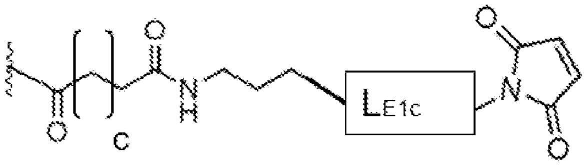

(XII)

wherein c represents an integer larger than or equal to 0, preferably c represents an integer in the range of 5-15, more preferably c represents 9, wherein $L_{E1c}$ represents a hydrazone and/or a 1, 2, 3-triazole, preferably a 1, 2, 3-triazole.

$L_{E1a}$, $L_{E1b}$ and $L_{E1c}$ each preferably comprise less than 20 carbon atoms, more preferably $L_{E1a}$, $L_{E1b}$ and $L_{E1c}$ each independently represent a moiety according to formula (XIII), (XIV) or (XV), preferably $L_{E1a}$ is the 1,2,3-triazole of formula (XIII), $L_{E1b}$ is the hydrazone of formula (XIV) and $L_{E1c}$ is the 1,2,3-triazole of formula (XV):

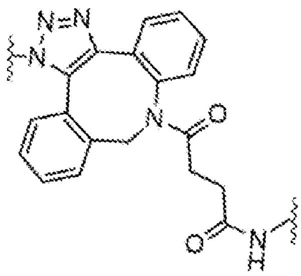

(XIII)

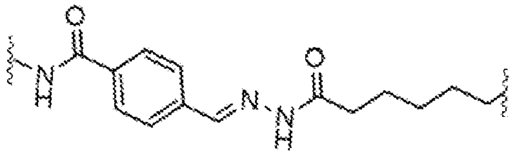

(XIV)

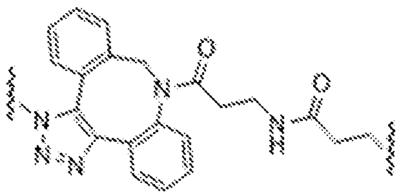

(XV)

Hence, in some embodiments the effector molecule conjugate is a compound of formula $(II)_E$, $(III)_E$ or $(IV)_E$ as described herein, wherein the effector moiety linker $L_E$ is the result of a coupling reaction between a compound of formula $(V)_E$, $(VII)_E$ or $(VIII)_E$ with a compound of formula $(VI)_E$, wherein the first precursor $L_{E1}$ is a terminal N-substituted maleimide of formula (X), (XI) or (XII), wherein $L_{E1a}$ is for example the 1 ,2,3-triazole of formula (XIII), $L_{E1b}$ is for example the hydrazone of formula (XIV) and $L_{E1c}$ is for example the 1,2,3-triazole of formula (XV).

*Effector molecule conjugate - Effector moiety*

An aspect of the invention relates to a pharmaceutical combination comprising:

- a first pharmaceutical composition comprising the saponin conjugate of the invention and optionally comprising a pharmaceutically acceptable excipient and/or a pharmaceutically acceptable diluent; and
- a second pharmaceutical composition comprising a second conjugate of an effector molecule and a ligand for ASGPR, wherein the ligand for ASGPR preferably comprises at least one GalNAc moiety, preferably three or four GalNAc moieties, more preferably the ligand for ASGPR comprises or consists of $(GalNAc)_3$Tris, or a third conjugate of an effector molecule and a binding molecule comprising a binding site for a cell-surface molecule, and optionally comprising a pharmaceutically acceptable excipient and/or pharmaceutically acceptable diluent.

An aspect of the invention relates to a pharmaceutical composition comprising:

- the saponin conjugate of the invention;
- a second conjugate of an effector molecule and a ligand for ASGPR wherein the ligand for ASGPR preferably comprises at least one GalNAc moiety, preferably three or four GalNAc moieties, more preferably the ligand for ASGPR comprises or consists of $(GalNAc)_3$Tris, or a third conjugate of an effector molecule and a binding molecule comprising a binding site for a cell-surface molecule, and optionally comprising a pharmaceutically acceptable excipient and/or pharmaceutically acceptable diluent.

An embodiment is the pharmaceutical combination of the invention comprising the saponin conjugate of the invention or the pharmaceutical composition of the invention comprising the saponin conjugate of the invention, wherein the effector molecule comprises or consists of at least one of a small molecule such as a drug molecule, a toxin such as a protein toxin, an oligonucleotide such as an AON such as a BNA, a xeno nucleic acid or an siRNA, an enzyme, a peptide, a protein, or any combination thereof, preferably, the effector molecule is a toxin, an enzyme or an oligonucleotide.

An embodiment is the pharmaceutical combination of the invention comprising the saponin conjugate of the invention or the pharmaceutical composition of the invention comprising the saponin conjugate of the invention, wherein the ligand for ASGPR comprises or is $(GalNAc)_3$Tris, and/or wherein the ligand for ASGPR and the effector molecule are conjugated via a covalent bond, preferably via at least one linker.

An embodiment is the pharmaceutical combination of the invention comprising the saponin conjugate of the invention or the pharmaceutical composition of the invention comprising the saponin conjugate of the invention, wherein the effector molecule is an oligonucleotide selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic Acid ($BNA^{NC}$), BNA-based siRNA, and BNA-based antisense oligonucleotide (BNA-AON).

An embodiment is the pharmaceutical combination of the invention comprising the saponin conjugate of the invention or the pharmaceutical composition of the invention comprising the saponin conjugate of the invention, wherein the effector molecule is an oligonucleotide selected from any one or more of a(n): anti-miRNA, a BNA-AON or an siRNA, such as BNA-based siRNA, selected from chemically modified siRNA, metabolically stable siRNA and chemically

modified, metabolically stable siRNA.

An embodiment is the pharmaceutical combination of the invention comprising the saponin conjugate of the invention or the pharmaceutical composition of the invention comprising the saponin conjugate of the invention, wherein the effector molecule is an oligonucleotide capable of, for example when present inside a mammalian cell, silencing any one of genes: apolipoprotein B (*apoB*), *HSP27,* transthyretin (*TTR*), proprotein convertase subtilisin/kexin type 9 (*PCSK9*), delta-aminolevulinate synthase 1 (*ALAS1*), anti-thrombin 3 (*AT3*), glycolate oxidase (*GO*), complement component C5 (*CC5*), *X* gene of hepatitis B virus (HBV), S gene of HBV, alpha-1 antitrypsin (*AAT*) and lactate dehydrogenase (*LDH*), and/or is an oligonucleotide capable of, for example when present inside a mammalian cell, targeting an aberrant miRNA. An embodiment is the pharmaceutical combination of the invention comprising the saponin conjugate of the invention or the pharmaceutical composition of the invention comprising the saponin conjugate of the invention, wherein the effector molecule is an oligonucleotide capable of, for example when present inside a mammalian cell, silencing any one of genes: *HSP27, apoB, TTR, PCSK9, TMPRSS6, ALAS1, AT3, GO*, *CC5*, X gene of HBV, S gene of HBV, *AAT,* miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3, GO*, *CC5, X* gene of HBV, S gene of HBV, AAT, miR-122, hepatitis B virus HbsAg, *LDHA, CEBPA* and *LDH,* and/or is an oligonucleotide capable of, for example when present inside a mammalian cell, targeting an aberrant miRNA. Preferred target genes are *HSP27, apoB, TTR, PCSK9, TMPRSS6, ALAS1, AAT,* miR-122, hepatitis B virus HbsAg, *LDHA* and *CEBPA,* more preferred are genes *HPS27* and *apoB.*

An embodiment is the pharmaceutical combination of the invention comprising the saponin conjugate of the invention or the pharmaceutical composition of the invention comprising the saponin conjugate of the invention, wherein the effector molecule is an oligonucleotide capable of, for example when present inside a mammalian cell, targeting an mRNA involved in expression of any one of proteins: apoB, HSP27, TTR, PCSK9, ALAS1, AT3, GO, CC5, expression product of X gene of HBV, expression product of S gene of HBV, AAT and LDH, or is capable of, for example when present inside a mammalian cell, antagonizing or restoring an miRNA function such as inhibiting an oncogenic miRNA (onco-miR) or suppression of expression of an onco-miR. An embodiment is the pharmaceutical combination of the invention comprising the saponin conjugate of the invention or the pharmaceutical composition of the invention comprising the saponin conjugate of the invention, wherein the effector molecule is an oligonucleotide capable of, for example when present inside a mammalian cell, targeting an mRNA involved in expression of any one of proteins: HSP27, apoB, TTR, PCSK9, TMPRSS6, ALAS1, AT3, GO, CC5, X gene of HBV, S gene of HBV, AAT, miR-122, hepatitis B virus HbsAg, LDHA, CEBPA and LDH, preferably HSP27, apoB, TTR, PCSK9, TMPRSS6, ALAS1, AAT, miR-122, hepatitis B virus HbsAg, LDHA and CEBPA, or is capable of, for example when present inside a mammalian cell, antagonizing or restoring an miRNA function such as inhibiting an oncogenic miRNA (onco-miR) or suppression of expression of an onco-miR.

An embodiment is the pharmaceutical combination of the invention comprising the saponin conjugate of the invention or the pharmaceutical composition of the invention comprising the saponin conjugate of the invention, wherein the effector molecule is or comprises a toxin which toxin comprises or consists of at least one molecule selected from any one or more of a peptide, a protein, an enzyme such as urease and Cre-recombinase, a proteinaceous toxin, a ribosome-inactivating protein, and/or a bacterial toxin, a plant toxin, more preferably selected from any one or more of a viral toxin such as apoptin; a bacterial toxin such as Shiga toxin, Shiga-like toxin, *Pseudomonas aeruginosa* exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin, and/or comprises or consists of at least one of a toxin targeting ribosome, a toxin targeting elongation factor, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-$\gamma$-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, $\alpha$-amanitin, a spliceostatin, a thailanstatin, ozogamicin, tesirine, Amberstatin269 and soravtansine, or a derivative thereof.

*Conjugates - Bridging moiety B*

The bridging moiety B present in the saponin conjugates of formula $(III)_S$ or $(IV)_S$ and in the effector molecule conjugates of formula as $(III)_E$ or $(IV)_E$ described herein represents any moiety suitable for covalently binding 2 or more,

preferably 3 or 4, more preferably 3 GalNAc moieties and the saponin moiety linker $L_S$ or the effector moiety linker $L_E$.

According to preferred embodiments, the bridging moiety B is a compound of formula (XV):

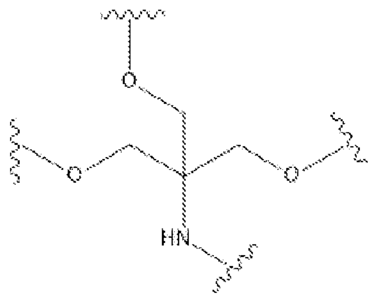

(XV)

wherein the oxygen atoms of the compound of formula (XV) are bound to GalNAc (corresponding to compounds of formula $(III)_S$ or $(III)_E$) or to the GalNAc linkers $L_{GAL}$ (corresponding to compounds of formula $(IV)_S$ or $(IV)_E$), and the nitrogen atom of the compound of formula (XV) is bound to the saponin moiety linker $L_S$ or the effector moiety linker $L_E$.

The skilled person will understand that these compounds of formula $(III)_S$, $(III)_E$, $(IV)_S$ or $(IV)_E$ wherein the bridging moiety B is a compound of formula (XV) correspond to effector molecule conjugates wherein the ASGPR ligand consists of $(GalNAc)_3$Tris.

*Conjugates - Linker $L_{GAL}$*

The GalNAc linkers $L_{GAL}$ represent any chemical moiety suitable for covalently binding GalNAc to the bridging moiety as in formula $(IV)_S$ or $(IV)_E$. The identity and size of the linker is not particularly limited.

According to embodiments, the GalNAc linkers $L_{GAL}$ each comprise 2-25 carbon atoms, preferably 7-15 carbon atoms, more preferably 11 carbon atoms. Preferably the GalNAc linkers $L_{GAL}$ comprise at least one, preferably two amide moieties. A particularly preferred GalNAc linker $L_{GAL}$ is a compound according to formula (XVI):

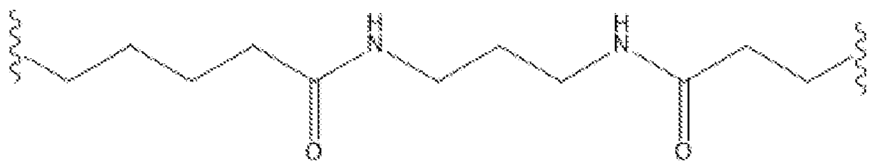

(XVI)

An embodiment is the pharmaceutical combination of the invention comprising the saponin conjugate of the invention or the pharmaceutical composition of the invention comprising the saponin conjugate of the invention, wherein the binding molecule comprising a binding site for a cell-surface molecule, comprised by the third conjugate, is a ligand for a cell-surface molecule or an antibody comprising a binding site for a cell-surface molecule or at least one domain or fragment thereof comprising the binding site.

An embodiment is the pharmaceutical combination of the invention comprising the saponin conjugate of the invention or the pharmaceutical composition of the invention comprising the saponin conjugate of the invention, wherein the third conjugate is any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate.

An embodiment is the pharmaceutical combination of the invention comprising the saponin conjugate of the invention or the pharmaceutical composition of the invention comprising the saponin conjugate of the invention, wherein the binding molecule comprising a binding site for a cell-surface molecule, comprised by the third conjugate, is capable of binding to any one of cell-surface molecules: CD71, CD63, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, asialoglycoprotein

receptor (ASGPR), preferably any one of: HER2, CD71, ASGPR and EGFR, more preferably CD71.

An embodiment is the pharmaceutical combination of the invention comprising the saponin conjugate of the invention or the pharmaceutical composition of the invention comprising the saponin conjugate of the invention, wherein the binding molecule comprising a binding site for a cell-surface molecule, comprised by the third conjugate, is or comprises any one of: an antibody, preferably a monoclonal antibody such as a human monoclonal antibody, an IgG, a molecule comprising or consisting of a single-domain antibody, at least one $V_{HH}$ domain, preferable a camelid $V_H$, a variable heavy chain new antigen receptor ($V_{NAR}$) domain, a Fab, an scFv, an Fv, a dAb, an F(ab)2 and a Fcab fragment.

An aspect of the invention relates to the pharmaceutical combination of the invention comprising the saponin conjugate of the invention or to the pharmaceutical composition of the invention comprising the saponin conjugate of the invention, for use as a medicament. An aspect of the invention relates to the oligonucleotide conjugate of the invention, for use as a medicament.

An aspect of the invention relates to the pharmaceutical combination of the invention comprising the saponin conjugate of the invention or to the pharmaceutical composition of the invention comprising the saponin conjugate of the invention, for use in the treatment or prophylaxis of a disease or health problem in which an expression product is involved of any one or more of genes: *apoB, TTR, PCSK9, ALAS1, AT3, GO*, *CC5, X* gene of HBV, *S* gene of HBV, *AAT* and *LDH.* An aspect of the invention relates to the pharmaceutical combination of the invention comprising the saponin conjugate of the invention or to the pharmaceutical composition of the invention comprising the saponin conjugate of the invention, for use in the treatment or prophylaxis of a disease or health problem in which an expression product is involved of any one or more of genes: *HSP27, apoB, TTR, PCSK9, TMPRSS6, ALAS1, AT3,* GO, *CC5, X* gene of HBV, *S* gene of HBV, *AAT,* miR-122, hepatitis B virus HbsAg, *LDHA, CEBPA* and *LDH,* preferably *HSP27, apoB, TTR, PCSK9, TMPRSS6, ALAS1, AAT,* miR-122, hepatitis B virus HbsAg, *LDHA* and *CEBPA,* more preferably *HSP27, apoB.*

An embodiment is the pharmaceutical combination of the invention comprising the saponin conjugate of the invention or the pharmaceutical composition of the invention comprising the saponin conjugate of the invention, or the pharmaceutical combination comprising the saponin conjugate of the invention or pharmaceutical composition comprising the saponin conjugate of the invention for use of the invention, for use in the treatment or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, $\alpha$1-antitrypsin deficiency, $\beta$-thalassaemia, or an auto-immune disease.

An embodiment is the pharmaceutical combination comprising the saponin conjugate of the invention for use of the invention or the pharmaceutical composition comprising the saponin conjugate of the invention for use of the invention or the pharmaceutical composition comprising the oligonucleotide conjugate of the invention, wherein the saponin is a saponin derivative, preferably a QS-21 derivative or an SO1861 derivative or an SO1832 derivative according to the invention.

An aspect of the invention relates to an *in vitro* or ex *vivo* method for transferring the second conjugate or the third conjugate of the invention from outside a cell to inside said cell, preferably subsequently transferring the effector molecule comprised by the second conjugate or the third conjugate according to the invention into the cytosol of said cell, comprising the steps of:

a) providing a cell which expresses ASGPR on its surface, and, when the third conjugate is to be transferred into the cell, which expresses the cell-surface molecule for which the third conjugate comprises a binding molecule for binding to said cell-surface molecule, the cell preferably selected from a liver cell, a virally infected cell and a tumor cell;
b) providing the second conjugate or the third conjugate of the invention, for transferring into the cell provided in step a);
c) providing the saponin conjugate of the invention;
d) contacting the cell of step a) *in vitro* or ex *vivo* with the second conjugate or the third conjugate of step b) and the saponin conjugate of step c), therewith effecting the transfer of the second conjugate or the third conjugate from outside the cell into said cell, and preferably therewith subsequently effecting the transfer of the second conjugate or the third conjugate into the cytosol of said cell, or preferably therewith subsequently effecting the transfer of at least the effector molecule comprised by the second conjugate or the third conjugate into the cytosol of said cell.

An embodiment is the oligonucleotide conjugate of the invention, wherein the oligonucleotide is any one of a BNA, a xeno nucleic acid, an siRNA, an antisense oligonucleotide.

An embodiment is the oligonucleotide conjugate of the invention, wherein the oligonucleotide is selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic acid ($BNA^{NC}$), BNA-based siRNA, and BNA-based antisense oligonucleotide (BNA-AON).

An embodiment is the oligonucleotide conjugate of the invention, wherein the oligonucleotide is selected from any one or more of a(n): anti-miRNA, a BNA-AON or an siRNA, such as BNA-based siRNA, selected from chemically modified siRNA, metabolically stable siRNA and chemically modified, metabolically stable siRNA.

An embodiment is the oligonucleotide conjugate of the invention, wherein the oligonucleotide is an oligonucleotide capable of, for example when present inside a mammalian cell and preferably when present inside a human cell, silencing any one of genes: apolipoprotein B (*apoB*), *HSP27,* transthyretin (*TTR*), proprotein convertase subtilisin/kexin type 9 (*PCSK9*), *TMPRSS6,* delta-aminolevulinate synthase 1 (*ALAS1*), antithrombin 3 (*AT3*), glycolate oxidase (*GO*), complement component C5 (*CC5*), *X* gene of hepatitis B virus (*HBV*), S gene of HBV, alpha-1 antitrypsin (*AAT*), miR-122, hepatitis B virus HbsAg, *LDHA, CEBPA* and lactate dehydrogenase (*LDH*), and/or is an oligonucleotide capable of, for example when present inside a mammalian cell, targeting an aberrant miRNA. Preferably, the gene is any one of the genes *HSP27, apoB, TTR, PCSK9, TMPRSS6, ALAS1, AAT,* miR-122, hepatitis B virus HbsAg, *LDHA* and *CEBPA* for expressing proteins HSP27, apoB, TTR, PCSK9, TMPRSS6, ALAS1, AAT, miR-122, hepatitis B virus HbsAg, LDHA and CEBPA.

An embodiment is the oligonucleotide conjugate of the invention, wherein the oligonucleotide is an oligonucleotide capable of, for example when present inside a mammalian cell and preferably when present inside a human cell, silencing any one of genes: apolipoprotein B (apoB) and HSP27.

An embodiment is the oligonucleotide conjugate of the invention, wherein the oligonucleotide is an oligonucleotide capable of, for example when present inside a mammalian cell and preferably when present inside a human cell, targeting an mRNA involved in expression of any one of proteins: apoB, HSP27, TTR, PCSK9, TMPRSS6, ALAS1, AT3, GO, CC5, expression product of X gene of HBV, expression product of S gene of HBV, AAT, miR-122, hepatitis B virus HbsAg, LDHA, CEBPA and LDH, or is capable of, for example when present inside a mammalian cell and preferably when present inside a human cell, antagonizing or restoring an miRNA function such as inhibiting an oncogenic miRNA (onco-miR) or suppression of expression of an onco-miR. Preferably, the protein is a liver protein selected from: HSP27, apoB, TTR, PCSK9, TMPRSS6, ALAS1, AAT, miR-122, hepatitis B virus HbsAg, LDHA and CEBPA.

An embodiment is the oligonucleotide conjugate of the invention, wherein the oligonucleotide is an oligonucleotide capable of, for example when present inside a mammalian cell and preferably when present inside a human cell, targeting an mRNA involved in expression of any one of proteins: apoB and HSP27.

An aspect of the invention relates to a method for providing the oligonucleotide conjugate of the invention, comprising the steps of:

(a) providing at least one saponin moiety comprising a covalently bound first linker, wherein the first linker comprises at least one first reactive group for covalent binding to a second reactive group on a second linker or to a seventh reactive group on a seventh linker;

(b) providing an oligonucleotide comprising a covalently bound third linker, wherein the third linker comprises a third reactive group for covalent binding to a fourth reactive group on a fourth linker or to an eighth reactive group on the seventh linker;

(c) providing at least one GalNAc moiety comprising a covalently bound fifth linker, wherein the fifth linker comprises a fifth reactive group for covalent binding to a sixth reactive group on a sixth linker or to a ninth reactive group on the seventh linker; and

either

(d1) linking the first linker to the second linker through formation of a covalent bond between the first reactive group and the second reactive group, linking the third linker to the fourth linker through formation of a covalent bond between the third reactive group and the fourth reactive group, linking the fifth linker to the sixth linker through formation of a covalent bond between the fifth reactive group and the sixth reactive group, and covalently linking the second linker, fourth linker and sixth linker together, therewith providing the oligonucleotide,

or

(d2) linking the first linker to the seventh linker through formation of a covalent bond between the first reactive group and the seventh reactive group, linking the third linker to the seventh linker through formation of a covalent bond between the third reactive group and the eighth reactive group, linking the fifth linker to the seventh linker through formation of a covalent bond between the fifth reactive group and the ninth reactive group, therewith providing the oligonucleotide conjugate.

An embodiment is the method for providing the oligonucleotide conjugate of the invention, wherein the seventh linker is a tri-functional linker, such as the tri-functional linker represented by formula (XXI):

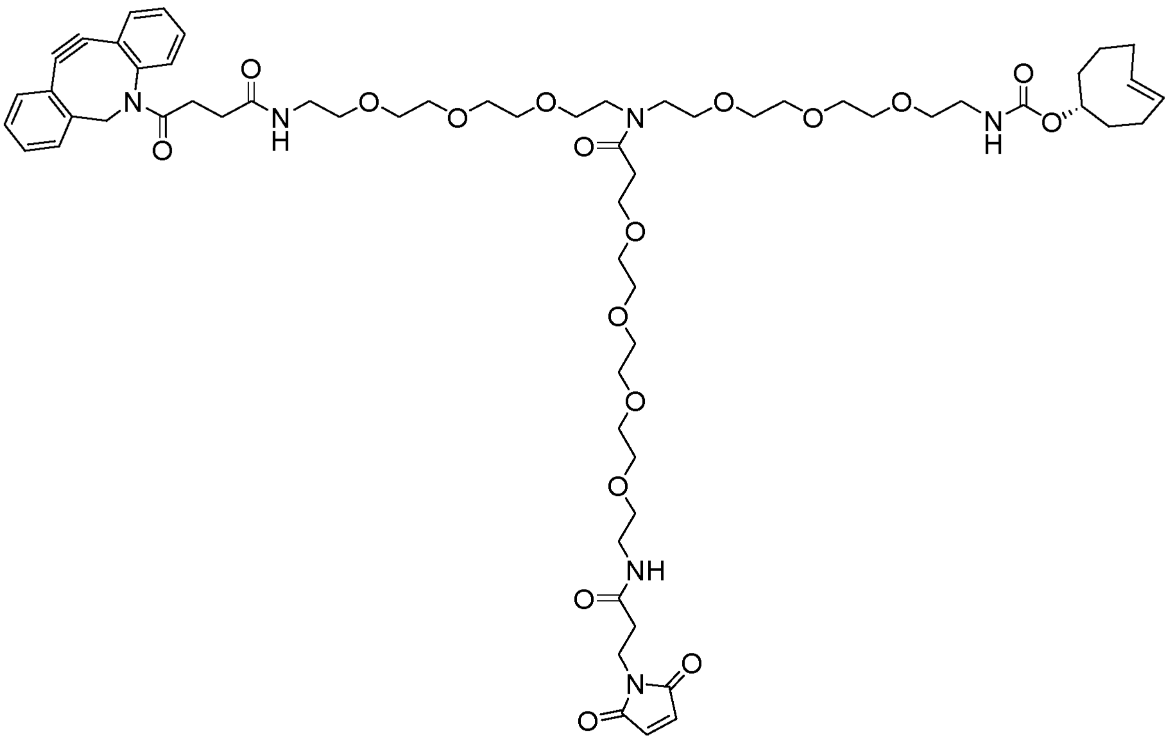

(XXI).

Preferred is the method for providing the oligonucleotide conjugate of the invention, wherein the at least one saponin moiety are 1-16 saponin moieties, preferably 1-8 saponin moieties, such as 1, 4 or 8 saponin moieties.

Preferred is the method for providing the oligonucleotide conjugate of the invention, wherein the saponin is SO1861, SO1832, QS-21, or any functional derivative thereof, preferably SO1861 or SO1832.

Preferred is the method for providing the oligonucleotide conjugate of the invention, wherein the saponin moiety or the saponin moieties is/are covalently linked via a hydrazone bond or a semicarbazone bond.

Preferred is the method for providing the oligonucleotide conjugate of the invention, wherein the at least one GalNAc moiety are 1-4 GalNAc moieties, preferably 1 or 3 GalNAc moieties.

An aspect of the invention relates to a pharmaceutical composition comprising the oligonucleotide conjugate of the invention, and optionally a pharmaceutically acceptable excipient and/or optionally a pharmaceutically acceptable diluent.

An aspect of the invention relates to a pharmaceutical composition comprising the oligonucleotide conjugate of the invention, and optionally a pharmaceutically acceptable excipient and/or optionally a pharmaceutically acceptable diluent, for use as a medicament. An aspect of the invention relates to the oligonucleotide conjugate of the invention, for use as a medicament.

An aspect of the invention relates to a pharmaceutical composition comprising the oligonucleotide conjugate of the invention, and optionally a pharmaceutically acceptable excipient and/or optionally a pharmaceutically acceptable diluent or to the oligonucleotide conjugate of the invention, for use in the treatment or prophylaxis of a disease or health problem in which an expression product is involved of any one or more of genes: *HSP27, apoB, TTR, PCSK9, TMPRSS6, ALAS1, AT3, GO*, *CC5*, *X* gene of HBV, *S* gene of HBV, *AAT,* miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3, GO*, *CC5*, *X* gene of HBV, *S* gene of HBV, *AAT,* miR-122, hepatitis B virus HbsAg, *LDHA, CEBPA* and *LDH.* For example, the disease is a cancer associated with (over-)expression of HSP27 in the tumor cell. Administering the oligonucleotide conjugate of the invention comprising an oligonucleotide for silencing *HSP27* gene to a patient suffering from a tumor for which tumor growth is associated with (over-)expression of HSP27, results in silencing of the *HSP27* gene and therewith with halting tumor growth. For example, a health problem associated with *apoB* expression is a health problem related to elavated levels of LDL-cholesterol in the blood of a human subject, *e.g.* a patient at risk for atherosclerosis and/or hypercholesterolemia, and/or a patient suffering from said atherosclerosis and/or hypercholesterolemia. Administering to said human subject or patient an oligonucleotide of the invention comprising an oligonucleotide for silencing the *apoB* gene results in inhibiting or lowering apoB expression and therewith as a consequence, lowering of LDL (-cholesterol), since apoB is the protein component of the LDL particle. A lowered level

of circulating LDL is associated with less circulating LDL-cholesterol and therewith a diminished risk for LDL-cholesterol associated risk for health and disease symptoms as apparent in a patient suffering from atherosclerosis and/or hypercholesterolemia or in a subject at risk for developing atherosclerosis and/or hypercholesterolemia under influence of above normal blood level of LDL-cholesterol.

An aspect of the invention relates to a pharmaceutical composition comprising the oligonucleotide conjugate of the invention or to the oligonucleotide conjugate of the invention, for use in the treatment or prophylaxis of a disease or health problem in which an expression product is involved of any one or more of genes: *HSP27, apoB, TTR, PCSK9, TMPRSS6, ALAS1, AAT,* miR-122, hepatitis B virus HbsAg, *LDHA* and *CEBPA,* and/or for use in the treatment or prophylaxis of a disease or health problem which involves any one or more of genes: *HSP27, apoB, TTR, PCSK9, TMPRSS6, ALAS1, AAT,* miR-122, hepatitis B virus HbsAg, *LDHA* and *CEBPA.*

An embodiment is the pharmaceutical composition, for use according to the invention, comprising the oligonucleotide conjugate of the invention, or is the oligonucleotide conjugate of the invention, for use according to the invention, wherein said use is in the treatment or in the prophylaxis of a disease or health problem in which an expression product is involved of any one or more of genes: *HSP27* and *apoB,* preferably *apoB,* and/or for use in the treatment or prophylaxis of a disease or health problem which involves any one or more of genes: *HSP27* and *apoB,* preferably *apoB.*

An embodiment is the pharmaceutical composition for use according to the invention, comprising the oligonucleotide conjugate of the invention, or is the oligonucleotide conjugate of the invention, for use according to the invention, wherein said use is in the treatment or in the prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, or an auto-immune disease.

An embodiment is the pharmaceutical composition for use according to the invention, comprising the oligonucleotide conjugate of the invention, or is the oligonucleotide conjugate of the invention, for use according to the invention, wherein said use is in the treatment or in the prophylaxis of a cancer such as endometrial carcinoma, breast cancer, lung cancer and hypercholesterolemia, preferably hypercholesterolemia.

An aspect of the invention relates to a pharmaceutical composition comprising the oligonucleotide conjugate of the invention, or to the oligonucleotide conjugate of the invention, for use in the treatment or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, $\alpha$1-antitrypsin deficiency, $\beta$-thalassaemia, or an auto-immune disease.

An aspect of the invention relates to a pharmaceutical composition comprising the oligonucleotide conjugate of the invention, or to the oligonucleotide conjugate of the invention, for use in the treatment or prophylaxis of a cancer such as endometrial carcinoma, breast cancer, lung cancer or hepatocellular carcinoma, and/or a cardiovascular disease such as atherosclerosis and/or hypercholesterolemia, preferably atherosclerosis and/or hypercholesterolemia.

For example, the disease is a cancer associated with (over-)expression of HSP27 in the tumor cell. Administering the oligonucleotide conjugate of the invention comprising an oligonucleotide for silencing *HSP27* gene to a patient suffering from a tumor for which tumor growth is associated with (over-)expression of *HSP27,* results in silencing of the *HSP27* gene and therewith with halting tumor growth. For example, a health problem associated with apoB expression is a health problem related to elevated levels of LDL-cholesterol in the blood of a human subject, *e.g.* a patient at risk for atherosclerosis and/or hypercholesterolemia, and/or a patient suffering from said atherosclerosis and/or hypercholesterolemia. Administering to said human subject or patient an oligonucleotide conjugate of the invention comprising an oligonucleotide for silencing the *apoB* gene results in inhibiting or lowering apoB expression and therewith as a consequence, lowering of LDL(-cholesterol), since apoB is the protein component of the LDL particle. A lowered level of circulating LDL is associated with less circulating LDL-cholesterol and therewith a diminished risk for LDL-cholesterol associated risk for health and disease symptoms as apparent in a patient suffering from atherosclerosis and/or hypercholesterolemia or in a subject at risk for developing atherosclerosis and/or hypercholesterolemia under influence of above normal blood level of LDL-cholesterol.

An aspect of the invention relates to a pharmaceutical composition comprising the oligonucleotide conjugate of the invention, or to the oligonucleotide conjugate of the invention, for use in the lowering of LDL-cholesterol in a subject. An aspect of the invention relates to a pharmaceutical composition comprising the oligonucleotide conjugate of the invention, or to the oligonucleotide conjugate of the invention, for use in a method for lowering of LDL-cholesterol concentration in blood of a human subject. An aspect of the invention relates to a pharmaceutical composition comprising the oligonucleotide conjugate of the invention, or to the oligonucleotide conjugate of the invention, for use in a method for the treatment or prophylaxis of a cardiovascular disease. Typically, the treatment or prophylaxis is in a human patient suffering from a cardiovascular disease or in a human subject at risk for developing a cardiovascular disease. Typically, the cardiovascular disease is associated with an elevated blood level of LDL-cholesterol compared to the upper limit of the range of normal LDL-cholesterol levels.

An aspect of the invention relates to an *in vitro* or ex *vivo* method for transferring the oligonucleotide conjugate of the invention from outside a cell to inside said cell, preferably for subsequently transferring the oligonucleotide comprised by the oligonucleotide conjugate of the invention into the cytosol and/or into the nucleus of said cell, comprising the steps of:

a) providing a cell which expresses ASGPR, preferably ASGPR1, on its surface, the cell preferably selected from a liver cell, a virally infected mammalian cell and a mammalian tumor cell, wherein preferably said cell is a human cell;
b) providing the oligonucleotide conjugate of the invention for transferring into the cell provided in step a);
c) contacting the cell of step a) *in vitro* or ex *vivo* with the oligonucleotide conjugate of step b), preferably in a liquid medium, therewith effecting the transfer of the oligonucleotide conjugate from outside the cell into said cell, and optionally and preferably therewith subsequently effecting the transfer of the oligonucleotide comprised by the oligonucleotide conjugate into the cytosol and/or nucleus of said cell.

Surprisingly, the inventors have found that a saponin derivative based on a saponin comprising a triterpene aglycone core structure and at least one of a first saccharide chain '$R^1$' and a second saccharide chain '$R^2$', as herein defined, linked to the aglycone core structure, preferably quillaic acid, wherein the saponin derivative comprises an aglycone core structure comprising an aldehyde group, wherein the aldehyde group is transformed into a semicarbazone functional group according to formula (I1):

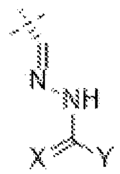

(I1)

wherein $R^1$ and $R^2$ are independently selected from hydrogen, a monosaccharide, a linear oligosaccharide and a branched oligosaccharide,
X = O, P or S, and
Y = $NR^3R^4$, wherein $R^3$ and $R^4$ independently represent H, an unsubstituted C1 - C10 straight chain, branched or cyclic alkyl, an unsubstituted C2 - C10 straight chain, branched or cyclic alkenyl or an unsubstituted C2 - C10 straight chain or branched alkynyl, or a covalently bound linker, preferably one of $R^3$ and $R^4$ is H; or

Y = 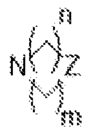,

wherein n and m each are an integer independently selected from 1, 2, or 3,
Z = $CH_2$, O, S, P or $NR^5$, and
wherein $R^5$ represent H, an unsubstituted C1 - C10 straight chain, branched or cyclic alkyl, an unsubstituted C2 - C10 straight chain, branched or cyclic alkenyl, an unsubstituted C2 - C10 straight chain or branched alkynyl, or a covalently bound linker, or a maleimide moiety according to formula (II1)a:

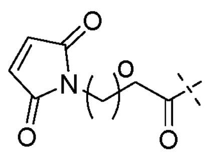

(II1)a

or an azide moiety according to formula (II1)b

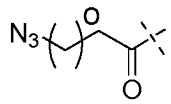

(II1)b

wherein o is an integer selected from 0-10, preferably 2-7, more preferably 4-6,

has a reduced toxicity when cell viability is considered of cells contacted with the saponin derivatives; has activity when potentiation of e.g. toxin cytotoxicity or BNA-mediated gene silencing is considered (without wishing to be bound by any theory: relating to similar or improved endosomal escape enhancing activity of the modified saponin (i.e. the saponin derivative)), if the aldehyde functional group is transformed to a semicarbazone functional group according to formula (I1); and/or has reduced hemolytic activity, when compared with the toxicity, activity and haemolytic activity of unmodified saponin. That is to say, the saponin derivative has at least one, preferably to, more preferably all three of:

(i) a reduced toxicity when cell viability is considered of cells contacted with the saponin derivatives;
(ii) enhanced activity when potentiation of e.g. BNA-mediated gene silencing is considered (without wishing to be bound by any theory: relating to similar or improved endosomal escape enhancing activity of the modified saponin), if the aldehyde functional group is transformed to a semicarbazone functional group according to formula (I1); and/or
(iii) reduced hemolytic activity,

when compared with the toxicity, activity and haemolytic activity of unmodified saponin on which the saponin derivative is based. Therewith, the inventors provide saponin derivatives with an improved therapeutic window, since for the saponin derivatives the cytotoxicity is lower than cytotoxicity determined for their naturally occurring counterparts, the haemolytic activity is lower than haemolytic activity determined for the naturally occurring counterparts of the saponin derivatives, the ratio between IC50 values for cell toxicity and *e.g.* IC50 values for toxin potentiation or IC50 values for gene silencing is similar or increased, and/or since the ratio between IC50 values for saponin haemolytic activity and *e.g.* IC50 values for toxin potentiation or IC50 values for gene silencing is similar or increased. The term "endosomal escape enhancing activity" can be abbreviated as 'activity'.

In addition, the inventors surprisingly established (tumor) cell killing by contacting such cells with a saponin conjugate based on a saponin derivative comprising the semicarbazone functional group, together with an ADC such as an antibody - protein toxin conjugate, despite the medium to low expression of the cell-surface receptor targeted by the cell-surface molecule binding-molecule (*e.g.* antibody) comprised by the saponin conjugate and/or despite the medium to low expression of the cell-surface receptor targeted by the ADC. The saponin derivative and the saponin conjugate comprise the semicarbazone functional group. For example, in a comparative example, such cell killing could not be established or only to a lower extent, when a saponin conjugate comprising the same cell-surface molecule binding-molecule (*e.g.*, antibody) but comprising a hydrazone functional group (=N-N(H)-C(O)-) instead of the semicarbazone functional group, was contacted with the cells. Therewith, the inventors provided for a more potent saponin derivative and saponin conjugate, when the activation or potentiating of an effector moiety such as an effector moiety comprised by an ADC or AOC, is considered.

Furthermore, the inventors have found that the saponin derivatives comprising the semicarbazone functional group hydrolyses more rapidly and in an higher amount towards the corresponding native saponin comprising a "free" aldehyde functional group, as compared to saponin derivatives comprising a hydrazone functional group (=N-N(H)-C(O)-) known in the art, under acidic conditions which are the conditions present in endosomes and/or lysosomes of mammalian cells. This has the benefit that a lower amount of the saponin derivatives according to the invention should be administered to a patient in need of potentiation of *e.g.* an ADC or an AOC or a gene-silencing oligonucleotide such as a BNA, *e.g.* coupled to one or more GalNAc moieties, to obtain the same amount of native saponin comprising the "free" aldehyde to act as endosomal escape enhancers for targeted toxins or targeted oligonucleotides, compared to the required amount of saponin derivative comprising *e.g.* the hydrazone functional group (=N-N(H)-C(O)-). Without wishing to be bound by any theory, faster and more efficacious release of the saponin comprising the aldehyde functional group from the saponin derivative that comprises the semicarbazone functional group, when compared to release of the saponin comprising the aldehyde functional group from the saponin derivative that comprises *e.g.* the hydrazone functional group, is at the basis for the improved activity of the saponin conjugate comprising a saponin derivative comprising a semicarbazone functional group as established by the inventors, such as a conjugate comprising the saponin, an oligonucleotide and a cell-surface receptor ligand such as one to three GalNAc moieties (for targeting (liver) cells expressing ASGPR), wherein the activity is

the potentiation of the effector moiety activity such as a gene-silencing oligonucleotide, inside the cytosol or nucleus of the targeted cell by endosomal escape enhancement.

Surprisingly, transformation (derivatisation) of the aldehyde group at C-23 of the aglycone of the saponin into a semicarbazone functional group according to formula (I1), results in a decrease in cytotoxicity when such saponin derivatives are contacted with cells, *i.e*. various types of cells. It is thus beneficial that these series of saponin derivatives with decreased cytotoxicity are provided, wherein the decrease in cytotoxicity is relative to the cytotoxicity as determined for the unmodified naturally occurring saponin counterparts. The saponin derivatives can be formed from such naturally occurring saponins, such as SO1861, equally active SO1832 and QS-21 (isoforms), preferably from SO1861 or SO1832. When the decrease in cytotoxicity is considered, saponin derivatives comprising the semicarbazone functional group, are equally suitable, when saponins with decreased cytotoxicity are to be provided.

The inventors thus have provided saponin derivatives with an improved therapeutic window when cytotoxicity is considered and/or when haemolytic activity is considered, and when the potentiation of *e.g*. (gene-silencing) oligonucleotides is considered compared to the corresponding underivatised saponin. Such saponin derivatives comprising the semicarbazone functional group are for example in particular suitable for application in a therapeutic regimen involving a GalNAc-oligonucleotide conjugate which is combined with the saponin derivative or which conjugate is the oligonucleotide conjugate of the invention comprising the covalently linked saponin together with the GalNAc moiety/moieties and the oligonucleotide, for the prophylaxis or treatment of *e.g*. a cancer, hypercholesterolemia, cardiovascular disease or enhanced level of LDL-cholesterol above normal levels for healthy subjects in a (human) subject in need thereof. The safety of such saponin derivatives is improved when cytotoxicity and/or haemolytic activity is considered, especially when such saponin derivatives are administered to a patient in need of e.g. treatment with an ADC or with and AOC or with a conjugate comprising at least one GalNAc moiety and comprising an oligonucleotide, *e.g*. a BNA for silencing a gene such as *HSP27* and *apoB,* or when the saponin derivative comprising the semicarbazone functional group is part of the oligonucleotide conjugate of the invention.

An embodiment is the oligonucleotide conjugate of the invention comprising one saponin moiety. An embodiment is the oligonucleotide conjugate of the invention, wherein the at least one GalNAc moiety, the at least one saponin and the oligonucleotide are covalently bound via a tri-functional linker, preferably with each of the GalNAc moiety, the saponin and the oligonucleotide covalently bound to a separate arm of the tri-functional linker. A preferred oligonucleotide conjugate of the invention is an oligonucleotide conjugate, wherein the at least one GalNAc moiety, preferably three GalNAc moieties, at least one saponin, preferably 1-16 saponin moieties, more preferably 1-8 saponin moieties such as 1, 4 or 8 saponin moieties, and the oligonucleotide are covalently bound via a trifunctional linker, preferably with each of the GalNAc moiety/moieties, the saponin/saponin moieties and the oligonucleotide covalently bound to a separate arm of the trifunctional linker. An example of a trifunctional linker that is suitable for incorporation into an oligonucleotide conjugate of the invention is the trifunctional linker represented by formula (XXI):

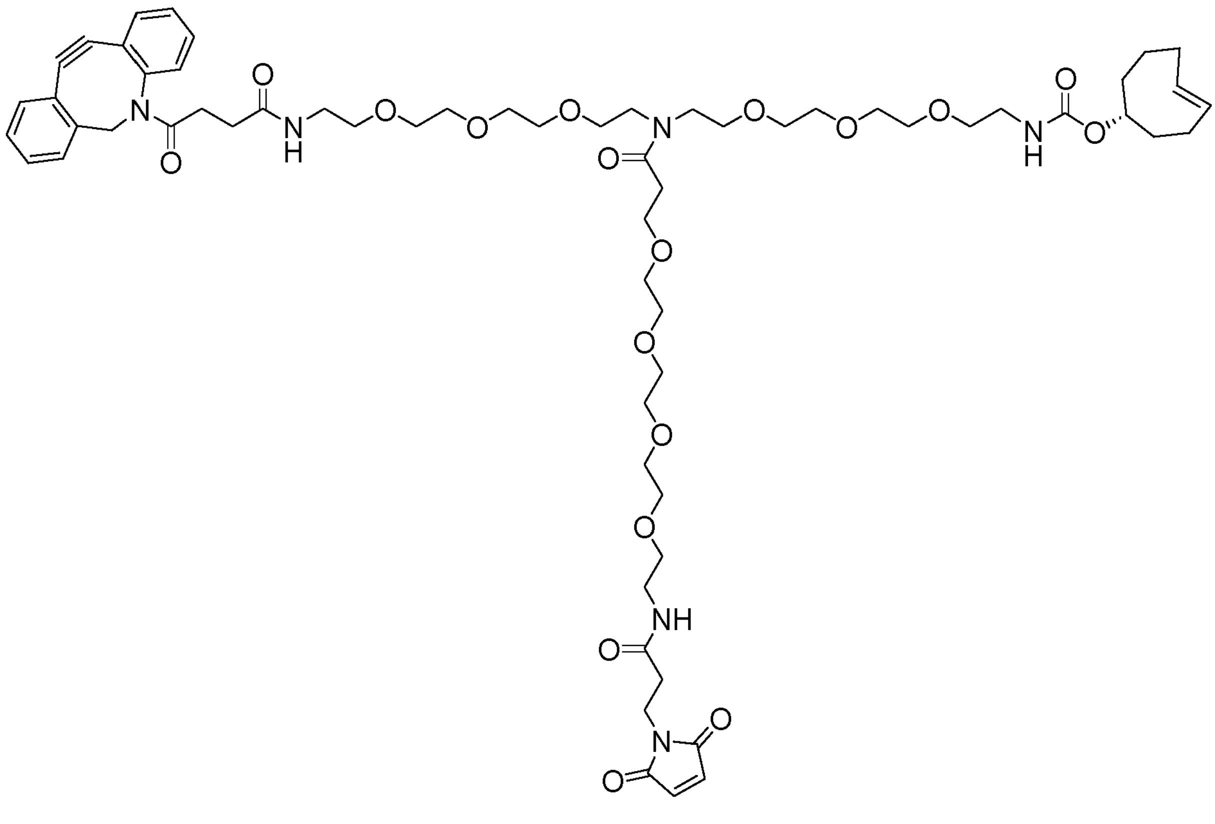

(XXI).

An embodiment is the oligonucleotide conjugate of the invention wherein a trifunctional linker, such as the trifunctional linker represented by formula (XXI), is covalently bound to one saponin moiety or more saponin moieties via a first arm of the linker, preferably 1-16 saponin moieties, more preferably 1-8 saponin moieties such as 1, 4 or 8 saponin moieties, covalently bound to at least one GalNAc moiety, preferably 1-4 GalNAc moieties, more preferably 3 GalNAc moieties, via a second arm of the linker, and covalently bound to an oligonucleotide, preferably an AON such as a BNA or an siRNA, via a third arm of the trifunctional linker. Preferred is the oligonucleotide conjugate of the invention, wherein the at least one GalNAc moiety, preferably three GalNAc moieties, the at least one saponin, preferably 1-16 saponin moieties, more preferably 1-8 saponin moieties such as 1, 4 or 8 saponin moieties and preferably 1, 4 or 8 saponin moieties, and the oligonucleotide are covalently bound via a tri-functional linker, preferably with each of the GalNAc moiety or GalNAc moieties, the saponin or the saponin moieties and the oligonucleotide covalently bound to a separate arm of the tri-functional linker.

An embodiment is the oligonucleotide conjugate of the invention comprising at least one saponin covalently linked to a ligand for asialoglycoprotein receptor (ASGPR), wherein the ligand for ASGPR comprises at least one *N*-acetylgalactosamine (GalNAc) moiety, preferably three or four GalNAc moieties, more preferably three GalNAc moieties, more preferably the ligand for ASGPR comprises or consists of $(GalNAc)_3$Tris, and further covalently linked to an oligonucleotide, wherein the at least one saponin is a monodesmosidic or bidesmosidic penta-cyclic triterpene saponin of the 12,13-dehydrooleanane type, preferably with an aldehyde function in position C-23 of the aglycone core structure of the saponin, wherein the oligonucleotide conjugate comprises 1-16 saponin moieties, preferably 1-8 saponin moieties, more preferably 1 saponin moiety, 4 saponin moieties or 8 saponin moieties.

An embodiment is the oligonucleotide conjugate of the invention, wherein the at least one GalNAc moiety, preferably three GalNAc moieties, the at least one saponin, preferably 1-16 saponin moieties, more preferably 1-8 saponin moieties such as 1, 4 or 8 saponin moieties, and the oligonucleotide are covalently bound via a tri-functional linker, preferably with each of the GalNAc moiety or moieties, the saponin or saponin moieties and the oligonucleotide covalently bound to a separate arm of the tri-functional linker.

An embodiment is the oligonucleotide conjugate of the invention, comprising one saponin moiety, 4 saponin moieties or 8 saponin moieties.

An embodiment is the oligonucleotide conjugate of the invention, wherein the at least one saponin moiety is linked via a hydrazone bond or via a semicarbazone bond. The at least one saponin is preferably covalently bound in the oligonucleotide conjugate via a semicarbazone bond. Such a hydrazone bond or semicarbazone bond (with a linker) is for

example formed involving the aldehyde group at the C-23 atom of the quillaic acid aglycone core structure of the saponin or of the gypsogenin aglycone core structure of the saponin (see Table A1 for saponins of these types).

An aspect of the invention relates to oligonucleotide conjugate according to molecule (EE):

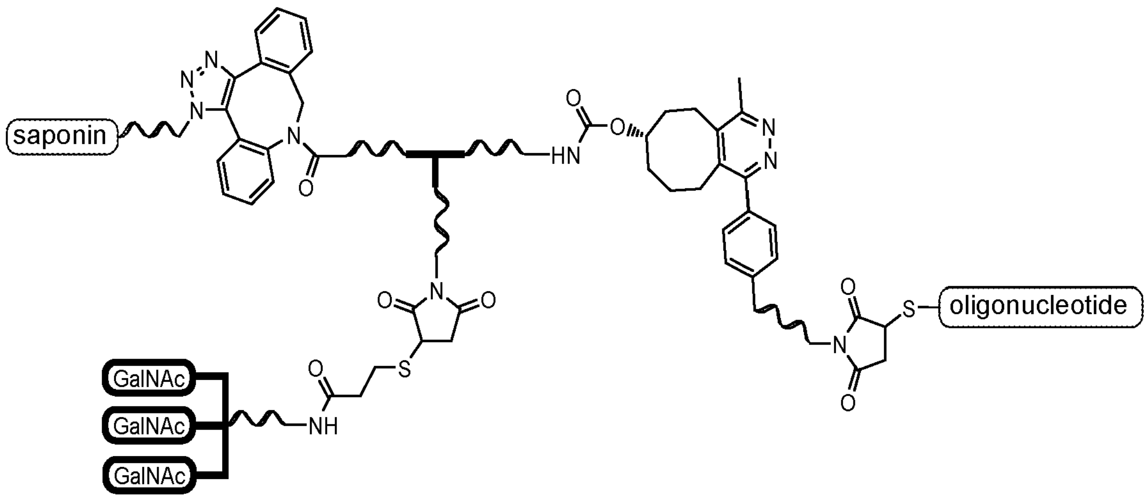

(EE)

, which molecule (EE) is the covalent conjugation product obtained by the covalent conjugation of the tri-functional linker according to formula (XXI):

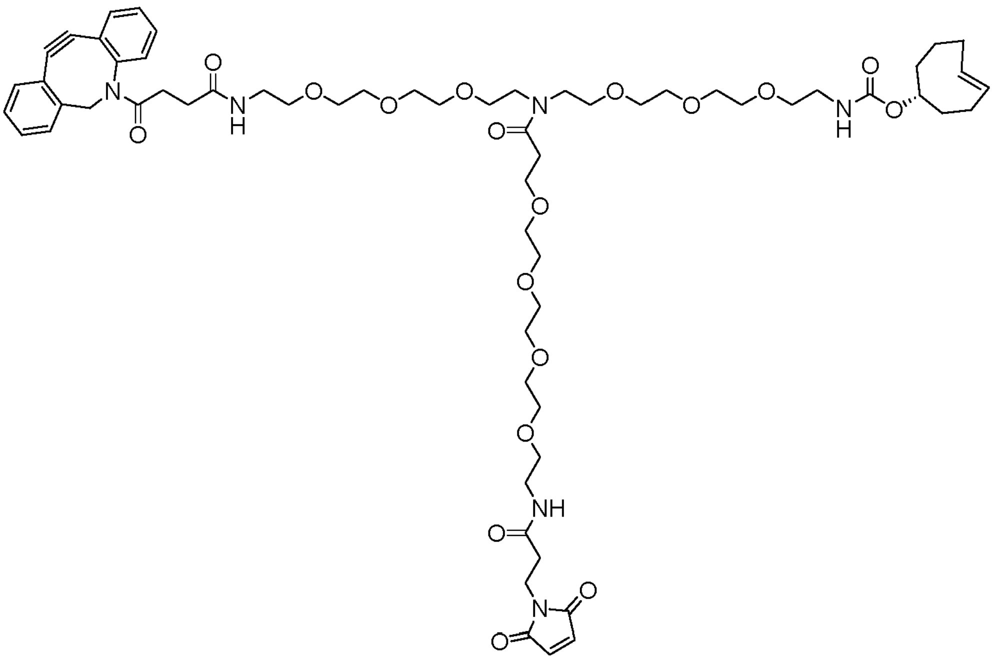

(XXI)

with

(1) the saponin derivative according to molecule (AA):

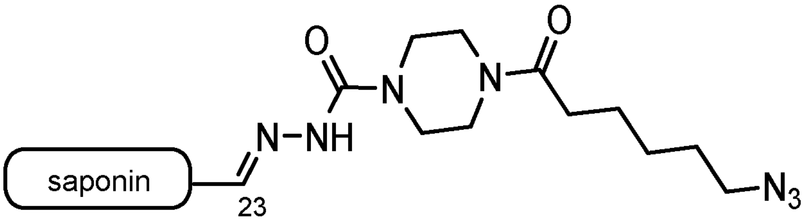

(AA)

, wherein

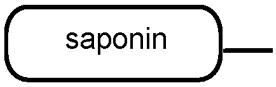

represents a saponin moiety according to formula (SM):

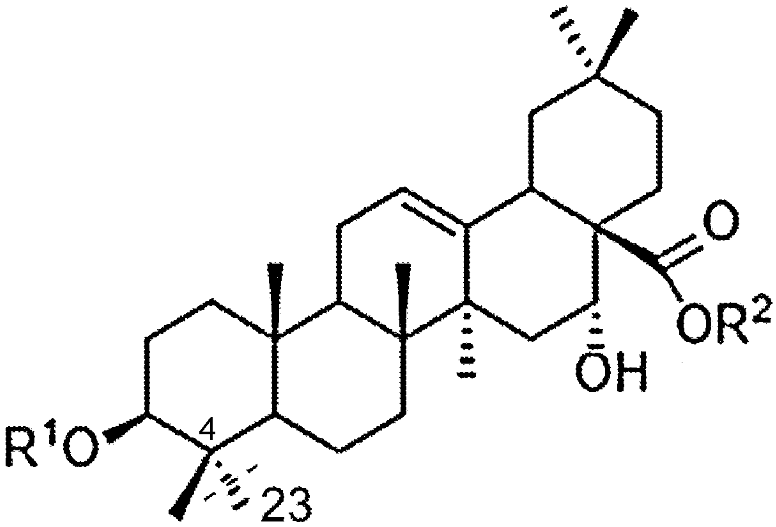

(SM)

, wherein $R^1$ and $R^2$ are independently selected from hydrogen, a monosaccharide, a linear oligosaccharide and a branched oligosaccharide, and wherein the saponin moiety according to formula (SM) is based on a saponin comprising an aldehyde group in position C-23,
and with
(2) GalNAc conjugate according to molecule (FF):

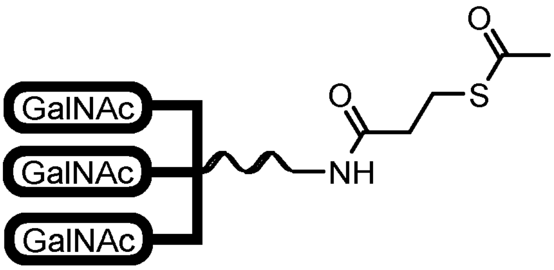

(FF)
, wherein

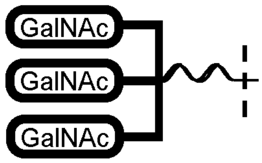

represents the tri-GalNAc conjugate according to molecule (DD1) or according to molecule (DD2):

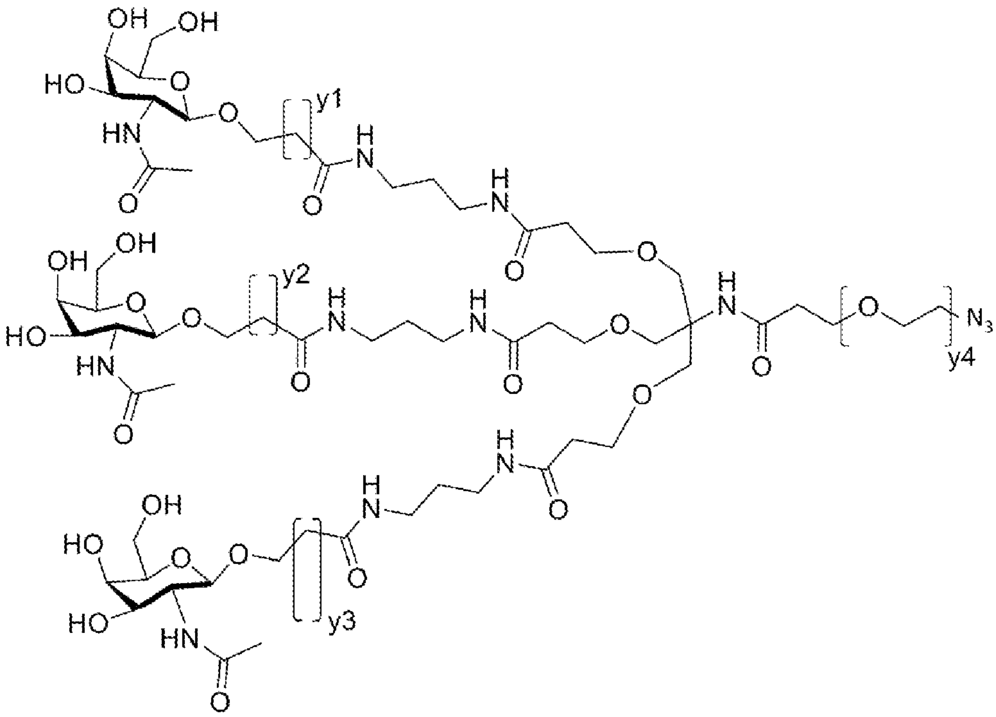

(DD1),

wherein y1, y2 and y3 each are an integer independently selected from 0-20, preferably 1-15, more preferably 2-12, even more preferably 2-10, even more preferably 2-8, most preferably 2 and 3, and preferably y1, y2 and y3 are the same, and y4 is an integer selected from 1-100, preferably 2-80, more preferably 3-70, even more preferably 4-60, even more preferably 4-50, even more preferably 4-40, even more preferably 4-30, even more preferably 4-20, even more preferably 4-6, most preferably 4-5, such as 4;

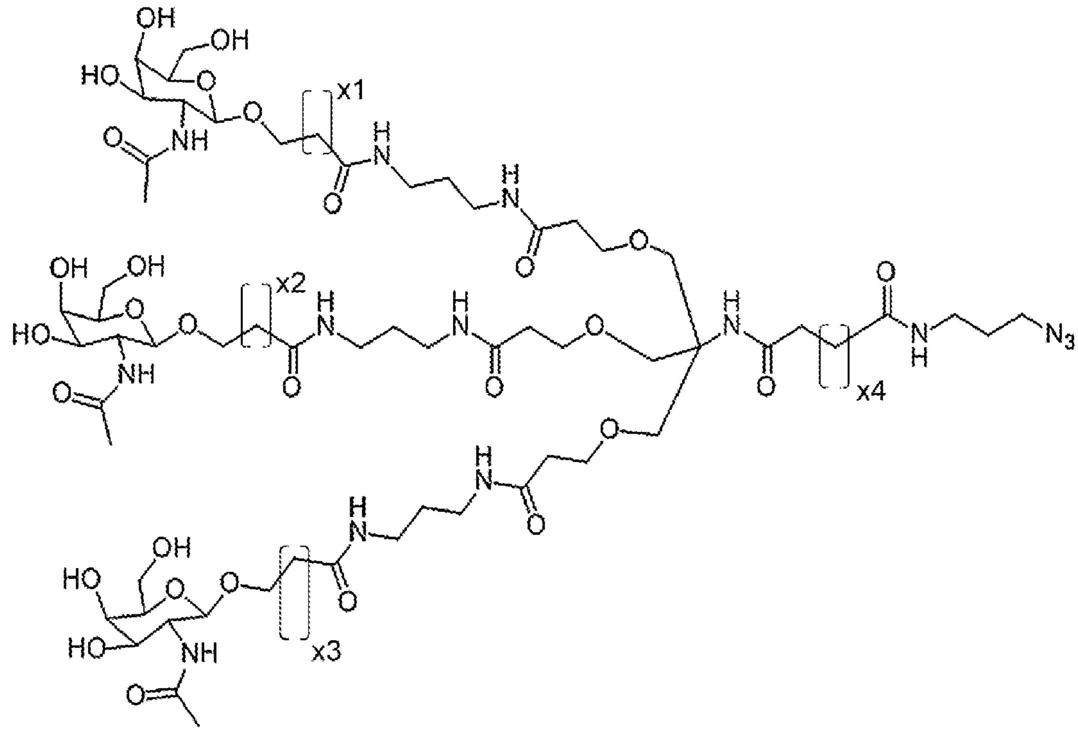

(DD2),

wherein x1, x2 and x3 each are an integer independently selected from 0-20, preferably 1-15, more preferably 2-12, even more preferably 2-10, even more preferably 2-8, most preferably 2 and 3, and preferably x1, x2 and x3 are the same, and x4 is an integer selected from 1-50, preferably 2-40, more preferably 3-30, even more preferably 4-20, even more preferably 5-15, most preferably 8-12, such as 9,
and preferably represents the tri-GalNAc conjugate according to molecule (DD3) or according to molecule (DD4):

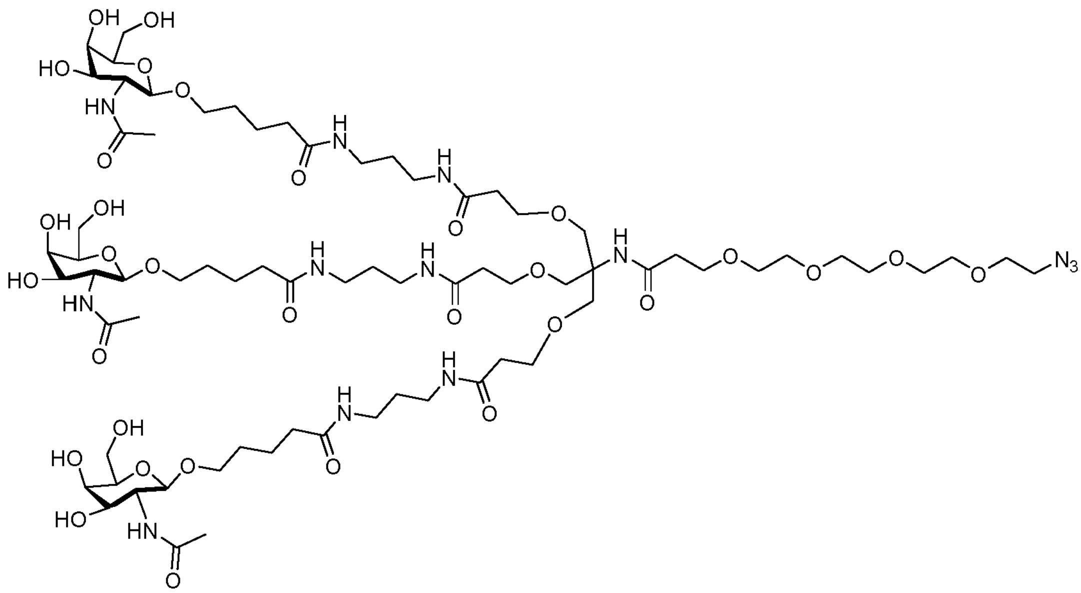

(DD3),

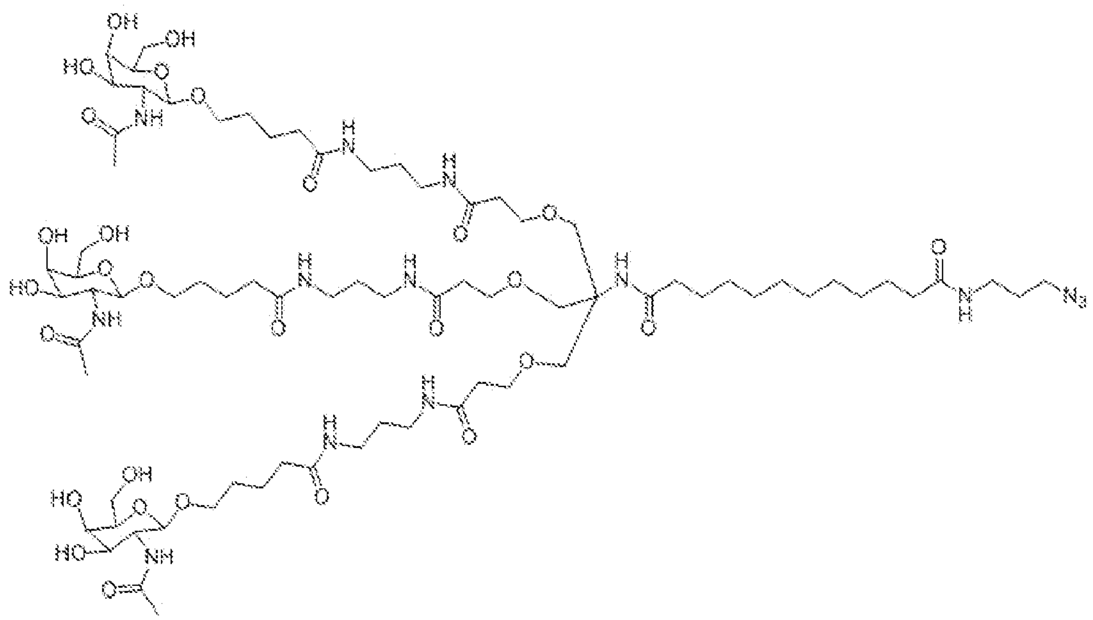

(DD4),

and with
(3) the oligonucleotide provided with a linker according to molecule (GG):

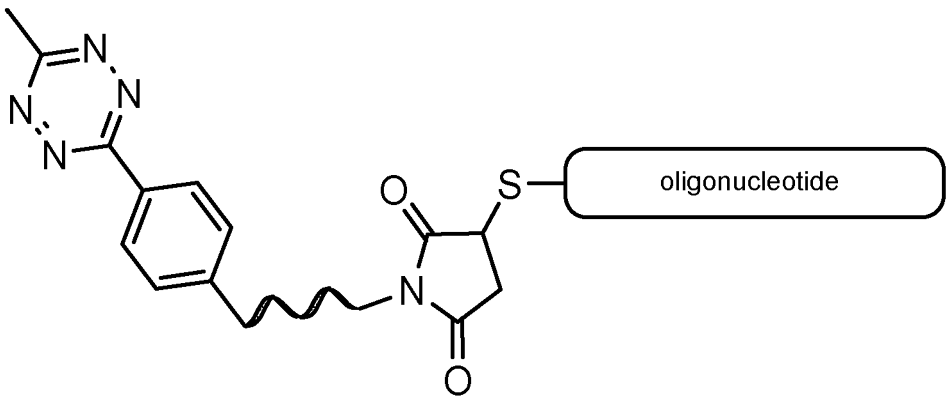

(GG)

, wherein the molecule (GG) represents the conjugation product of the conjugation reaction between the linker (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide and the oligonucleotide-linker molecule according to molecule (HH):

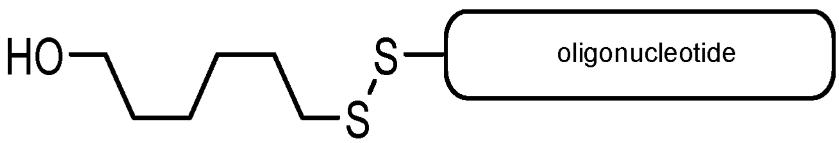

(HH).

Such an oligonucleotide conjugate according to molecule (EE) of the invention displays a surprisingly improved

potency when the endosomal escape enhancing activity of the saponin moiety is considered. It is thought that improved efficacy relates to improved de-coupling (release) of the saponin from the conjugate by cleavage of the semicarbazone functional group under influence of the slightly acidic pH in the endosome of cells which have taken up the conjugate mediated by binding of the conjugate to the ASGPR. Therewith, endosomal escape of the oligonucleotide is enhanced and (gene-silencing) activity of the oligonucleotide in the cytosol and/or in the nucleus is enhanced, compared to applying the similar conjugate though without the covalently linked saponin moiety.

An example of an oligonucleotide conjugate of the invention comprising a single saponin moiety is the oligonucleotide displayed in Fig. 35D as molecule 13. Synthesis of such an oligonucleotide of the invention comprising a single saponin moiety is exemplified by the detailed description of the synthesis of molecule 13 (Fig. 35D) in the Examples section here below and displayed in Fig. 35. The saponin moiety is linked to the trifunctional linker via a linker, wherein the saponin moiety is linked to the linker via a hydrazone bond. A further example of such an oligonucleotide conjugate of the invention comprising a single saponin moiety is the oligonucleotide displayed in Fig. 38C as molecule 31. Synthesis of such an oligonucleotide conjugate of the invention comprising a single saponin moiety is exemplified by the detailed description of the synthesis of molecule 31 (Fig. 38C) in the Examples section here below and displayed in Fig. 38. The saponin moiety is linked to the tri-functional linker via a linker, wherein the saponin moiety is linked to said linker via a semicarbazone bond (see for example molecule 28 in Fig. 38A). See also the saponin derivative according to molecule (AA), here above.

An embodiment is the oligonucleotide conjugate of the invention comprising four saponin moieties. An embodiment is the oligonucleotide conjugate of the invention comprising four saponin moieties covalently bound to a dendron, preferably a G2 dendron. An example of such a (G2) dendron is N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide). Synthesis of a dendron linked to four saponin moieties is for example displayed in Fig. 39B and in the Examples section for synthesis of Intermediate 20 (molecule 39) form molecule 37 (saponin linked to maleimide involving a semicarbazone bond between the saponin, here SO1861, and the linker bearing the maleimide group), and molecule 38, which is dendron N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate. An aspect of the invention relates to oligonucleotide conjugate according to molecule (PP):

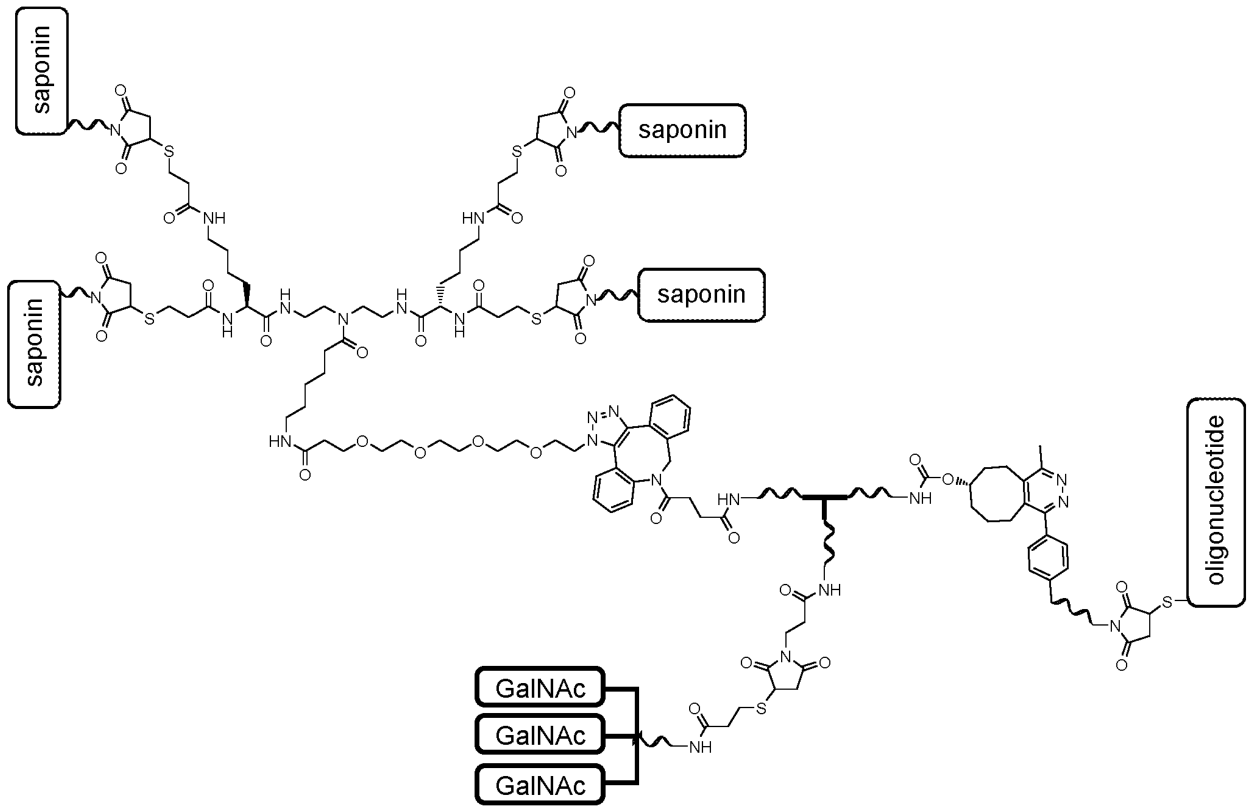

(PP)

, which molecule (PP) is the covalent conjugation product obtained by the covalent conjugation of the saponin-GalNAc conjugate according to molecule (LL):

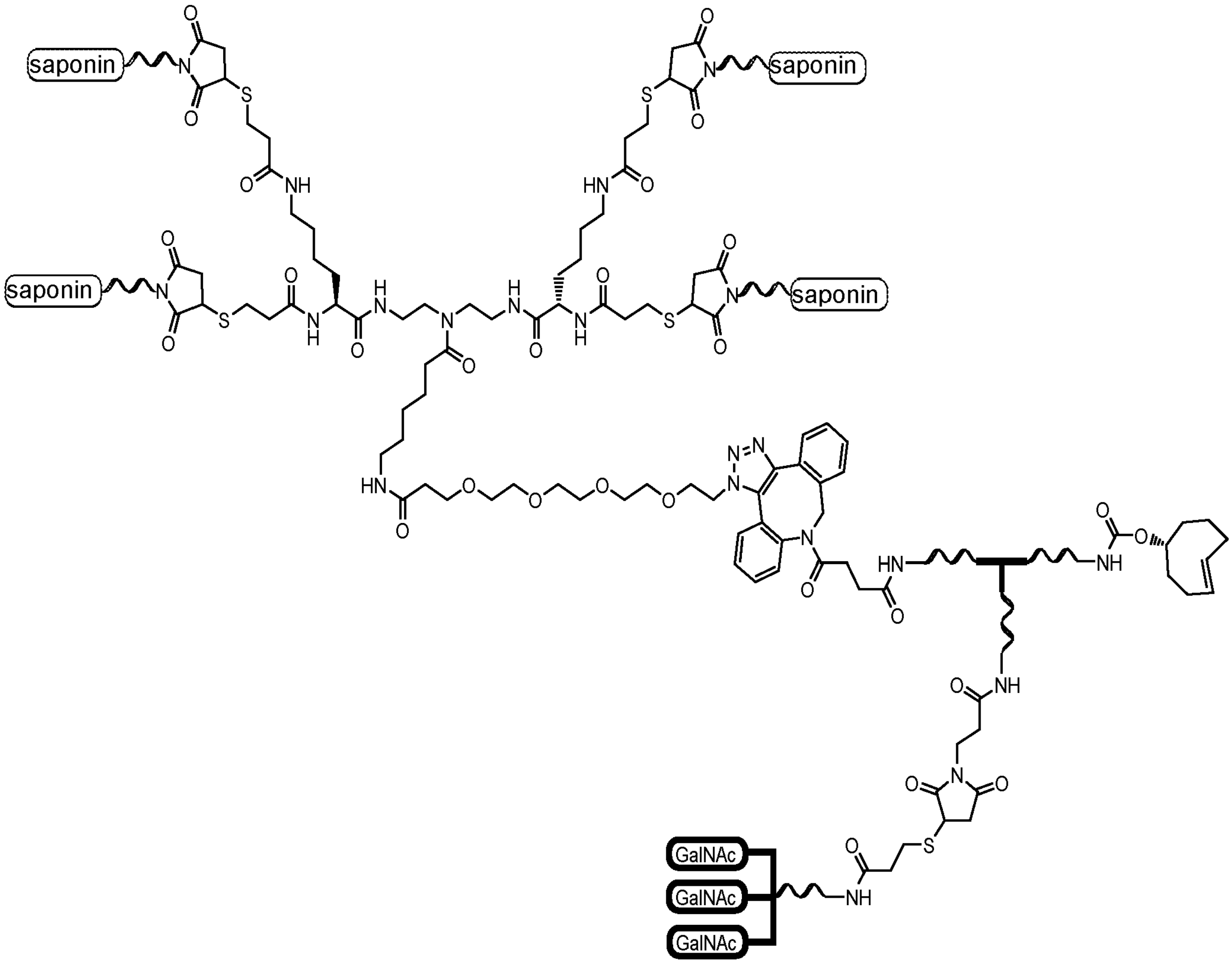

(LL)

with the oligonucleotide provided with a linker according to molecule (GG):

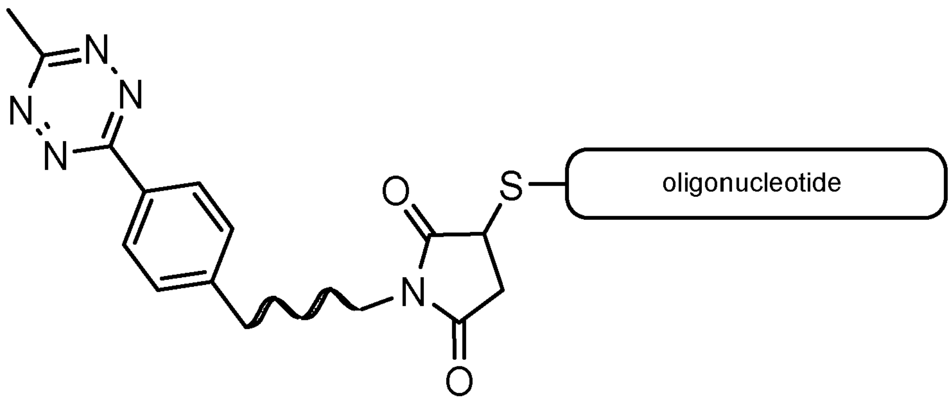

(GG)

, wherein the molecule (GG) represents the conjugation product of the conjugation reaction between the linker

(E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide and the oligonucleotide-linker molecule according to molecule (HH):

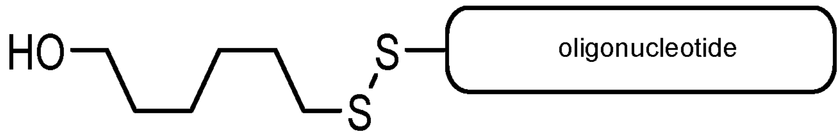

(HH)

, which molecule (LL) is the covalent conjugation product obtained by the covalent conjugation of the saponin derivative according to molecule (JJ):

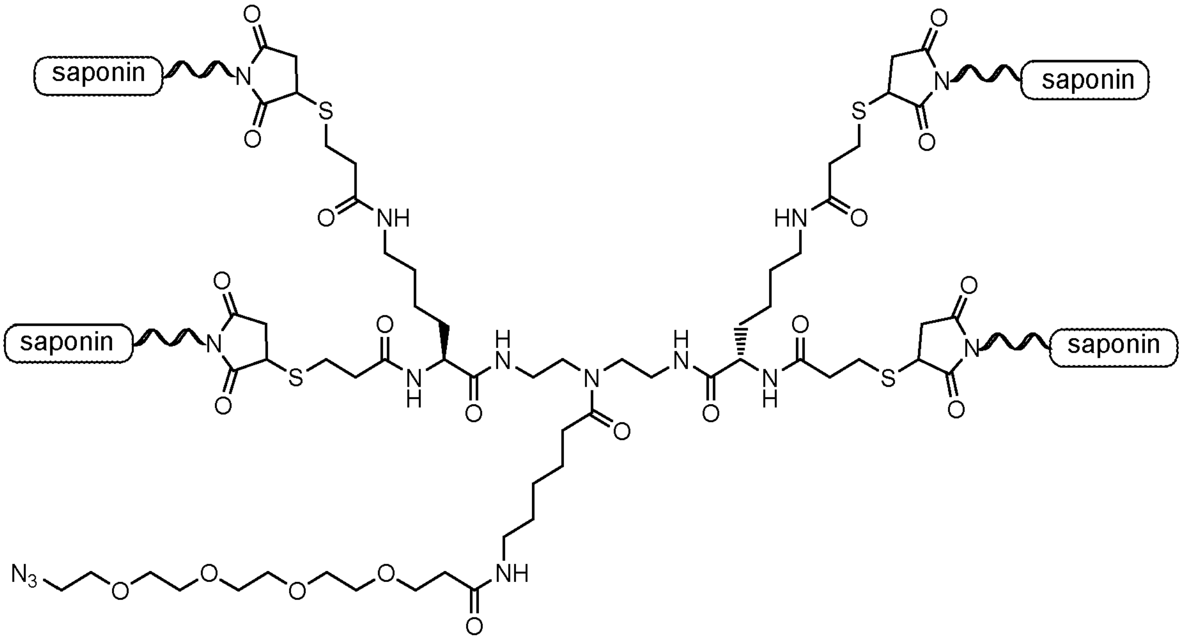

(JJ)

with the conjugate of the tri-functional linker and GalNAc according to molecule (MM):

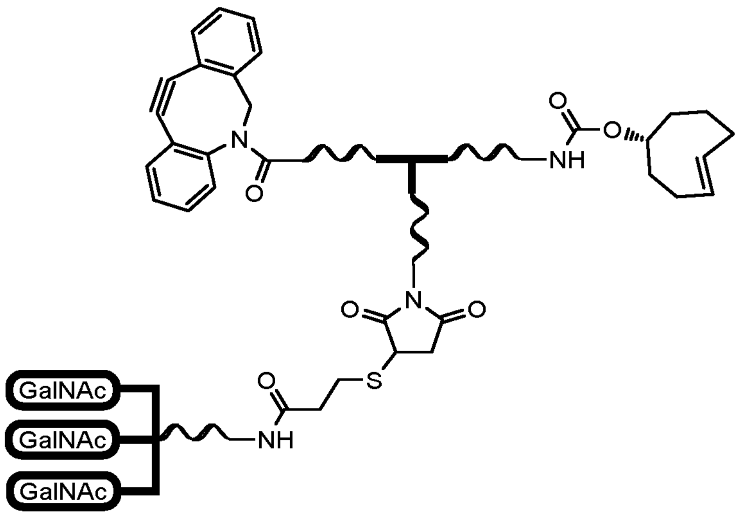

(MM)

, which molecule (JJ) is the conjugate product of conjugation of N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide first with the saponin derivative according to molecule (KK):

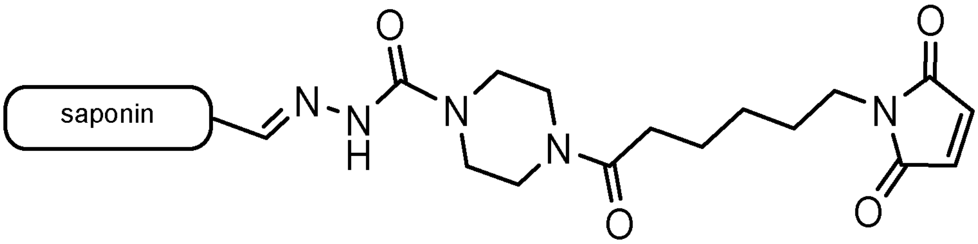

(KK)

, wherein

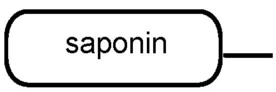

represents a saponin moiety according to formula (SM):

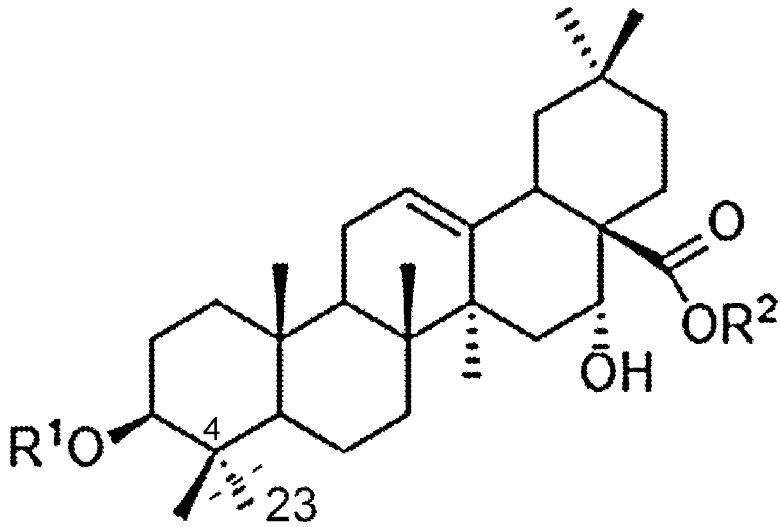

(SM)

, wherein $R^1$ and $R^2$ are independently selected from hydrogen, a monosaccharide, a linear oligosaccharide and a branched oligosaccharide according to the invention, and wherein the saponin moiety according to formula (SM) is based on a saponin comprising an aldehyde group in position C-23 according to any one of the saponins of the invention, and for example listed in Table A1, such as QS-21, SO1861, SO1832,

and subsequently with 2,5-dioxopyrrolidin-1-yl 1-azido-3,6,9,12-tetraoxapentadecan-15-oate,

, and wherein molecule (MM) is the conjugate of the tri-functional linker according to formula (XXI):

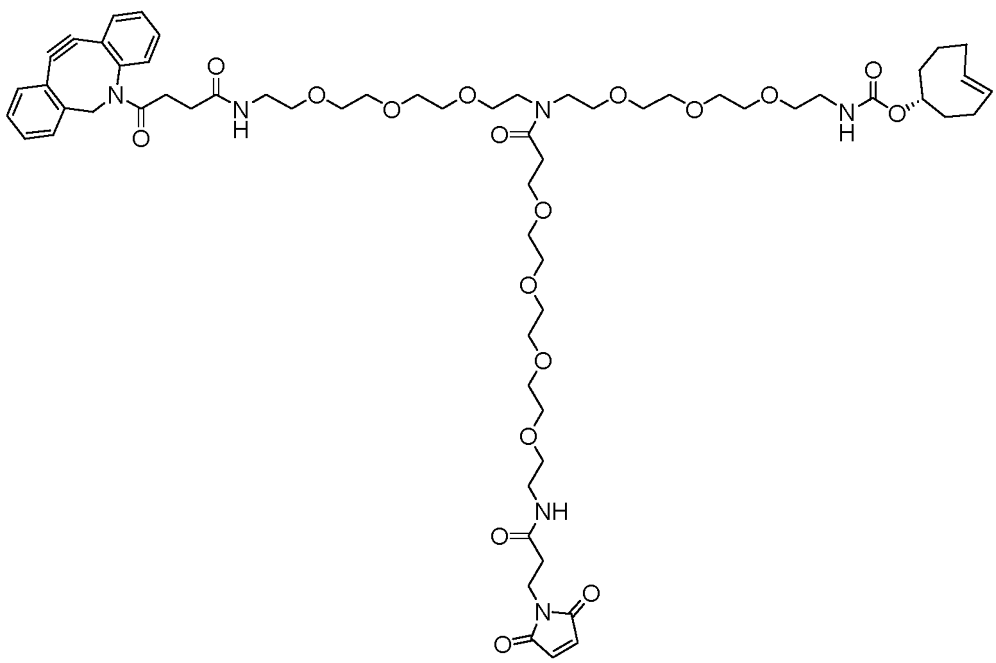

(XXI)

and the GalNAc conjugate according to molecule (NN):

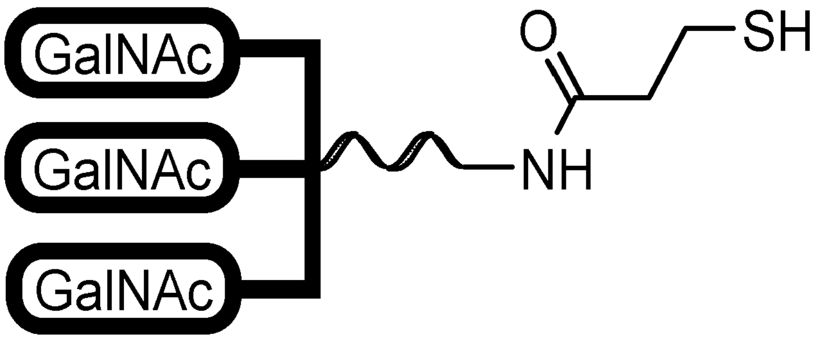

(NN)

, wherein

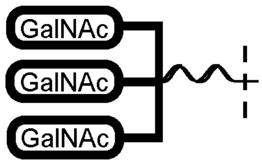

represents the tri-GalNAc conjugate according to molecule (DD1) or molecule (DD2) as detailed here above, preferably molecule (DD3) or molecule (DD4) as detailed here above, more preferably molecule (DD3) as detailed here above.

An example of such an oligonucleotide conjugate of the invention comprising four saponin moieties is the oligonucleotide conjugate displayed in Fig. 36E as molecule 22. Synthesis of such an oligonucleotide conjugate of the invention comprising four saponin moieties is exemplified by the detailed description of the synthesis of molecule 22 (Fig. 36E) in the Examples section here below and displayed in Fig. 36. The saponin moieties are linked to the dendron via a linker, wherein each saponin moiety is linked to a linker via a hydrazone bond. A further example of such an oligonucleotide conjugate of the invention comprising four saponin moieties is the oligonucleotide conjugate displayed in Fig. 39F as molecule 42. Synthesis of such an oligonucleotide conjugate of the invention comprising four saponin moieties is exemplified by the detailed description of the synthesis of molecule 42 (Fig. 39F) in the Examples section here below and displayed in Fig. 39. The saponin moieties are linked to the dendron via a linker, wherein each saponin moiety is linked to a linker via a semicarbazone bond. See also the saponin derivative according to molecule (KK), here above.

An embodiment is the oligonucleotide conjugate of the invention comprising eight saponin moieties. As said, preferred is the oligonucleotide conjugate of the invention, wherein the at least one GalNAc moiety, preferably three GalNAc moieties, the at least one saponin, preferably 1-16 saponin moieties, more preferably 1-8 saponin moieties such as 8 saponin moieties, and the oligonucleotide are covalently bound via a tri-functional linker, preferably with each of the GalNAc moiety or GalNAc moieties, the single saponin or the more than one saponin moieties and the oligonucleotide covalently bound to a separate arm of the tri-functional linker.

An embodiment is the oligonucleotide conjugate of the invention comprising eight saponin moieties covalently bound to a dendron, preferably a G3 dendron. An example of such a (G3) dendron is (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide. Synthesis of a dendron linked to eight saponin moieties is for example displayed in Fig. 40A and in the Examples section for synthesis of Intermediate 25 (molecule 44) form molecule 37 (saponin linked to maleimide involving a semicarbazone bond between the saponin, here SO1861, and the linker bearing the maleimide group), and molecule 43, which is dendron (2S)-N-[(1S)-1-{[2-(6-amino-N-{2-[(2S)-2,6-bis[(2S)-2,6-bis(3-sulfanylpropanamido)hexanamido]hexanamido]ethyl} hexanamido)ethyl]carbamoyl}-5-[(2S)-2,6-bis(3-sulfanylpropanamido)hexanamido]pentyl]-2,6-bis(3-sulfanylpropanamido)hexanamide formate.

An aspect of the invention relates to oligonucleotide conjugate according to molecule (SS):

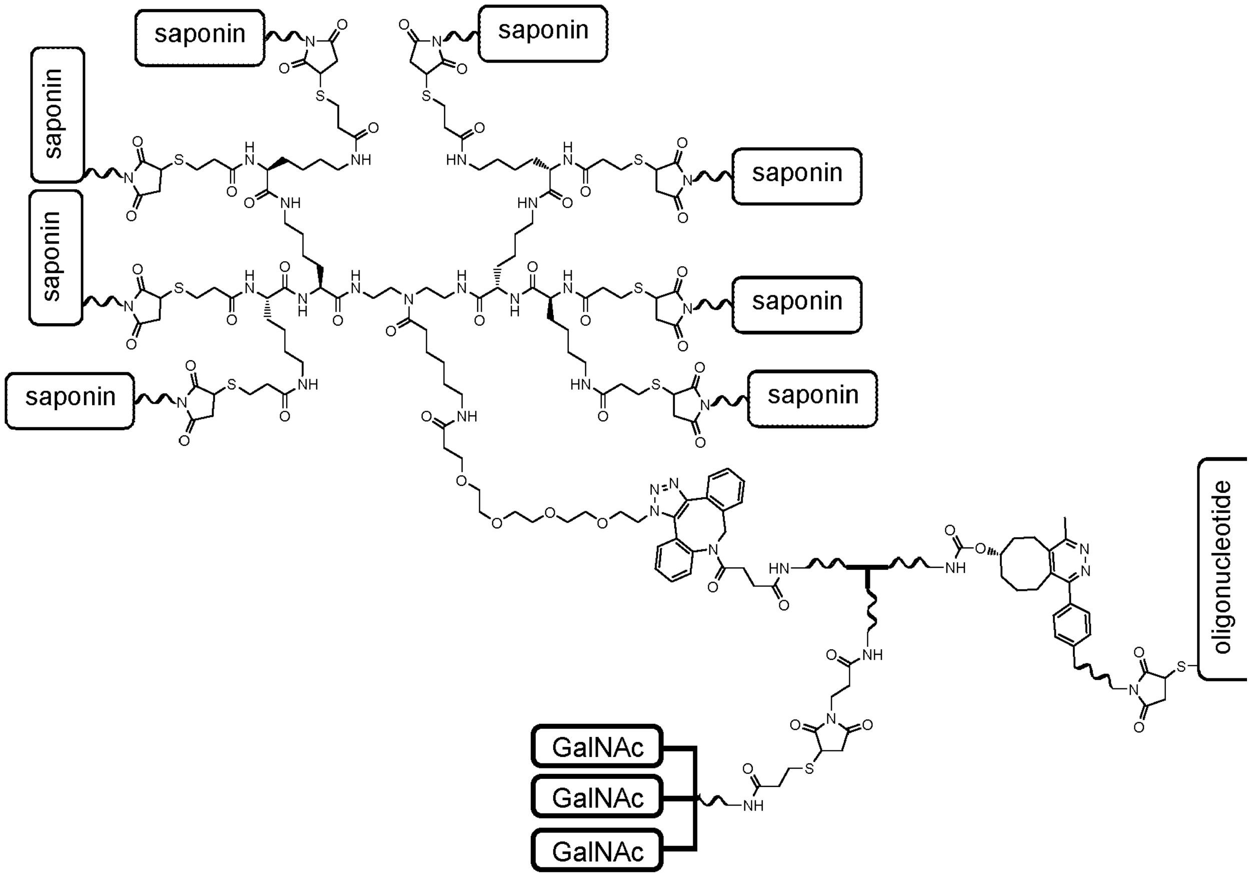

(SS)

, which molecule (SS) is the covalent conjugation product obtained by the covalent conjugation of the saponin-GalNAc conjugate according to molecule (RR):

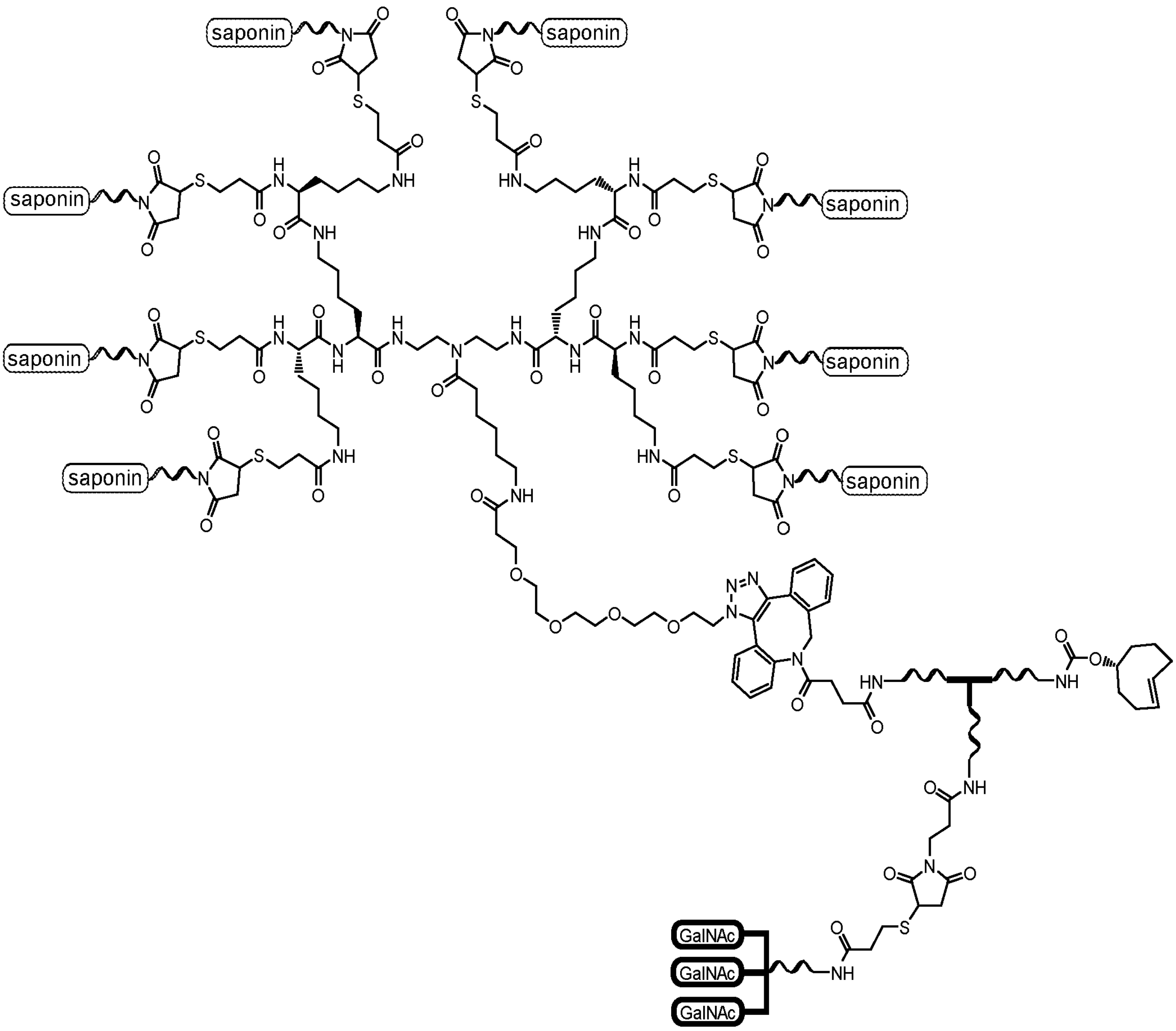

(RR)

with the oligonucleotide provided with a linker according to molecule (GG):

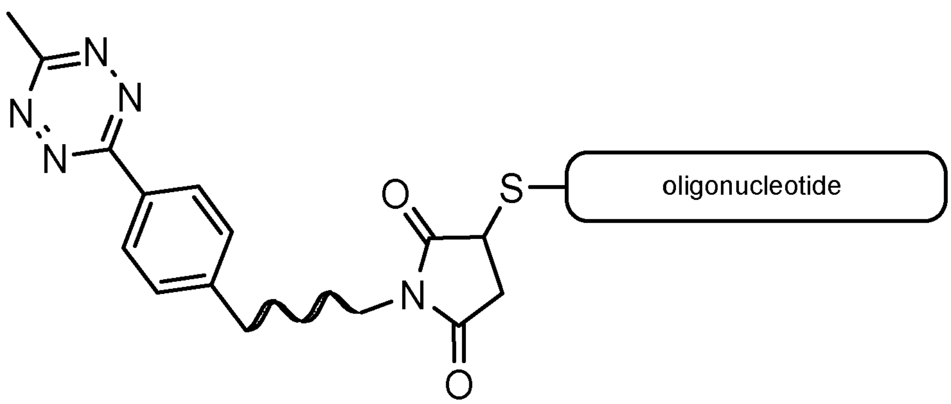

(GG)

, wherein the molecule (GG) represents the conjugation product of the conjugation reaction between the linker

(E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide and the oligonucleotide-linker molecule according to molecule (HH):

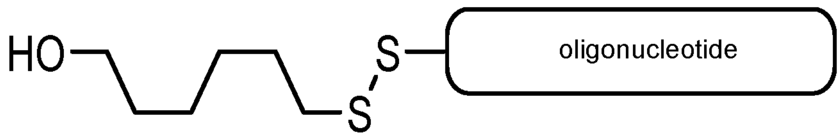

(HH)

, which molecule (RR) is the covalent conjugation product obtained by the covalent conjugation of the saponin derivative according to molecule (QQ):

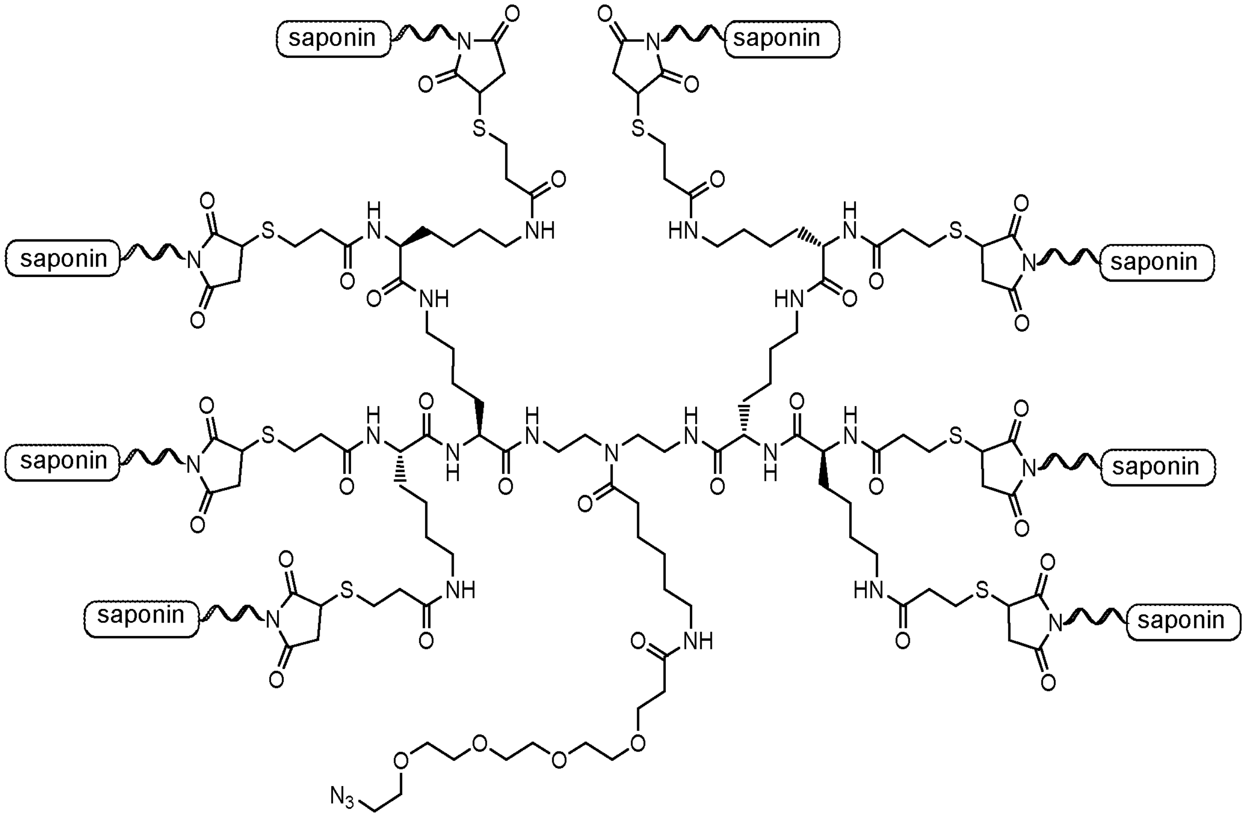

(QQ)

with the conjugate of the tri-functional linker and GalNAc according to molecule (MM):

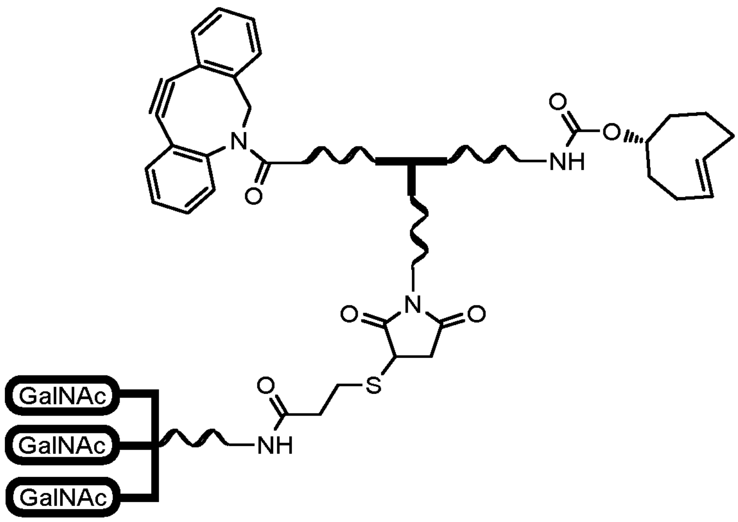

(MM)

, which molecule (QQ) is the conjugate product of conjugation of (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate first

(a) with the saponin derivative according to molecule (KK):

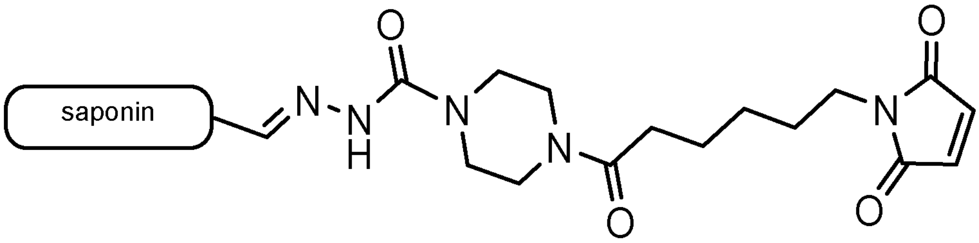

(KK)

, wherein

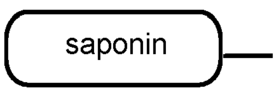

represents a saponin moiety according to formula (SM):

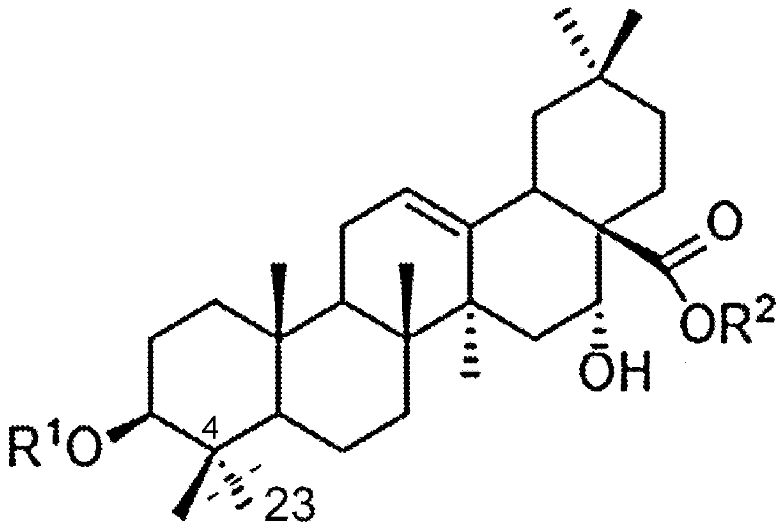

(SM)

, wherein $R^1$ and $R^2$ are independently selected from hydrogen, a monosaccharide, a linear oligosaccharide and a branched oligosaccharide according to the invention, and wherein the saponin moiety according to formula (SM) is based on a saponin comprising an aldehyde group in position C-23 according to any one of the saponins of the invention, and for example listed in Table A1, such as QS-21, SO1861, SO1832,
and subsequently
(b) with 2,5-dioxopyrrolidin-1-yl 1-azido-3,6,9,12-tetraoxapentadecan-15-oate,
and wherein molecule (MM) is the conjugate of the tri-functional linker according to formula (XXI):

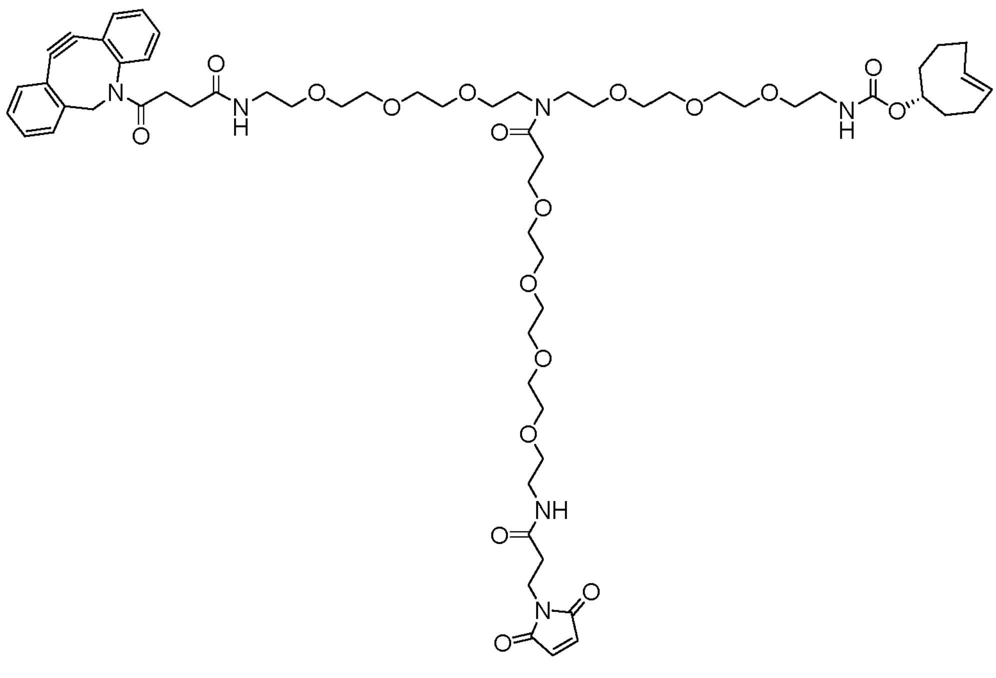

(XXI)

and the GalNAc conjugate according to molecule (NN) (See here above), wherein

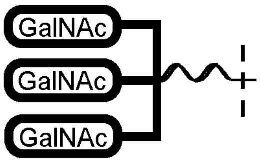

represents the tri-GalNAc conjugate according to molecule (DD1) or molecule (DD2) as detailed here above, preferably molecule (DD3) or molecule (DD4) as detailed here above, more preferably molecule (DD3) as detailed here above.

An example of such an oligonucleotide conjugate of the invention comprising eight saponin moieties is the oligonucleotide conjugate displayed in Fig. 37C as molecule 26. Synthesis of such an oligonucleotide conjugate of the invention comprising eight saponin moieties is exemplified by the detailed description of the synthesis of molecule 26 (Fig. 37C) in the Examples section here below and displayed in Fig. 37. The saponin moieties are linked to the dendron via a linker, wherein each saponin moiety is linked to a linker via a hydrazone bond. A further example of such an oligonucleotide conjugate of the invention comprising eight saponin moieties is the oligonucleotide conjugate displayed in Fig. 40D as molecule 47. Synthesis of such an oligonucleotide conjugate of the invention comprising eight saponin moieties is exemplified by the detailed description of the synthesis of molecule 47 (Fig. 40D) in the Examples section here below and displayed in Fig. 40. The saponin moieties are linked to the dendron via a linker, wherein each saponin moiety is linked to a separate linker via a semicarbazone bond. See also the saponin derivative according to molecule (KK), here above.

Preferred is the oligonucleotide conjugate of the invention, comprising one saponin moiety, or 4 saponin moieties, preferably 4 saponin moieties covalently bound to a dendron, preferably a G2 dendron such as for example N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide), or 8 saponin moieties, preferably 8 saponin moieties covalently bound to a dendron, preferably a G3 dendron such as for example (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide.

An embodiment is the oligonucleotide conjugate according to the invention and for example according to any one of the molecules (EE), (PP) and (SS), wherein the oligonucleotide conjugate is based on a saponin according to any one of the saponins listed here above and in Table A1.

Preferred is the oligonucleotide conjugate according to the invention and for example according to any one of the molecules (EE), (PP) and (SS), wherein the oligonucleotide conjugate is based on a saponin selected from SO1861, SO1832 and QS-21, preferably SO1861 and SO1832, more preferably SO1861 or SO1832. When the saponin is SO1861, the saponin derivative according to molecule (VII)a:

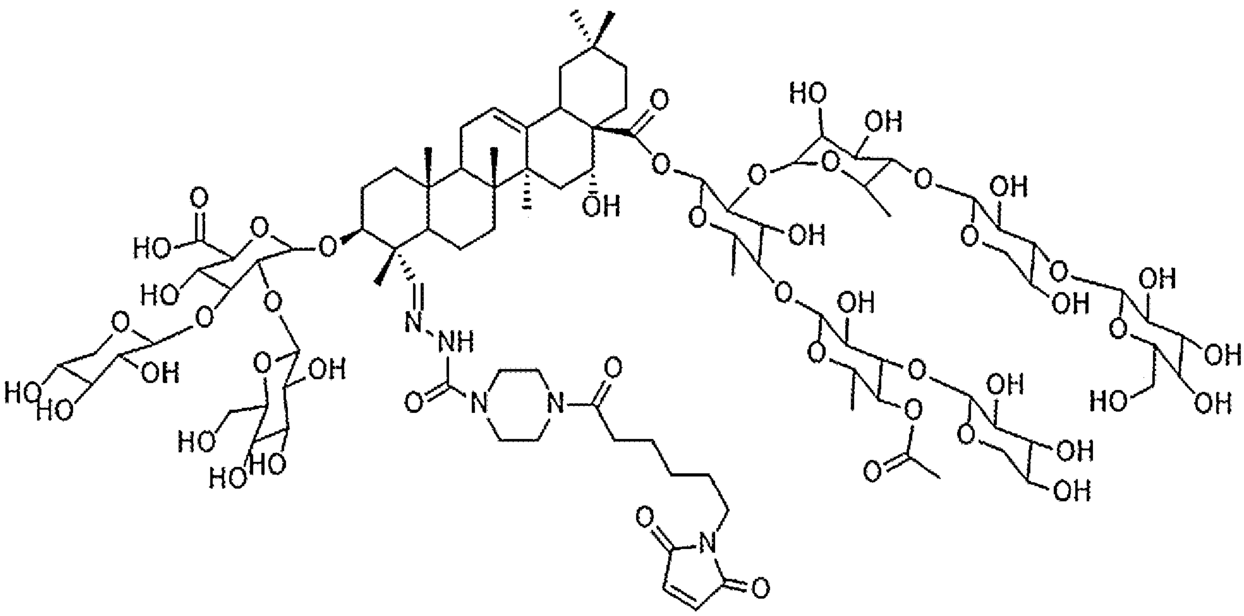

(VII)a

is conjugated with an oligonucleotide and with at least one GalNAc moiety, to provide an oligonucleotide conjugate of the invention.

Also preferred is the oligonucleotide conjugate according to the invention and for example according to any one of the molecules (EE), (PP) and (SS), wherein the semicarbazone functional group is subject to hydrolysis *in vivo* under acidic conditions as present in endosomes and/or lysosomes of mammalian cells, preferably human cells, preferably at pH 4.0 - 6.5, and more preferably at pH ≤ 5.5.

An embodiment is the oligonucleotide conjugate of the invention, wherein the saponin comprised by the conjugate is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Gypsophila elegans* saponin mixture, or a saponin isolated from *Gypsophila elegans*;
- Saponinum album saponin mixture, or a saponin isolated from Saponinum album;
- *Saponaria officinalis* saponin mixture, or a saponin isolated from *Saponaria officinalis*; and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C: AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, 10 SO1832, SO1861, SO1862, SO1904, QS-7, QS-7 api, QS-17, QS-18, QS-21 A-apio, QS-21 A-xylo, QS-21 B-apio and QS-21 B-xylo; or
d) a saponin comprising a 12, 13-dehydrooleanane type aglycone core structure without an aldehyde group at the C-23 position of the aglycone, selected from list D:
Aescin la, aescinate, alpha-Hederin, AMA-1, AMR, AS6.2, AS64R, Assamsaponin F, dipsacoside B, esculentoside A, macranthoidin A, NP-005236, NP-012672, Primula acid 1, saikosaponin A, saikosaponin D, Teaseed saponin I and Teaseedsaponin J,

preferably, the saponin is any one or more of a saponin selected from list A, B or C, more preferably, a saponin selected from list B or C,
even more preferably, a saponin selected from list C.

Preferred is the oligonucleotide conjugate of the invention, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* Saponarioside B, SO1542, SO1584, SO1658, SO1674, SO1700, SO1730, SO1772, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

Also preferred is the oligonucleotide conjugate of the invention, wherein the saponin is a saponin isolated from *Saponaria officinalis,* preferably wherein the saponin is any one or more of Saponarioside B, SO1542, SO1584, SO1658, SO1674, SO1700, SO1730, SO1772, SO1832, SO1861, SO1862 and SO1904; more preferably wherein the saponin is any one or more of SO1542, SO1584, SO1658, SO1674, SO1700, SO1730, SO1772, SO1832, SO1861, SO1862 and SO1904; even more preferably wherein the saponin is any one or more of SO1832, SO1861 and SO1862; even more preferably wherein the saponin is SO1832 and SO1861; most preferably being SO1861.

An embodiment is the oligonucleotide conjugate of the invention, wherein the saponin is SO1861, and wherein the oligonucleotide conjugate is provided by conjugation of SO1861 saponin derivative according to molecule (VII)a:

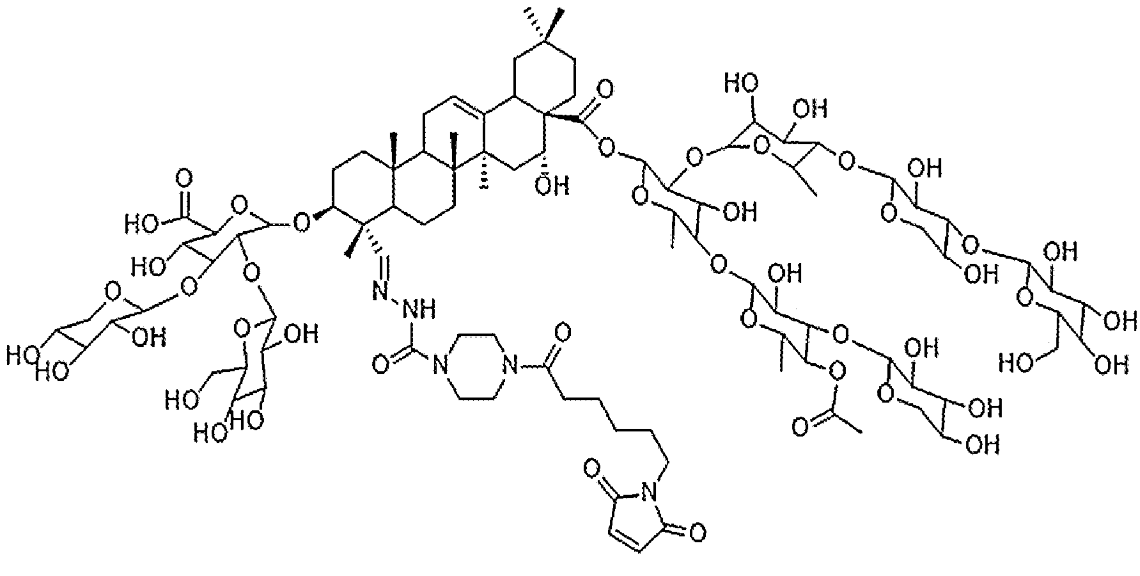

(VII)a

with an oligonucleotide and with at least one GalNAc moiety, to provide the oligonucleotide conjugate.

An embodiment is the oligonucleotide conjugate of the invention, wherein the oligonucleotide comprised by said conjugate is defined as a nucleic acid no longer than 150 nt, preferably wherein the oligonucleotide has a size of 5 - 150 nt, preferably being 8 - 100 nt, most preferably being 10 - 50 nt.

An embodiment is the oligonucleotide conjugate of the invention, wherein the semicarbazone functional group is subject to hydrolysis *in vivo* under acidic conditions as present in endosomes and/or lysosomes of mammalian cells, preferably human cells, preferably at pH 4.0 - 6.5, and more preferably at pH ≤ 5.5. Upon said hydrolysis, the aldehyde group of the saponin or of the saponin moieties comprised by the oligonucleotide conjugate is/are formed.

An embodiment is the oligonucleotide conjugate of the invention, wherein the hydrazone functional group is subject to hydrolysis *in vivo* under acidic conditions as present in endosomes and/or lysosomes of mammalian cells, preferably human cells, preferably at pH 4.0 - 6.5, and more preferably at pH ≤ 5.5. Upon said hydrolysis, the aldehyde group of the saponin or of the saponin moieties comprised by the oligonucleotide conjugate is/are formed.

An embodiment is the oligonucleotide conjugate according to the invention and for example according to any one of the molecules (EE), (PP) and (SS), comprising an oligonucleotide, wherein the oligonucleotide is an AON such as any one of a BNA, a xeno nucleic acid, an siRNA.

An embodiment is the oligonucleotide conjugate according to the invention and for example according to any one of the molecules (EE), (PP) and (SS), comprising an oligonucleotide, wherein the oligonucleotide is selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic acid ($BNA^{NC}$), BNA-based siRNA, and BNA-based antisense oligonucleotide (BNA-AON).

An embodiment is the oligonucleotide conjugate according to the invention and for example according to any one of the molecules (EE), (PP) and (SS), comprising an oligonucleotide, wherein the oligonucleotide is selected from any one or more of a(n): anti-miRNA, a BNA-AON or an siRNA, such as BNA-based siRNA, selected from chemically modified siRNA, metabolically stable siRNA and chemically modified, metabolically stable siRNA.

An embodiment is the oligonucleotide conjugate according to the invention and for example according to any one of the molecules (EE), (PP) and (SS), comprising an oligonucleotide, wherein the oligonucleotide is an oligonucleotide capable of, for example when present inside a mammalian cell and preferably when present inside a human cell, silencing any one of genes: apolipoprotein B (*apoB*), *HSP27,* transthyretin (*TTR*), proprotein convertase subtilisin/kexin type 9 (*PCSK9*), *TMPRSS6,* delta-aminolevulinate synthase 1 (*ALAS1*), anti-thrombin 3 (*AT3*), glycolate oxidase (GO), complement component C5 (*CC5*), *X* gene of hepatitis B virus (HBV), S gene of HBV, alpha-1 antitrypsin (*AAT*), miR-122, hepatitis B virus HbsAg, *LDHA, CEBPA* and lactate dehydrogenase (*LDH*), and/or is an oligonucleotide capable of, for example when present inside a mammalian cell, targeting an aberrant miRNA.

An embodiment is the oligonucleotide conjugate according to the invention and for example according to any one of the molecules (EE), (PP) and (SS), comprising an oligonucleotide, wherein the oligonucleotide is an oligonucleotide capable of, for example when present inside a mammalian cell and preferably when present inside a human cell, silencing any one of genes: apolipoprotein B (*apoB*) and *HSP27.*

An embodiment is the oligonucleotide conjugate according to the invention and for example according to any one of the molecules (EE), (PP) and (SS), comprising an oligonucleotide, wherein the oligonucleotide is an oligonucleotide capable of, for example when present inside a mammalian cell and preferably when present inside a human cell, targeting an mRNA involved in expression of any one of proteins: apoB, HSP27, TTR, PCSK9, TMPRSS6, ALAS1, AT3, GO, CC5, expression product of X gene of HBV, expression product of S gene of HBV, AAT, miR-122, hepatitis B virus HbsAg, LDHA, CEBPA and LDH, or is capable of, for example when present inside a mammalian cell and preferably when present inside a human cell, antagonizing or restoring an miRNA function such as inhibiting an oncogenic miRNA (onco-miR) or suppression of expression of an onco-miR.

An embodiment is the oligonucleotide conjugate according to the invention and for example according to any one of the molecules (EE), (PP) and (SS), comprising an oligonucleotide, wherein the oligonucleotide is an oligonucleotide capable of, for example when present inside a mammalian cell and preferably when present inside a human cell, targeting an mRNA involved in expression of any one of proteins: HSP27, apoB, TTR, PCSK9, TMPRSS6, ALAS1, AAT, miR-122, hepatitis B virus HbsAg, LDHA and CEBPA.

An embodiment is the oligonucleotide conjugate according to the invention and for example according to any one of the molecules (EE), (PP) and (SS), comprising an oligonucleotide, wherein the oligonucleotide is an oligonucleotide capable of, for example when present inside a mammalian cell and preferably when present inside a human cell, targeting an mRNA involved in expression of any one of proteins: apoB and HSP27.

An aspect of the invention relates to a second pharmaceutical composition comprising the oligonucleotide conjugate of the invention and for example according to any one of the molecules (EE), (PP) and (SS), and optionally a pharmaceutically acceptable excipient and/or optionally a pharmaceutically acceptable diluent.

An aspect of the invention relates to the second pharmaceutical composition of the invention or to the oligonucleotide conjugate of the invention and for example according to any one of the molecules (EE), (PP) and (SS), for use as a medicament.

An aspect of the invention relates to the second pharmaceutical composition of the invention or to the oligonucleotide conjugate of the invention and for example according to any one of the molecules (EE), (PP) and (SS), for use in the treatment or prophylaxis of a disease or health problem in which an expression product is involved of any one or more of genes: *HSP27, apoB, TTR, PCSK9, TMPRSS6, ALAS1, AT3, GO, CC5*, *X* gene of HBV, S gene of HBV, AAT, miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3, GO, CC5, X* gene of HBV, *S* gene of HBV, *AAT,* miR-122, hepatitis B virus HbsAg, *LDHA, CEBPA* and *LDH.*

An aspect of the invention relates to the second pharmaceutical composition of the invention or to the oligonucleotide conjugate of the invention and for example according to any one of the molecules (EE), (PP) and (SS), for use in the treatment or prophylaxis of a disease or health problem in which an expression product is involved of any one or more of genes: *HSP27, apoB, TTR, PCSK9, TMPRSS6, ALAS1, AAT,* miR-122, hepatitis B virus HbsAg, *LDHA* and *CEBPA,* and/or for use in the treatment or prophylaxis of a disease or health problem which involves any one or more of genes: *HSP27, apoB, TTR, PCSK9, TMPRSS6, ALAS1, AAT,* miR-122, hepatitis B virus HbsAg, *LDHA* and *CEBPA.*

An embodiment is the second pharmaceutical composition of the invention or the oligonucleotide conjugate of the invention and for example according to any one of the molecules (EE), (PP) and (SS), for use as here above outlined, wherein said use is in the treatment or prophylaxis of a disease or health problem in which an expression product is involved of any one or more of genes: *HSP27* and *apoB,* preferably *apoB,* and/or for use in the treatment or prophylaxis of a disease or health problem which involves any one or more of genes: *HSP27* and *apoB,* preferably *apoB.*

An embodiment is the second pharmaceutical composition of the invention or the oligonucleotide conjugate of the invention and for example according to any one of the molecules (EE), (PP) and (SS), for use as here above outlined, for use in the treatment or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, $\alpha$1-antitrypsin deficiency, $\beta$-thalassaemia, or an auto-immune disease.

An embodiment is the second pharmaceutical composition of the invention or the oligonucleotide conjugate of the invention and for example according to any one of the molecules (EE), (PP) and (SS), for use as here above outlined, wherein said use is in the treatment or prophylaxis of a cancer such as endometrial carcinoma, breast cancer, lung cancer or hepatocellular carcinoma, and/or a cardiovascular disease such as hypercholesterolemia, preferably hypercholesterolemia.

An aspect of the invention relates to the second pharmaceutical composition of the invention or to the oligonucleotide conjugate of the invention and for example according to any one of the molecules (EE), (PP) and (SS), for use in the lowering of LDL-cholesterol in a subject. Preferably, the subject is a human subject.

It is preferred that the saponin of the oligonucleotide conjugate of the invention and for example according to any one of the molecules (EE), (PP) and (SS), is a saponin derived from a plant. The saponin comprised by the oligonucleotide conjugate of the invention is preferably a saponin isolated from a plant. For example, the saponin is isolated from the root of

a plant. Examples of such plants from which the saponin is derived (isolated) are *Quillaja saponaria, Gypsophila paniculate* L., *Saponaria officinalis* such as *Saponaria officinalis* L. and *Gypsophila elegans* such as *Gypsophila elegans* M. Bieb. Preferably, the oligonucleotide conjugate comprises a single type of saponin, preferably a single type of saponin derived from plant material such as the roots of a plant, such as SO1861, SO1862 or SO1832 from *Saponaria officinalis* (e.g., *Saponaria officinalis* L., preferably *Saponaria officinalis* L.) (roots) or such as a QS-21, a QS-7 or a QS-17 from *Quillaja saponaria* (roots). Suitable sources for isolating saponins according to the invention, i.e. those that display endosomal escape enhancing activity, are *Quillaja saponaria, Gypsophila paniculate* L., *Saponaria officinalis* and *Gypsophila elegans,* and Quillaja bark.

An aspect of the invention relates to an *in vitro* or ex *vivo* method for transferring the oligonucleotide conjugate of any one of the here above outlined embodiments of the invention from outside a cell to inside said cell, preferably subsequently transferring the oligonucleotide comprised by said oligonucleotide conjugate into the cytosol of said cell, comprising the steps of:

a) providing a cell which expresses ASGPR on its surface, the cell preferably selected from a liver cell, a virally infected cell and a tumor cell, and providing the oligonucleotide conjugate of the invention for transferring into the provided cell;
b) contacting the cell of step a) *in vitro* or ex *vivo* with the oligonucleotide conjugate of step a), therewith effecting the transfer of the oligonucleotide conjugate from outside the cell into said cell, and preferably therewith subsequently effecting the transfer of the oligonucleotide comprised by the oligonucleotide conjugate into the cytosol of said cell.

While the invention has been described in terms of several embodiments, it is contemplated that alternatives, modifications, permutations and equivalents thereof will become apparent to one having ordinary skill in the art upon reading the specification and upon study of the drawings. The invention is not limited in any way to the illustrated embodiments. Changes can be made without departing from the scope which is defined by the appended claims.

EMBODIMENTS of the invention

1. Saponin conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of $(GalNAc)_3$Tris, wherein the at least one saponin is selected from monodesmosidic triterpenoid saponins and bidesmosidic triterpenoid saponins.

2. Saponin conjugate of embodiment 1, wherein the saponin comprises an aglycone core structure selected from the group consisting of:

2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
quillaic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en;
gypsogenic acid,
and
derivatives thereof,

preferably the saponin comprises an aglycone core structure selected from quillaic acid and gypsogenin or derivatives thereof, more preferably the saponin aglycone core structure is quillaic acid or a derivative thereof.

3. Saponin conjugate of embodiment 1 or 2, wherein

- the saponin comprises a saccharide chain bound to the aglycone core structure, which is selected from group A:

GlcA-,
Glc-,
Gal-,
Rha-(1→2)-Ara-,
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof,

or

- the saponin comprises a saccharide chain bound to the aglycone core structure, which is selected from group B:

Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1→4)-Rha-(1→2)-[R1-(→4)]-Fuc- wherein R1 is 4E-Methoxycinnamic acid,
Xyl-(1→4)-Rha-(1→2)-[R2-(→4)]-Fuc- wherein R2 is 4Z-Methoxycinnamic acid,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- wherein R3 is 4E-Methoxycinnamic acid,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- or Xyl-)(1→3)-(Ara- or Xyl-)(1→4)-(Rha- or Fuc-)(1→2)-[4-OAc-(Rha- or Fuc-)(1→4)]-(Rha- or Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,

Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1→4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-40Ac-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(→4)]-Fuc- wherein R6 is 5-O-[5-O-Rha-(1→ 2)-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
Glc-(1→3)-[Glc-(1→6)]-Gal-, and
derivatives thereof,

or

- the saponin is a bidesmosidic triterpene glycoside comprising a first saccharide chain selected from the group A bound to the aglycone core structure and comprising a second saccharide chain selected from the group B bound to the aglycone core structure.

4. Saponin conjugate of any one of the embodiments 1-3, wherein the saponin is selected from the group consisting of: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin Ia, Teaseed saponin I, Teaseedsaponin J, Assamsaponin F, Digitonin, Primula acid 1 and AS64R, stereoisomers thereof, derivatives thereof, and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

5. Saponin conjugate of embodiment 3 or 4, wherein the saponin is a saponin derivative wherein

i. the saponin derivative comprises an aglycone core structure comprising an aldehyde group which has been derivatised;
ii. the saponin derivative comprises a saccharide chain, preferably a saccharide chain selected from group A as defined in embodiment 3, the saccharide chain comprising a carboxyl group which has been derivatised;
iii. the saponin derivative comprises a saccharide chain, preferably a saccharide chain selected from group B as defined in embodiment 3, the saccharide chain comprising an acetoxy (Me(CO)O-) group which has been derivatised; or
iv. the saponin derivative comprises any combination of derivatisations i., ii. and iii., preferably any combination of two derivatisations of derivatisations i., ii. and iii.

6. Saponin conjugate of any one of the embodiments 1-5, wherein the saponin is any one or more of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

7. Saponin conjugate of any one of the embodiments 1-6, wherein the saponin is a saponin derivative of the quillaic acid saponin or the gypsogenin saponin of embodiment 2 and is represented by Molecule 1:

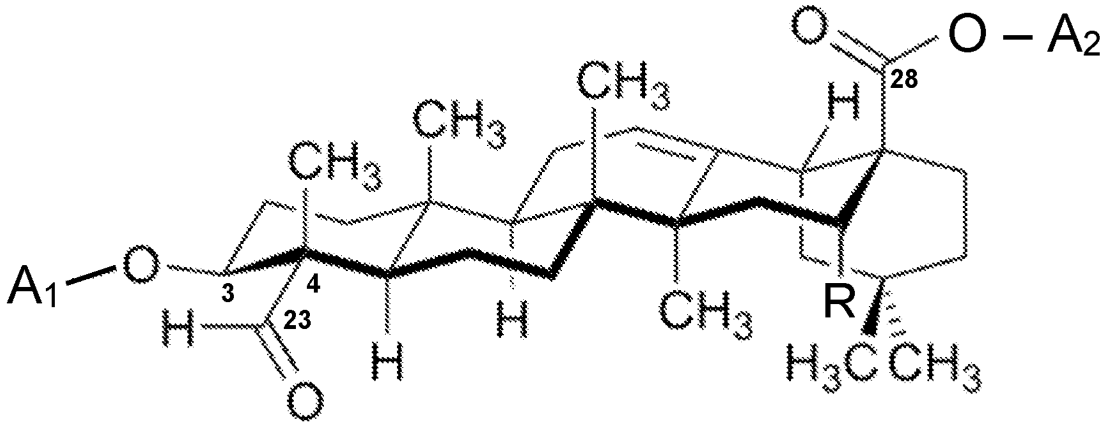

(Molecule 1)

wherein

$A_1$ represents hydrogen, a monosaccharide or a linear or branched oligosaccharide, preferably $A_1$ represents a saccharide chain selected from group A as defined in embodiment 3, more preferably $A_1$ represents a saccharide chain selected from group A as defined in embodiment 3 and $A_1$ comprises or consists of a glucuronic acid moiety; $A_2$ represents hydrogen, a monosaccharide or a linear or branched oligosaccharide, preferably $A_2$ represents a saccharide chain selected from group B as defined in embodiment 3, more preferably $A_2$ represents a saccharide chain selected from group B as defined in embodiment 3 and $A_2$ comprises at least one acetoxy (Me(CO)O-) group, such as one, two, three or four acetoxy groups,

wherein at least one of $A_1$ and $A_2$ is not hydrogen, preferably both $A_1$ and $A_2$ are an oligosaccharide chain; and R is hydrogen in gypsogenin or hydroxyl in quillaic acid;
wherein the saponin derivative corresponds to the saponin represented by Molecule 1 wherein at least one, preferably one or two, more preferably one, of the following derivatisations is present:

i. the aldehyde group at position $C_{23}$ of the quillaic acid or gypsogenin has been derivatised;
ii. the carboxyl group of a glucuronic acid moiety of $A_1$, when $A_1$ represents a saccharide chain selected from group A as defined in embodiment 3 and $A_1$ comprises or consists of a glucuronic acid moiety, has been derivatised; and
iii. one or more, preferably all, of acetoxy group(s) of one saccharide moiety or of two or more saccharide moieties of $A_2$, when $A_2$ represents a saccharide chain selected from group B as defined in embodiment 3 and $A_2$ comprises at least one acetoxy group, has/have been derivatised.

8. Saponin conjugate of embodiment 7, wherein $A_1$ represents a saccharide chain selected from group A as defined in embodiment 3 and comprises or consists of a glucuronic acid moiety and wherein the carboxyl group of a glucuronic acid moiety of $A_1$ has been derivatised and/or wherein $A_2$ represents a saccharide chain selected from group B as defined in embodiment 3 and $A_2$ comprises at least one acetoxy group and wherein at least one acetoxy group of $A_2$

has been derivatised.

9. Saponin conjugate of embodiment 7 or 8, wherein the saponin represented by Molecule 1 is a bidesmosidic triterpene saponin.

10. Saponin conjugate of any one of the embodiments 7-9, wherein the saponin derivative corresponds to the saponin represented by Molecule 1 wherein at least one, preferably one or two, more preferably one, of the following derivatisations is present:

i. the aldehyde group at position $C_{23}$ of the quillaic acid or gypsogenin has been derivatised by;

- reduction to an alcohol;
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
- transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;

ii. the carboxyl group of a glucuronic acid moiety of $A_1$, when $A_1$ represents a saccharide chain selected from group A as defined in embodiment 3 and $A_1$ comprises or consists of a glucuronic acid moiety, has been derivatised by transformation into an amide bond through reaction with 2-amino-2-methyl-1,3-propanediol (AMPD) or *N*-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of $A_2$, when $A_2$ represents a saccharide chain selected from group B as defined in embodiment 3 and $A_2$ comprises at least one acetoxy group, has/have been derivatised by transformation into a hydroxyl group (HO-) by deacetylation.

11. Saponin conjugate of any one of the embodiments 7-10, wherein $A_1$ is Gal-(1→2)-[Xyl-(1→3)]-GlcA and/or $A_2$ is Glc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAc-beta-D-quinovopyranosyl-(1→4)]-beta-D-fucopyranoside, more preferably the saponin is any one or more of: SO1861, GE1741, SA1641 and QS-21, or a derivative thereof, most preferably SO1861 or a derivative thereof.

12. Saponin conjugate of any one of the embodiments 5-11, wherein the saponin is a saponin derivative wherein

i. the saponin derivative comprises an aglycone core structure comprising an aldehyde group which has been derivatised by:

- reduction to an alcohol;
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
- transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;

ii. the saponin derivative comprises a saccharide chain, preferably a saccharide chain selected from group A as defined in embodiment 3, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu- AMPD or a SO1861-Glu-AMPD or a saponin-Glu-AEM such as a QS-21-Glu-AEM or a SO1861-Glu-AEM;
iii. the saponin derivative comprises a saccharide chain, preferably a saccharide chain selected from group B as defined in embodiment 3, the saccharide chain comprising an acetoxy (Me(CO)O-) group which has been derivatised by transformation into a hydroxyl group (HO-) by deacetylation; or
iv. the saponin derivative comprises any combination of derivatisations i., ii. and iii., preferably any combination of two derivatisations of derivatisations i., ii. and iii.;

preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thio-ether bond with mercaptoethanol.

13. Saponin conjugate of embodiment 12, wherein the saponin is a saponin derivative wherein

i. the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH;
ii. the saponin derivative comprises a saccharide chain, preferably a saccharide chain selected from group A as defined in embodiment 3, the saccharide chain comprising a carboxyl group, preferably a carboxyl group of a glucuronic acid moiety which has been derivatised by transformation into an amide bond through reaction with N-(2-aminoethyl)maleimide (AEM), therewith providing a saponin-Glu-AEM such as a QS-21-Glu-AEM or a SO1861-Glu-AEM; or
iii. the saponin derivative comprises a combination of derivatisations i. and ii.

14. Saponin conjugate of embodiment 12, wherein the saponin derivative comprises an aglycone core structure wherein the aglycone core structure comprises an aldehyde group and wherein the saponin derivative comprises a saccharide chain, preferably a saccharide chain selected from group A as defined in embodiment 3, the saccharide chain comprising a carboxyl group, preferably a carboxyl group of a glucuronic acid moiety, which glucuronic acid moiety has been derivatised by transformation into an amide bond through reaction with N-(2-aminoethyl)maleimide (AEM).

15. Saponin conjugate of any one of the embodiments 1-12, wherein the saponin is a saponin derivative represented by Molecule 2:

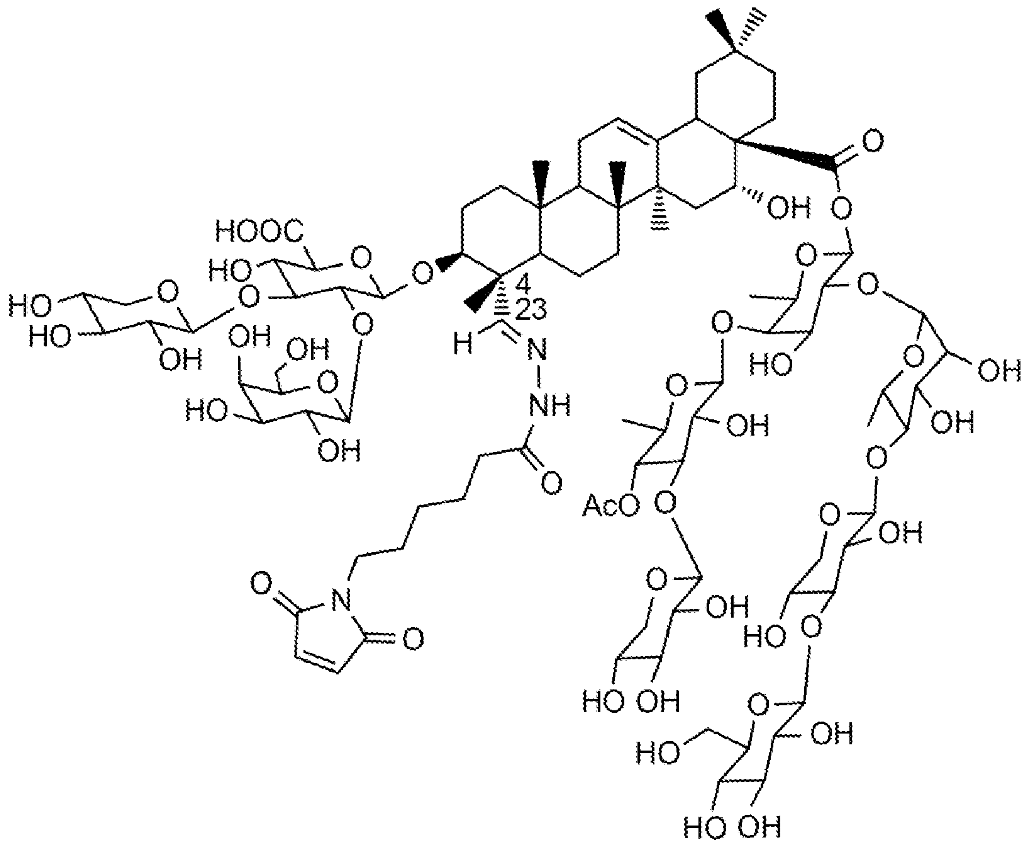

(Molecule 2)

or wherein the saponin is a saponin derivative represented by Molecule 3:

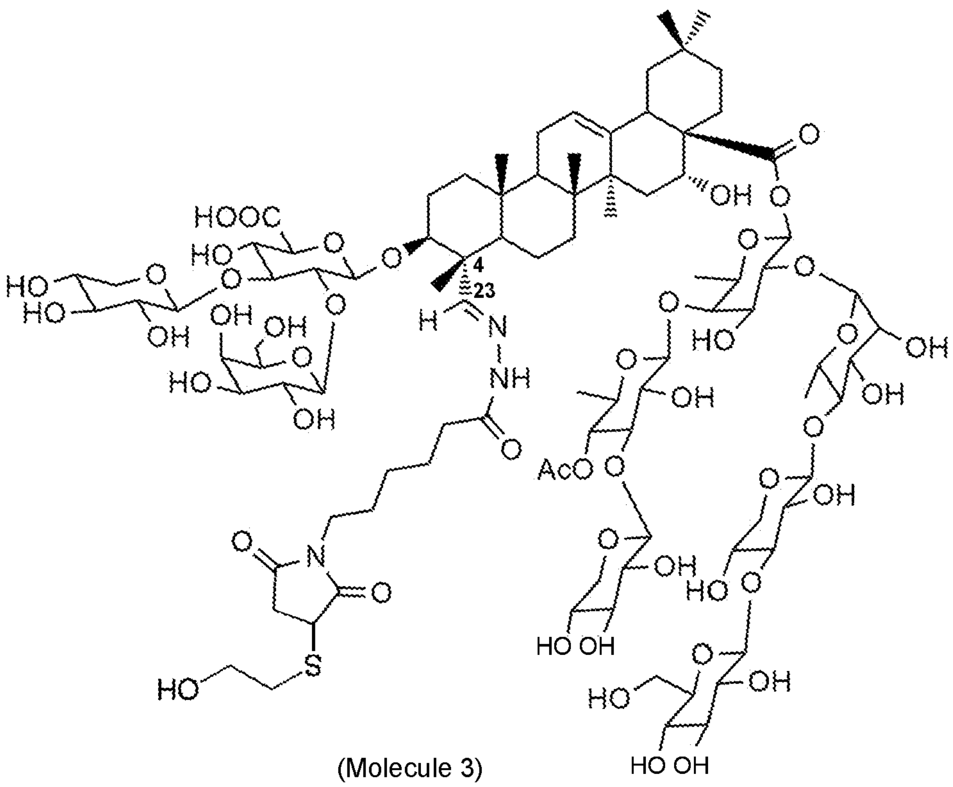

(Molecule 3)

16. Saponin conjugate of any one of the embodiments 1-15, wherein the at least one saponin and the ligand for ASGPR are covalently linked directly or via at least one linker.

17. Saponin conjugate of any one of the embodiments 1-16, wherein the GalNAc moiety is bound to the saponin S, preferably via a saponin linker $L_S$, as shown in formula $(II)_S$:

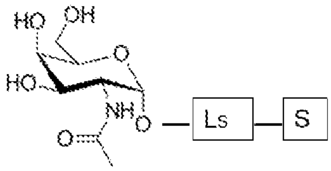

$(II)_S$.

18. Saponin conjugate of any one of the embodiments 1-16, wherein the GalNAc moieties are each separately covalently bound via the oxygen on position "1" of the GalNAc moiety to a central bridging moiety B, which effectively forms a bridge between the GalNAc moieties and the saponin moiety, preferably via a saponin moiety linker $L_S$, as shown in formula $(III)_S$:

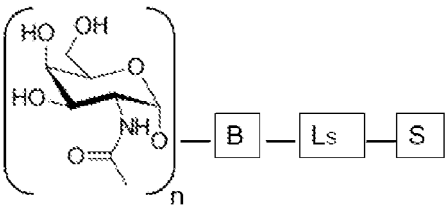

$(III)_S$

wherein n is an integer larger than or equal to 2, preferably n is 3, $L_S$ is a saponin moiety linker and S is the saponin moiety.

19. Saponin conjugate of embodiment 18, wherein the GalNAc moieties are bound to the bridging moiety B via GalNAc linkers $L_{GAL}$ as shown in formula $(IV)_S$:

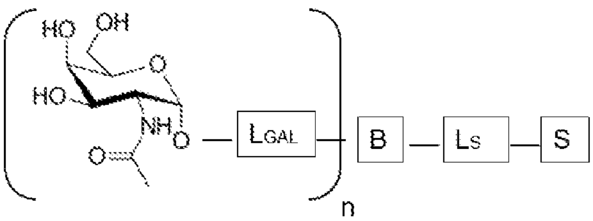

$(IV)_S$

wherein n is an integer larger than or equal to 2, preferably n is 3, $L_S$ is a saponin moiety linker and S is the saponin moiety.

20. Saponin conjugate of any one of embodiments 17-19, wherein the saponin moiety linker Ls represents any chemical moiety suitable for covalently binding a saponin to GalNAc as in formula $(II)_S$ or to the bridging moiety B as in formula $(III)_S$ and $(IV)_S$.

21. Saponin conjugate of any one of embodiments 17-20, wherein the saponin moiety linker $L_S$ is the result of a coupling reaction between at least a first precursor $L_{S1}$ covalently bound to GalNAc or the bridging moiety B and a second precursor $L_{S2}$ covalently bound to the saponin moiety, wherein $L_{S1}$ is a precursor of the saponin moiety linker $L_S$ which is covalently bound to GalNAc or to the bridging moiety B and $L_{S2}$ is a precursor of the saponin moiety linker $L_S$ which is covalently bound to the saponin moiety.

22. Saponin conjugate of any one of embodiments 17-21, wherein the saponin moiety linker $L_S$ is the result of a coupling reaction between at least a first precursor $L_{S1}$ covalently bound to GalNAc or the bridging moiety B and a second precursor $L_{S2}$ covalently bound to the saponin moiety, wherein the coupling reaction is selected from the group consisting of an azide-alkyne cycloaddition, a thiol maleimide coupling, a Staudinger reaction, a nucleophilic ring-opening of strained heterocyclic electrophiles, a carbonyl reaction of the non-aldol type and an addition to a carbon-carbon double bond, preferably wherein the coupling reaction is an azide-alkyne cycloaddition, a thiol maleimide coupling, a Staudinger reaction, a nucleophilic ring-opening of strained heterocyclic electrophiles, more preferably wherein the coupling reaction is an azide-alkyne cycloaddition or a thiol maleimide coupling.

23. Saponin conjugate of any one of embodiments 17-22, wherein the saponin moiety linker $L_S$ is the result of a coupling reaction between a first precursor $L_{S1}$ covalently bound to GalNAc or the bridging moiety B, the first precursor $L_{S1}$ comprising an azide; and a second precursor $L_{S2}$ covalently bound to the saponin moiety, the second precursor $L_{S2}$ comprising an alkyne and preferably comprising a hydrazone resulting from a hydrazide/aldehyde coupling to an aldehyde of the saponin moiety.

24. Saponin conjugate of any one of embodiments 21-23, wherein the structure for the precursor $L_{S1}$ is the following azide of formula (XVII):

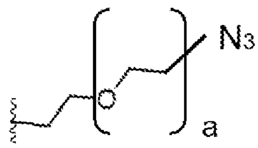

(XVII)

wherein a represents an integer larger than or equal to 0, preferably a represents an integer selected from 1, 2, and 3, more preferably a represents 2, or
wherein the structure for the precursor $L_{S1}$ comprises the following azide of formula (XVIII):

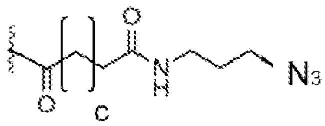

(XVIII)

wherein c represents an integer larger than or equal to 0, preferably c represents an integer in the range of 5-15, more preferably c represents 9.

25. Saponin conjugate of any one of embodiments 21-24, wherein the structure for the precursor $L_{S2}$ comprises the following hydrazone with formula (XIX):

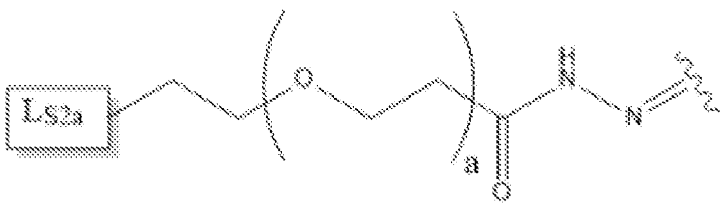

(XIX)

wherein a represents an integer larger than or equal to 0, preferably a represents an integer in the range of 2-6, more preferably a represents 4, and wherein $L_{S2a}$ represents an alkyne-containing moiety.

26. Saponin conjugate of embodiment 25, wherein $L_{S2a}$ comprises less than 20 carbon atoms, preferably $L_{S2a}$ represents a moiety according to formula (XX):

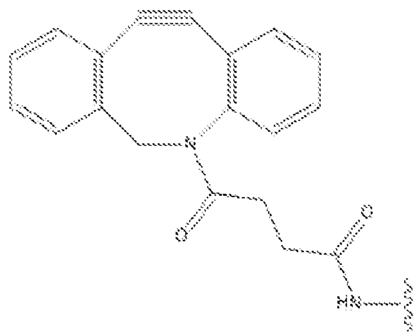

(XX).

27. Saponin conjugate of any one of embodiments 18-26, wherein the bridging moiety is a compound of formula (XV):

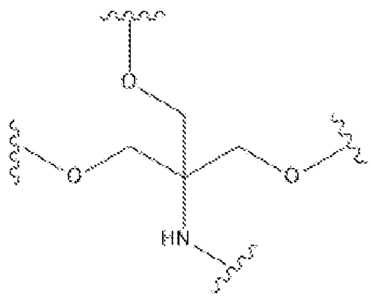

(XV)

wherein the oxygen atoms of the compound of formula (XV) are bound to GalNAc or to the GalNAc linkers $L_{GAL}$, and the nitrogen atom of the compound of formula (XV) is bound to the saponin moiety linker $L_S$.

28. Saponin conjugate of any one of embodiments 19-27, wherein $L_{GAL}$ represents any chemical moiety suitable for covalently binding GalNAc to the bridging moiety B.

29. Saponin conjugate of any one of embodiments 19-28, wherein $L_{GAL}$ comprises 2-25 carbon atoms, preferably 7-15 carbon atoms, more preferably 11 carbon atoms and wherein $L_{GAL}$ comprises at least one, preferably two amide moieties.

30. Saponin conjugate of any one of embodiments 19-29, wherein $L_{GAL}$ is a compound according to formula (XVI):

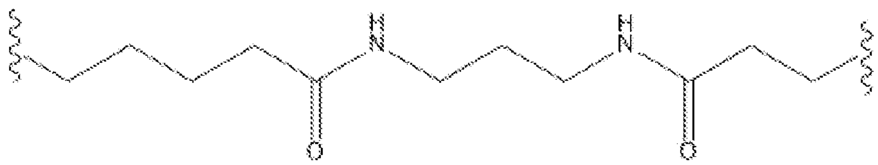

(XVI)

31. Saponin conjugate of any one of the embodiments 17-30, wherein the ligand for ASGPR is the $(GalNAc)_3$Tris represented by Molecule (DD3) or Molecule (DD4):

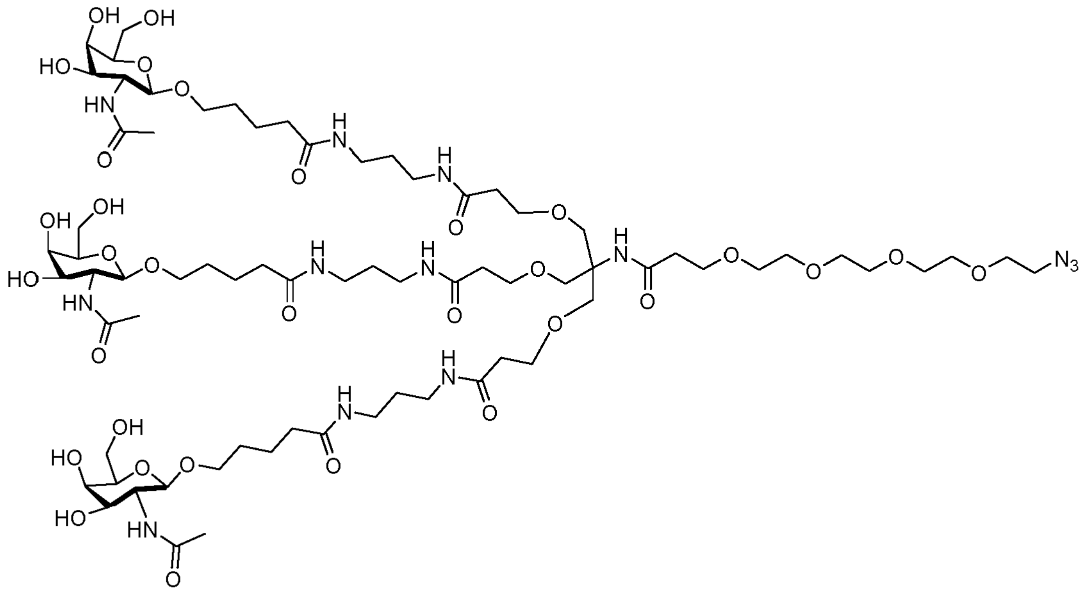

(DD3),

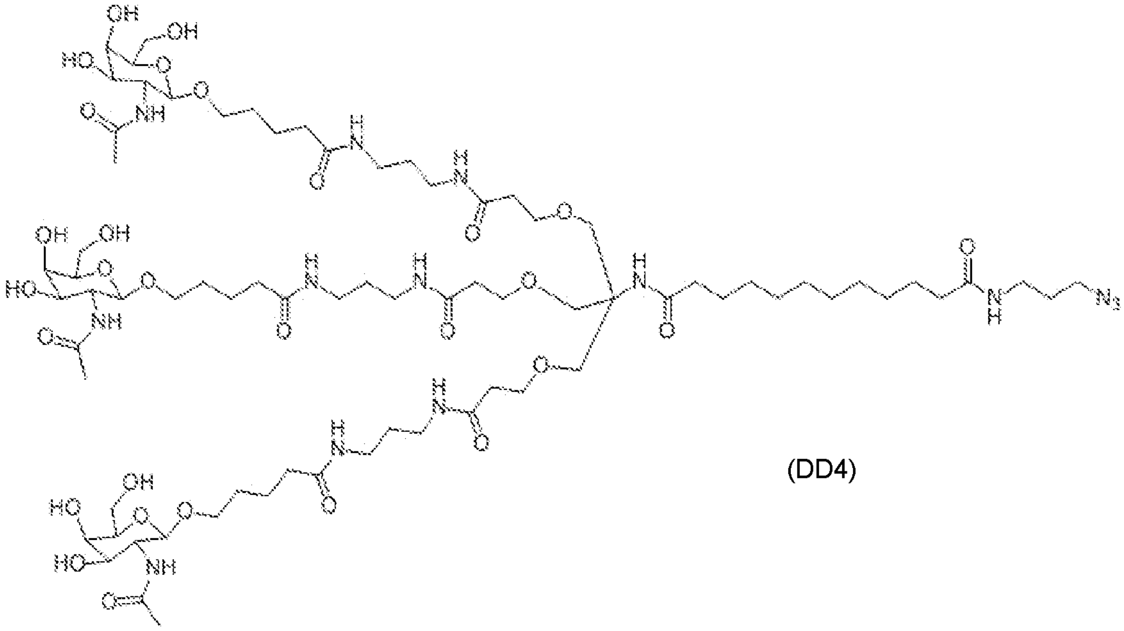

(DD4)

, or wherein the ligand for ASGPR is mono-GalNAc represented by Molecule II':

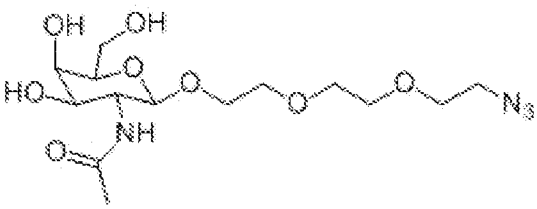

(Molecule II')

32. Saponin conjugate of any one of the embodiments 1-31, wherein the at least one saponin is covalently bound to Molecule (DD3) or Molecule (DD4) or Molecule II' of embodiment 31 via a linker represented by Molecule III':

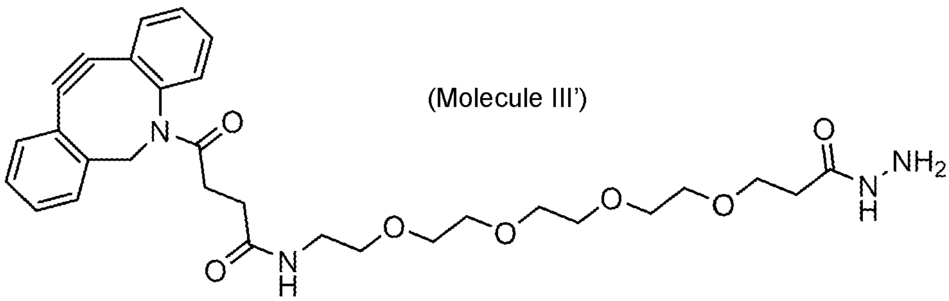

(Molecule III')

wherein the hydrazide moiety of Molecule III' formed a covalent hydrazone bond with an aldehyde group in the saponin, and wherein the dibenzocyclooctyne group formed a covalent bond with the azide group of Molecule (DD3) or Molecule (DD4) or Molecule II'.

33. Saponin conjugate of any one of the embodiments 1-32, wherein the at least one saponin is covalently bound to the ligand for ASGPR via at least one cleavable linker.

34. Saponin conjugate of embodiment 33, wherein the cleavable linker is subject to cleavage under acidic conditions, reductive conditions, enzymatic conditions and/or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulfide bond.

35. Saponin conjugate of embodiment 33 or 34, wherein the cleavable linker is subject to cleavage *in vivo* under acidic conditions such as for example present in endosomes and/or lysosomes of mammalian cells, preferably human cells, preferably the cleavable linker is subject to cleavage *in vivo* at pH 4.0 - 6.5, and more preferably at pH $\leq$ 5.5.

36. Saponin conjugate of any one of the embodiments 1-35, wherein the conjugate comprises 1, 2, 3, 4, 5, 6, 8, 10, 16, 32, 64, 128 or 1-100 saponin moieties, or any number of saponin moieties therein between, such as 7, 9, 12 saponin moieties.

37. Pharmaceutical combination comprising:

- a first pharmaceutical composition comprising the saponin conjugate of any one of the embodiments 1-36 and optionally comprising a pharmaceutically acceptable excipient and/or a pharmaceutically acceptable diluent; and
- a second pharmaceutical composition comprising a second conjugate of an effector molecule and a ligand for ASGPR, wherein the ligand for ASGPR preferably comprises at least one GalNAc moiety, preferably three or four GalNAc moieties, more preferably the ligand for ASGPR comprises or consists of $(GalNAc)_3$Tris, or a third conjugate of an effector molecule and a binding molecule comprising a binding site for a cell-surface molecule, and optionally comprising a pharmaceutically acceptable excipient and/or pharmaceutically acceptable diluent.

38. Pharmaceutical composition comprising:

- the saponin conjugate of any one of the embodiments 1-36;
- a second conjugate of an effector molecule and a ligand for ASGPR wherein the ligand for ASGPR preferably comprises at least one GalNAc moiety, preferably three or four GalNAc moieties, more preferably the ligand for ASGPR comprises or consists of $(GalNAc)_3$Tris, or a third conjugate of an effector molecule and a binding molecule comprising a binding site for a cell-surface molecule, and optionally comprising a pharmaceutically acceptable excipient and/or pharmaceutically acceptable diluent.

39. Pharmaceutical combination of embodiment 37 or pharmaceutical composition of embodiment 38, wherein the effector molecule comprises or consists of at least one of a small molecule such as a drug molecule, a toxin such as a protein toxin, an oligonucleotide such as an AON such as a BNA, a xeno nucleic acid or an siRNA, an enzyme, a peptide, a protein, or any combination thereof, preferably, the effector molecule is a toxin, an enzyme or an oligonucleotide.

40. Pharmaceutical combination of embodiment 37 or 39, or pharmaceutical composition of embodiment 38 or 39,

wherein the ligand for ASGPR comprises or is $(GalNAc)_3$Tris, and/or wherein the ligand for ASGPR and the effector molecule are conjugated via a covalent bond, preferably via at least one linker.

41. Pharmaceutical combination of any one of the embodiments 37 or 39-40 or pharmaceutical composition of any one of the embodiments 38-40, wherein the effector molecule is an oligonucleotide selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic acid ($BNA^{NC}$), BNA-based siRNA, and BNA-based antisense oligonucleotide (BNA-AON).

42. Pharmaceutical combination of any one of the embodiments 37 or 39-41 or pharmaceutical composition of any one of the embodiments 38-41, wherein the effector molecule is an oligonucleotide selected from any one or more of a(n): anti-miRNA, a BNA-AON or an siRNA, such as BNA-based siRNA, selected from chemically modified siRNA, metabolically stable siRNA and chemically modified, metabolically stable siRNA.

43. Pharmaceutical combination of any one of the embodiments 37 or 39-42 or pharmaceutical composition of any one of the embodiments 38-42, wherein the effector molecule is an oligonucleotide capable of, for example when present inside a mammalian cell, silencing any one of genes: apolipoprotein B (*apoB*), *HSP27,* transthyretin (*TTR*), proprotein convertase subtilisin/kexin type 9 (*PCSK9*), delta-aminolevulinate synthase 1 (*ALAS1*), antithrombin 3 (*AT3*), glycolate oxidase (*GO*), complement component C5 (*CC5*), *X* gene of hepatitis B virus (HBV), *S* gene of HBV, alpha-1 antitrypsin (*AAT*) and lactate dehydrogenase (*LDH*), and/or is an oligonucleotide capable of, for example when present inside a mammalian cell, targeting an aberrant miRNA.

44. Pharmaceutical combination of any one of the embodiments 37 or 39-43 or pharmaceutical composition of any one of the embodiments 38-43, wherein the effector molecule is an oligonucleotide capable of, for example when present inside a mammalian cell, targeting an mRNA involved in expression of any one of proteins: apoB, HSP27, TTR, PCSK9, ALAS1, AT3, GO, CC5, expression product of X gene of HBV, expression product of S gene of HBV, AAT and LDH, or is capable of, for example when present inside a mammalian cell, antagonizing or restoring an miRNA function such as inhibiting an oncogenic miRNA (onco-miR) or suppression of expression of an onco-miR.

45. Pharmaceutical combination of any one of the embodiments 37 or 39-44 or pharmaceutical composition of any one of the embodiments 38-44, wherein the effector molecule is or comprises a toxin which toxin comprises or consists of at least one molecule selected from any one or more of a peptide, a protein, an enzyme such as urease and Cre-recombinase, a proteinaceous toxin, a ribosome-inactivating protein, and/or a bacterial toxin, a plant toxin, more preferably selected from any one or more of a viral toxin such as apoptin; a bacterial toxin such as Shiga toxin, Shiga-like toxin, *Pseudomonas aeruginosa* exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin *e.g.* dianthin-30 or dianthin-32, saporin *e.g.* saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin, and/or comprises or consists of at least one of a toxin targeting ribosome, a toxin targeting elongation factor, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-$\gamma$-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, $\alpha$-amanitin, a spliceostatin, a thailanstatin, ozogamicin, tesirine, Amberstatin269 and soravtansine, or a derivative thereof.

46. Pharmaceutical combination of any one of the embodiments 37 or 39-45 or pharmaceutical composition of any one of the embodiments 38-45, wherein the binding molecule comprising a binding site for a cell-surface molecule, comprised by the third conjugate, is a ligand for a cell-surface molecule or an antibody comprising a binding site for a cell-surface molecule or at least one domain or fragment thereof comprising the binding site.

47. Pharmaceutical combination of any one of the embodiments 37 or 39-46 or pharmaceutical composition of any one

of the embodiments 38-46, wherein the third conjugate is any one or more of: an antibody-toxin conjugate, a receptor-ligand - toxin conjugate, an antibody-drug conjugate, a receptor-ligand - drug conjugate, an antibody-nucleic acid conjugate or a receptor-ligand - nucleic acid conjugate.

48. Pharmaceutical combination of any one of the embodiments 37 or 39-47 or pharmaceutical composition of any one of the embodiments 38-47, wherein the binding molecule comprising a binding site for a cell-surface molecule, comprised by the third conjugate, is capable of binding to any one of cell-surface molecules: CD71, CD63, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, asialoglycoprotein receptor (ASGPR), preferably any one of: HER2, CD71, ASGPR and EGFR, more preferably CD71.

49. Pharmaceutical combination of any one of the embodiments 37 or 39-48 or pharmaceutical composition of any one of the embodiments 38-48, wherein the binding molecule comprising a binding site for a cell-surface molecule, comprised by the third conjugate, is or comprises any one of: an antibody, preferably a monoclonal antibody such as a human monoclonal antibody, an IgG, a molecule comprising or consisting of a single-domain antibody, at least one $V_{HH}$ domain, preferable a camelid $V_H$, a variable heavy chain new antigen receptor ($V_{NAR}$) domain, a Fab, an scFv, an Fv, a dAb, an F(ab)2 and a Fcab fragment.

50. Pharmaceutical combination of any one of the embodiments 37, 39-49 or pharmaceutical composition of any one of the embodiments 38-49, for use as a medicament.

51. Pharmaceutical combination of any one of the embodiments 37, 39-49 or pharmaceutical composition of any one of the embodiments 38-49, for use in the treatment or prophylaxis of a disease or health problem in which an expression product is involved of any one or more of genes: apoB, TTR, PCSK9, ALAS1, AT3, GO, CC5, X gene of HBV, S gene of HBV, AAT and LDH.

52. Pharmaceutical combination of any one of the embodiments 37, 39-49 or pharmaceutical composition of any one of the embodiments 38-49, or pharmaceutical combination or pharmaceutical composition for use of embodiment 51, for use in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, or an auto-immune disease.

53. Pharmaceutical combination for use of embodiment 51 or 52 or pharmaceutical composition for use of embodiment 51 or 52, wherein the saponin is a saponin derivative, preferably a QS-21 derivative or an SO1861 derivative of any one of the embodiments 4-15.

*54. In vitro* or ex *vivo* method for transferring the second conjugate or the third conjugate of any one of the embodiments 37-48 from outside a cell to inside said cell, preferably subsequently transferring the effector molecule comprised by the second conjugate or the third conjugate of any one of the embodiments 37-48 into the cytosol of said cell, comprising the steps of:

a) providing a cell which expresses ASGPR on its surface, and, when the third conjugate is to be transferred into the cell, which expresses the cell-surface molecule for which the third conjugate comprises a binding molecule for binding to said cell-surface molecule, the cell preferably selected from a liver cell, a virally infected cell and a tumor cell;
b) providing the second conjugate or the third conjugate of any one of the embodiments 37-48 for transferring into the cell provided in step a);
c) providing the saponin conjugate of any one of the embodiments 1-36;
d) contacting the cell of step a) *in vitro* or ex *vivo* with the second conjugate or the third conjugate of step b) and the saponin conjugate of step c), therewith effecting the transfer of the second conjugate or the third conjugate from outside the cell into said cell, and preferably therewith subsequently effecting the transfer of the second conjugate or the third conjugate into the cytosol of said cell, or preferably therewith subsequently effecting the transfer of at least the effector molecule comprised by the second conjugate or the third conjugate into the cytosol of said cell.

An embodiment is the oligonucleotide conjugate of the invention and/or the saponin conjugate of the invention, wherein the saponin comprised by the oligonucleotide conjugate or the saponin conjugate is isolated from a plant. Preferably, the saponin is isolated from a part of a plant, such as the root, or from a part of a tree, such as the bark. Preferably, the saponin is isolated from roots derived from a plant.

EMBODIMENTS of the invention

I. Oligonucleotide conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of $(GalNAc)_3$Tris, and further covalently linked to an oligonucleotide, wherein the at least one saponin is selected from monodesmosidic triterpenoid saponins and bidesmosidic triterpenoid saponins.

II. Oligonucleotide conjugate of embodiment I, wherein the saponin is a penta-cyclic triterpene saponin of the 12,13-dehydrooleanane type, preferably with an aldehyde function in position C-23 of the aglycone core structure of the saponin.

III. Oligonucleotide conjugate of embodiment I or II, wherein the saponin is a monodesmosidic or bidesmosidic penta-cyclic triterpene saponin of the 12,13-dehydrooleanane type, preferably with an aldehyde function in position C-23 of the aglycone core structure of the saponin.

IV. Oligonucleotide conjugate of any one of the embodiments I-III, wherein the saponin comprises an aglycone core structure selected from the group consisting of:

2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
quillaic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en;
gypsogenic acid,
and
derivatives thereof,

preferably the saponin comprises an aglycone core structure selected from quillaic acid and gypsogenin or derivatives thereof, more preferably the saponin aglycone core structure is quillaic acid or a derivative thereof.

V. Oligonucleotide conjugate of any one of the embodiments I-IV, wherein

- the saponin comprises a saccharide chain bound to the aglycone core structure, which is selected from group A:

GlcA-,
Glc-,
Gal-,
Rha-(1→2)-Ara-,
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,

Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof,

or

- the saponin comprises a saccharide chain bound to the aglycone core structure, which is selected from group B:

Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1→4)-Rha-(1→2)-[R1-(→4)]-Fuc- wherein R1 is 4E-Methoxycinnamic acid,
Xyl-(1→4)-Rha-(1→2)-[R2-(→4)]-Fuc- wherein R2 is 4Z-Methoxycinnamic acid,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- wherein R3 is 4E-Methoxycinnamic acid,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- or Xyl-)(1→3)-(Ara- or Xyl-)(1→4)-(Rha- or Fuc-)(1--->2)-[4-OAc-(Rha- or Fuc-)(1→4)]-(Rha- or Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1→4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,

Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
Glc-(1→3)-[Glc-(1→6)]-Gal-, and
derivatives thereof,

or

- the saponin is a bidesmosidic triterpene glycoside comprising a first saccharide chain selected from the group A bound to the aglycone core structure and comprising a second saccharide chain selected from the group B bound to the aglycone core structure.

VI. Oligonucleotide conjugate of any one of the embodiments I-V, wherein the saponin is selected from the group consisting of: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin Ia, Teaseed saponin I, Teaseedsaponin J, Assamsaponin F, Digitonin, Primula acid 1 and AS64R, stereoisomers thereof, derivatives thereof, and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative, SO1861 and combinations thereof, most preferably the saponin is a SO1861 derivative or SO1861.

VII. Oligonucleotide conjugate of embodiment V or VI, wherein the saponin is a saponin derivative wherein

i. the saponin derivative comprises an aglycone core structure comprising an aldehyde group which has been derivatised;
ii. the saponin derivative comprises a saccharide chain, preferably a saccharide chain selected from group A as defined in embodiment V, the saccharide chain comprising a carboxyl group which has been derivatised;
iii. the saponin derivative comprises a saccharide chain, preferably a saccharide chain selected from group B as defined in embodiment V, the saccharide chain comprising an acetoxy (Me(CO)O-) group which has been derivatised; or
iv. the saponin derivative comprises any combination of derivatisations i., ii. and iii., preferably any combination of two derivatisations of derivatisations i., ii. and iii.

VIII. Oligonucleotide conjugate of any one of the embodiments I-VII, wherein the saponin is any one or more of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative, SO1861 and combinations thereof, most preferably the saponin is a SO1861 derivative or SO1861.

IX. Oligonucleotide conjugate of any one of the embodiments I-VIII, wherein the saponin is a saponin derivative of the quillaic acid saponin or the gypsogenin saponin of embodiment IV and is represented by Molecule 1:

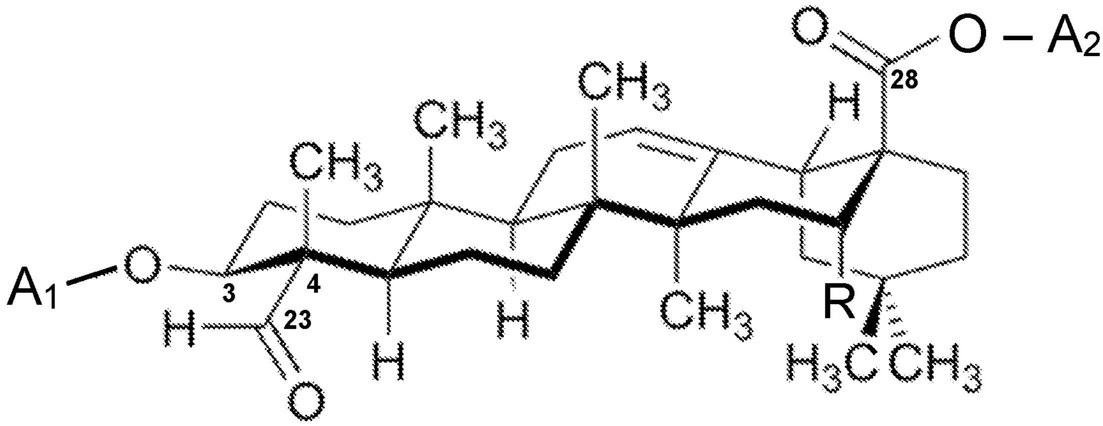

(Molecule 1)

wherein

$A_1$ represents hydrogen, a monosaccharide or a linear or branched oligosaccharide, preferably $A_1$ represents a saccharide chain selected from group A as defined in embodiment V, more preferably $A_1$ represents a saccharide chain selected from group A as defined in embodiment V and $A_1$ comprises or consists of a glucuronic acid moiety; $A_2$ represents hydrogen, a monosaccharide or a linear or branched oligosaccharide, preferably $A_2$ represents a saccharide chain selected from group B as defined in embodiment V, more preferably $A_2$ represents a saccharide chain selected from group B as defined in embodiment V and $A_2$ comprises at least one acetoxy (Me(CO)O-) group, such as one, two, three or four acetoxy groups,

wherein at least one of $A_1$ and $A_2$ is not hydrogen, preferably both $A_1$ and $A_2$ are an oligosaccharide chain; and R is hydrogen in gypsogenin or hydroxyl in quillaic acid;
wherein the saponin derivative corresponds to the saponin represented by Molecule 1 wherein at least one, preferably one or two, more preferably one, of the following derivatisations is present:

i. the aldehyde group at position $C_{23}$ of the quillaic acid or gypsogenin has been derivatised;
ii. the carboxyl group of a glucuronic acid moiety of $A_1$, when $A_1$ represents a saccharide chain selected from group A as defined in embodiment V and $A_1$ comprises or consists of a glucuronic acid moiety, has been derivatised; and
iii. one or more, preferably all, of acetoxy group(s) of one saccharide moiety or of two or more saccharide moieties of $A_2$, when $A_2$ represents a saccharide chain selected from group B as defined in embodiment V and $A_2$ comprises at least one acetoxy group, has/have been derivatised.

X. Oligonucleotide conjugate of embodiment IX, wherein $A_1$ represents a saccharide chain selected from group A as defined in embodiment V and comprises or consists of a glucuronic acid moiety and wherein the carboxyl group of a glucuronic acid moiety of $A_1$ has been derivatised and/or wherein $A_2$ represents a saccharide chain selected from group B as defined in embodiment V and $A_2$ comprises at least one acetoxy group and wherein at least one acetoxy group of $A_2$ has been derivatised.

XI. Oligonucleotide conjugate of embodiment IX or X, wherein the saponin represented by Molecule 1 is a bidesmosidic triterpene saponin.

XII. Oligonucleotide conjugate of any one of the embodiments IX-XI, wherein the saponin derivative corresponds to the saponin represented by Molecule 1 wherein at least one, preferably one or two, more preferably one, of the following derivatisations is present:

i. the aldehyde group at position $C_{23}$ of the quillaic acid or gypsogenin has been derivatised by;

- reduction to an alcohol;
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
- transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;

ii. the carboxyl group of a glucuronic acid moiety of $A_1$, when $A_1$ represents a saccharide chain selected from group A as defined in embodiment V and $A_1$ comprises or consists of a glucuronic acid moiety, has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of $A_2$, when $A_2$ represents a saccharide chain selected from group B as defined in embodiment V and $A_2$ comprises at least one acetoxy group, has/have been derivatised by transformation into a hydroxyl group (HO-) by deacetylation.

XIII. Oligonucleotide conjugate of any one of the embodiments IX-XII, wherein $A_1$ is Gal-(1→2)-[Xyl-(1→3)]-GlcA and/or $A_2$ is Glc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAc-beta-D-quinovopyranosyl-(1→4)]-beta-D-fucopyranoside, more preferably the saponin is any one or more of: SO1861, GE1741, SA1641 and QS-21, or a derivative thereof, most preferably SO1861 or a derivative thereof.

XIV. Oligonucleotide conjugate of any one of the embodiments VII-XIII, wherein the saponin is a saponin derivative wherein

i. the saponin derivative comprises an aglycone core structure comprising an aldehyde group which has been derivatised by:

- reduction to an alcohol;
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thio-ether bond with mercaptoethanol;
- transformation into a hydrazone bond through reaction with N-[β-maleimidopropionic acid] hydrazide (BMPH) wherein the maleimide group of the BMPH is optionally derivatised by formation of a thio-ether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thio-ether bond with mercaptoethanol;

ii. the saponin derivative comprises a saccharide chain, preferably a saccharide chain selected from group A as defined in embodiment V, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu- AMPD or a SO1861-Glu-AMPD or a saponin-Glu-AEM such as a QS-21-Glu-AEM or a SO1861-Glu-AEM;
iii. the saponin derivative comprises a saccharide chain, preferably a saccharide chain selected from group B as defined in embodiment V, the saccharide chain comprising an acetoxy (Me(CO)O-) group which has been derivatised by transformation into a hydroxyl group (HO-) by deacetylation; or

iv. the saponin derivative comprises any combination of derivatisations i., ii. and iii., preferably any combination of two derivatisations of derivatisations i., ii. and iii.;

preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thio-ether bond with mercaptoethanol.

XV. Oligonucleotide conjugate of embodiment XIV, wherein the saponin is a saponin derivative wherein

a) the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH;
b) the saponin derivative comprises a saccharide chain, preferably a saccharide chain selected from group A as defined in embodiment V, the saccharide chain comprising a carboxyl group, preferably a carboxyl group of a glucuronic acid moiety which has been derivatised by transformation into an amide bond through reaction with N-(2-aminoethyl)maleimide (AEM), therewith providing a saponin-Glu-AEM such as a QS-21-Glu-AEM or a SO1861-Glu-AEM; or
c) the saponin derivative comprises a combination of derivatisations i. and ii.

XVI. Oligonucleotide conjugate of embodiment XIV, wherein the saponin derivative comprises an aglycone core structure wherein the aglycone core structure comprises an aldehyde group and wherein the saponin derivative comprises a saccharide chain, preferably a saccharide chain selected from group A as defined in embodiment V, the saccharide chain comprising a carboxyl group, preferably a carboxyl group of a glucuronic acid moiety, which glucuronic acid moiety has been derivatised by transformation into an amide bond through reaction with N-(2-aminoethyl)maleimide (AEM).

XVII. Oligonucleotide conjugate of any one of the embodiments I-XIV, wherein the saponin is a saponin derivative represented by Molecule 2:

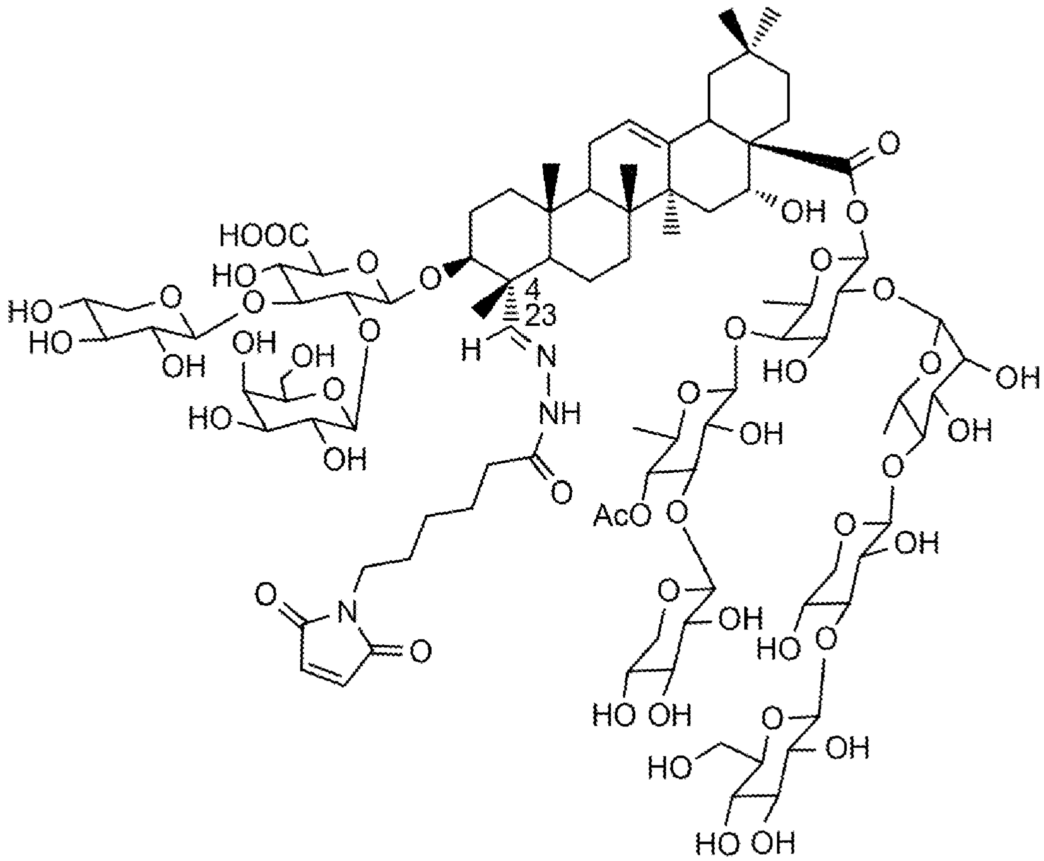

(Molecule 2)

or wherein the saponin is a saponin derivative represented by Molecule 3:

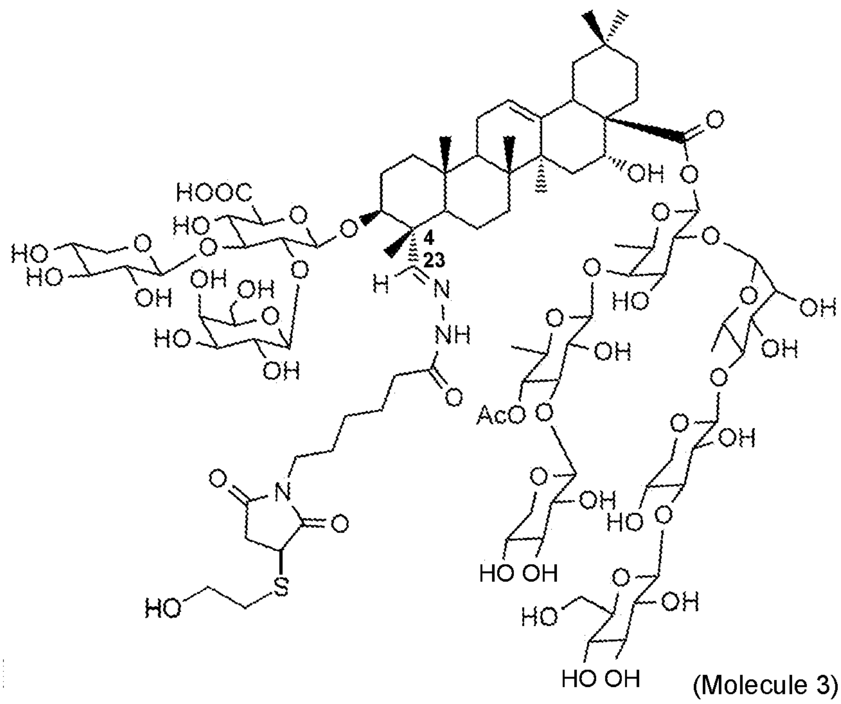

(Molecule 3)

XVIII. Oligonucleotide conjugate of any one of the embodiments I-XVII, wherein the at least one saponin and the ligand for ASGPR are covalently linked directly or via at least one linker, and/or wherein the at least one saponin and the

oligonucleotide are covalently linked directly or via at least one linker, and/or wherein the ligand for ASGPR and the oligonucleotide are covalently linked directly or via at least one linker, preferably, the at least one saponin, the ligand for ASGPR and the oligonucleotide are linked via at least one linker.

XIX. Oligonucleotide conjugate of any one of the embodiments I-XVIII, wherein the at least one GalNAc moiety, the at least one saponin and the oligonucleotide are covalently bound via a tri-functional linker, preferably with each of the GalNAc moiety, the saponin and the oligonucleotide covalently bound to a separate arm of the tri-functional linker.

XX. Oligonucleotide conjugate of any one of the embodiments I-XIX, wherein the ligand for ASGPR is the $(GalNAc)_3$Tris represented by Molecule (DD3) or Molecule (DD4):

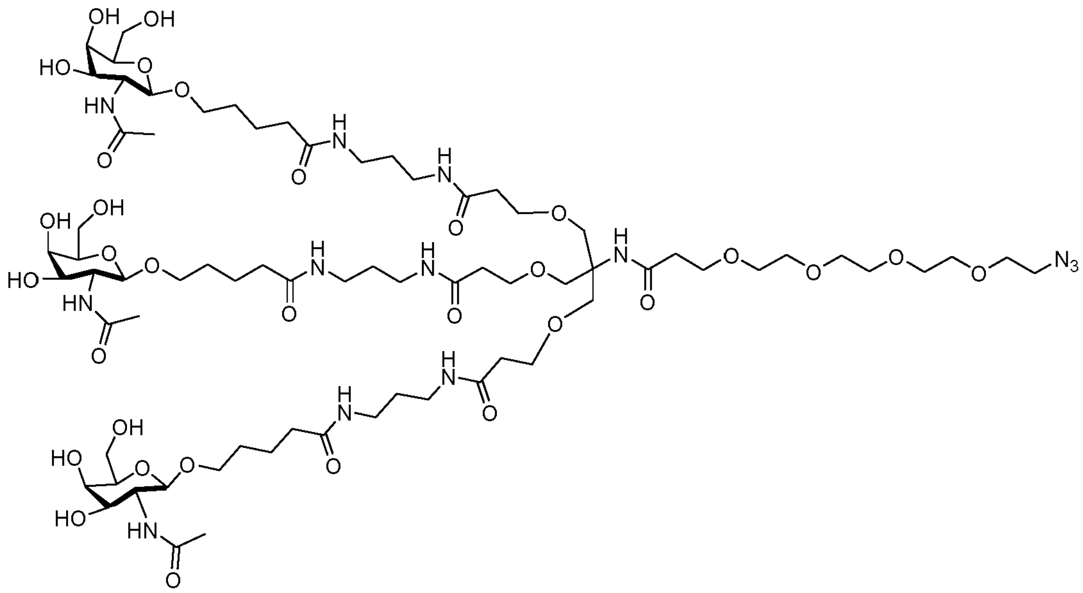

(DD3),

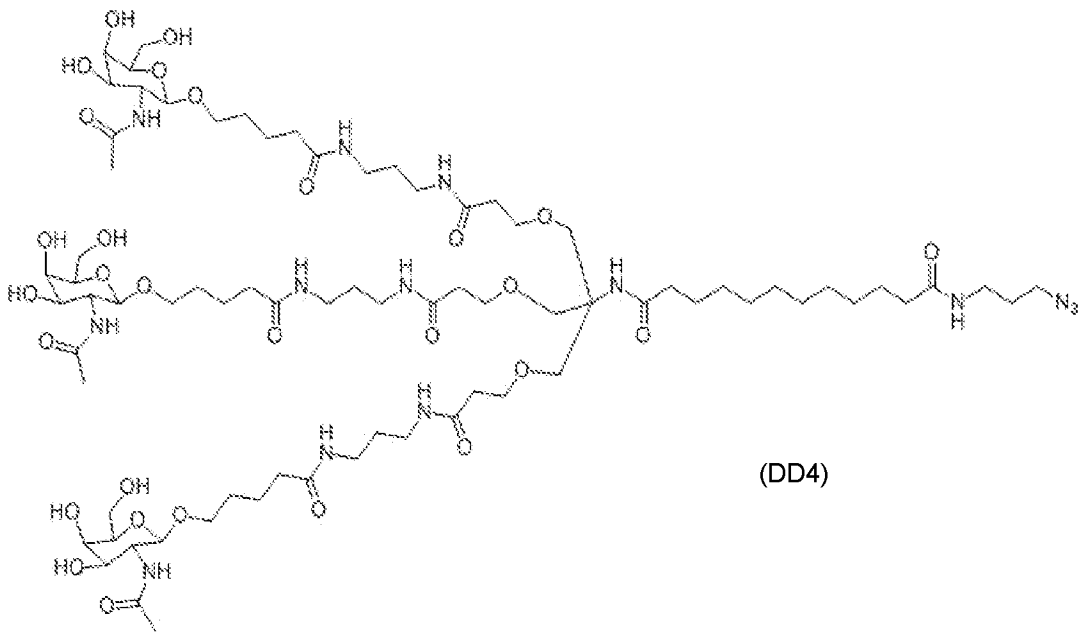

(DD4)

, or wherein the ligand for ASGPR is mono-GalNAc represented by Molecule II':

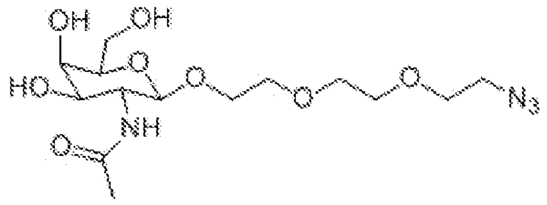

(Molecule II')

.

XXI. Oligonucleotide conjugate of any one of the embodiments I-XX, wherein the at least one saponin is covalently bound to the ligand for ASGPR via at least one cleavable linker, and/or wherein the at least one saponin is covalently bound to the oligonucleotide via at least one cleavable linker.

XXII. Oligonucleotide conjugate of embodiment XIX, wherein the at least one saponin is covalently bound to an arm of the tri-functional linker via at least one cleavable linker.

XXIII. Oligonucleotide conjugate of embodiment XXI or XXII, wherein the cleavable linker is subject to cleavage under acidic conditions, reductive conditions, enzymatic conditions and/or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulfide bond.

XXIV. Oligonucleotide conjugate of any one of the embodiments XXI-XXIII, wherein the cleavable linker is subject to cleavage *in vivo* under acidic conditions such as for example present in endosomes and/or lysosomes of mammalian cells, preferably human cells, preferably the cleavable linker is subject to cleavage *in vivo* at pH 4.0 - 6.5, and more preferably at pH $\leq$ 5.5.

XXV. Oligonucleotide conjugate of any one of the embodiments I-XXIV, wherein the oligonucleotide conjugate comprises 1, 2, 3, 4, 5, 6, 8, 10, 16, 32, 64, 128 or 1-100 saponin moieties, or any number of saponin moieties therein between, such as 7, 9, 12 saponin moieties.

XXVI. Oligonucleotide conjugate of any one of the embodiments I-XXIV, wherein the oligonucleotide conjugate comprises 1 saponin moiety.

XXVII. Oligonucleotide conjugate of any one of the embodiments I-XXVI, wherein the oligonucleotide is any one of a BNA, a xeno nucleic acid, an siRNA, an antisense oligonucleotide.

XXVIII. Oligonucleotide conjugate of any one of the embodiments I-XXVI, wherein the oligonucleotide is selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), DNA, antisense DNA, locked nucleic acid (LNA), bridged nucleic acid (BNA), 2'-O,4'-aminoethylene bridged nucleic acid ($BNA^{NC}$), BNA-based siRNA, and BNA-based antisense oligonucleotide (BNA-AON).

XXIX. Oligonucleotide conjugate of any one of the embodiments I-XXVI, wherein the oligonucleotide is selected from any one or more of a(n): anti-miRNA, a BNA-AON or an siRNA, such as BNA-based siRNA, selected from chemically modified siRNA, metabolically stable siRNA and chemically modified, metabolically stable siRNA.

XXX. Oligonucleotide conjugate of any one of the embodiments I-XXIX, wherein the oligonucleotide is an oligonucleotide capable of, for example when present inside a mammalian cell and preferably when present inside a human cell, silencing any one of genes: apolipoprotein B (*apoB), HSP27,* transthyretin (*TTR*), proprotein convertase subtilisin/kexin type 9 (PCSK9), delta-aminolevulinate synthase 1 (*ALAS1*), anti-thrombin 3 (*AT3*), glycolate oxidase (*GO*), complement component C5 (*CC5*), *X* gene of hepatitis B virus (*HBV*), *S* gene of HBV, alpha-1 antitrypsin (*AAT*) and lactate dehydrogenase (*LDH*), and/or is an oligonucleotide capable of, for example when present inside a mammalian cell, targeting an aberrant miRNA.

XXXI. Oligonucleotide conjugate of any one of the embodiments I-XXIX, wherein the oligonucleotide is an oligonucleotide capable of, for example when present inside a mammalian cell and preferably when present inside a human cell, silencing any one of genes: apolipoprotein B (*apoB*) and *HSP27.*

XXXII. Oligonucleotide conjugate of any one of the embodiments I-XXXI, wherein the oligonucleotide is an oligonucleotide capable of, for example when present inside a mammalian cell and preferably when present inside a human cell, targeting an mRNA involved in expression of any one of proteins: apoB, HSP27, TTR, PCSK9, ALAS1, AT3, GO, CC5, expression product of X gene of HBV, expression product of S gene of HBV, AAT and LDH, or is capable of, for example when present inside a mammalian cell and preferably when present inside a human cell, antagonizing or restoring an miRNA function such as inhibiting an oncogenic miRNA (onco-miR) or suppression of expression of an onco-miR.

XXXIII. Oligonucleotide conjugate of any one of the claims I-XXXI, wherein the oligonucleotide is an oligonucleotide capable of, for example when present inside a mammalian cell and preferably when present inside a human cell, targeting an mRNA involved in expression of any one of proteins: HSP27, apoB, TTR, PCSK9, TMPRSS6, ALAS1, AAT, miR-122, hepatitis B virus HbsAg, LDHA and CEBPA.

XXXIV. Oligonucleotide conjugate of any one of the embodiments I-XXXII, wherein the oligonucleotide is an oligonucleotide capable of, for example when present inside a mammalian cell and preferably when present inside a human cell, targeting an mRNA involved in expression of any one of proteins: apoB and HSP27.

XXXV. Pharmaceutical composition comprising the oligonucleotide conjugate of any one of the embodiments I-XXXIV, and optionally a pharmaceutically acceptable excipient and/or optionally a pharmaceutically acceptable diluent.

XXXVI. Pharmaceutical composition of embodiment XXXV, for use as a medicament.

XXXVII. Pharmaceutical composition of claim XXXV, for use in the treatment or prophylaxis of a disease or health problem in which an expression product is involved of any one or more of genes: *HSP27, apoB, TTR, PCSK9, TMPRSS6, ALAS1, AT3, GO, CC5, X* gene of HBV, *S* gene of HBV, *AAT,* miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3,* GO, *CC5, X* gene of HBV, *S* gene of HBV, *AAT,* miR-122, hepatitis B virus HbsAg, *LDHA, CEBPA* and *LDH.*

XXXVIII. Pharmaceutical composition of claim XXXV, for use in the treatment or prophylaxis of a disease or health problem in which an expression product is involved of any one or more of genes: *HSP27, apoB, TTR, PCSK9, TMPRSS6, ALAS1, AAT,* miR-122, hepatitis B virus HbsAg, *LDHA* and *CEBPA,* and/or for use in the treatment or prophylaxis of a disease or health problem which involves any one or more of genes: *HSP27, apoB, TTR, PCSK9, TMPRSS6, ALAS1, AAT,* miR-122, hepatitis B virus HbsAg, *LDHA* and *CEBPA.*

XXXIX. Pharmaceutical composition for use of claim XXXVI or XXXVII, for use in the treatment or prophylaxis of a disease or health problem in which an expression product is involved of any one or more of genes: *HSP27* and *apoB,* preferably *apoB,* and/or for use in the treatment or prophylaxis of a disease or health problem which involves any one or more of genes: *HSP27* and *apoB,* preferably *apoB.*

XL. Pharmaceutical composition for use of any one of the claims XXXVI-XXXIX, for use in the treatment or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, $\alpha$1-antit$\eta$psin deficiency, $\beta$-thalassaemia, or an auto-immune disease.

XLI. Pharmaceutical composition for use of any one of the claims XXXVI-XXXIX, for use in the treatment or prophylaxis of a cancer such as endometrial carcinoma, breast cancer, lung cancer or hepatocellular carcinoma, and a cardiovascular disease such as hypercholesterolemia, preferably hypercholesterolemia.

XLII. *In vitro* or ex *vivo* method for transferring the oligonucleotide conjugate of any one of the embodiments I-XXXIV from outside a cell to inside said cell, preferably for subsequently transferring the oligonucleotide comprised by the oligonucleotide conjugate of any one of the embodiments I-XXXIV into the cytosol and/or into the nucleus of said cell, comprising the steps of:

a) providing a cell which expresses ASGPR, preferably ASGPR1, on its surface, the cell preferably selected from a liver cell, a virally infected mammalian cell and a mammalian tumor cell, wherein preferably said cell is a human cell;
b) providing the oligonucleotide conjugate of any one of the embodiments I-XXXIV for transferring into the cell provided in step a);
c) contacting the cell of step a) *in vitro* or ex *vivo* with the oligonucleotide conjugate of step b), preferably in a liquid medium, therewith effecting the transfer of the oligonucleotide conjugate from outside the cell into said cell, and optionally and preferably therewith subsequently effecting the transfer of the oligonucleotide comprised by the oligonucleotide conjugate into the cytosol and/or nucleus of said cell.

An aspect of the invention relates to a method for providing the oligonucleotide conjugate of the invention, comprising the steps of:

(a) providing at least one saponin moiety comprising a covalently bound first linker, wherein the first linker comprises at least one first reactive group for covalent binding to a second reactive group on a second linker or to a seventh reactive group on a seventh linker;
(b) providing an oligonucleotide comprising a covalently bound third linker, wherein the third linker comprises a third reactive group for covalent binding to a fourth reactive group on a fourth linker or to an eighth reactive group on the seventh linker;
(c) providing at least one GalNAc moiety comprising a covalently bound fifth linker, wherein the fifth linker comprises a fifth reactive group for covalent binding to a sixth reactive group on a sixth linker or to a ninth reactive group on the seventh linker; and

either

(d1) linking the first linker to the second linker through formation of a covalent bond between the first reactive group and the second reactive group, linking the third linker to the fourth linker through formation of a covalent bond between the third reactive group and the fourth reactive group, linking the fifth linker to the sixth linker through formation of a covalent bond between the fifth reactive group and the sixth reactive group, and covalently linking the second linker, fourth linker and sixth linker together, therewith providing the oligonucleotide,
or
(d2) linking the first linker to the seventh linker through formation of a covalent bond between the first reactive group and the seventh reactive group, linking the third linker to the seventh linker through formation of a covalent bond between the third reactive group and the eighth reactive group, linking the fifth linker to the seventh linker through formation of a covalent bond between the fifth reactive group and the ninth reactive group, therewith providing the oligonucleotide conjugate.

An embodiment is the method for providing the oligonucleotide conjugate of the invention, wherein the seventh linker is a tri-functional linker, such as the tri-functional linker represented by formula (XXI):

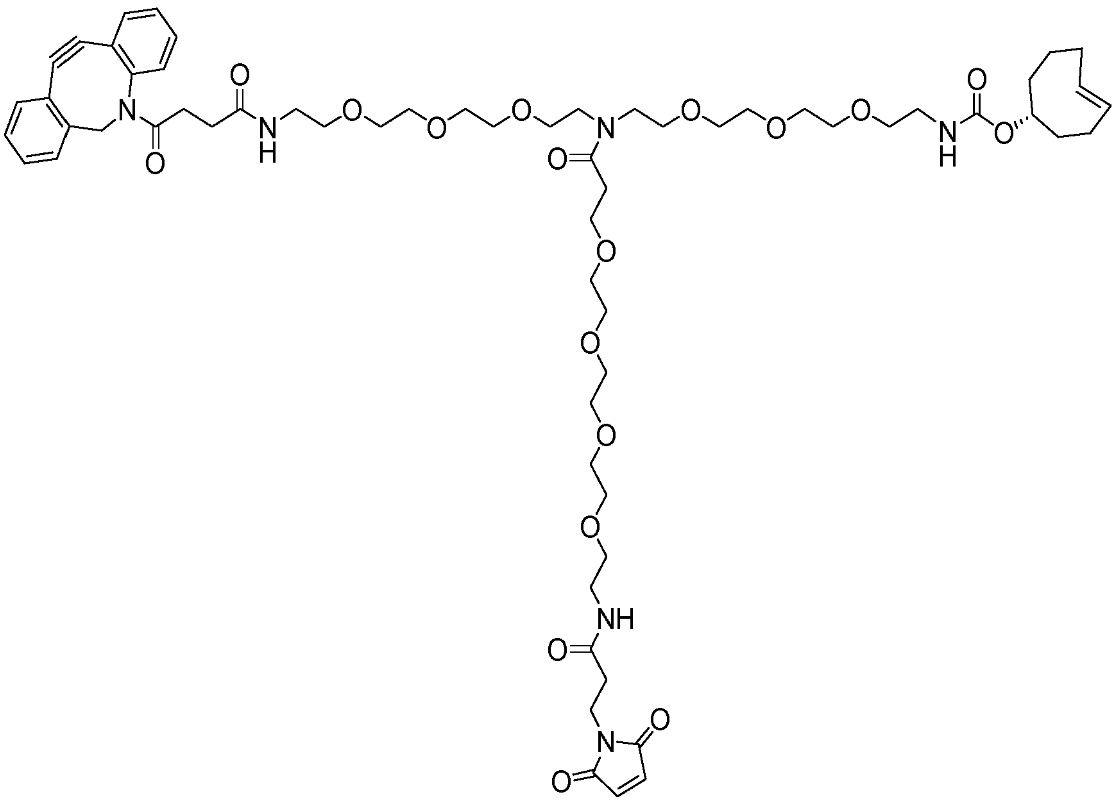

(XXI).

Preferred is the method for providing the oligonucleotide conjugate of the invention, wherein the at least one saponin moiety are 1-16 saponin moieties, preferably 1-8 saponin moieties, such as 1, 4 or 8 saponin moieties.

Preferred is the method for providing the oligonucleotide conjugate of the invention, wherein the saponin is SO1861, SO1832, QS-21, or any functional derivative thereof, preferably SO1861 or SO1832.

Preferred is the method for providing the oligonucleotide conjugate of the invention, wherein the saponin moiety or the saponin moieties is/are covalently linked via a hydrazone bond or a semicarbazone bond.

Preferred is the method for providing the oligonucleotide conjugate of the invention, wherein the at least one GalNAc moiety are 1-4 GalNAc moieties, preferably 1 or 3 GalNAc moieties.

An embodiment is the oligonucleotide conjugate of the invention, wherein the saponin comprised by the oligonucleotide conjugate is isolated from a plant. Preferably, the saponin is isolated from a part of a plant, such as the root, or from a part of a tree, such as the bark. Preferably, the saponin is isolated from roots derived from a plant.

The following is a summary of preferred embodiments of the oligonucleotide conjugate according to the invention:

1A. Oligonucleotide conjugate comprising at least one saponin covalently linked to a ligand for asialoglycoprotein receptor (ASGPR), wherein the ligand for ASGPR comprises at least one N-acetylgalactosamine (GalNAc) moiety, preferably three or four GalNAc moieties, more preferably three GalNAc moieties, more preferably the ligand for ASGPR comprises or consists of $(GalNAc)_3$Tris, and further covalently linked to an oligonucleotide, wherein the at least one saponin is a monodesmosidic or bidesmosidic penta-cyclic triterpene saponin of the 12,13-dehydrooleanane type, preferably with an aldehyde function in position C-23 of the aglycone core structure of the saponin, wherein the oligonucleotide conjugate comprises 1-16 saponin moieties, preferably 1-8 saponin moieties, more preferably 1 saponin moiety, 4 saponin moieties or 8 saponin moieties.

2A. The oligonucleotide conjugate of embodiment 1A, wherein the at least one GalNAc moiety, preferably three GalNAc moieties, the at least one saponin, preferably 1-16 saponin moieties, more preferably 1-8 saponin moieties such as 1, 4 or 8 saponin moieties, and the oligonucleotide are covalently bound via a tri-functional linker, preferably with each of the GalNAc moiety or moieties, the saponin or saponin moieties and the oligonucleotide covalently bound to a separate arm of the tri-functional linker.

3A. The oligonucleotide conjugate of embodiment 1A or 2A, comprising one saponin moiety, or 4 saponin moieties, preferably 4 saponin moieties covalently bound to a dendron, preferably a G2 dendron such as for example N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide), or 8 saponin moieties, preferably 8 saponin moieties covalently bound to a dendron, preferably a G3 dendron such as for example (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide.

4A. The oligonucleotide conjugate of any one of the embodiments 1A-3A, wherein the oligonucleotide conjugate comprises one saponin moiety and wherein the oligonucleotide conjugate is according to molecule (EE):

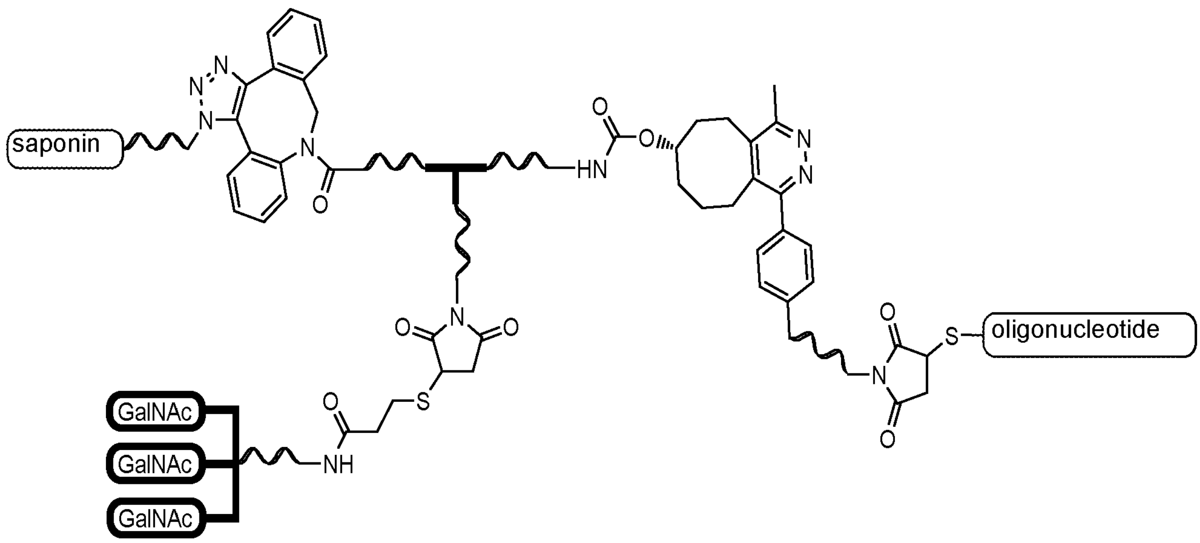

(EE)

, which molecule (EE) is the covalent conjugation product obtained by the covalent conjugation of tri-functional linker according to formula (XXI):

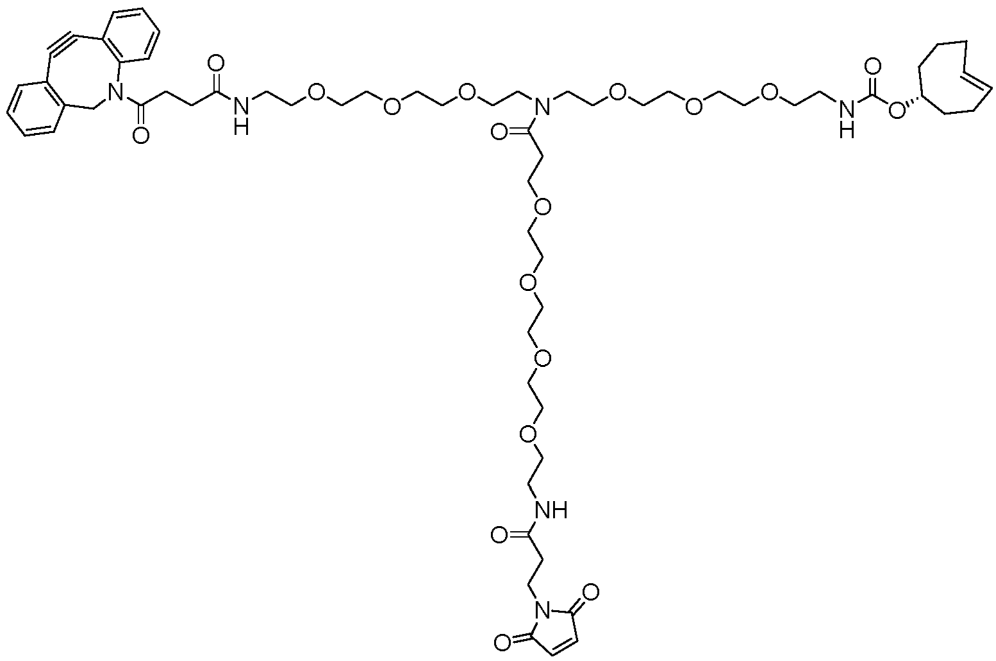

(XXI)

with

(1) saponin derivative according to molecule (AA):

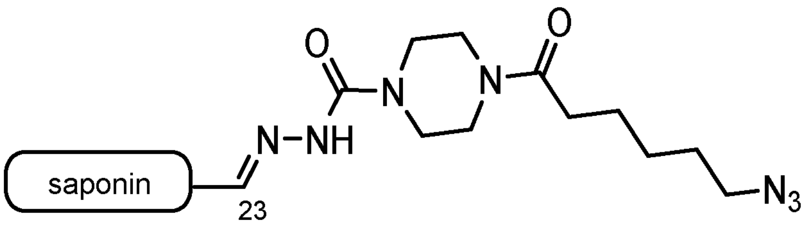

(AA)

, wherein

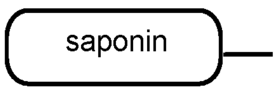

represents a saponin moiety according to formula (SM):

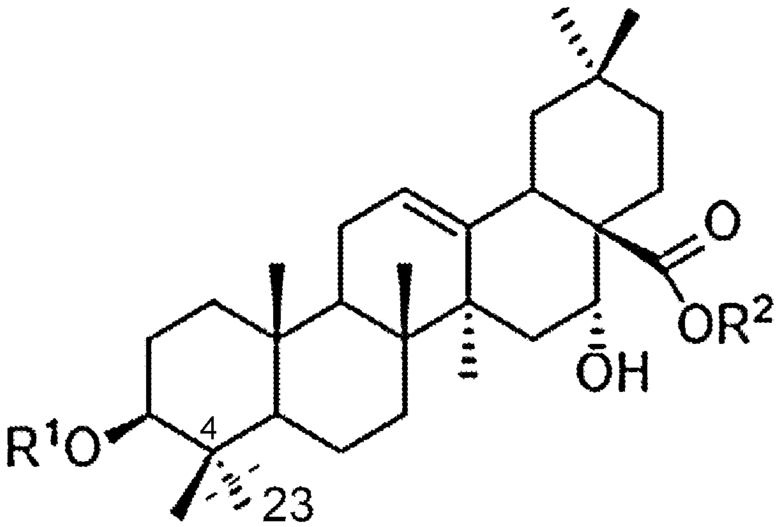

(SM)

, wherein $R^1$ and $R^2$ are independently selected from hydrogen, a monosaccharide, a linear oligosaccharide and a branched oligosaccharide, and wherein the saponin moiety according to formula (SM) is based on a saponin comprising an aldehyde group in position C-23,
and with

(2) GalNAc conjugate according to molecule (FF):

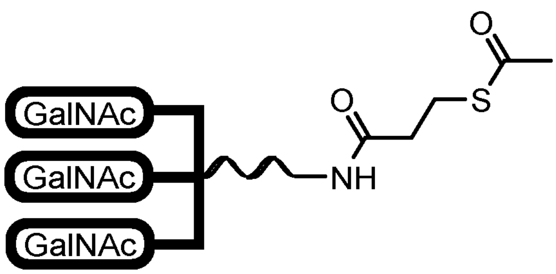

(FF)

, wherein

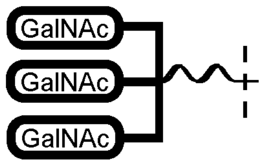

represents the tri-GalNAc conjugate according to molecule (DD1) or molecule (DD2) as detailed here above, preferably molecule (DD3) or molecule (DD4) as detailed here above, more preferably molecule (DD3) as detailed here above,
and with

(3) the oligonucleotide provided with a linker according to molecule (GG):

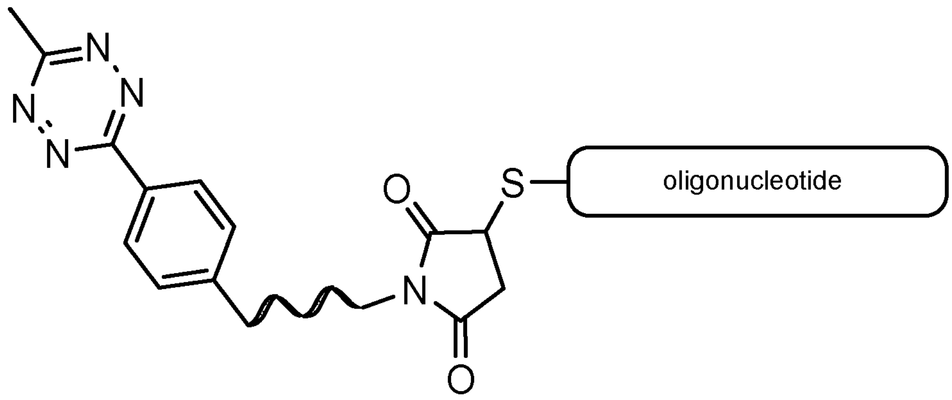

(GG)

, wherein the molecule (GG) represents the conjugation product of the conjugation reaction between the linker (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide and the oligonucleotide-linker molecule according to molecule (HH):

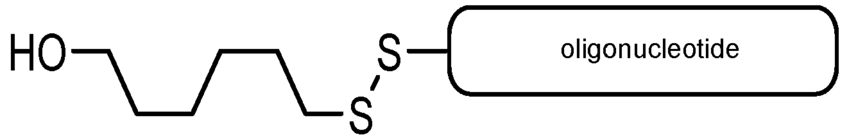

(HH).

5A. The oligonucleotide conjugate of any one of the embodiments 1A-3A, comprising four saponin moieties and wherein the oligonucleotide conjugate is according to molecule (PP):

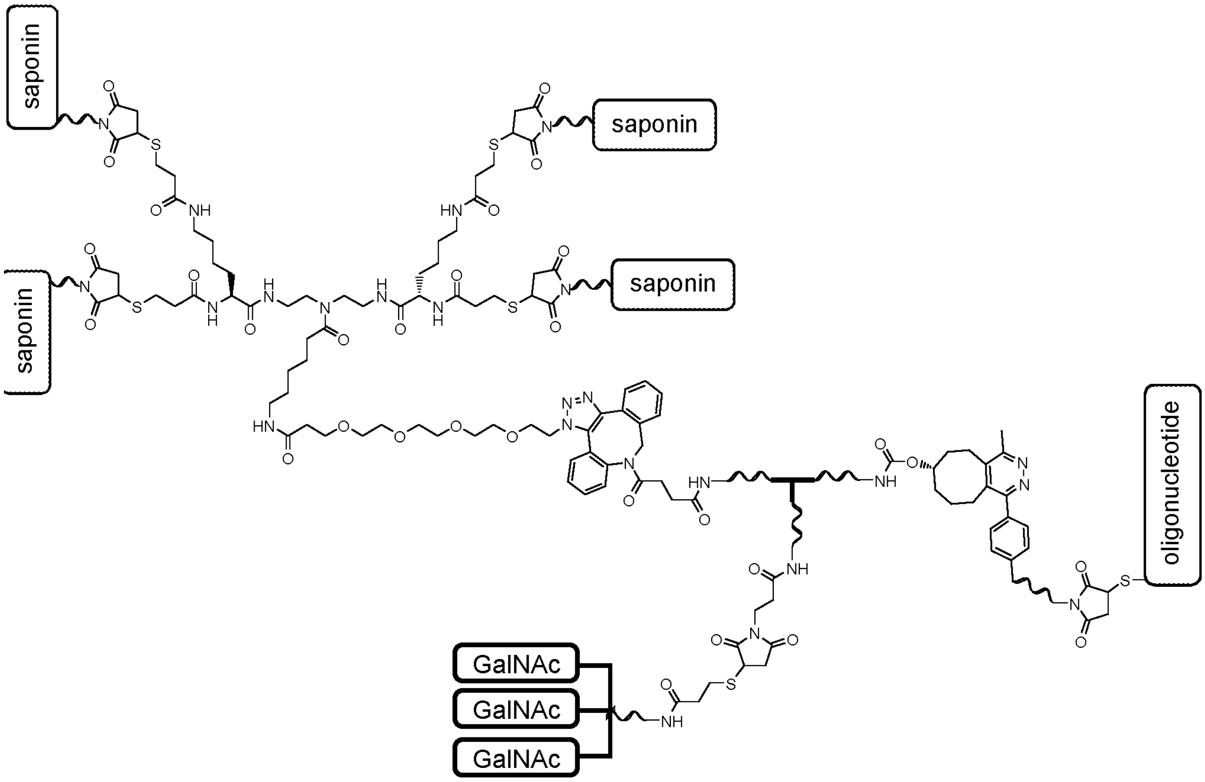

(PP)

, which molecule (PP) is the covalent conjugation product obtained by the covalent conjugation of saponin-GalNAc conjugate according to molecule (LL):

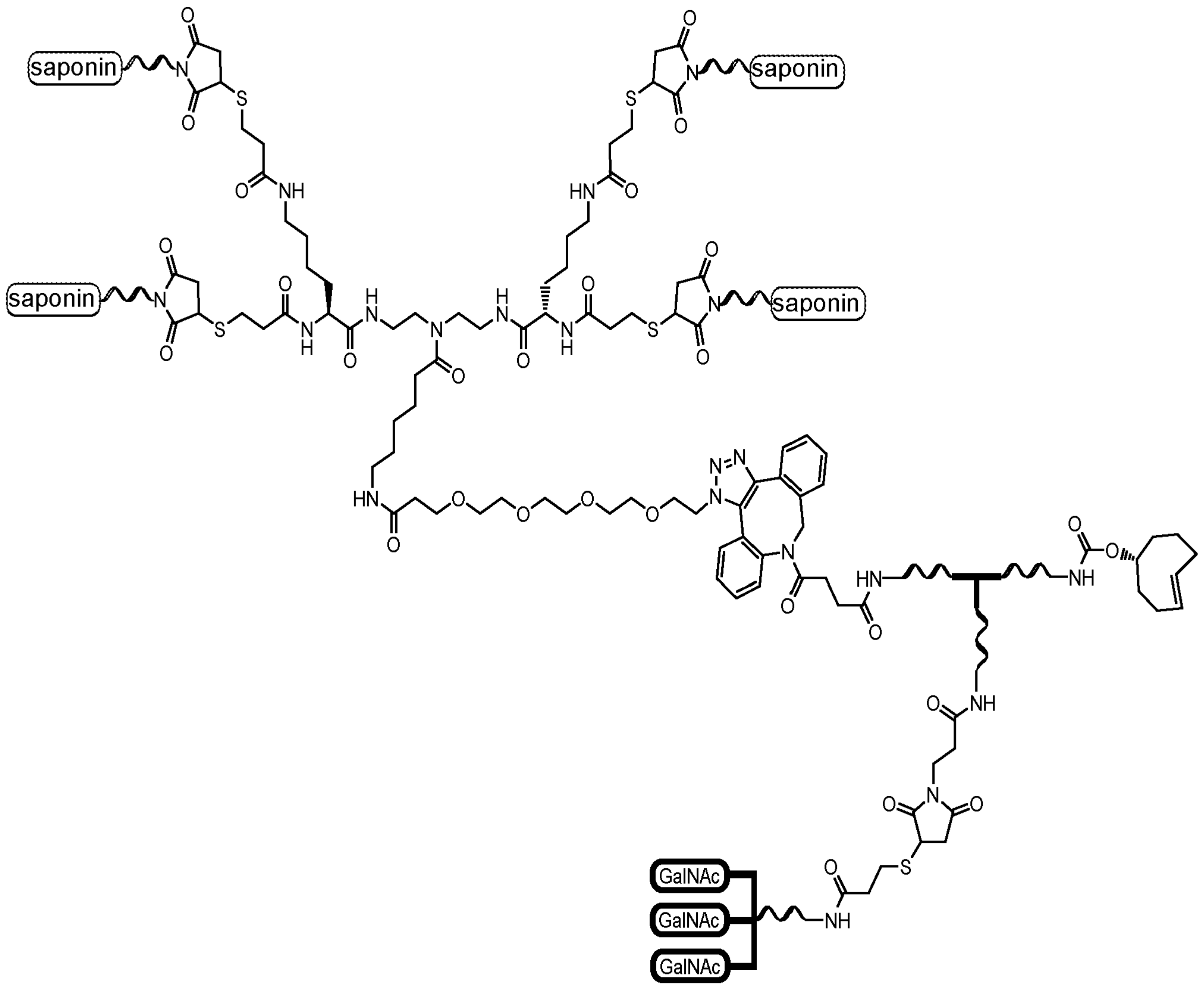

(LL)

with the oligonucleotide provided with a linker according to molecule (GG):

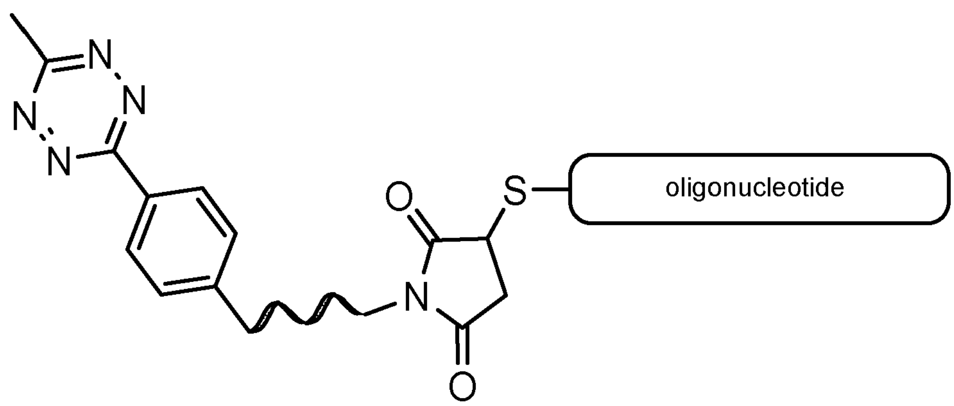

(GG)

, wherein the molecule (GG) represents the conjugation product of the conjugation reaction between the linker (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide and the oligonucleotide-linker molecule according to molecule (HH):

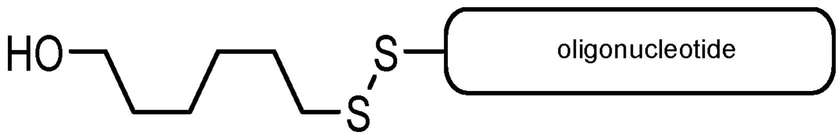

(HH)

, which molecule (LL) is the covalent conjugation product obtained by the covalent conjugation of saponin derivative according to molecule (JJ):

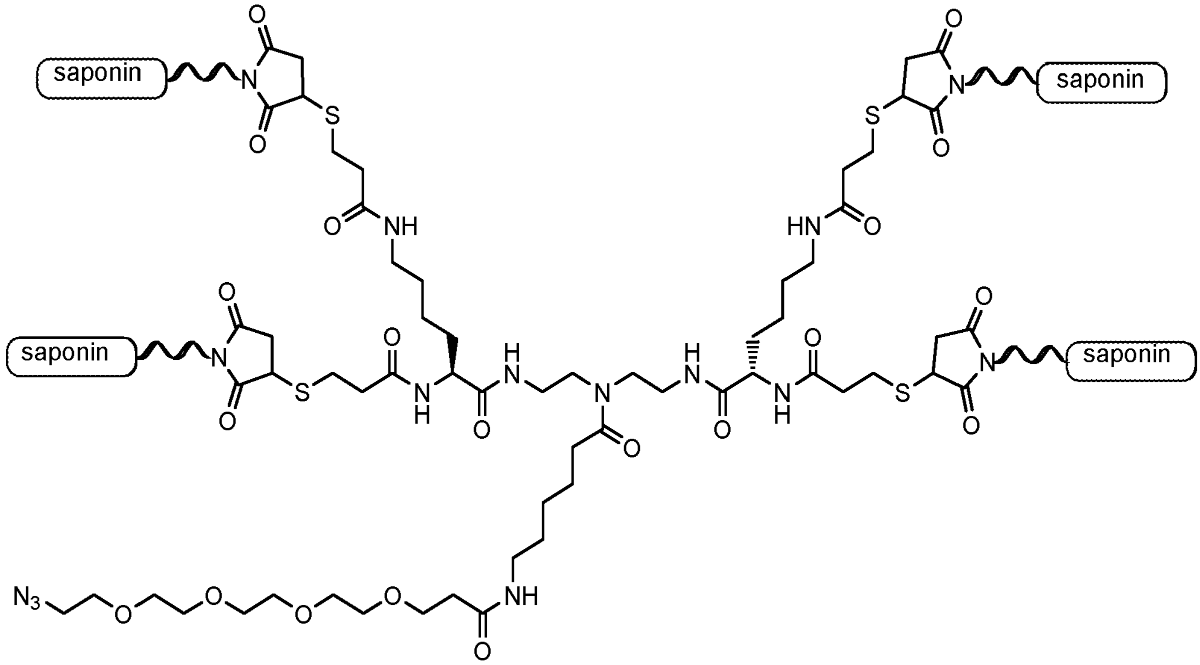

(JJ)

with the conjugate of tri-functional linker and GalNAc according to molecule (MM):

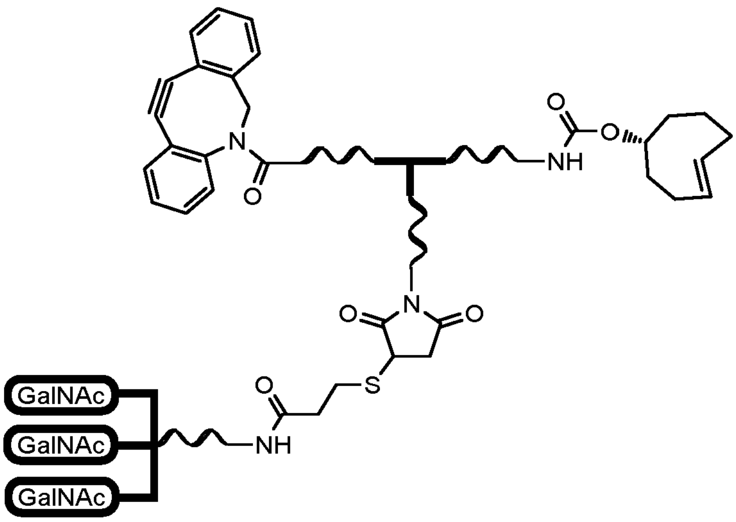

(MM)

, which molecule (JJ) is the conjugate product of conjugation of N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide first with saponin derivative according to molecule (KK):

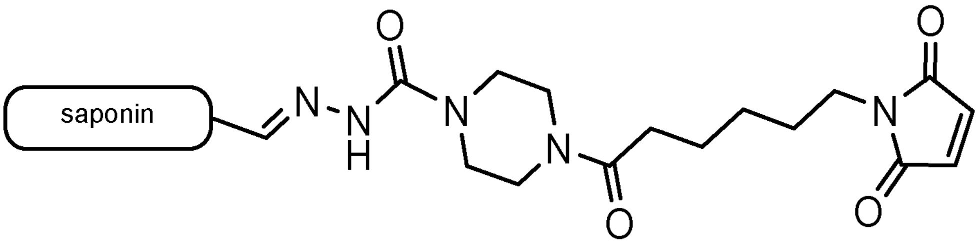

(KK)

, wherein

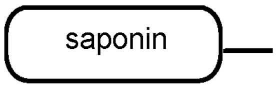

represents a saponin moiety according to formula (SM):

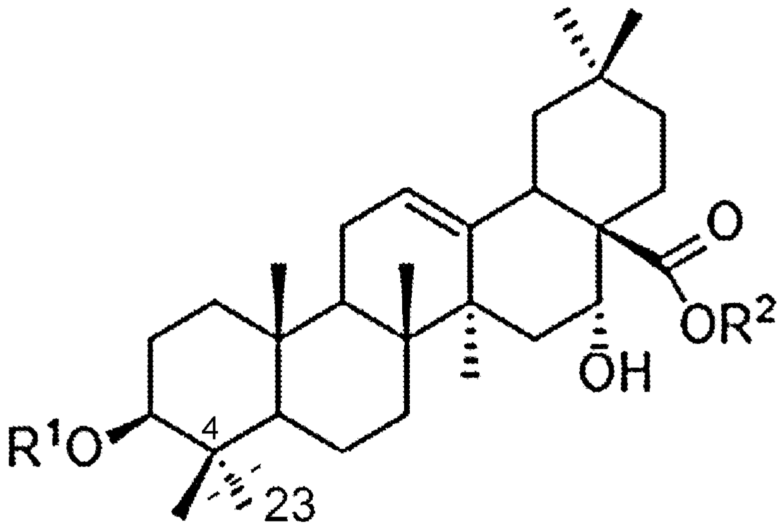

SM)

, wherein $R^1$ and $R^2$ are independently selected from hydrogen, a monosaccharide, a linear oligosaccharide and a branched oligosaccharide according to the invention, and wherein the saponin moiety according to formula (SM) is based on a saponin comprising an aldehyde group in position C-23,

and subsequently with 2,5-dioxopyrrolidin-1-yl 1-azido-3,6,9,12-tetraoxapentadecan-15-oate,

, and wherein molecule (MM) is the conjugate of tri-functional linker according to formula (XXI):

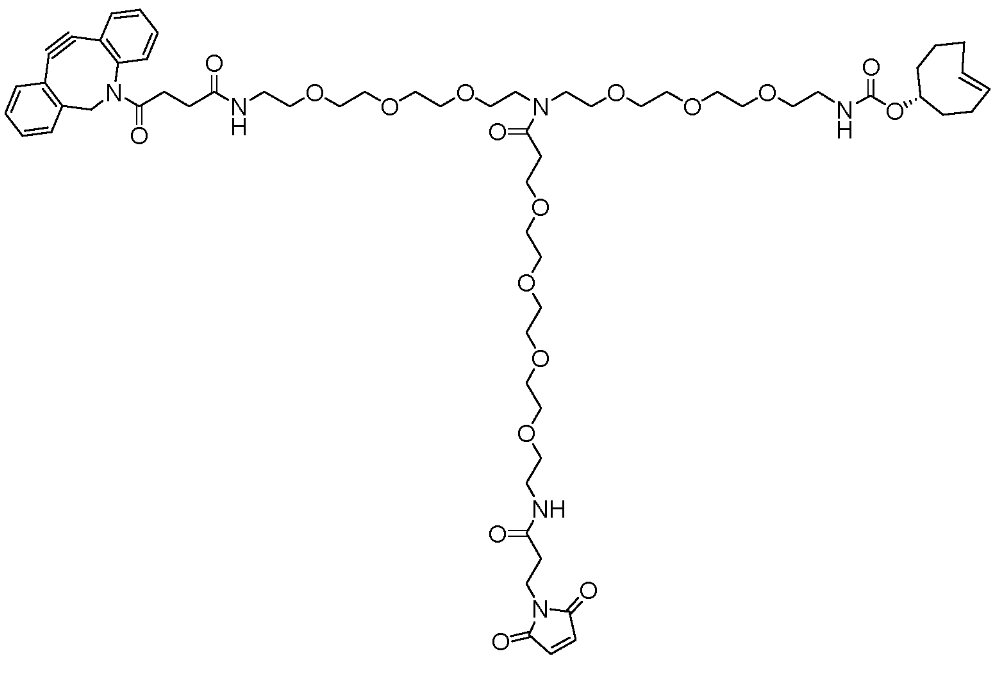

(XXI)

and GalNAc conjugate according to molecule (NN):

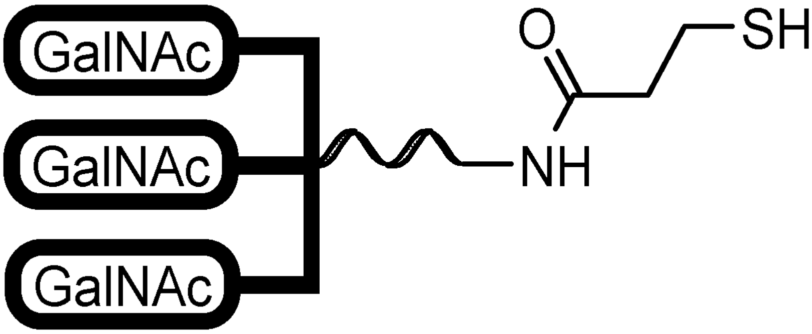

(NN)

, wherein

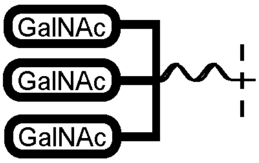

represents the tri-GalNAc conjugate according to molecule (DD1) or molecule (DD2) as detailed here above, preferably molecule (DD3) or molecule (DD4) as detailed here above, more preferably molecule (DD3) as detailed here above.

6A. The oligonucleotide conjugate of any one of the embodiments 1A-3A, comprising eight saponin moieties and wherein the oligonucleotide conjugate is according to molecule (SS):

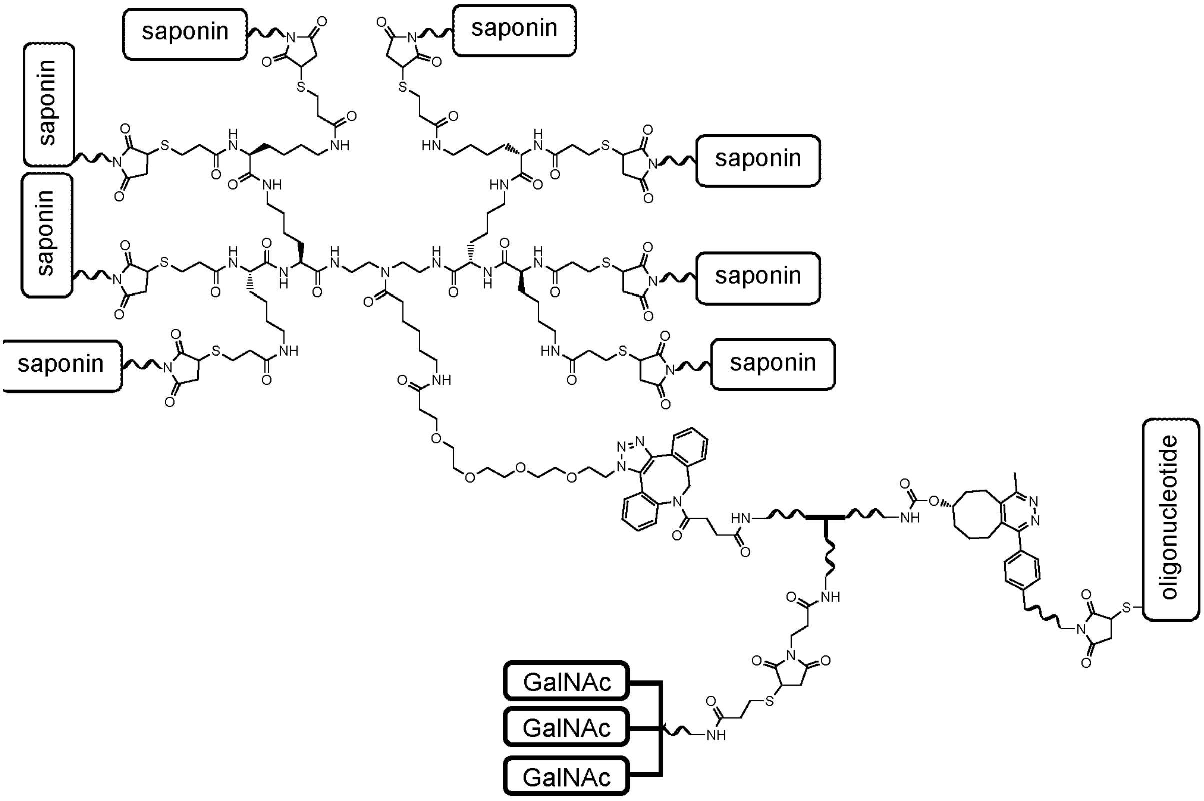

(SS)

, which molecule (SS) is the covalent conjugation product obtained by the covalent conjugation of saponin-GalNAc conjugate according to molecule (RR):

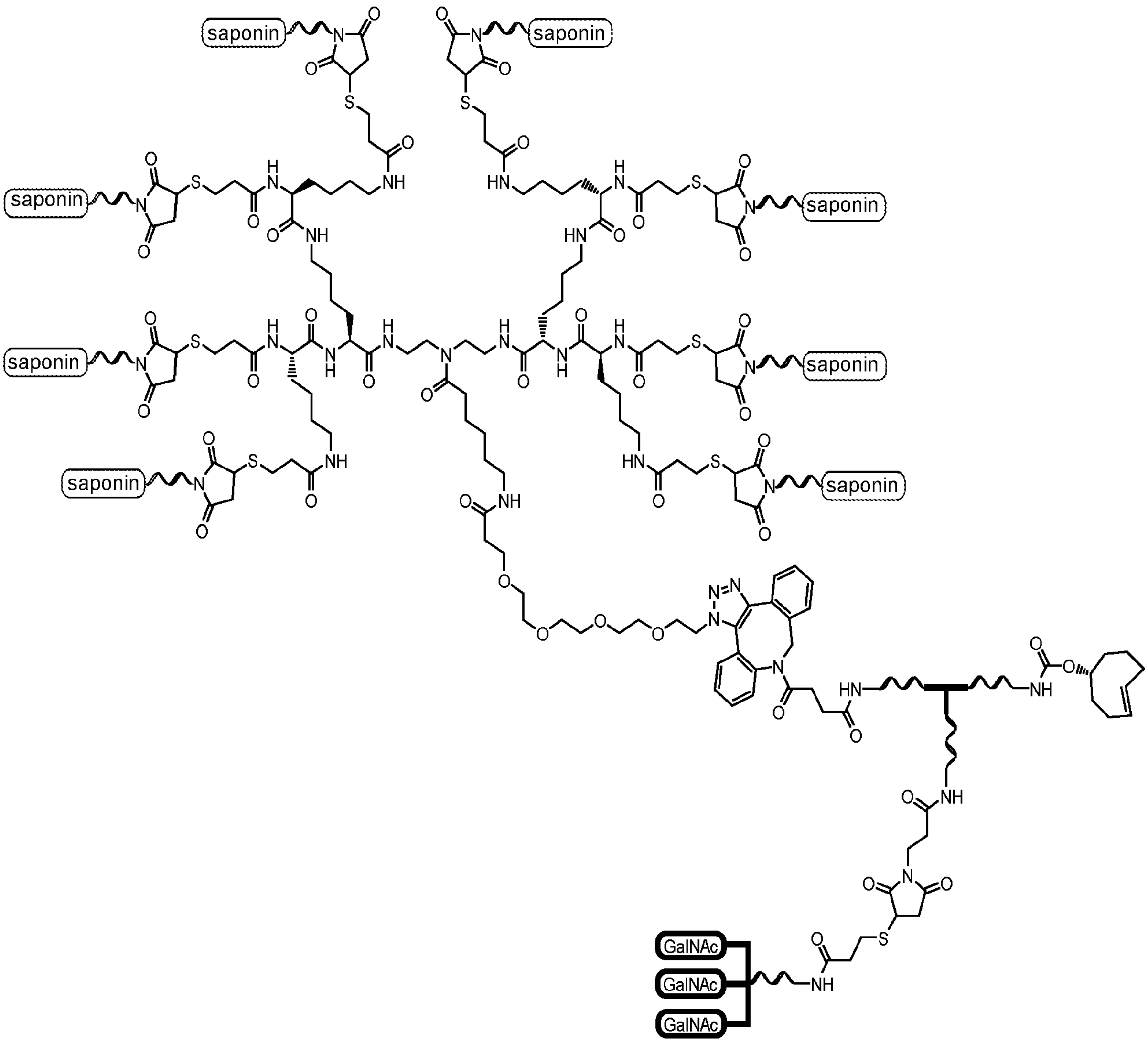

(RR)

with oligonucleotide provided with a linker according to molecule (GG):

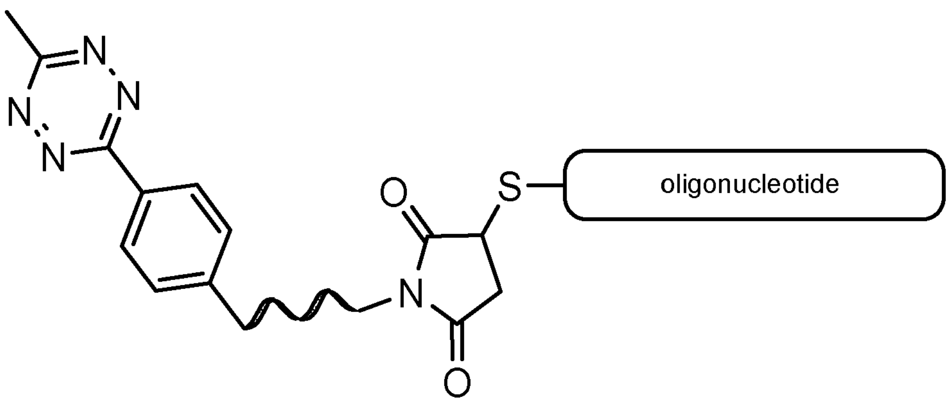

(GG)

, wherein the molecule (GG) represents the conjugation product of the conjugation reaction between the linker

(E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide and the oligonucleotide-linker molecule according to molecule (HH):

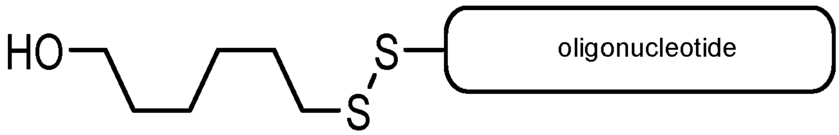

(HH)

, which molecule (RR) is the covalent conjugation product obtained by the covalent conjugation of saponin derivative according to molecule (QQ):

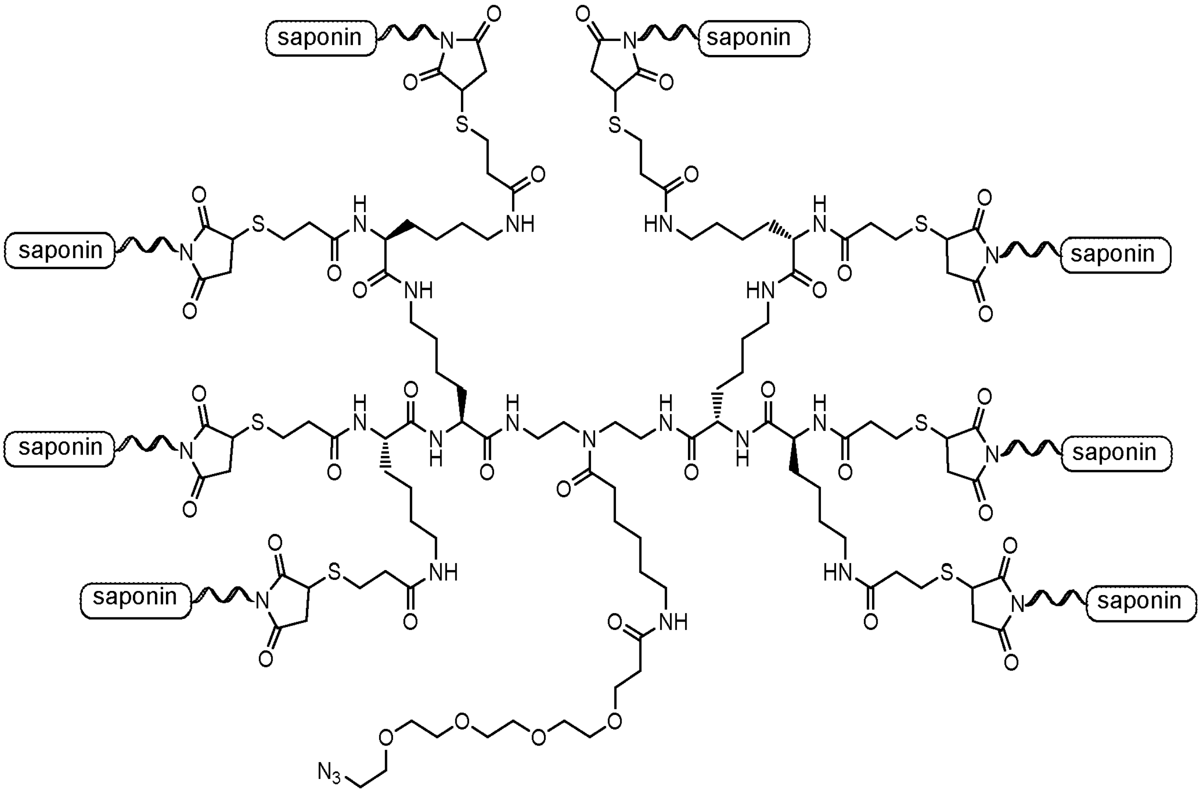

(QQ)

with conjugate of tri-functional linker and GalNAc according to molecule (MM):

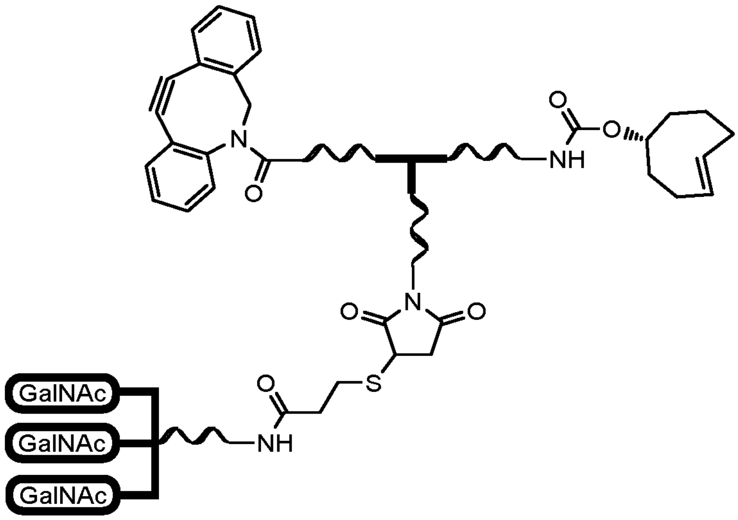

(MM)

, which molecule (QQ) is the conjugate product of conjugation of (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate first

(a) with saponin derivative according to molecule (KK):

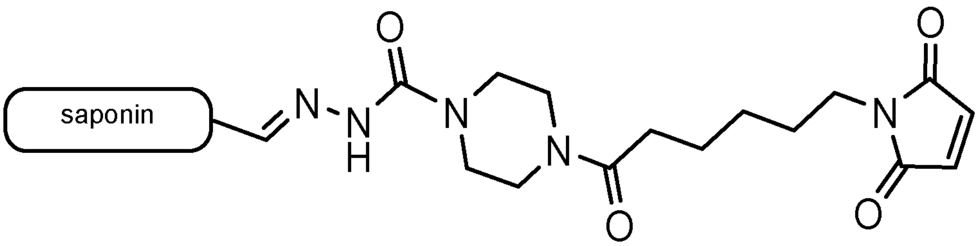

(KK)

, wherein

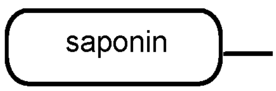

represents a saponin moiety according to formula (SM):

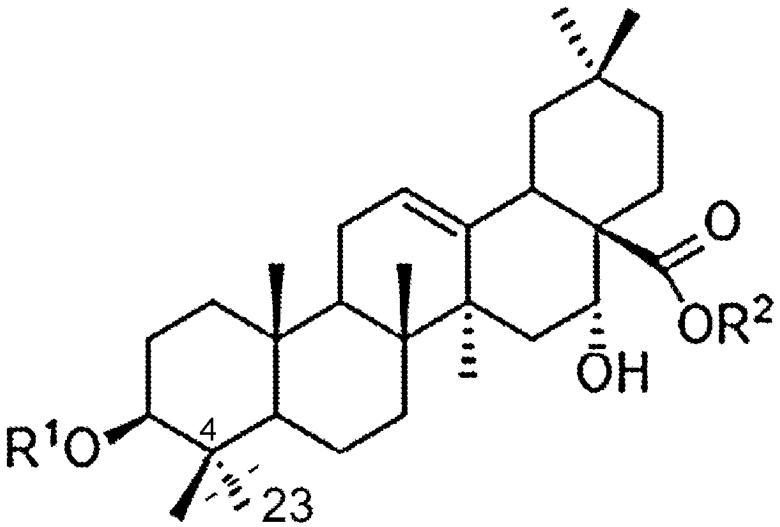

(SM)

, wherein $R^1$ and $R^2$ are independently selected from hydrogen, a monosaccharide, a linear oligosaccharide and a branched oligosaccharide according to the invention, and wherein the saponin moiety according to formula (SM) is based on a saponin comprising an aldehyde group in position C-23,
and subsequently

(b) with 2,5-dioxopyrrolidin-1-yl 1-azido-3,6,9,12-tetraoxapentadecan-15-oate,
and wherein molecule (MM) is the conjugate of tri-functional linker according to formula (XXI):

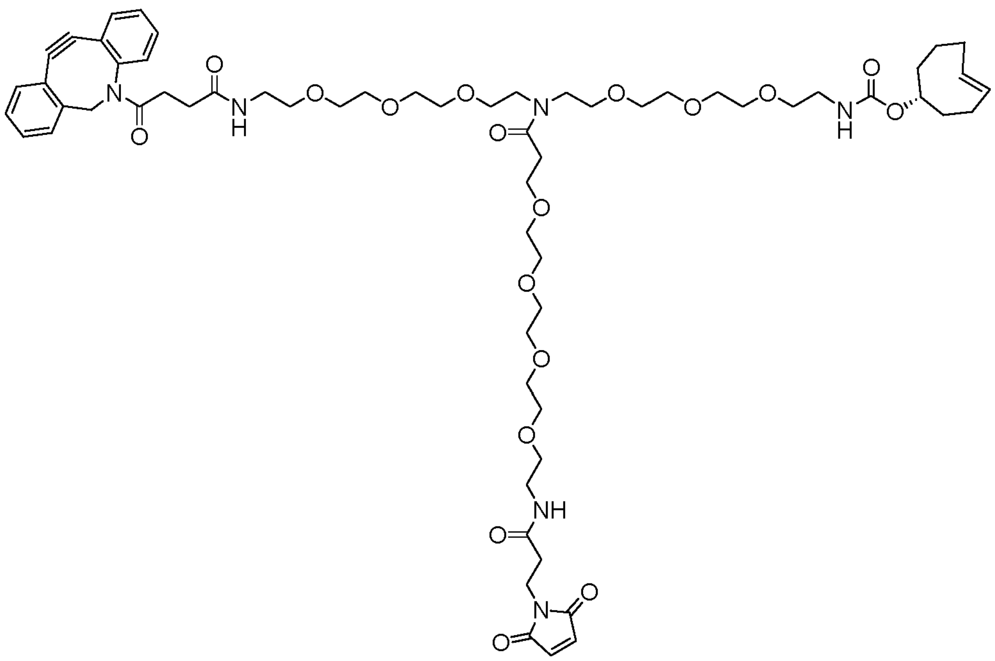

(XXI)

and GalNAc conjugate according to molecule (NN) (See for molecule (NN) embodiment 5A, here above), wherein

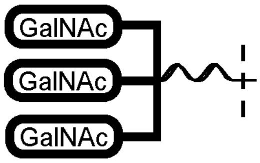


represents the tri-GalNAc conjugate according to molecule (DD1) or molecule (DD2) as detailed here above, preferably molecule (DD3) or molecule (DD4) as detailed here above, more preferably molecule (DD3) as detailed here above.

7A. The oligonucleotide conjugate of any one of the embodiments 1A-6A, wherein the saponin comprised by the conjugate is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Gypsophila elegans* saponin mixture, or a saponin isolated from *Gypsophila elegans;*
- Saponinum album saponin mixture, or a saponin isolated from Saponinum album;
- *Saponaria officinalis* saponin mixture, or a saponin isolated from *Saponaria officinalis*; and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B: SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C: AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, 10 SO1832, SO1861, SO1862, SO1904, QS-7, QS-7 api, QS-17, QS-18, QS-21 A-apio, QS-21 A-xylo, QS-21 B-apio and QS-21 B-xylo; or
d) a saponin comprising a 12, 13-dehydrooleanane type aglycone core structure without an aldehyde group at the C-23 position of the aglycone, selected from list D:
Aescin la, aescinate, alpha-Hederin, AMA-1, AMR, AS6.2, AS64R, Assamsaponin F, dipsacoside B, esculentoside A, macranthoidin A, NP-005236, NP-012672, Primula acid 1, saikosaponin A, saikosaponin D, Teaseed saponin I and Teaseedsaponin J,

preferably, the saponin is any one or more of a saponin selected from list A, B or C, more preferably, a saponin selected from list B or C,
even more preferably, a saponin selected from list C.

8A. The oligonucleotide conjugate of any one of the embodiments 1A-7A, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* Saponarioside B, SO1542, SO1584, SO1658, SO1674, SO1700, SO1730, SO1772, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

9A. The oligonucleotide conjugate of any one of the embodiments 1A-8A, wherein the saponin is a saponin isolated from *Saponaria officinalis,* preferably wherein the saponin is any one or more of Saponarioside B, SO1542, SO1584, SO1658, SO1674, SO1700, SO1730, SO1772, SO1832, SO1861, SO1862 and SO1904; more preferably wherein the saponin is any one or more of SO1542, SO1584, SO1658, SO1674, SO1700, SO1730, SO1772, SO1832, SO1861, SO1862 and SO1904; even more preferably wherein the saponin is any one or more of SO1832, SO1861 and SO1862; even more preferably wherein the saponin is SO1832 and SO1861; most preferably being SO1861.

10A. The oligonucleotide conjugate of any one of the embodiments 1A-9A, wherein the saponin is SO1861, and wherein the oligonucleotide conjugate is provided by conjugation of SO1861 saponin derivative according to molecule (VII)a:

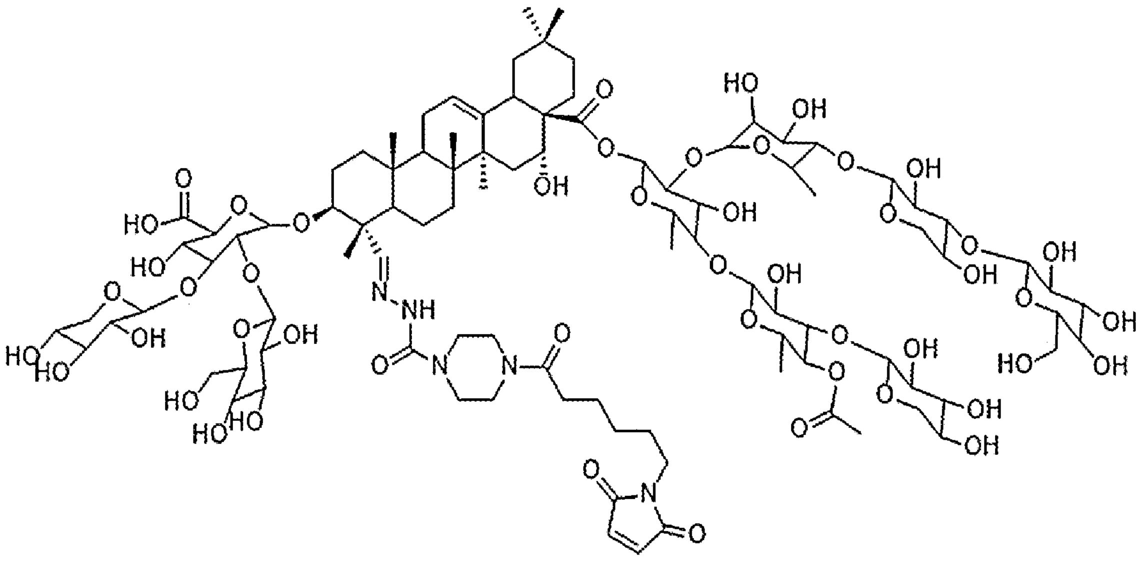

(VII)a

with an oligonucleotide and with at least one GalNAc moiety, to provide the oligonucleotide conjugate.

11A. The oligonucleotide conjugate of any one of the embodiments 1A-10A, wherein the oligonucleotide comprised by said conjugate is defined as a nucleic acid no longer than 150 nt, preferably wherein the oligonucleotide has a size of 5 - 150 nt, preferably being 8 - 100 nt, most preferably being 10 - 50 nt.

12A. Second pharmaceutical composition comprising the oligonucleotide conjugate of any one of the embodiments 1A-11A, and optionally a pharmaceutically acceptable excipient and/or optionally a pharmaceutically acceptable diluent.

13A. Second pharmaceutical composition of embodiment 12A or the oligonucleotide conjugate of any one of the embodiments 1A-11A, for use as a medicament.

14A. Second pharmaceutical composition of embodiment 12A or the oligonucleotide conjugate of any one of the embodiments 1A-11A, for use in the treatment or prophylaxis of a disease or health problem in which an expression product is involved of any one or more of genes: *HSP27, apoB, TTR, PCSK9, TMPRSS6, ALAS1, AT3, GO*, *CC5, X* gene of HBV, *S* gene of HBV, *AAT,* miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3,* GO, *CC5*, *X* gene of HBV, *S* gene of HBV, *AAT,* miR-122, hepatitis B virus HbsAg, *LDHA, CEBPA* and *LDH.*

15A. Second pharmaceutical composition of embodiment 12A or the oligonucleotide conjugate of any one of the embodiments 1A-11A, for use in the treatment or prophylaxis of a disease or health problem in which an expression product is involved of any one or more of genes: *HSP27, apoB, TTR, PCSK9, TMPRSS6, ALAS1, AAT,* miR-122, hepatitis B virus HbsAg, *LDHA* and *CEBPA,* and/or for use in the treatment or prophylaxis of a disease or health problem which involves any one or more of genes: *HSP27, apoB, TTR, PCSK9, TMPRSS6, ALAS1, AAT,* miR-122, hepatitis B virus HbsAg, *LDHA* and *CEBPA.*

16A. Second pharmaceutical composition of embodiment 12A or the oligonucleotide conjugate of any one of the embodiments 1A-11A, for use according to embodiment 14A or 15A, wherein said use is in the treatment or prophylaxis of a disease or health problem in which an expression product is involved of any one or more of genes: *HSP27* and *apoB,* preferably *apoB,* and/or for use in the treatment or prophylaxis of a disease or health problem which involves any one or more of genes: *HSP27* and *apoB,* preferably apoB.

17A. Second pharmaceutical composition of embodiment 12A or the oligonucleotide conjugate of any one of the embodiments 1A-11A, for use according to any one of the embodiments 14A-16A, wherein said use is in the treatment

or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, a1-antitrypsin deficiency, β-thalassaemia, or an auto-immune disease.

18A. Second pharmaceutical composition of embodiment 12A or the oligonucleotide conjugate of any one of the embodiments 1A-11A, for use according to any one of the embodiments 14A-17A, wherein said use is in the treatment or prophylaxis of a cancer such as endometrial carcinoma, breast cancer, lung cancer or hepatocellular carcinoma, and/or a cardiovascular disease such as hypercholesterolemia, preferably hypercholesterolemia.

19A. Second pharmaceutical composition of embodiment 12A or the oligonucleotide conjugate of any one of the embodiments 1A-11A, for use in the lowering of LDL-cholesterol in a subject.

20A. *In vitro* or *ex vivo* method for transferring the oligonucleotide conjugate of any one of the embodiments 1A-11A from outside a cell to inside said cell, preferably subsequently transferring the oligonucleotide comprised by said oligonucleotide conjugate into the cytosol of said cell, comprising the steps of:

a) providing a cell which expresses ASGPR on its surface, the cell preferably selected from a liver cell, a virally infected cell and a tumor cell, and providing the oligonucleotide conjugate of any one of the embodiments 1A-11A for transferring into the provided cell;
b) contacting the cell of step a) *in vitro* or *ex vivo* with the oligonucleotide conjugate of step a), therewith effecting the transfer of the oligonucleotide conjugate from outside the cell into said cell, and preferably therewith subsequently effecting the transfer of the oligonucleotide comprised by the oligonucleotide conjugate into the cytosol of said cell.

Preferably, the subject is a human subject.

It is preferred that the saponin of the oligonucleotide conjugate is a saponin derived from a plant. The saponin comprised by the oligonucleotide conjugate of the invention is preferably a saponin isolated from a plant. For example, the saponin is isolated from the root of a plant. Examples of such plants from which the saponin is derived (isolated) are *Quillaja saponaria, Gypsophila paniculate L., Saponaria officinalis* such as *Saponaria officinalis* L. and *Gypsophila elegans* such as *Gypsophila elegans* M. Bieb. Preferably, the oligonucleotide conjugate comprises a single type of saponin, preferably a single type of saponin derived from plant material such as the roots of a plant, such as SO1861, SO1862 or SO1832 from *Saponaria officinalis* (e.g., *Saponaria officinalis* L., preferably *Saponaria officinalis* L.) (roots) or such as a QS-21, a QS-7 or a QS-17 from *Quillaja saponaria* (roots). Suitable sources for isolating saponins according to the invention, i.e. those that display endosomal escape enhancing activity, are *Quillaja saponaria, Gypsophila paniculate L., Saponaria officinalis* and *Gypsophila elegans,* and Quillaja bark.

An embodiment is the oligonucleotide conjugate of the invention, wherein the saponin comprised by the oligonucleotide conjugate is isolated from a plant. Preferably, the saponin is isolated from a part of a plant, such as the root, or from a part of a tree, such as the bark. Preferably, the saponin is isolated from roots derived from a plant.

The present invention has been described above with reference to a number of exemplary embodiments. The invention is further illustrated by the following examples.

EXAMPLES AND EXEMPLARY EMBODIMENTS

**Example 1.** *CD71-saporin + 4000 nM trivalent GalNAc-L-SO1861*

Trivalent-GalNAc is a targeting ligand that recognizes and binds the ASGPR1 receptor on hepatocytes. Trivalent GalNAc was produced (Figure 1A-C and Figure 8A-G) and SO1861-EMCH was conjugated (labile, in a similar manner as described in Figure 2A-B and Figure 4 for underivatised SO1861, wherein SPT001 is synonymous to SO1861) to trivalent-GalNAc, with a DAR 1, (trivalent-GalNAc-SO1861). SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. The 'trivalent-GalNAc' as depicted in Figures 1A-C and 8A-G is a typical $(GalNAc)_3$Tris conjugate suitable for coupling to an effector molecule or to saponin. As a control, SO1861-EMCH was also conjugated (labile) to monovalent-GalNAc (Figure 3), with a DAR 1, (GalNAc-SO1861). HepG2 ($ASGPR1^+$; $CD71^+$, Table A3) and Huh7 ($ASGPR1^{+1-}$; $CD71^+$, Table A3) cells were treated with a concentration range of trivalent-GalNAc-L-SO1861 and GalNAc-L-SO1861 in the presence and absence of 10 pM CD71-saporin (monoclonal antibody clone OKT-9 targeting CD71 conjugated to the protein toxin saporin). Cell treatment in absence of CD71-saporin show that the trivalent-GalNAc-L-SO1861 (or trivalent-GalNAc) as single compound is not toxic up to 15000 nM (Figure 9).

HepG2 and Huh7 cells were also treated with a range of concentrations of CD71-saporin in combination with a fixed concentration of 1000 nM or 4000 nM trivalent-GalNAc-SO1861 (DAR1). Targeted protein toxin mediated cell killing of ASGPR1/CD71 expressing cells (HepG2 and Huh7) was determined. This revealed strong cell killing at low concentrations of CD71-Saporin (IC50 = 1 pM) in both cell lines, whereas equivalent concentrations CD71-Saporin, CD71-Saporin + 1000 nM trivalent-GalNAc or CD71-Saporin + 4000 nM trivalent-GalNAc could only induce cell killing at high concentrations CD71-Saporin (IC50 = 100 pM) in both cell lines (Figure 10).

All this shows that trivalent-GalNAc-SO1861 in combination with low concentrations of CD71-Saporin effectively induce cell killing in ASGPR1/CD71 expressing cells. Thus trivalent-GalNAc-SO1861 effectively induces endosomal escape of a protein toxin in ASGPR1 expressing cells.

**Example 1A.** *CD71-saporin + concentration series of trivalent GalNAc-L-SO1861, trivalent GalNAc-(L-SO1861)4, SO1861-EMCH*

Trivalent GalNAc was produced (Figure 1A-C and Figure 8A-G) and SO1861-EMCH was conjugated (labile, in a similar manner as described in Figure 2A-B and Figure 4 for underivatised SO1861, wherein SPT and SPT001 are synonymous to saponin SO1861) to trivalent-GalNAc, with a DAR 1, (trivalent-GalNAc-SO1861; (GN)3-SPT)) or with a DAR 4 by first coupling the SO1861-EMCH to a dendron (trivalent-GalNAc-dendron$(SO1861)_4$; (GN)3-dSPT4; Figure 23D-E). Again, SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. The 'trivalent-GalNAc' as depicted in Figure 1A-C and Figure 8A-G is a typical $(GalNAc)_3$Tris conjugate suitable for coupling to an effector molecule or to saponin. As a control, SO1861-EMCH was also conjugated (labile) to monovalent-GalNAc (Figure 3), with a DAR 1 or with a DAR 4 (using a dendron), (GalNAc-SO1861 and GalNAc-$(SO1861)_4$). Primary hepatocytes, which highly express ASGPR1 ($ASGPR1^{high}$), were treated with a concentration range of trivalent-GalNAc-L-SO1861, SO1861-EMCH or $(GN)_3$-dSPT4 in the presence of 10 pM CD71-saporin (monoclonal antibody OKT-9 targeting CD71 conjugated to the protein toxin saporin). Cell treatment in absence of CD71-saporin show that the trivalent-GalNAc-L-SO1861 as single compound is not toxic up to at least 1.000 nM (Figure 18A; relative cell viability). Cell viability of cells treated with the combination of anti CD71 MoAb-saporin ('MoAb' = monoclonal antibody) and trivalent GalNAc conjugated with SO1861 (either DAR 1, or DAR 4) is about a factor 100 lower compared to cell viability of cells treated with anti CD71 MoAb-saporin (CD71-SPRN) combined with SO1861-EMCH (SPT-EMCH) (Figure 18A).

All this shows that trivalent-GalNAc-SO1861 and trivalent-GalNAc-(SO1861)4 in combination with low concentrations of CD71-Saporin effectively induces cell killing in ASGPR1/CD71 expressing cells at concentrations of CD71-Saporin ineffective in absence of SO1861. Thus, trivalent-GalNAc-SO1861 effectively induces endosomal escape of a protein toxin in ASGPR1 expressing cells. Thus, SPT001 and GalNAc targeted SPT001 (DAR1) both increase potency of industry standard GalNAc targeted ASO; SO1861-EMCH with about a factor 10, and both (GN)3-SPT and (GN)3-dSPT4 about 100x, when cell viability of liver cells is considered.

**Example 1B.** *Trivalent GalNAc conjugated with ApoB antisense oligonucleotide + trivalent GalNAc-L-SO1861, or a concentration series of conjugate trivalent GalNAc-(L-SO1861)-ApoB antisense oligonucleotide* (a saponin-oligonucleotide-ASGPR ligand conjugate; Figure 22A-C)

Trivalent GalNAc was produced (Figure 1A-C and Figure 8A-G) and SO1861-EMCH was conjugated (labile, in a similar manner as described in Figure 2A-B and Figure 4 for underivatised SO1861, wherein SPT and SPT001 are synonymous to saponin SO1861) to trivalent-GalNAc, with a DAR 1, (trivalent-GalNAc-SO1861; $(GalNAc)_3$-SPT001); Figure 19C). Again, SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. Also, the covalent conjugate of trivalent GalNAc coupled to both the ApoB antisense BNA oligonucleotide ApoBBNA (SEQ ID NO: 2) and a dendron comprising four covalently coupled SO1861-EMCH, was prepared (Figure 20E). See for the outlines for preparing the conjugates also the section **'Trivalent-GalNAc-SO1861 and GalNAc-SO1861 synthesis (Figure 2A-B, 4)'** and the section **'Trivalent GalNAc-BNA oligo synthesis (Figure 5 A-B; Figure 6A-B, Figure 7)'** and the sections **'Trivalent GalNAc-S-ApoB (or HSP27) BNA oligo synthesis'** and **'Trivalent GalNAc-L-ApoB (or HSP27) BNA oligo synthesis'** and the section **'Dendron(-L-SO1861)$_4$-trivalent GalNAc and dendron(-L-SO1861)$_8$-trivalent GalNAc synthesis'** and **'Dendron(-L-SO1861)$_4$-trivalent GalNAc synthesis'** and **'Dendron(-L-SO1861)$_4$-L-BNA oligo-trivalent GalNAc and Dendron(-L-SO1861)$_8$-L-BNA oligo-trivalent GalNAc synthesis'** and section **'Dendron(-L-SO1861)$_4$-L-ApoB BNA oligo-trivalent GalNAc synthesis',** here below.

Primary hepatocytes, which highly express ASGPR1 ($ASGPR1^{high}$) were treated with a concentration range of trivalent-GalNAc-*Apo*BBNA ($(GalNAc)_3$-ApoB), either or not in the presence of 300 nM trivalent-GalNAc-SO1861 ($(GalNAc)_3$-SPT001) (Figure 18B). Comparing cell treatment in absence and presence of $(GalNAc)_3$-SPT001 show that

the trivalent-GalNAc-L-SO1861 is highly effective in increasing the potency of the ASO to reduce the *apoB* gene expression in the primary hepatocytes compared to *apoB* gene expression in cells treated with $(GalNAc)_3$-ApoB only (Figure 18B).

All this shows that trivalent-GalNAc-SO1861 in combination with $(GalNAc)_3$-ApoB effectively improves silencing of *apoB* gene expression in ASGPR1 expressing cells. Thus trivalent-GalNAc-SO1861 effectively induces endosomal escape of a BNA in ASGPR1 expressing cells.

In addition, the primary hepatocytes were contacted with a concentration series of a saponin-oligonucleotide-ASGPR ligand conjugate, *i.e.* trivalent GalNAc conjugated with both SO1861 (DAR is 4) and ApoB BNA (SEQ ID NO: 2) ($(GalNAc)_3$-$dSPT_4$-ApoB; Figure 24D), and apoB gene expression was assessed (Figure 18B and Figure 20A). The apoB gene silencing potency of the $(GalNAc)_3$-$dSPT_4$-ApoB is about a factor 100 higher compared with the $(GalNAc)_3$-ApoB conjugate lacking the covalently linked saponin.

**Example 1C.** *Trivalent GalNAc conjugated with SO1861, or a concentration series of conjugate trivalent GalNAc-(L-SO1861) in the presence of a fixed dose of OKT-9 anti-CD71 monoclonal antibody*

Trivalent GalNAc was produced (Figure 1A-B and Figure 8A-G) and SO1861-EMCH was conjugated (labile, in a similar manner as described in Figure 2A-B and Figure 4 for underivatised SO1861, wherein SPT and SPT001 are synonymous to saponin SO1861) to trivalent-GalNAc, with a DAR 1, (trivalent-GalNAc-SO1861; $(GalNAc)_3$-SPT001); Figure 19C). Again, SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. See for the outline for preparing the conjugate also the section **'Trivalent-GalNAc-SO1861 and GalNAc-SO1861 synthesis (Figure 2A-B, Figure 4)'** here below.

Primary hepatocytes which highly express ASGPR1 ($ASGPR1^{high}$) and Huh7 hepatocyte cell-line ($ASGPR1^{+1-}$ expression) were treated with a concentration range of trivalent-GalNAc-SO1861 (TrivalentGalNAc-SPT001), either in the absence or in the presence of 10 pM CD71-saporin (monoclonal antibody OKT-9 targeting CD71 conjugated to the protein toxin saporin; Figure 19D). Cell treatment in absence of CD71-saporin show that the trivalent-GalNAc-SO1861 as single compound is not toxic up to at least 1000 nM (Figure 19A, 19B; relative cell viability). Cell viability of cells treated with the combination of anti-CD71 MoAb-saporin and a low nM dose of trivalent GalNAc conjugated with SO1861 (DAR 1; Trivalent GalNAc-SPT001) is highly effective in killing the primary hepatocytes compared to cell viability of Huh7 cells treated with the same dose of anti-CD71 MoAb-saporin (CD71-saporin) combined with the same dose of trivalentGalNAc-SPT001 (Figure 19A and 19B).

All this shows that low dose of trivalent-GalNAc-SO1861 in combination with a low concentration of CD71-Saporin effectively induce cell killing in ASGPR1 expressing cells, whereas neither compound induces killing in absence of the other. Thus, trivalent-GalNAc-SO1861 effectively induces endosomal escape of a protein toxin in ASGPR1 expressing cells. Thus, low nM TrivalentGalNAc-SPT001 enhances cytoplasmic delivery of low pM ADC anti-CD71 MoAb-saporin in primary hepatocytes; and hepatocyte cell lines with low ASGPR1 expression (Huh7) are not responsive at low nM concentrations of TrivalentGalNAc-SPT001.

**Example 1D.** *Trivalent GalNAc conjugated with ApoB antisense oligonucleotide + trivalent GalNAc-L-SO1861, or a concentration series of conjugate trivalent GalNAc-(L-SO1861)-ApoB antisense oligonucleotide* (a saponin-oligonucleotide-ASGPR ligand conjugate; Figure 22A-C, TrivalentGalNAc-*ApoB*BNA-SPT001)

Trivalent GalNAc was produced and SO1861-EMCH was conjugated to trivalent-GalNAc, with a DAR 1, as described in Example 1B (trivalent-GalNAc-SO1861; (GalNAc)3-SPT001); Figure 19C). Also, the covalent conjugate of trivalent GalNAc coupled to both, the *ApoB* antisense BNA oligonucleotide ApoBBNA (SEQ ID NO: 2) and SO1861-EMCH, was prepared (Figure 20D). In addition, the conjugate of trivalent GalNAc and the *ApoB* antisense BNA oligonucleotide *Apo*BBNA (SEQ ID NO: 2) was prepared (Figure 20C). See for the outlines for preparing the conjugates also the section **'Trivalent-GalNAc-SO1861 and GalNAc-SO1861 synthesis (Figure 2A-B, Figure 4)'** and the section **'Trivalent GalNAc-BNA oligo synthesis (Figure 5A, 5B; Figure 6A-B, Figure 7)'** and the sections **'Trivalent GalNAc-S-ApoB (or HSP27) BNA oligo synthesis'** and **'Trivalent GalNAc-L-ApoB (or HSP27) BNA oligo synthesis'** and the section **'Dendron(-L-SO1861)$_4$-trivalent GalNAc and dendron(-L-SO1861)$_8$-trivalent GalNAc synthesis'** and **'Dendron(-L-SO1861)$_4$-trivalent GalNAc synthesis'** and **'Dendron(-L-SO1861)$_4$-L-BNA oligo-trivalent GalNAc and Dendron(-L-SO1861)$_8$-L-BNA oligo-trivalent GalNAc synthesis'** and section **'Dendron(-L-SO1861)$_4$-L-ApoB BNA oligo-trivalent GalNAc synthesis',** here below (Figure 19E; Figure 20E; Figure 23, Figure 24).

Primary hepatocytes which highly express ASGPR1 ($ASGPR1^{high}$) and Huh7 hepatocyte cell line (low ASGPR1 expression) were treated with a concentration range of trivalent-GalNAc-*Apo*BBNA (TrivalentGalNAc-*Apo*BBNA), either in absence or presence of 300 nM trivalent-GalNAc-SO1861 (TrivalentGalNAc-SPT001) (Figure 20A and B). Cell treatment in absence of TrivalentGalNAc-SPT001 show that the TrivalentGalNAc-SPT001 is highly effective (~factor

100 more potent) in reducing the *apoB* mRNA expression in the primary hepatocytes compared to *apoB* mRNA expression in the primary liver cells treated with TrivalentGalNAc-*ApoB*BNA only (Figure 20A). Since the Huh7 cells express very low levels of ASGPR1, neither TrivalentGalNAc-*Apo*BBNA, nor the combination of TrivalentGalNAc-ApoBBNA and TrivalentGalNAc-SPT001 induce lowering of the *apoB* mRNA expression in these liver cells.

All this shows that TrivalentGalNAc-SPT001 in combination with TrivalentGalNAc-*ApoB*BNA effectively improves silencing of *apoB* mRNA expression in ASGPR1 expressing cells over control conditions without TrivalentGalNAc-SPT001 present. Thus, TrivalentGalNAc-SPT001 effectively induces endosomal escape of a BNA in ASGPR1 expressing cells.

In addition, the primary hepatocytes and the liver cells of cell line Huh7 were contacted with a concentration series of a saponin-oligonucleotide-ASGPR ligand conjugate, *i.e.* trivalent GalNAc conjugated with both SO1861 (DAR is 4, in a dendron with four SO1861 molecules) and ApoB BNA (SEQ ID NO: 2) ((GalNAc)3-dSPT4-ApoB (Figure 20E), or TrivalentGalNAc-*Apo*BBNA-dendron$(SO1861)_4$), and *apoB* mRNA expression was assessed (Figure 20A and B). The *apoB* mRNA expression silencing potency of the ((GalNAc)3-dSPT4-ApoB is about a factor 100 higher compared with the TrivalentGalNAc-*ApoB*BNA conjugate lacking the covalently linked SO1861.

Primary hepatocytes which highly express ASGPR1 ($ASGPR1^{high}$) and Huh7 hepatocyte cell-line (low ASGPR1 expression) were treated with a concentration range of trivalent-GalNAc-*Apo*BBNA (TrivalentGalNAc-*Apo*BBNA), either in the absence or presence of 300 nM trivalent-GalNAc-SO1861 (TrivalentGalNAc-SPT001) (Figure 21A and B) and apoB protein expression was assessed and compared. Cell treatment in absence of TrivalentGalNAc-SPT001 show that the TrivalentGalNAc-SPT001 is highly effective (with a factor of over 1000 more potency) in reducing the apoB protein expression in the primary hepatocytes compared to apoB protein expression in the primary liver cells treated with TrivalentGalNAc-*ApoB*BNA only (Figure 21A). Since the Huh7 cells express very low levels of ASGPR1, neither TrivalentGalNAc-*ApoB*BNA, nor the combination of TrivalentGalNAc-*ApoB*BNA and TrivalentGalNAc-SPT001 induced marked lowering of the apoB protein expression in these liver cells (Figure 21B).

All this shows that TrivalentGalNAc-SPT001 in combination with TrivalentGalNAc-*ApoB*BNA effectively improves silencing of apoB protein expression by TrivalentGalNAc-*ApoB*BNA in high ASGPR1 expressing cells. Thus, TrivalentGalNAc-SPT001 effectively induces endosomal escape of a BNA in high ASGPR1 expressing cells.

Thus, low nM trivalentGalNAc-*Apo*BBNA + trivalentGalNAc-SPT001 can enhance endosomal escape and cytoplasmic delivery of *Apo*BBNA resulting in *apoB* mRNA silencing in primary hepatocytes; low nM ((GalNAc)3-dSPT4-ApoB can enhance endosomal escape and cytoplasmic delivery of *Apo*BBNA resulting in *apoB* mRNA silencing in primary hepatocytes; hepatocytes that have very low ASGPR1 expression are not responsive for both therapies; low nM trivalentGalNAc-*Apo*BBNA + trivalentGalNAc-SPT001 can enhance endosomal escape and cytoplasmic delivery of *Apo*BBNA resulting in apoB protein decay in primary hepatocytes; low nM ((GalNAc)3-dSPT4-ApoB can enhance endosomal escape and cytoplasmic delivery of *Apo*BBNA resulting in apoB protein decay in primary hepatocytes; and cells with very low ASGPR1 expression are not responsive for both therapies when apoB expression is considered.

**Example 2** *trivalent-GalNAc-L/S-BNA + SO1861-EMCH*

HSP27BNA was conjugated to trivalent-GalNAc (Figure 5A, B, Figure 6, Figure 7) and HepG2 ($ASGPR1^+$) cells or Huh7 ($ASGPR1^{+/-}$) were treated with a range of concentrations of trivalent-GalNAc-L-HSP27BNA (labile conjugation, Figure 5A-B, Figure 6A-B) or trivalent-GalNAc-S-HSP27BNA (stable conjugation, Figure 7) in combination with a fixed concentration of 4000 nM SO1861-EMCH. HSP27 gene silencing in HepG2 and Huh7 cells was determined. Only in combination with 4000 nM SO1861-EMCH, effective gene silencing was observed at low concentrations of trivalent-GalNAc-L-HSP27BNA (HepG2: IC50 = 0.1 nM; Huh7: IC50 = 3 nM) or trivalent-GalNAc-S-HSP27BNA (HepG2: IC50 = 0.1 nM; Huh7: IC50 = 3 nM) in both HEPG2 and Huh7 cells, whereas equivalent concentrations trivalent-GalNAc-L-HSP27BNA (HepG2/Huh7: IC50> 2000 nM) or trivalent-GalNAc-S-HSP27BNA (HepG2/Huh7: IC50> 2000 nM) did not show gene silencing activity in HepG2 and Huh7 cell lines (Figure 11).

Next, ApoBBNA was conjugated in a similar manner (Figure 5 A-B, Figure 6A-B, Figure 7) to trivalent-GalNAc and HepG2 ($ASGPR1^+$) cells or Huh7 ($ASGPR1^{+/-}$) were treated with a range of concentrations of trivalent-GalNAc-L-ApoBBNA (labile conjugation, Figure 5A-B; Figure 6A-B, Figure 12, Figure 13) or trivalent GalNAc-S-ApoBBNA (stable conjugation, Figure 7, Figure 14, Figure 15) or a trivalent GalNAc-L-$ApoB_{scrambled}$BNA or trivalent GalNAc-S-$ApoB_{scrambled}$BNA (where the scrambled ApoBBNA is an antisense scrambled oligonucleotide sequence that does not bind the *apoB* mRNA; off-target control BNA) in combination with a fixed concentration of 4000 nM SO1861-EMCH. ApoB gene silencing in HepG2 ($ASGPR1^+$) cells or Huh7 ($ASGPR1^{+/-}$) was determined. Only in combination with 4000 nM SO1861-EMCH effective gene silencing was observed at low concentrations of trivalent-GalNAc-L-ApoBBNA (IC50 = 10 nM) or trivalent-GalNAc-S-ApoBBNA (IC50 = 10 nM) in HepG2 cells, whereas equivalent concentrations trivalent-GalNAc-L-ApoBBNA (IC50 > 2000 nM) or trivalent-GalNAc-S-ApoBBNA (IC50 > 2000 nM) did not show gene silencing activity in HepG2 cells (Figure 12, Figure 14). The treatments did not affect the viability of the cell lines ((Figure 13, Figure 15). The 'L' refers to a 'labile' linker, which indicates that the linker is cleaved in the cell, *i.e.* at pH as apparent in the endosome and the lysosome.

In contrast, the 'S' refers to a 'stable' linker, which indicates that the linker is not cleaved in the cell, *i.e.* at pH as apparent in the endosome and the lysosome. Thus, a conjugate comprising a labile bond between any two molecules forming the conjugate or comprised by the conjugate will be cleaved at a position in the labile linker, therewith dissociating the two linked or bound molecules. An example is the cleavage of a hydrazone bond under acidic conditions as apparent in the endosome and lysosome of mammalian cells such as human cells.

All this shows that SO1861-EMCH can enhance endosomal escape and target gene silencing of trivalent-GalNAc-L-BNA or trivalent-GalNAc-S-BNA for two independent gene targets. The gene silencing is more efficient in HepG2 cells compared to Huh7 cells, suggesting that higher levels of ASGPR1 expression at the plasma membrane of the cell facilitates increased uptake of the GalNAc-BNA conjugates.

**Example 3** *trivalent-GalNAc-L/S-BNA + trivalent-GalNAc-L-SO1861*

SO1861-EMCH (also referred to as SPT) was conjugated (labile, in a similar manner as described in Figure 2A-B and Figure 4 for underivatised SO1861) to trivalent-GalNAc ((GalNAc)3 or (GN)3), with a DAR 1, (trivalent-GalNAc-SO1861). HepG2 (ASGPR1$^+$) cells or Huh7 (ASGPR1$^{+/-}$) cells were treated with a range of concentrations of trivalent-GalNAc-L-ApoBBNA (labile conjugation Figure 16, Figure 17), trivalent-GalNAc-S-ApoBBNA (stable conjugation (Figure 16, Figure 17) or the scrambled (Scr.) off-target control conjugates (trivalent-GalNAc-L-ApoB$_{scrambled}$BNA or trivalent-GalNAc-S-ApoB$_{scrambled}$BNA) in combination with a fixed concentration of 4000 nM trivalent-GalNAc-L-SO1861 (DAR1). ApoB gene silencing in HepG2 (ASGPR1$^+$) cells and Huh7 (ASGPR1$^{+/-}$) cells was determined. Only in combination with 4000 nM trivalent-GalNAc-L-SO1861 effective gene silencing was observed at low concentrations of trivalent-GalNAc-L-ApoBBNA (IC50 = 50 nM) or trivalent-GalNAc-S-ApoBBNA (IC50 = 50 nM) in HepG2 cells (ASGPR1$^+$). Both combination treatments showed less effective gene silencing in Huh7 cells (ASGPR1$^{+/-}$) (trivalent-GalNAc-L-ApoBBNA: IC50 = 700 nM; trivalent-GalNAc-S-ApoBBNA: IC50 = 700 nM). Equivalent concentrations of trivalent-GalNAc-L-ApoBBNA (HepG2/Huh7: IC50> 1000nM) or trivalent-GalNAc-S-ApoBBNA (HepG2/Huh7: IC50 > 1000 nM) did not show gene silencing activity in HepG2 and Huh7 cell lines, nor did 4000 nM trivalent-GalNAc-L-SO1861 induce/enhance gene silencing, of scrambled trivalent-GalNAc-L-ApoB$_{scrambled}$BNA + (HepG2/Huh7: IC50 > 1000 nM), or scrambled trivalent-GalNAc-S-ApoB$_{scrambled}$BNA (HepG2/Huh7: IC50 > 1000 nM) (Figure 16, Figure 17). The treatments did not affect the viability of the cell lines (Figure 16 C, D).

All this shows that in combination with trivalent-GalNAc-L-SO1861, very low concentrations of trivalent-GalNAc-L-HSP27BNA or trivalent-GalNAc-S-HSP27BNA effectively induce gene silencing in ASGPR1 expressing cells, and again stronger effects are seen in cells with a higher ASGPR1 expression.

**Materials and methods**

**Abbreviations**

| | |
|---|---|
| AEM | *N*-(2-Aminoethyl)maleimide trifluoroacetate salt |
| AMPD | 2-Amino-2-methyl-1,3-propanediol |
| BOP | (Benzotriazol-1-yloxy)tris(dimethylamino)phosphonium hexafluorophosphate |
| DIPEA | N,N-diisopropylethylamine |
| DMF | N,N-dimethylformamide |
| EDCI.HCl | 1-Ethyl-3-(3-dimethylaminopropyl)carbodiimide hydrochloride |
| EMCH.TFA | N-(ε-maleimidocaproic acid) hydrazide, trifluoroacetic acid salt |
| HATU | 1-[Bis(dimethylamino)methylene]-1H-1,2,3-triazolo[4,5-b]pyridinium 3-oxid hexafluorophosphate |
| min | minutes |
| NMM | 4-Methylmorpholine |
| r.t. | retention time |
| TCEP | tris(2-carboxyethyl)phosphine hydrochloride |
| Temp | temperature |
| TFA | trifluoroacetic acid |

Analytical methods

LC-MS method 1

Apparatus: Waters IClass; Bin. Pump: UPIBSM, SM: UPISMFTN with SO; UPCMA, PDA: UPPDATC, 210-320

nm, SQD: ACQ-SQD2 ESI, mass ranges depending on the molecular weight of the product: neg or neg/pos within in a range of 1500-2400 or 2000-3000; ELSD: gas pressure 40 psi, drift tube temp: 50°C; column: Acquity C18, 50×2.1 mm, 1.7 µm Temp: 60°C, Flow: 0.6 mL/min, lin. Gradient depending on the polarity of the product:

$$^{A}t_{0} = 2\%\ A,\ t_{5.0min} = 50\%\ A,\ t_{6.0min} = 98\%\ A$$

$$^{B}t_{0} = 2\%\ A,\ t_{5.0min} = 98\%\ A,\ t_{6.0min} = 98\%\ A$$

Posttime: 1.0 min, Eluent A: acetonitrile, Eluent B: 10 mM ammonium bicarbonate in water (pH=9.5).

LC-MS method 2

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50 °C; column: Waters XSelect™ CSH C18, 50×2.1 mm, 2.5 µm, Temp: 25°C, Flow: 0.5 mL/min, Gradient: $t_{0min}$ = 5% A, $t_{2.0min}$ = 98% A, $t_{2.7min}$ = 98% A, Posttime: 0.3 min, Eluent A: acetonitrile, Eluent B: 10 mM ammonium bicarbonate in water (pH=9.5).

LC-MS method 3

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product pos/neg 105-800, 500-1200 or 1500-2500; ELSD: gas pressure 40 psi, drift tube temp: 50 °C; column: Waters XSelect™ CSH C18, 50×2.1mm, 2.5µm, Temp: 40°C, Flow: 0.5 mL/min, Gradient: $t_{0min}$ = 5% A, $t_{2.0min}$ = 98% A, $t_{2.7min}$ = 98% A, Posttime: 0.3 min, Eluent A: 0.1% formic acid in acetonitrile, Eluent B: 0.1% formic acid in water.

LC-MS method 4

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50 °C column: Waters Acquity Shield RP18, 50×2.1mm, 1.7µm, Temp: 25°C, Flow: 0.5 mL/min, Gradient: $t_{0min}$ = 5% A, $t_{2.0min}$ = 98% A, $t_{2.7min}$ = 98% A, Posttime: 0.3 min, Eluent A: acetonitrile, Eluent B: 10 mM ammonium bicarbonate in water (pH=9.5).

Preparative methods

Preparative MP-LC method 1

Instrument type: Reveleris™ prep MPLC; column: Waters XSelect™ CSH C18 (145×25 mm, 10µm); Flow: 40 mL/min; Column temp: room temperature; Eluent A: 10 mM ammonium bicarbonate in water pH = 9.0); Eluent B: 99% acetonitrile + 1% 10 mM ammonium bicarbonate in water; Gradient:

$$^{A}t_{0min} = 5\%\ B,\ t_{1min} = 5\%\ B,\ t_{2min} = 10\%\ B,\ t_{17min} = 50\%\ B,\ t_{18min} = 100\%\ B,\ t_{23min} = 100\%\ B$$

$$^{A}t_{0min} = 5\%\ B,\ t_{1min} = 5\%\ B,\ t_{2min} = 20\%\ B,\ t_{17min} = 60\%\ B,\ t_{18min} = 100\%\ B,\ t_{23min} = 100\%\ B$$

$$^{B}t_{0min} = 2\%\ B,\ t_{1min} = 2\%\ B,\ t_{2min} = 2\%\ B,\ t_{17min} = 30\%\ B,\ t_{18min} = 100\%\ B,\ t_{23min} = 100\%\ B$$

$$^{C}t_{0min} = 5\%\ B,\ t_{1min} = 5\%\ B,\ t_{2min} = 10\%\ B,\ t_{17min} = 50\%\ B,\ t_{18min} = 100\%\ B,\ t_{23min} = 100\%\ B$$

$$^{D}t_{0min} = 5\%\ B,\ t_{1min} = 5\%\ B,\ t_{2min} = 5\%\ B,\ t_{17min} = 40\%\ B,\ t_{18min} = 100\%\ B,\ t_{23min} = 100\%\ B$$

; Detection UV: 210, 235, 254 nm and ELSD.

Preparative MP-LC method 2

Instrument type: Reveleris™ prep MPLC; Column: Phenomenex LUNA C18(3) (150×25 mm, 10μm); Flow: 40 mL/min; Column temp: room temperature; Eluent A: 0.1% (v/v) Formic acid in water, Eluent B: 0.1% (v/v) Formic acid in acetonitrile; Gradient:

$$^{A}t_{0min} = 5\%\ B,\ t_{1min} = 5\%\ B,\ t_{2min} = 20\%\ B,\ t_{17min} = 60\%\ B,\ t_{18min} = 100\%\ B,\ t_{23min} = 100\%\ B$$

; Detection UV : 210, 235, 254 nm and ELSD.

Preparative LC-MS method 3

MS instrument type: Agilent Technologies G6130B Quadrupole; HPLC instrument type: Agilent Technologies 1290 preparative LC; Column: Waters XSelect™ CSH (C18, 150×19mm, 10μm); Flow: 25 ml/min; Column temp: room temperature; Eluent A: 100% acetonitrile; Eluent B: 10 mM ammonium bicarbonate in water pH=9.0; Gradient:

$$^{A}t_{0} = 20\%\ A,\ t_{2.5min} = 20\%\ A,\ t_{11min} = 60\%\ A,\ t_{13min} = 100\%\ A,\ t_{17min} = 100\%\ A$$

$$^{B}t_{0} = 5\%\ A,\ t_{2.5min} = 5\%\ A,\ t_{11min} = 40\%\ A,\ t_{13min} = 100\%\ A,\ t_{17min} = 100\%\ A$$

; Detection: DAD (210 nm); Detection: MSD (ESI pos/neg) mass range: 100 - 800; Fraction collection based on DAD.

Preparative LC-MS method 4

MS instrument type: Agilent Technologies G6130B Quadrupole; HPLC instrument type: Agilent Technologies 1290 preparative LC; Column: Waters XBridge Protein (C4, 150×19 mm, 10 μm); Flow: 25 ml/min; Column temp: room temperature; Eluent A: 100% acetonitrile; Eluent B: 10 mM ammonium bicarbonate in water pH=9.0; Gradient:

$$^{A}t_{0} = 2\%\ A,\ t_{2.5min} = 2\%\ A,\ t_{11min} = 30\%\ A,\ t_{13min} = 100\%\ A,\ t_{17min} = 100\%\ A$$

$$^{B}t_{0} = 10\%\ A,\ t_{2.5min} = 10\%\ A,\ t_{11min} = 50\%\ A,\ t_{13min} = 100\%\ A,\ t_{17min} = 100\%\ A$$

$$^{C}t_{0} = 5\%\ A,\ t_{2.5min} = 5\%\ A,\ t_{11min} = 40\%\ A,\ t_{13min} = 100\%\ A,\ t_{17min} = 100\%\ A$$

; Detection: DAD (210 nm); Detection: MSD (ESI pos/neg) mass range: 100 - 800; Fraction collection based on DAD

Flash chromatography

Grace Reveleris X2® C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50 bar (725 psi); Detection: UV 200-400 nm, combination of up to 4 UV signals and scan of entire UV range, ELSD; Column sizes: 4-330g on instrument, luer type, 750g up to 3000g with optional holder.

**SO1861-EMCH synthesis (SO1861-EMCH is also referred to as SO1861-Ald-EMCH and SPT-EMCH)**

To SO1861 (121 mg, 0.065 mmol) and EMCH.TFA (110 mg, 0.325 mmol) was added methanol (extra dry, 3.00 mL) and TFA (0.020 mL, 0.260 mmol). The reaction mixture stirred at room temperature. After 1.5 hours the reaction mixture was subjected to preparative MP-LC. Fractions corresponding to the product were immediately pooled together,

frozen and lyophilized overnight to give the title compound (120 mg, 90%) as a white fluffy solid. Purity based on LC-MS 96%.

**Trivalent GalNAc-azide synthesis**

Intermediate 1:

**tert-butyl 1-azido-17,17-bis((3-(tert-butoxy)-3-oxopropoxy)methyl)-15-oxo-3,6,9,12,19-pentaoxa-16-azadocosan-22-oate**

To di-tert-butyl 3,3'-((2-amino-2-((3-(tert-butoxy)-3-oxopropoxy)methyl)propane-1,3-diyl)bis(oxy))dipropionate (1.27 g, 2.51 mmol) was added a solution of 3-Azido(peg4)propionic acid N-hydroxysuccinimide ester (977 mg, 2.51 mmol) in DMF (10 mL). Next, DIPEA (657 μL, 3.77 mmol) was added and the reaction mixture was stirred overnight at room temperature. The reaction mixture was evaporated *in vacuo* and the residue was dissolved in ethyl acetate (100 mL). The resulting solution was washed with 0.5 N potassium bisulphate solution (2 × 100 mL) and brine (100 mL), dried over $Na_2SO_4$, filtered and evaporated in vacuo. The residue was purified by flash chromatography (DCM - 10% methanol in DCM (v/v) gradient 100:0 rising to 0:100) to give the title compound (1.27 g, 65%) as a colorless oil. Purity based on LC-MS 100% (ELSD).

LRMS (m/z): 780 $[M+1]^{1+}$

LC-MS r.t. (min): 2.10[2]

Intermediate 2:

**1-azido-17,17-bis((2-carboxyethoxy)methyl)-15-oxo-3,6,9,12,19-pentaoxa-16-azadocosan-22-oic acid**

To a solution of tert-butyl 1-azido-17,17-bis((3-(tert-butoxy)-3-oxopropoxy)methyl)-15-oxo-3,6,9,12,19-pentaoxa-16-azadocosan-22-oate (1.27 g, 1.63 mmol) in DCM (5.0 mL) was added TFA (5.0 mL, 65 mmol). The reaction mixture was stirred at room temperature. After 1.5 hours the reaction mixture was evaporated *in vacuo,* co-evaporated with toluene (3 × 10 mL) and DCM (3 × 10 mL) to give the crude title product as a colorless oil.
LRMS (m/z): 611 $[M+1]^{1+}$

Intermediate 3:

**di-tert-butyl (10-(1-azido-3,6,9,12-tetraoxapentadecan-15-amido)-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate**

1-azido-17,17-bis((2-carboxyethoxy)methyl)-15-oxo-3,6,9,12,19-pentaoxa-16-azadocosan-22-oic acid (997 mg, 1.63 mmol), Oxyma Pure (1.04 g, 7.35 mmol) and EDCI.HCl (1.17 g, 6.12 mmol) were dissolved in DMF (10.0 mL). Next, DIPEA (1.99 mL, 11.4 mmol) was added, followed directly by the addition of a solution of N-BOC-1,3-propanediamine (1.07 g, 6.12 mmol) in DMF (10.0 mL). The reaction mixture was stirred overnight at room temperature. The reaction mixture was evaporated *in vacuo* and the residue was dissolved in ethyl acetate (100 mL). The resulting solution was washed with 0.5 N potassium bisulphate solution (100 mL), saturated sodium bicarbonate solution (2 × 100 mL) and brine (100 mL), dried over $Na_2SO_4$, filtered and evaporated *in vacuo.* The residue was purified by flash chromatography (DCM - 10% methanol in DCM (v/v) gradient 0:100 rising to 100:0, staying at 100:0 until the product eluted) to give the title compound (1.16 g, 66%) as a yellowish viscous oil. LC-MS 99% (ELSD).

LRMS (m/z): 1080 $[M+1]^{1+}$
LC-MS r.t. (min): 1.51[3]

Intermediate 4:

**3,3'-((2-((3-((3-aminopropyl)amino)-3-oxopropoxy)methyl)-2-(1-azido-3,6,9,12-tetraoxapentadecan-15-amido) propane-1,3-diyl)bis(oxy))bis(N-(3-aminopropyl)propanamide) tris(2,2,2-trifluoroacetate) synthesis**

To a solution of di-tert-butyl (10-(1-azido-3,6,9,12-tetraoxapentadecan-15-amido)-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate (1.16 g,

1.08 mmol) in DCM (10 mL) was added TFA (10 mL, 131 mmol). The reaction mixture was stirred at room temperature. After 2 hours the reaction mixture was evaporated *in vacuo,* co-evaporated with toluene (3 × 10 mL) and DCM (3 × 10 mL) to give the crude title product as a yellowish viscous oil.
LRMS (m/z): 260 $[M+3]^{3+}$, 390 $[M+2]^{2+}$, 780 $[M+1]^{1+}$,

Intermediate 5:

**(2R,3R,4R,5R,6R)-5-acetamido-2-(acetoxymethyl)-6-((5-((2,5-dioxopyrrolidin-1-yl)oxy)-5-oxopentyl)oxy)tetrahydro-2H-pyran-3,4-diyl diacetate**

5-(((2R,3R,4R,5R,6R)-3-acetamido-4,5-diacetoxy-6-(acetoxymethyl)tetrahydro-2H-pyran-2-yl)oxy)pentanoic acid (obtain according J. Am. Chem Soc., 2014, 136, 16958-16961, 3.00 g, 6.70 mmol) and *N*-Hydroxysuccinimide (926 mg, 8.05 mmol) were dissolved in DCM (50 mL). Next, EDCI.HCl (1.54 g, 8.05 mmol) and 4-(Dimethylamino)pyridine (82 mg, 0.67 mmol) were added and the reaction mixture was stirred overnight at room temperature. The reaction mixture was diluted with DCM and the resulting solution was washed with 0.5 N potassium bisulphate solution (150 mL), saturated sodium bicarbonate solution (150 mL) and brine (150 mL), dried over $Na_2SO_4$, filtered and evaporated *in vacuo* to give the title compound (3.60 g, 99%) as a white foam. Purity based on LC-MS 99% (ELSD).

LRMS (m/z): 545 $[M+1]^{1+}$

LC-MS r.t. (min): 1.07[3]

Intermediate 6:

**[(3R,6R)-3,4-bis(acetyloxy)-6-{4-[(3-{3-[2-(1-azido-3,6,9,12-tetraoxapentadecan-15-amido)-3-(2-{[3-(5-{[(2R,5R)-4,5-bis(acetyloxy)-6-[(acetyloxy)methyl]-3-acetamidooxan-2-yl]oxy}pentanam ido)propyl]carbamoyl}ethoxy)-2-[(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)methyl]propoxy]propanamido}propyl)carbamoyl ]butoxy}-5-acetamidooxan-2-yl]methyl acetate**

3,3'-((2-((3-((3-aminopropyl)amino)-3-oxopropoxy)methyl)-2-(1-azido-3,6,9,12-tetraoxapentadecan-15-amido)propane-1,3-diyl)bis(oxy))bis(N-(3-aminopropyl)propanamide) tris(2,2,2-trifluoroacetate) (1.21 g, 1.08 mmol) was dissolved in a mixture of DMF (10 mL) and DIPEA (1.69 mL, 9.70 mmol). Next, (2R,3R,4R,5R,6R)-5-acetamido-2-(acetoxymethyl)-6-((5-((2,5-dioxopyrrolidin-1-yl)oxy)-5-oxopentyl)oxy)tetrahydro-2H-pyran-3,4-diyl diacetate (2.20 g, 4.04 mmol) was added and the reaction mixture was stirred over the weekend at room temperature. Next, the reaction mixture was evaporated *in vacuo* and the residue was purified by flash chromatography (DCM - 30% methanol in DCM (v/v) gradient 0:100 rising to 100:0) to give the title compound (1.84 g, 83%) as a yellowish foam. LC-MS 95% (ELSD).

LRMS (m/z): 2068 $[M+1]^{1+}$

LC-MS r.t. (min): 1.18[3]

Intermediate 7:

**Trivalent GalNAc-azide**

[(3R,6R)-3,4-bis(acetyloxy)-6-{4-[(3-{3-[2-(1-azido-3,6,9,12-tetraoxapentadecan-15-amido)-3-(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)-2-[(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)methyl]propoxy]propanamido}propyl)carbamoyl]butoxy}-5-acetamidooxan-2-yl]methyl acetate (300 mg, 0.145 mmol) was dissolved in a mixture of triethylamine (2.00 mL, 14.4 mmol), methanol (2.00 mL) and water (2.00 mL) and the reaction mixture was stirred at room temperature. After 2 hours the reaction mixture was evaporated *in vacuo.* The residue was purified by preparative MP-LC.[2B] Fractions corresponding to the product were immediately pooled together, frozen and lyophilized overnight to give the title compound (164 mg, 67%) as a white solid. Purity based on LC-MS 97%.

LRMS (m/z): 1688 $[M-1]^{1-}$

LC-MS r.t. (min): 1.99[1A]

**Trivalent-GalNAc-SO1861 and GalNAc-SO1861 synthesis (Figure 2, Figure 4)**

Intermediate 8:

**SO1861-$NH_2$**

SO1861-azide (6.89 mg, 3.20 μmol) was dissolved in mixture of 32 mM potassium carbonate (150 μL, 4.80 μmol) and acetonitrile (150 μL). Directly afterwards, a 1.0 M trimethylphosphine solution in THF (32 μL, 32 μmol) was added and the resulting mixture was shaken for 1 min and left standing at room temperature. After 30 min. the reaction mixture was subjected to preparative MP-LC. Fractions corresponding to the product were immediately pooled together, frozen and lyophilized overnight to give the title compound (5.30 mg, 78%) as a white fluffy solid. Purity based on LC-MS 94%.

LRMS (m/z): 1062 $[M-2]^{2-}$

LC-MS r.t. (min): 2.51[1B]

Intermediate 9:

**SO1861-DBCO**

To SO1861-amine (48.6 mg, 22.9 μmol) and DBCO-NHS (13.0 mg, 32.4 μmol) was added to a solution of DIPEA (5.98 μL, 34.3 μmol) and DMF (2.0 mL). The reaction mixture was shaken for 1 min and left standing at room temperature. After 4 hours the reaction mixture was diluted with a solution of 50 mM sodium bicarbonate (1.00 mL, 50 μmol). The resulting mixture was shaken for 1 min and left standing at room temperature. After 30 min the reaction mixture was subjected to preparative MP-LC.[1B] Fractions corresponding to the product were immediately pooled together, frozen and lyophilized overnight to give the title compound (16.9 mg, 31%) as a white fluffy solid. Purity based on LC-MS 94%.

LRMS (m/z): 2412 $[M-1]^{1-}$

LC-MS r.t. (min): 2.45[1B]

**SO1861-L-trivalent GalNAc synthesis**

SO1861-DBCO (7.50 mg, 3.11 μmol) and trivalent GalNAc-azide (5.31 mg, 3.14 μmol) were dissolved in a mixture of water/acetonitrile (2:1, v/v, 0.90 mL). The reaction mixture was shaken for 1 min and left standing at room temperature. After 2 hours the reaction mixture was subjected to preparative LC-MS.[3B] Fractions corresponding to the product were immediately pooled together, frozen and lyophilized overnight to give the title compound (9.60 mg, 75%) as a white fluffy solid. Purity based on LC-MS 99%.

LRMS (m/z): 2050 $[M-2]^{2-}$

LC-MS r.t. (min): 2.04[1B]

**SO1861-L-GalNAc synthesis**

SO1861-DBCO (1.75 mg, 0.73 μmol) and GalNAc-PEG3-azide (0.82 mg, 2.18 μmol) were dissolved in a mixture of water/acetonitrile (1:1, v/v, 1.00 mL). The reaction mixture was shaken for 1 min and left standing at room temperature. After 2 hours the reaction mixture was subjected to preparative LC-MS.[3B] Fractions corresponding to the product were immediately pooled together, frozen and lyophilized overnight to give the title compound (1.63 mg, 81%) as a white fluffy solid. Purity based on LC-MS 100%.

LRMS (m/z): 2790 $[M-1]^{1-}$

LC-MS r.t. (min): 2.15[1B]

**Trivalent GalNAc-BNA oligo synthesis (Figure 6, 7)**

Intermediate 10:

**4-{2-azatricyclo[10.4.0.0$^{4,9}$]hexadeca-1(12),4(9),5,7,13,15-hexaen-10-yn-2-yl}-N-[2-(2-{2-[2-({4-[(E)-{[6-(2,5-dioxo-2,5-dihydro-1H-pyrrol-1-yl)hexanamido]imino}methyl]phenyl}formamido)ethoxy]ethoxy}ethoxy)ethyl]-4-oxobutanamide**

4-{2-azatricyclo[10.4.0.0$^{4,9}$]hexadeca-1(12),4(9),5,7,13,15-hexaen-10-yn-2-yl}-N-{2-[2-(2-{2-[(4-formylphenyl)formamido]ethoxy}ethoxy)ethoxy]ethyl}-4-oxobutanamide (25.0 mg, 40.9 μmol) and EMCH.TFA (20.8 mg, 61.3 μmol) were dissolved in methanol (extra dry, 2.00 mL). Next, TFA (9.39 μL, 123 μmol) was added. The reaction mixture was shaken for 1 min and left standing overnight at room. The reaction mixture was subjected to preparative MP-LC.[1B] Fractions corresponding to the product were immediately pooled together, frozen and lyophilized overnight to give the title compound (16.2 mg, 48%) as a white solid. Purity based on LC-MS 91%.

LRMS (m/z): 410.2 $[M+2]^{2+}$

LC-MS r.t. (min): 1.41[2]

Intermediate 11:

**Trivalent GalNAc-L-maleimide**

Trivalent GalNAc-azide (20.3 mg, 12.0 μmol) and 4-{2-azatricyclo[10.4.0.0$^{4,9}$]hexadeca-1(12),4(9),5,7,13,15-hexaen-10-yn-2-yl}-N-[2-(2-{2-[2-({4-[(E)-{[6-(2,5-dioxo-2,5-dihydro-1H-pyrrol-1-yl)hexanamido]imino}methyl]phenyl}formamido)ethoxy]ethoxy}ethoxy)ethyl]-4-oxobutanamide (11.8 mg, 14.4 μmol) were dissolved in a mixture of water/-acetonitrile (2:1, v/v, 0.90 mL). The reaction mixture was shaken for 1 min and left standing at room temperature. After 2 hours the reaction mixture was subjected to preparative MP-LC.[2C] Fractions corresponding to the product were immediately pooled together, frozen and lyophilized overnight to give the title compound (25.6 mg, 85%) as a white solid. Purity based on LC-MS 88%.

LRMS (m/z): 2101 $[M-405]^{1-}$, 2304 $[M-202]^{1-}$, 2507 $[M-1]^{1-}$
LC-MS r.t. (min): 1.90[1B] (double peaks due to isomers)

Intermediate 12:

**Trivalent GalNAc-S-maleimide**

Trivalent GalNAc-azide (20.3 mg, 12.0 μmol) and DBCO-maleimide (10.3 mg, 24.0 μmol) were dissolved in a mixture of water/acetonitrile (2:1, v/v, 0.90 mL). The reaction mixture was shaken for 1 min and left standing at room temperature. After 2 hours the reaction mixture was subjected to preparative MP-LC.[2D] Fractions corresponding to the product were immediately pooled together, frozen and lyophilized overnight to give the title compound (22.2 mg, 87%) as a white solid. Purity based on LC-MS 85%. Contains 10% of the hydrolysed maleimide.

LRMS (m/z): 2115 $[M-1]^{1-}$

LC-MS r.t. (min): 1.60[1B] (double peaks due to isomers)

**Trivalent GalNAc-S-ApoB (or HSP27) BNA oligo synthesis**

To ApoB BNA oligo disulfide (5.00 mg, 0.686 μmol) was added a solution of 20 mM ammonium bicarbonate with 2.5 mM TCEP (1.00 mL, 2.5 μmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-S-maleimide (3.02 mg, 1.43 μmol). The reaction mixture was shaken for 1 min and left standing at room temperature. After 1.5 hours the reaction mixture was subjected to preparative LC-MS.[4A] Fractions corresponding to the product were immediately pooled together, frozen and lyophilized overnight to give the title compound (6.75 mg, quant.) as a white fluffy solid. Purity based on LC-MS 94% (very broad peak). See SEQ ID NO: 2 for the ApoB BNA sequence.

LRMS (m/z): 2318 $[M-4]^{4-}$

LC-MS r.t. (min): 1.65[1A]

**Trivalent GalNAc-S-ApoB (or HSP27) scrambled BNA oligo synthesis**

To ApoB scrambled BNA oligo disulfide (5.00 mg, 0.688 μmol) was added a solution of 20 mM ammonium bicarbonate with 2.5 mM TCEP (1.00 mL, 2.5 μmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-S-maleimide (3.09 mg, 1.46 μmol). The reaction mixture was shaken for 1 min and left standing at room temperature. After 1.5 hours the reaction mixture was subjected to preparative LC-MS.[4A] Fractions corresponding to the product were immediately pooled together, frozen and lyophilized overnight to give the title compound (5.91 mg, 93%) as a white fluffy solid. Purity based on LC-MS 88% (very broad peak).

LRMS (m/z): 2311 $[M-4]^{4-}$
LC-MS r.t. (min): 1.60[1A]

**Trivalent GalNAc-L-ApoB (or HSP27) BNA oligo synthesis**

To ApoB BNA oligo disulfide (5.00 mg, 0.686 μmol) was added a solution of 20 mM ammonium bicarbonate with 2.5 mM TCEP (1.00 mL, 2.5 μmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-L-maleimide (3.63 mg, 1.45 μmol). The reaction mixture was shaken for 1 min and left standing at room temperature. After 1.5 hours the reaction mixture was subjected to preparative LC-MS.[4A] Fractions corresponding to the product were immediately pooled together, frozen and lyophilized overnight to give the title compound (6.68 mg, quant.) as a white fluffy solid. Purity based on LC-MS 99% (very broad peak).

LRMS (m/z): 2416 $[M-4]^{4-}$

LC-MS r.t. (min): 1.97[1A]

**Trivalent GalNAc-L-ApoB (or HSP27) scrambled BNA oligo synthesis**

To ApoB scrambled BNA oligo disulfide (5.00 mg, 0.688 μmol) was added a solution of 20 mM ammonium bicarbonate with 2.5 mM TCEP (1.00 mL, 2.5 μmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-L-maleimide (3.68 mg, 1.47 μmol). The reaction mixture was shaken for 1 min and left standing at room temperature. After 1.5 hours the reaction mixture was subjected to preparative LC-MS.[4A] Fractions corresponding to the product were immediately pooled together, frozen and lyophilized overnight to give the title compound (4.71 mg, 71%) as a white fluffy solid. Purity based on LC-MS 96% (very broad peak).

LRMS (m/z): 2409 $[M-4]^{4-}$

LC-MS r.t. (min): 1.93[1A]

**Dendron(-L-SO1861)$_4$-trivalent GalNAc (Figure 23D-E) and dendron(-L-SO1861)$_8$-trivalent GalNAc synthesis**

Intermediate 13:

**Trivalent GalNAc-amine formate**

Trivalent GalNAc-azide (36.5 mg, 21.6 μmol) (molecule 21) is the Intermediate 7 as shown in Fig. 8G, which was dissolved in a solution of potassium carbonate (5.97 mg, 43.2 μmol) in water (1.00 mL) and acetonitrile (1.00 mL). Next, a 1.0 M trimethylphosphine solution in THF (216 μL, 216 μmol) was added and the resulting mixture was shaken for 1 min and left standing at room temperature. After 45 min the reaction mixture was evaporated *in vacuo* and the residue was dissolved in water/acetonitrile (9:1, v/v, 1 mL). The resulting solution was directly subjected to preparative MP-LC.[2B] Fractions corresponding to the product were immediately pooled together, frozen and lyophilized overnight to give the title compound (36.1 mg, 98%) as a white solid. Purity based on LC-MS 100%.

LRMS (m/z): 1662 $[M-1]^{1-}$

LC-MS r.t. (min): 1.62[1A]

Intermediate 14:

**Trivalent GalNAc-DBCO**

Trivalent GalNAc-amine formate (17.4 mg, 10.2 μmol) and DBCO-NHS (6.14 mg, 15.3 μmol) were dissolved in a solution of NMM (2.24 μL, 20.3 μmol) in DMF (0.50 mL). The reaction mixture was shaken for 1 min and left standing at room temperature. After 2 hours the reaction mixture was evaporated *in vacuo* and the residue was dissolved in water/acetonitrile (8:2, v/v, 1 mL). The resulting solution was directly subjected to preparative MP-LC.[2C] Fractions corresponding to the product were immediately pooled together, frozen and lyophilized overnight to give the title compound (14.2 mg, 72%) as a white solid. Purity based on LC-MS 96%.

LRMS (m/z): 1950 $[M-1]^{1}$

LC-MS r.t. (min): 1.86[1B]

Intermediate 15:

**dendron(-L-SO1861)$_8$-azide**

Dendron(-L-SO1861)$_8$-amine (19.6 mg, 1.08 μmol) and 2,5-dioxopyrrolidin-1-yl 1-azido-3,6,9,12-tetraoxapentadecan-15-oate (4.17 mg, 10.8 μmol) were dissolved in DMF (1.50 mL). Next, DIPEA (1.87 μL, 10.8 μmol) was added and the mixture was shaken for 1 min and left standing overnight at room temperature. The reaction mixture was subjected to preparative LC-MS.[4B] Fractions corresponding to the product were immediately pooled together, frozen and lyophilized overnight to give the title compound (11.7 mg, 59%) as a white fluffy solid. Purity based on LC-MS 92% (very broad peak).

LRMS (m/z): 2316 $[M-8]^{8-}$, ): 2647 $[M-7]^{7-}$

LC-MS r.t. (min): 4.29[1A]

**Dendron(-L-SO1861)$_4$-trivalent GalNAc synthesis**

Dendron(-L-SO1861)$_4$-azide (2.50 mg, 0.266 μmol) and trivalent GalNAc-DBCO (1.56 mg, 0.799 μmol) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.[4B] Fractions corresponding to the product were immediately pooled together, frozen and lyophilized overnight to give the title compound (2.74 mg, 91%) as a white fluffy solid. Purity based on LC-MS 86% (very broad peak).

LRMS (m/z): 2832 $[M-4]^{4-}$

LC-MS r.t. (min): 4.07[1A]

**Dendron(-L-SO1861)$_8$-trivalent GalNAc synthesis**

Dendron(-L-SO1861)$_8$-azide (2.50 mg, 0.135 μmol) and trivalent GalNAc-DBCO (0.79 mg, 0.405 μmol) were

dissolved in a mixture of water/acetonitrile (3:1, v/v, 1.00 mL). The reaction mixture was shaken for 1 min and left standing at room temperature. After 2 hours the reaction mixture was subjected to preparative LC-MS.[4B] Fractions corresponding to the product were immediately pooled together, frozen and lyophilized overnight to give the title compound (2.03 mg, 74%) as a white fluffy solid. Purity based on LC-MS 100% (very broad peak).

LRMS (m/z): 2559 $[M-8]^{8-}$, 2925 $[M-7]^{7-}$

LC-MS r.t. (min): 4.18[1A]

**Dendron(-L-SO1861)$_4$-L-BNA oligo-trivalent GalNAc and Dendron(-L-SO1861)$_8$-L-BNA oligo-trivalent GalNAc synthesis**

Intermediate 16:

**Trivalent GalNAc-thioacetate**

Trivalent GalNAc-amine formate (18.7 mg, 11.0 μmol) and 4-nitrophenyl 3-(acetylthio)propanoate (5.90 mg, 21.9 μmol) were dissolved in a solution of NMM (2.41 μL, 21.9 μmol) in DMF (0.50 mL). The reaction mixture was shaken for 1 min and left standing at room temperature. After 2 hours the reaction mixture was evaporated *in vacuo* and the residue was dissolved in a mixture of 0.1% formic acid in water and 0.1% formic acid in acetonitrile (9:1, v/v, 1 mL). The resulting solution was directly subjected to preparative MP-LC.[2B] Fractions corresponding to the product were immediately pooled together, frozen and lyophilized overnight to give the title compound (13.0 mg, 66%) as a white solid. Purity based on LC-MS 100%.

LRMS (m/z): 1749 $[M-43]^{1-}$, 1792 $[M-1]^{1}$

LC-MS r.t. (min): 1.24[1B]

Intermediate 17:

**DBCO-TCO-trivalent GalNAc**

Trivalent GalNAc-thioacetate (13.0 mg, 7.25 μmol) was dissolved in methanol (0.50 mL). Next, a 1 M solution of sodium hydroxide (7.98 μL, 7.98 μmol) was added. The reaction mixture was shaken for 1 min and left standing at room temperature. After 30 min the reaction mixture was added to a freshly prepared solution of trifunctional linker (Ls-t) (i.e., is an example of a saponin moiety linker Ls) (7.39 mg, 6.13 μmol) in 20 mM ammonium bicarbonate/acetonitrile (3:1, v/v, 2.00 mL).

The trifunctional linker has the IUPAC name: 5,8,11,18,21,24,27-heptaoxa-2,14,30-triazatritriacontanoic acid, 14-[16-(11,12-didehydrodibenz[*b,f*]azocin-5(6*H*)-yl)-13,16-dioxo-3,6,9-trioxa-12-azahexadec-1-yl]-33-(2,5-dihydro-2,5-dioxo-1*H*-pyrrol-1-yl)-15,31-dioxo-, (1*R*,4*E*)-4-cycloocten-1-yl ester. The trifunctional linker has the following formula (XXI):

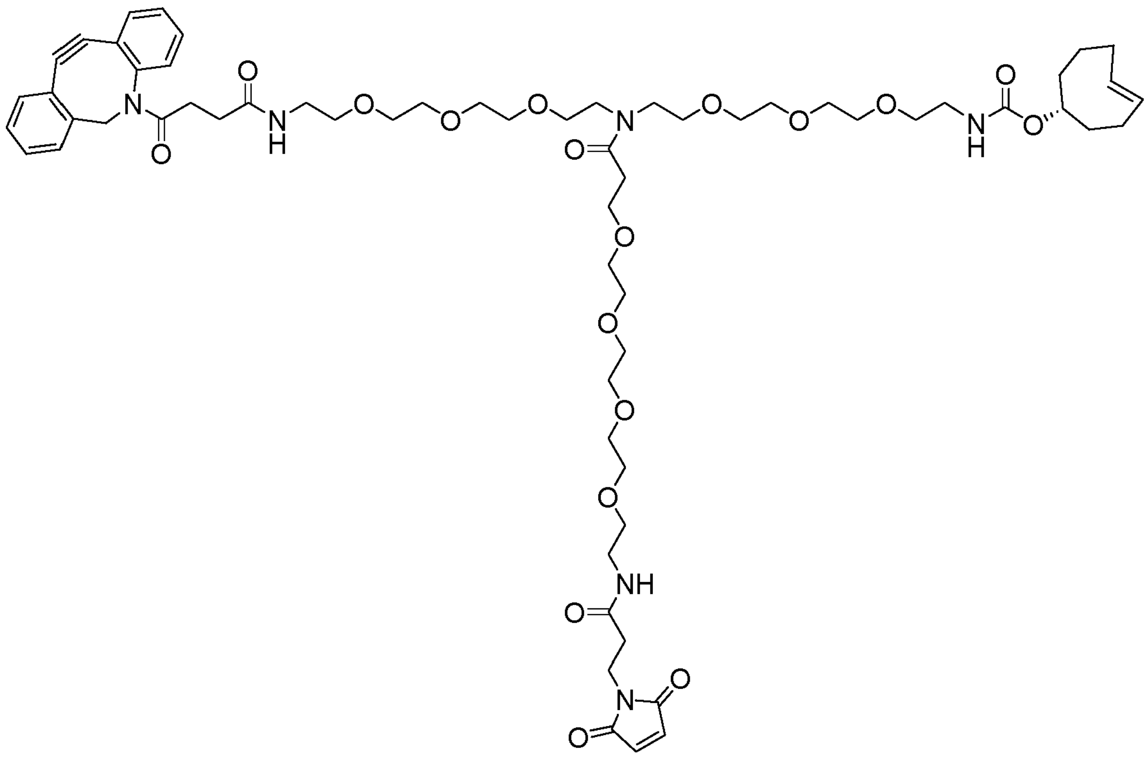

(XXI).

The resulting mixture was shaken for 1 min and left standing at room temperature. After 2 hours the reaction mixture was subjected to preparative MP-LC.[2A] Fractions corresponding to the product were immediately pooled together, frozen and lyophilized overnight to give the title compound (3.83 mg, 21%) as a white solid. Purity based on LC-MS 89%.

LRMS (m/z): 2409 $[M-546]^{1-}$, 2955 $[M-1]^{1-}$

LC-MS r.t. (min): 2.66[1B]

Intermediate 18:

**Methyltetrazine-L-ApoB BNA oligo**

To ApoB BNA oligo disulfide (5.00 mg, 0.686 μmol) was added a solution of 20 mM ammonium bicarbonate with 2.5 mM TCEP (1.00 mL, 2.5 μmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (1.36 mg, 1.73 μmol) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 μm syringe filter. The filtrate was lyophilized overnight to yield the title product (5.44 mg, quant) as a pink fluffy solid. Purity based on LC-MS 90% (very broad peak).

LRMS (m/z): 2648 $[M-3]^{3-}$
LC-MS r.t. (min): 0.62[4]

Intermediate 19:

**Methyltetrazine-L-ApoB scrambled BNA oligo**

To ApoB scrambled BNA oligo disulfide (5.00 mg, 0.688 μmol) was added a solution of 20 mM ammonium bicarbonate with 2.5 mM TCEP (1.00 mL, 2.5 μmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (1.34 mg, 1.70 μmol) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 μm syringe filter. The filtrate was lyophilized overnight to yield the title product (5.48 mg, quant) as a pink fluffy solid. Purity based on LC-MS 84% (very broad peak).

LRMS (m/z): 2639 $[M-3]^{3-}$
LC-MS r.t. (min): 0.58[4]

Intermediate 20:

**DBCO-L-ApoB BNA oligo-trivalent GalNAc**

To methyltetrazine-L-ApoB BNA oligo (5.44 mg, 0.684 μmol) and DBCO-TCO-trivalent GalNAc (2.03 mg, 0.687 μmol) was added 20 mM ammonium bicarbonate/acetonitrile (3:1, v/v, 1.00 mL). The reaction mixture was shaken for 1 min and left standing at room temperature. After 2 hours the reaction mixture was subjected to preparative LC-MS.[4C] Fractions corresponding to the product were immediately pooled together, frozen and lyophilized overnight to give the title compound (2.87 mg, 39%) as a white fluffy solid. LC-MS shows multiple broad peaks with the correct m/z values.

LRMS (m/z): 2175 $[M-5]^{5-}$, 2718 $[M-6]^{4-}$

LC-MS r.t. (min): 2.60-3.00[1A]

Intermediate 21:

**DBCO-L-ApoB scrambled BNA oligo-trivalent GalNAc**

To methyltetrazine-L-ApoB scrambled BNA oligo (5.48 mg, 0.692 μmol) and DBCO-TCO-trivalent GalNAc (1.80 mg, 0.609 μmol) was added 20 mM ammonium bicarbonate/acetonitrile (3:1, v/v, 1.00 mL). The reaction mixture was shaken for 1 min and left standing at room temperature. After 2 hours the reaction mixture was subjected to preparative LC-MS.[4C] Fractions corresponding to the product were immediately pooled together, frozen and lyophilized overnight to give the title compound (2.15 mg, 33%) as a white fluffy solid. LC-MS shows multiple broad peaks with the correct m/z values.

LRMS (m/z): 2169 $[M-5]^{5-}$, 2711 $[M-6]^{4-}$

LC-MS r.t. (min): 2.58-3.20[1A]

**Dendron(-L-SO1861)$_4$-L-ApoB BNA oligo-trivalent GalNAc synthesis (molecule 34, Figure 24D)**

To DBCO-L-ApoB BNA oligo-trivalent GalNAc (1.49 mg, 0.137 μmol; molecule 31, Figure 24C) and dendron(-L-SO1861)$_4$-azide (1.33 mg, 0.142 μmol; molecule 19, Figure 23D; the dendron(-L-SO1861)$_4$-azide is synthesized based on dendron(SPT001)$_4$-$NH_2$ (molecule 15, Figure 23C) wherein this amine dendron was treated with azido-PEG$_4$-NHS ester (molecule 18) in DMF with DIPEA as a base) was added 20 mM ammonium bicarbonate/acetonitrile (3:1, v/v, 0.5 mL) such that dendron(-L-SO1861)4-L-ApoB BNA oligo-trivalent GalNAc (molecule 34; 'Trivalent GalNAc-ApoB BNA-SPT001 conjugate') is synthesized (Figure 24D). The reaction mixture was shaken for 1 min and left standing at room temperature. After 3 hours the reaction mixture was subjected to preparative LC-MS.[4B] Fractions corresponding to the

product were immediately pooled together, frozen and lyophilized overnight to give the title compound (0.96 mg, 35%) as a white fluffy solid. Purity based on LC-MS 93% (very broad peak).

LRMS (m/z): 2025 $[M-10]^{10-}$, 2250 $[M-9]^{9-}$, 2532 $[M-8]^{8-}$, 2893 $[M-7]^{7-}$

LC-MS r.t. (min): 3.68[1A]

The DBCO-L-ApoB BNA oligo-trivalent GalNAc (molecule 31) was synthesized by conjugating methyltetrazine-L-ApoB BNA oligo (molecule 30) to DBCO-TCO-trivalent GalNAc (molecule 29) (Figure 24A-B and 24C), wherein the DBCO-TCO-trivalent GalNAc (molecule 29) was synthesized with molecule 27 and molecule 28 (trifunctional linker), wherein molecule 27 is GalNAc-thioacetate obtained from the reaction between molecule 22, the formic salt of trivalent GalNAc-amine, and molecule 26 (4-nitrophenyl 3-(acetylthio)propanate (Figure 24A-B).

The ApoB BNA, the trivalent GalNAc and SPT001 (SO1861) are conjugated into a single covalent conjugate using tri-functional linker Figure 24D).

Using a similar approach as for synthesis of dendron(-L-SO1861)4-L-ApoB BNA oligo-trivalent GalNAc (molecule 34; 'Trivalent GalNAc-ApoB BNA-SPT001 conjugate'), a conjugate in which the ApoB BNA was replaced for a BNA with a scrambled ApoB nucleic acid sequence, was synthesized, for control testing purposes.

Using a similar approach as for synthesis of dendron(-L-SO1861)4-L-ApoB BNA oligo-trivalent GalNAc (molecule 34; 'Trivalent GalNAc-ApoB BNA-SPT001 conjugate'), a conjugate dendron(-L-SO1861)8-L-ApoB BNA oligo-trivalent GalNAc was synthesized in which the ApoB BNA oligonucleotide was either ApoB BNA for silencing mRNA / protein expression, or was a BNA with a scrambled ApoB nucleic acid sequence.

**SO1861-L-ApoB BNA oligo-trivalent GalNAc synthesis (Trivalent GalNAc-*ApoB* BNA-SPT001 conjugate, Figure 22A-C)**

Referring to Figure 22A-C, the conjugate comprising trivalent GalNAc, saponin SO1861 and ApoB BNA was synthesized, using the trivalent linker TFL with molecular structure:

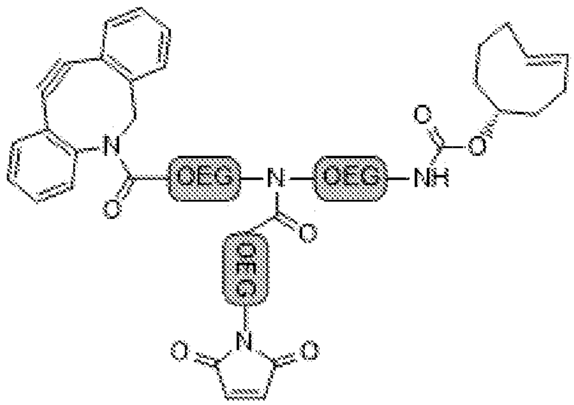

(TFL).

The conjugate SO1861-L-ApoB BNA oligo-trivalent GalNAc synthesis (Trivalent GalNAc-*ApoB* BNA-SPT001 conjugate) was formed.

**Dendron(-L-SO1861)$_8$-L-ApoB BNA oligo-trivalent GalNAc synthesis**

To DBCO-L-ApoB BNA oligo-trivalent GalNAc (1.38 mg, 0.127 µmol) and dendron(-L-SO1861)$_8$-azide (2.51 mg, 0.135 µmol) was added 20 mM ammonium bicarbonate/acetonitrile (3:1, v/v, 0.5 mL). The reaction mixture was shaken for 1 min and left standing at room temperature. After 4 hours the reaction mixture was subjected to preparative LC-MS.[4B] Fractions corresponding to the product were immediately pooled together, frozen and lyophilized overnight to give the title compound (0.75 mg, 20%) as a white fluffy solid. Purity based on LC-MS 99% (very broad peak).

LRMS (m/z): 2099 $[M-14]^{14-}$, 2261 $[M-13]^{13-}$, 2450 $[M-12]^{12-}$, 2672 $[M-11]^{11-}$, 2940 $[M-10]^{10-}$

LC-MS r.t. (min): 3.90[1A]

**Dendron(-L-SO1861)$_4$-L-ApoB scrambled BNA oligo-trivalent GalNAc synthesis**

To DBCO-L-ApoB scrambled BNA oligo-trivalent GalNAc (1.09 mg, 0.100 μmol) and dendron(-L-SO1861)$_4$-azide (0.96 mg, 0.102 μmol) was added 20 mM ammonium bicarbonate/acetonitrile (3:1, v/v, 0.5 mL). The reaction mixture was shaken for 1 min and left standing at room temperature. After 3 hours the reaction mixture was subjected to preparative LC-MS.[4B] Fractions corresponding to the product were immediately pooled together, frozen and lyophilized overnight to give the title compound (0.45 mg, 22%) as a white fluffy solid. LC-MS shows multiple broad peaks with the correct m/z values.

LRMS (m/z): 2023 $[M-10]^{10-}$, 2248 $[M-9]^{9-}$, 2528 $[M-8]^{8-}$, 2890 $[M-7]^{7-}$

LC-MS r.t. (min): 3.60-4.00[1A]

**antiCD71-saporin synthesis**

Custom CD71mab-saporin conjugate was produced and purchased from Advanced Targeting Systems (San Diego, CA). InVivoMab grade CD71 monoclonal antibody was purchased from BioXCell (anti-human CD71, clone OKT-9).

**HSP27BNA, ApoB and ApoB$_{scrambled}$ oligo sequences**

HSP27 (5'-GGCacagccagtgGCG-3') [SEQ ID NO: 1] (The antisense BNA (HSP27) was an BNA$^{(NC)}$ with oligo nucleic acid sequence 5'-GGCacagccagtgGCG-3' modified from Zhang et al. (2011) [Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333]), ApoB (5'-GCCTCa gtctgcttcGCACC-3') [SEQ ID NO: 2] and ApoB$_{scrambled}$ (5'-GGCCTctctacaccgCTCGT-3') [SEQ ID NO: 3] BNA oligos were ordered with 5'-Thiol C6 linker at Bio-Synthesis Inc. (Lewisville, Texas).

**Human primary hepatocyte treatment**

Cryopreserved primary human hepatocytes (Cytes Biotechnologies S.L., Spain) were thawed in hepatocyte thawing media (Cytes Biotechnologies S.L., Spain). Cells were then re-suspended in hepatocyte plating media (Cytes Biotechnologies S.L., Spain). Next, cells were seeded onto collagen-I coated plates at a density of 215.600 cells/well or 66.600 cells/well for the 48 or 96-well plates (Greiner BioOne). Cells were pre-cultured for 4-6 hrs at 37°C allowing for cell attachment to cell culture plates before the start of treatment. Plating medium was replaced by 315 μl or 108 μl maintenance media (Cytes Biotechnologies S.L., Spain), after which conjugates were added from a 10x concentrated stock solution in PBS. Plates were incubated for 72 hr at 37°C being harvested for gene expression and cell viability analysis.

**RNA isolation and gene expression analysis from human cell culture**

RNA from cells was isolated and analysed according to standard protocols (Biorad). qPCR primers that were used are indicated in Table A2.

**Table A2.** Primers used in qPCR are shown below:

| Gene | Primer | Sequence (5'-3') |
|---|---|---|
| HSP27 | Forward | GCAGTCCAACGAGATCACCA [SEQ ID NO: 4] |
| | Reverse | TAAGGCTTTACTTGGCGGCA [SEQ ID NO: 5] |
| ApoB | Forward | AGGGTCCGGGAATCTGATGA [SEQ ID NO: 6] |
| | Reverse | TGGGCACGTTGTCTTTCAGAG [SEQ ID NO: 7] |
| HBMS | Forward | CACCCACACACAGCCTACTT [SEQ ID NO: 8] |
| | Reverse | GTACCCACGCGAATCACTCT [SEQ ID NO: 9] |
| GUSB | Forward | GAAAATACGTGGTTGGAGAGCT [SEQ ID NO: 10] |

(continued)

| Gene | Primer | Sequence (5'-3') |
|---|---|---|
| | Reverse | CCGAGTGAAGATCCCCTTTTTA [SEQ ID NO: 11] |

**Cell viability assay (MTS)**

Cells were incubated for 72 hrs at 37°C following compound addition before cell viability was determined by MTS-assay, performed according to the manufacturer's instruction (CellTiter 96® AQueous One Solution Cell Proliferation Assay, Promega). Briefly, the MTS solution was diluted 20× in DMEM without phenol red (PAN-Biotech GmbH) supplemented with 10% FBS. The cells were washed once with 200 μL/PBS well, after which 100 μL/well diluted MTS solution was added. The plate was incubated for approximately 20-30 minutes at 37°C. Subsequently, the OD at 492 nm was measured on a Thermo Scientific Multiskan FC plate reader (Thermo Scientific). For quantification, the background signal of 'medium only' wells was subtracted from all other wells before relative percentage cell viability percentage of was calculated by dividing the background corrected signal of compound treated wells by background corrected signal of untreated wells (x 100 for percentage).

**FACS analysis**

Cells were seeded in DMEM (PAN-Biotech GmbH) supplemented with 10% fetal bovine serum (PAN-Biotech GmbH) and 1% penicillin/streptomycin (PAN-Biotech GmbH) in T75 flasks at appropriate density for each cell-line. Cultures were incubated for 72-96 hrs (5% $CO_2$, 37°C) until a confluency of 90% was reached. Next, cells were trypsinized (TryplE Express, Gibco Thermo Scientific) to obtain a single cell suspension, transferred to a 15 mL falcon tube, and centrifuged (1,400 rpm, 3 min). The supernatant was discarded while leaving the cell pellet submerged. 500.000 cells were transferred to round bottom FACS tubes and the washed with 3 mL cold DPBS ($Mg^{2+}$ and $Ca^{2+}$ free, 2% FBS). The cells were centrifuged at 1800 rpm, 3 min 4°C and resuspended in 200 μL cold DPBS ($Mg^{2+}$ and $Ca^{2+}$ free, 2% FBS) or 200 μL antibody solution; containing 5 μL antibody in 195 μL cold DPBS ($Mg^{2+}$ and $Ca^{2+}$ free, 2% FBS). PE anti-human CD71 (#334106, Biolegend) was used to stain the transferrin receptor, PE Mouse IgG2a, κ isotype Ctrl FC (#400212, Biolegend) was used as its matched isotype control. PE anti-human ASGPR1 (#130-122-963, Miltenyi) was used to stain the ASGPR1 receptor and PE Mouse IgG1, isotype Ctrl (#130-113-762, Miltenyi) was used as its matched isotype control. Samples were incubated for 30 min. at 4°C. Afterwards, the cells were washed 2x with cold DPBS ($Mg^{2+}$ and $Ca^{2+}$ free, 2% FBS) and fixated for 20 min at room temperature using a 2% PFA solution in DPBS ($Mg^{2+}$ and $Ca^{2+}$ free, 2% FBS). Cells were washed 1x with cold DPBS and resuspended in 1000 μL cold DPBS for FACS analysis. Samples were analyzed with a Sysmex Cube 8 flow cytometry system (Sysmex) and FCS Express 7 Research edition software. Results of the FACS analyses are summarized in Table A3.

*Table A3. Cell membrane receptor expression levels of ASGPR1 and CD71 of HepG2 and Huh7 cells*

| **Cell line** | **ASGPR1 expression level (MFI)** | **CD71 expression level (MFI)** |
|---|---|---|
| **HepG2** | 8.8 | 74.7 |
| **Huh7** | 1.6 | 109 |

**Example 4**

**Materials and methods**

**Abbreviations**

| | |
|---|---|
| AEM | *N*-(2-Aminoethyl)maleimide trifluoroacetate salt |
| AMPD | 2-Amino-2-methyl-1,3-propanediol |
| BOP | (Benzotriazol-1-yloxy)tris(dimethylamino)phosphonium hexafluorophosphate |
| DIPEA | N,N-diisopropylethylamine |
| DMF | N,N-dimethylformamide |
| DTME | Dithiobismaleimidoethane |
| DTT | Dithiothreitol |

| | |
|---|---|
| EDCI.HCl | 1-Ethyl-3-(3-dimethylaminopropyl)carbodiimide hydrochloride |
| EDTA | Ethylenediaminetetraacetic acid |
| EMCH.TFA | N-(ε-maleimidocaproic acid) hydrazide, trifluoroacetic acid salt |
| HATU | 1-[Bis(dimethylamino)methylene]-1H-1,2,3-triazolo[4,5-b]pyridinium 3-oxid hexafluorophosphate |
| min | minutes |
| NMM | 4-Methylmorpholine |
| r.t. | retention time |
| SEC | Size exclusion chromatography |
| TBEU | (Tris-(hydroxymethyl)-aminomethan)-Borat-EDTA-Urea |
| TCEP | tris(2-carboxyethyl)phosphine hydrochloride |
| Temp | temperature |
| TFA | trifluoroacetic acid |
| TFL | trifunctional linker |

Analytical methods

LC-MS method 1

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: neg or neg/pos within in a range of 1500-2400 or 2000-3000; ELSD: gas pressure 40 psi, drift tube temp: 50°C; column: Acquity C18, 50×2.1 mm, 1.7 μm Temp: 60°C, Flow: 0.6 mL/min, lin. Gradient depending on the polarity of the product:

$$^{A}t_{0} = 2\%\ A,\ t_{5.0min} = 50\%\ A,\ t_{6.0min} = 98\%\ A$$

$$^{B}t_{0} = 2\%\ A,\ t_{5.0min} = 98\%\ A,\ t_{6.0min} = 98\%\ A$$

Posttime: 1.0 min, Eluent A: acetonitrile, Eluent B: 10 mM ammonium bicarbonate in water (pH=9.5).

LC-MS method 2

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50 °C; column: Waters XSelect™ CSH C18, 50×2.1 mm, 2.5 μm, Temp: 25°C, Flow: 0.5 mL/min, Gradient: $t_{0min}$ = 5% A, $t_{2.0min}$ = 98% A, $t_{2.7min}$ = 98% A, Posttime: 0.3 min, Eluent A: acetonitrile, Eluent B: 10 mM ammonium bicarbonate in water (pH=9.5).

LC-MS method 3

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product pos/neg 105-800, 500-1200 or 1500-2500; ELSD: gas pressure 40 psi, drift tube temp: 50°C; column: Waters XSelect™ CSH C18, 50×2.1mm, 2.5 μm, Temp: 40°C, Flow: 0.5 mL/min, Gradient: $t_{0min}$ = 5% A, $t_{2.0min}$= 98% A, $t_{2.7min}$= 98% A, Posttime: 0.3 min, Eluent A: 0.1% formic acid in acetonitrile, Eluent B: 0.1% formic acid in water.

LC-MS method 4

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gas pressure 40 psi, drift tube temp: 50°C column: Waters Acquity Shield RP18, 50×2.1mm, 1.7μm, Temp: 25°C, Flow: 0.5 mL/min, Gradient: $t_{0min}$ = 5% A, $t_{2.0min}$ = 98% A, $t_{2.7min}$ = 98% A, Posttime: 0.3 min, Eluent A: acetonitrile, Eluent B: 10 mM ammonium bicarbonate in water (pH=9.5).

Preparative methods

Preparative MP-LC method 1

Instrument type: Reveleris™ prep MPLC; column: Waters XSelect™ CSH C18 (145×25 mm, 10μm); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient:

$$^{A}t_{0min} = 5\%\ B,\ t_{1min} = 5\%\ B,\ t_{2min} = 10\%\ B,\ t_{17min} = 50\%\ B,\ t_{18min} = 100\%\ B,\ t_{23min} = 100\%\ B$$

$$^{A}t_{0min} = 5\%\ B,\ t_{1min} = 5\%\ B,\ t_{2min} = 20\%\ B,\ t_{17min} = 60\%\ B,\ t_{18min} = 100\%\ B,\ t_{23min} = 100\%\ B$$

; Detection UV: 210, 235, 254 nm and ELSD.

Preparative MP-LC method 2

Instrument type: Reveleris™ prep MPLC; Column: Phenomenex LUNA C18(3) (150×25 mm, 10μm); Flow: 40 mL/min; Column temp: room temperature; Eluent A: 0.1% (v/v) Formic acid in water, Eluent B: 0.1% (v/v) Formic acid in acetonitrile; Gradient:

$$^{A}t_{0min} = 5\%\ B,\ t_{1min} = 5\%\ B,\ t_{2min} = 20\%\ B,\ t_{17min} = 60\%\ B,\ t_{18min} = 100\%\ B,\ t_{23min} = 100\%\ B$$

$$^{B}t_{0min} = 2\%\ B,\ t_{1min} = 2\%\ B,\ t_{2min} = 2\%\ B,\ t_{17min} = 30\%\ B,\ t_{18min} = 100\%\ B,\ t_{23min} = 100\%\ B$$

$$^{C}t_{0min} = 5\%\ B,\ t_{1min} = 5\%\ B,\ t_{2min} = 10\%\ B,\ t_{17min} = 50\%\ B,\ t_{18min} = 100\%\ B,\ t_{23min} = 100\%\ B$$

$$^{D}t_{0min} = 5\%\ B,\ t_{1min} = 5\%\ B,\ t_{2min} = 5\%\ B,\ t_{17min} = 40\%\ B,\ t_{18min} = 100\%\ B,\ t_{23min} = 100\%\ B$$

; Detection UV: 210, 235, 254 nm and ELSD.

Preparative LC-MS method 3

MS instrument type: Agilent Technologies G6130B Quadrupole; HPLC instrument type: Agilent Technologies 1290 preparative LC; Column: Waters XSelect™ CSH (C18, 150×19mm, 10μm); Flow: 25 ml/min; Column temp: room temperature; Eluent A: 100% acetonitrile; Eluent B: 10 mM ammonium bicarbonate in water pH=9.0; Gradient:

$$^{A}t_{0} = 20\%\ A,\ t_{2.5min} = 20\%\ A,\ t_{11min} = 60\%\ A,\ t_{13min} = 100\%\ A,\ t_{17min} = 100\%\ A$$

$$^{B}t_{0} = 5\%\ A,\ t_{2.5min} = 5\%\ A,\ t_{11min} = 40\%\ A,\ t_{13min} = 100\%\ A,\ t_{17min} = 100\%\ A$$

; Detection: DAD (210 nm); Detection: MSD (ESI pos/neg) mass range: 100 - 800; Fraction collection based on DAD.

Preparative LC-MS method 4

MS instrument type: Agilent Technologies G6130B Quadrupole; HPLC instrument type: Agilent Technologies 1290 preparative LC; Column: Waters XBridge Protein (C4, 150×19mm, 10μm); Flow: 25 ml/min; Column temp: room temperature; Eluent A: 100% acetonitrile; Eluent B: 10 mM ammonium bicarbonate in water pH=9.0; Gradient:

$$^{A}t_{0} = 2\%\ A,\ t_{2.5min} = 2\%\ A,\ t_{11min} = 30\%\ A,\ t_{13min} = 100\%\ A,\ t_{17min} = 100\%\ A$$

$^{B}t_0 = 10\%$ A, $t_{2.5min} = 10\%$ A, $t_{11min} = 50\%$ A, $t_{13min} = 100\%$ A, $t_{17min} = 100\%$ A

$^{C}t_0 = 5\%$ A, $t_{2.5min} = 5\%$ A, $t_{11min} = 40\%$ A, $t_{13min} = 100\%$ A, $t_{17min} = 100\%$ A

; Detection: DAD (210 nm); Detection: MSD (ESI pos/neg) mass range: 100 - 800; Fraction collection based on DAD.

Flash chromatography

Grace Reveleris X2® C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50bar (725psi); Detection: UV 200-400 nm, combination of up to 4 UV signals and scan of entire UV range, ELSD; Column sizes: 4-330g on instrument, luer type, 750g up to 3000g with optional holder.

UV-vis spectrophotometry

Protein concentrations were determined using a Thermo Nanodrop 2000 spectrometer and an extinction coefficient of ε280 ((mg/ml)-1 cm-1); Oligo concentrations were determined using an axtinction coefficient of ε260 153,000 M-1 cm-1.

Ellman's assay was carried out using a Perkin Elmer Lambda 25 Spectrophotometer and a literature molar extinction coefficient ε412 of 14150 M-1 cm-1 for TNB. Experimentally determined molar extinction coefficient ε495 = 58,700 M-1 cm-1 and Rz280:495 = 0.428 were used for SAMSA-fluorescein.

Size exclusion chromatography (SEC) analysis

The conjugates were analysed by SEC using an Akta Purifier 10 system and Biosep SEC-s3000 column eluting with DPBS:IPA (85:15). Conjugate purity was determined by integration of the conjugate peak UV signal over time versus impurities/aggregate form peak integrated area.

SDS-PAGE and Western Blotting

Native proteins and conjugates were analysed under heat denaturing non-reducing and reducing conditions by SDS-PAGE against a protein ladder using a 4-12% bis-tris gel and MES as running buffer (200V, ~40 minutes). Samples were prepared to 0.5 mg/ml, comprising LDS sample buffer and MOPS running buffer as diluent. For reducing samples, DTT was added to a final concentration of 50 mM. Samples were heat treated for 2 minutes at 90-95°C and 5 μg (10 μl) was added to each well. Protein ladder (10 μl) was loaded without pre-treatment. Empty wells were filled with 1× LDS sample buffer (10 μl). After the gel was run, it was washed thrice with DI water (100 ml) with shaking (15 minutes, 200 rpm). Coomassie staining was performed by shaker-incubating the gel with PAGEBlue protein stain (30 ml) (60 minutes, 200 rpm). Excess staining solution was removed, after which the gel was rinsed twice with DI water (100 ml) and destained with DI water (100 ml) (60 minutes, 200 rpm). The resulting gel was imaged and subsequently further processed using ImageJ (Rasband, W.S., ImageJ, U. S. National Institutes of Health, Bethesda, Maryland, USA).

TBEU-PAGE

Native protein, conjugates and BNA standard were analysed under heat denaturing non-reducing and reducing conditions by TBEU-PAGE against an oligo ladder using a 15% TBE-Urea gel and TBE as running buffer (180V, ~60 minutes). Samples were prepared to 0.5 mg/ml, and BNA standard was prepared to 20 μg/ml, respectively, all comprising TBE Urea sample buffer and purified $H_2O$ as diluent. Samples and standards were heat treated for 3 minutes at 70°C and 10 μl was added to each well, equating to 5 μg of protein and conjugate samples, and 0.2 μg of BNA, per lane. Oligo ladder reconstituted to 0.1 μg/band/ml in TE pH 7.5 (2 μl) was loaded without pre-treatment. After the gel was run, it was stained with freshly prepared ethidium bromide solution (1 μg/ml) with shaking (40 minutes, 200 rpm). The resulting gel was visualised by UV epi-illumination (254nm), imaged and further processed using ImageJ (Rasband, W.S., ImageJ, U. S. National Institutes of Health, Bethesda, Maryland, USA).

MALDI-TOF-MS

MALDI-TOF spectra were recorded on a MALDI-Mass Spectrometer (Bruker Ultrafex III). Typically, the sample

dissolved in MilliQ water in nanomolar to micromolar range was spotted on the target (MTP 384 target plate polished steel T F, Bruker Daltons) using either super-DHB (99%, Fluka) or sinapinic acid (SA, 99%, Sigma-Aldrich) as the matrix dissolved in acetonitrile (MADLI-TOF-MS tested, Sigma) / 0.1 % TFA (7:3 v/v) via the dried-droplet-method. PepMix (Peptide Calibration Standard, Bruker Daltons) or ProteMass (Protein Calibration Standard, Sigma-Aldrich) served as calibration standards.

**Trifunctional linker-(L-hydrazone-SO1861)-(L-BNA oligo) - (Trivalent-GalNAc) synthesis (Figure 35)**

The trifunctional linker (TFL) has the IUPAC name: 5,8,11,18,21,24,27-Heptaoxa-2,14,30-triazatritriacontanoic acid, 14-[16-(11,12-didehydrodibenz[*b,f*]azocin-5(6*H*)-yl)-13,16-dioxo-3,6,9-trioxa-12-azahexadec-1-yl]-33-(2,5-dihydro-2,5-dioxo-1*H*-pyrrol-1-yl)-15,31-dioxo-, (1*R*,4*E*)-4-cycloocten-1-yl ester. The trifunctional linker has the following formula (XXI):

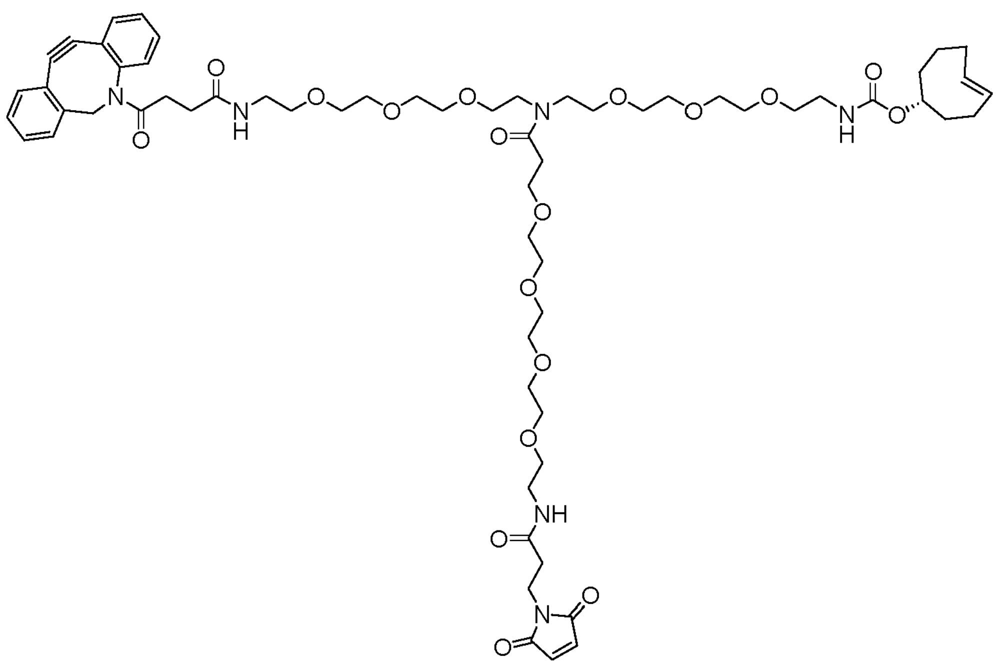

(XXI).

Intermediate 1:

**SO1861-L-hydrazone-azide** (molecule 3)

To SO1861 (60 mg, 0.032 mmol, molecule 1) and 1-azido-3,6,9,12-tetraoxapentadecane-15-hydrazide (39.3 mg, 0.129 mmol, molecule 2) was added methanol (extra dry, 1.00 mL) and TFA (9.86 μl, 0.129 mmol) and the reaction mixture was shaken for 1 min and left standing at room temperature. After 2 hours, the reaction mixture was subjected to preparative MP-LC.[1] Fractions corresponding to the product were immediately pooled together, frozen and lyophilized overnight to give the title compound (58.4 mg, 84%) as a white fluffy solid. Purity based on LC-MS 99%.

LRMS (m/z): 2150 $[M-H]^{1-}$
LC-MS r.t. (min): $1.10^{3B}$

Intermediate 2:

**Trifunctional linker-(L-hydrazone-SO1861)-(TCO)-(Maleimide)** (molecule 5)

A solution of TFL-(DBCO)-(TCO)-(Maleimide) (15 mg, 12.5 μmol, molecule 4) in DMF (2.0 mL) was added to SO1861-L-hydrazone-azide (26.8 mg, 12.5 μmol, molecule 3). The reaction mixture was shaken for 30 min and left

standing at room temperature. After 30 min, the reaction mixture was submitted to preparative MP-LC.[1C] Fractions corresponding to the product were immediately pooled together, frozen and lyophilized overnight to give the title compound (31.4 mg, 75%) as a white fluffy solid. Purity based on LC-MS 96%.
MS (m/z): 3354 $[M-H]^{1-}$

Intermediate 3:

**Trivalent GalNAc-thioacetate** (molecule 8)

Trivalent GalNAc-amine formate (18.7 mg, 11.0 μmol, molecule 6) and 4-nitrophenyl 3-(acetylthio)propanoate (5.90 mg, 21.9 μmol, molecule 7) were dissolved in a solution of NMM (2.41 μL, 21.9 μmol) in DMF (0.50 mL). The trivalent GalNAc-amine formate is obtained based on the trivalent GalNAc-azide which is the Intermediate 7 as shown in Fig. 8G. In Figure 35B, the formate of molecule 6 is not shown for simplicity. The reaction mixture was shaken for 1 min and left standing at room temperature. After 2 hours, the reaction mixture was evaporated *in vacuo* and the residue was dissolved in a mixture of 0.1% formic acid in water and 0.1% formic acid in acetonitrile (9:1, v/v, 1 mL). The resulting solution was directly subjected to preparative MP-LC.[2B] Fractions corresponding to the product were immediately pooled together, frozen and lyophilized overnight to give the title compound (13.0 mg, 66%) as a white solid. Purity based on LC-MS 100%.

LRMS (m/z): 1749 $[M-43]^{1-}$, 1792 $[M-H]^{1}$

LC-MS r.t. (min): 1.24[1B]

Intermediate 4:

**Trifunctional linker-(L-SO1861)-(TCO)-(trivalent GalNAc)** (molecule 9)

Trivalent GalNAc-thioacetate (13.0 mg, 7.25 μmol, molecule 8) was dissolved in methanol (0.50 mL). Next, a 1 M solution of sodium hydroxide (7.98 μL, 7.98 μmol) was added. The reaction mixture was shaken for 1 min and left standing at room temperature. After 30 min, the reaction mixture was added to a freshly prepared solution of TFL-(DBCO)-(TCO)-(Maleimide) (20.6 mg, 6.13 μmol, molecule 5) in 20 mM ammonium bicarbonate/acetonitrile (3:1, v/v, 2.00 mL).

The resulting mixture was shaken for 1 min and left standing at room temperature. After 2 hours the reaction mixture was subjected to preparative MP-LC.[2A] Fractions corresponding to the product were immediately pooled together, frozen and lyophilized overnight to give the title compound (3.83 mg, 21%) as a white solid. Purity based on LC-MS 89%.
MS (m/z): 5106 $[M + H]^{+}$

Intermediate 5:

**methyltetrazine-L-ApoB BNA oligo** (molecule 12)

To ApoB#02 BNA oligo disulfide (3.3 mg, 0.686 μmol, molecule 10) was added a solution of 20 mM ammonium bicarbonate with 2.5 mM TCEP (1.00 mL, 2.5 μmol). The reaction mixture was shaken for 1 min and left standing at room temperature. After 1 hour, the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (1.36 mg, 1.73 μmol, molecule 11) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 μm syringe filter. The filtrate was lyophilized overnight to yield the title product (3.4 mg, 89%) as a pink fluffy solid. Purity based on LC-MS 90%.
MS (m/z): 5579 $[M+Na]^{+}$

**Trifunctional linker-(L-hydrazone-SO1861)-(L-BNA oligo)-(trivalent GalNAc)** (molecule 13)

TFL-(L-hydrazone-SO1861)-(TCO)-(trivalent GalNAc) (16.1 mg, 3.15 μmol, molecule 9) and methyltetrazine-BNA oligo (10.2 mg, 1.83 μmol, molecule 12) were dissolved in a solution of 20 mM ammonium bicarbonate/acetonitrile

(3:1, v/v, 1.00 mL). The reaction mixture was shaken for 1 min and left standing at room temperature. After 3 hours, the reaction mixture was frozen and lyophilized overnight. The crude product was dissolved in 20 mM ammonium bicarbonate (1 mL) and the resulting solution was directly subjected to preparative LC-MS.[1B] Fractions corresponding to the product were immediately pooled together, frozen and lyophilized overnight to give the title compound (27.2 mg, 81%) as a white fluffy solid. Purity based on LC-MS 91%.
MS (m/z): 10660 $[M + H]^+$

Using a similar approach as for synthesis of Trifunctional linker-(L-hydrazone-SO1861)-(L-BNA oligo)-(trivalent GalNAc) (molecule 13); a conjugate in which the ApoB BNA is replaced for a BNA with a scrambled ApoB nucleic acid sequence (molecule 12 a), can be synthesized, for control testing purposes (molecule 13a).

**Trifunctional linker-(dendron(-L-hydrazone-SO1861)4)- (L-BNA oligo) - (Trivalent-GalNAc) synthesis (Figure 36)**

<u>Intermediate 6:</u>

**SO1861-EMCH** (molecule 15)

To SO1861 (121 mg, 0.065 mmol, molecule 1) and EMCH.TFA (110 mg, 0.325 mmol, molecule 14) was added methanol (extra dry, 3.00 mL) and TFA (0.020 mL, 0.260 mmol). The reaction mixture stirred at room temperature. After 1.5 hours, the reaction mixture was subjected to preparative MP-LC.1 Fractions corresponding to the product were immediately pooled together, frozen and lyophilized overnight to give the title compound (120 mg, 90%) as a white fluffy solid. Purity based on LC-MS 96%.

LRMS (m/z): 2069 [M-1]1-

LC-MS r.t. (min): 1.084

<u>Intermediate 7:</u>

**dendron(-L-SO1861)$_4$-amine** (molecule 17)

N,N'-((9S,199)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate (2.73 mg, 3.13 μmol, molecule 16) was dissolved in a mixture of 20 mM $NH_4HCO_3$ with 0.5 mM TCEP/acetonitrile (3:1, v/v, 3.00 mL). Next, SO1861-EMCH (29.2 mg, 0.014 mmol, molecule 15) was added and the reaction mixture was stirred at room temperature. After 1.5 hours, the reaction mixture was subjected to preparative LC-MS.[3B] Fractions corresponding to the product were immediately pooled together, frozen and lyophilized overnight to give the title compound (12.3 mg, 43%) as a white fluffy solid. Purity based on LC-MS 97%.

LRMS (m/z): 1517 $[M-6]^{6-}$, 1821 $[M-5]^{5-}$, 2276 $[M-4]^{4-}$
LC-MS r.t. (min): 4.39[5A]

<u>Intermediate 8</u>

**dendron(-L-SO1861)$_4$-azide** (molecule 19)

Dendron(SO1861)$_4$-amine (6.81 mg, 0.748 μmol, molecule 17) and 2,5-dioxopyrrolidin-1-yl 1-azido-3,6,9,12-tetraoxapentadecan-15-oate (2.90 mg, 7.48 μmol, molecule 18) were dissolved in DMF(1.00 mL). Next, DIPEA (1.302 μL, 7.48 μmol) was added and the mixture was shaken for 1 min and left standing at room temperature. After 2 hours, the reaction mixture was subjected to preparative LC-MS.[3C] Fractions corresponding to the product were immediately pooled together, frozen and lyophilized overnight to give the title compound (5.86 mg, 84%) as a white fluffy solid. Purity based on LC-MS 90%.

LRMS (m/z): 2344 $[M-4]^{4-}$
LC-MS r.t. (min): 4.78[5B]

<u>Intermediate 9:</u>

**Trifunctional linker (DBCO)-(TCO)-(trivalent GalNAc)** (molecule 20)

Trivalent GalNAc-thioacetate (13.0 mg, 7.25 μmol, molecule 8) was dissolved in methanol (0.50 mL). Next, a 1 M solution of sodium hydroxide (7.98 μL, 7.98 μmol) was added. The reaction mixture was shaken for 1 min and left standing at room temperature. After 30 min, the reaction mixture was added to a freshly prepared solution of trifunctional linker (7.39 mg, 6.13 μmol, molecule 4) in 20 mM ammonium bicarbonate/acetonitrile (3:1, v/v, 2.00 mL). The resulting mixture was shaken for 1 min and left standing at room temperature. After 2 hours, the reaction mixture was subjected to preparative MP-LC.[2A] Fractions corresponding to the product were immediately pooled together, frozen and lyophilized overnight to give the title compound (3.83 mg, 21%) as a white solid. Purity based on LC-MS 89%.

LRMS (m/z): 2409 $[M-546]^{1-}$, 2955 $[M-1]^{1-}$

LC-MS r.t. (min): 2.66[1B]

Intermediate 10:

**Trifunctional linker (DBCO)-(L-ApoB BNA oligo)-(trivalent GalNAc)** (molecule 21)

To methyltetrazine-L-ApoB BNA oligo (5.82 mg, 0.684 μmol, molecule 12) and TFL-(DBCO)-(TCO)-(trivalent GalNAc) (2.03 mg, 0.687 μmol, molecule 20) was added 20 mM ammonium bicarbonate/acetonitrile (3:1, v/v, 1.00 mL). The reaction mixture was shaken for 1 min and left standing at room temperature. After 2 hours, the reaction mixture was subjected to preparative LC-MS.[4C] Fractions corresponding to the product were immediately pooled together, frozen and lyophilized overnight to give the title compound (3.73 mg, 64%) as a white fluffy solid. LC-MS shows multiple broad peaks with the correct m/z values.
MS (m/z): 8511 $[M+H]^+$

**Trifunctional linker (Dendron(-L-hydrazone-SO1861)$_4$)-(L-ApoB BNA oligo)-(trivalent GalNAc synthesis)** (molecule 22)

To TFL-(DBCO)-(L-ApoB BNA oligo)-(trivalent GalNAc) (1.16 mg, 0.137 μmol; molecule 21) and dendron(-L-SO1861)$_4$-azide (1.39 mg, 0.142 μmol; molecule 19) was added 20 mM ammonium bicarbonate/acetonitrile (3:1, v/v, 0.5 mL). The reaction mixture was shaken for 1 min and left standing at room temperature. After 3 hours, the reaction mixture was subjected to preparative LC-MS.[4B] Fractions corresponding to the product were immediately pooled together, frozen and lyophilized overnight to give the title compound (1.36 mg, 54%) as a white fluffy solid. Purity based on LC-MS 92% (very broad peak).
MS (m/z): 18336 $[M+H]^+$
Using a similar approach as for synthesis of Trifunctional linker (Dendron(-L-hydrazone-SO1861)4)-(L-ApoB BNA oligo)-(trivalent GalNAc synthesis) (molecule 22); a conjugate in which the ApoB BNA is replaced for a BNA with a scrambled ApoB nucleic acid sequence (molecule 12 a), can be synthesized, for control testing purposes (molecule 22a).

**Trifunctional linker-(dendron(-L-hydrazone-SO1861)8)- (BNA oligo) - (Trivalent-GalNAc) synthesis (Figure 37)**

Intermediate 11:

**dendron(-L-SO1861)$_8$-amine** (molecule 24)

(2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate (0.52 mg, 0.299 μmol, molecule 23) and SO1861-EMCH (29.2 mg, 0.014 mmol, molecule 15) were dissolved in a mixture of 20 mM $NH_4HCO_3$ with 0.5 mM TCEP/acetonitrile (3:1, v/v, 1.00 mL) and the resulting mixture was shaken for 1 min and left standing at room temperature. After 30 min, TCEP (0.30 mg, 1.05 μmol) was added and the reaction mixture was shaken for 1 min. Next, the mixture was directly subjected to preparative LC-MS.[3B] Fractions corresponding to the product were immediately pooled together, frozen and lyophilized overnight to give the title compound (2.17 mg, 40%) as a white fluffy solid. Purity based on LC-MS 97%.

LRMS (m/z): 2282 $[M-8]^{8-}$, 2607 $[M-7]^{7-}$
LC-MS r.t. (min): 4.41[5A]

Intermediate 12:

**dendron(-L-SO1861)$_8$-azide** (molecule 25)

Dendron(-L-SO1861)$_8$-amine (19.6 mg, 1.08 μmol, molecule 24) and 2,5-dioxopyrrolidin-1-yl 1-azido-3,6,9,12-tetraoxapentadecan-15-oate (4.17 mg, 10.8 μmol, molecule 18) were dissolved in DMF (1.50 mL). Next, DIPEA (1.87 μL, 10.8 μmol) was added and the mixture was shaken for 1 min and left standing overnight at room temperature. The reaction mixture was then subjected to preparative LC-MS.[4B] Fractions corresponding to the product were immediately pooled together, frozen and lyophilized overnight to give the title compound (11.7 mg, 59%) as a white fluffy solid. Purity based on LC-MS 92% (very broad peak).

LRMS (m/z): 2316 $[M-8]^{8-}$, ): 2647 $[M-7]^{7-}$
LC-MS r.t. (min): 4.29[1A]

**Trifunctional linker (Dendron(-L-hydrazone-SO1861)$_8$)-(L-ApoB BNA oligo)-(trivalent GalNAc synthesis)** (molecule 26)

To TFL-(DBCO)-(L-ApoB BNA oligo)-(trivalent GalNAc) (1.16 mg, 0.137 μmol; molecule 21) and dendron(-L-SO1861)$_8$-azide (2.72 mg, 0.142 μmol; molecule 25) was added 20 mM ammonium bicarbonate/acetonitrile (3:1, v/v, 0.5 mL). The reaction mixture was shaken for 1 min and left standing at room temperature. After 3 hours, the reaction mixture was subjected to preparative LC-MS.[4B] Fractions corresponding to the product were immediately pooled together, frozen and lyophilized overnight to give the title compound (1.6 mg, 42%) as a white fluffy solid. Purity based on LC-MS 93% (very broad peak).
MS (m/z): 27655 $[M+H]^+$

Using a similar approach as for synthesis of Trifunctional linker (Dendron(-L-hydrazone-SO1861)8)-(L-ApoB BNA oligo)-(trivalent GalNAc synthesis) (molecule 26), a conjugate in which the ApoB BNA is replaced for a BNA with a scrambled ApoB nucleic acid sequence (molecule 12 a) can be synthesized, for control testing purposes (molecule 26a).

**Trifunctional linker-(L-semicarbazone-SO1861)- (BNA oligo) - (Trivalent-GalNAc synthesis) (Figure 38)**

Intermediate 13:

**SO1861-L-semicarbazone-azide** (molecule 28)

To SO1861 (60 mg, 0.032 mmol, molecule 1) and 4-(6-azidohexanoyl)piperazine-1-carbohydrazide (36.5 mg, 0.129 mmol, molecule 27) was added methanol (extra dry, 1.00 mL) and TFA (9.86 μl, 0.129 mmol). The reaction mixture was shaken for 1 min and left standing at room temperature. After 2 hours, the reaction mixture was subjected to preparative MP-LC.[1] Fractions corresponding to the product were immediately pooled together, frozen and lyophilized overnight to give the title compound (30 mg, 44%) as a white fluffy solid. Purity based on LC-MS 99%.
MS (m/z): 2126 $[M-1]^{1-}$

Intermediate 14:

**Trifunctional linker-(L-semicarbazone-SO1861)-(TCO)-(Maleimide)** (molecule 29)

A solution of TFL-(DBCO)-(TCO)-(Maleimide) (15 mg, 12.5 μmol, molecule 4) in DMF (2.0 mL) was added to SO1861-L-azide (26.6 mg, 12.5 μmol, molecule 28). The reaction mixture was shaken for 30 min and left standing at room temperature. After 30 min, the reaction mixture was submitted to preparative MP-LC.[1C] Fractions corresponding to the product were immediately pooled together, frozen and lyophilized overnight to give the title compound (27.5 mg, 66%) as a white fluffy solid. Purity based on LC-MS 96%.
MS (m/z): 3331 $[M-1]^{1-}$

Intermediate 15:

**Trifunctional linker-(L-semicarbazone-SO1861)-(TCO)-(trivalent GalNAc)** (molecule 30)

Trivalent GalNAc-thioacetate (13.0 mg, 7.25 μmol, molecule 8) was dissolved in methanol (0.50 mL). Next, a 1 M solution of sodium hydroxide (7.98 μL, 7.98 μmol) was added. The reaction mixture was shaken for 1 min and left standing

at room temperature. After 30 min, the reaction mixture was added to a freshly prepared solution of TFL-(L-semicarbazone-SO1861)-(TCO)-(maleimide) (20.4 mg, 6.13 μmol, molecule 29) in 20 mM ammonium bicarbonate/acetonitrile (3:1, v/v, 2.00 mL).

The resulting mixture was shaken for 1 min and left standing at room temperature. After 2 hours, the reaction mixture was subjected to preparative MP-LC.[2A] Fractions corresponding to the product were immediately pooled together, frozen and lyophilized overnight to give the title compound (16.7 mg, 54%) as a white solid. Purity based on LC-MS 94%. MS (m/z): 5081 $[M-1]^{1-}$

**Trifunctional linker-(L- semicarbazone-SO1861)-(L-BNA oligo)-(trivalent GalNAc)** (molecule 31)

TFL-(L-semicarbazone-SO1861)-(TCO)-(trivalent GalNAc) (16 mg, 3.15 μmol, molecule 30) and Methyltetrazine-BNA oligo (10.2 mg, 1.83 μmol, molecule 12) were dissolved in a solution of 20 mM ammonium bicarbonate/acetonitrile (3:1, v/v, 1.00 mL). The reaction mixture was shaken for 1 min and left standing at room temperature. After 3 hours, the reaction mixture was frozen and lyophilized overnight. The crude product was dissolved in 20 mM ammonium bicarbonate (1 mL) and the resulting solution was directly subjected to preparative LC-MS.[1B] Fractions corresponding to the product were immediately pooled together, frozen and lyophilized overnight to give the title compound (11.3 mg, 59%) as a white fluffy solid. Purity based on LC-MS 92%.
MS (m/z): 10638 $[M+H]^+$

Using a similar approach as for synthesis of Trifunctional linker-(L-semicarbazone-SO1861)-(L-BNA oligo)-(trivalent GalNAc) (molecule 31); a conjugate in which the ApoB BNA was replaced for a BNA with a scrambled ApoB nucleic acid sequence (molecule 12 a), was synthesized, for control testing purposes (molecule 31a).

**Trifunctional linker - dendron(L-semicarbazone-SO1861)4 - (L-BNA oligo) - (Trivalent-GalNAc) synthesis (Figure 39)**

<u>Intermediate 16:</u>

**benzyl 4-(2-(*tert*-butoxycarbonyl)hydrazine-1-carbonyl)piperazine-1-carboxylate** (molecule 33)

To a stirring solution of phosgene in toluene (20%, w/w, 15.8 mL, 30.0 mmol) at 0 °C was added slowly (10 min) a solution of 1-Cbz-piperazine (1.93 mL, 10.0 mmol, molecule 1) in dichloromethane (25 mL) and DIPEA (3.83 mL, 22.0 mmol). The reaction mixture was stirred at room temperature. After 30 min, the reaction mixture was evaporated *in vacuo* and co-evaporated with dichloromethane (2 × 50 mL). Next, the residue was dissolved in dichloromethane (100 mL) and resulting solution was stirred at 0 °C. A solution of Boc hydrazine (2.33 mL, 15.0 mmol) in dichloromethane (20 mL) and DiPEA (3.83 mL, 22.0 mL) was added and the reaction mixture was stirred overnight at room temperature. The reaction mixture was then diluted with dichloromethane (100 mL) and the resulting solution was washed with 0.5 N potassium bisulphate solution (2 × 100 mL) and brine (100 mL), dried over $Na_2SO_4$, filtered and evaporated *in vacuo.* The residue was purified by flash chromatography (first by an ethyl acetate - heptane gradient, 5:95 (v/v) rising to 100% ethyl acetate followed by a flush with 10% methanol in DCM (v/v)) to give the title compound (2.42 g, 64%) as a white solid. Purity based on LC-MS 100%.

LRMS (m/z): 279/323/401$[M-99/M-55/M+23]^{1+}$
LC-MS r.t. (min): 1.27[1]

<u>Intermediate 17:</u>

**tert-butyl 2-(piperazine-1-carbonyl)hydrazine-1-carboxylate** (molecule 34)

To benzyl 4-(2-(*tert*-butoxycarbonyl)hydrazine-1-carbonyl)piperazine-1-carboxylate (2.42 g, 6.39 mmol, molecule 33) was added methanol (50 mL). The mixture was heated to obtain a clear solution. Next, palladium (10% on activated carbon, 50% wet with water, 1.50 g) was added and the resulting mixture was stirred under a hydrogen atmosphere by using a balloon. After 1 hour the reaction mixture was filtered over celite and the filtrate evaporated *in vacuo* to give the title product (1.56 g, quant.) as a white solid.
LRMS (m/z): 189/245 $[M-55/M+1]^{1+}$

<u>Intermediate 18:</u>

***tert*-butyl 2-(4-(6-(2,5-dioxo-2,5-dihydro-1*H*-pyrrol-1-yl)hexanoyl)piperazine-1-carbonyl)hydrazine-1-carboxylate** (molecule 36)

6-maleimidocaproic acid (810 mg, 3.84 mmol, molecule 35), *tert*-butyl 2-(piperazine-1-carbonyl)hydrazine-1-carboxylate (781 mg, 3.20 mmol, molecule 34), EDCl.HCl (735 mg, 3.84 mmol) and Oxyma Pure (591 mg, 4.16 mmol) were dissolved in a mixture of dichloromethane (25 mL) and DIPEA (835 μL, 4.80 mmol) and the reaction mixture was stirred at room temperature. After 2 hours, the reaction mixture was evaporated *in vacuo* and the residue was dissolved in ethyl acetate (50 mL). The resulting solution was washed with 0.5 N potassium bisulphate solution (50 mL), saturated sodium bicarbonate solution (2 × 50 mL) and brine (50 mL), dried over $Na_2SO_4$, filtered and evaporated *in vacuo.* The residue was purified by flash chromatography (DCM - 10% methanol in DCM (v/v) gradient 100:0 rising to 40:60) to give the title compound (634 mg, 45%) as a white solid. Purity based on LC-MS 97%.

LRMS (m/z): 338/382/460 [M-99/M-55/M+23]$^{1+}$
LC-MS r.t. (min): 1.02[1]

Intermediate 19:

**S01861-SC-Mal** (molecule 37)

*tert*-butyl 2-(4-(6-(2,5-dioxo-2,5-dihydro-1*H*-pyrrol-1-yl)hexanoyl)piperazine-1-carbonyl)hydrazine-1-carboxylate (25.0 mg, 57.1 μmol, molecule 36) was dissolved in a mixture of dichloromethane (500 μL) and TFA (500 μL) and the reaction mixture was stirred at room temperature. After 30 min, the reaction mixture was evaporated *in vacuo* and co-evaporated with dichloromethane (3 × 5 mL) and methanol (5 mL). The residue and SO1861 (21.3 mg, 11.4 μmol, molecule 1) were dissolved in methanol (extra dry, 1.00 mL) and the resulting mixture was shaken for 1 min and left standing at room temperature. After 4 hours, the reaction mixture was subjected to to preparative MP-LC.[2] Fractions corresponding to the product were immediately pooled together, frozen and lyophilized overnight to yield the title compound (13.7 mg, 55%) as a white fluffy solid. Purity based on LC-MS 97%.

LRMS (m/z): 2181 [M-1]$^{1-}$
LC-MS r.t. (min): 2.13[3]

Intermediate 20:

**dendron(L-semicarbazone-SO1861)$_4$-amine** (molecule 39)

N,N'-((9S,199)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate (2.73 mg, 3.13 μmol, molecule 38) was dissolved in a mixture of 1M NaOH and MeOH (1:1, v/v, 3.00 mL) and stirred for 30 min. Next, 5 mL of Trimethylphosphine (9.5 mg, 125 μmol) dissolved in THF was added and the mixture was stirred for 30 min. Next, SO1861-SC-Mal (30.54 mg, 0.014 mmol, molecule 37) dissolved in 20 mM $NH_4HCO_3$ with acetonitrile (3:1, v/v, 3.00 mL) was added and the reaction mixture was stirred at room temperature. After 1.5 hours, the reaction mixture was subjected to preparative LC-MS.[3B] Fractions corresponding to the product were immediately pooled together, frozen and lyophilized overnight to give the title compound (19 mg, 64 %) as a white fluffy solid. Purity based on LC-MS 95%.
MS (m/z): 9553 [M+H]$^+$

Intermediate 21:

**dendron(-L-SO1861)$^4$-azide** (molecule 40)

Dendron(L-semicarbazone-SO1861)$_4$-amine (7.1 mg, 0.748 μmol, molecule 39) and 2,5-dioxopyrrolidin-1-yl 1-azido-3,6,9,12-tetraoxapentadecan-15-oate (2.90 mg, 7.48 μmol, molecule 18) were dissolved in DMF(1.00 mL). Next, DIPEA (1.302 μL, 7.48 μmol) was added and the mixture was shaken for 1 min and left standing at room temperature. After 2 hours, the reaction mixture was subjected to preparative LC-MS.[3C] Fractions corresponding to the product were immediately pooled together, frozen and lyophilized overnight to give the title compound (6.4 mg, 87%) as a white fluffy solid. Purity based on LC-MS 93%.
MS (m/z): 9826 [M+H]$^+$

Intermediate 22

**Trivalent GalNAc-thiol** (molecule 41)

Trivalent GalNAc-thioacetate (16.2 mg, 9.02 μmol, molecule 8) was dissolved in methanol (500 μL). Next, a 1.00 M solution of sodium hydroxide (11.0 μL, 11.0 μmol) was added. The reaction mixture was shaken for 1 min and left standing at room temperature. After 30 min, the reaction mixture was submitted to preparative MP-LC.[1A] Fractions corresponding to the product were immediately pooled together, frozen and lyophilized overnight to give the title compound (13.1 mg, 83%) as a white solid. Purity based on LC-MS 98%.

LRMS (m/z): 1748 $[M-H]^{1-}$
LC-MS r.t. (min): 1.78[1A]

Intermediate 23:

**TFL-(DBCO)-(TCO) - (trivalent GalNAc)** (molecule 20)

TFL-(DBCO)-(TCO)-(Mal) (15.0 mg, 12.4 μmol, molecule 4) was dissolved in acetonitrile (0.50 mL). Next, a solution of 20 mM ammonium bicarbonate (1.50 mL) was added and the resulting solution was directly transferred to trivalent GalNAc-thiol (22.7 mg, 13.0 μmol, molecule 41). The reaction mixture was shaken for 1 min and left standing at room temperature. After 1 hour, the reaction mixture was frozen and lyophilized overnight to yield the crude title product as a white solid. Purity based on LC-MS 84%.

LRMS (m/z): 2409 $[M-546]^{1-}$, 2955 $[M-1]^{1-}$
LC-MS r.t. (min): 2.63[1B]

Intermediate 24:

**TFL-(dendron(L-semicarbazone-SO1861)4)-(TCO)-(trivalent GalNAc)** (molecule 41)

Dendron(L-semicarbazone-SO1861)$_4$-azide (5.2 mg, 0.529 μmol, molecule 40) and TFL-(DBCO)-(TCO)-(trivalent GalNAc) (1.5 mg, 0.5 μmol, molecule 20) were dissolved in DMF (2.00 mL). The mixture was shaken for 10 min and left standing at room temperature. After 2 hours, the reaction mixture was subjected to preparative LC-MS.[3C] Fractions corresponding to the product were immediately pooled together, frozen and lyophilized overnight to give the title compound (6.1 mg, 90%) as a white fluffy solid. Purity based on LC-MS 93%.
MS (m/z): 12781 $[M+H]^+$

**TFL-(-dendron(L-semicarbazone-SO1861)4)-(L-BNA oligo)-(trivalent GalNAc)** (molecule 42)

TFL-(dendron(L-semicarbazone-SO1861)4)-(TCO)-(trivalent GalNAc) (5.2 mg, 0.41 μmol, molecule 41) and Methyltetrazine-BNA oligo (2.7 mg, 0.5 μmol, molecule 12) were dissolved in a solution of 20 mM ammonium bicarbonate/acetonitrile (3:1, v/v, 1.00 mL). The reaction mixture was shaken for 1 min and left standing at room temperature. After 3 hours the reaction mixture was frozen and lyophilized overnight. The crude product was dissolved in 20 mM ammonium bicarbonate (1 mL) and the resulting solution was directly subjected to preparative LC-MS.[1B] Fractions corresponding to the product were immediately pooled together, frozen and lyophilized overnight to give the title compound (1.8 mg, 24%) as a white fluffy solid. Purity based on LC-MS 96% (multiple (broad) peaks due to regioisomers).
MS (m/z): 18336 $[M+H]^+$

Using a similar approach as for synthesis of Trifunctional linker (Dendron(-L-semicarbazone-SO1861)4)-(L-ApoB BNA oligo)-(trivalent GalNAc synthesis) (molecule 42), a conjugate in which the ApoB BNA is replaced for a BNA with a scrambled ApoB nucleic acid sequence (molecule 12 a) can be synthesized for control testing purposes (molecule 42a).

**Trifunctional linker-(dendron(-L- semicarbazone -SO1861)8)- BNA oligo - Trivalent-GalNAc synthesis (Figure 40)**

Intermediate 25:

**dendron(L-semicarbazone-SO1861)$^8$-amine** (molecule 44)

(2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate (0.61 mg, 0.299 μmol, molecule 43) was dissolved in a mixture of 1M NaOH and MeOH (1:1, v/v, 3.00 mL) and stirred for 30 min. Next, 5 mL of Trimethylphosphine (2 mg, 24 μmol) dissolved in THF was added and the mixture was stirred for 30 min. Next, SO1861-SC-Mal (30.54 mg, 0.014 mmol, molecule 37) dissolved in 20 mM $NH_4HCO_3$ with acetonitrile (3:1, v/v, 3.00 mL) was added and the reaction mixture was stirred at room temperature. After 1.5 hours, the reaction mixture was subjected to preparative LC-MS.[3B] Fractions corresponding to the product were immediately pooled together, frozen and lyophilized overnight to give the title compound (4.6 mg, 81 %) as a white fluffy solid. Purity based on LC-MS 95%.
MS (m/z): 19146 $[M+H]^+$

Intermediate 26:

**dendron(-L-SO1861)$_8$-azide** (molecule 45)

Dendron(L-semicarbazone-SO1861)$_8$-amine (5.4 mg, 0.28 μmol, molecule 44) and 2,5-dioxopyrrolidin-1-yl 1-azido-3,6,9,12-tetraoxapentadecan-15-oate (1.1 mg, 2.8 μmol, molecule 18) were dissolved in DMF(1.00 mL). Next, DIPEA (1.302 μL) was added and the mixture was shaken for 1 min and left standing at room temperature. After 2 hours, the reaction mixture was subjected to preparative LC-MS.[3C] Fractions corresponding to the product were immediately pooled together, frozen and lyophilized overnight to give the title compound (4.35 mg, 80%) as a white fluffy solid. Purity based on LC-MS 93%.
MS (m/z): 19420 $[M+H]^+$

Intermediate 27:

**TFL-(dendron(L-semicarbazone-SO1861)8)-(TCO)-(trivalent GalNAc)** (molecule 46)

Dendron(L-semicarbazone-SO1861)$_8$-azide (10.3 mg, 0.529 μmol, molecule 45) and TFL-(DBCO)-(TCO)-(trivalent GalNAc) (1.5 mg, 0.5 μmol, molecule 20) were dissolved in DMF (2.00 mL). The mixture was shaken for 10 min and left standing at room temperature. After 2 hours, the reaction mixture was subjected to preparative LC-MS.[3C] Fractions corresponding to the product were immediately pooled together, frozen and lyophilized overnight to give the title compound (8.75 mg, 74 %) as a white fluffy solid. Purity based on LC-MS 91%.
MS (m/z): 22373 $[M+H]^+$

**TFL-(dendron(-L-semicarbazone-SO1861)8)-(L-BNA oligo)-(trivalent GalNAc)** (molecule 47)

TFL-(dendron(L-semicarbazone-SO1861)8)-(TCO)-(trivalent GalNAc) (9.2 mg, 0.41 μmol, molecule 46) and Methyltetrazine-BNA oligo (2.8 mg, 0.5 μmol, molecule 12) were dissolved in a solution of 20 mM ammonium bicarbonate/acetonitrile (3:1, v/v, 1.00 mL). The reaction mixture was shaken for 1 min and left standing at room temperature. After 3 hours, the reaction mixture was frozen and lyophilized overnight. The crude product was dissolved in 20 mM ammonium bicarbonate (1 mL) and the resulting solution was directly subjected to preparative LC-MS.[1B] Fractions corresponding to the product were immediately pooled together, frozen and lyophilized overnight to give the title compound (1.26 mg, 11 %) as a white fluffy solid. Purity based on LC-MS 97%.
MS (m/z): 27928 $[M+H]^+$

Using a similar approach as for synthesis of Trifunctional linker (Dendron(-L-semicarbazone-SO1861)8)-(L-ApoB BNA oligo)-(trivalent GalNAc synthesis) (molecule 47), a conjugate in which the ApoB BNA is replaced for a BNA with a scrambled ApoB nucleic acid sequence (molecule 12 a) can be synthesized for control testing purposes (molecule 47a).

**Trivalent linker-(L-SPT001)-(blocked TCO)-(trivalent GalNAc) synthesis (Figure 41)**

Intermediate 28:

***N*-(2-hydroxyethyl)-2-(4-(6-methyl-1,2,4,5-tetrazin-3-yl)phenyl)acetamide** (molecule 50)

To a solution of methyltetrazine-NHS ester (30.0 mg, 91.7 μmol, molecule 48) in DMF (1.00 mL) was added ethanolamine (11.1 μL, 0.184 mmol, molecule 49) and triethylamine (25.5 μL, 0.183 mmol). The reaction mixture was shaken for 1 min and left standing at room temperature. After 1 hour, the reaction mixture was submitted to preparative MP-LC.[1B] Fractions corresponding to the product were immediately pooled together, frozen and lyophilized overnight to give

the title compound (19.2 mg, 77%) as a purple solid. Purity based on LC-MS 89%.

LRMS (m/z): 274 $[M+1]^{1+}$
LC-MS r.t. (min): 0.85[2]

TCO blocking on TFL has been performed on the following conjugates:

TFL-(L-hydrazone-SPT001)-(blocked TCO)-(trivalent GalNAc) (molecule 51)
TFL-(dendron (L-hydrazone-SPT001)4)-(blocked TCO)-(trivalent GalNAc) (molecule 52)
TFL-(dendron-(L-hydrazone-SPT001)8)-(blocked TCO)-(trivalent GalNAc) (molecule 53)
TFL-(L-semicarbazone-SPT001)-(blocked TCO)-(trivalent GalNAc) (molecule 54)
TFL-(dendron((L-semicarbazone-SPT001)4)-(blocked TCO)-(trivalent GalNAc) (molecule 55)
TFL-(dendron(L-semicarbazone-SPT001)8)-(blocked TCO)-(trivalent GalNAc) (molecule 56)

The procedure is exemplary described for TFL-(L-hydrazone-SPT001)-(blocked TCO)-(trivalent GalNAc) (molecule 51):

**TFL-(L-hydrazone-SPT001)-(blocked TCO)-(trivalent GalNAc)** (molecule 51)

A solution of TLF-(DBCO)-(TCO)-(trivalent GalNAc) (36.8 mg, 12.5 μmol, molecule 20) in DMF (2.0 mL) was added to SO1861-L-hydrazone-azide (26.8 mg, 12.5 μmol, molecule 3). The reaction mixture was shaken for 1 min and left standing at room temperature. After 30 min, N-(2-hydroxyethyl)-2-(4-(6-methyl-1,2,4,5-tetrazin-3-yl)phenyl)acetamide (4.08 mg, 14.9 μmol, molecule 50) was added. The reaction mixture was shaken for 1 min and left standing at room temperature. After 30 min, the reaction mixture was submitted to preparative MP-LC.[1C] Fractions corresponding to the product were immediately pooled together, frozen and lyophilized overnight to give the title compound (37.3 mg, 56%) as a white fluffy solid. Purity based on LC-MS 96% (double peaks due to regioisomers).

LRMS (m/z): 2676 $[M-2]^{2-}$
LC-MS r.t. (min): 2.23[1B]

**Trivalent linker-(blocked DBCO)-(L-BNA oligo)-(trivalent GalNAc) synthesis (Figure 42)**

Intermediate 29:

**TFL-(blocked DBCO)-(TCO)-(trivalent GalNAc)** (molecule 58)

A solution of 1-azido-3,6,9-trioxaundecane-11-ol (2.17 mg, 9.88 μmol, molecule 57) in DMF (0.50 mL) was added to TFL-(DBCO)-(TCO)-(trivalent GalNAc) (14.6 mg, 4.94 μmol, molecule 20). The reaction mixture was shaken for 1 min and left standing at room temperature. After 2 hours, the reaction mixture was submitted to preparative MP-LC.[1C] Fractions corresponding to the product were immediately pooled together, frozen and lyophilized overnight to give the title compound (10.00 mg, 64%) as a white solid. Purity based on LC-MS 98%.

MS (m/z): 3175 $[M+H]^+$
LC-MS r.t. (min): 2.33[1B]

**Trivalent linker-(blocked DBCO)-(BNA oligo)-(trivalent GalNAc)** (molecule 59)

TFL-(blocked DBCO)-(TCO)-(trivalent GalNAc) (10.0 mg, 3.15 μmol, molecule 58) and Methyltetrazine-BNA oligo (10.0 mg, 1.83 μmol, molecule 12) were dissolved in a solution of 20 mM ammonium bicarbonate/acetonitrile (3:1, v/v, 1.00 mL). The reaction mixture was shaken for 1 min and left standing at room temperature. After 3 hours the reaction mixture was frozen and lyophilized overnight. The crude product was dissolved in 20 mM ammonium bicarbonate (1 mL) and the resulting solution was directly subjected to preparative LC-MS.[1B] Fractions corresponding to the product were immediately pooled together, frozen and lyophilized overnight to give the title compound (11.3 mg, 72%) as a white fluffy solid. Purity based on LC-MS 95%.

LRMS (m/z): 2149 $[M-4]^{4-}$, 2866 $[M-3]^{3-}$
LC-MS r.t. (min): 2.92[1A]

Using a similar approach as for synthesis of Trivalent linker-(blocked DBCO)-(BNA oligo)-(trivalent GalNAc) (molecule 59), a conjugate in which the ApoB BNA is replaced for a BNA with a scrambled ApoB nucleic acid sequence (molecule 12 a) can be synthesized for control testing purposes (molecule 59 a).

An overview of all conjugates 'CONJUGATE C1' produced by applying the Trifucntional linker (TFL) methodology is shown in Figure 43A and B. In addition, a conjugate according to the invention, comprising a single GalNAc moiety, has been synthesized and tested.

**Cell-titer Glo (CTG) cell viability assay**

After compound addition, cells were incubated for 72 hr at 37°C before the cell viability was determined using a luminescence-based cell viability assay performed according to the manufacturer's instruction (CellTiter-Glo® 2.0 Cell Viability Assay, Promega). Briefly, the cell plate was first equilibrated to RT for 30 minutes. Next, to each well containing 120 μL treatment media, 120 μL CTG solution was added. The plate was briefly mixed (10 sec, 600 rpm) and incubated for approximately 10 minutes in the dark at RT. Subsequently, the luminescence signal was measured on a Spectramax ID5 plate reader (Molecular Devices). For quantification, the background signal of 'medium only' wells was subtracted from all other well values before the relative cell viability percentage of treated cells was calculated by dividing the background corrected signal of treated wells by the background corrected signal of the untreated wells (x 100 for percentage).

**Human primary hepatocyte treatment**

Cryopreserved primary human hepatocytes (Cytes Biotechnologies S.L., Spain) were thawed in hepatocyte thawing media (Cytes Biotechnologies S.L., Spain). Cells were then re-suspended in hepatocyte plating media (Cytes Biotechnologies S.L., Spain) and seeded onto collagen-I coated plates at a density of 215.600 cells/well or 66.600 cells/well in 48 or 96-well plates, respectively (Greiner BioOne). Cells were pre-cultured for 4-6 hrs at 37°C allowing for cell attachment to cell culture plates before the start of treatment. Plating medium was replaced by 315 μl or 108 μl maintenance media (Cytes Biotechnologies S.L., Spain), after which conjugates were added from a 10x concentrated stock solution in PBS. Plates were incubated for 72 hr at 37°C being harvested for gene expression and cell viability analysis.

**ApoB#02 oligo sequences**

Murine and human sequence-compatible ApoB#02 (5'-GCattggtatTCA-3') [SEQ ID NO: 12] was a $BNA^{NC}$ oligonucleotide and synthesized with 5'-Thiol C6 linker at Bio-Synthesis Inc, with $BNA^{NC}$ bases in capitals, and fully phosphorothioated backbones (Lewisville, Texas).

**RNA isolation and gene expression analysis from primary mouse and human hepatocytes**

RNA from cells was isolated using TRI Reagent® Solution (Thermo Scientific) according to the manufacturer's instruction. Conversion into cDNA was performed using iScript™ cDNA Synthesis Kit (BioRad) using standard protocols. *ApoB* expression levels and levels of specific hepatocyte housekeeping genes were determined using quantitative real-time PCR assays (qRT-PCR) using iTaq™ Universal SYBR® Green Supermix (BioRad) and the Light Cycler 480 (Roche Diagnostics, Rotkreuz, Switzerland) with specific DNA primers, listed in Table A4 (study A) and Table A7 (study B), respectively. Analysis was done by the ΔCt method to determine apoB expression relative to 2 hepatocyte-specific housekeeping control mRNAs. Each analysis reaction was performed in triplicate.

**Table A4.** Primers used in qPCR analysis of mouse primary hepatocytes/liver tissue analysis (study A)

| | | |
|---|---|---|
| ApoB#02 | Forward | GCTAACACTAAGAACCAGAAGATC [SEQ ID NO: 13] |
| | Reverse | TGTCCGTCTAAGGATCCTGC [SEQ ID NO: 14] |
| Mm PPIA | Forward | GCGGCAGGTCCATCTACG [SEQ ID NO: 15] |
| | Reverse | GCCATCCAGCCATTCAGTC [SEQ ID NO: 16] |
| Mm SDHA | Forward | GAGGAAGCACACCCTCTCAT [SEQ ID NO: 17] |
| | Reverse | GGAGCGGATAGCAGGAGGTA [SEQ ID NO: 18] |

**FACS analysis**

Cells were seeded in DMEM (PAN-Biotech GmbH) supplemented with 10% fetal bovine serum (PAN-Biotech GmbH) and 1% penicillin/streptomycin (PAN-Biotech GmbH), in T75 flasks at appropriate density for each cell-line. Cultures were incubated for 72-96 hrs (5% $CO_2$, 37°C) until a confluency of 90% was reached. Next, the cells were trypsinized (TryplE Express, Gibco Thermo Scientific) to obtain a single cell suspension, transferred to a 15 mL falcon tube, and centrifuged (1,400 rpm, 3 min). The supernatant was discarded while leaving the cell pellet submerged. 500.000 cells were transferred to round bottom FACS tubes and washed with 3 mL cold DPBS ($Mg^{2+}$ and $Ca^{2+}$ free, 2% FBS). The cells were centrifuged at 1800 rpm, 3 min 4°C and resuspended in 200 μL cold DPBS ($Mg^{2+}$ and $Ca^{2+}$ free, 2% FBS) or 200 μL antibody solution; containing 5 μL antibody in 195 μL cold DPBS ($Mg^{2+}$ and $Ca^{2+}$ free, 2% FBS). PE anti-human CD71 (#334106, Biolegend) was used to stain the transferrin receptor, PE Mouse IgG2a, κ isotype Ctrl FC (#400212, Biolegend) was used as its matched isotype control. PE anti-human ASGPR1 (#130-122-963, Miltenyi) was used to stain the ASGPR1 receptor and PE Mouse IgG1 isotype Ctrl (#130-113-762, Miltenyi) was used as its matched isotype control. Samples were incubated for 30 min. at 4°C. Afterwards, the cells were washed 2x with cold DPBS ($Mg^{2+}$ and $Ca^{2+}$ free, 2% FBS) and fixated for 20 min at room temperature using a 2% PFA solution in DPBS ($Mg^{2+}$ and $Ca^{2+}$ free, 2% FBS). Cells were washed 1x with cold DPBS and resuspended in 1000 μL cold DPBS for FACS analysis. Samples were analyzed with a Sysmex Cube 8 flow cytometry system (Sysmex) and FCS Express 7 Research edition software. Results of the FACS analyses are summarized in Table A3.

***In vivo* tolerability and efficacy of trivalent GalNAc-conjugates in** a **C57BL/6J model**

i. Dose administration and in-life phase. Per group, 8 C57BL/6J male mice for treatment groups (in Study A, 14 mice in vehicle control group), approximately 6 - 7 weeks at arrival and 8 - 9 weeks at dosing, were dosed. The compounds, formulated in PBS or PBS alone (vehicle) according to Table A5 and Table A6, were dosed at 5 ml/kg in the tail vain (intravenously, i.v.), with dose volumes corresponding to the individual bodyweights. Where applicable, trivalent (GalNAc)3-SO1861 (EMCH) was co-administered at 5 ml/kg in the tail vain (i.v.) right after the trivalent GalNAc-ApoB conjugate. Animals were weighed weekly and clinical observations were performed 2 - 3 times daily within the first 48 hrs, and at least once daily thereafter until study end.

ii. Sampling of serum, liver, and kidney tissue. Blood was sampled before dosing (pre-dose) and at indicated post-dose timepoints (e.g., 24 hrs, 72 hrs, 196 hrs, 336 hrs, 672 hrs post-dose), and serum was generated from the collected blood for biomarker analysis, using standard procedures. At the relevant inter-study post-dose timepoints, 3 animals per timepoint per treatment group (6 animals in the vehicle control group in Study A) were sacrificed to collect terminal bleeds and collect relevant organs, such as liver and kidney tissues for further analyses. at end of study, the remaining 2 animals (4 in the control group in Study A) per group were sacrificed. One half of the liver and one kidney was frozen and used for tissue RNA analysis.

**Table A5.** Experimental design *in vivo* tolerability and efficacy of trivalent GalNAc-conjugates, Study A

| Group | Conjugate | Dose [mg/kg] | Relative BNA dose administered [mg/kg] | Co-administered trivalent GalNAc-SO1861-EMCH dose [mg/kg] |
|---|---|---|---|---|
| 1 | PBS (vehicle) | N/A | 0 | N/A |
| 2 | ApoB#02 BNA | 0.1 | 0.1 | N/A |
| 3 | ApoB#02 BNA | 1 | 1 | N/A |
| 4 | ApoB#02 BNA | 2 | 2 | N/A |
| 5 | (GalNAc)3-ApoB#02 BNA | 0.1 | 0.054 | N/A |
| 6 | (GalNAc)3-ApoB#02 BNA | 1 | 0.54 | N/A |
| 7 | (GalNAc)3-ApoB#02 BNA | 0.01 | 0.0054 | 5 |
| 8 | (GalNAc)3-ApoBBNA#02 | 0.1 | 0.054 | 5 |
| 9 | (GalNAc)3-SO1861 ApoB#02 (EMCH) BNA | 1 | 0.44 | N/A |

(continued)

| Group | Conjugate | Dose [mg/kg] | Relative BNA dose administered [mg/kg] | Co-administered trivalent GalNAc-SO1861-EMCH dose [mg/kg] |
|---|---|---|---|---|
| 10 | (GalNAc)3-SO1861 ApoB#02 (semicarbazone) BNA | 1 | 0.44 | N/A |

**Table A6.** Experimental design *in vivo* tolerability and efficacy of trivalent GalNAc-conjugates, Study B

| Group | Conjugate | Dose [mg/kg] | Relative BNA dose administered [mg/kg] | Co-administered trivalent GalNAc-SO1861-EMCH dose [mg/kg] |
|---|---|---|---|---|
| 1 | PBS (vehicle) | N/A | 0 | N/A |
| 2 | ApoB#02 BNA | 2 | 2 | N/A |
| 3 | (GalNAc)3-SO1861 ApoB#02 (semicarbazone) BNA | 1 | 0.44 | N/A |
| 4 | (GalNAc)3-SO1861 ApoB#02 (semicarbazone) BNA | 0.1 | 0.044 | N/A |
| 5 | (GalNAc)3-dSPT4-ApoB#02 (semicarbazone) BNA | 0.173 | 0.044 | N/A |
| 6 | (GalNAc)3-dSPT8-ApoB#02 (semicarbazone) BNA | 0.265 | 0.044 | N/A |
| 7 | (GalNAc)3-ApoB#02 BNA | 1 | 1 | 0.1 |
| 8 | (GalNAc)3-ApoBBNA#02 | 0.1 | 0.1 | 0.1 |

**Biomarker RNA analysis in liver tissue**

The mRNA was isolated from frozen liver tissue using TRIzol® Reagent (Thermo Scientific) according to the manufacturer's instruction. Conversion into cDNA was performed using iScript™ cDNA Synthesis Kit (BioRad). *ApoB* expression levels and levels of specific liver housekeeping genes were determined using quantitative real-time PCR assays (qRT-PCR) using iTaq™ Universal SYBR® Green Supermix (BioRad) and the Light Cycler 480 (Roche Diagnostics, Rotkreuz, Switzerland) with specific DNA primers, listed in Table A4 (Study A) and Table A7 (Study B), respectively. Analysis was done by the $\Delta$Ct method to determine *apoB* expression relative to 2 liver-specific housekeeping control mRNAs. Each analysis reaction was performed in triplicate.

**Table A7.** Primers used in qPCR analysis of liver tissue (Study B)

| | | |
|---|---|---|
| ApoB#02 | Forward | GCTAACACTAAGAACCAGAAGATC [SEQ ID NO: 13] |
| | Reverse | TGTCCGTCTAAGGATCCTGC [SEQ ID NO: 14] |
| Mm RPS17 | Forward | GTGCGAGGAGATCGCCATTA [SEQ ID NO: 19] |
| | Reverse | ATCCGCTTCATCAGATGCGT [SEQ ID NO: 20] |
| Mm PPIA | Forward | GCGGCAGGTCCATCTACG [SEQ ID NO: 15] |
| | Reverse | GCCATCCAGCCATTCAGTC [SEQ ID NO: 16] |

**Biomarker analysis apoB protein**

ApoB protein levels in mouse serum was determined using an ApoB ELISA (ab230932, Abcam, Cambridge, UK), according to the manufacturer's instruction.

**Biomarker analysis cholesterol**

LDL-cholesterol was measured in mouse serum, using an LDL-cholesterol specific two-step AU480 assay kit, respectively, from Beckman Coulter, with a photometric measurement according to the manufacturer's instructions.

**Biomarker analysis cholesterol**

Serum alanine transaminase (ALT) levels was measured using an AU480 assay kit from Beckman Coulter (photometric measurements), according to the manufacturer's instructions.

*Results*

***In vivo efficay* of $(GalNAc)_3$-SO1861-ApoB#02 *in C57BL/6J mice (Study A)***

In Study A, C57BL/6J mice were treated as described and dosed according to Table A5. The conjugates used in this study, $(GalNAc)_3$-ApoB#02, $(GalNAc)_3$-SO1861, $(GalNAc)_3$-SO1861-ApoB#02 (EMCH) and $(GalNAc)_3$-SO1861-ApoB#02 (sc) were produced as described in Figure 35 and Figure 38, Figure 41 and Figure 42. Clinical observations were recorded and different biomarkers of efficacy and tolerability were analysed.

As Figure 25 shows, after 24 hrs, the *apoB* RNA levels, *i.e.* gene expression, was not noticeably reduced in animals treated with 0.1 mg/kg ApoB#02 BNA (ApoB#02), and ca 15% and 21% reduced in groups dosed with 1 mg/kg or 2 mg/kg ApoB#02 BNA, respectively (all n = 3 animals per group), compared to the vehicle-dosed group (n = 5 animals). Likewise, $(GalNAc)_3$-ApoB#02, at a dose of 1 mg/kg (corresponding to 0.54 mg/kg ApoB#02 BNA) reduced *ApoB* expression by ca 53% (n = 3 animals) compared to the vehicle control after 24 hrs, while 0.1 mg/kg $(GalNAc)_3$-ApoB#02 alone did not show a noticeable reduction (n = 3) compared to vehicle. In contrast, when 0.1 mg/kg $(GalNAc)_3$-ApoB#02 was co-administered with 5 mg/kg $(GalNAc)_3$-SO1861, *apoB* expression was readily reduced by ca 69% (n = 2 animals) already after 24 hrs; while 0.01 mg/kg (GalNAc)3-ApoB#02, when co-administered with 5 mg/kg (GalNAc)3-SO1861 still show a minor reduction of 16% (n = 2 animals), compared to vehicle. Most notably, treatment with 1 mg/kg $(GalNAc)_3$-SO1861-ApoB#02 BNA (corresponding to 0.44 mg/kg ApoB#02 BNA) resulted in and ca 77%% reduction for $(GalNAc)_3$-SO1861-ApoB#02 BNA (EMCH) and 90% reduction for $(GalNAc)_3$-SO1861-ApoB#02 BNA (sc), compared to control already 24 hrs post dosing (n = 3 animals in both groups each).

After 72 hrs, the highest dose of ApoB#02 BNA administered (2 mg/kg) showed a maximal reduction of 39% (n = 3 animals) compared to vehicle (n = 5 animals), while 1 mg/kg $(GalNAc)_3$-ApoB#02 resulted in a 68% reduction of *apoB* expression (n = 3 animals). Again, in contrast, the 10-fold lower dose of 0.1 mg/kg $(GalNAc)_3$-ApoB#02, when co-administered with 5 mg/kg $(GalNAc)_3$-SO1861, resulted in even 82% reduction of *apoB* expression levels (n = 3 animals), while 0.1 mg/kg ($(GalNAc)_3$-ApoB#02 alone only show 12% reduction (n = 3 animals). Treatment with different 1 mg/kg $(GalNAc)_3$-SO1861-ApoB#02 BNA conjugates resulted in ca 76% reduction for $(GalNAc)_3$-SO1861-ApoB#02 BNA (EMCH) and 96% reduction for $(GalNAc)_3$-SO1861-ApoB#02 BNA (sc) (n = 3 animals in both groups each). The effect was durable, and after 336 hrs, *apoB* expression levels were reduced by 30% for 2 mg/kg ApoB#2 BNA (n = 2 animals) and 25% for 1 mg/kg $(GalNAc)_3$-ApoB#02. The co-administration group of 10-fold less payload (ie, 0.1 mg/kg $(GalNAc)_3$-ApoB#02 + 5 mg/kg (GalNAc)3-SO1861 still showed 25% decrease. Treatment with different 1 mg/kg $(GalNA_C)_3$-SO1861-ApoB#02 BNA conjugates resulted in ca 61% reduction for $(GalNA_C)_3$-SO1861-ApoB#02 BNA (EMCH) and 65% reduction for $(GalNAc)_3$-SO1861-ApoB#02 BNA (sc) (n = 3 animals in both groups each). In conclusion, conjugation of SO1861 to the payload markedly improves payload potency also *in vivo.*

As Figure 26 shows, the *apoB* mRNA down-modulation in the liver (shown in Figure 25) also translated to apoB protein reduction in serum. After 24 hrs, the apoB protein levels were reduced by over 50% for both, the 1 mg/kg $(GalNAc)_3$-ApoB#02 (n = 8 animals, 54%) and equally so for the co-administration group with 10-fold less $(GalNAc)_3$-ApoB#02 + 5 mg/kg $(GalNAc)_3$-SO1861, i.e., 0.1 mg/kg $(GalNAc)_3$-ApoB#02 + 5 mg/kg $(GalNAc)_3$-SO1861 (n = 8 animals, 56%); most noticeably, they were reduced for 1 mg/kg $(GalNAc)_3$-SO1861-ApoB#02 (EMCH) group (n = 8 animals, 69% reduction) and 1 mg/kg $(GalNAc)_3$-SO1861-ApoB#02 (sc) group (n = 8 animals, 83% reduction) compared to controls (n = 14 animals). After 72 hours, while all treatment groups showed reduction in apoB protein, compared to vehicle controls, the most pronounced effect was observed for 1 mg/kg $(GalNAc)_3$-SO1861-ApoB#02 (EMCH) group (n = 5 animals, 95% reduction) and 1 mg/kg $(GalNAc)_3$-SO1861-ApoB#02 (sc) group (n = 5 animals, 97% reduction), or when co-administering 0.1 mg/kg $(GalNAc)_3$-ApoB#02 with 5 mg/kg $(GalNAc)_3$-SO1861, resulting in an apoB reduction of almost 90% (n = 5 animals). Even 0.01 mg/kg $(GalNAc)_3$-ApoB#02 reduced ApoB protein by ca 25% when co-administering it with 5 mg/kg $(GalNAc)_3$-SO1861 (n = 4 animals), while the 10-fold higher dose of 0.1 mg/kg $(GalNAc)_3$-ApoB#02 alone reduced apoB protein by 35% (n = 5 animals) after 72 hrs post-dose. The effect was durable and apoB protein reduction was still observable at 192 and 336 hrs for all treatment groups analyzed; NB, levels for 0.1 mg/kg ApoB#02 (196 hrs), 0.01 mg/kg $(GalNAc)_3$-ApoB + 5 mg/kg $(GalNAc)_3$-SO1861 (196 hrs and 336 hrs) and 0. 1 mg/kg $(GalNAc)_3$-ApoB + 5 mg/kg $(GalNAc)_3$-SO1861 (196 hrs) are not reported. At 192 hrs, 1 mg/kg $(GalNAc)_3$-ApoB#02 showed 47% reduction (n = 2 animals) but 1 mg/kg $(GalNAc)_3$-SO1861-ApoB#02 (EMCH) and 1 mg/kg $(GalNAc)_3$-SO1861-ApoB#02 (sc) showed 87% and 90% reduction, respectively (n = 2 animals per group). At 336 hrs, 1 mg/kg $(GalNAc)_3$-ApoB#02 showed 28% reduction (n = 2 animals) but 1 mg/kg $(GalNAc)_3$-SO1861-ApoB#02 (EMCH) and 1 mg/kg $(GalNAc)_3$-SO1861-ApoB#02 (sc) showed still 66% and 65% reduction, respectively (n = 2 animals per group).

As Figure 27 shows, the observed serum apoB protein reduction (shown in Figure 26) translated well to serum LDL-cholesterol (LDL-C) levels. After 24 hrs, LDL-C levels were not noticeably reduced in animals treated with either 0.1, 1, or 2 mg/kg ApoB#02 BNA (n = 3 animals per group) compared to vehicle (n = 5 animals). Targeted $(GalNAc)_3$-ApoB#02 at a dose of 1 mg/kg reduced LDL-C to an average of 2.7 mg/dl within 24 hrs already (n = 3 animals), i.e., a 53% reduction compared to vehicle and ApoB#02 BNA groups. The same reduction of LDL-C to an average of 2.7 mg/dl within 24 hrs (n = 3 animals), *i.e.* a 53% reduction, was already observed with a 10-fold lower dose of 0.1 mg/kg $(GalNAc)_3$-ApoB#02 when co-administering it with 5 mg/kg $(GalNAc)_3$-SO1861, while 1 mg/kg $(GalNAc)_3$-SO1861-ApoB#02 (EMCH) and 1 mg/kg $(GalNAc)_3$-SO1861-ApoB#02 (sc) showed a reduction of 66% and 77%, respectively. After 72 hrs, the highest dose of ApoB#02 BNA (2 mg/kg) showed a ca 50% reduction (n = 3 animals) in LDL-C compared to vehicle (n = 5 animals), while 1 mg/kg $(GalNAc)_3$-ApoB#02 was 72% reduced (n = 3 animals), and the 10-fold lower dose of 0.1 mg/kg $(GalNAc)_3$-ApoB#02 + 5 mg/kg $(GalNAc)_3$-SO1861 was even 78% reduced (n = 3 animals); 1 mg/kg $(GalNAc)_3$-SO1861-ApoB#02 (EMCH) showed 72% reduction, while in the 1 mg/kg $(GalNAc)_3$-SO1861-ApoB#02 (sc), no LDL-C above LOD remained measurable, i.e., a 100% reduction in apoB protein compared to control (all n = 3 animals). The effect was durable, and after 336 hrs, LDL-C levels were still reduced (in n = 2 animals for all groups) by ca 40% for 2 mg/kg ApoB#02 BNA, and 48% for both, the 1 mg/kg $(GalNAc)_3$-ApoB#02 and for the co-administration group of 0.1 mg/kg $(GalNAc)_3$-ApoB#02 + 5 mg/kg $(GalNAc)_3$-SO1861, showing a 10-fold improved LDL-C reduction based in administered payload in the latter. Again, the most pronounced reductions were observed in the groups with 1 mg/kg $(GalNAc)_3$-SO1861-ApoB#02 (EMCH) with 74% reduction and 1 mg/kg $(GalNAc)_3$-SO1861-ApoB#02 (sc) showed a reduction of 57%.

In conclusion, this shows that the potency of ApoB#02 is markedly increased in combination with SO1861, is effectively inducing *apoB* RNA down-modulation, *i.e.*, gene silencing in the livers of C57BL/6J mice, and of all 'downstream' serum biomarkers of efficacy measured, such as apoB protein and LDL-cholesterol.

***In vivo tolerability* of (GalNAc)3- SO1861-ApoB#02 *in C57BL/6J mice (Study A)***

The conjugates used in this study, $(GalNAc)_3$-ApoB#02, $(GalNAc)_3$-SO1861, $(GalNAc)_3$-SO1861-ApoB#02 (EMCH) and $(GalNAc)_3$-SO1861-ApoB#02 (sc) were produced as described in Figure 35, Figure 38, Figure 41 and Figure 42).

During the course of the study, all treatments, doses, and dose regimens as described in Table A5 were well tolerated and showed no treatment-specific adverse findings or any specific clinical observations indicative of the conjugates not being tolerated. The mice in treatment groups gained weight comparable to the mice receiving vehicle. Serum ALT levels were determined and results are shown in Figure 28. During the study period, ALT levels for vehicle mice ranged from an average 47 to 61 U/L per group (n = 5 animals for vehicle). Levels of ALT transiently increased in the different dose groups at different time points, with sometimes large per-animal variation per group, without showing specific treatment relationship or dose relationship (n = 3 for all groups at 24 hrs and 72 hrs, respectively, except for 0.01 mg/kg $(GalNAc)_3$-ApoB#02 + 5 mg/kg $(GalNAc)_3$-SO1861, where n = 2 for both time points). Notably, after 336 hrs, ALT levels in all dosing groups were back to baseline and comparable to vehicle controls (n = 2 for all groups).

In conclusion, this shows that ApoB#02, $(GalNAc)_3$-ApoB#02, $(GalNAc)_3$-SO1861 (alone or in combination with $(GalNAc)_3$-ApoB#02), and $(GalNAc)_3$-SO1861-ApoB#02 (EMCH) and $(GalNAc)_3$-SO1861-ApoB#02 (sc) are well tolerated in the doses and dose regimen applied, and particularly, that administration of SO1861-containing conjugates $(GalNAc)_3$-SO1861-ApoB#02 (EMCH) and $(GalNAc)_3$-SO1861-ApoB#02 (sc) as well as co-administration of $(GalNAc)_3$-SO1861 with $(GalNAc)_3$-ApoB#02 has no immediate tolerability issues, while showing high potency in the C57BL/6J mouse model.

***In vitro potency of (GalNAc)3-SO1861-ApoB#02***

An apoB-targeting BNA, ApoB#02 (SEQ ID NO: 12), and SO1861-EMCH or SO1861-SC-Mal was conjugated to trivalent-GalNAc ($(GalNAc)_3$) to produce $(GalNAc)_3$-SO1861-ApoB#02 (EMCH) and $(GalNAc)_3$-SO1861-ApoB#02 (sc) (Figure 35 and Figure 38), $(GalNAc)_3$-SO1861 (Figure 41) and $(GalNAc)_3$-ApoB#02 BNA (Figure 42) and tested on primary human hepatocytes, as well as HepG2 ($ASGPR^+$) and Huh7 ($ASGPR^{+/-}$) cell lines to assess their effect on apoB RNA levels. In primary hepatocytes, this assessment revealed that $(GalNAc)_3$-SO1861-ApoB#02 (EMCH) and $(GalNAc)_3$-SO1861-ApoB#02 (sc) showed an IC50 of = 1 nM (relative ApoB#02 BNA concentration), whereas $(GalNAc)_3$-ApoB#02 BNA without SO1861 showed an IC50 = 7 nM (relative ApoB#02 BNA concentration), and ApoB#02 BNA transfection with RNAi-MAX showed an IC50 = 8 nM. ApoB#02 alone (without any transfection agent) showed an IC50 = 400 nM, while $(GalNAc)_3$-SO1861 had no effect on apoB RNA levels (Figure 29A). All this shows that $(GalNAc)_3$-SO1861-ApoB#02 enhances the cytoplasmic delivery of a the ApoB#02 BNA in human primary hepatocytes, resulting in around 10-fold improved potency at low nM levels of ApoB#02 BNA oligo.

For HepG2 cells ($ASGPR1^+$), only $(GalNAc)_3$-SO1861-ApoB#02 (EMCH) and $(GalNAc)_3$-SO1861-ApoB#02 (sc) showed activity at the concentrations measured (IC50 = 40 nM and IC50 = 20 nM, respectively) (Figure 29 B), whereas

in Huh7 cells (ASGPR1$^{+/-}$), $(GalNAc)_3$-SO1861-ApoB#02 (EMCH) showed activity at IC50 = 100 nM and $(GalNAc)_3$-SO1861-ApoB#02 (sc) showed activity at IC50 = 40 nM (Figure 29 C).

***In vitro potency of $(GalNAc)_3$-dSPT4-ApoB#02 and $(GalNAc)_3$-dSPT8-ApoB#02***

An apoB-targeting BNA, ApoB#02 (SEQ ID NO: 12), and SO1861-EMCH or SO1861-sc-Mal was conjugated to trivalent-GalNAc ($(GalNAc)_3$) (Figure 1, Figure 8) to produce $(GalNAc)_3$-SO1861-ApoB#02 (EMCH) (Figure 35), $(GalNAc)_3$-SO1861-ApoB#02 (sc) (Figure 38), $(GalNAc)_3$-d$(SO1861)_4$-ApoB#02 (sc) ($(GalNAc)_3$-dSPT4-ApoB#02; Figure 39), $(GalNAc)_3$-d$(SO1861)_8$-ApoB#02 (sc) ($(GalNAc)_3$-dSPT8-ApoB#02; Figure 40) and $(GalNAc)_3$-ApoB#02 BNA (Figure 42) and conjugates were tested on primary human hepatocytes (Figure 30) to assess their effect on apoB RNA levels. In primary hepatocytes, this assessment revealed that $(GalNAc)_3$-d$(SO1861)_8$-ApoB#02 (sc) showed an IC50 of 1.5 nM (relative conjugate concentration), whereas $(GalNAc)_3$-SO1861-ApoB#02 (EMCH), $(GalNAc)_3$-SO1861-ApoB#02 (sc), $(GalNAc)_3$-d$(SO1861)_4$-ApoB#02 (sc) or $(GalNAc)_3$-ApoB#02 were less potent (IC50 between 5 and 8 nM) (Figure 30). This shows that an increased amount of SO1861 molecules per GalNAc conjugate enhances the potency of the conjugate, thereby in turn reducing the amounts of ApoB#02 BNA payload that is needed to reduce the levels of apoB mRNA.

***In vivo efficacy* of $(GalNAc)_3$-SO1861-ApoB#02 *in C57BL/6J mice (Study B)***

In Study B, C57BL/6J mice were treated as described and dosed according to Table A6. The conjugates used in this study, $(GalNA_C)_3$-SO1861-ApoB#02 (sc), $(GalNAc)_3$-d(SO1861)4-ApoB#02 (sc), $(GalNAc)_3$-d(SO1861)8-ApoB#02 (sc), $(GalNAc)_3$-ApoB#02 and $(GalNAc)_3$-SO1861) were produced as described in Figure 38, Figure 39, Figure 40, Figure 41, Figure 42. Clinical observations were recorded and different biomarkers of efficacy and tolerability were analysed.

As Figure 31 shows, after 72 hrs, the *apoB* RNA levels, *i.e.* gene expression, was reduced in animals treated with the benchmark 2 mg/kg ApoB#02 BNA (53% from control), but was even more reduced (to 24% and 21% of vehicle control, respectively) in the groups dosed with (1 mg/kg $(GalNAc)_3$-ApoB#02 + 0.1 mg/kg $(GalNAc)_3$-SO1861) and with 1 mg/kg $(GalNAc)_3$-SO1861-ApoB#02 (sc), respectively, showing the markedly improved potency when adding SO1861 to the conjugate (n = 3 animals per group). Moreover, when increasing the saponin-per-payload, by dosing 10-times less payload (0.173 mg/kg $(GalNAc)_3$-d(SO1861)4-ApoB#02 (sc) (referred to, in short, in Figure 31 as 0.1 mg/kg for brevity), also named $(GalNAc)_3$-dSPT4-ApoB#02 (sc) or 0.265 mg/kg $(GalNAc)_3$-d(SO1861)8-ApoB#02 (sc), also named $GalNAc)_3$-dSPT8-ApoB#02 (sc)), corresponding to the same payload doses (0.044 mg/kg ApoB02 BNA) contained in 0.1 mg/kg $(GalNAc)_3$-SO1861-ApoB#02 (sc), but 4-times and 8-times more SO1861, respectively, apoB expression was reduced to 74% and 38%, respectively, while it was 89% of control for 0.1 mg/kg $(GalNAc)_3$- SO1861-ApoB#02 (sc), showing that an increase of saponin markedly increases potency of the conjugate. A decrease of saponin amount (0.1 mg/kg $(GalNAc)_3$-SO1861 (sc) + 0.1 mg/kg $(GalNAc)_3$-ApoB#02) yielded less potency (71% apoB expression compared to vehicle). After 336 hrs, the effect of expression downmodulation was still preserved in the 1 mg/kg $(GalNAc)_3$-SO1861-ApoB#02 (sc) dose group and was 49% of vehicle control (n=3 mice).

As Figure 32 shows, the *apoB* RNA down-modulation in the liver (shown in Figure 31) also translated to apoB protein reduction in serum. Predose levels ranged from 156 - 198 μg/ml over the groups (n = 8 per group). At 72 hrs post-dose, the *apoB* protein levels were reduced in animals treated with the benchmark 2 mg/kg ApoB#02 BNA (57 μg/ml average compared to 156 μg/ml of the vehicle control), but was even more reduced (to 18 μg/ml and 21 μg/ml of vehicle control, respectively) in the groups dosed with (1 mg/kg $(GalNAc)_3$-ApoB#02 + 0.1 mg/kg $(GalNAc)_3$-SO1861) and with 1 mg/kg $(GalNAc)_3$-SO1861-ApoB#02 (sc), respectively, showing the markedly improved potency when adding SO1861 to the conjugate (n = 8 animals per group). As with the RNA levels, when increasing the saponin-per-payload, by dosing 0.173 mg/kg $(GalNAc)_3$-d(SO1861)4-ApoB#02 (sc) or 0.265 mg/kg $(GalNAc)_3$-d(SO1861)8-ApoB#02 (sc), respectively, serum apoB protein was reduced to 89 μg/ml and 50 μg/ml, respectively, while it was 141 μg/ml for 0.1 mg/kg $(GalNAc)_3$-SO1861-ApoB#02 (sc), showing that an increase of saponin markedly increases potency of the conjugate in reducing apoB protein. A decrease of saponin amount (0.1 mg/kg $(GalNAc)_3$-SO1861 (sc) + 0.1 mg/kg $(GalNAc)_3$-ApoB#02) yielded less potency (122 μg/ml apoB protein). After 336 hrs, the effect of protein reduction was still preserved in the 1 mg/kg $(GalNAc)_3$-SO1861-ApoB#02 (sc) dose group and was 59 μg/ml, compared to 143 μg/ml in the vehicle control (n=5 mice in all groups); at 672 hrs, apoB protein levels varied between 100 - 152 μg/ml in all grops (n = 2 mice).

As Figure 33 shows, the observed serum apoB protein reduction (shown in Figure 32) translated well to serum LDL-cholesterol (LDL-C) levels. At 72 hrs post-dose, the LDL-C levels were reduced in animals treated with the benchmark 2 mg/kg ApoB#02 BNA (2 mg/dl compared to 4 mg/dl of the vehicle control), but was even more reduced (to 0.7 mg/dl and 1.3 mg/dl μg/ml, respectively) in the groups dosed with (1 mg/kg $(GalNAc)_3$-ApoB#02 + 0.1 mg/kg $(GalNAc)_3$-SO1861) and with 1 mg/kg $(GalNAc)_3$-SO1861-ApoB#02 (sc), respectively, showing the markedly improved potency when adding SO1861 to the conjugate (n = 3 animals per group). As with the RNA and apoB protein levels, when increasing the saponin-per-payload, by dosing 0.173 mg/kg $(GalNAc)_3$-d(SO1861)4-ApoB#02 (sc) or 0.265 mg/kg $(GalNAc)_3$-d(SO1861)8-

ApoB#02 (sc), respectively, LDL-C was reduced, to 2.3 mg/dl and 1.3 mg/dl, respectively, while it was 4.3 mg/dl for 0.1 mg/kg $(GalNAc)_3$- SO1861-ApoB#02 (sc), showing that an increase of saponin markedly increases potency of the conjugate in reducing LDL-C. A decrease of saponin amount (0.1 mg/kg $(GalNAc)_3$- SO1861 (sc) + 0.1 mg/kg $(GalNAc)_3$-ApoB#02) yielded less potency (3.7 mg/dl LDL-C). After 336 hrs, the effect of LDL-C reduction was still preserved in the 1 mg/kg $(GalNAc)_3$-SO1861-ApoB#02 (sc) dose group (2 mg/dl; n = 3 animals) and in the (1 mg/kg $(GalNAc)_3$-ApoB#02 + 0.1 mg/kg $(GalNAc)_3$-SO1861), where it was 3.3 mg/dl (n = 3 animals). Showing the durability of the effect; at 672 hrs, LDL-C levels varied between 3 - 4 mg/dl in all grops (n = 2 mice).

In conclusion, this shows that the potency of ApoB#02 is markedly increased by the combination with SO1861 and is effectively inducing *apoB* RNA down-modulation, *i.e.*, gene silencing in the livers of C57BL/6J mice, as well as of all 'downstream' serum biomarkers of efficacy measured, such as apoB protein and LDL-cholesterol.

***In vivo tolerability* of (GalNAc)3- SO1861-ApoB#02 *in C57BL/6J mice (Study B)***

The conjugates used in this study, $(GalNAc)_3$-SO1861-ApoB#02 (sc), $(GalNAc)_3$-d(SO1861)4_ApoB#02 (sc), $(GalNAc)_3$-d(SO1861)8-ApoB#02 (sc), $(GalNAc)_3$-ApoB#02 and $(GalNAc)_3$-SO1861) were produced as described in Figure 38, Figure 39, Figure 40, Figure 41, Figure 42.

During the course of the study, all treatments, doses, and dose regimens as described in Table A6 were well tolerated and showed no treatment-specific adverse findings or any specific clinical observations or gross organ morphology indicative of the conjugates not being tolerated. The mice in treatment groups gained weight comparable to the mice receiving vehicle. Serum ALT levels were determined and results are shown in Figure 34. Levels of ALT appeared to transiently increase in the 0.173 mg/kg $(GalNAc)_3$-d(SO1861)4-ApoB#02 (sc) group at 72 hrs but returned to levels of vehicle control by 336 hrs post-dose; likewise, it increased in the 0.265 mg/kg $(GalNAc)_3$-d(SO1861)4-ApoB#02 (sc) group, but returned to vehicle levels at later timepoints post-dose. Notably, 1 mg/kg $(GalNAc)_3$-SO1861-ApoB#02 (sc) showed to elevation post-dose up to 336 hrs but was measured at 166 U/L at 672 hrs (n = 2 mice).

In conclusion, this data shows that the saponin conjugates are well tolerated in the doses and dose regimen applied, and particularly, that administration of SO1861-containing conjugates $(GalNAc)_3$-SO1861-ApoB#02 (sc), $(GalNAc)_3$-d(SO1861)4-ApoB#02 (sc), and $(GalNAc)_3$-d(SO1861)8-ApoB#02 (sc), as well as co-administration of $(GalNAc)_3$-SO1861 with $(GalNAc)_3$-ApoB#02 has no immediate tolerability issues, while showing high potency in the C57BL/6J mouse model.

## Claims

1. Oligonucleotide conjugate comprising at least one saponin covalently linked to a ligand for asialoglycoprotein receptor (ASGPR), wherein the ligand for ASGPR comprises or consists of $(GalNAc)_3$Tris moiety according to molecule (DD1):

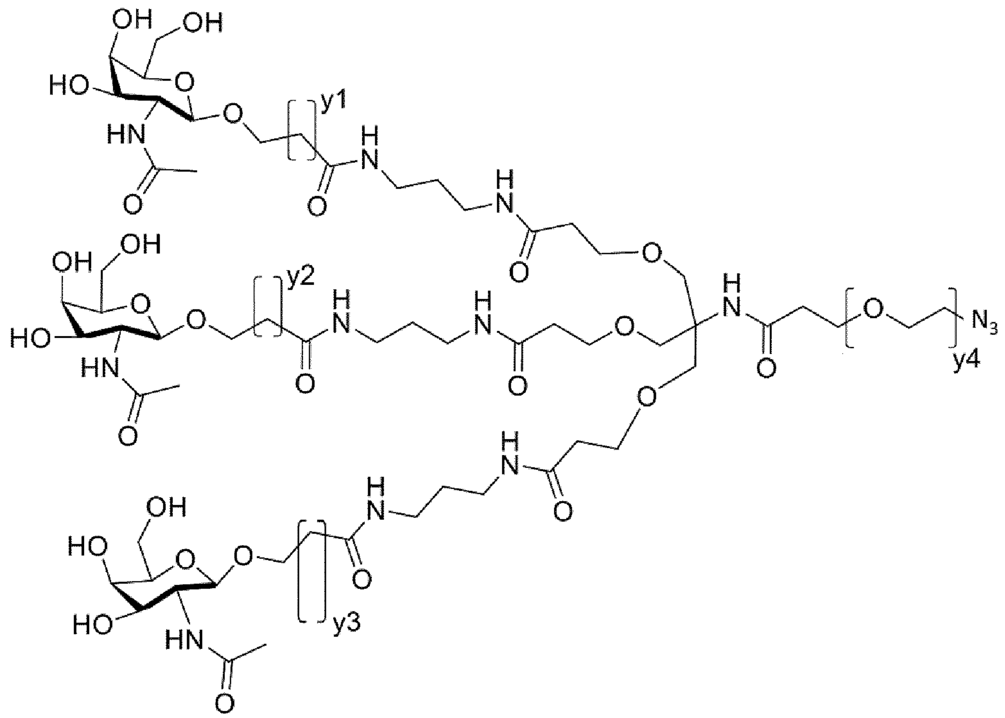

(DD1),

wherein y1, y2 and y3 each are an integer independently selected from 0-20, preferably 1-15, more preferably 2-12, even more preferably 2-10, even more preferably 2-8, most preferably 2 and 3, and preferably y1, y2 and y3 are the same, and y4 is an integer selected from 1-100, preferably 2-80, more preferably 3-70, even more preferably 4-60, even more preferably 4-50, even more preferably 4-40, even more preferably 4-30, even more preferably 4-20, even more preferably 4-6, most preferably 4-5, such as 4,
and further covalently linked to an oligonucleotide, wherein the at least one saponin is a monodesmosidic or bidesmosidic penta-cyclic triterpene saponin of the 12,13-dehydrooleanane type, preferably with an aldehyde function in position C-23 of the aglycone core structure of the saponin,
wherein the oligonucleotide conjugate comprises 1-16 saponin moieties, preferably 1-8 saponin moieties, more preferably 1 saponin moiety, 4 saponin moieties or 8 saponin moieties.

**2.** The oligonucleotide conjugate of claim 1, wherein the (GalNAc)$_3$Tris moiety is according to molecule (DD3):

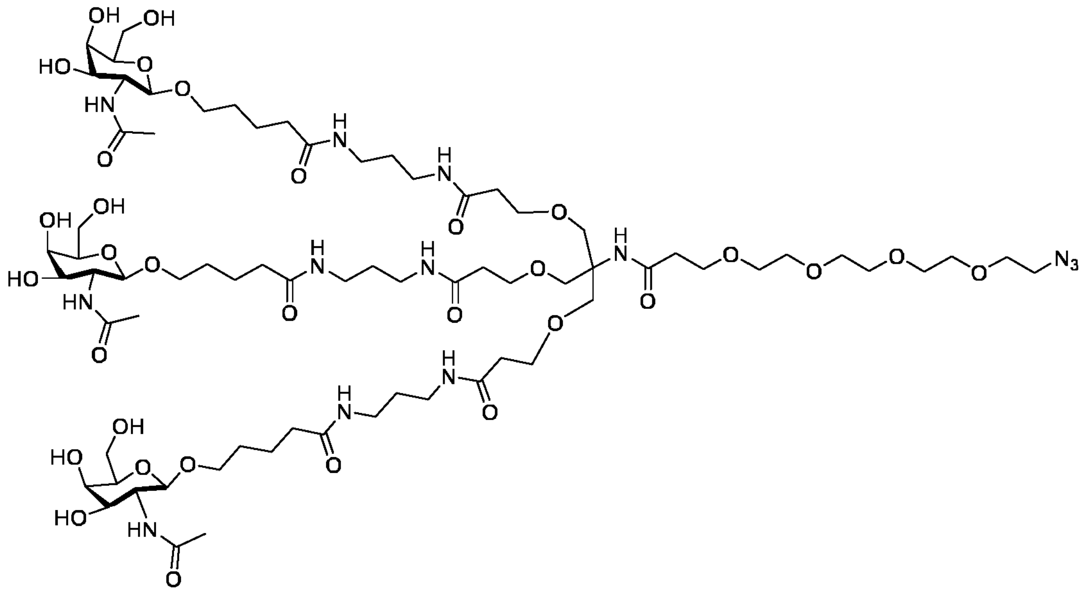

(DD3).

3. The oligonucleotide conjugate of any one of the claims 1-2, wherein the $(GalNAc)_3$Tris moiety, the at least one saponin, preferably 1-16 saponin moieties, more preferably 1-8 saponin moieties such as 1, 4 or 8 saponin moieties, and the oligonucleotide are covalently bound via a tri-functional linker, preferably with the $(GalNAc)_3$Tris moiety, the saponin or saponin moieties and the oligonucleotide covalently bound to a separate arm of the tri-functional linker.

4. The oligonucleotide conjugate of any one of the claims 1-3, comprising one saponin moiety, or 4 saponin moieties, preferably 4 saponin moieties covalently bound to a dendron, preferably a G2 dendron such as for example N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide), or 8 saponin moieties, preferably 8 saponin moieties covalently bound to a dendron, preferably a G3 dendron such as for example (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide.

5. The oligonucleotide conjugate of any one of the claims 1-4, wherein the saponin comprised by the conjugate is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Gypsophila elegans* saponin mixture, or a saponin isolated from *Gypsophila elegans*;
- *Saponinum album* saponin mixture, or a saponin isolated from *Saponinum album*;
- *Saponaria officinalis* saponin mixture, or a saponin isolated from *Saponaria officinalis*; and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, 10 SO1832, SO1861, SO1862, SO1904, QS-7, QS-7 api, QS-17, QS-18, QS-21 A-apio, QS-21 A-xylo, QS-21 B-apio and QS-21 B-xylo; or
d) a saponin comprising a 12, 13-dehydrooleanane type aglycone core structure without an aldehyde group at the C-23 position of the aglycone, selected from list D:

Aescin Ia, aescinate, alpha-Hederin, AMA-1, AMR, AS6.2, AS64R, Assamsaponin F, dipsacoside B, esculentoside A, macranthoidin A, NP-005236, NP-012672, Primula acid 1, saikosaponin A, saikosaponin D, Teaseed saponin I and Teaseedsaponin J,
preferably, the saponin is any one or more of a saponin selected from list A, B or C, more preferably, a saponin selected from list B or C,
even more preferably, a saponin selected from list C.

6. The oligonucleotide conjugate of any one of the claims 1-5, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* Saponarioside B, SO1542, SO1584, SO1658, SO1674, SO1700, SO1730, SO1772, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

7. The oligonucleotide conjugate of any one of the claims 1-6, wherein the saponin is a saponin isolated from *Saponaria officinalis,* preferably wherein the saponin is any one or more of Saponarioside B, SO1542, SO1584, SO1658, SO1674, SO1700, SO1730, SO1772, SO1832, SO1861, SO1862 and SO1904; more preferably wherein the saponin is any one or more of SO1542, SO1584, SO1658, SO1674, SO1700, SO1730, SO1772, SO1832, SO1861, SO1862 and SO1904; even more preferably wherein the saponin is any one or more of SO1832, SO1861 and SO1862; even more preferably wherein the saponin is SO1832 and SO1861; most preferably being SO1861.

8. The oligonucleotide conjugate of any one of the claims 1-7, wherein the saponin is SO1861, and wherein the oligonucleotide conjugate is provided by conjugation of SO1861 saponin derivative according to molecule (VII)a:

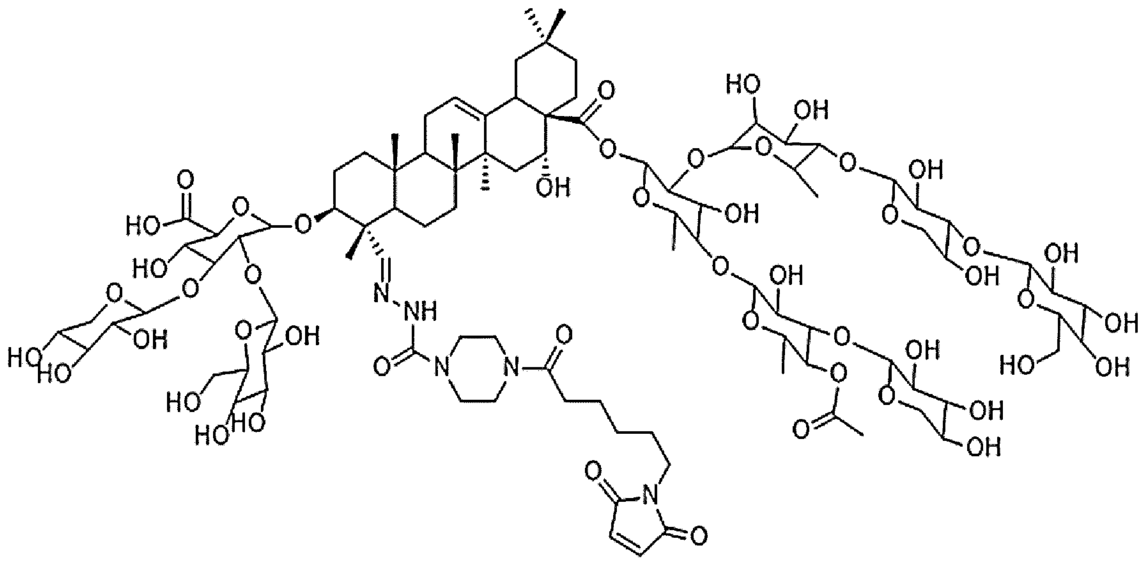

(VII)a

with an oligonucleotide and with the $(GalNAc)_3$Tris moiety, to provide the oligonucleotide conjugate.

9. The oligonucleotide conjugate of any one of the claims 1-8, wherein the oligonucleotide comprised by said conjugate is defined as a nucleic acid that is no longer than 150 nt, preferably wherein the oligonucleotide has a size of 5 - 150 nt, preferably being 8 - 100 nt, most preferably being 10 - 50 nt.

10. Pharmaceutical composition comprising the oligonucleotide conjugate of any one of the claims 1-9, and optionally a pharmaceutically acceptable excipient and/or optionally a pharmaceutically acceptable diluent.

11. Pharmaceutical composition of claim 10 or the oligonucleotide conjugate of any one of the claims 1-10, for use as a medicament
or

for use in the treatment or prophylaxis of a disease or health problem in which an expression product is involved of any one or more of genes: *HSP27, apoB, TTR, PCSK9, TMPRSS6, ALAS1, AT3, GO*, *CC5*, *X* gene of HBV, *S* gene of HBV, *AAT,* miR-122, hepatitis B virus HbsAg, *LDHA, CEBPA* and *LDH,* and/or for use in the treatment or prophylaxis of a disease or health problem which involves any one or more of genes: *HSP27, apoB, TTR, PCSK9, TMPRSS6, ALAS1, AT3, GO*, *CC5*, *X* gene of HBV, *S* gene of HBV, *AAT,* miR-122, hepatitis B virus HbsAg, *LDHA, CEBPA* and *LDH,*

or

for use in the treatment or prophylaxis of a disease or health problem in which an expression product is involved of any one or more of genes: *HSP27, apoB, TTR, PCSK9, TMPRSS6, ALAS1, AAT,* miR-122, hepatitis B virus HbsAg, *LDHA* and *CEBPA,* and/or for use in the treatment or prophylaxis of a disease or health problem which involves any one or more of genes: *HSP27, apoB, TTR, PCSK9, TMPRSS6, ALAS1, AAT,* miR-122, hepatitis B virus HbsAg, *LDHA* and *CEBPA,*

or

wherein said use is in the treatment or prophylaxis of a disease or health problem in which an expression product is involved of any one or more of genes: *HSP27* and *apoB*, preferably *apoB,* and/or for use in the treatment or prophylaxis of a disease or health problem which involves any one or more of genes: *HSP27* and *apoB,* preferably *apoB.*

**12.** Pharmaceutical composition of claim 10 or the oligonucleotide conjugate of any one of the claims 1-9, for use according to claim 11,
wherein said use is in the treatment or prophylaxis of a cancer, an infectious disease, a viral infection, hypercholesterolemia, cardiovascular disease, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, hepatitis C infection, $\alpha$1-antitrypsin deficiency, $\beta$-thalassaemia, or an auto-immune disease, wherein preferably said use is in the treatment or prophylaxis of a cancer such as endometrial carcinoma, breast cancer, lung cancer or hepatocellular carcinoma, and/or a cardiovascular disease such as hypercholesterolemia, preferably hypercholesterolemia.

**13.** Pharmaceutical composition of claim 10 or the oligonucleotide conjugate of any one of the claims 1-9, for use in the lowering of LDL-cholesterol in a subject, preferably a human subject.

**14.** *In vitro* or *ex vivo* method for transferring the oligonucleotide conjugate of any one of the claims 1-9 from outside a cell to inside said cell, preferably subsequently transferring the oligonucleotide comprised by said oligonucleotide conjugate into the cytosol of said cell, comprising the steps of:

a) providing a cell which expresses ASGPR on its surface, the cell preferably selected from a liver cell, a virally infected cell and a tumor cell, and providing the oligonucleotide conjugate of any one of the claims 1-9 for transferring into the provided cell;
b) contacting the cell of step a) *in vitro* or *ex vivo* with the oligonucleotide conjugate of step a), therewith effecting the transfer of the oligonucleotide conjugate from outside the cell into said cell, and preferably therewith subsequently effecting the transfer of the oligonucleotide comprised by the oligonucleotide conjugate into the cytosol of said cell.

**15.** The oligonucleotide conjugate of any one of the claims 1-9, wherein the saponin comprised by the oligonucleotide conjugate is isolated from a plant.

## Patentansprüche

**1.** Oligonukleotidkonjugat, umfassend zumindest ein Saponin, das kovalent an einen Liganden für einen Asialoglycoproteinrezeptor (ASGPR) gebunden ist, wobei der Ligand für ASGPR eine $(GalNAc)_3$-Tris-Einheit nach Molekül (DD1) umfasst oder daraus besteht:

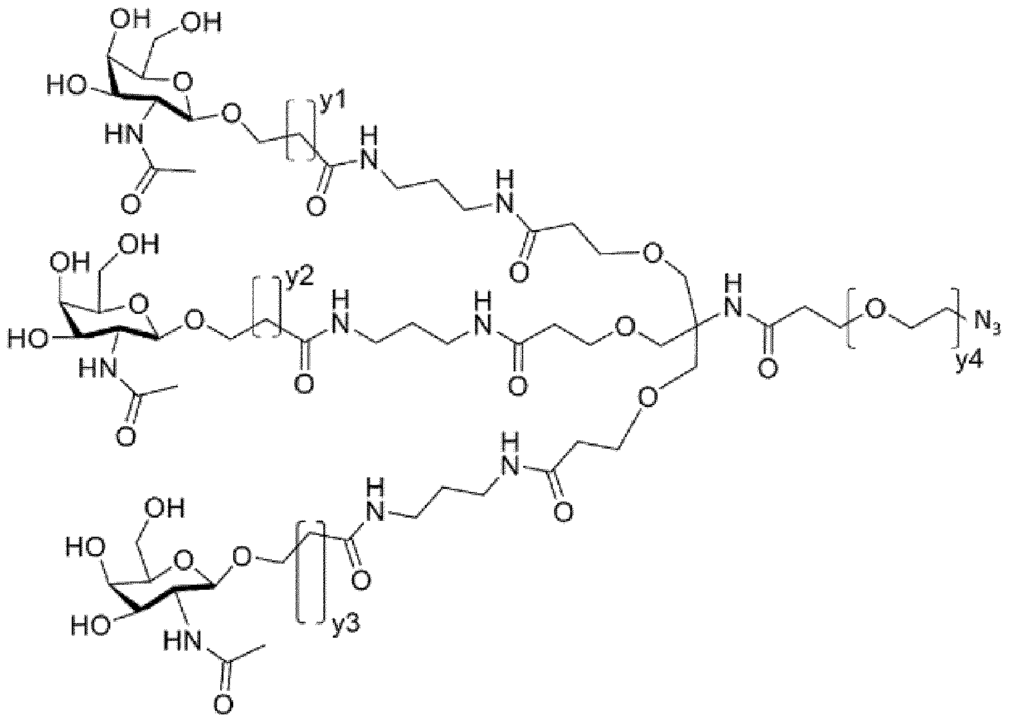

(DD1),

wobei y1, y2 und y3 jeweils eine Ganzzahl sind, die unabhängig voneinander aus 0-20, bevorzugt 1-15, mehr bevorzugt 2-12, noch mehr bevorzugt 2-10, noch mehr bevorzugt 2-8, am meisten bevorzugt 2 und 3, ausgewählt sind, und bevorzugt y1, y2 und y3 gleich sind, und y4 eine Ganzzahl ist, ausgewählt aus 1-100, bevorzugt 2-80, mehr bevorzugt 3-70, noch mehr bevorzugt 4-60, noch mehr bevorzugt 4-50, noch mehr bevorzugt 4-40, noch mehr bevorzugt 4-30, noch mehr bevorzugt 4-20, noch mehr bevorzugt 4-6, am meisten bevorzugt 4-5, wie etwa 4,

und ferner kovalent an ein Oligonukleotid gebunden ist, wobei das zumindest eine Saponin ein monodesmosidisches oder bidesmosidisches pentazyklisches Triterpensaponin vom 12,13-Dehydrooleanan-Typ, bevorzugt mit einer Aldehydfunktion in Position C-23 der Aglykon-Kernstruktur des Saponins, ist,

wobei das Oligonukleotidkonjugat 1-16 Saponin-Einheiten, bevorzugt 1-8 Saponin-Einheiten, mehr bevorzugt 1 Saponin-Einheit, 4 Saponin-Einheiten oder 8 Saponin-Einheiten umfasst.

**2.** Oligonukleotidkonjugat von Anspruch 1, wobei die $(GalNAc)_3$-Tris-Einheit gemäß Molekül (DD3) ist:

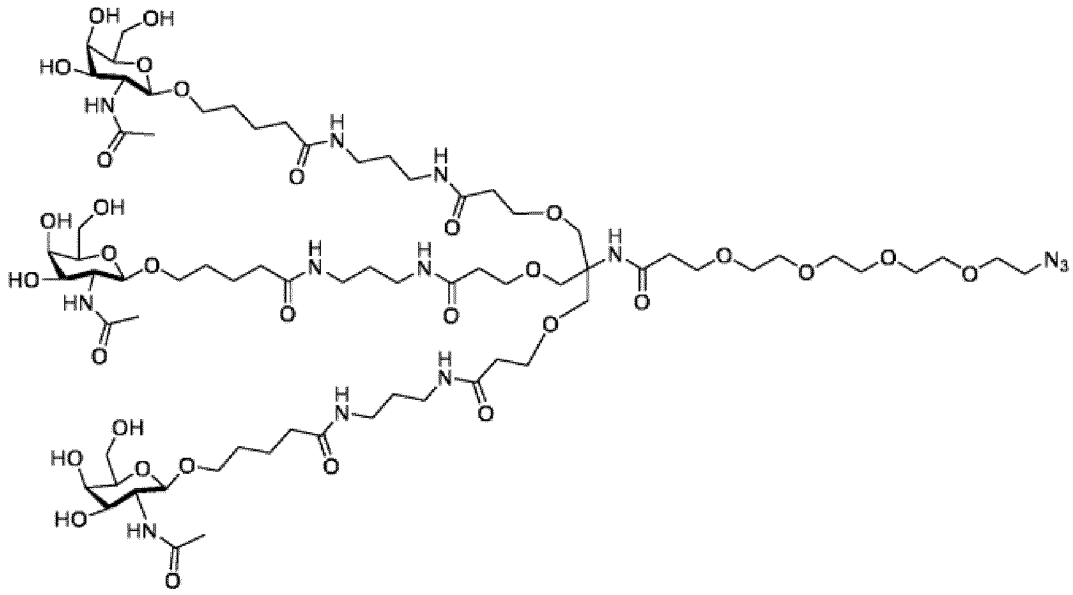

(DD3).

3. Oligonukleotidkonjugat von einem der Ansprüche 1-2, wobei die (GalNAc)$_3$-Tris-Einheit, das zumindest eine Saponin, bevorzugt 1-16 Saponin-Einheiten, mehr bevorzugt 1-8 Saponin-Einheiten, wie etwa 1, 4 oder 8 Saponin-Einheiten, und das Oligonukleotid über einen trifunktionalen Linker kovalent gebunden sind, bevorzugt mit der (GalNAc)$_3$-Tris-Einheit, wobei das Saponin oder die Saponin-Einheiten und das Oligonukleotid kovalent an einen separaten Arm des trifunktionalen Linkers gebunden sind.

4. Oligonukleotidkonjugat von einem der Ansprüche 1-3, umfassend eine Saponin-Einheit, oder 4 Saponin-Einheiten, bevorzugt 4 Saponin-Einheiten, die kovalent an ein Dendron gebunden sind, bevorzugt ein G2-Dendron, wie etwa beispielsweise N,N'-((9S,19S)-14-(6-Aminohexanoyl)-1-mercapto-9-(3-mercaptopropanamido)-3,10,18-trioxo-4,11,14,17-tetraazatricosane-19,23-diyl)bis(3-mercaptopropanamid), oder 8 Saponin-Einheiten, bevorzugt 8 Saponin-Einheiten, die kovalent an ein Dendron gebunden sind, bevorzugt ein G3-Dendron, wie etwa beispielsweise (2S)-N-[(1S)-1-{[2-(6-amino-N-{2-[(2S)-2,6-bis[(2S)-2,6-bis(3-sulfanylpropanamido)hexanamido]hexanamido] ethyl}hexanamido)ethyl ]carbamoyl}-5-[(2S)-2,6-bis(3-sulfanylpropanamido)hexanamido]pentyl]-2,6-bis(3-sulfanylpropanamido)hexanamid.

5. Oligonukleotidkonjugat von einem der Ansprüche 1-4, wobei das Saponin, umfasst durch das Konjugat, eines oder mehrere aus:

a) Saponin, ausgewählt aus einem oder mehreren von Liste A:

- *Quillaja saponaria* Saponin-Gemisch oder ein aus *Quillaja saponaria* isoliertes Saponin, beispielsweise Quil-A, QS-17-api, QS-17-xyl, QS-21, QS-21A, QS-21B, QS-7-xyl;
- *Gypsophila elegans* Saponin-Gemisch oder ein aus *Gypsophila elegans* isoliertes Saponin;
- *Saponinum album* Saponin-Gemisch oder ein aus *Saponinum album* isoliertes Saponin;
- *Saponaria officinalis* Saponin-Gemisch oder ein aus *Saponaria officinalis* isoliertes Saponin; und
- Quillaja-Rinden-Saponin-Gemisch oder ein aus Quillaja-Rinde isoliertes Saponin, beispielsweise Quil-A, QS-17-api, QS-17-xyl, QS-21, QS-21A, QS-21B, QS-7-xyl; oder

b) ein Saponin, umfassend eine Gypsogenin-Aglycon-Kernstruktur, ausgewählt aus Liste B:
SA1641, Gypsosid A, NP-017772, NP-017774, NP-017777, NP-017778, NP-018109, NP-017888, NP-017889, NP-018108, SO1658 und Phytolaccagenin; oder
c) ein Saponin, umfassend eine Quillajasäure-Aglykon-Kernstruktur, ausgewählt aus Liste C:
AG1856, AG1, AGZ, Agrostemmosid E, GE1741, Gypsophila Saponin 1 (Gyp1), NP-017674, NP-017810, NP-003881, NP-017676, NP-017677, NP-017705, NP-017706, NP-017773, NP-017775, SA1657, Saponariosid B, SO1542, SO1584, SO1674, SO1700, SO1730, SO1772, 10 SO1832, SO1861, SO1862, SO1904, QS-7, QS-7 api, QS-17, QS-18, QS-21 A-apio, QS-21 A-xylo, QS-21 B-apio und QS-21 B-xylo; oder

d) ein Saponin, umfassend eine 12,13-Dehydroolean-Typ-Aglykon-Kernstruktur ohne eine Aldehydgruppe an der C-23-Position des Aglykons, ausgewählt aus Liste D: Aescin Ia, Aescinat, alpha-Hederin, AMA-1, AMR, AS6.2, AS64R, Assamsaponin F, Dipsacosid B, Esculentosid A, Macranthoidin A, NP-005236, NP-012672, Primulasäure 1, Saikosaponin A, Saikosaponin D, Teesamen-Saponin I und Teesamen-Saponin J, ist,

bevorzugt ist das Saponin ein oder mehrere von einem Saponin, ausgewählt aus Liste A, B oder C, mehr bevorzugt ein Saponin, ausgewählt aus Liste B oder C, noch mehr bevorzugt ein Saponin, ausgewählt aus Liste C.

**6.** Oligonukleotidkonjugat von einem der Ansprüche 1-5, wobei das Saponin eines oder mehrere von AG1856, GE1741, ein aus *Quillaja saponaria* isoliertes Saponin, Quil-A, QS-17, QS-21, QS-7, SA1641, ein aus *Saponaria officinalis* isoliertes Saponin, Saponariosid B, SO1542, SO1584, SO1658, SO1674, SO1700, SO1730, SO1772, SO1832, SO1861, SO1862 und SO1904 ist; wobei das Saponin bevorzugt eines oder mehrere von QS-21, SO1832, SO1861, SA1641 und GE1741 ist; wobei das Saponin mehr bevorzugt QS-21, SO1832 oder SO1861 ist; am meisten bevorzugt SO1861 ist.

**7.** Oligonukleotidkonjugat von einem der Ansprüche 1-6, wobei das Saponin ein aus *Saponaria officinalis* isoliertes Saponin ist, wobei das Saponin bevorzugt eines oder mehrere von Saponariosid B, SO1542, SO1584, SO1658, SO1674, SO1700, SO1730, SO1772, SO1832, SO1861, SO1862 und SO1904 ist; wobei das Saponin mehr bevorzugt eines oder mehrere von SO1542, SO1584, SO1658, SO1674, SO1700, SO1730, SO1772, SO1832, SO1861, SO1862 und SO1904 ist; wobei das Saponin noch mehr bevorzugt eines oder mehrere von SO1832, SO1861 und SO1862 ist; wobei das Saponin noch mehr bevorzugt SO1832 und SO1861 ist; am meisten bevorzugt SO1861 ist.

**8.** Oligonukleotidkonjugat von einem der Ansprüche 1-7, wobei das Saponin SO1861 ist, und wobei das Oligonukleotidkonjugat durch Konjugation eines SO1861-Saponinderivats gemäß Molekül (VII)a:

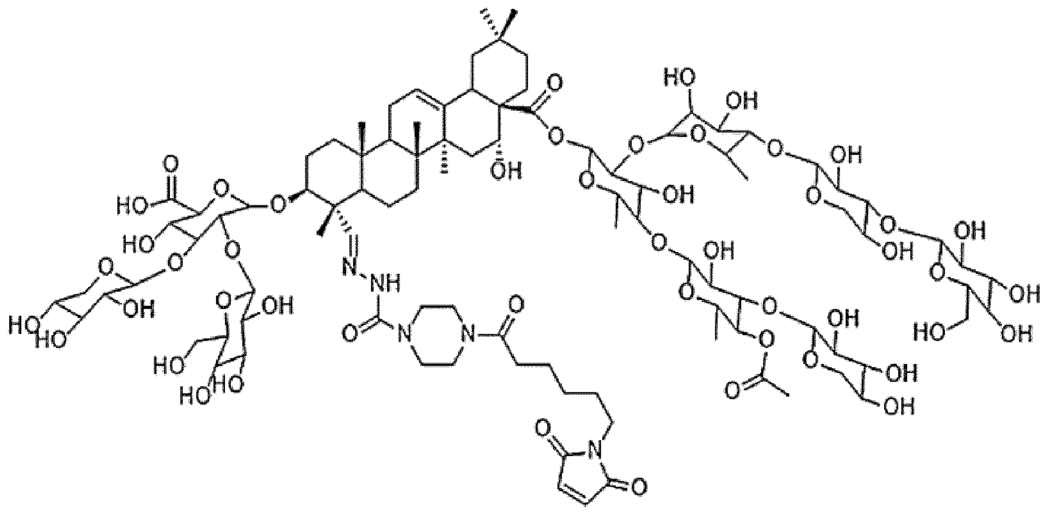

(VII)a

mit einem Oligonukleotid und mit der $(GalNAc)_3$-Tris-Einheit bereitgestellt ist, um das Oligonukleotidkonjugat bereitzustellen.

**9.** Oligonukleotidkonjugat von einem der Ansprüche 1-8, wobei das Oligonukleotid, umfasst durch das Konjugat, definiert ist als eine Nukleinsäure, die nicht länger als 150 nt ist, wobei das Oligonukleotid bevorzugt eine Größe von 5-150 nt aufweist, bevorzugt 8-100 nt ist, am meisten bevorzugt 10-50 nt ist.

**10.** Pharmazeutische Zusammensetzung, umfassend das Oligonukleotidkonjugat von einem der Ansprüche 1-9 und optional einen pharmazeutisch akzeptablen Hilfsstoff und/oder optional ein pharmazeutisch akzeptables Verdünnungsmittel.

**11.** Pharmazeutische Zusammensetzung von Anspruch 10 oder das Oligonukleotidkonjugat von einem der Ansprüche

1-10,

zur Verwendung als Medikament
oder
zur Verwendung bei der Behandlung oder Prophylaxe einer Krankheit oder eines Gesundheitsproblems, in welchem ein Expressionsprodukt eines oder mehrerer der Gene: HSP27, apoB, TTR, PCSK9, TMPRSS6, ALAS1, AT3, GO, CC5, X-Gen von HBV, S-Gen von HBV, AAT, miR-122, Hepatitis-B-Virus HbsAg, LDHA, CEBPA und LDH, einbezogen ist, und/oder zur Verwendung bei der Behandlung oder Prophylaxe einer Krankheit oder eines Gesundheitsproblems, welches eines oder mehrere der Gene: HSP27, apoB, TTR, PCSK9, TMPRSS6, ALAS1, AT3, GO, CC5, X-Gen von HBV, S-Gen von HBV, AAT, miR-122, Hepatitis-B-Virus HbsAg, LDHA, CEBPA und LDH, einbezieht,
oder
zur Verwendung bei der Behandlung oder Prophylaxe einer Krankheit oder eines Gesundheitsproblems, in welchem ein Expressionsprodukt eines oder mehrerer der Gene: HSP27, apoB, TTR, PCSK9, TMPRSS6, ALAS1, AAT, miR-122, Hepatitis-B-Virus HbsAg, LDHA und CEBPA, einbezogen ist, und/oder zur Verwendung bei der Behandlung oder Prophylaxe einer Krankheit oder eines Gesundheitsproblems, welches eines oder mehrere der Gene: HSP27, apoB, TTR, PCSK9, TMPRSS6, ALAS1, AAT, miR-122, Hepatitis-B-Virus HbsAg, LDHA und CEBPA, einbezieht,
oder
wobei die Verwendung in der Behandlung oder Prophylaxe einer Krankheit oder eines Gesundheitsproblems besteht, in welchem ein Expressionsprodukt eines oder mehrerer der Gene: HSP27 und apoB, bevorzugt apoB, einbezogen ist, und/oder zur Verwendung bei der Behandlung oder Prophylaxe einer Krankheit oder eines Gesundheitsproblems, welches eines oder mehrere der Gene: HSP27 und apoB, bevorzugt apoB, einbezieht.

**12.** Pharmazeutische Zusammensetzung von Anspruch 10 oder das Oligonukleotidkonjugat von einem der Ansprüche 1-9 zur Verwendung nach Anspruch 11,

wobei die Verwendung in der Behandlung oder Prophylaxe eines Krebses, einer Infektionskrankheit, einer viralen Infektion, Hypercholesterinämie, kardiovaskulären Krankheit, primären Hyperoxalurie, Hämophilie A, Hämophilie B, AAT-assoziierten Leberkrankheit, akuten hepatischen Porphyrie, TTR-vermittelten Amyloidose, hereditären TTR-Amyloidose (hATTR), komplementvermittelten Krankheit, Hepatitis-B-Infektion, Hepatitis-C-Infektion, $\alpha$1-Antitrypsin-Mangels, B-Thalassämie oder einer Autoimmunerkrankung erfolgt,
wobei die Verwendung bevorzugt in der Behandlung oder Prophylaxe eines Krebses wie Endometriumkarzinom, Brustkrebs, Lungenkrebs oder hepatozellulärem Karzinom und/oder einer kardiovaskulären Erkrankung, wie etwa Hypercholesterinämie, bevorzugt Hypercholesterinämie erfolgt.

**13.** Pharmazeutische Zusammensetzung von Anspruch 10 oder das Oligonukleotidkonjugat von einem der Ansprüche 1-9 zur Verwendung bei der Senkung von LDL-Cholesterin in einem Subjekt, bevorzugt einem menschlichen Subjekt.

**14.** In-vitro- oder ex-vivo-Verfahren zum Transferieren des Oligonukleotidkonjugats von einem der Ansprüche 1-9 von außerhalb einer Zelle in das Innere der Zelle, bevorzugt anschließendes Transferieren des Oligonukleotids, umfasst durch das Oligonukleotidkonjugat, in das Cytosol der Zelle, umfassend die Schritte von:

a) Bereitstellen einer Zelle, welche ASGPR auf ihrer Oberfläche exprimiert, die Zelle bevorzugt aus einer Leberzelle, einer viral infizierten Zelle und einer Tumorzelle ausgewählt ist, und Bereitstellen des Oligonukleotidkonjugats von einem der Ansprüche 1-9 zum Transfer in die bereitgestellte Zelle;
b) In-vitro- oder ex-vivo-Inkontaktbringen der Zelle aus Schritt a) mit dem Oligonukleotidkonjugat aus Schritt a), damit den Transfer des Oligonukleotidkonjugats von außerhalb der Zelle in die Zelle bewirkend, und bevorzugt damit anschließend den Transfer des Oligonukleotids, umfasst durch das Oligonukleotidkonjugat, in das Cytosol der Zelle bewirkend.

**15.** Oligonukleotidkonjugat von einem der Ansprüche 1-9, wobei das Saponin, umfasst durch das Oligonukleotidkonjugat, aus einer Pflanze isoliert ist.

## Revendications

**1.** - Conjugué d’oligonucléotide comprenant au moins une saponine liée de manière covalente à un ligand pour le

récepteur de l'asialoglycoprotéine (ASGPR), le ligand pour ASGPR comprenant ou consistant en la fraction $(GalNAc)_3$Tris conformément à la molécule (DD1) :

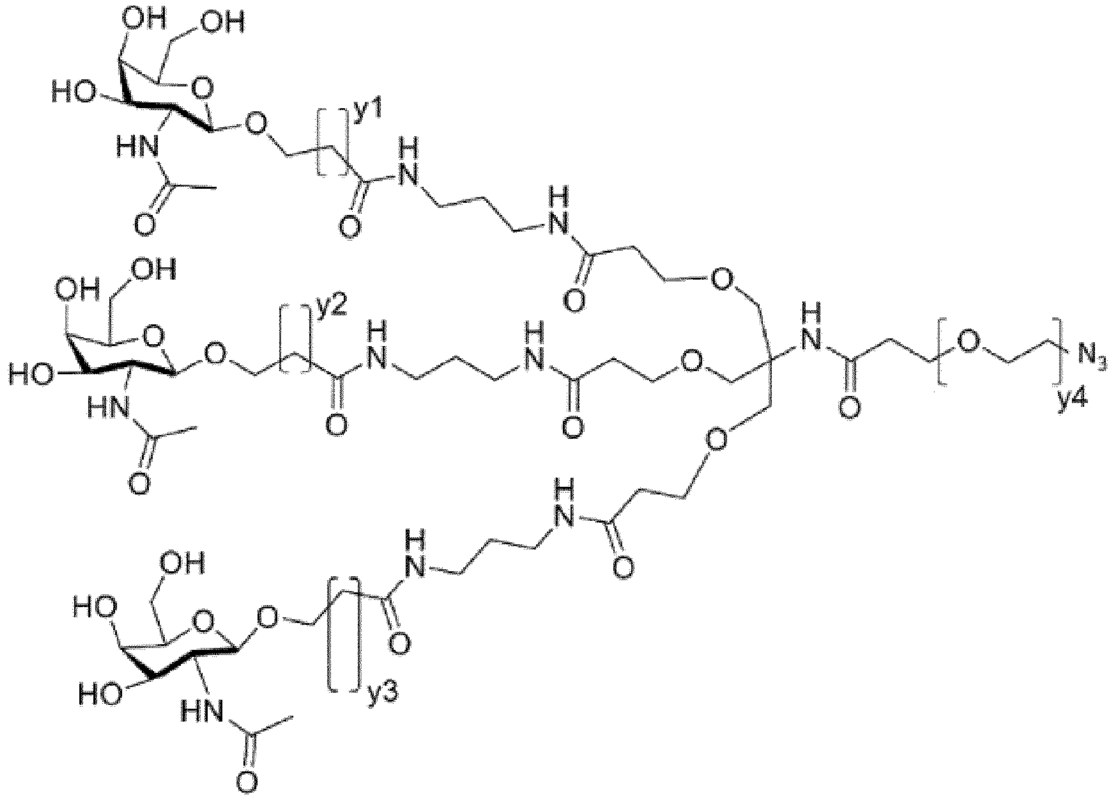

(DD1),

dans laquelle y1, y2 et y3 sont chacun un entier indépendamment choisi parmi 0 à 20, de préférence 1 à 15, de façon plus préférée 2 à 12, de façon encore plus préférée 2 à 10, de façon encore plus préférée 2 à 8, de façon le plus préférée 2 et 3, et de préférence y1, y2 et y3 sont identiques, et y4 est un entier choisi parmi 1 à 100, de préférence 2 à 80, de façon plus préférée 3 à 70, de façon encore plus préférée 4 à 60, de façon encore plus préférée 4 à 50, de façon encore plus préférée 4 à 40, de façon encore plus préférée 4 à 30, de façon encore plus préférée 4 à 20, de façon encore plus préférée 4 à 6, de façon le plus préférée 4 à 5, tel que 4,
et encore liée de manière covalente à un oligonucléotide, dans lequel ladite au moins une saponine étant une saponine triterpénique pentacyclique monodesmosidique ou bidesmosidique du type 12,13-déshydrooléanane, de préférence avec une fonction aldéhyde en position C-23 de la structure de noyau aglycone de la saponine, dans lequel le conjugué d'oligonucléotide comprenant de 1 à 16 fractions de saponine, de préférence de 1 à 8 fractions de saponine, de façon plus préférée 1 fraction de saponine, 4 fractions de saponine ou 8 fractions de saponine.

**2.** - Conjugué d'oligonucléotide selon la revendication 1, dans lequel la fraction $(GalNAc)_3$Tris est conforme à la molécule (DD3) :

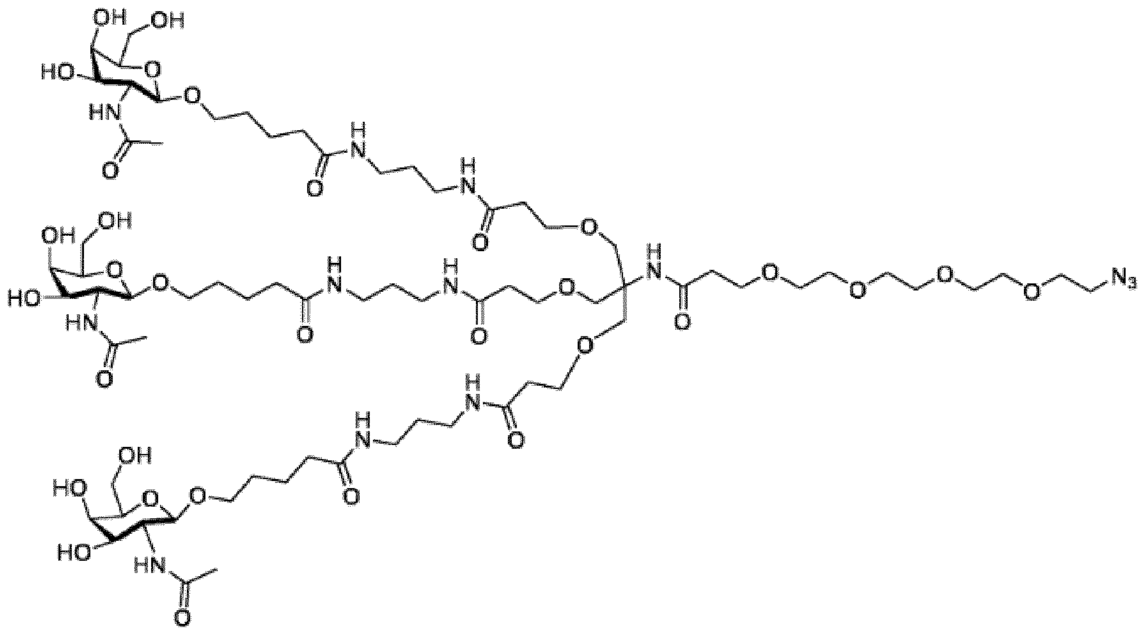

(DD3) .

3. - Conjugué d'oligonucléotide selon l'une quelconque des revendications 1 et 2, dans lequel la fraction $(GalNAc)_3$Tris, ladite au moins une saponine, de préférence 1 à 16 fractions de saponine, de façon plus préférée 1 à 8 fractions de saponine telles que 1, 4 ou 8 fractions de saponine, et l'oligonucléotide sont liés de manière covalente par l'intermédiaire d'un lieur trifonctionnel, de préférence avec la fraction $(GalNAc)_3$Tris, la saponine ou les fractions de saponine et l'oligonucléotide étant liés de manière covalente à un bras séparé du lieur trifonctionnel.

4. - Conjugué d'oligonucléotide selon l'une quelconque des revendications 1 à 3, comprenant une fraction de saponine, ou 4 fractions de saponine, de préférence 4 fractions de saponine liées de manière covalente à un dendron, de préférence un dendron G2 tel que par exemple le N,N'-((9S,19S)-14-(6-aminohexanoyl)-1-mercapto-9-(3-mercaptopropanamido)-3,10,18-trioxo-4,11,14,17-tétraazatricosane-19,23-diyl)bis(3-mercaptopropanamide), ou 8 fractions de saponine, de préférence 8 fractions de saponine liées de manière covalente à un dendron, de préférence un dendron G3 tel que par exemple le (2S)-N-[(1S)-1-{[2-(6-amino-N-{2-[(2S)-2,6-bis[(2S)-2,6-bis(3-sulfanylpropanamido)hexanamido]hexanamido]éthyl}hexanamido) éthyl]carbamoyl}-5-[(2S)- 2,6-bis(3-sulfanylpropanamido)hexanamido]pentyl]-2,6-bis(3-sulfanylpropanamido)hexanamide.

5. - Conjugué d'oligonucléotide selon l'une quelconque des revendications 1 à 4, dans lequel la saponine comprise dans le conjugué est l'une quelconque ou plusieurs parmi :

a) une saponine choisie parmi l'une quelconque ou plusieurs de la liste A :

- mélange de saponines de *Quillaja saponaria,* ou une saponine isolée de *Quillaja saponaria,* par exemple Quil-A, QS-17-api, QS-17-xyl, QS-21, QS-21A, QS-21B, QS-7-xyl ;
- mélange de saponines de *Gypsophila elegans,* ou une saponine isolée de *Gypsophila elegans ;*
- mélange de saponines de *Saponinum album,* ou une saponine isolée de *Saponinum album ;*
- mélange de saponines de *Saponaria officinalis,* ou une saponine isolée de *Saponaria officinalis ;* et
- mélange de saponines d'écorce de Quillaja, ou une saponine isolée de l'écorce de Quillaja, par exemple Quil-A, QS-17-api, QS-17-xyl, QS-21, QS-21A, QS-21B, QS-7-xyl ; ou

b) une saponine comprenant une structure de base aglycone de gypsogénine, choisie dans la liste B : SA1641, gypsoside A, NP-017772, NP-017774, NP-017777, NP-017778, NP-018109, NP-017888, NP-017889, NP-018108, S01658 et Phytolaccagénine ; ou
c) une saponine comprenant une structure de base aglycone d'acide quillaïque, choisie dans la liste C : AG1856, AG1, AG2, Agrostemmoside E, GE1741, Gypsophila saponine 1 (Gyp1), NP-017674, NP-017810, NP-003881, NP-017676, NP-017677, NP-017705, NP-017706, NP-017773, NP- 017775, SA1657, Saponarioside B, S01542, 501584, S01674, 501700, 501730, S01772, 10 501832, 501861, S01862, S01904, QS-7, QS-7

api, QS-17, QS-18, QS-21 A-apio, QS-21 A-xylo, QS-21 B-apio et QS-21 B-xylo ; ou
d) une saponine comprenant une structure de base aglycone de type 12, 13-déshydrooléanane sans groupe aldéhyde en position C-23 de l'aglycone, choisie dans la liste D :
Aescine Ia, aescinate, alpha-Hédérine, AMA-1, AMR, AS6.2, AS64R, Assamsaponine F, dipsacoside B, esculentoside A, macranthoïdine A, NP-005236, NP-012672, acide Primula 1, saikosaponine A, saikosaponine D, Saponine de graine de thé I et Saponine de graine de thé J,
de préférence, la saponine est l'une quelconque ou plusieurs parmi une saponine choisie dans la liste A, B ou C, de façon plus préférée, une saponine choisie dans la liste B ou C,
de façon encore plus préférée, une saponine choisie dans la liste C.

**6.** - Conjugué d'oligonucléotide selon l'une quelconque des revendications 1 à 5, dans lequel la saponine est l'une quelconque ou plusieurs parmi AG1856, GE1741, une saponine isolée de *Quillaja saponaria,* Quil-A, QS-17, QS-21, QS-7, SA1641, une saponine isolée de *Saponaria officinalis,* Saponarioside B, S01542, S01584, S01658, 501674, S01700, S01730, S01772, S01832, SO1861, S01862 et S01904 ; de préférence dans lequel la saponine est l'une quelconque ou plusieurs parmi QS-21, SO1832, S01861, SA1641 et GE1741 ; de façon plus préférée dans lequel la saponine est QS-21, S01832 ou S01861 ; de la façon le plus préfère étant S01861.

**7.** - Conjugué d'oligonucléotide selon l'une quelconque des revendications 1 à 6, dans lequel la saponine est une saponine isolée de *Saponaria officinalis,* de préférence dans lequel la saponine est l'une quelconque ou plusieurs parmi Saponarioside B, S01542, 501584, SO1658, S01674, 501700, S01730, S01772, SO1832, SO1861, S01862 et S01904 ; de façon plus préférée dans lequel la saponine est l'une quelconque ou plusieurs parmi S01542, S01584, S01658, S01674, S01700, S01730, S01772, S01832, S01861, S01862 et S01904 ; de façon encore plus préférée dans lequel la saponine est l'une quelconque ou plusieurs parmi S01832, S01861 et S01862 ; de façon encore plus préférée dans lequel la saponine est SO1832 et S01861 ; de la façon le plus préfère étant S01861.

**8.** - Conjugué d'oligonucléotide selon l'une quelconque des revendications 1 à 7, dans lequel la saponine est SO1861, et dans lequel le conjugué d'oligonucléotide est fourni par conjugaison d'un dérivé de saponine S01861 conformément à la molécule (VII)a :

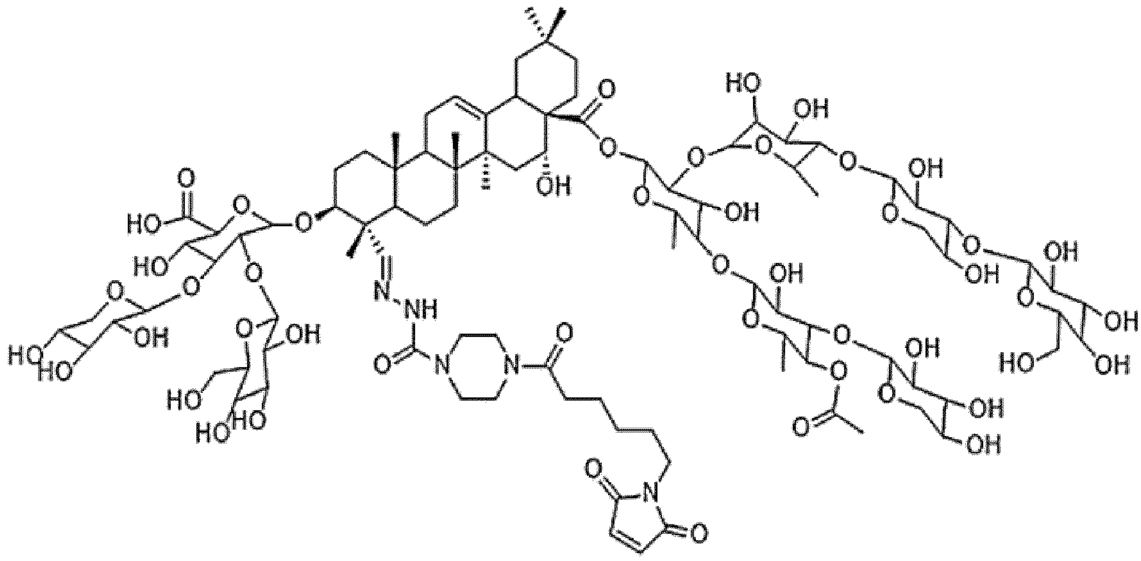

(VII)a

avec un oligonucléotide et avec la fraction $(GalNAc)_3$Tris, pour fournir le conjugué d'oligonucléotide.

**9.** - Conjugué d'oligonucléotide selon l'une quelconque des revendications 1 à 8, dans lequel l'oligonucléotide compris dans ledit conjugué est défini comme un acide nucléique qui ne dépasse pas 150 nt, de préférence dans lequel l'oligonucléotide a une taille de 5 à 150 nt, de préférence étant de 8 à 100 nt, de la façon le plus préfère de 10 à 50 nt.

**10.** - Composition pharmaceutique comprenant le conjugué d'oligonucléotide selon l'une quelconque des revendications 1 à 9, et facultativement un excipient pharmaceutiquement acceptable et/ou facultativement un diluant pharmaceutiquement acceptable.

**11.** - Composition pharmaceutique selon la revendication 10 ou conjugué d'oligonucléotide selon l'une quelconque des revendications 1 à 10, pour une utilisation comme médicament ou

pour une utilisation dans le traitement ou la prophylaxie d'une maladie ou d'un problème de santé dans lequel est impliqué un produit d'expression de l'un quelconque ou de plusieurs des gènes : *HSP27, apoB, TTR, PCSK9, TMPRSS6, ALAS1, AT3, GO, CC5,* gène *X* du VHB, gène S du VHB, *AAT,* miR-122, HbsAg du virus de l'hépatite B, *LDHA, CEBPA* et *LDH,* et/ou pour une utilisation dans le traitement ou la prophylaxie d'une maladie ou d'un problème de santé qui implique l'un quelconque ou plusieurs des gènes : *HSP27, apoB, TTR, PCSK9, TMPRSS6, ALAS1, AT3, GO, CC5,* gène *X* du VHB, gène S du VHB, *AAT*, miR-122, HbsAg du virus de l'hépatite B, *LDHA, CEBPA* et *LDH,*
ou
pour une utilisation dans le traitement ou la prophylaxie d'une maladie ou d'un problème de santé dans lequel est impliqué un produit d'expression de l'un quelconque ou de plusieurs des gènes *: HSP27, apoB, TTR, PCSK9, TMPRSS6, ALAS1, AAT,* miR-122, HbsAg du virus de l'hépatite B, *LDHA* et *CEBPA,* et/ou pour une utilisation dans le traitement ou la prophylaxie d'une maladie ou d'un problème de santé qui implique l'un quelconque ou plusieurs des gènes : *HSP27, apoB, TTR, PCSK9, TMPRSS6, ALAS1, AAT,* miR-122, HbsAg du virus de l'hépatite B, *LDHA* et *CEBPA,*
ou
dans lequel ladite utilisation est dans le traitement ou la prophylaxie d'une maladie ou d'un problème de santé dans lequel est impliqué un produit d'expression de l'un quelconque ou de plusieurs des gènes : *HSP27* et *apoB,* de préférence *apoB,* et/ou pour une utilisation dans le traitement ou la prophylaxie d'une maladie ou d'un problème de santé qui implique l'un quelconque ou plusieurs des gènes : *HSP27* et *apoB,* de préférence *apoB.*

**12.** - Composition pharmaceutique selon la revendication 10 ou conjugué d'oligonucléotide selon l'une quelconque des revendications 1 à 9, pour une utilisation selon la revendicaton 11,

dans lequel ladite utilisation est dans le traitement ou la prophylaxie d'un cancer, d'une maladie infectieuse, d'une infection virale, l'hypercholestérolémie, d'une maladie cardiovasculaire, d'une hyperoxalurie primaire, d'une hémophilie A, d'une hémophilie B, d'une maladie hépatique liée à l'AAT, d'une porphyrie hépatique aiguë, d'une amyloïdose médiée par TTR, d'une amyloïdose héréditaire à TTR (hATTR), d'une maladie médiée par le complément, d'une infection par l'hépatite B, d'une infection par l'hépatite C, d'un déficit en $\alpha$1-antitrypsine, d'une $\beta$-thalassémie ou d'une maladie auto-immune,
dans lequel de préférence ladite utilisation est dans le traitement ou la prophylaxie d'un cancer tel que le carcinome de l'endomètre, le cancer du sein, le cancer du poumon ou le carcinome hépatocellulaire, et/ou une maladie cardiovasculaire telle que l'hypercholestérolémie, de préférence l'hypercholestérolémie.

**13.** - Composition pharmaceutique selon la revendication 10 ou conjugué d'oligonucléotide selon l'une quelconque des revendications 1 à 9, pour une utilisation dans la réduction du cholestérol LDL chez un sujet, de préférence un sujet humain.

**14.** - Procédé *in vitro* ou *ex vivo* pour transférer le conjugué d'oligonucléotide selon l'une quelconque des revendications 1 à 9 de l'extérieur d'une cellule à l'intérieur de ladite cellule, de préférence transférer par la suite l'oligonucléotide compris dans ledit conjugué d'oligonucléotide dans le cytosol de ladite cellule, comprenant les étapes suivantes :

a) fournir une cellule qui exprime ASGPR à sa surface, la cellule étant de préférence choisie parmi une cellule hépatique, une cellule infectée par un virus et une cellule tumorale, et fournir le conjugué d'oligonucléotide selon l'une quelconque des revendications 1 à 9 pour le transfert dans la cellule fournie ;
b) mettre en contact la cellule de l'étape a) *in vitro* ou *ex vivo* avec le conjugué d'oligonucléotide de l'étape a), effectuant ainsi le transfert du conjugué d'oligonucléotide de l'extérieur de la cellule dans ladite cellule, et de préférence ainsi effectuant ensuite le transfert de l'oligonucléotide compris dans le conjugué d'oligonucléotide dans le cytosol de ladite cellule.

**15.** - Conjugué d'oligonucléotides selon l'une quelconque des revendications 1 à 9, dans lequel la saponine comprise dans le conjugué d'oligonucléotide est isolée à partir d'une plante.

**Fig. 1**
**A**

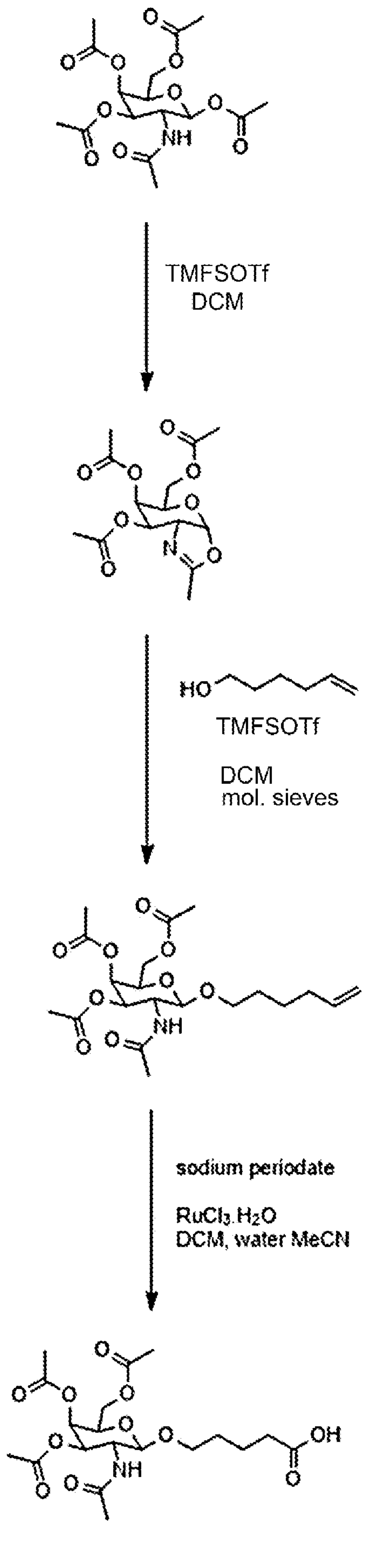
TMFSOTf
DCM
TMFSOTf
DCM
mol. sieves
sodium periodate
RuCl3.H2O
DCM, water MeCN

**Fig. 1**

**B**

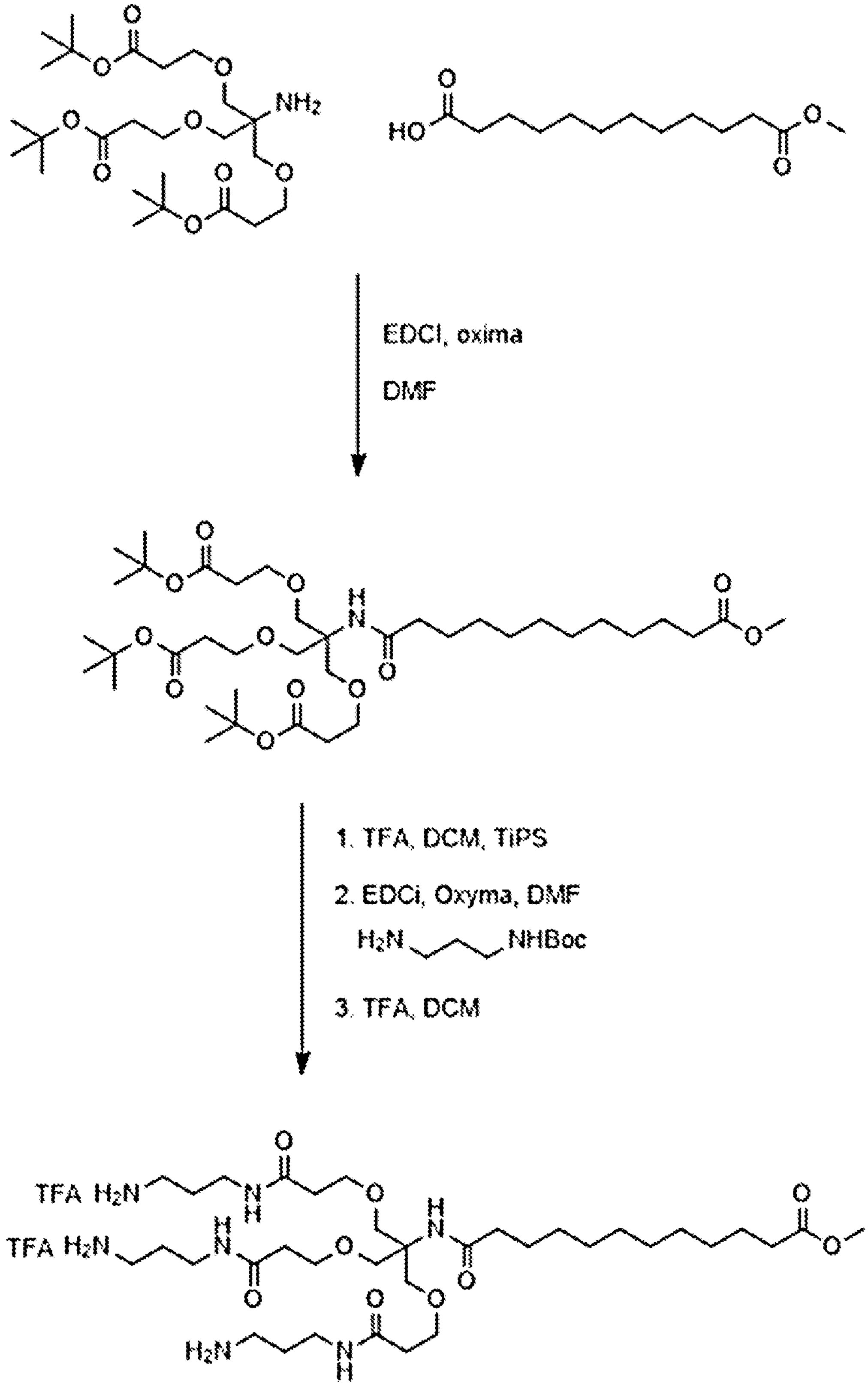

# Fig. 1

## C

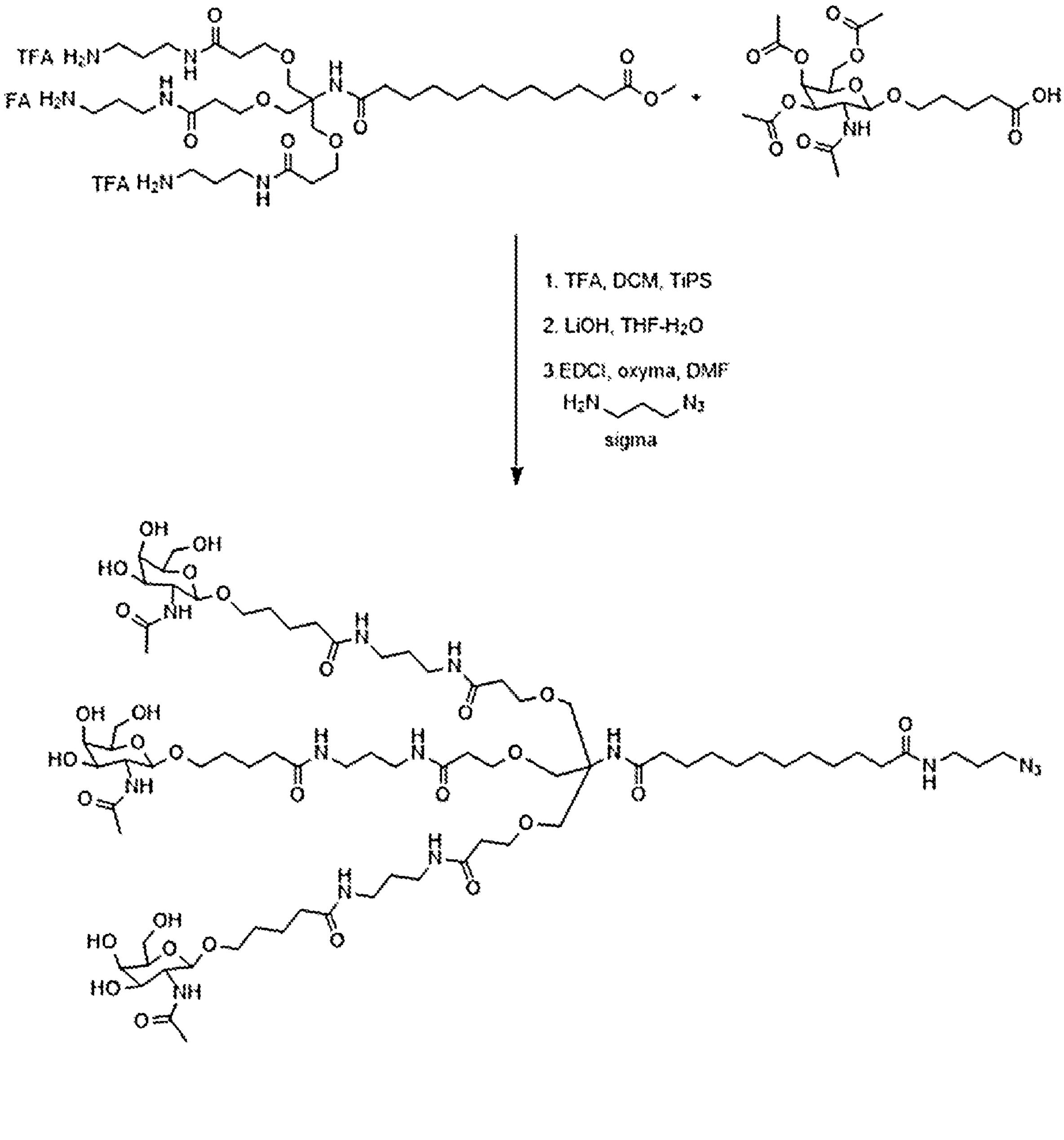

Fig. 2
A

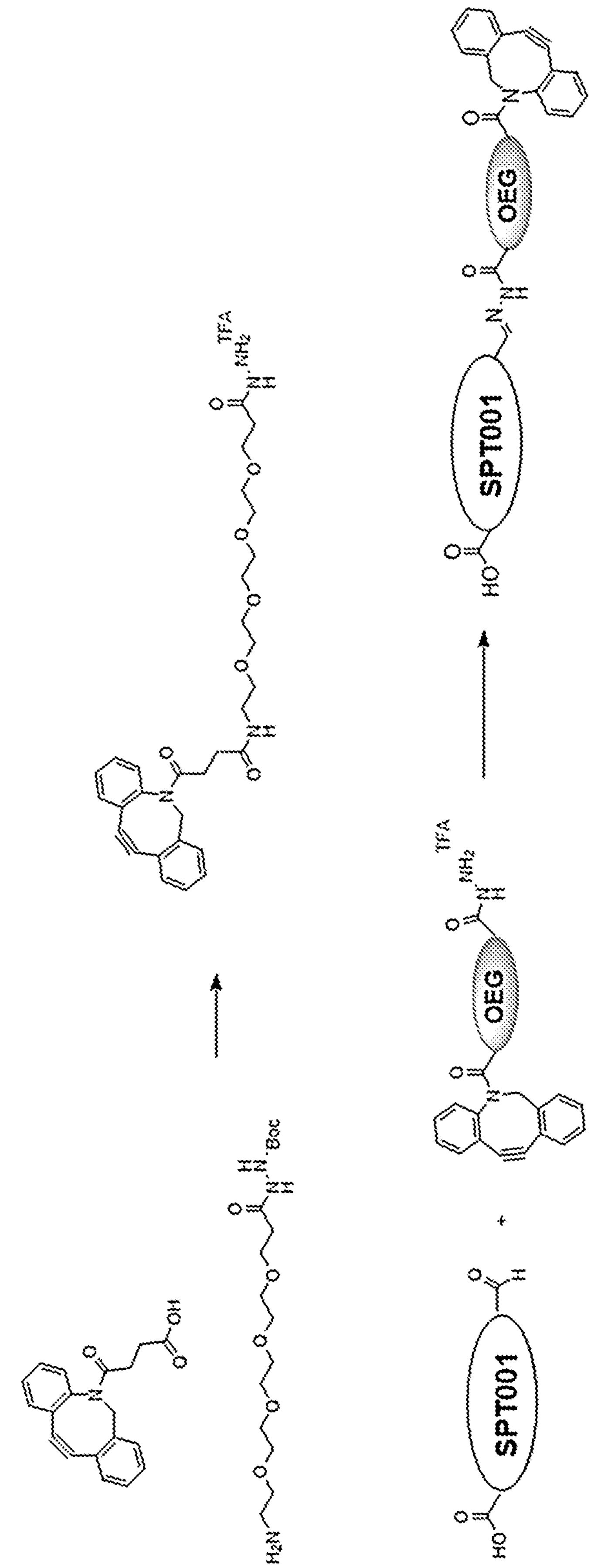

**Fig. 2**
**B**

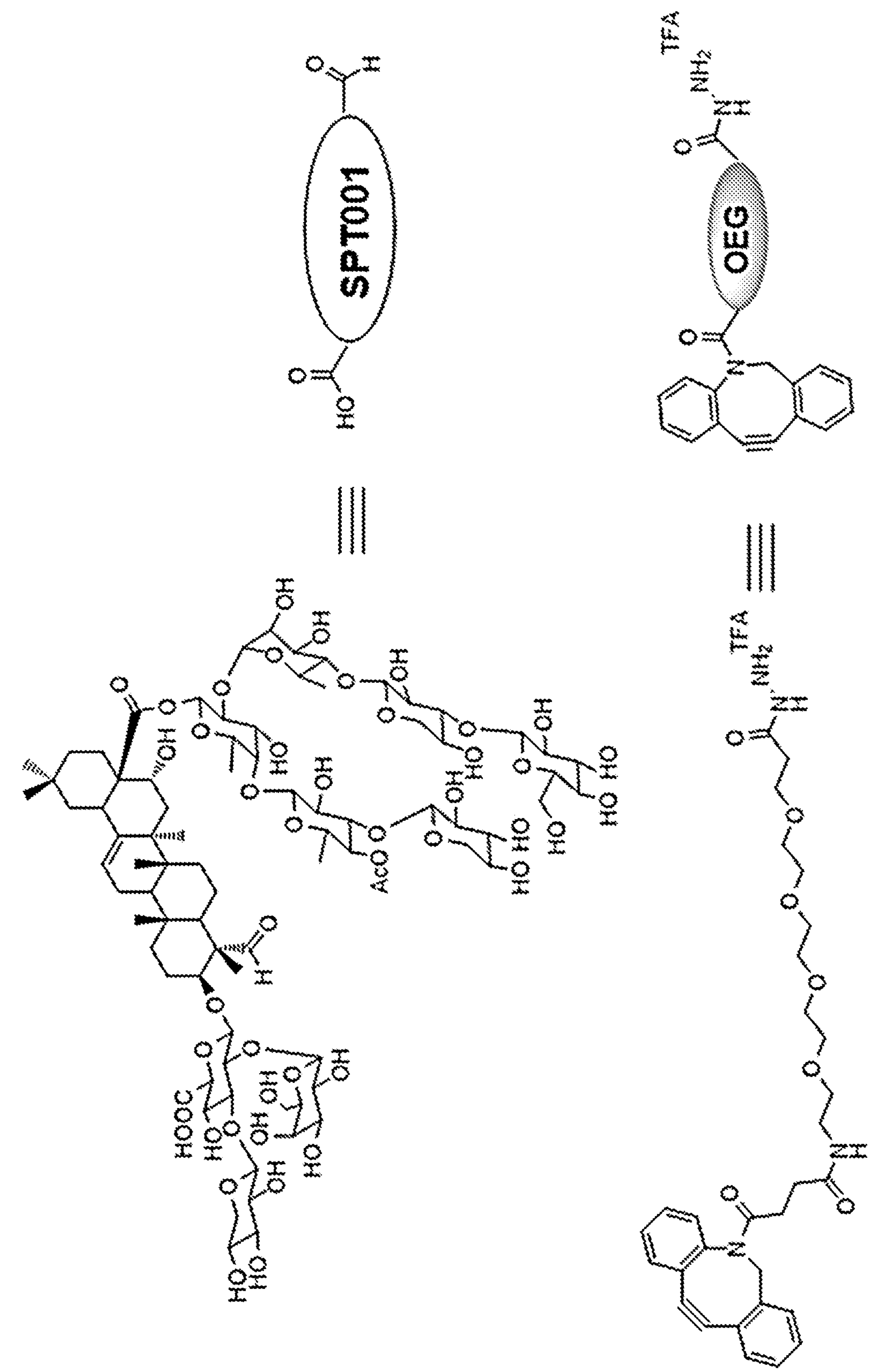

**Fig. 3**

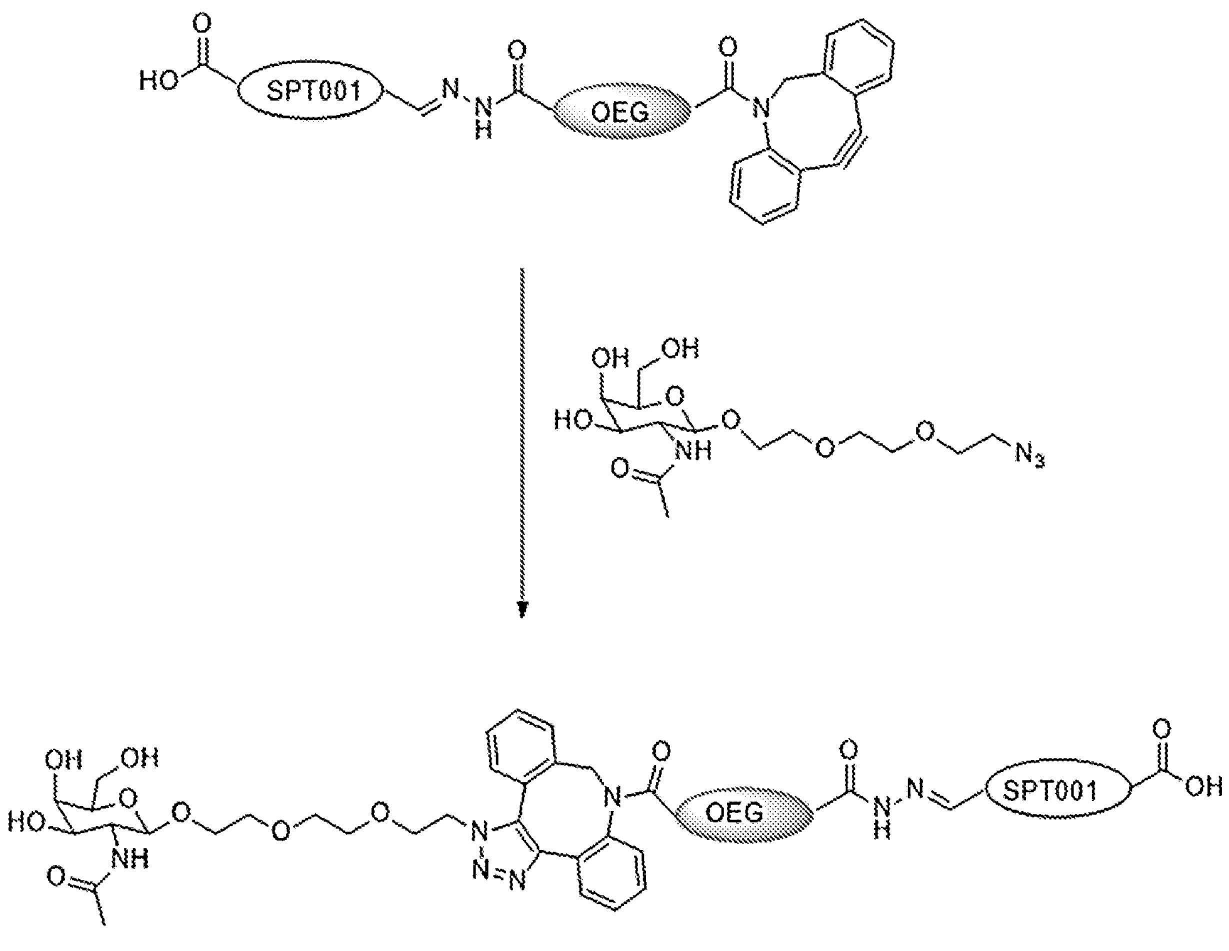

Fig. 4

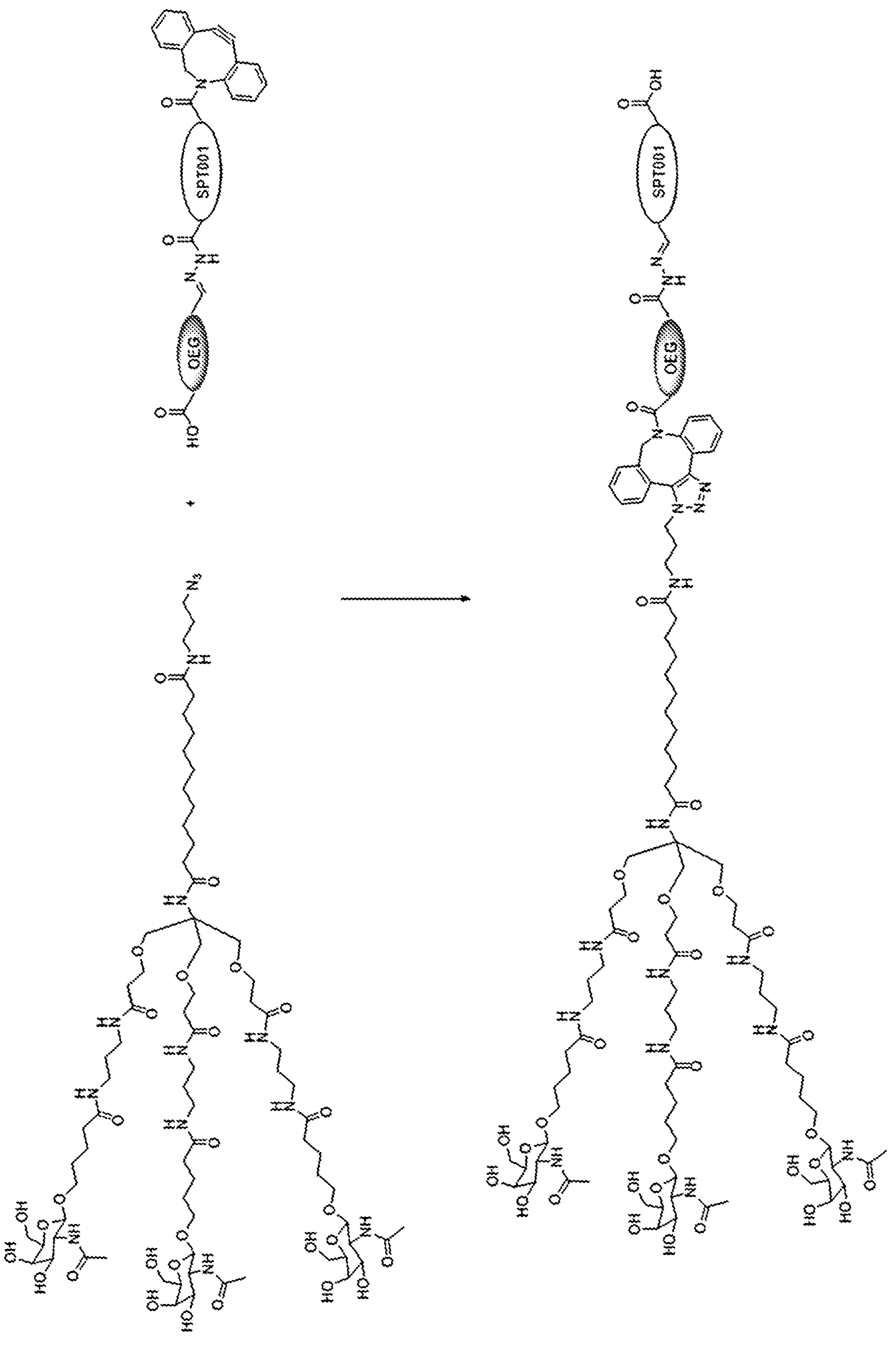

# Fig. 5

## A

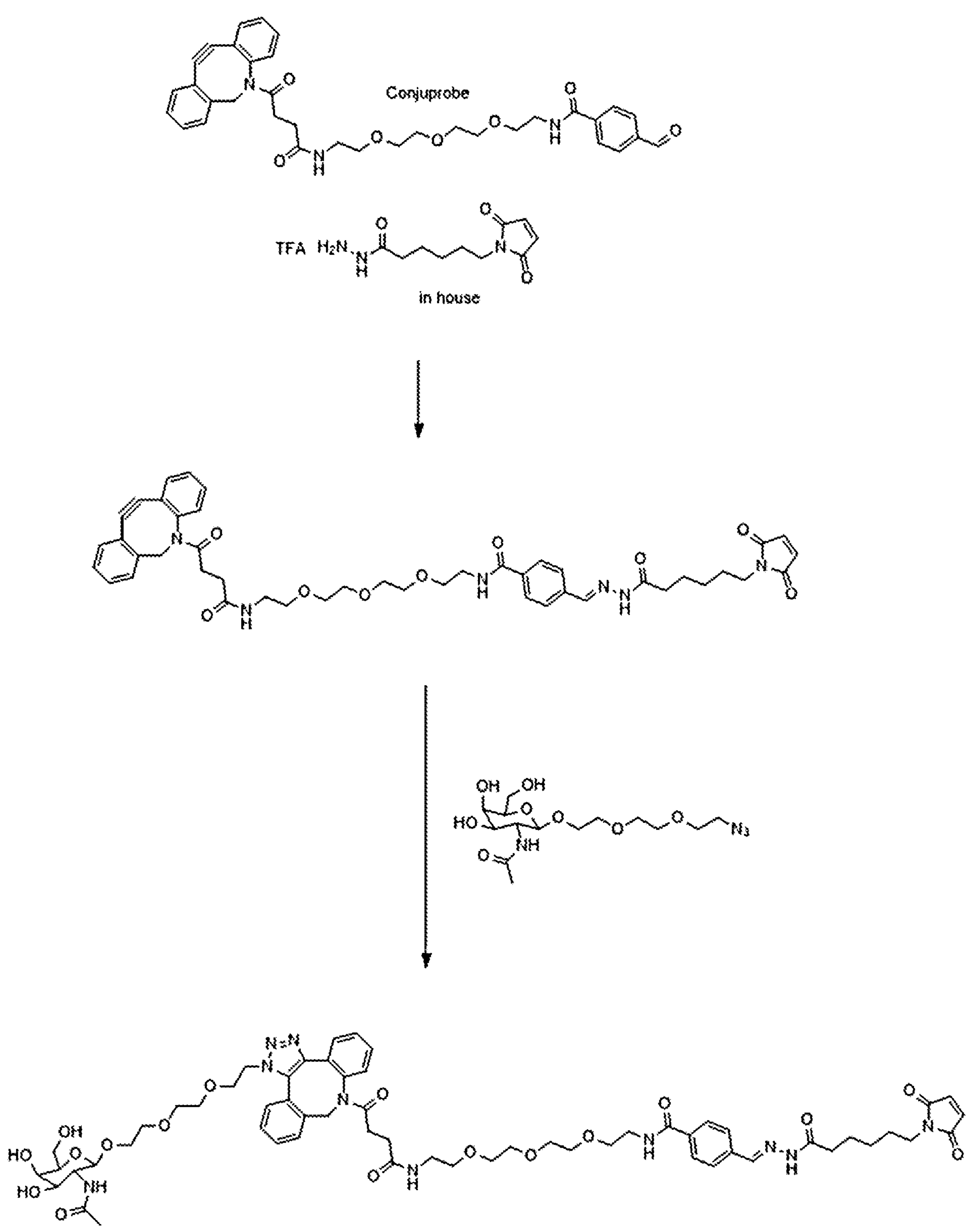

Fig. 5

B

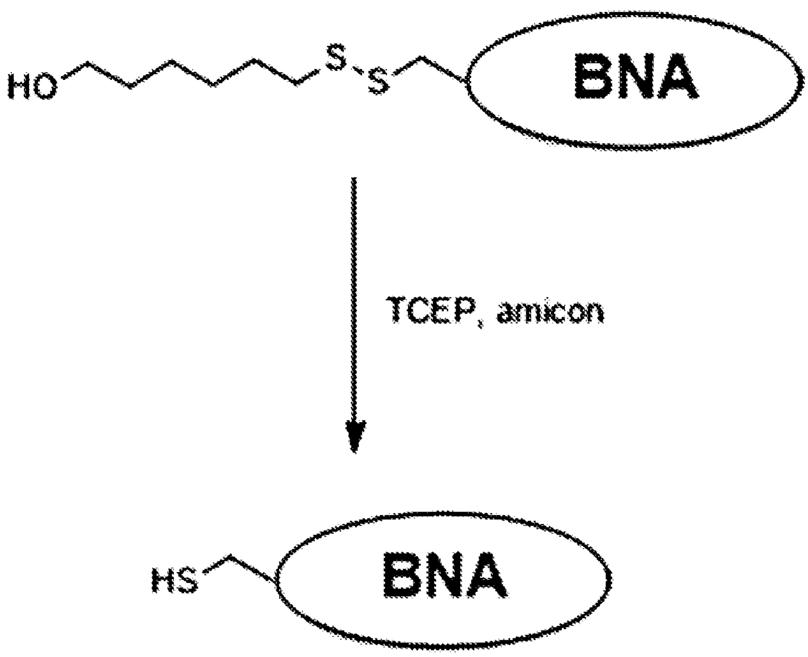

Fig. 5
C

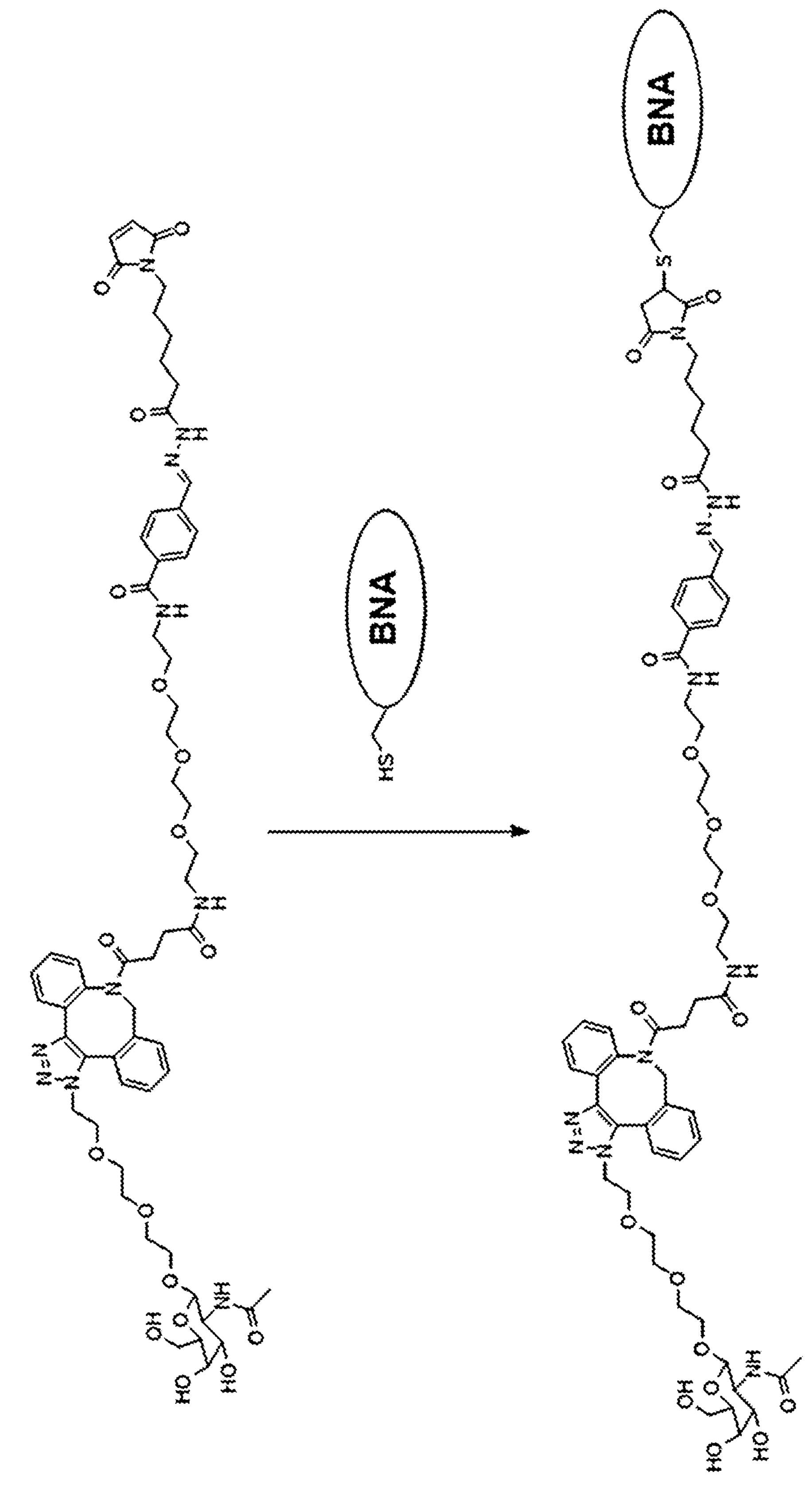

**Fig. 6**

**A**

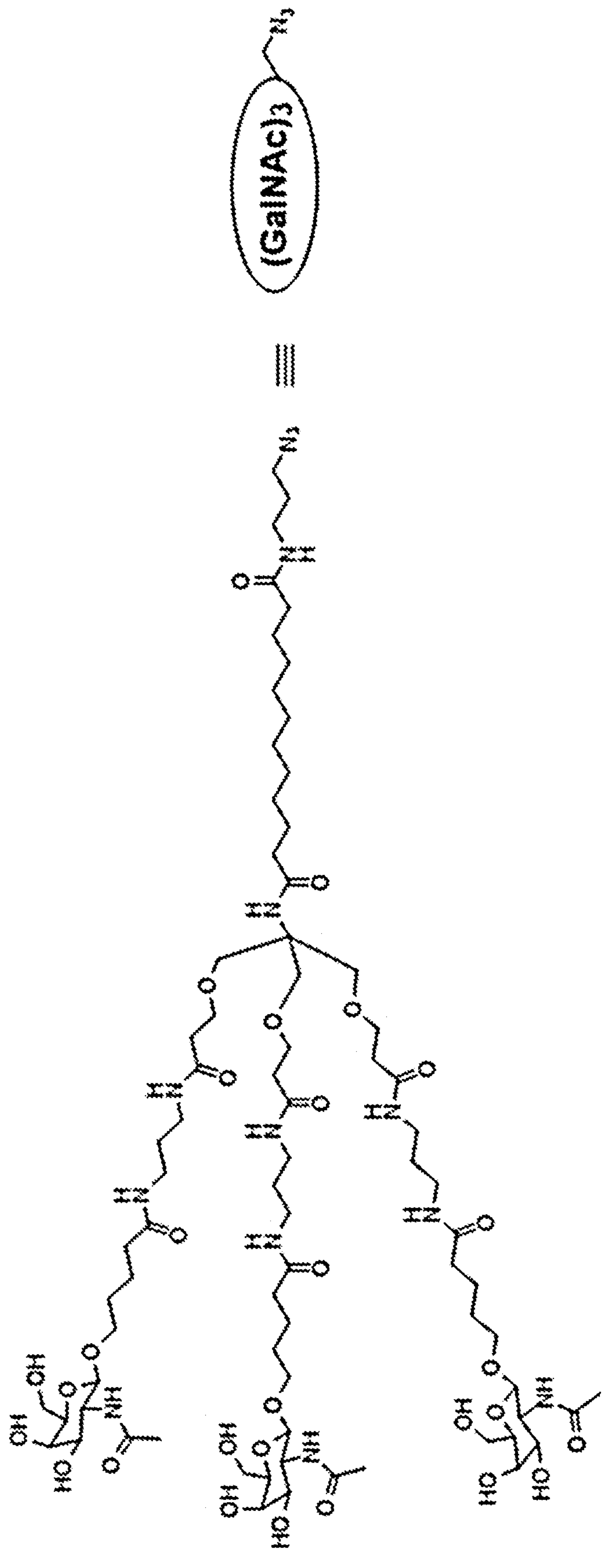

Fig. 6
B

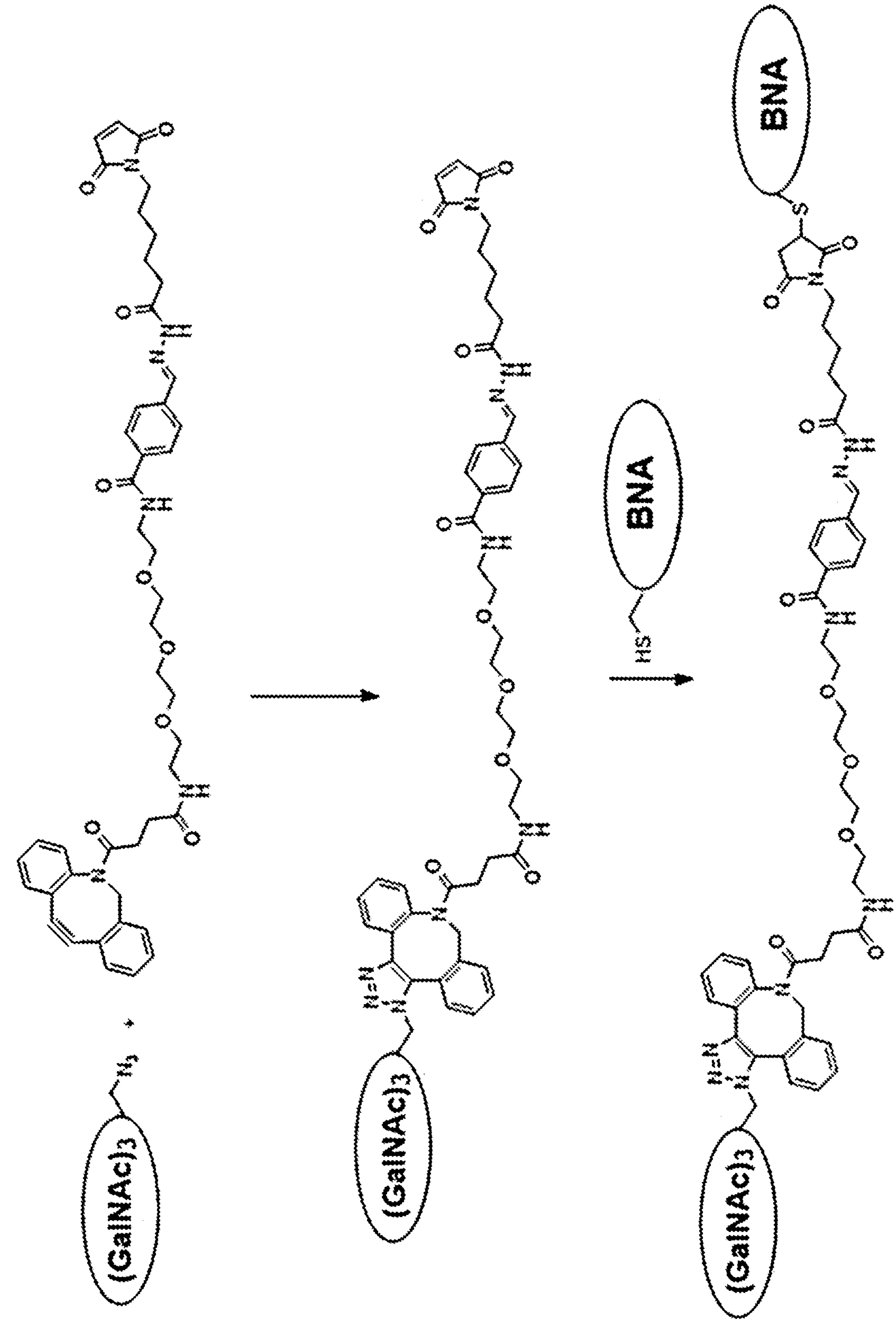

Fig. 7

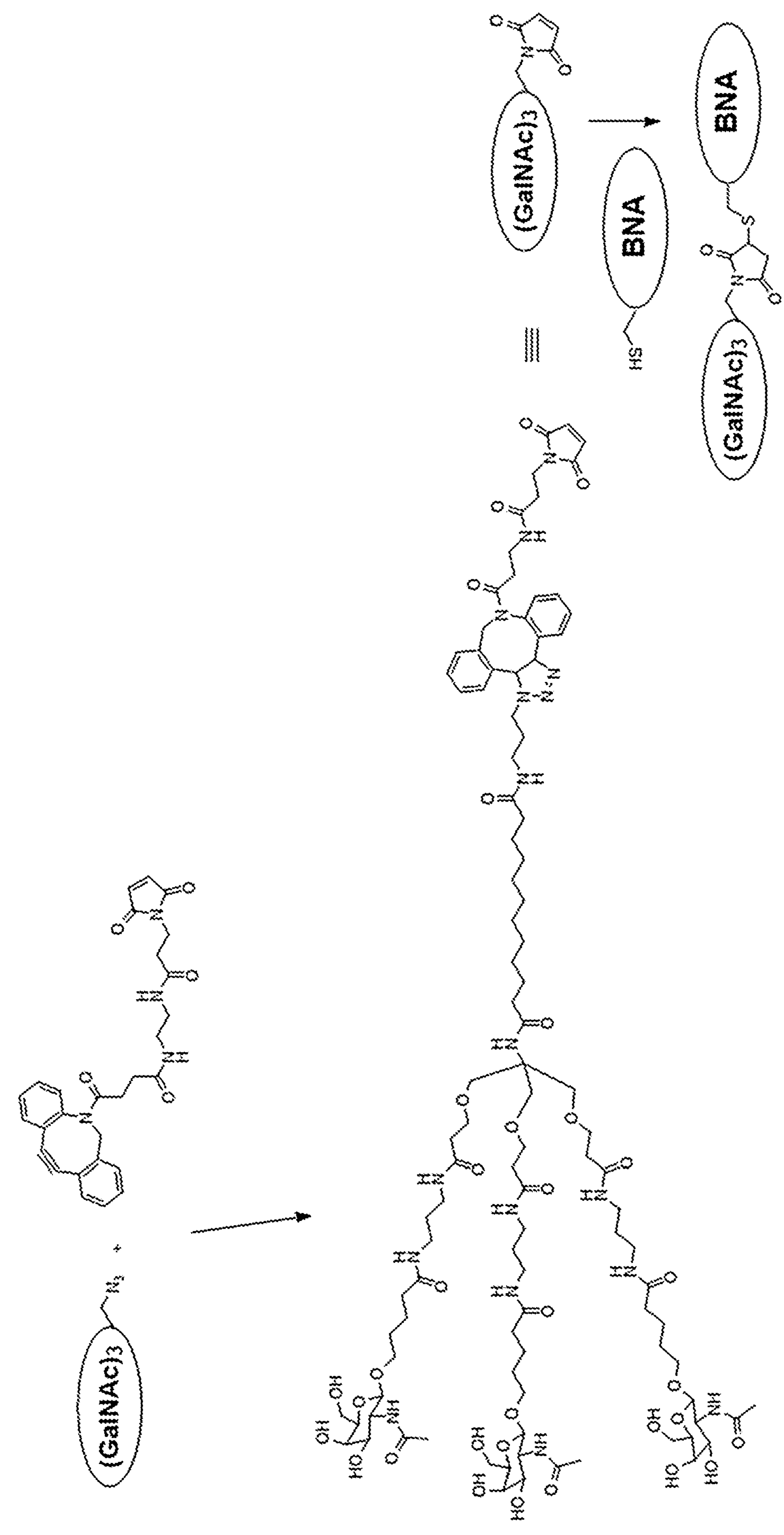

**Fig. 8**
**A**

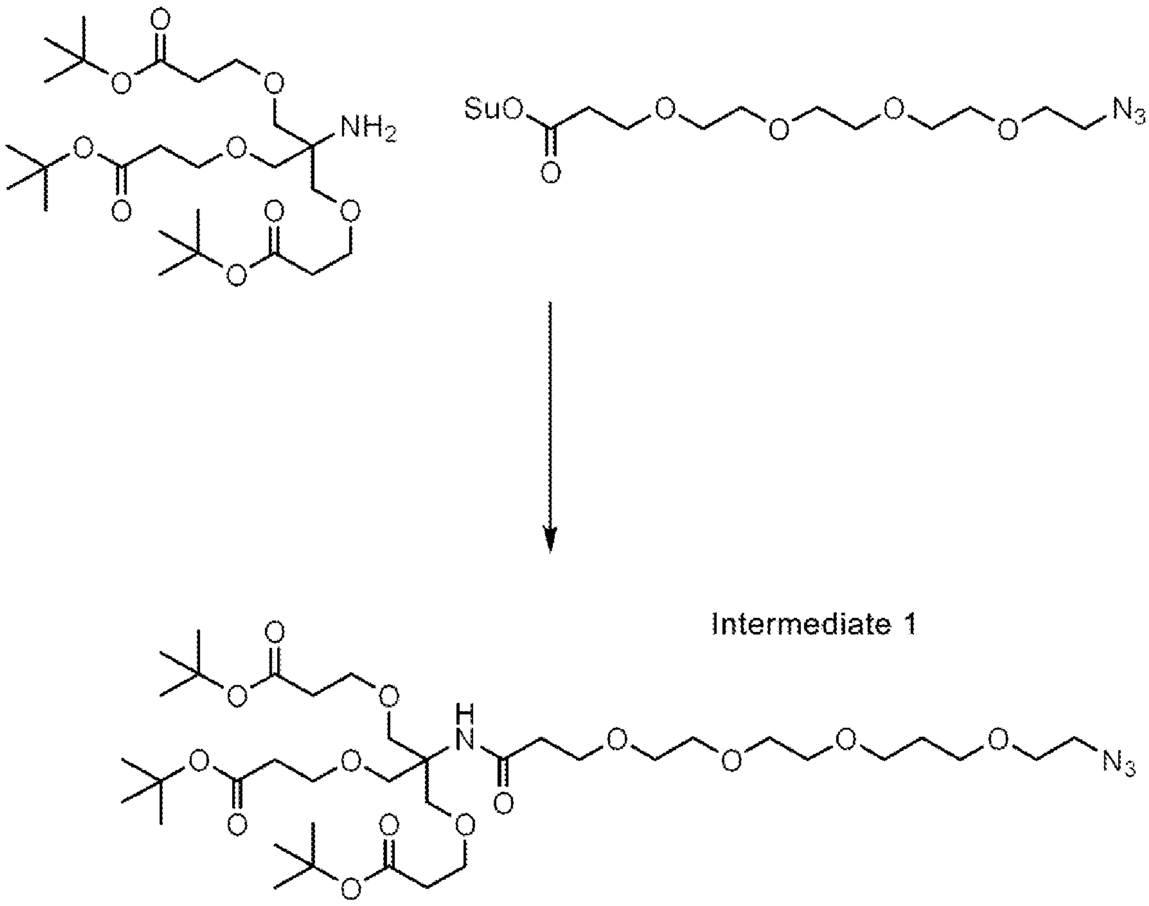

Intermediate 1

**Fig. 8**
**B**

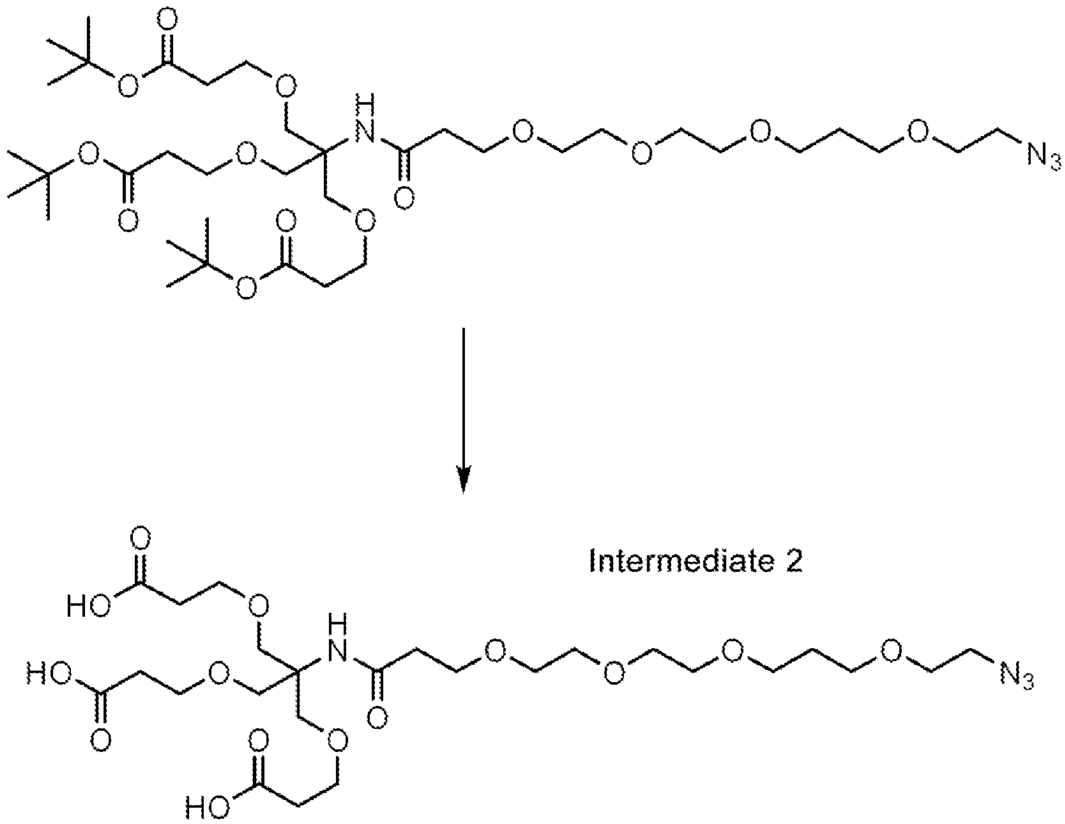

Intermediate 2

**Fig. 8**
**C**

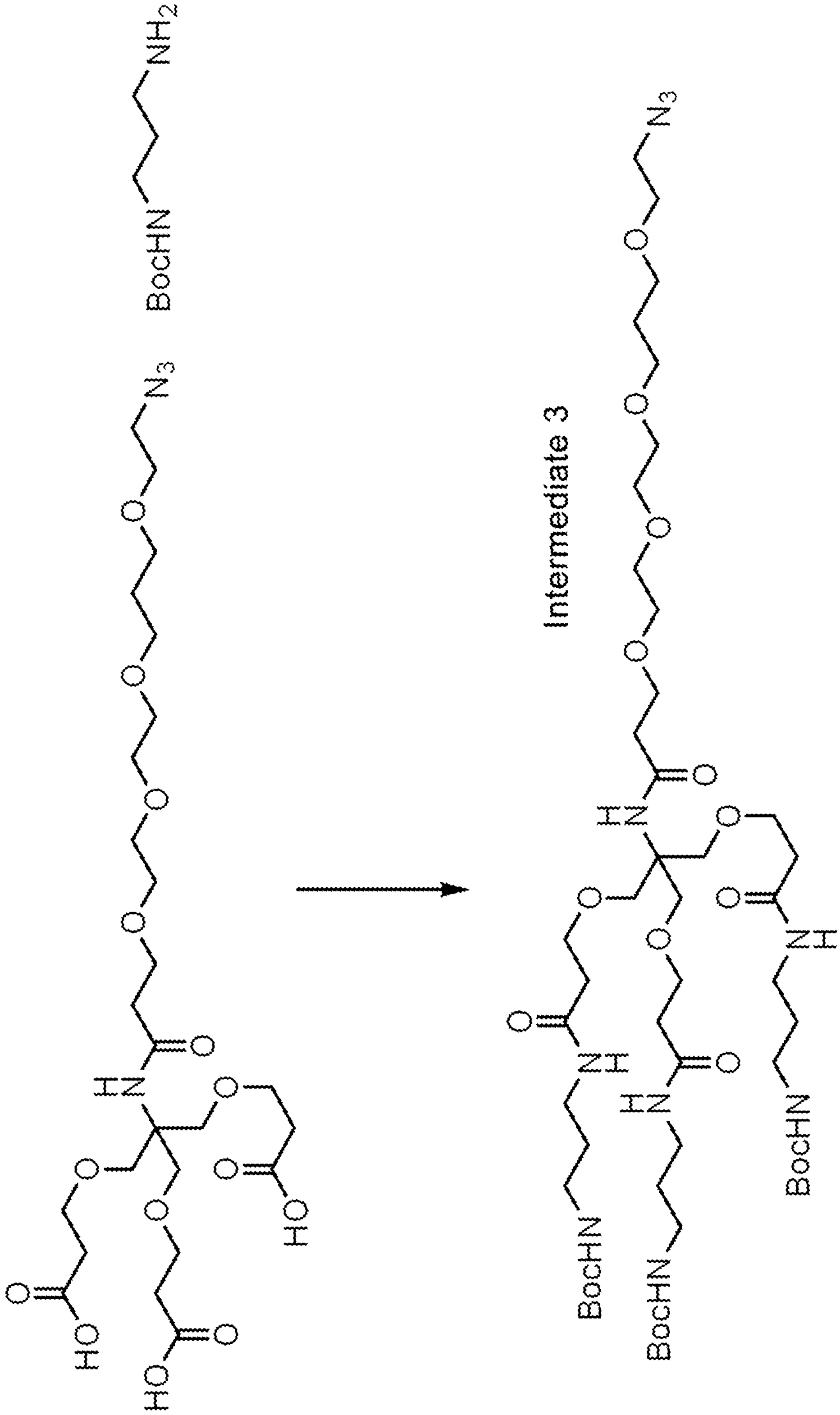

Intermediate 3

Fig. 8
D

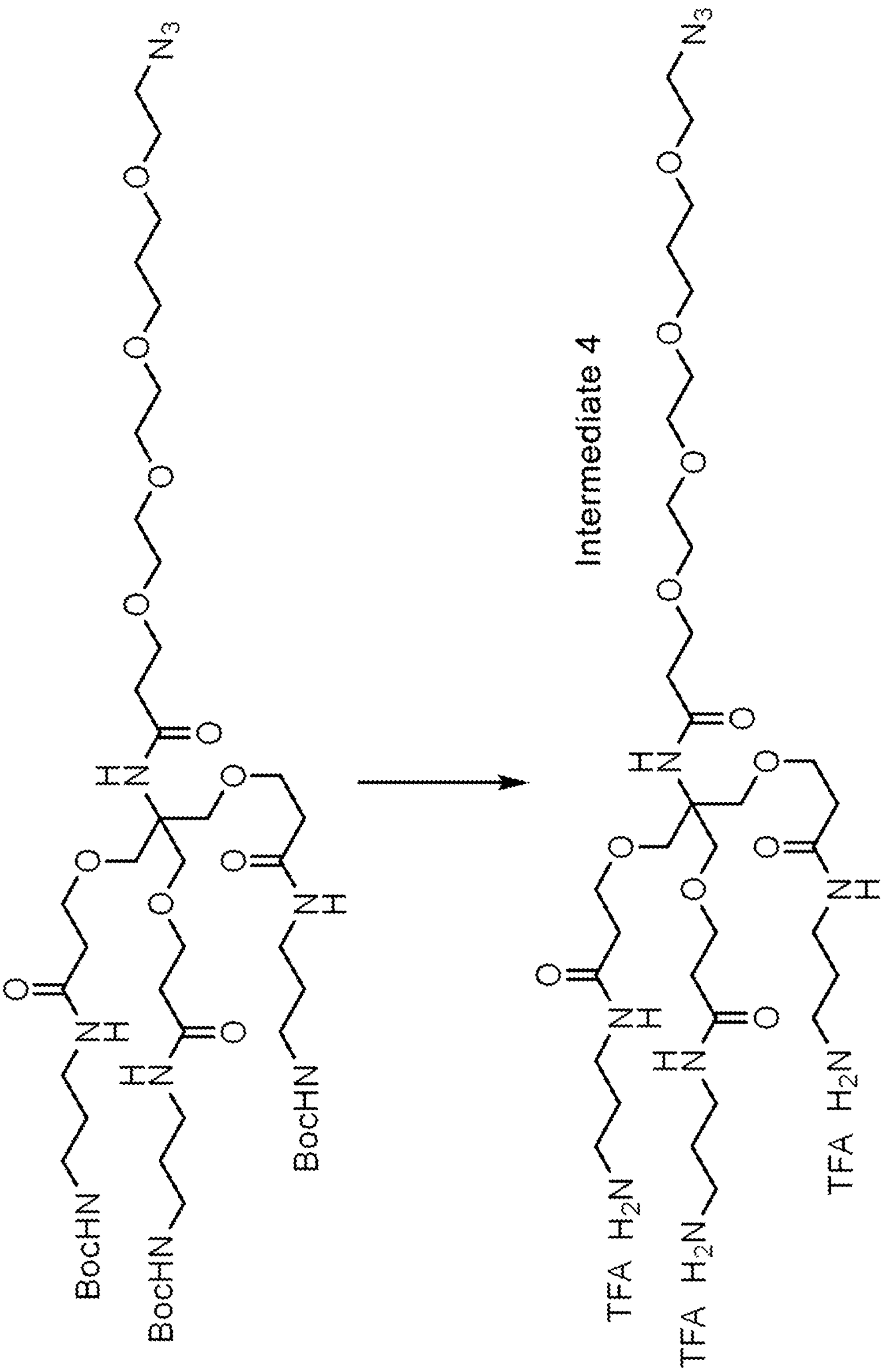

Intermediate 4

**Fig. 8**
**E**

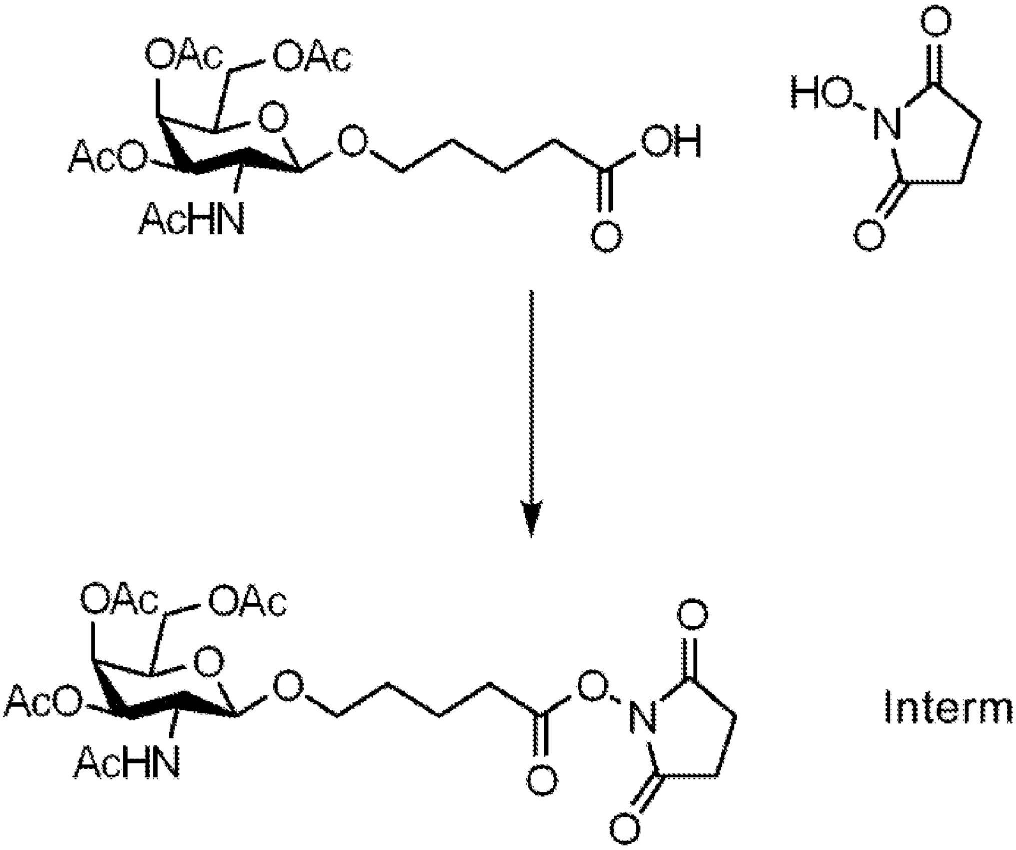

Intermediate 5

**Fig. 8**
**F**

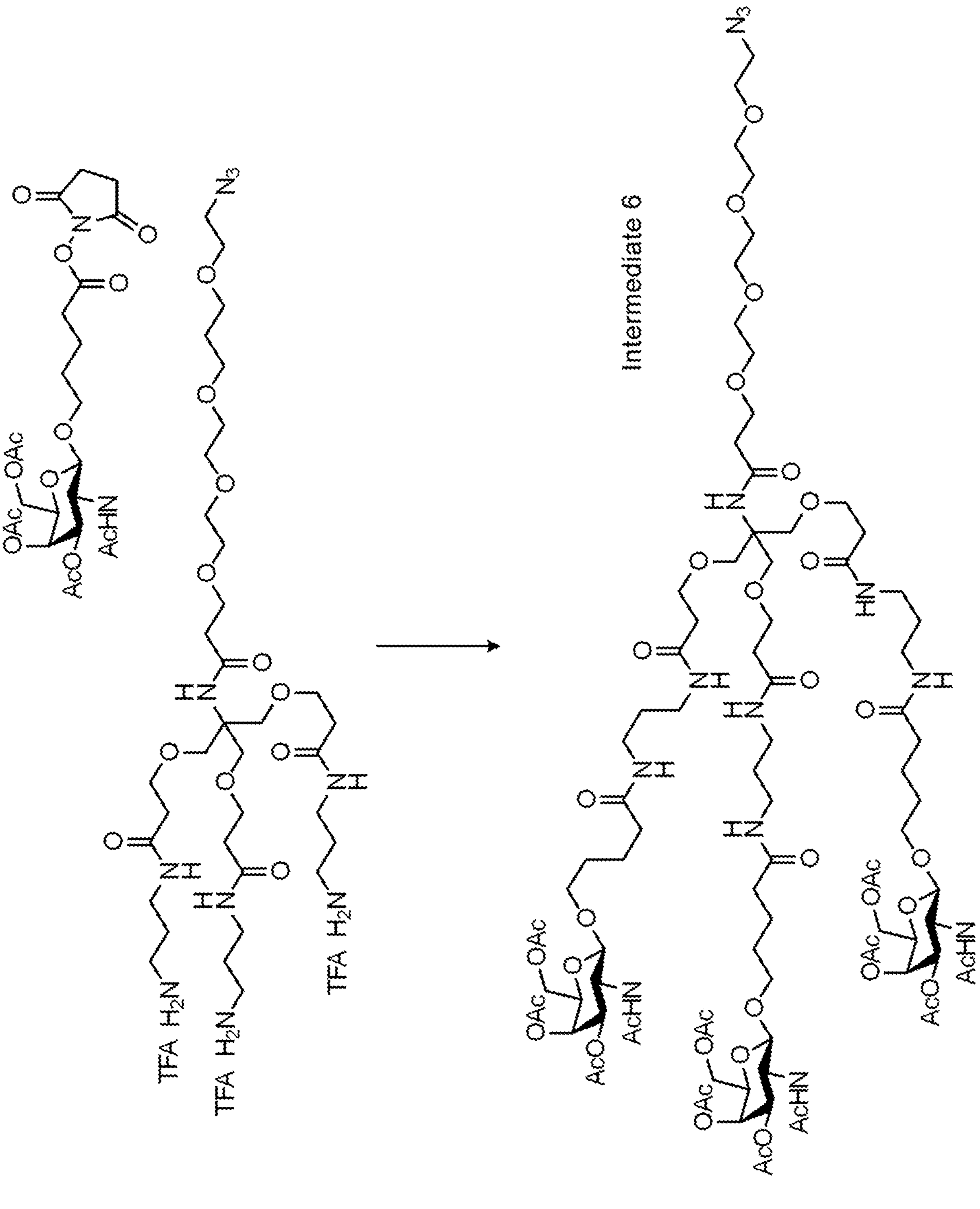

Intermediate 6

# Fig. 8
## G

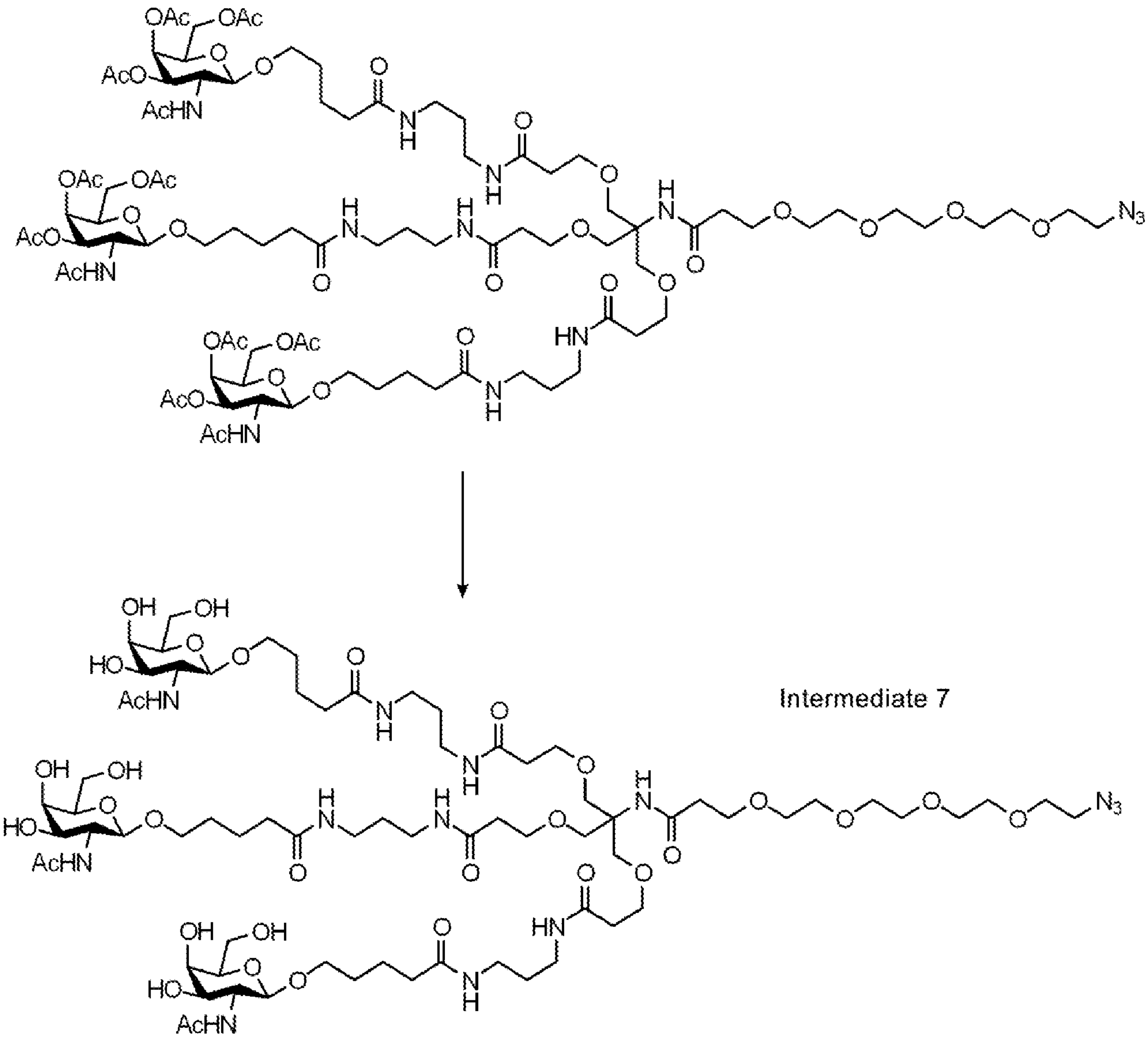

Intermediate 7

=

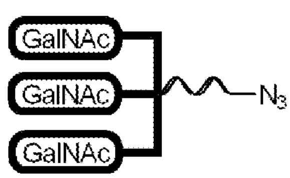

# Fig. 9

## A

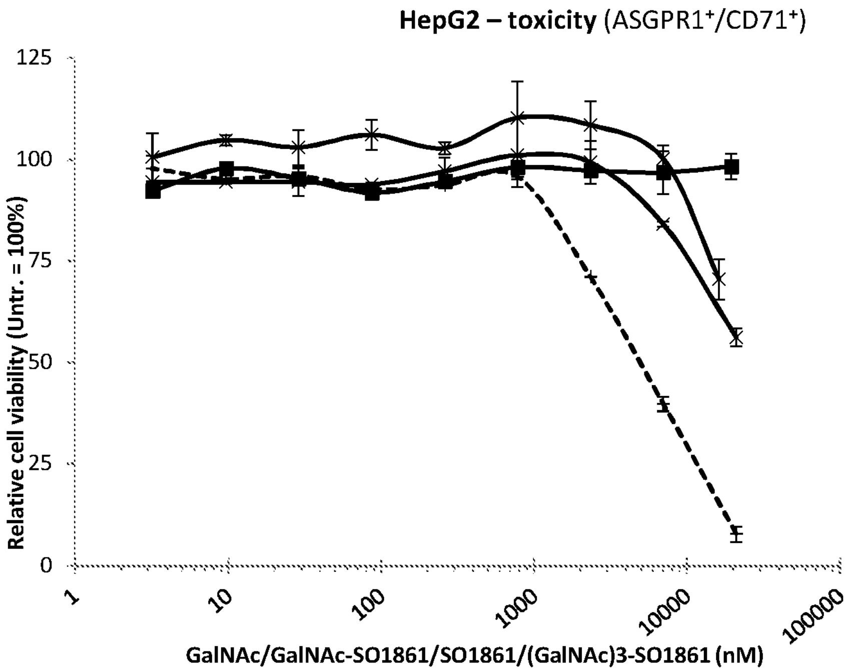
HepG2 – toxicity (ASGPR1+/CD71+)
125
100
75
50
25
0
Relative cell viability (Untr. = 100%)
1
10
100
1000
10000
100000
GalNAc/GalNAc-SO1861/SO1861/(GalNAc)3-SO1861 (nM)

## B

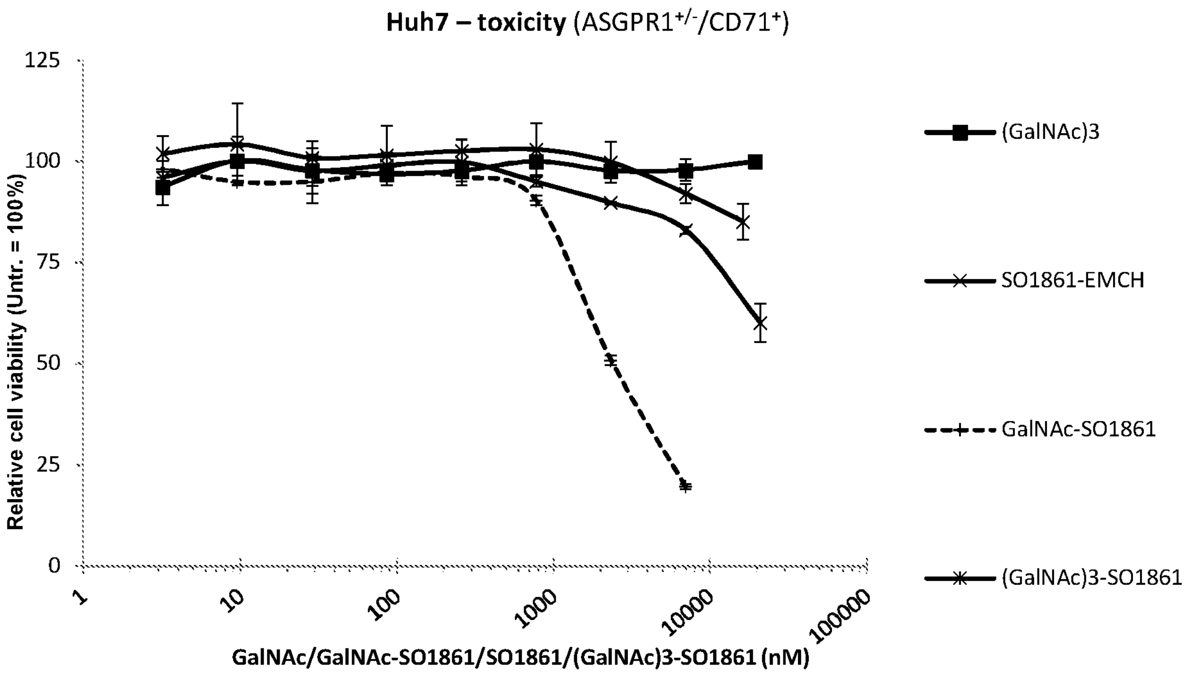
Huh7 – toxicity (ASGPR1+/-/CD71+)
125
100
75
50
25
0
Relative cell viability (Untr. = 100%)
1
10
100
1000
10000
100000
GalNAc/GalNAc-SO1861/SO1861/(GalNAc)3-SO1861 (nM)
(GalNAc)3
SO1861-EMCH
GalNAc-SO1861
(GalNAc)3-SO1861

Fig. 10

A

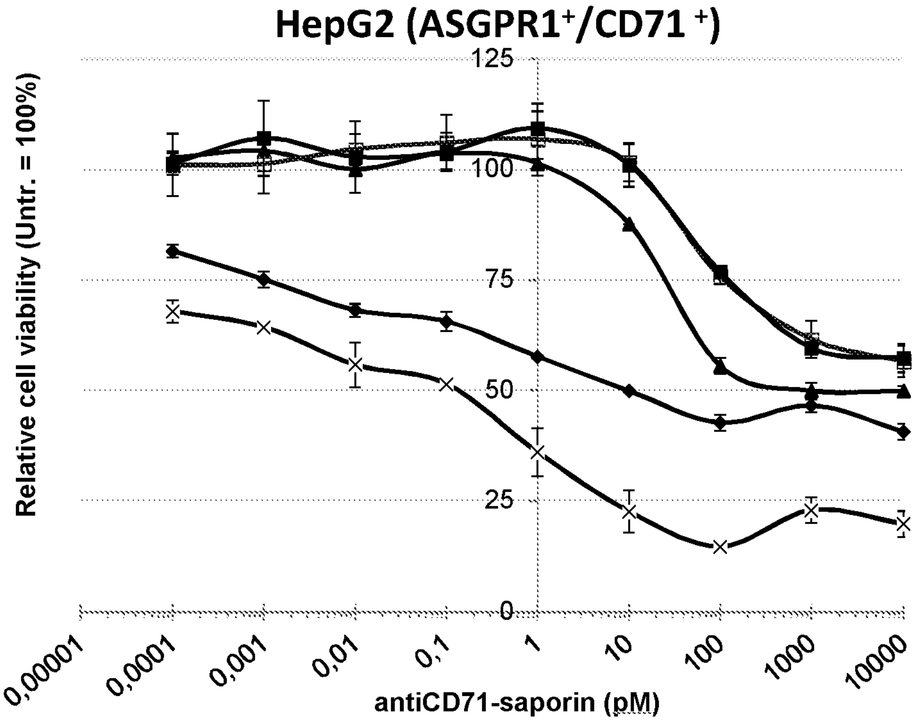
HepG2 (ASGPR1+/CD71 +)
Relative cell viability (Untr. = 100%)
125
100
75
50
25
0
0,00001
0,0001
0,001
0,01
0,1
1
10
100
1000
10000
antiCD71-saporin (pM)

B

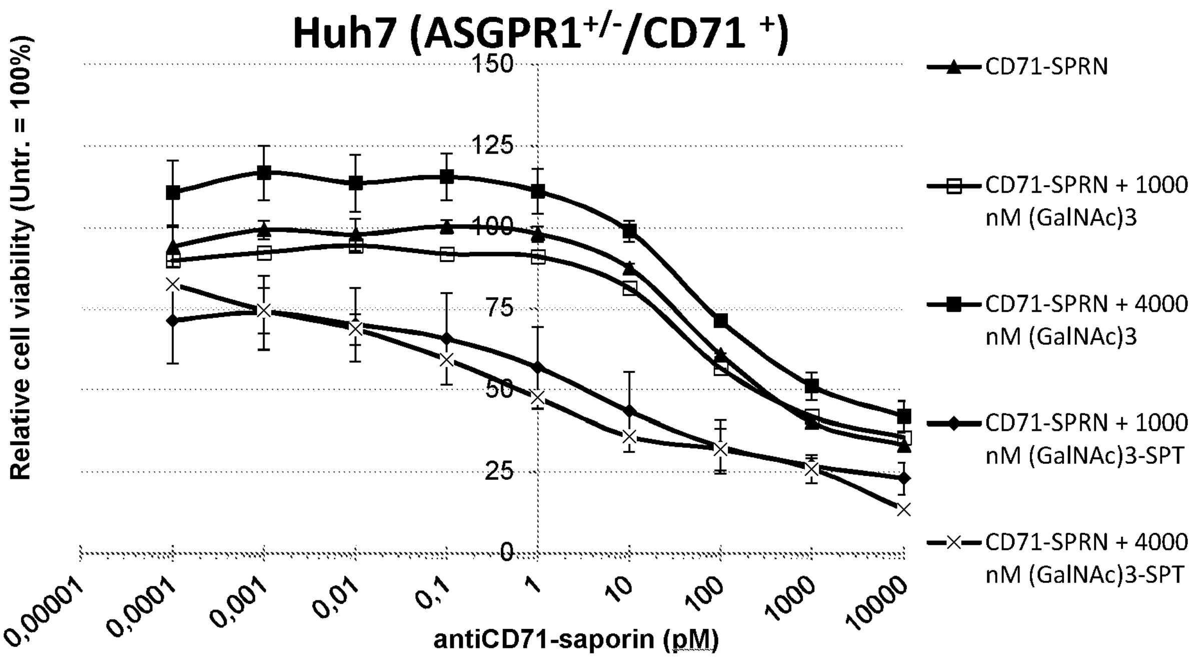
Huh7 (ASGPR1+/-/CD71 +)
Relative cell viability (Untr. = 100%)
150
125
100
75
50
25
0
0,00001
0,0001
0,001
0,01
0,1
1
10
100
1000
10000
antiCD71-saporin (pM)
CD71-SPRN
CD71-SPRN + 1000 nM (GalNAc)3
CD71-SPRN + 4000 nM (GalNAc)3
CD71-SPRN + 1000 nM (GalNAc)3-SPT
CD71-SPRN + 4000 nM (GalNAc)3-SPT

Fig. 11

A

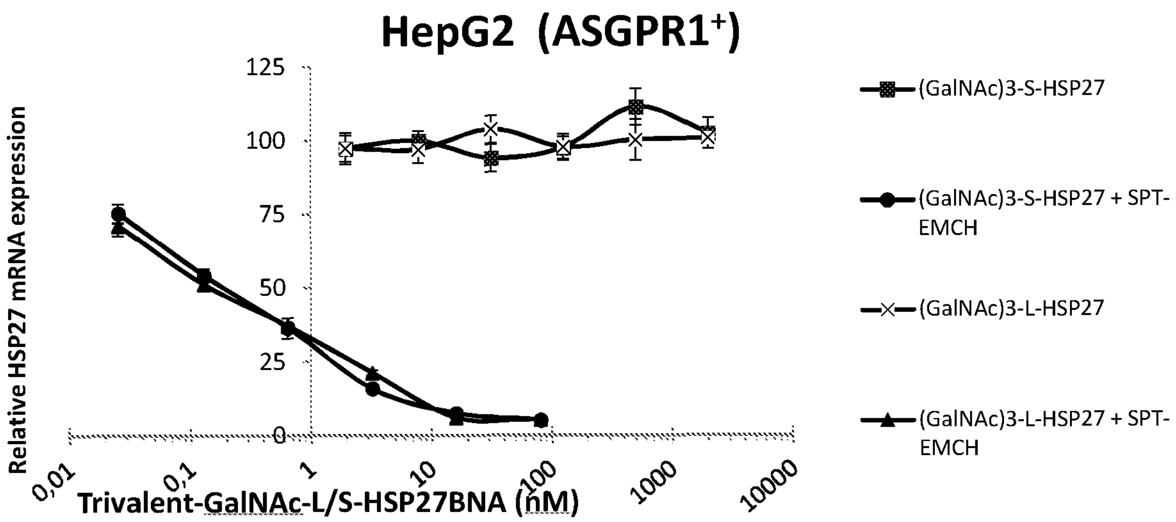
HepG2 (ASGPR1+)
125
100
75
50
25
0
0,01
0,1
1
10
100
1000
10000
Relative HSP27 mRNA expression
Trivalent-GalNAc-L/S-HSP27BNA (nM)
(GalNAc)3-S-HSP27
(GalNAc)3-S-HSP27 + SPT-EMCH
(GalNAc)3-L-HSP27
(GalNAc)3-L-HSP27 + SPT-EMCH

B

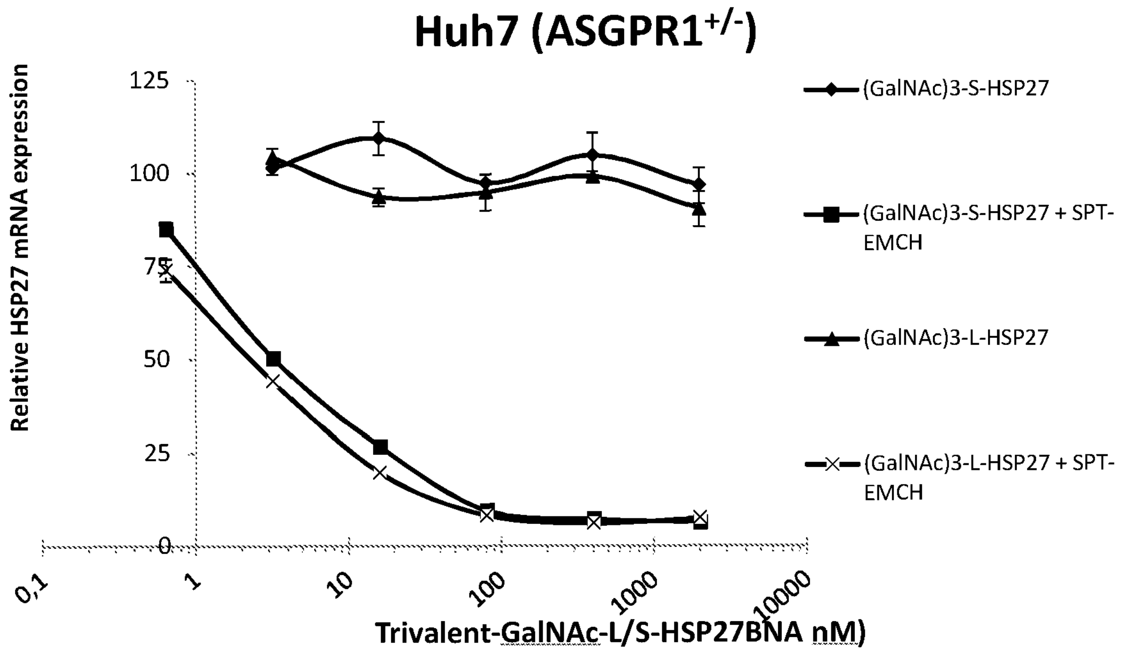
Huh7 (ASGPR1+/-)
125
100
75
50
25
0
0,1
1
10
100
1000
10000
Relative HSP27 mRNA expression
Trivalent-GalNAc-L/S-HSP27BNA nM)
(GalNAc)3-S-HSP27
(GalNAc)3-S-HSP27 + SPT-EMCH
(GalNAc)3-L-HSP27
(GalNAc)3-L-HSP27 + SPT-EMCH

# Fig. 12

## A

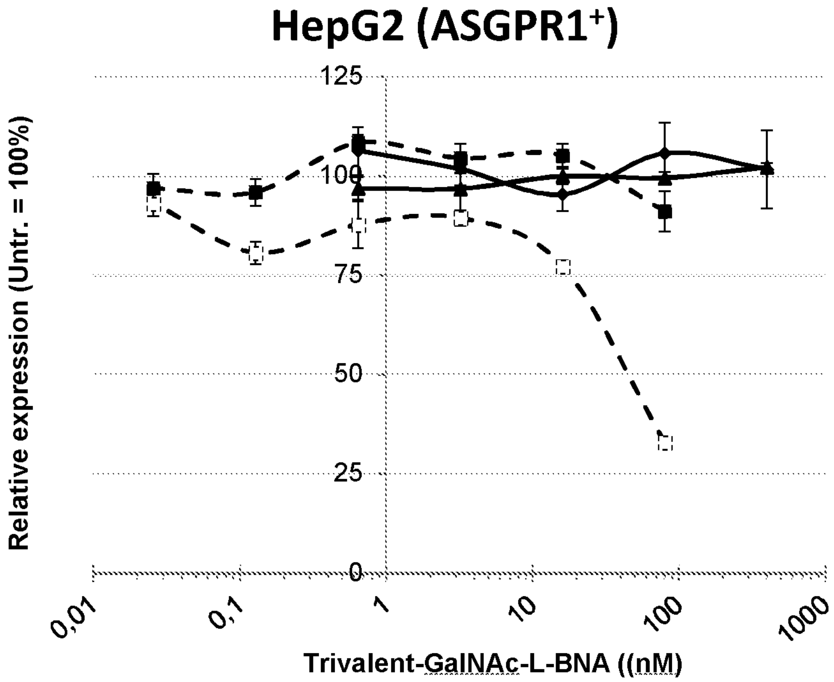
HepG2 (ASGPR1+)
Relative expression (Untr. = 100%)
125
100
75
50
25
0
0,01
0,1
1
10
100
1000
Trivalent-GalNAc-L-BNA ((nM)

## B

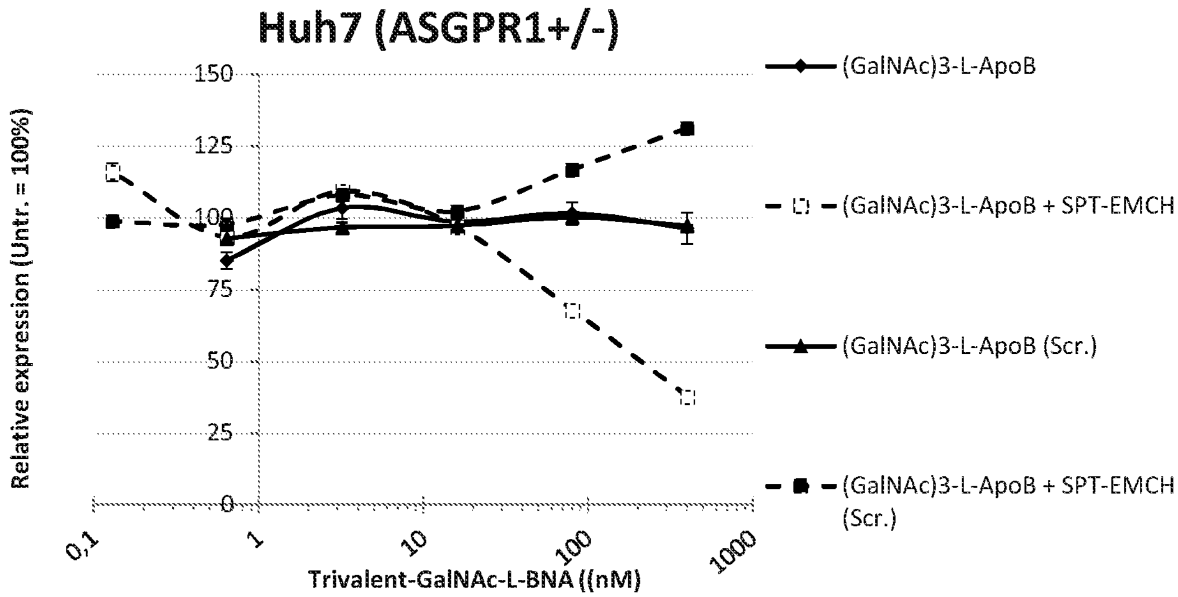
Huh7 (ASGPR1+/-)
Relative expression (Untr. = 100%)
150
125
100
75
50
25
0
0,1
1
10
100
1000
Trivalent-GalNAc-L-BNA ((nM)
(GalNAc)3-L-ApoB
(GalNAc)3-L-ApoB + SPT-EMCH
(GalNAc)3-L-ApoB (Scr.)
(GalNAc)3-L-ApoB + SPT-EMCH (Scr.)

# Fig. 13

## A

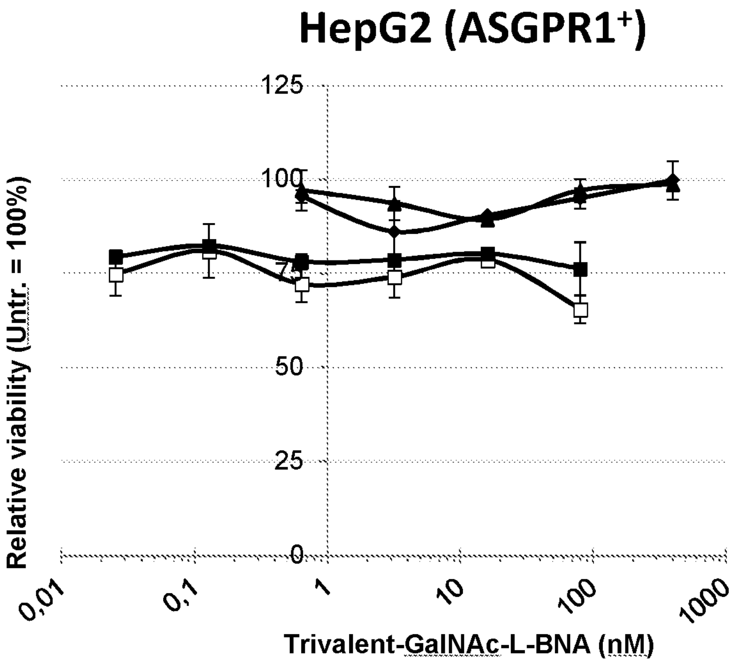
HepG2 (ASGPR1⁺)
Relative viability (Untr. = 100%)
125
100
75
50
25
0
0,01
0,1
1
10
100
1000
Trivalent-GalNAc-L-BNA (nM)

## B

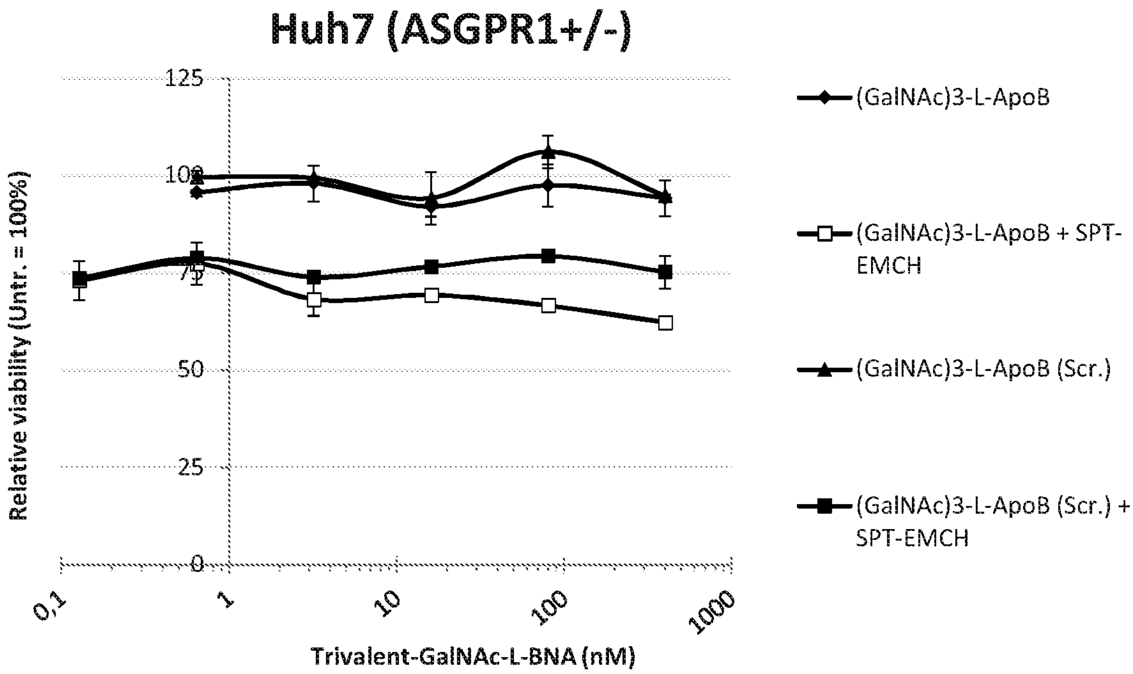
Huh7 (ASGPR1+/-)
Relative viability (Untr. = 100%)
125
100
75
50
25
0
0,1
1
10
100
1000
Trivalent-GalNAc-L-BNA (nM)
(GalNAc)3-L-ApoB
(GalNAc)3-L-ApoB + SPT-EMCH
(GalNAc)3-L-ApoB (Scr.)
(GalNAc)3-L-ApoB (Scr.) + SPT-EMCH

## Fig. 14
### A

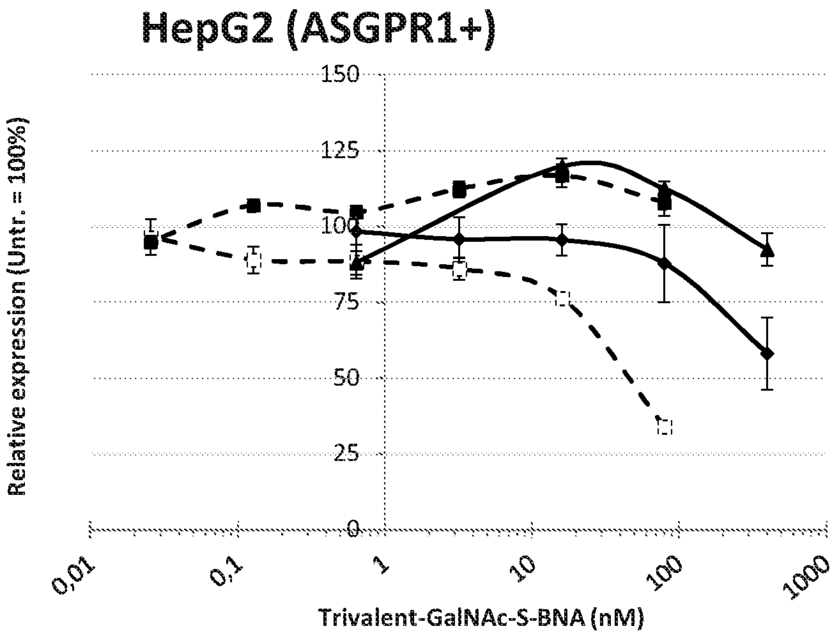
HepG2 (ASGPR1+)
Relative expression (Untr. = 100%)
150
125
100
75
50
25
0
0,01
0,1
1
10
100
1000
Trivalent-GalNAc-S-BNA (nM)

### B

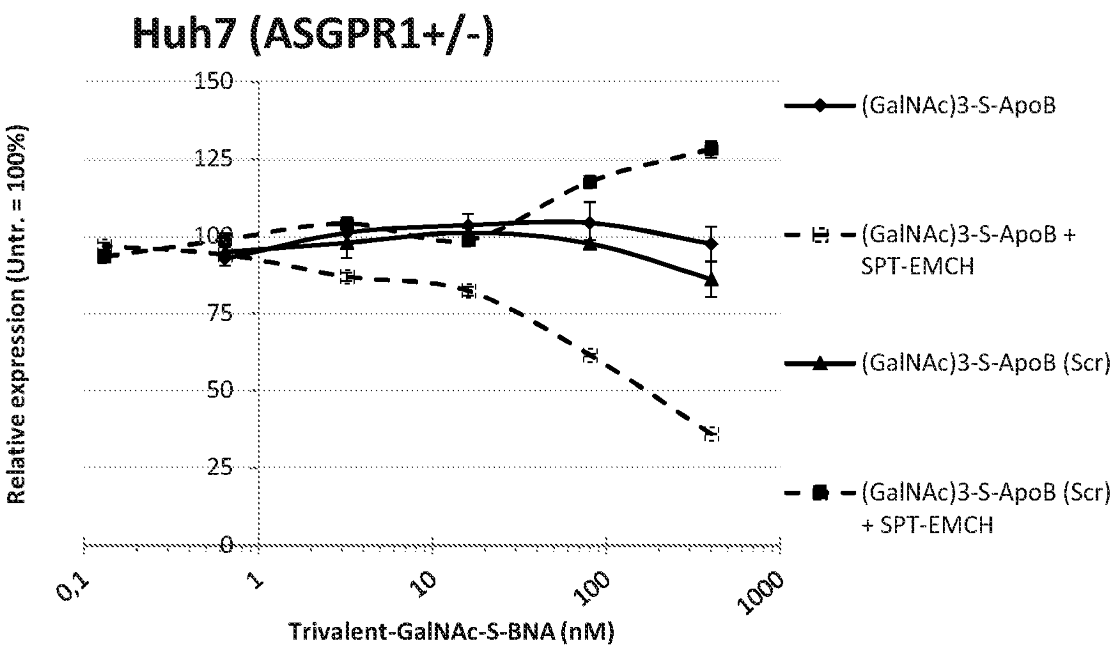
Huh7 (ASGPR1+/-)
Relative expression (Untr. = 100%)
150
125
100
75
50
25
0
0,1
1
10
100
1000
Trivalent-GalNAc-S-BNA (nM)
(GalNAc)3-S-ApoB
(GalNAc)3-S-ApoB + SPT-EMCH
(GalNAc)3-S-ApoB (Scr)
(GalNAc)3-S-ApoB (Scr) + SPT-EMCH

Fig. 15

A

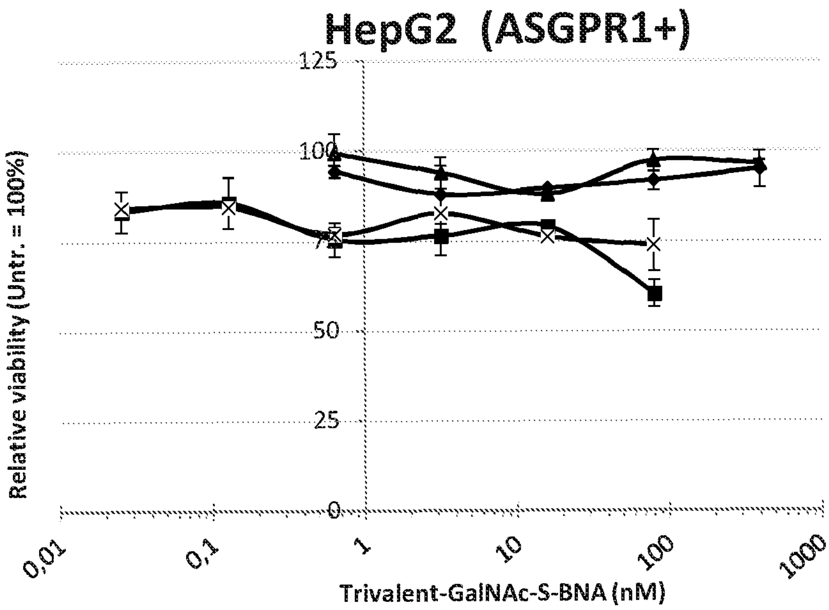
HepG2 (ASGPR1+)
125
100
75
50
25
0
Relative viability (Untr. = 100%)
0,01
0,1
1
10
100
1000
Trivalent-GalNAc-S-BNA (nM)

B

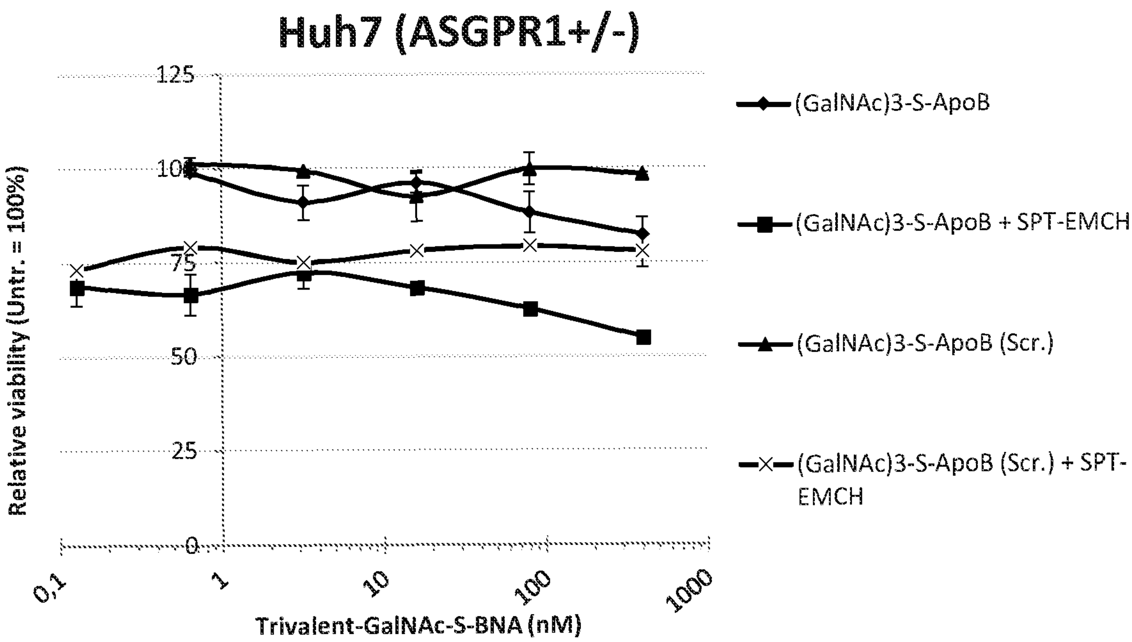
Huh7 (ASGPR1+/-)
125
100
75
50
25
0
Relative viability (Untr. = 100%)
0,1
1
10
100
1000
Trivalent-GalNAc-S-BNA (nM)
(GalNAc)3-S-ApoB
(GalNAc)3-S-ApoB + SPT-EMCH
(GalNAc)3-S-ApoB (Scr.)
(GalNAc)3-S-ApoB (Scr.) + SPT-EMCH

**Fig. 16**

**A**

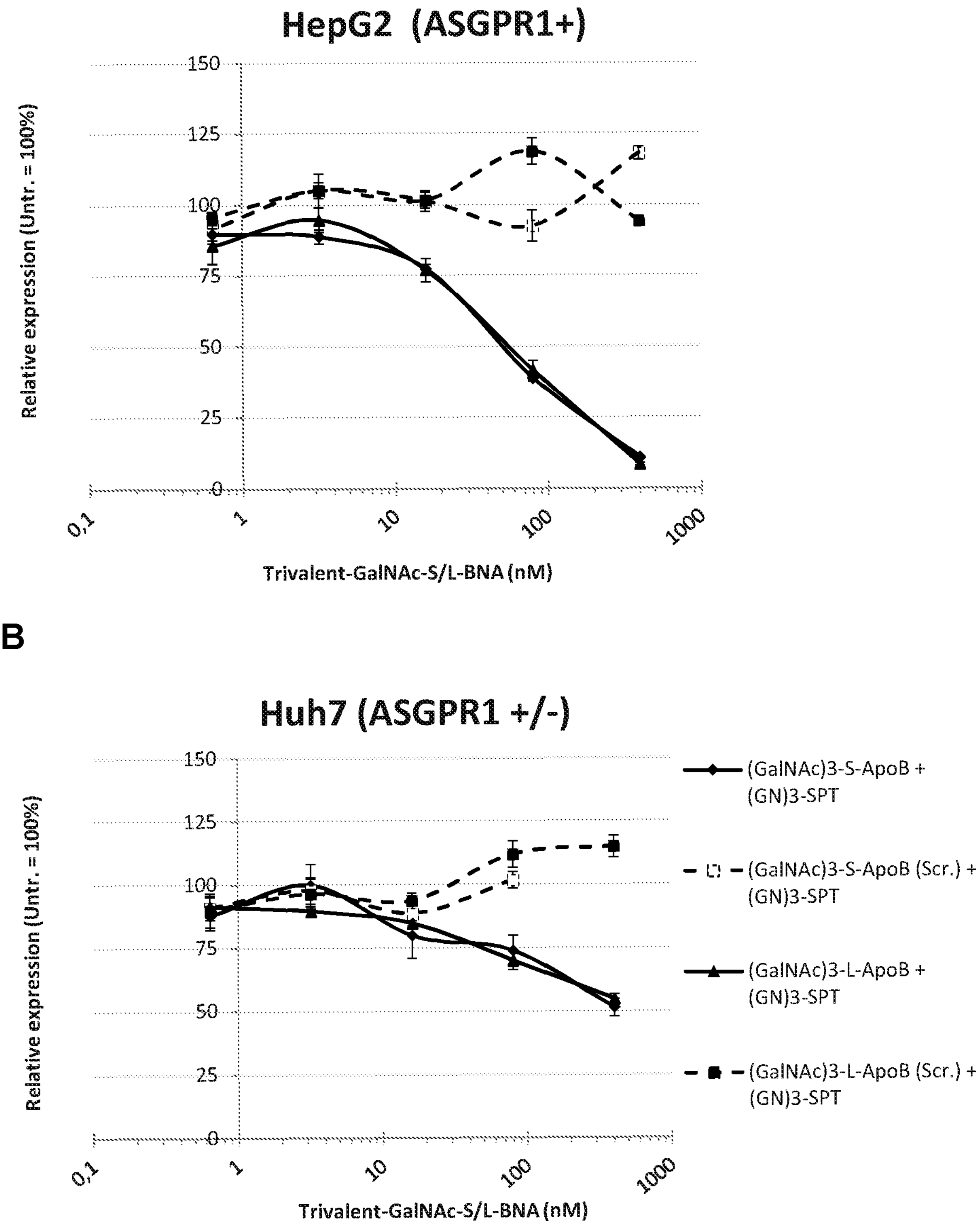
HepG2 (ASGPR1+)
Relative expression (Untr. = 100%)
150
125
100
75
50
25
0
0,1
1
10
100
1000
Trivalent-GalNAc-S/L-BNA (nM)
B
Huh7 (ASGPR1 +/-)
(GalNAc)3-S-ApoB + (GN)3-SPT
(GalNAc)3-S-ApoB (Scr.) + (GN)3-SPT
(GalNAc)3-L-ApoB + (GN)3-SPT
(GalNAc)3-L-ApoB (Scr.) + (GN)3-SPT

Fig. 16
C

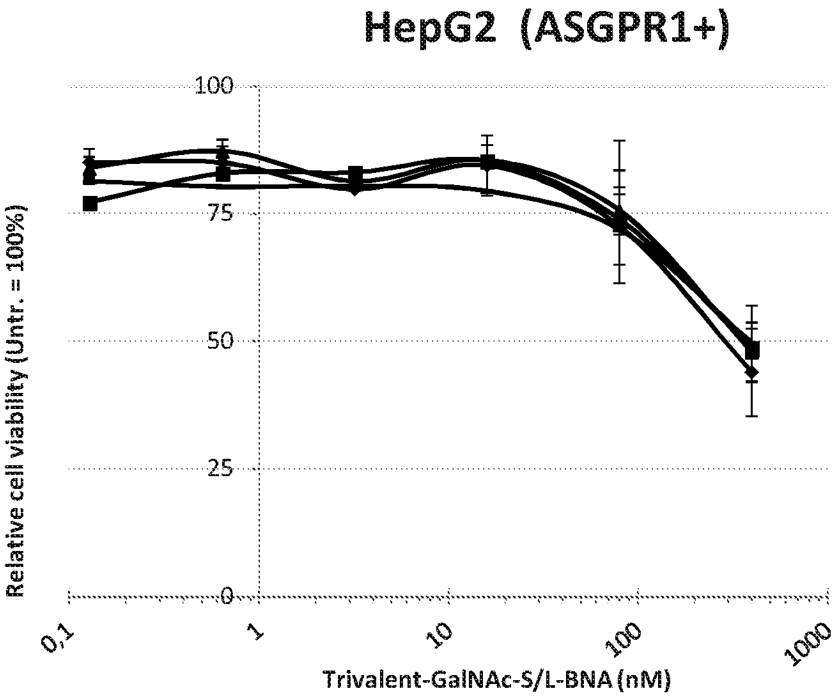


Fig. 16
D

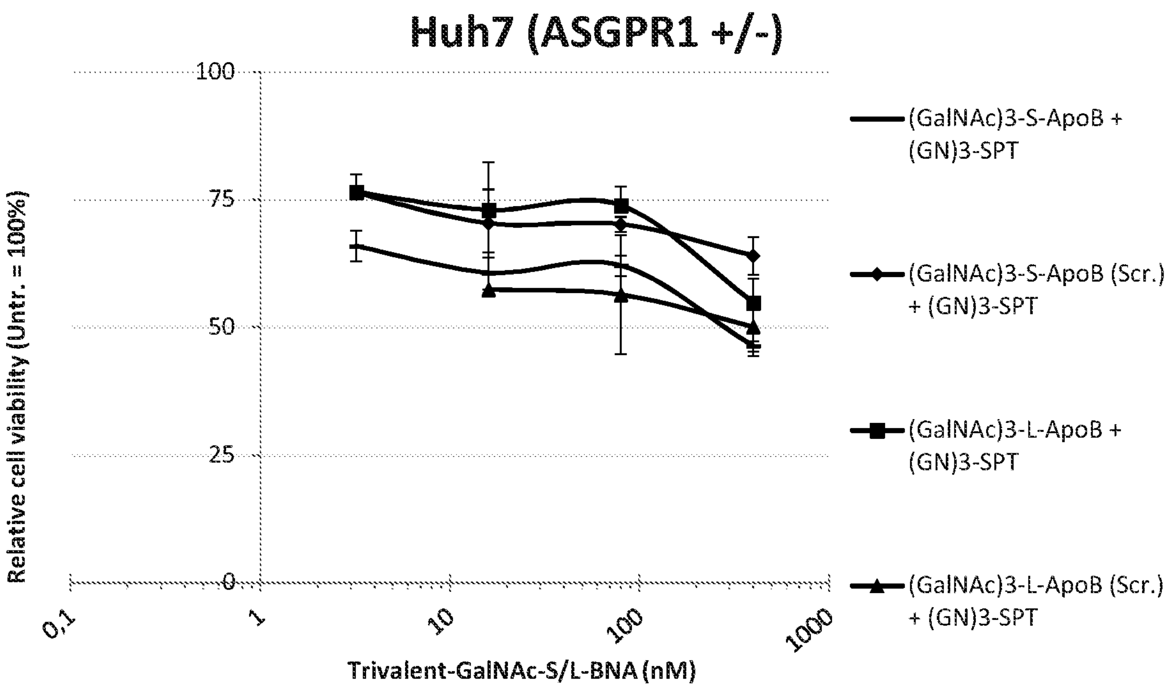

# Fig. 17

## A

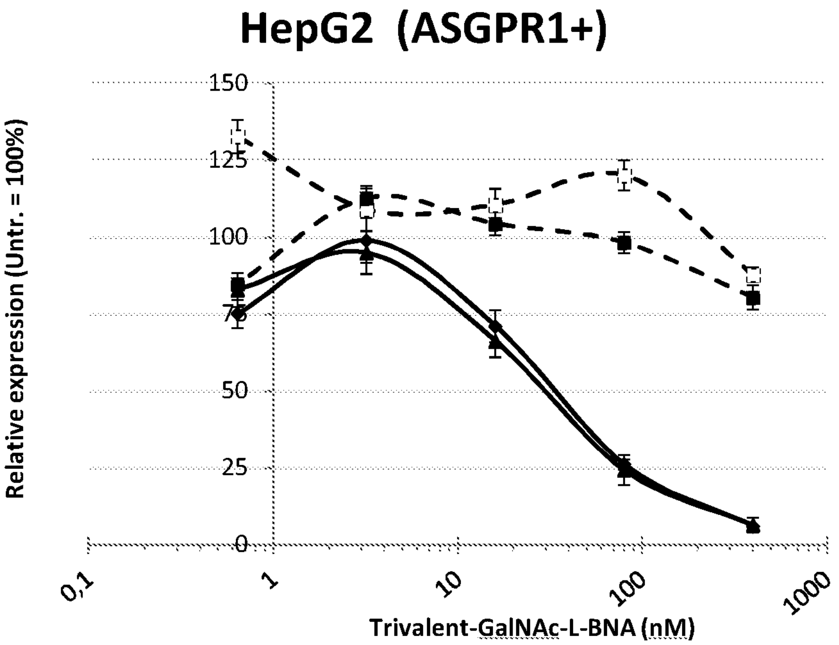
HepG2 (ASGPR1+)
Relative expression (Untr. = 100%)
150
125
100
75
50
25
0
0,1
1
10
100
1000
Trivalent-GalNAc-L-BNA (nM)

## B

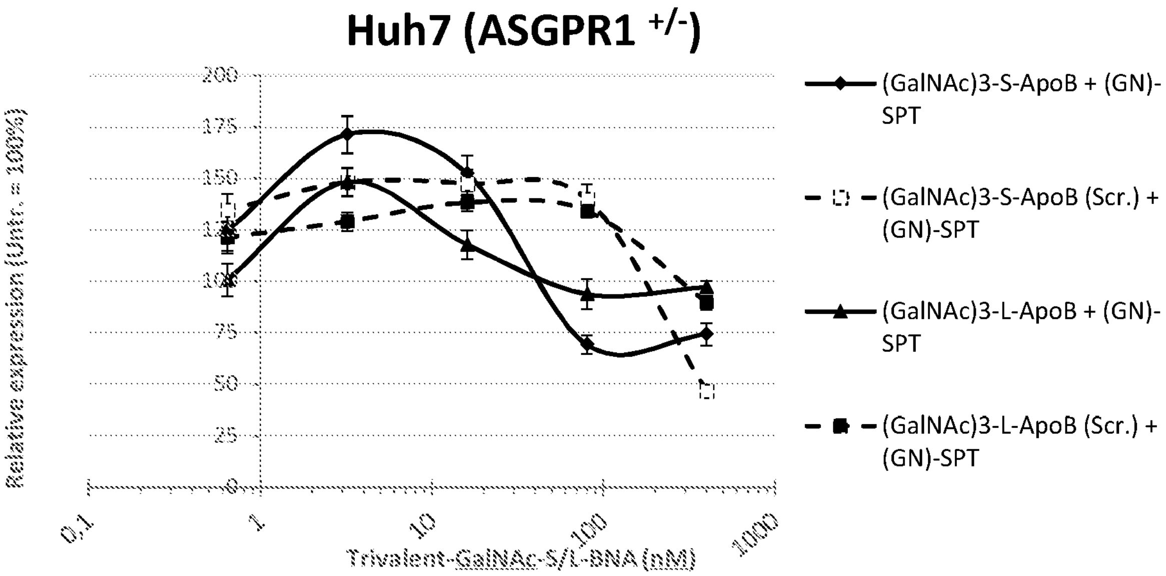
Huh7 (ASGPR1 +/-)
Relative expression (Untr. = 100%)
200
175
150
125
100
75
50
25
0
0,1
1
10
100
1000
Trivalent-GalNAc-S/L-BNA (nM)
(GalNAc)3-S-ApoB + (GN)-SPT
(GalNAc)3-S-ApoB (Scr.) + (GN)-SPT
(GalNAc)3-L-ApoB + (GN)-SPT
(GalNAc)3-L-ApoB (Scr.) + (GN)-SPT

FIG. 18
A

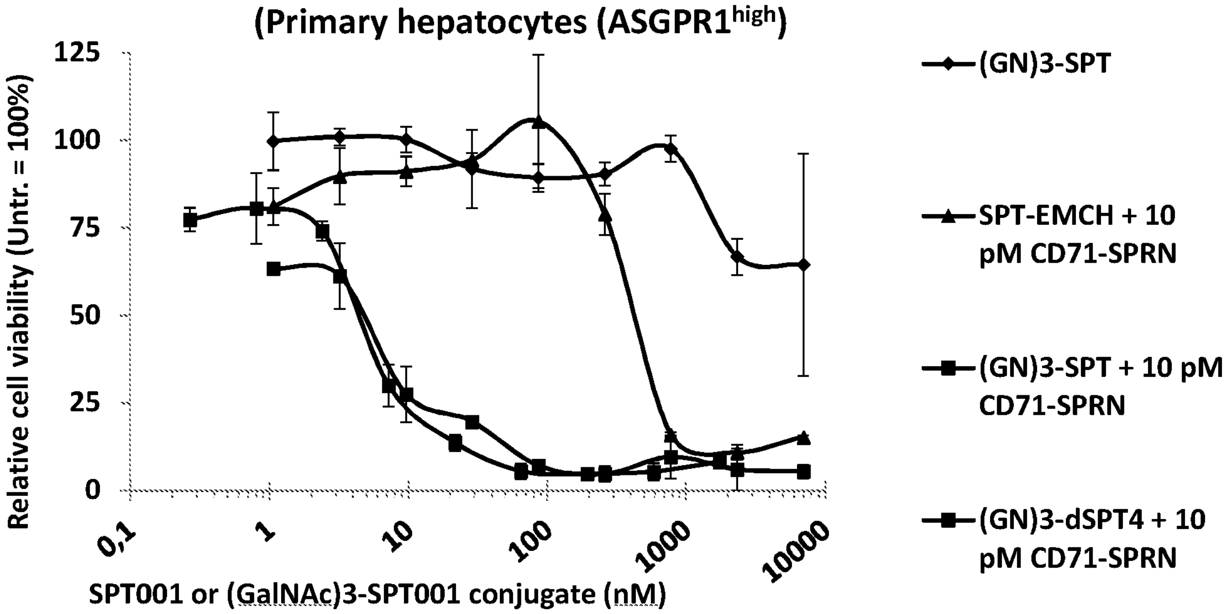
(Primary hepatocytes (ASGPR1high)
125
100
75
50
25
0
Relative cell viability (Untr. = 100%)
0,1
1
10
100
1000
10000
SPT001 or (GalNAc)3-SPT001 conjugate (nM)
(GN)3-SPT
SPT-EMCH + 10 pM CD71-SPRN
(GN)3-SPT + 10 pM CD71-SPRN
(GN)3-dSPT4 + 10 pM CD71-SPRN

B

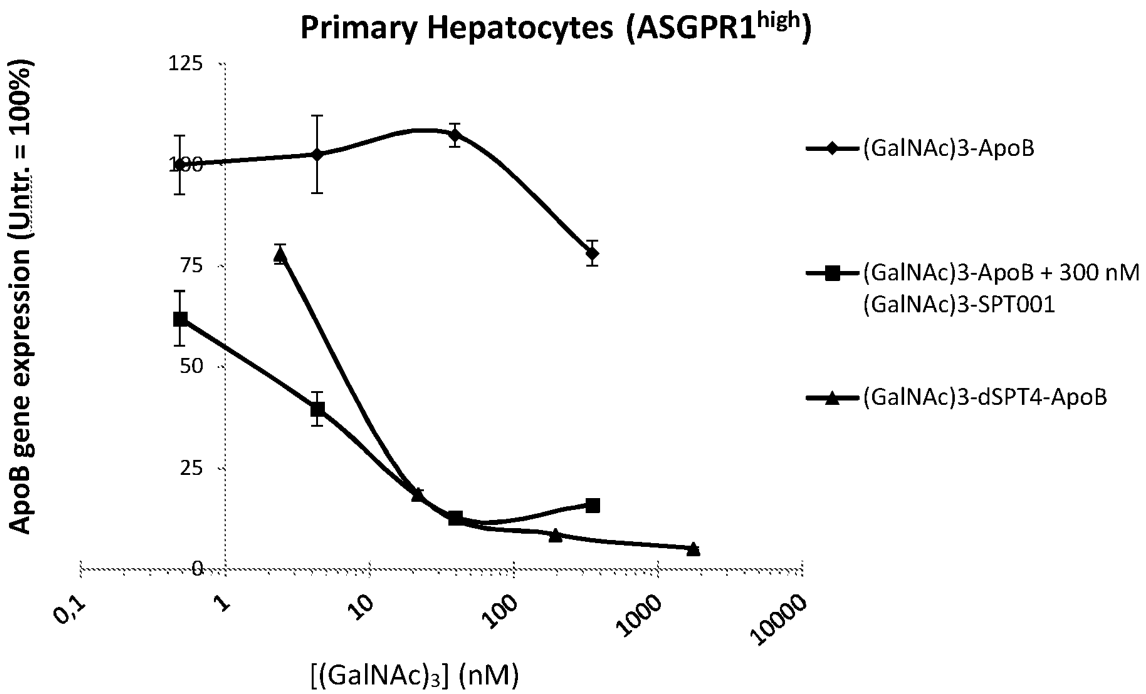
Primary Hepatocytes (ASGPR1high)
125
100
75
50
25
0
ApoB gene expression (Untr. = 100%)
0,1
1
10
100
1000
10000
[(GalNAc)3] (nM)
(GalNAc)3-ApoB
(GalNAc)3-ApoB + 300 nM (GalNAc)3-SPT001
(GalNAc)3-dSPT4-ApoB

FIG. 19

A

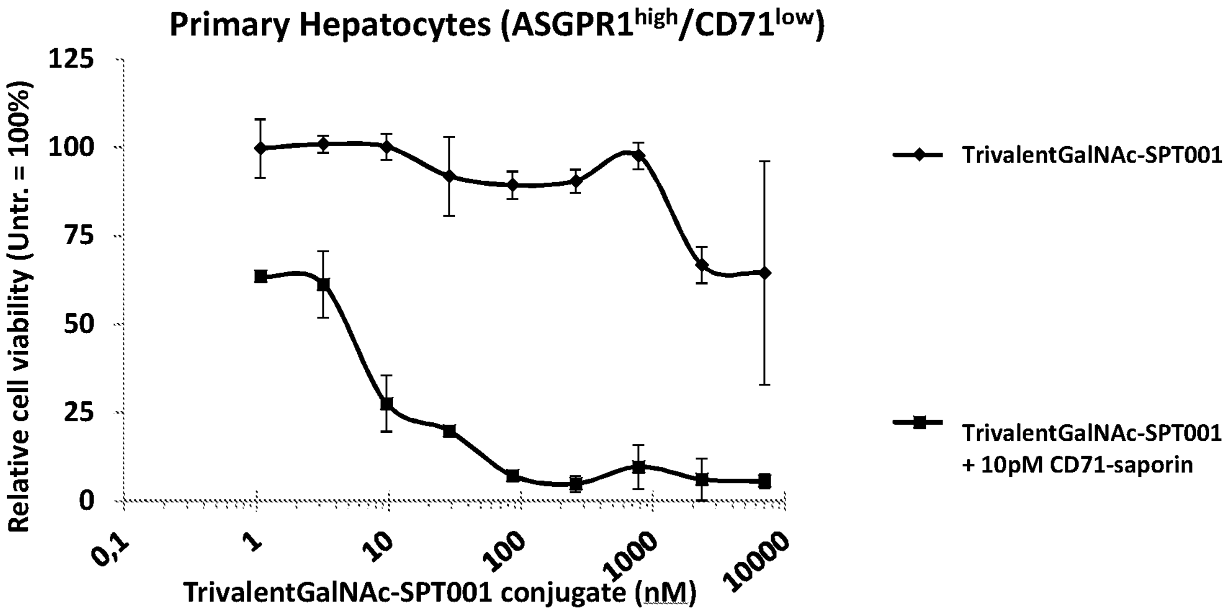
Primary Hepatocytes (ASGPR1high/CD71low)
Relative cell viability (Untr. = 100%)
125
100
75
50
25
0
0,1
1
10
100
1000
10000
TrivalentGalNAc-SPT001 conjugate (nM)
TrivalentGalNAc-SPT001
TrivalentGalNAc-SPT001
+ 10pM CD71-saporin

B

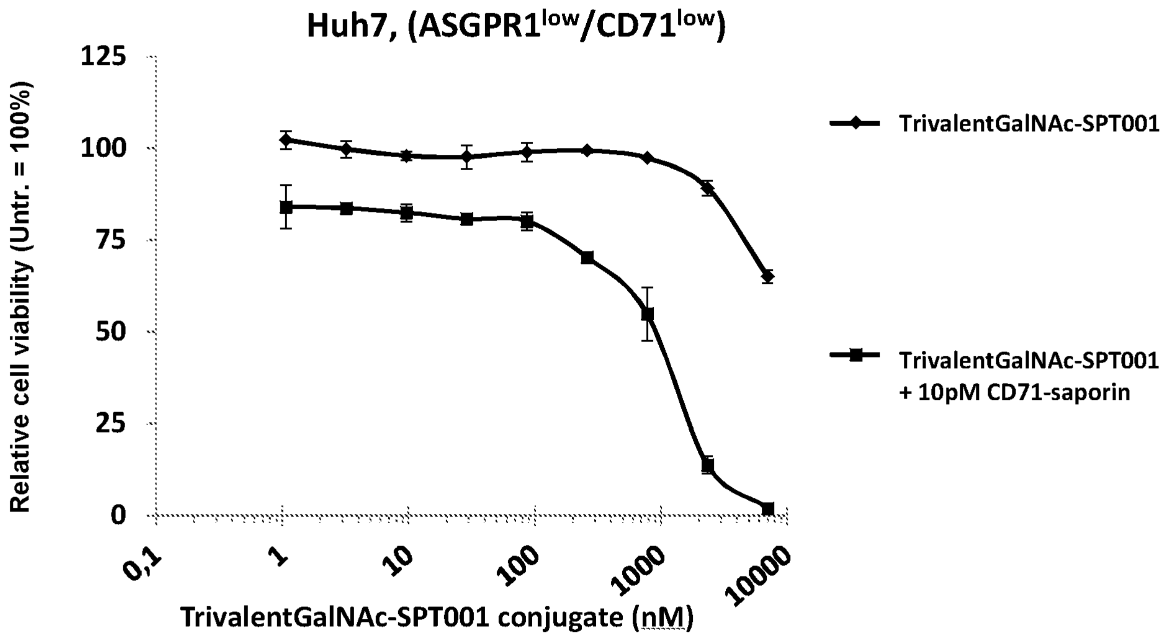
Huh7, (ASGPR1low/CD71low)
Relative cell viability (Untr. = 100%)
125
100
75
50
25
0
0,1
1
10
100
1000
10000
TrivalentGalNAc-SPT001 conjugate (nM)
TrivalentGalNAc-SPT001
TrivalentGalNAc-SPT001
+ 10pM CD71-saporin

FIG. 19
C

Trivalent GalNAc-SPT001 conjugate

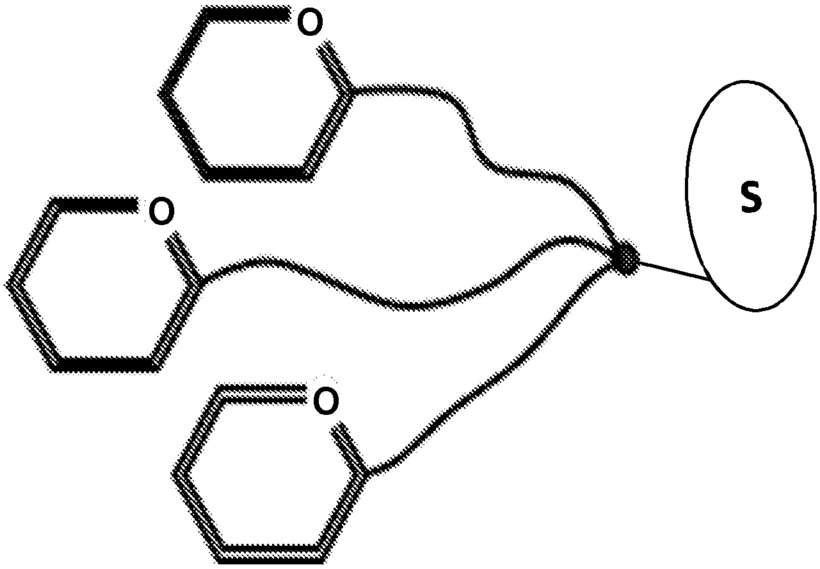

D

Anti-CD71 antibody – saporin conjugate

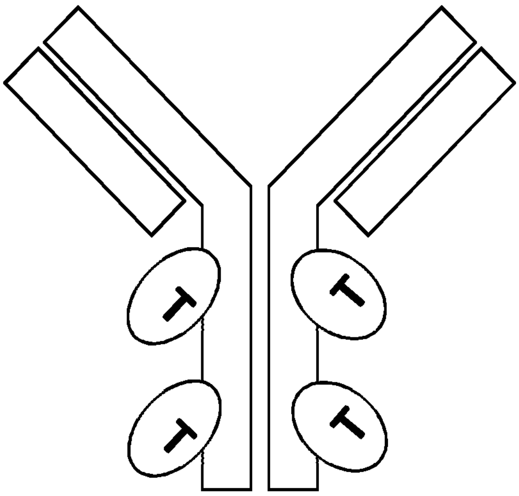

# Fig. 19

## E

(GalNAc)3-dSPT4 conjugate

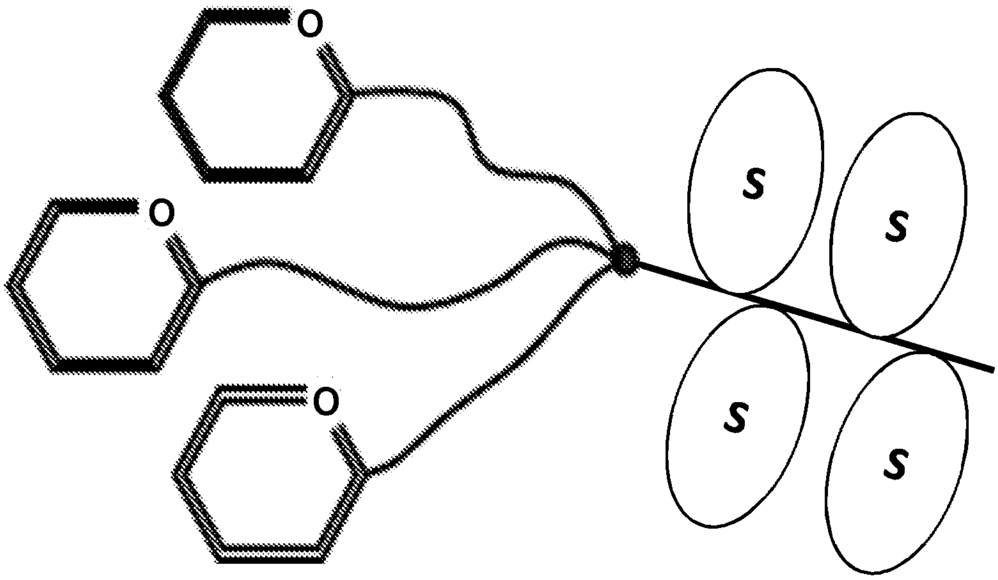

# FIG. 20

## A

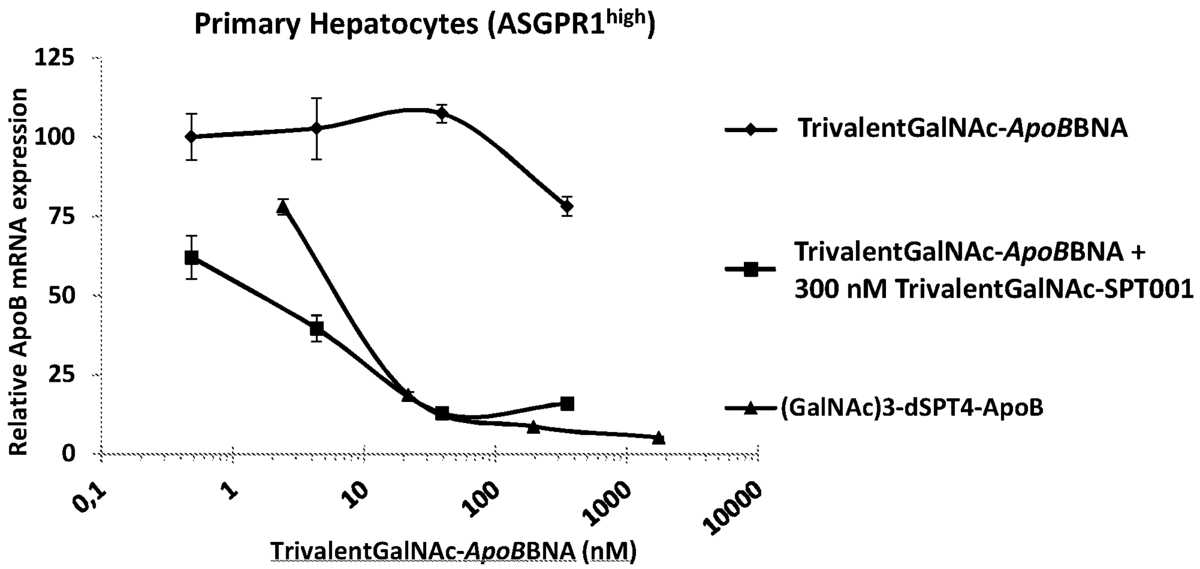
Primary Hepatocytes (ASGPR1high)
Relative ApoB mRNA expression
125
100
75
50
25
0
0,1
1
10
100
1000
10000
TrivalentGalNAc-ApoBBNA (nM)
TrivalentGalNAc-ApoBBNA
TrivalentGalNAc-ApoBBNA + 300 nM TrivalentGalNAc-SPT001
(GalNAc)3-dSPT4-ApoB

## B

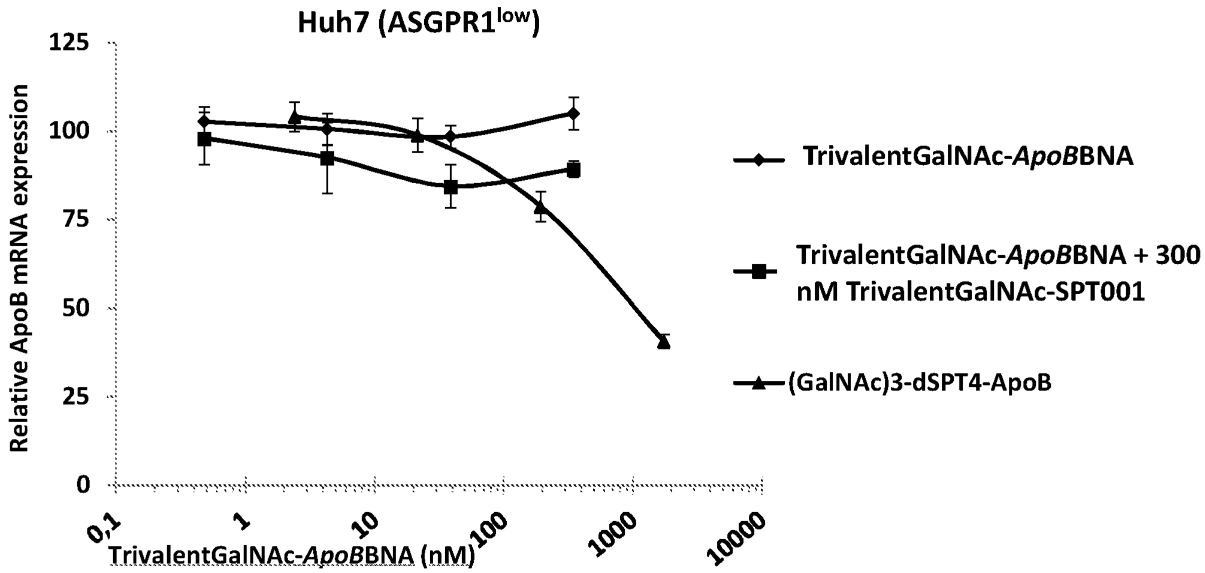
Huh7 (ASGPR1low)
Relative ApoB mRNA expression
125
100
75
50
25
0
0,1
1
10
100
1000
10000
TrivalentGalNAc-ApoBBNA (nM)
TrivalentGalNAc-ApoBBNA
TrivalentGalNAc-ApoBBNA + 300 nM TrivalentGalNAc-SPT001
(GalNAc)3-dSPT4-ApoB

FIG. 20
C

**Trivalent GalNAc-*ApoB*BNA conjugate**

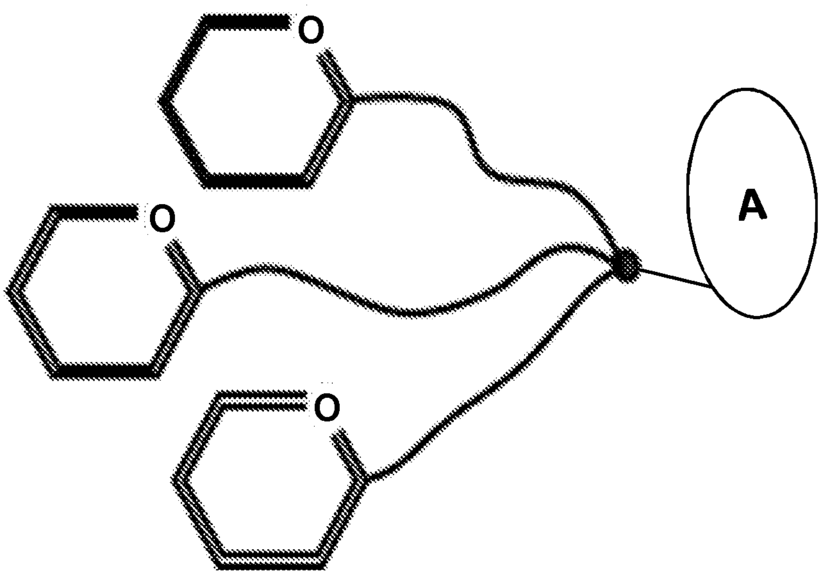


D

**Trivalent GalNAc-*ApoB*BNA-SPT001 conjugate**

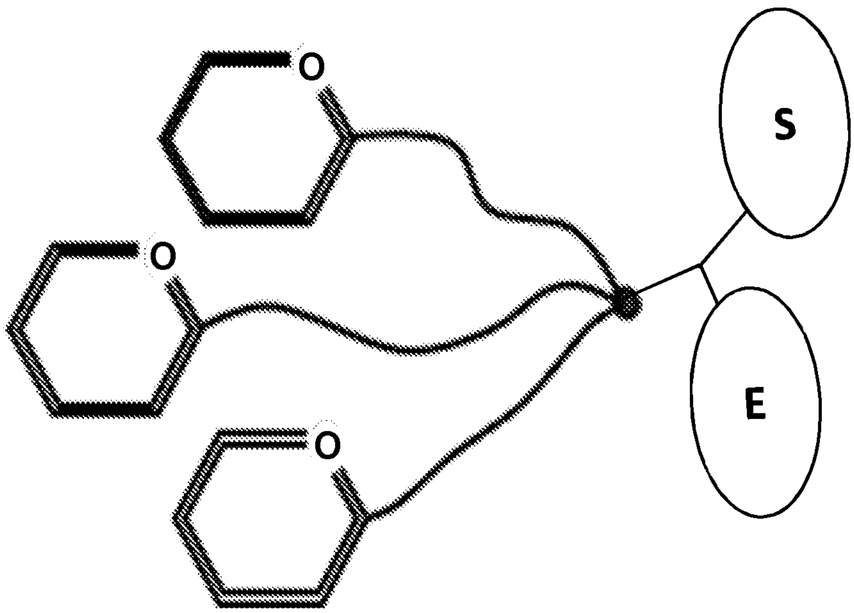

Fig. 20
E

(GalNAc)$_3$-dSPT4-*ApoB* conjugate

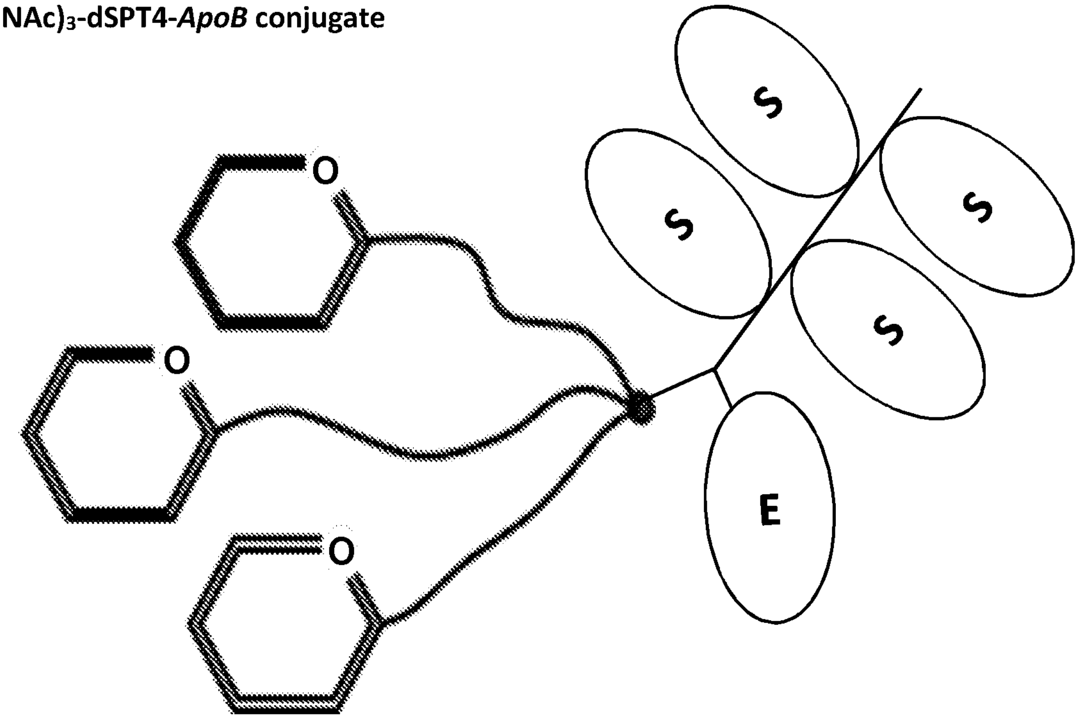

# FIG. 21

A

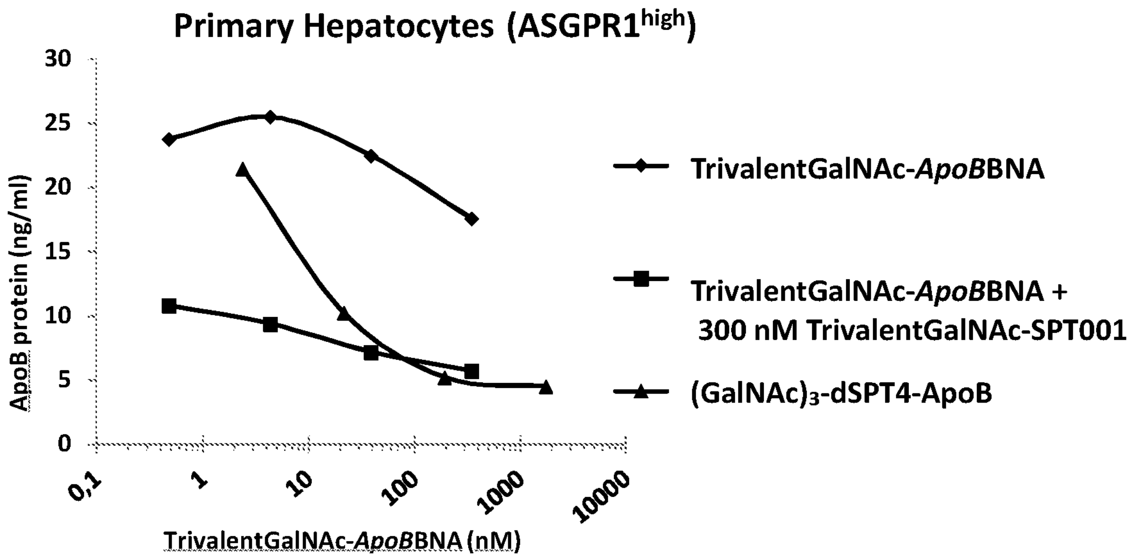

Primary Hepatocytes (ASGPR1high)
ApoB protein (ng/ml)
30
25
20
15
10
5
0
0,1
1
10
100
1000
10000
TrivalentGalNAc-ApoBBNA (nM)
TrivalentGalNAc-ApoBBNA
TrivalentGalNAc-ApoBBNA +
300 nM TrivalentGalNAc-SPT001
(GalNAc)3-dSPT4-ApoB


B

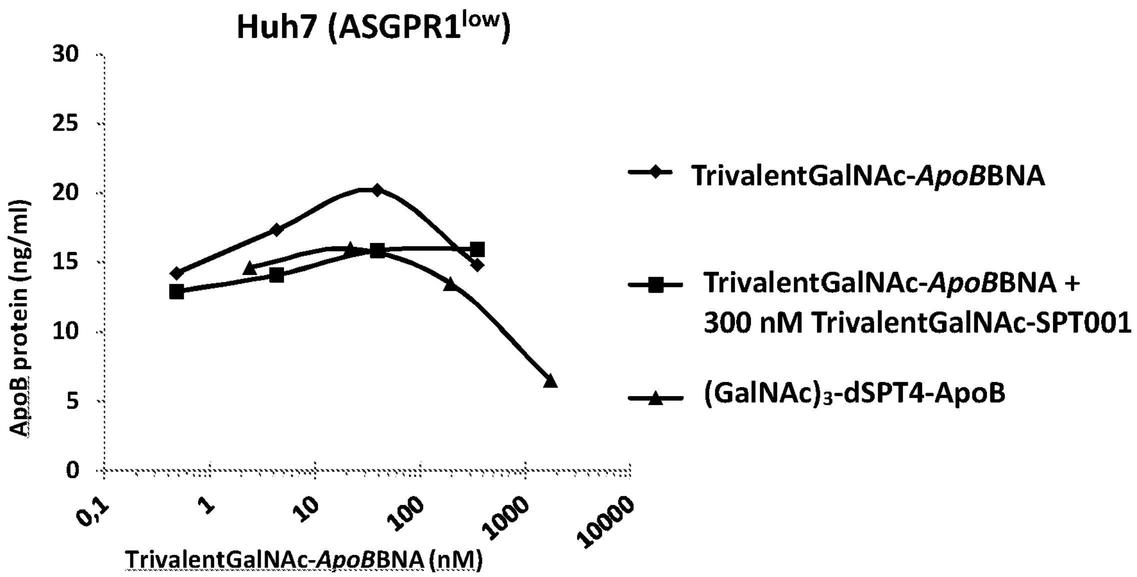

Huh7 (ASGPR1low)
ApoB protein (ng/ml)
30
25
20
15
10
5
0
0,1
1
10
100
1000
10000
TrivalentGalNAc-ApoBBNA (nM)
TrivalentGalNAc-ApoBBNA
TrivalentGalNAc-ApoBBNA +
300 nM TrivalentGalNAc-SPT001
(GalNAc)3-dSPT4-ApoB

Fig. 22
A

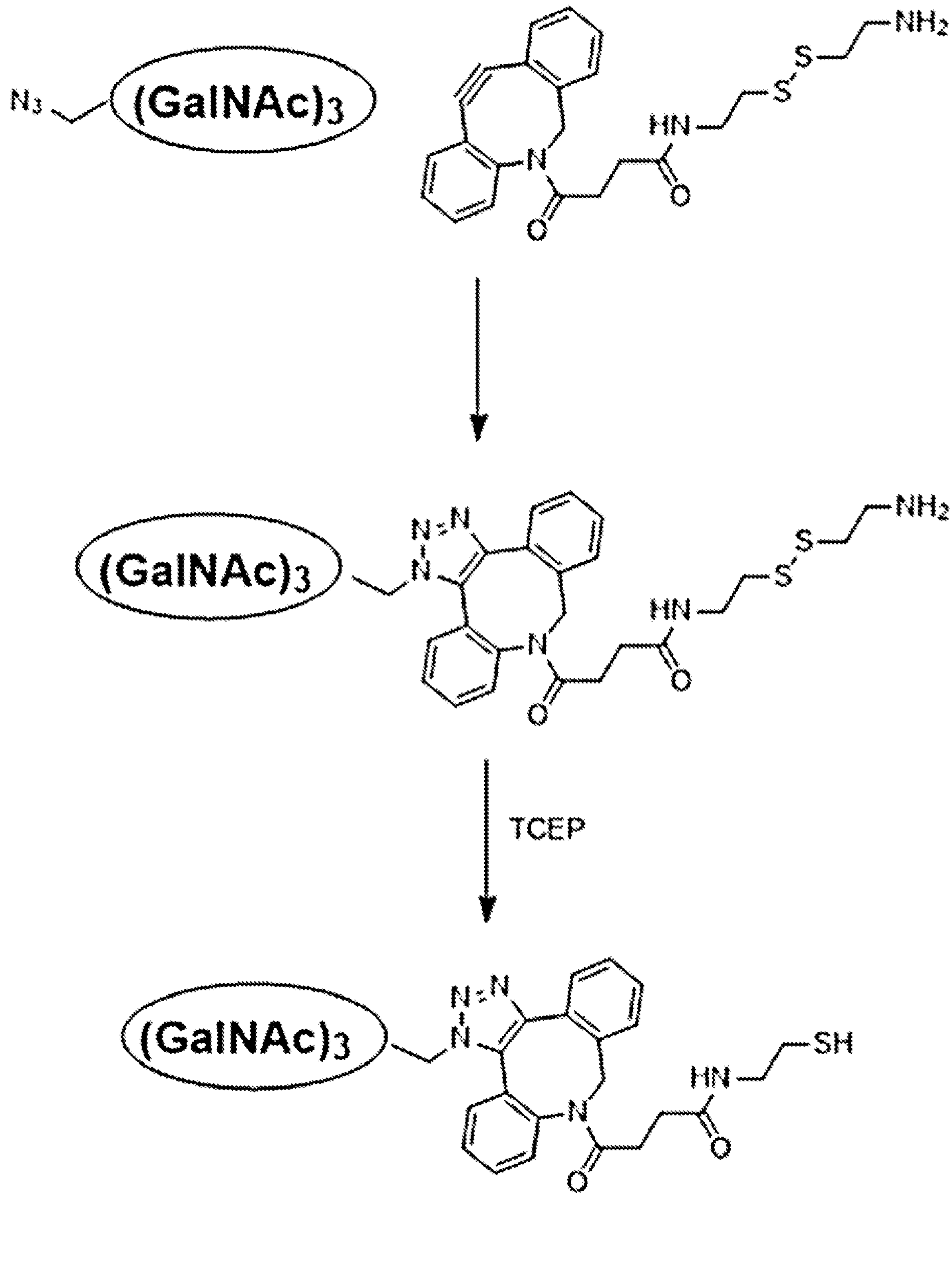

**Fig. 22**
**B**

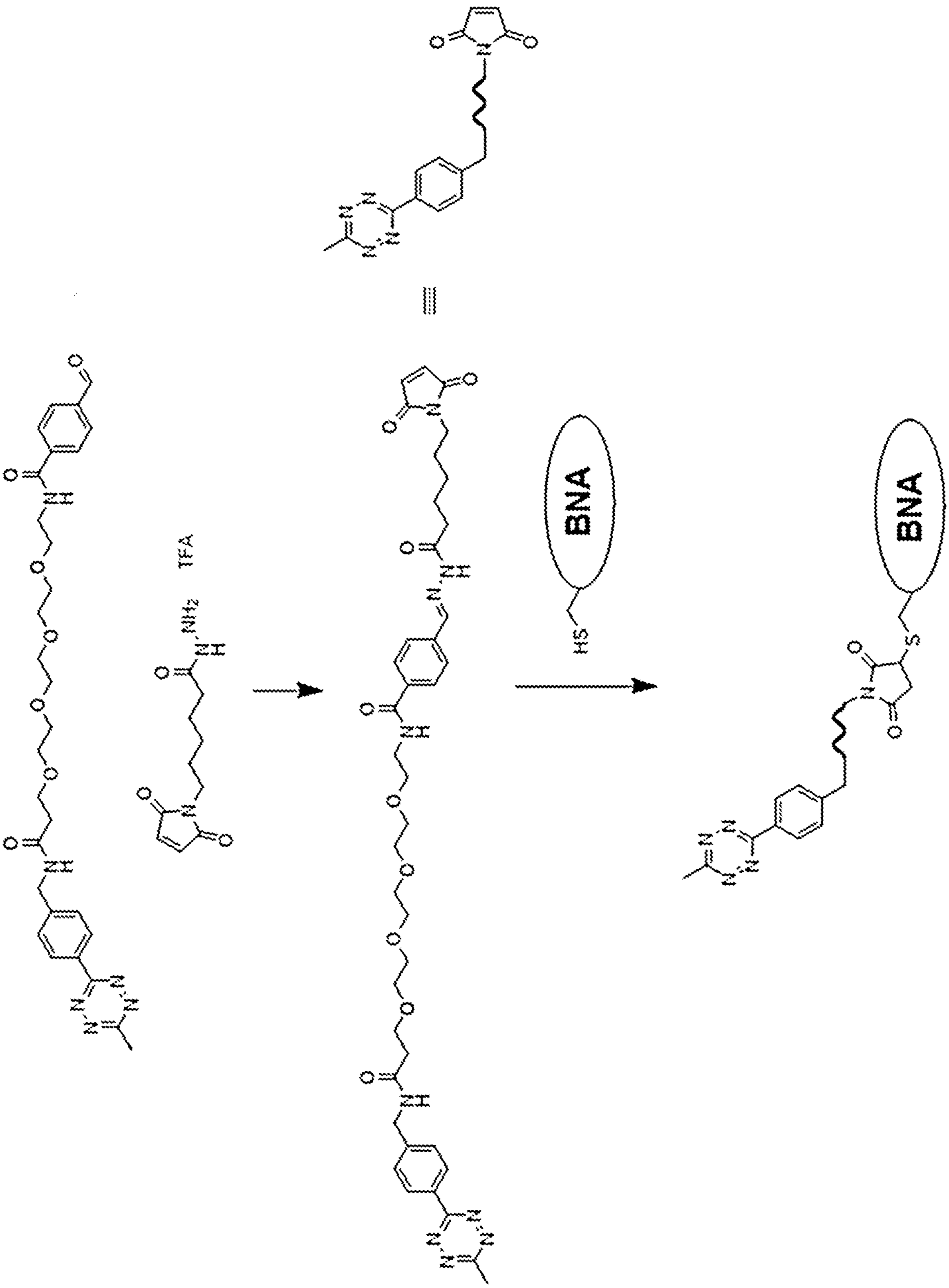

Fig. 22
C

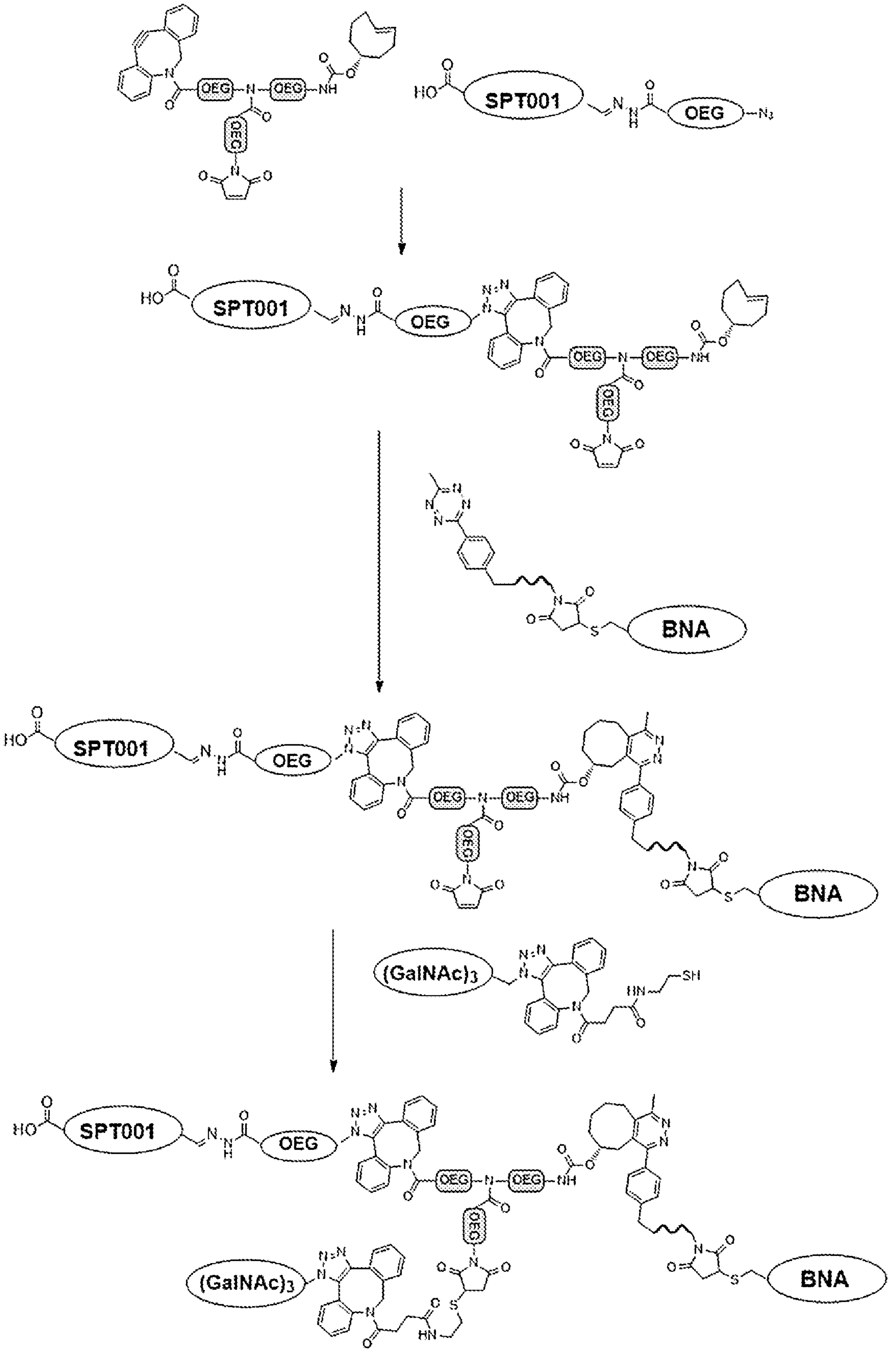

# FIG. 23

## A

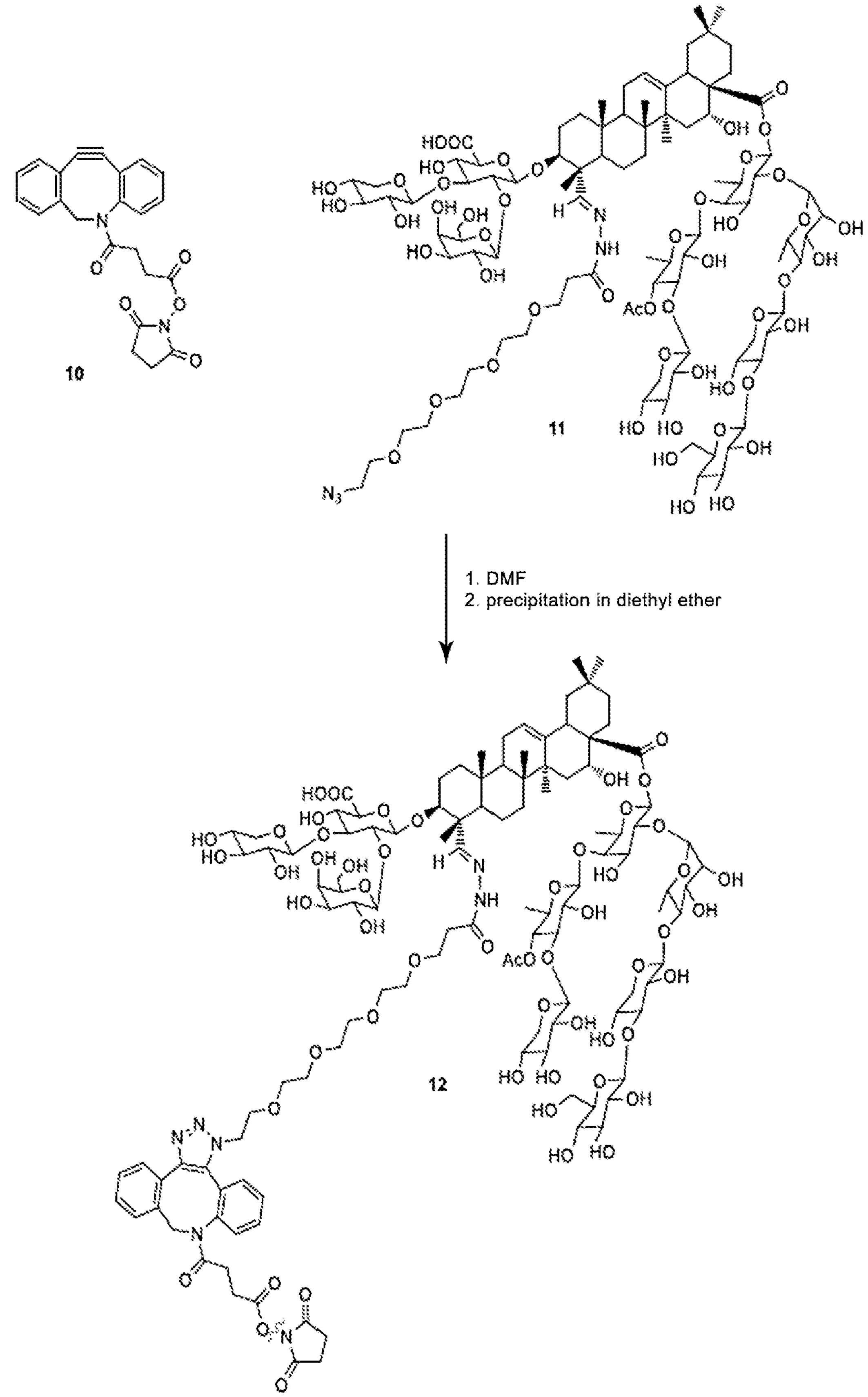


10

11

12

# FIG. 23

## B

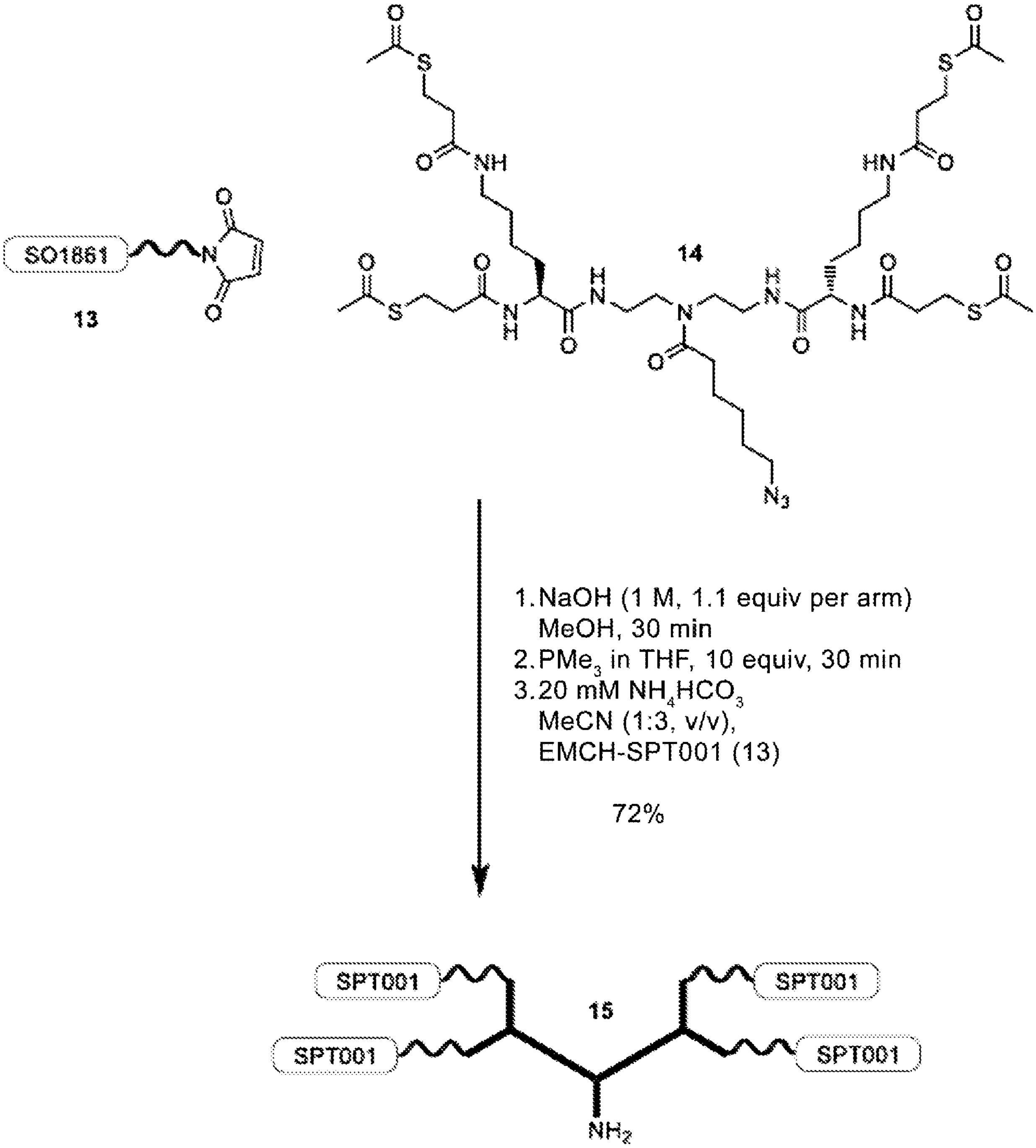


13

14

15

MW = 9110

# FIG. 23

## C

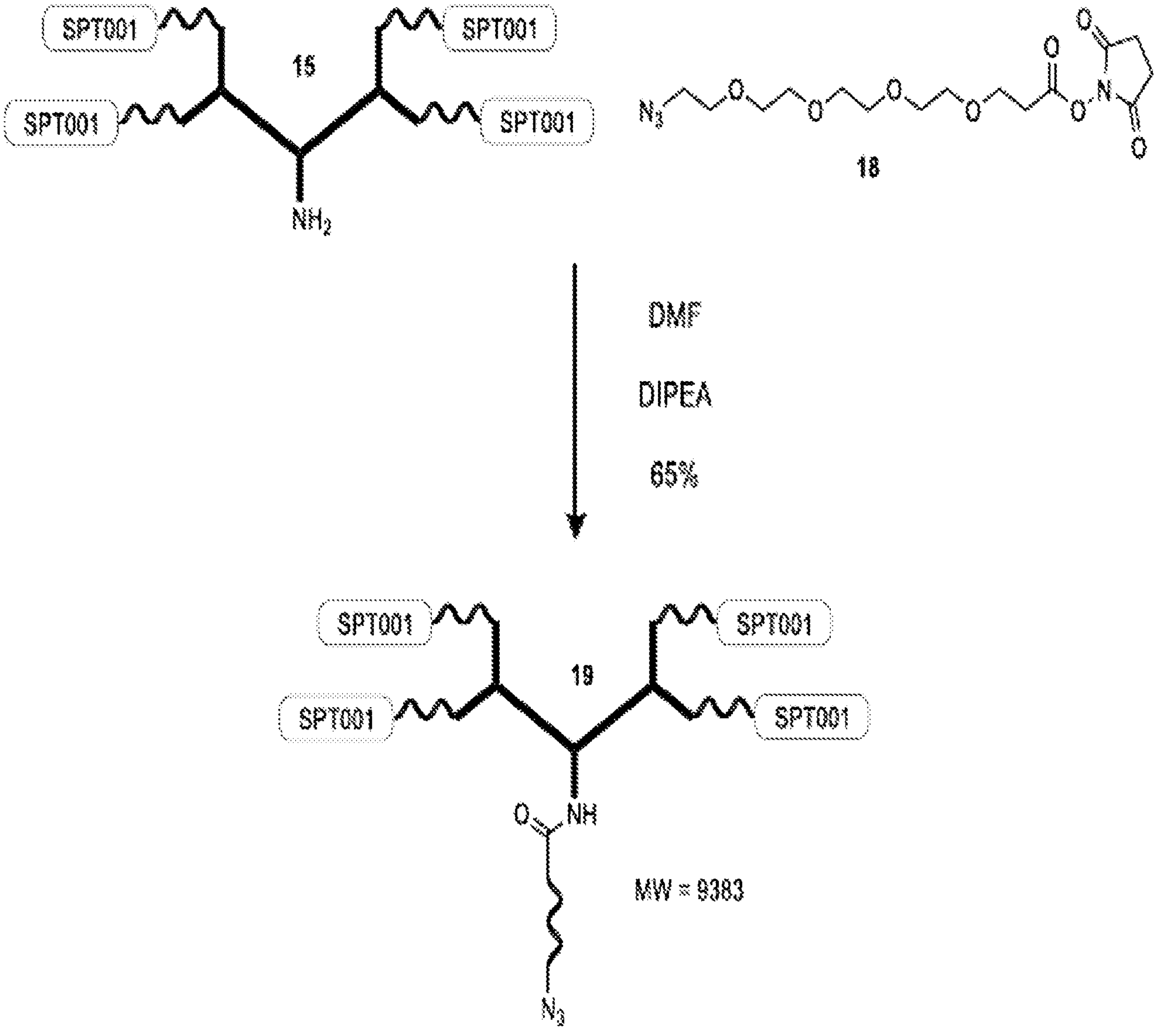

# FIG. 23

## D

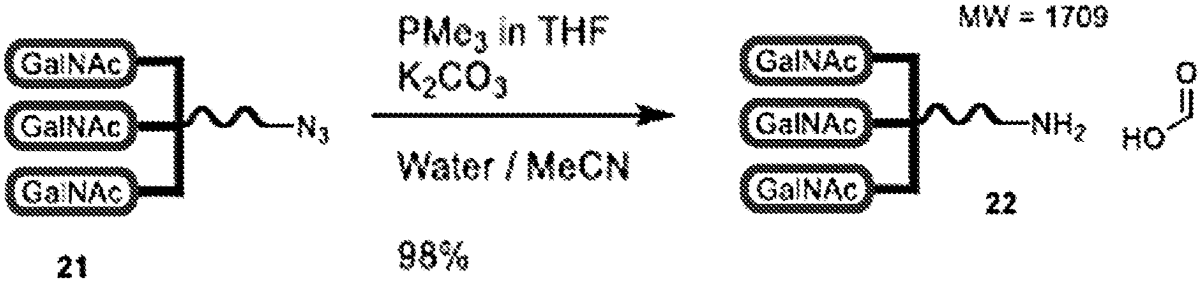


21

22

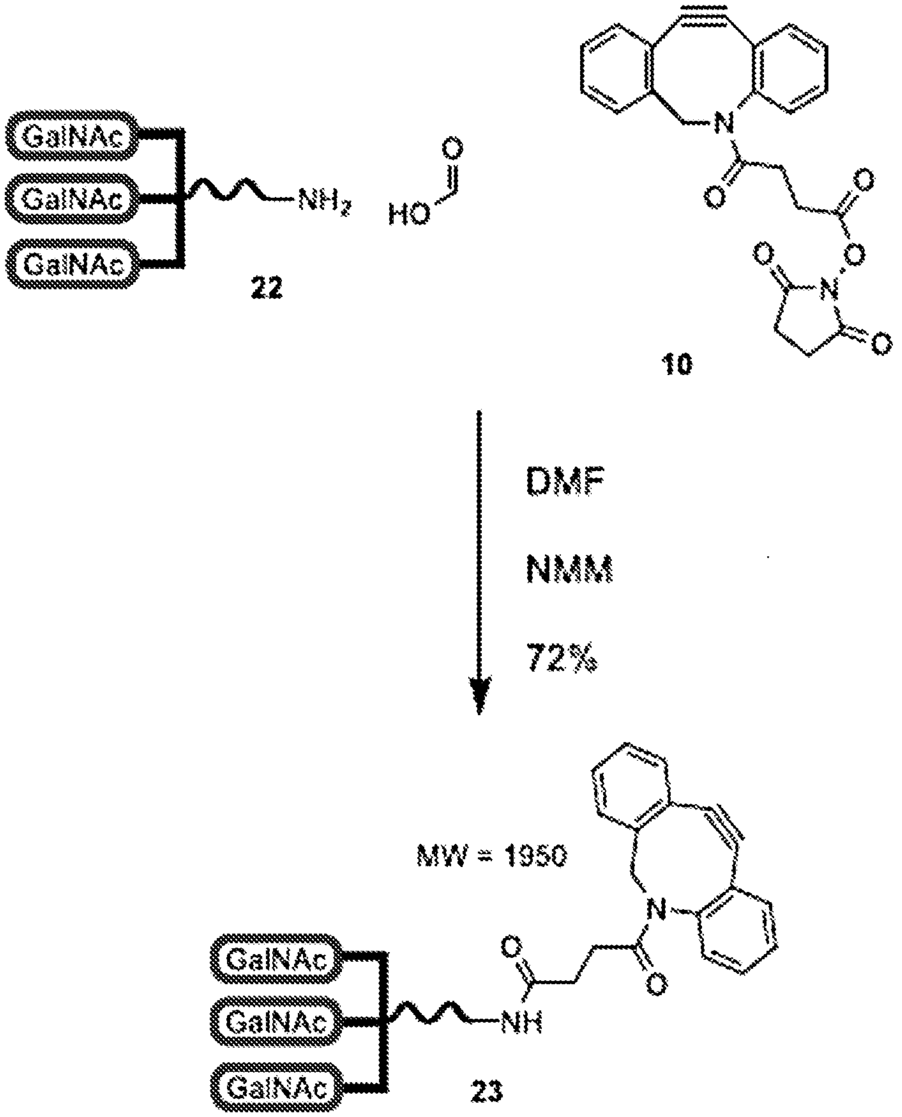


22

10

23

## FIG. 23

## E

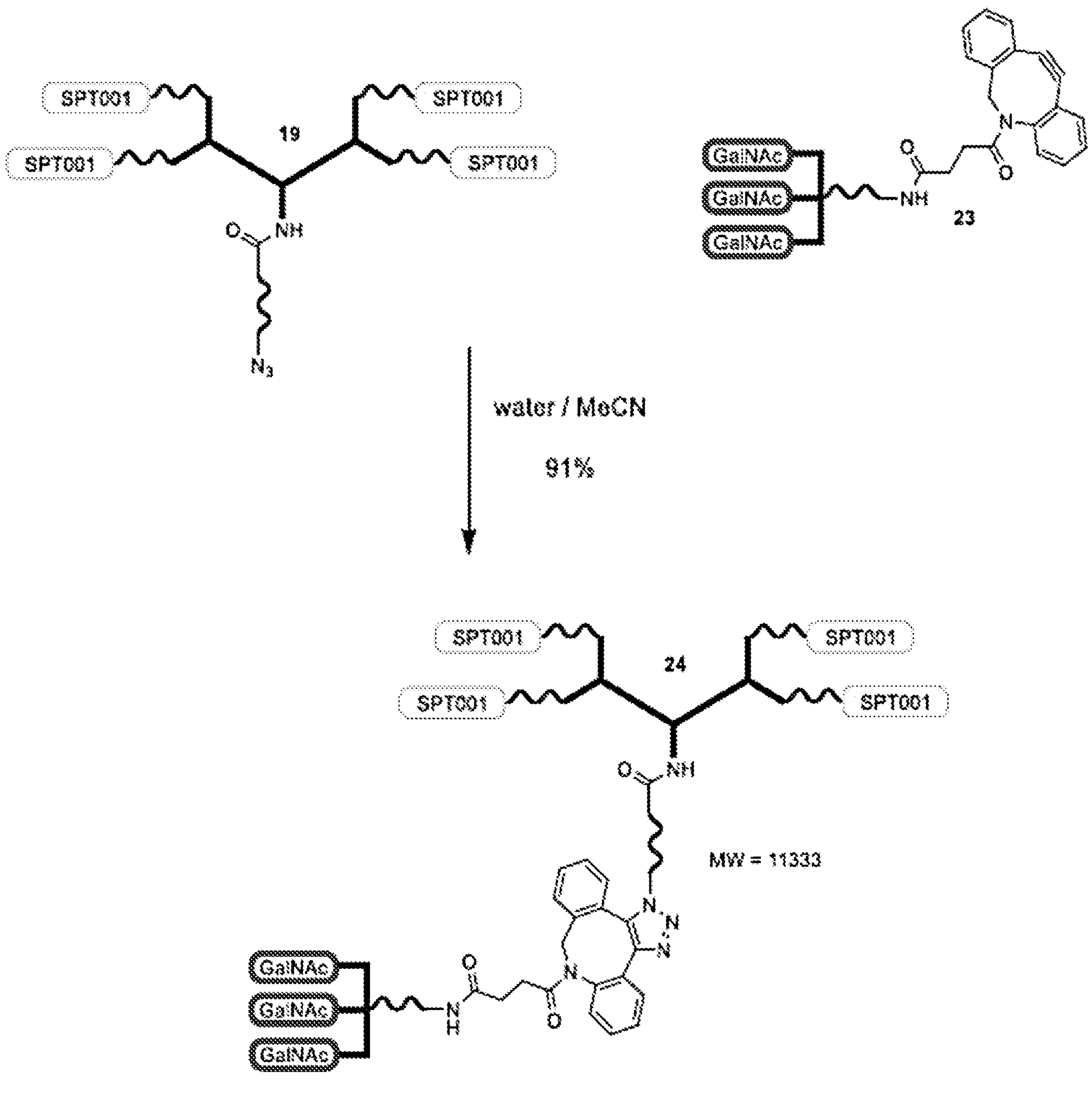

FIG. 24

A

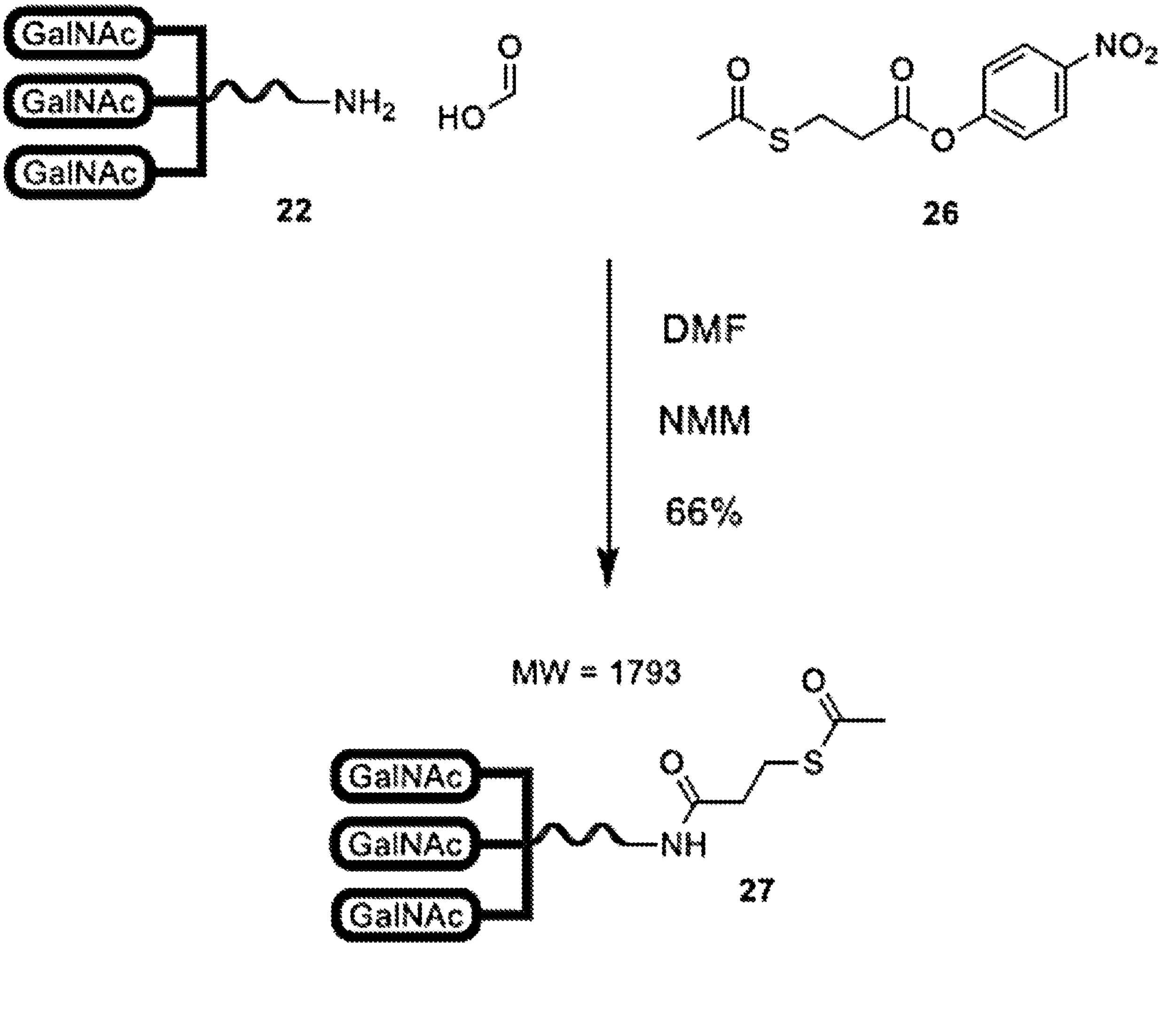


22

26

27

# FIG. 24

## B

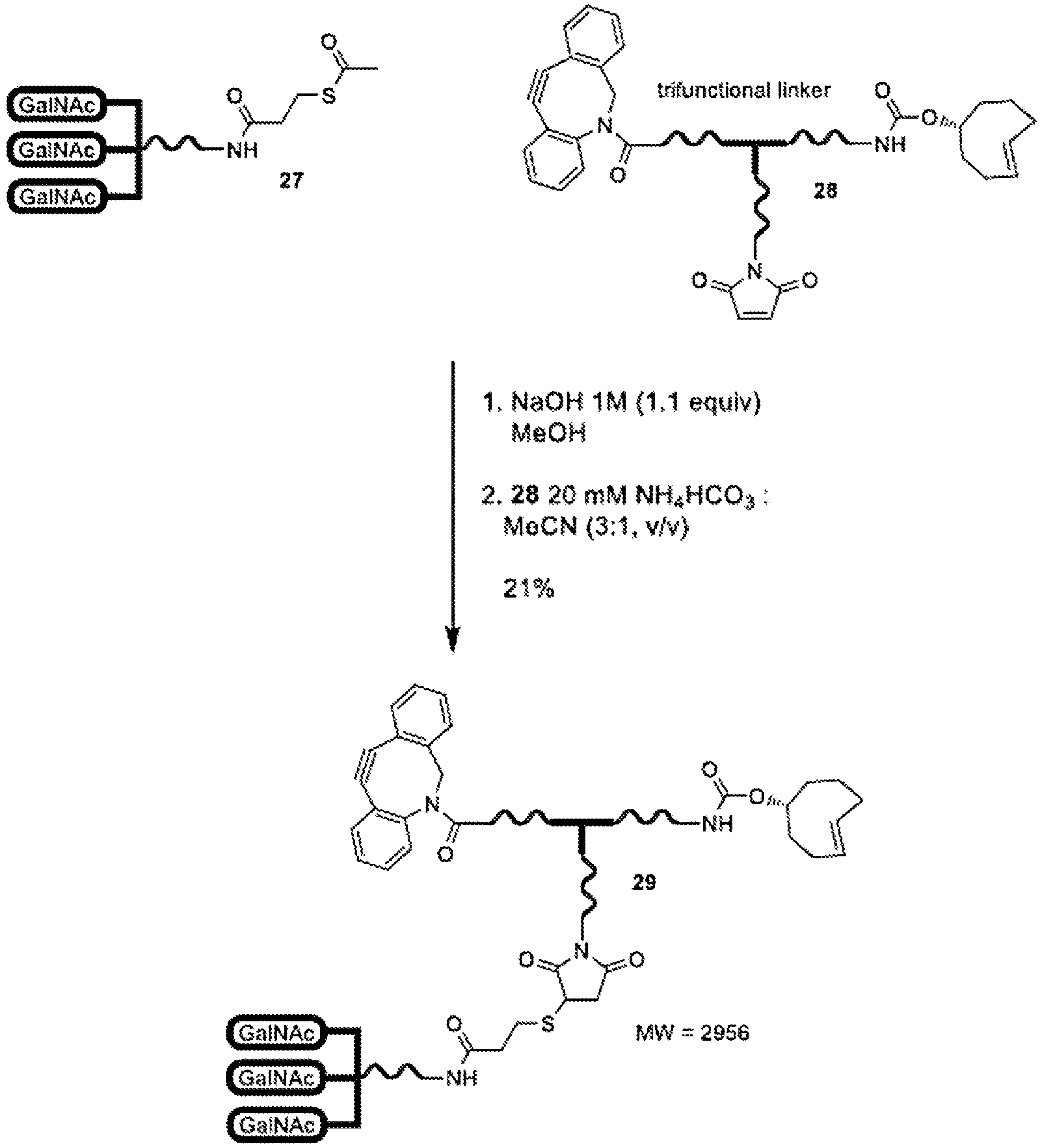
27
trifunctional linker
28
1. NaOH 1M (1.1 equiv)
MeOH
2. 28 20 mM $NH_4HCO_3$ :
MeCN (3:1, v/v)
21%
29
MW = 2956

# FIG. 24
# C

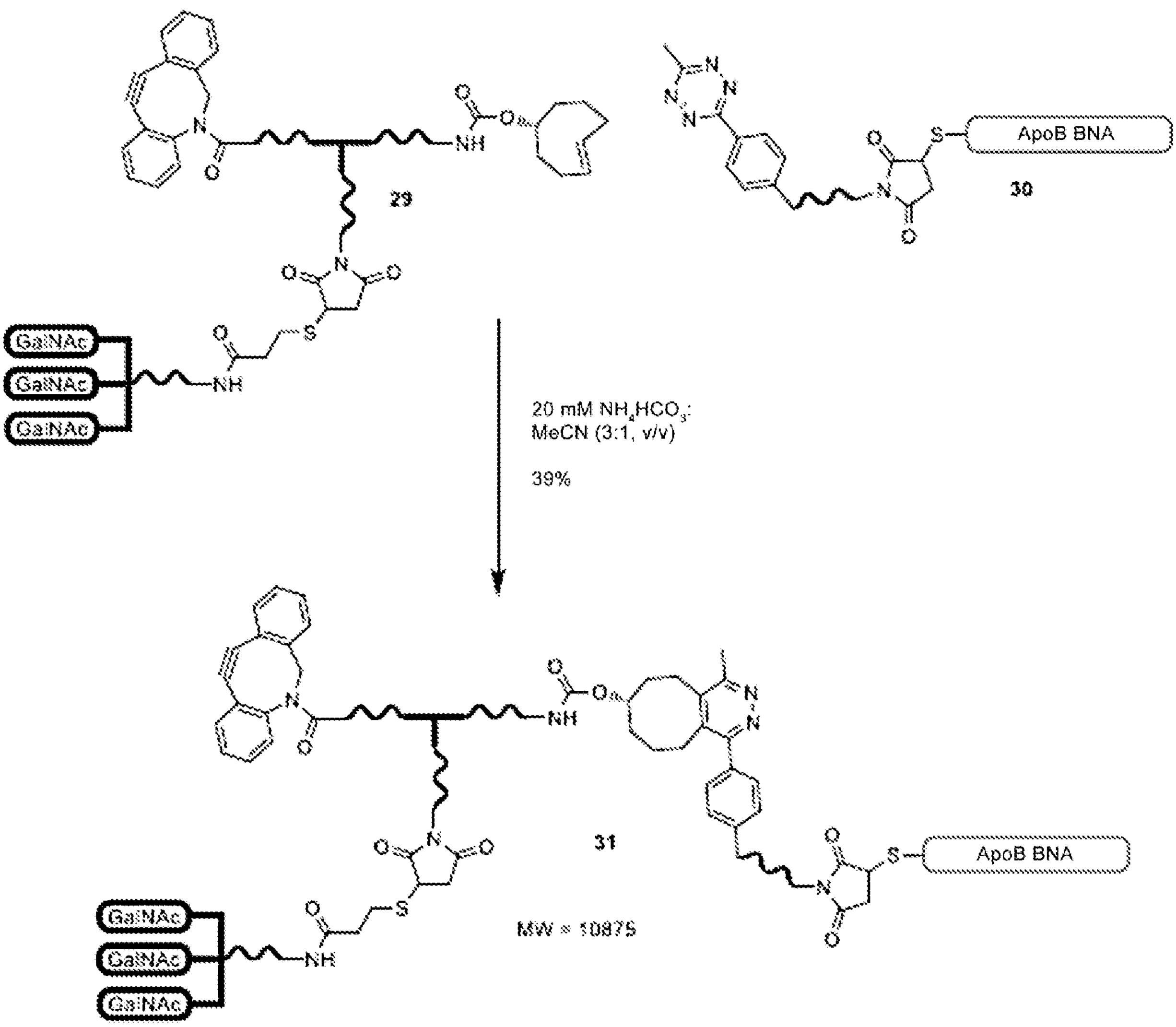


29

30

31

# FIG. 24
# D

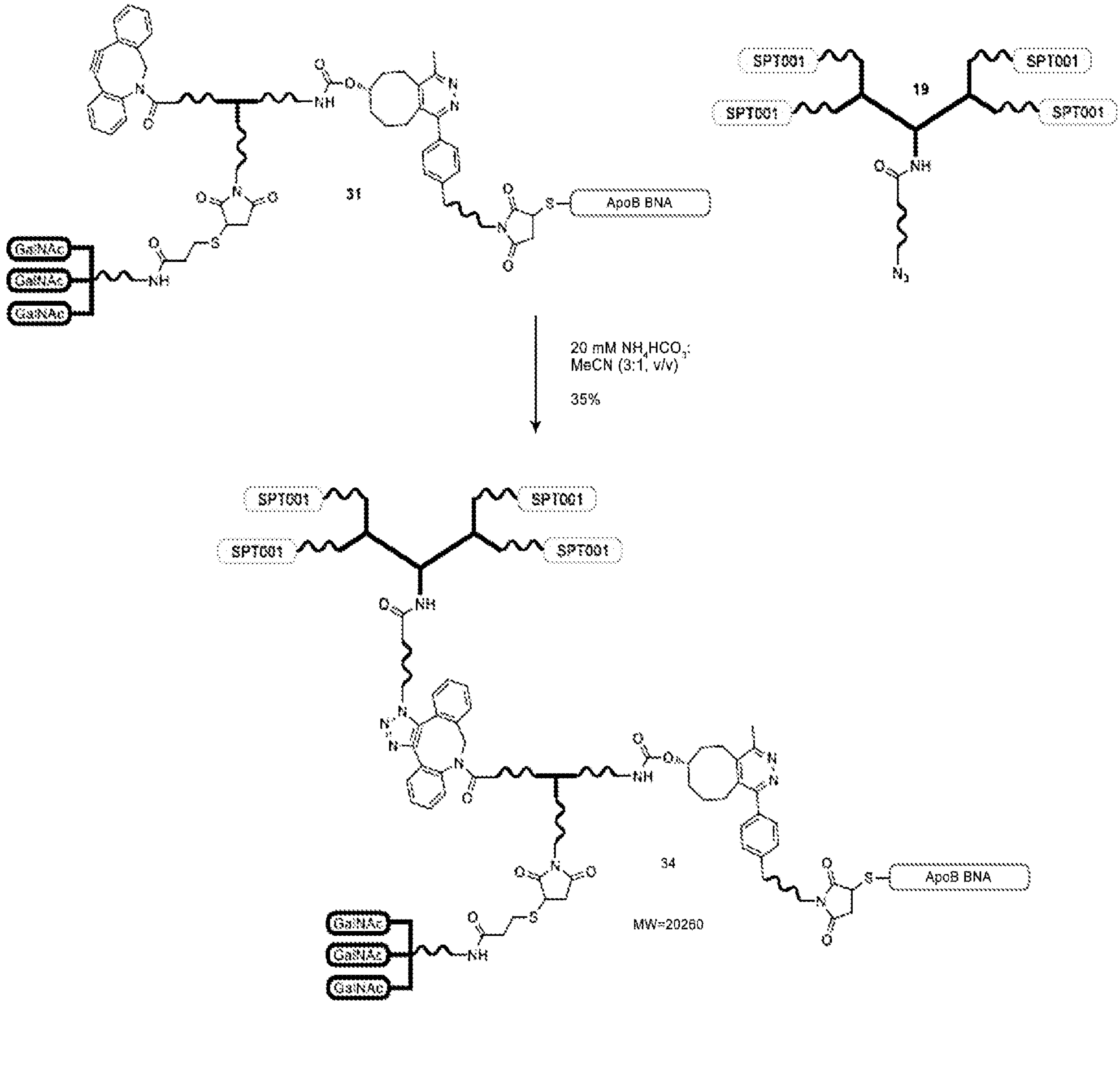


31

19

34

MW=20260

Fig. 25

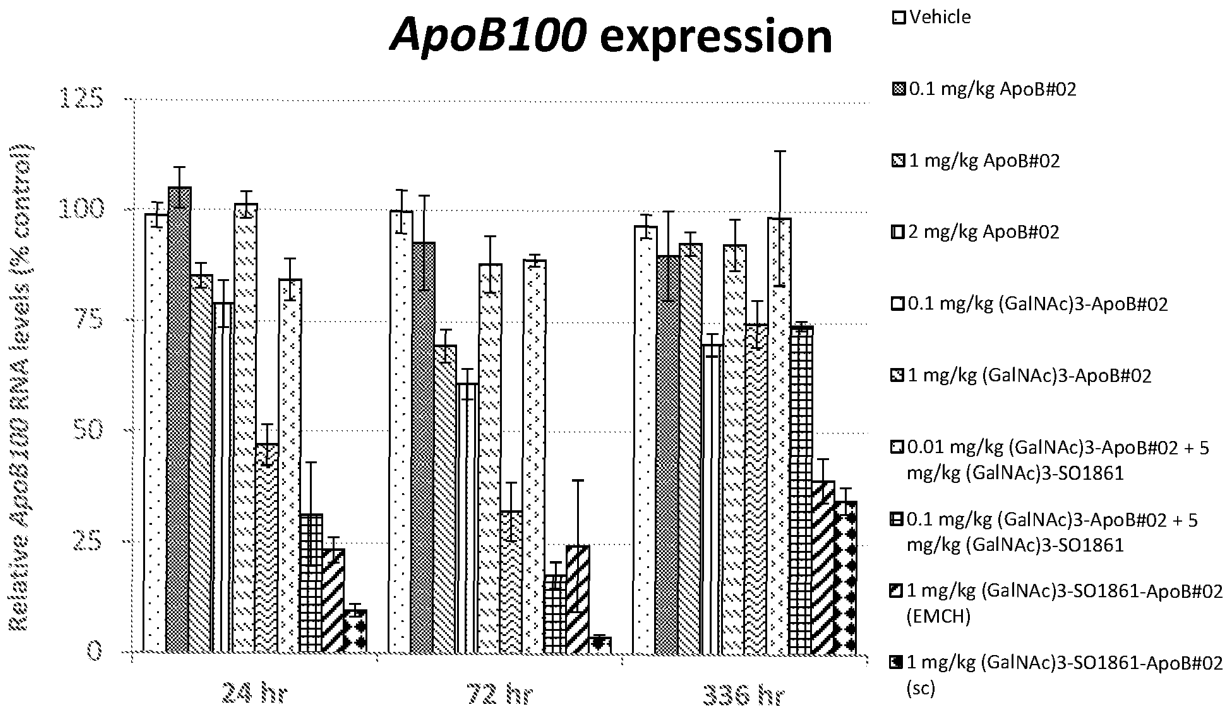
ApoB100 expression
Relative ApoB100 RNA levels (% control)
125
100
75
50
25
0
24 hr
72 hr
336 hr
Vehicle
0.1 mg/kg ApoB#02
1 mg/kg ApoB#02
2 mg/kg ApoB#02
0.1 mg/kg (GalNAc)3-ApoB#02
1 mg/kg (GalNAc)3-ApoB#02
0.01 mg/kg (GalNAc)3-ApoB#02 + 5 mg/kg (GalNAc)3-SO1861
0.1 mg/kg (GalNAc)3-ApoB#02 + 5 mg/kg (GalNAc)3-SO1861
1 mg/kg (GalNAc)3-SO1861-ApoB#02 (EMCH)
1 mg/kg (GalNAc)3-SO1861-ApoB#02 (sc)

**Fig. 26**

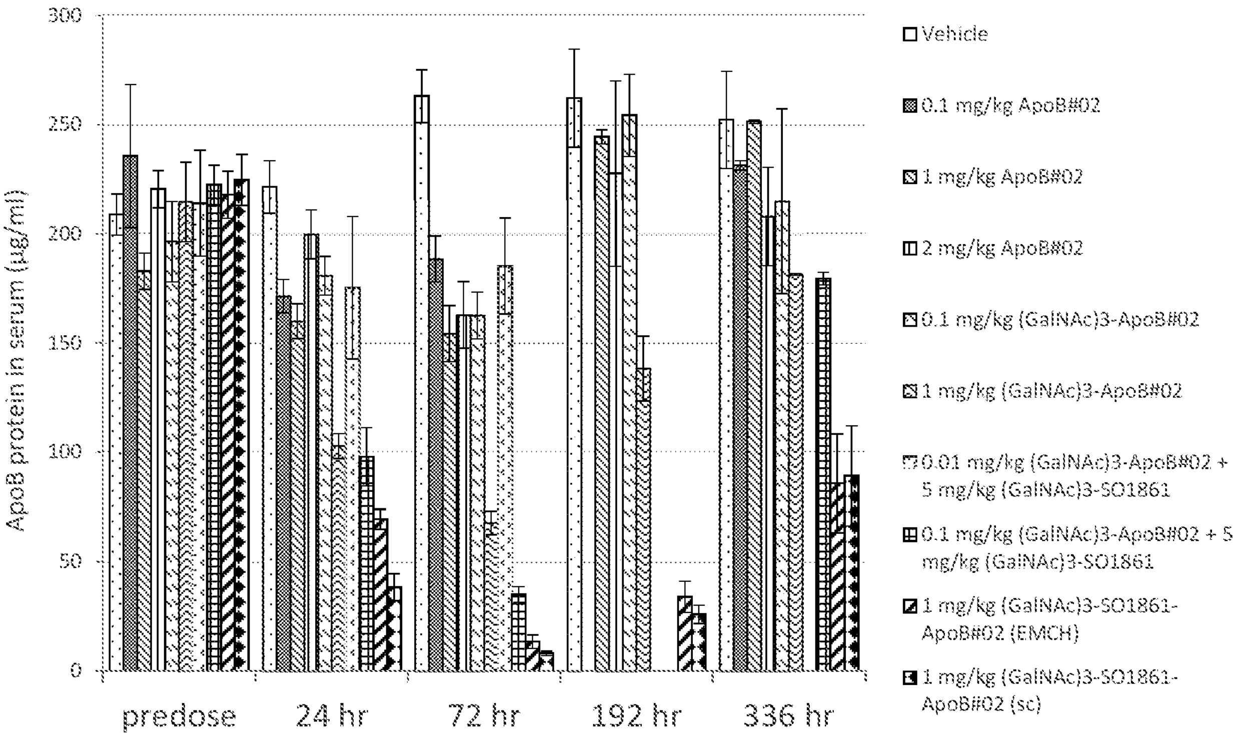

300
250
200
150
100
50
0
ApoB protein in serum (µg/ml)
predose
24 hr
72 hr
192 hr
336 hr
Vehicle
0.1 mg/kg ApoB#02
1 mg/kg ApoB#02
2 mg/kg ApoB#02
0.1 mg/kg (GalNAc)3-ApoB#02
1 mg/kg (GalNAc)3-ApoB#02
0.01 mg/kg (GalNAc)3-ApoB#02 + 5 mg/kg (GalNAc)3-SO1861
0.1 mg/kg (GalNAc)3-ApoB#02 + 5 mg/kg (GalNAc)3-SO1861
1 mg/kg (GalNAc)3-SO1861-ApoB#02 (EMCH)
1 mg/kg (GalNAc)3-SO1861-ApoB#02 (sc)

# Fig. 27

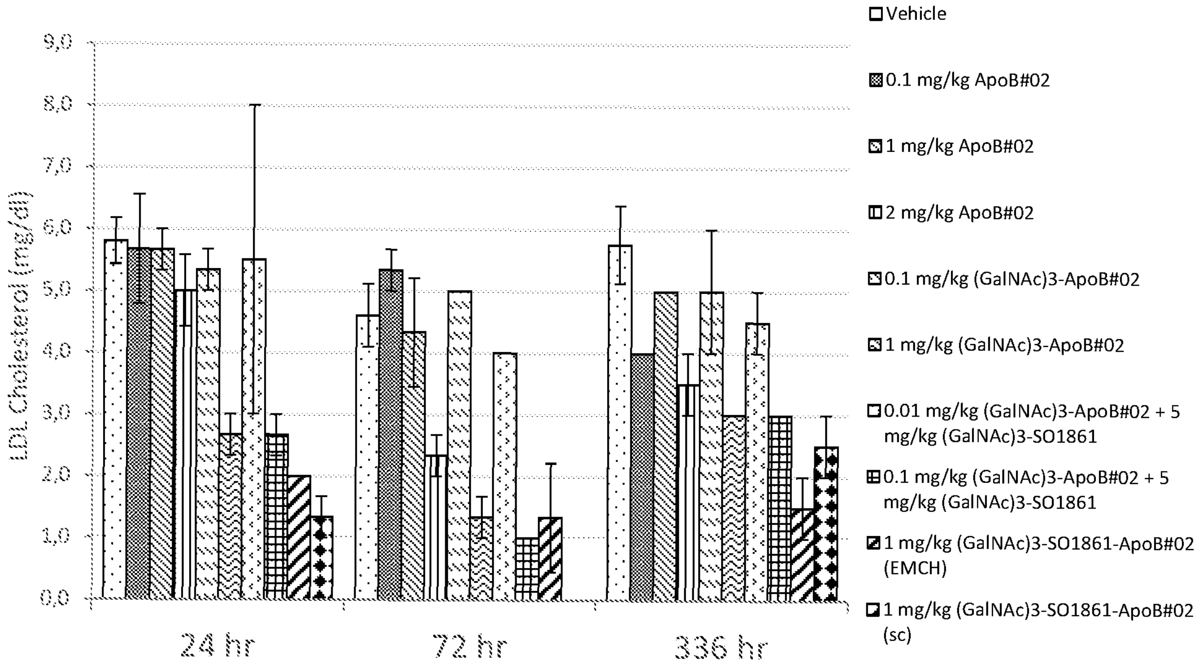
LDL Cholesterol (mg/dl)
9,0
8,0
7,0
6,0
5,0
4,0
3,0
2,0
1,0
0,0
24 hr
72 hr
336 hr
Vehicle
0.1 mg/kg ApoB#02
1 mg/kg ApoB#02
2 mg/kg ApoB#02
0.1 mg/kg (GalNAc)3-ApoB#02
1 mg/kg (GalNAc)3-ApoB#02
0.01 mg/kg (GalNAc)3-ApoB#02 + 5 mg/kg (GalNAc)3-SO1861
0.1 mg/kg (GalNAc)3-ApoB#02 + 5 mg/kg (GalNAc)3-SO1861
1 mg/kg (GalNAc)3-SO1861-ApoB#02 (EMCH)
1 mg/kg (GalNAc)3-SO1861-ApoB#02 (sc)

# Fig. 28

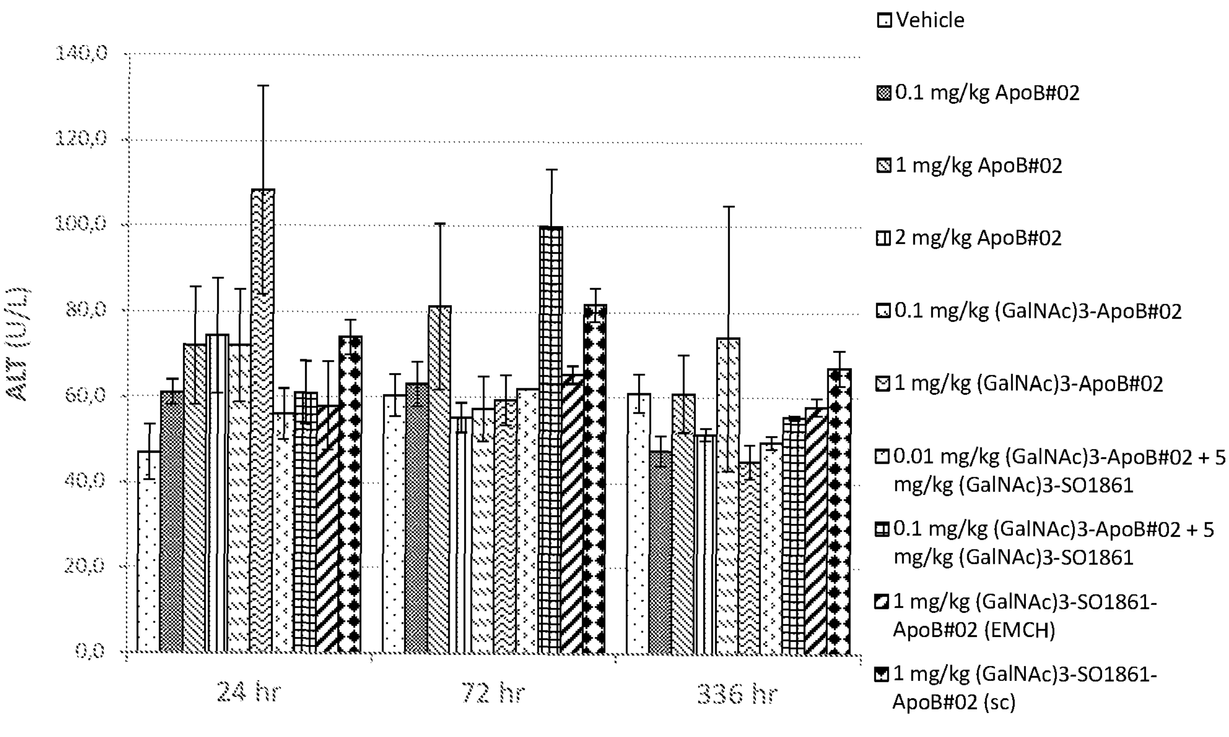
ALT (U/L)
140,0
120,0
100,0
80,0
60,0
40,0
20,0
0,0
24 hr
72 hr
336 hr
Vehicle
0.1 mg/kg ApoB#02
1 mg/kg ApoB#02
2 mg/kg ApoB#02
0.1 mg/kg (GalNAc)3-ApoB#02
1 mg/kg (GalNAc)3-ApoB#02
0.01 mg/kg (GalNAc)3-ApoB#02 + 5 mg/kg (GalNAc)3-SO1861
0.1 mg/kg (GalNAc)3-ApoB#02 + 5 mg/kg (GalNAc)3-SO1861
1 mg/kg (GalNAc)3-SO1861-ApoB#02 (EMCH)
1 mg/kg (GalNAc)3-SO1861-ApoB#02 (sc)

Fig. 29

A

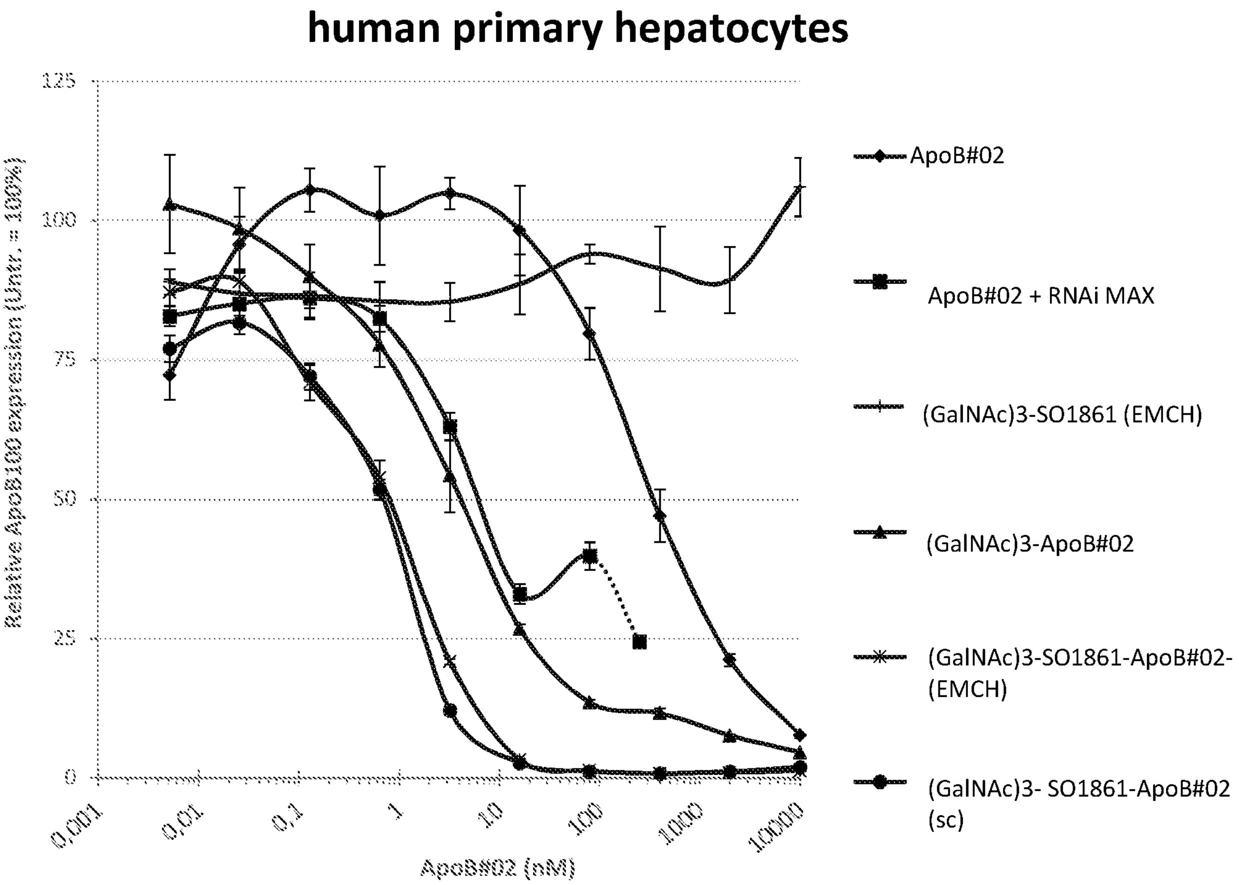
human primary hepatocytes
125
100
75
50
25
0
Relative ApoB100 expression (Untr. = 100%)
0,001
0,01
0,1
1
10
100
1000
10000
ApoB#02 (nM)
ApoB#02
ApoB#02 + RNAi MAX
(GalNAc)3-SO1861 (EMCH)
(GalNAc)3-ApoB#02
(GalNAc)3-SO1861-ApoB#02-(EMCH)
(GalNAc)3- SO1861-ApoB#02 (sc)

# Fig. 29

## B

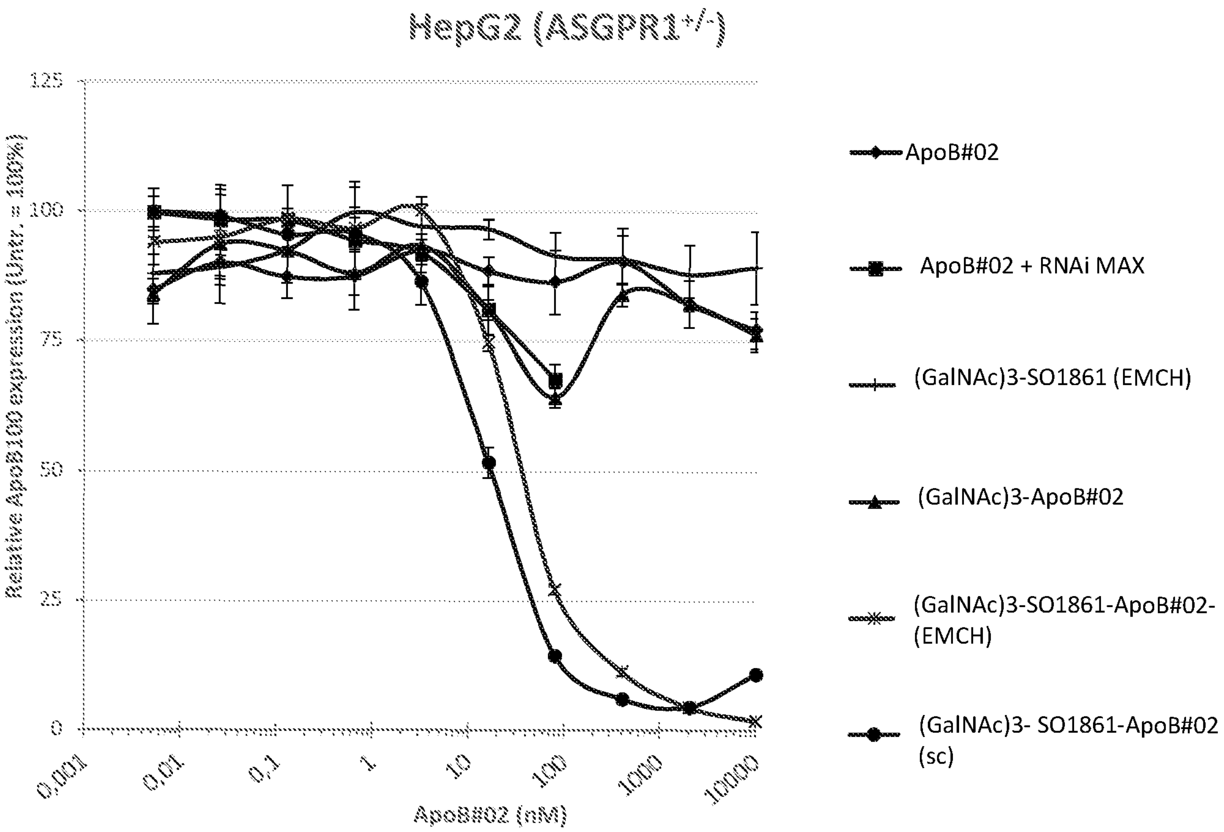
HepG2 (ASGPR1+/-)
Relative ApoB100 expression (Untr. = 100%)
125
100
75
50
25
0
0,001
0,01
0,1
1
10
100
1000
10000
ApoB#02 (nM)
ApoB#02
ApoB#02 + RNAi MAX
(GalNAc)3-SO1861 (EMCH)
(GalNAc)3-ApoB#02
(GalNAc)3-SO1861-ApoB#02-(EMCH)
(GalNAc)3- SO1861-ApoB#02 (sc)

# Fig. 29

## C

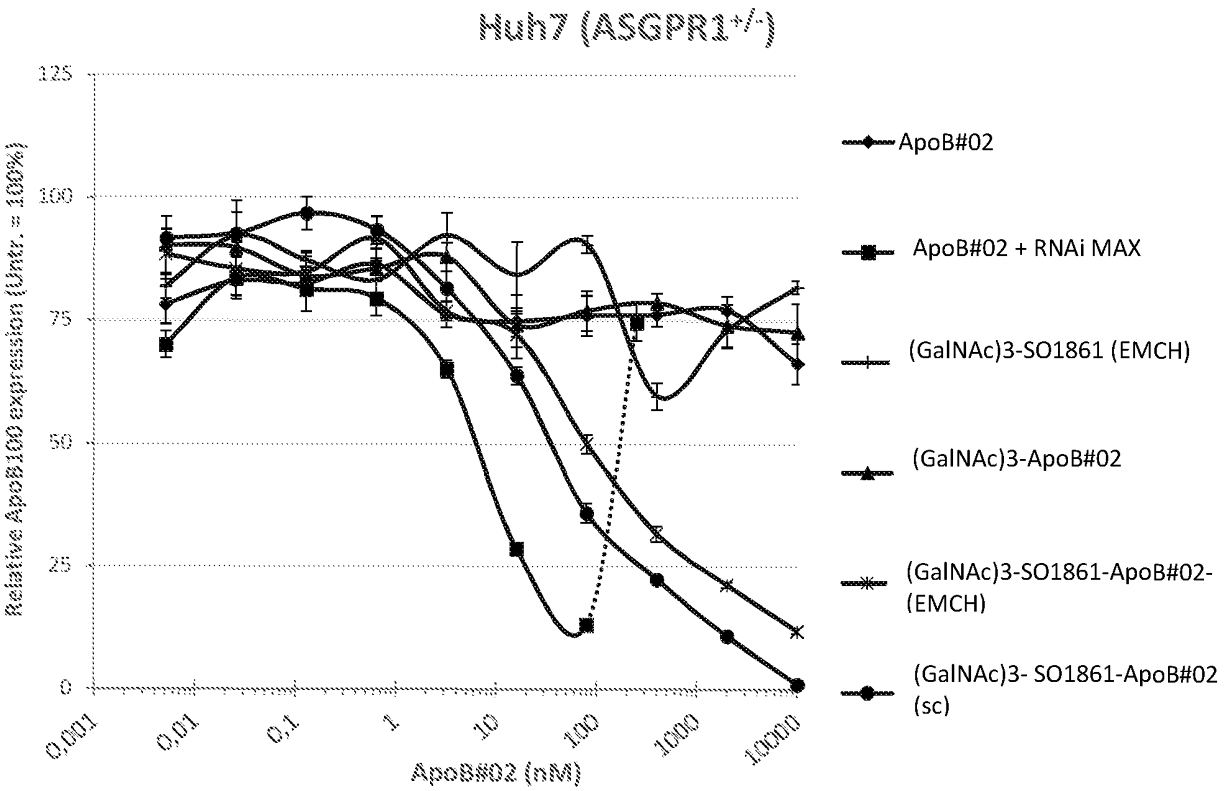
Huh7 (ASGPR1+/-)
Relative ApoB100 expression (Untr. = 100%)
125
100
75
50
25
0
0,001
0,01
0,1
1
10
100
1000
10000
ApoB#02 (nM)
ApoB#02
ApoB#02 + RNAi MAX
(GalNAc)3-SO1861 (EMCH)
(GalNAc)3-ApoB#02
(GalNAc)3-SO1861-ApoB#02-(EMCH)
(GalNAc)3- SO1861-ApoB#02 (sc)

Fig. 30

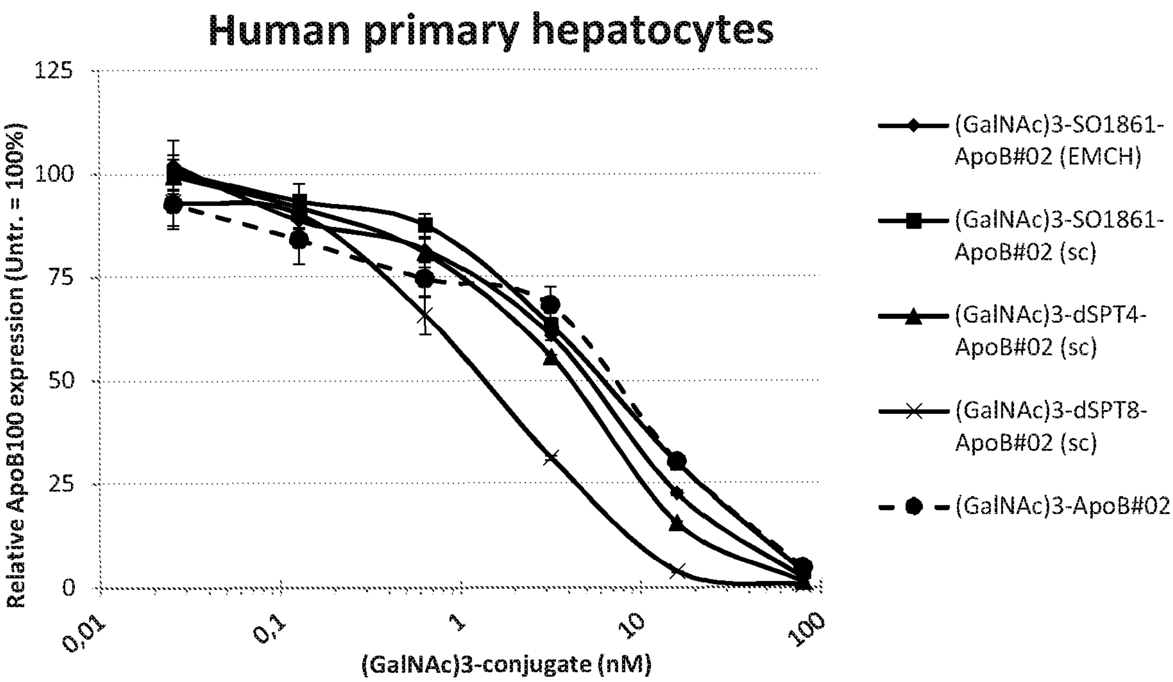
Human primary hepatocytes
Relative ApoB100 expression (Untr. = 100%)
125
100
75
50
25
0
0,01
0,1
1
10
100
(GalNAc)3-conjugate (nM)
(GalNAc)3-SO1861-ApoB#02 (EMCH)
(GalNAc)3-SO1861-ApoB#02 (sc)
(GalNAc)3-dSPT4-ApoB#02 (sc)
(GalNAc)3-dSPT8-ApoB#02 (sc)
(GalNAc)3-ApoB#02

# Fig. 31

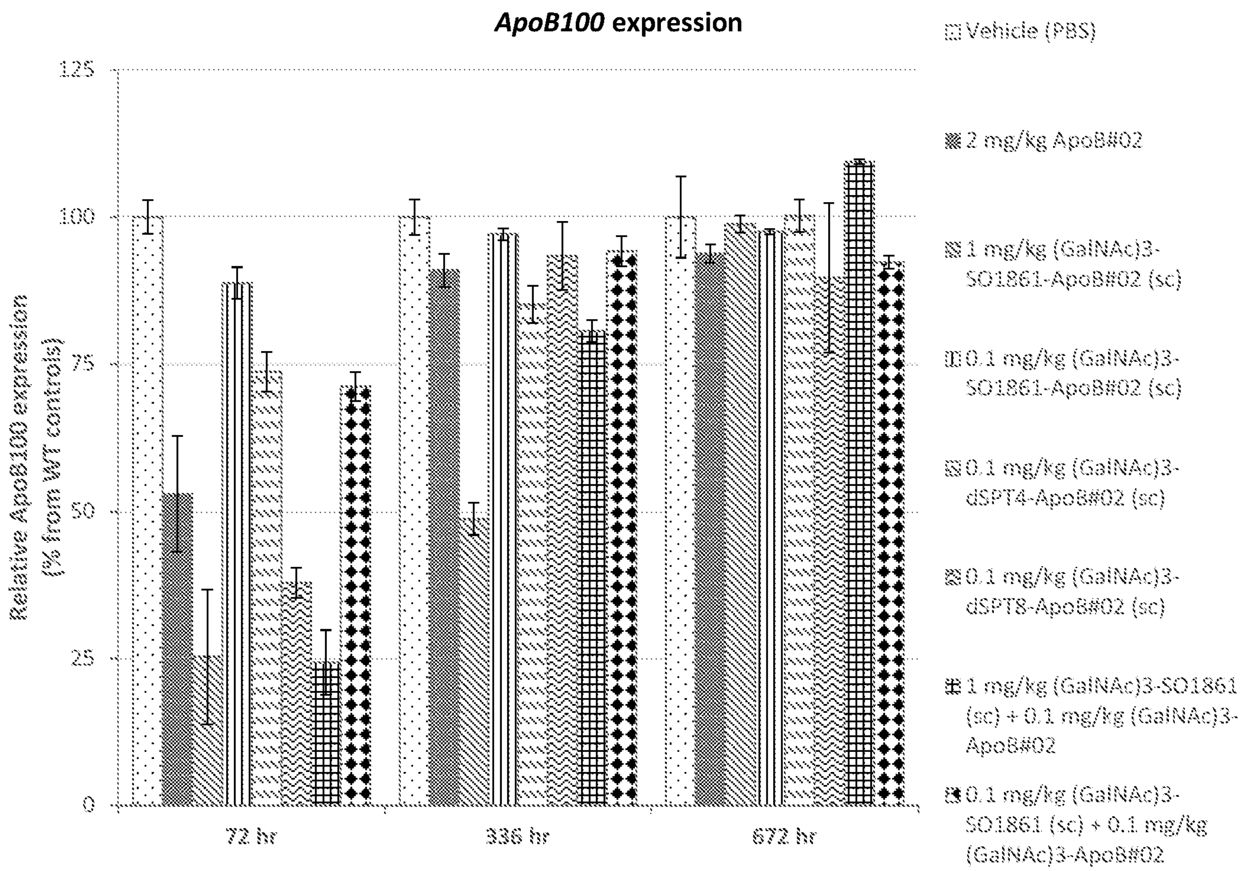
*ApoB100* expression
Relative ApoB100 expression (% from WT controls)
0
25
50
75
100
125
72 hr
336 hr
672 hr
Vehicle (PBS)
2 mg/kg ApoB#02
1 mg/kg (GalNAc)3-SO1861-ApoB#02 (sc)
0.1 mg/kg (GalNAc)3-SO1861-ApoB#02 (sc)
0.1 mg/kg (GalNAc)3-dSPT4-ApoB#02 (sc)
0.1 mg/kg (GalNAc)3-dSPT8-ApoB#02 (sc)
1 mg/kg (GalNAc)3-SO1861 (sc) + 0.1 mg/kg (GalNAc)3-ApoB#02
0.1 mg/kg (GalNAc)3-SO1861 (sc) + 0.1 mg/kg (GalNAc)3-ApoB#02

# Fig. 32

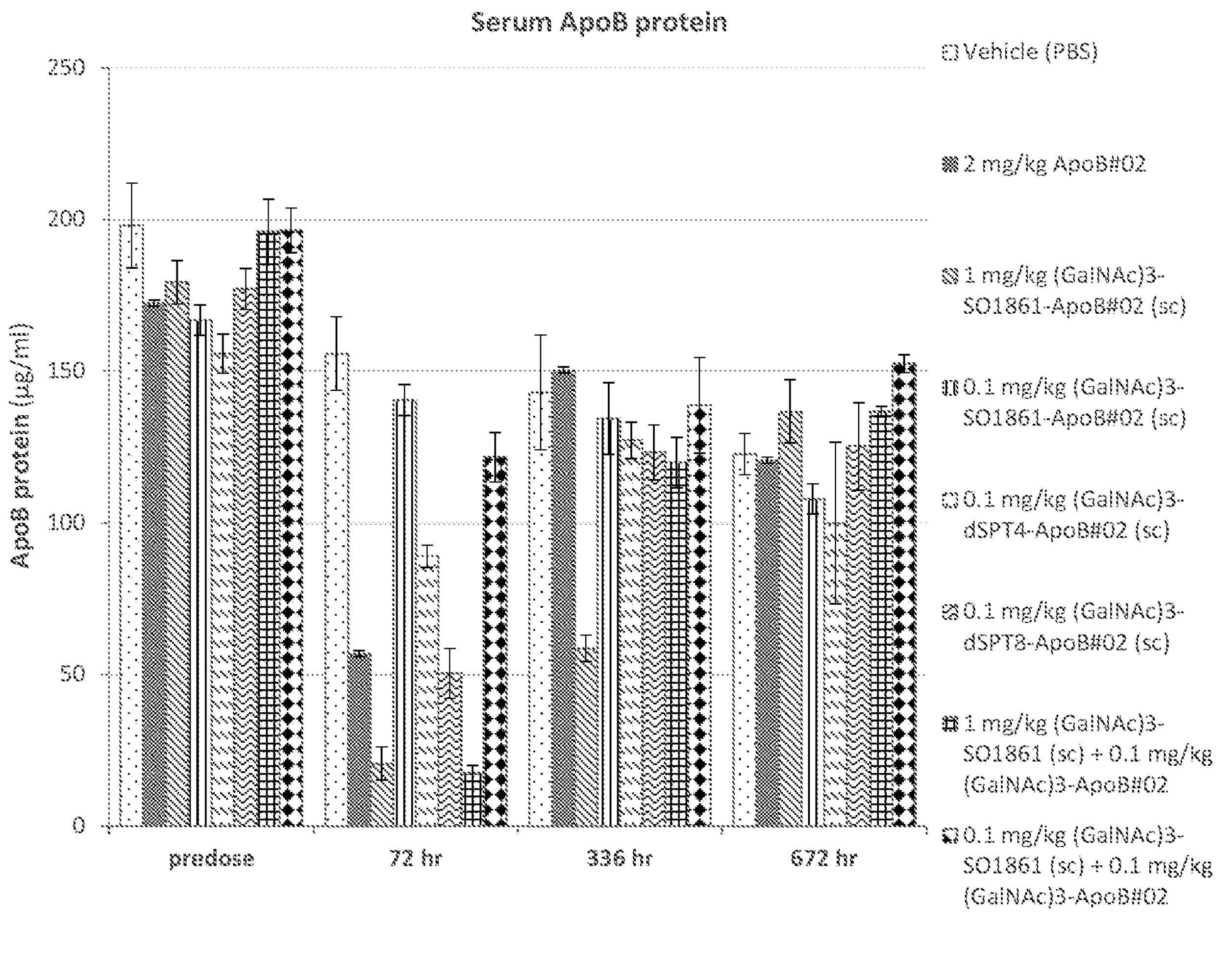
Serum ApoB protein
ApoB protein (µg/ml)
250
200
150
100
50
0
predose
72 hr
336 hr
672 hr
Vehicle (PBS)
2 mg/kg ApoB#02
1 mg/kg (GalNAc)3-SO1861-ApoB#02 (sc)
0.1 mg/kg (GalNAc)3-SO1861-ApoB#02 (sc)
0.1 mg/kg (GalNAc)3-dSPT4-ApoB#02 (sc)
0.1 mg/kg (GalNAc)3-dSPT8-ApoB#02 (sc)
1 mg/kg (GalNAc)3-SO1861 (sc) + 0.1 mg/kg (GalNAc)3-ApoB#02
0.1 mg/kg (GalNAc)3-SO1861 (sc) + 0.1 mg/kg (GalNAc)3-ApoB#02

Fig. 33

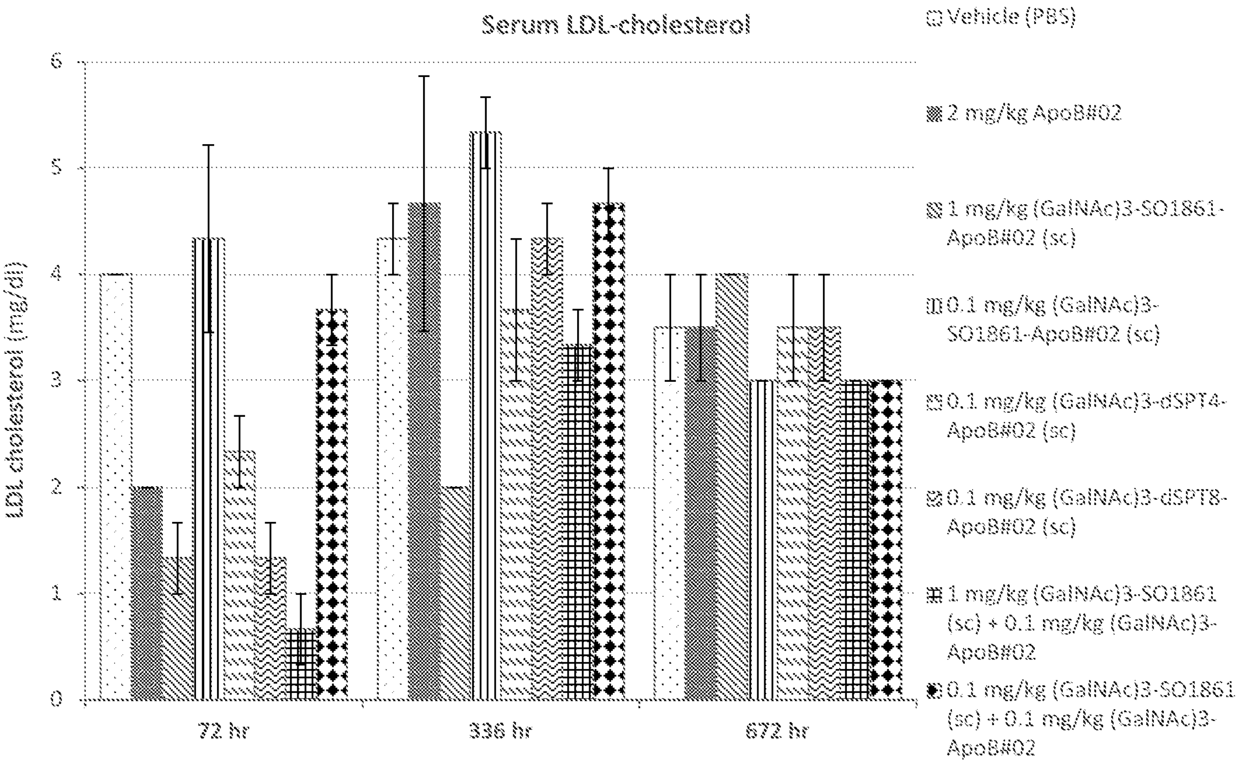
Serum LDL-cholesterol
LDL cholesterol (mg/dl)
0
1
2
3
4
5
6
72 hr
336 hr
672 hr
Vehicle (PBS)
2 mg/kg ApoB#02
1 mg/kg (GalNAc)3-SO1861-ApoB#02 (sc)
0.1 mg/kg (GalNAc)3-SO1861-ApoB#02 (sc)
0.1 mg/kg (GalNAc)3-dSPT4-ApoB#02 (sc)
0.1 mg/kg (GalNAc)3-dSPT8-ApoB#02 (sc)
1 mg/kg (GalNAc)3-SO1861 (sc) + 0.1 mg/kg (GalNAc)3-ApoB#02
0.1 mg/kg (GalNAc)3-SO1861 (sc) + 0.1 mg/kg (GalNAc)3-ApoB#02

**Fig. 34**

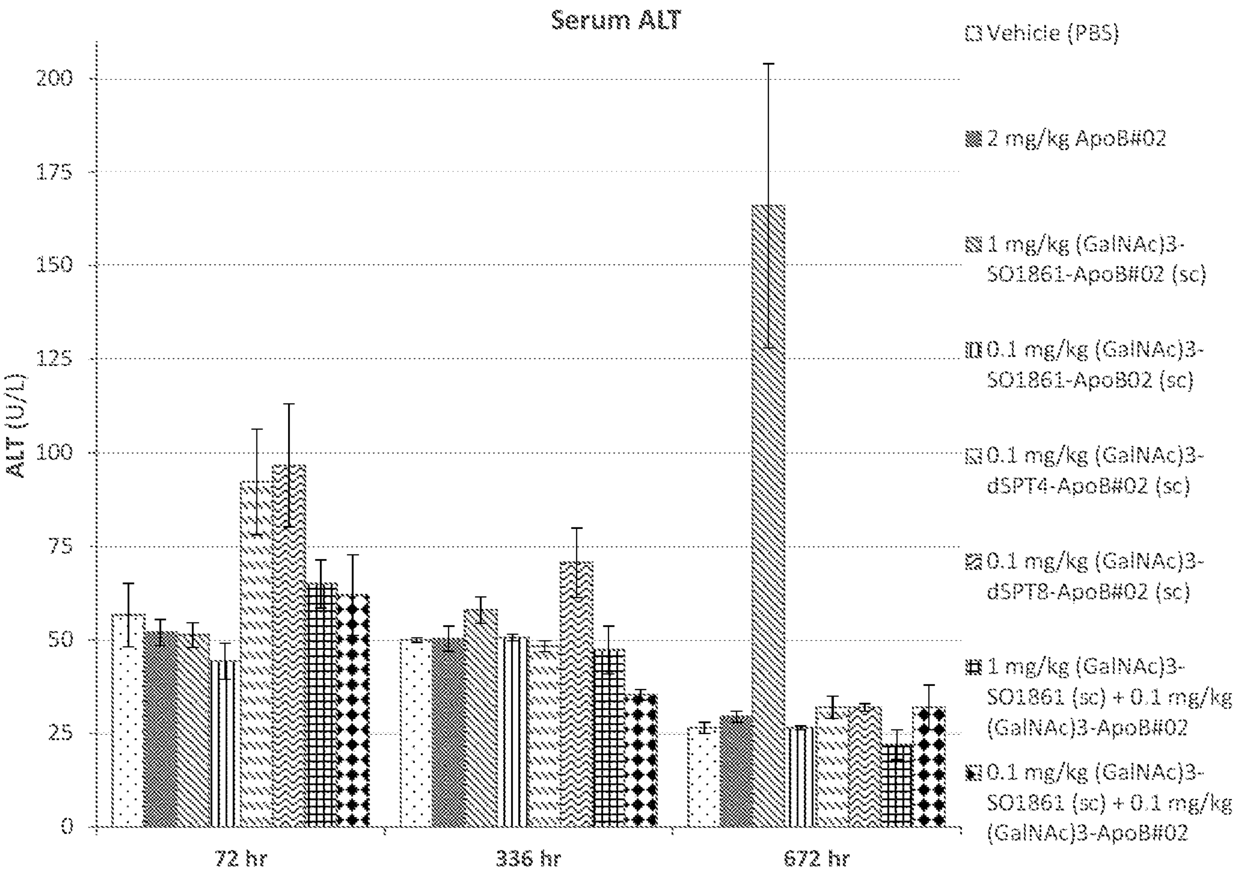
Serum ALT
ALT (U/L)
0
25
50
75
100
125
150
175
200
72 hr
336 hr
672 hr
Vehicle (PBS)
2 mg/kg ApoB#02
1 mg/kg (GalNAc)3-SO1861-ApoB#02 (sc)
0.1 mg/kg (GalNAc)3-SO1861-ApoB02 (sc)
0.1 mg/kg (GalNAc)3-dSPT4-ApoB#02 (sc)
0.1 mg/kg (GalNAc)3-dSPT8-ApoB#02 (sc)
1 mg/kg (GalNAc)3-SO1861 (sc) + 0.1 mg/kg (GalNAc)3-ApoB#02
0.1 mg/kg (GalNAc)3-SO1861 (sc) + 0.1 mg/kg (GalNAc)3-ApoB#02

# Fig. 35
## A

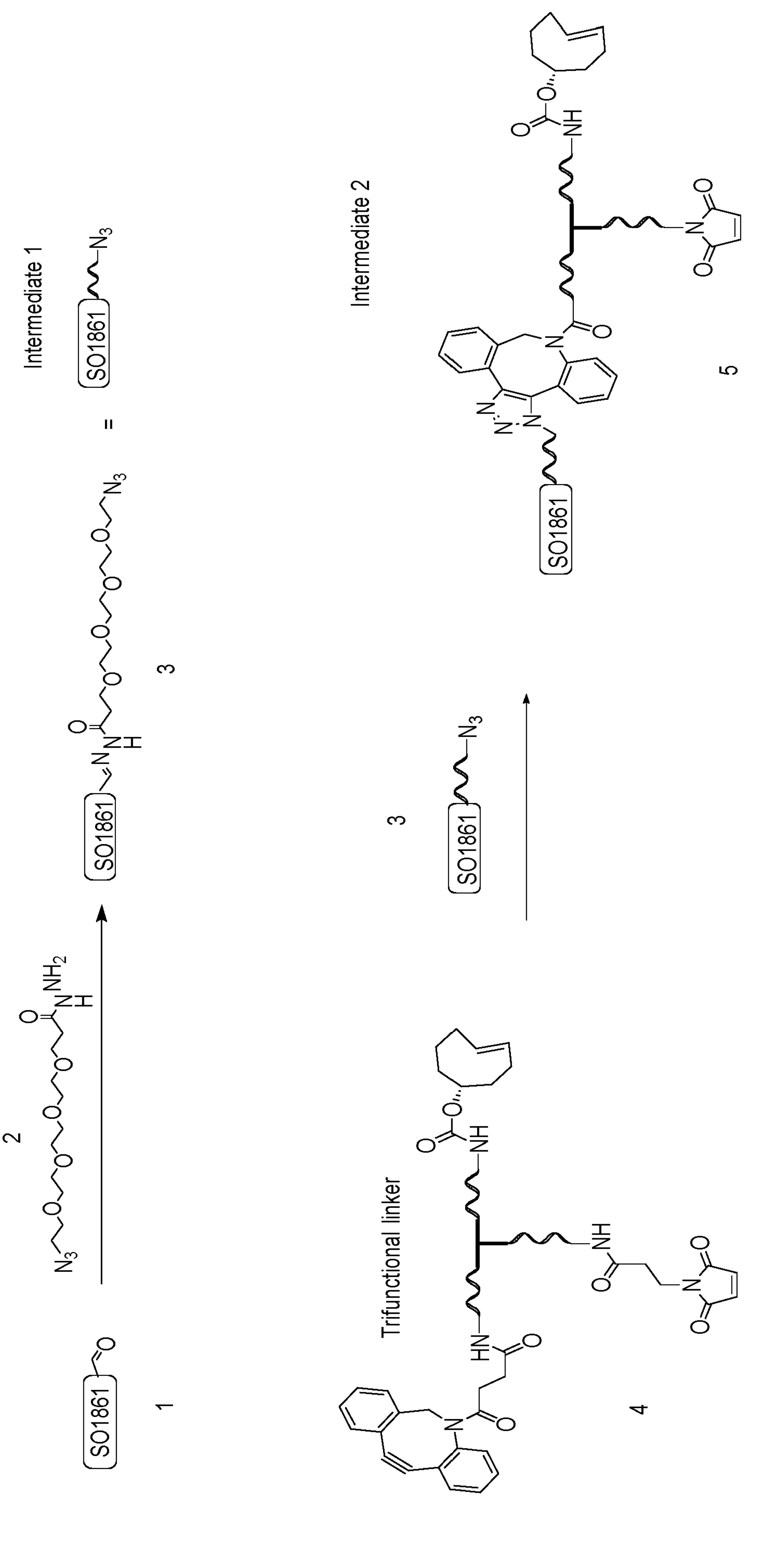

1
2
3
Intermediate 1
Trifunctional linker
4
3
5
Intermediate 2

**Fig. 35**
**B**

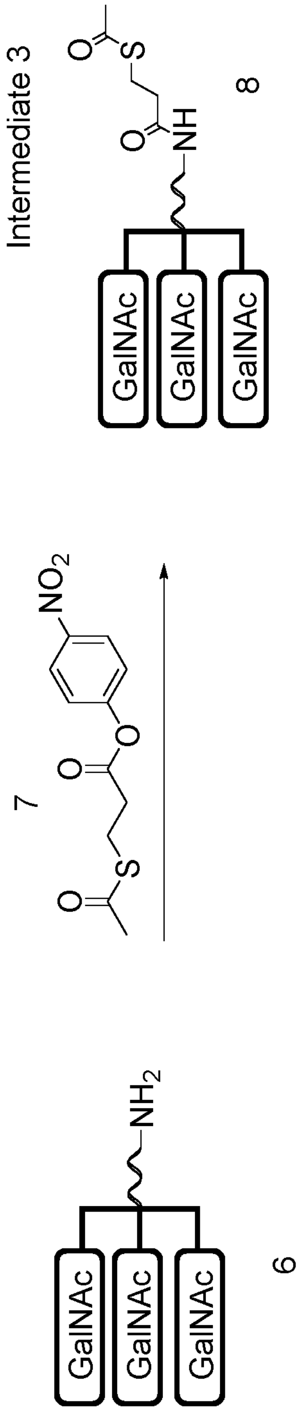
6

7

Intermediate 3

8

# Fig. 35 C

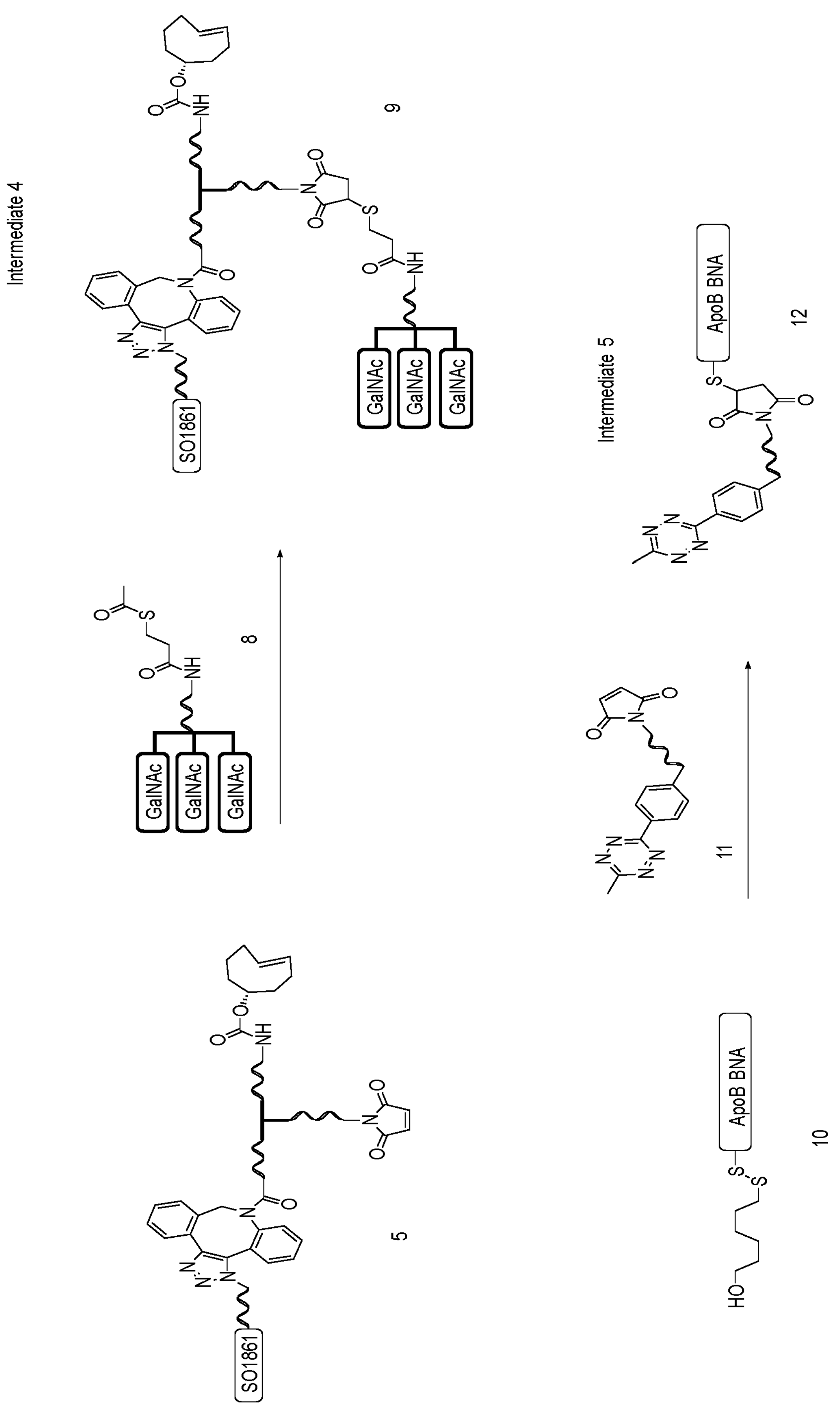

5

8

9

Intermediate 4

10

11

12

Intermediate 5

# Fig. 35
# D

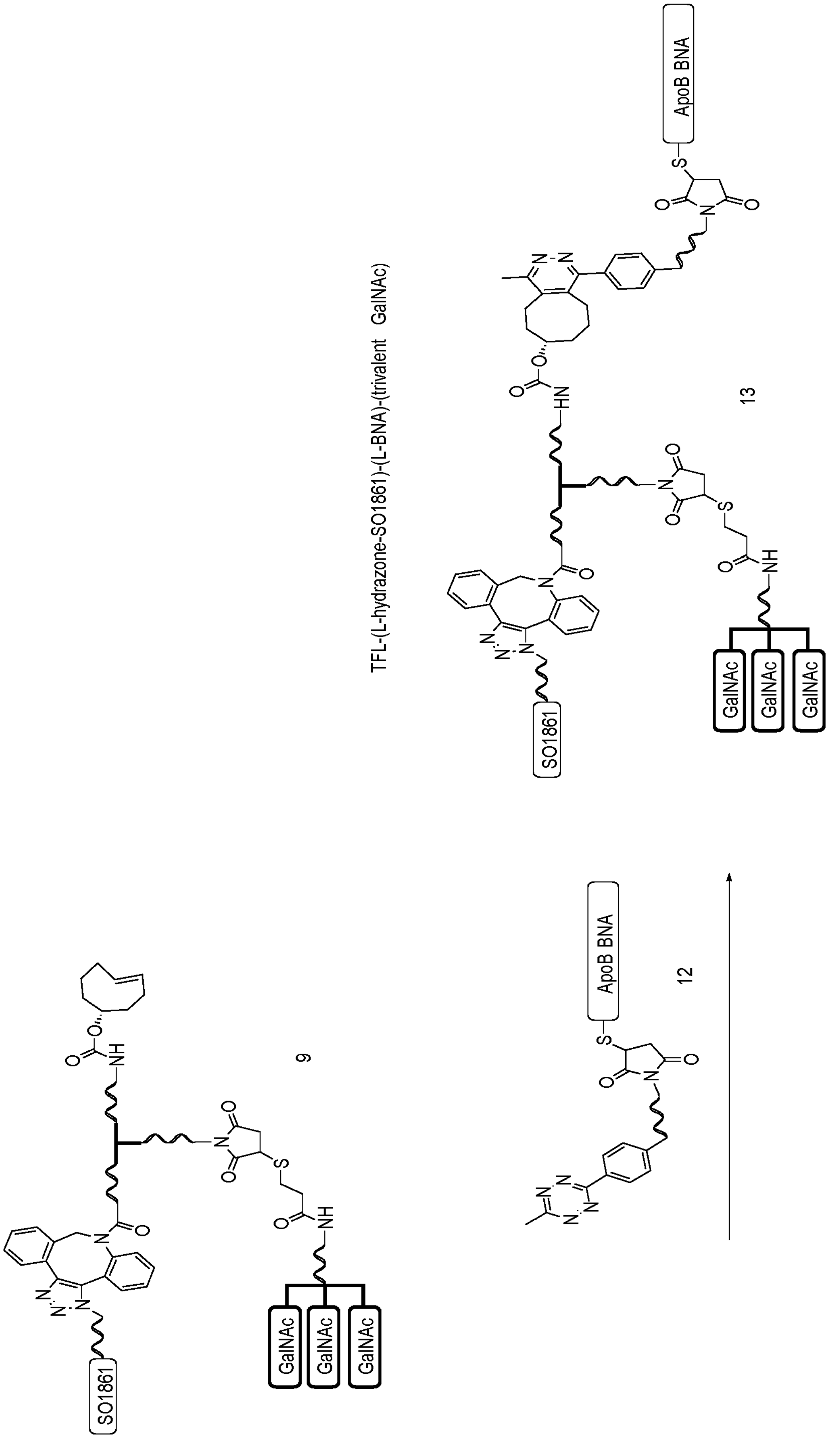

9

12

13

TFL-(L-hydrazone-SO1861)-(L-BNA)-(trivalent GalNAc)

**Fig. 36**
**A**

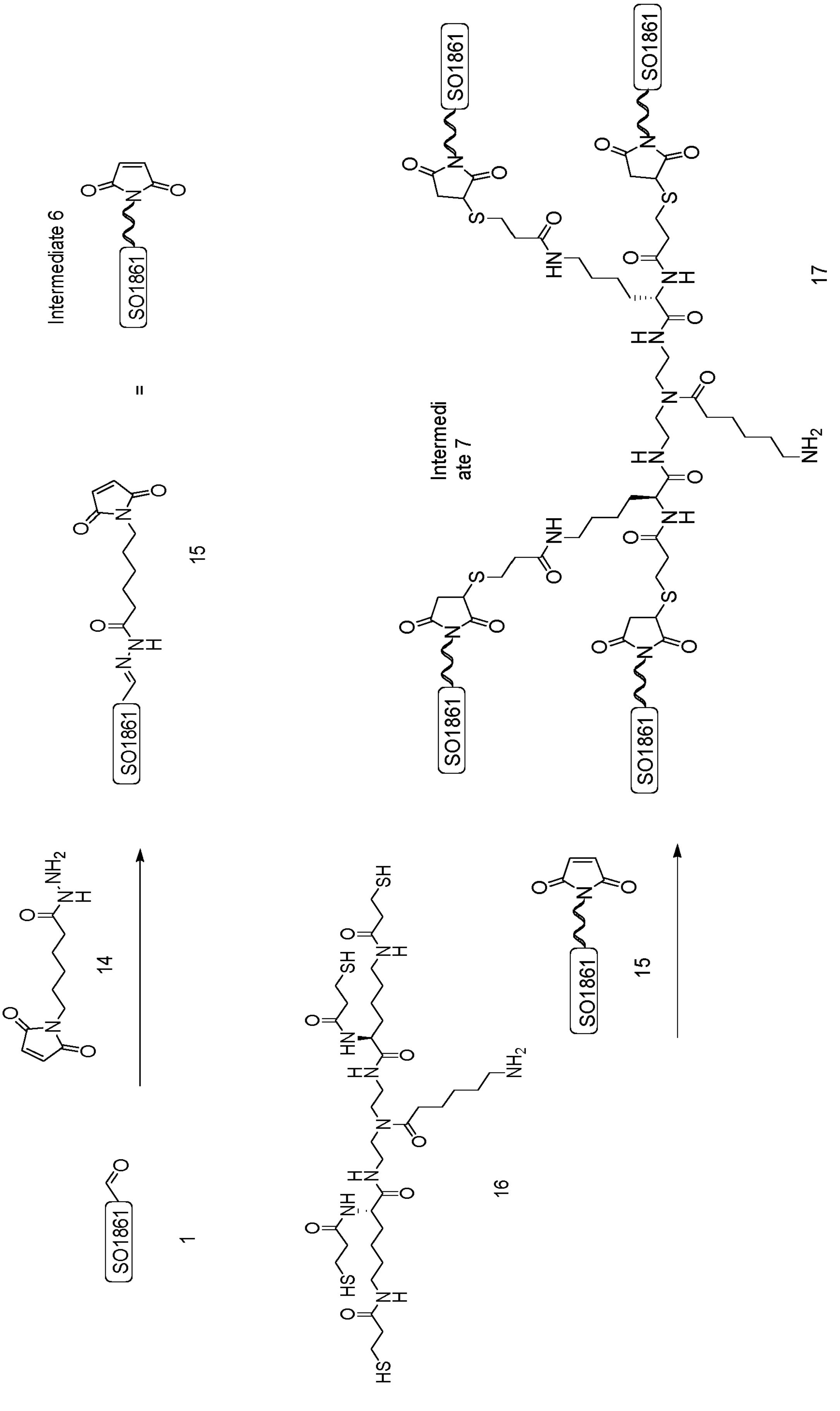

1

14

15

= Intermediate 6

16

15

17

Intermedi
ate 7

**Fig. 36**
**B**

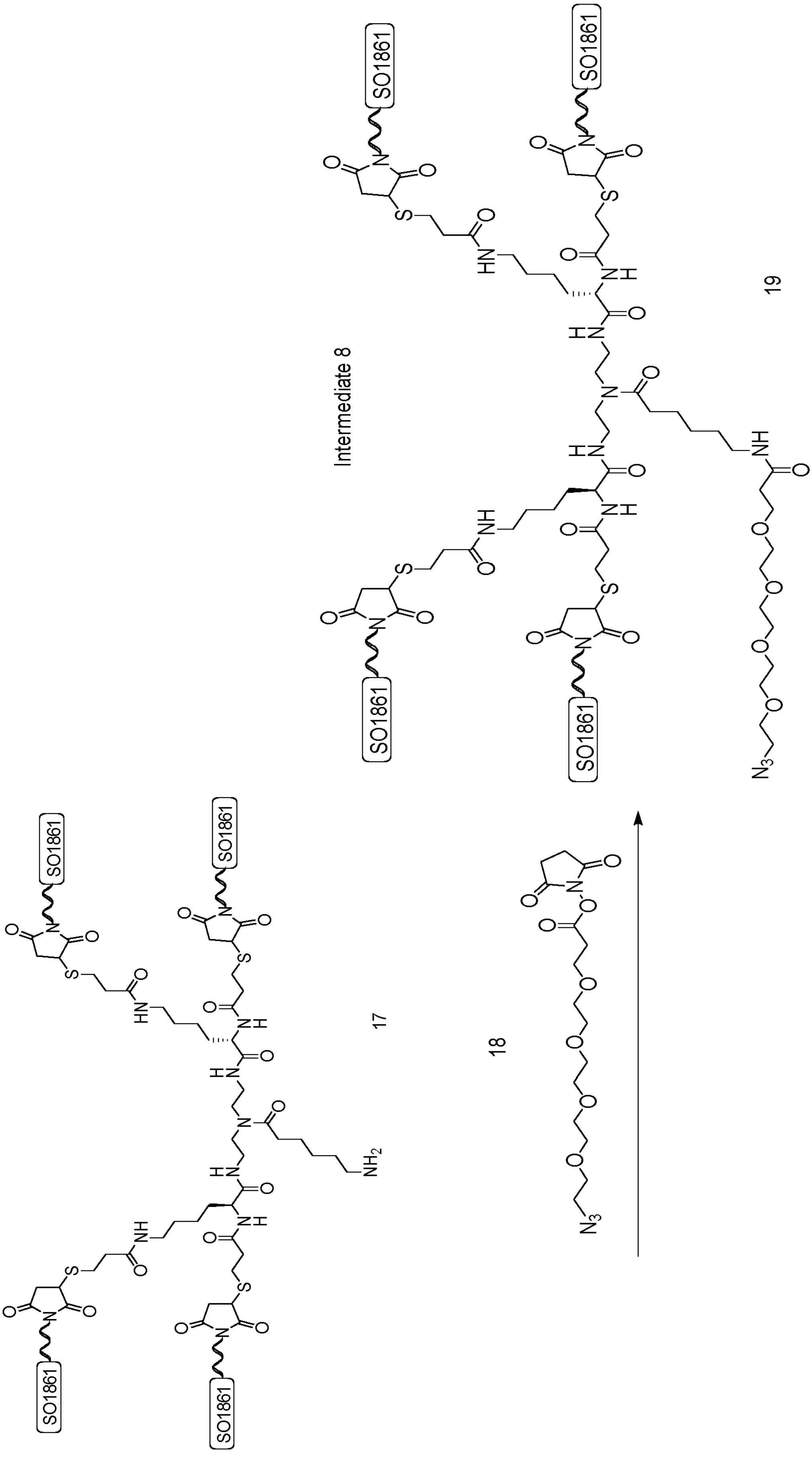

17

18

Intermediate 8

19

**Fig. 36**
**C**

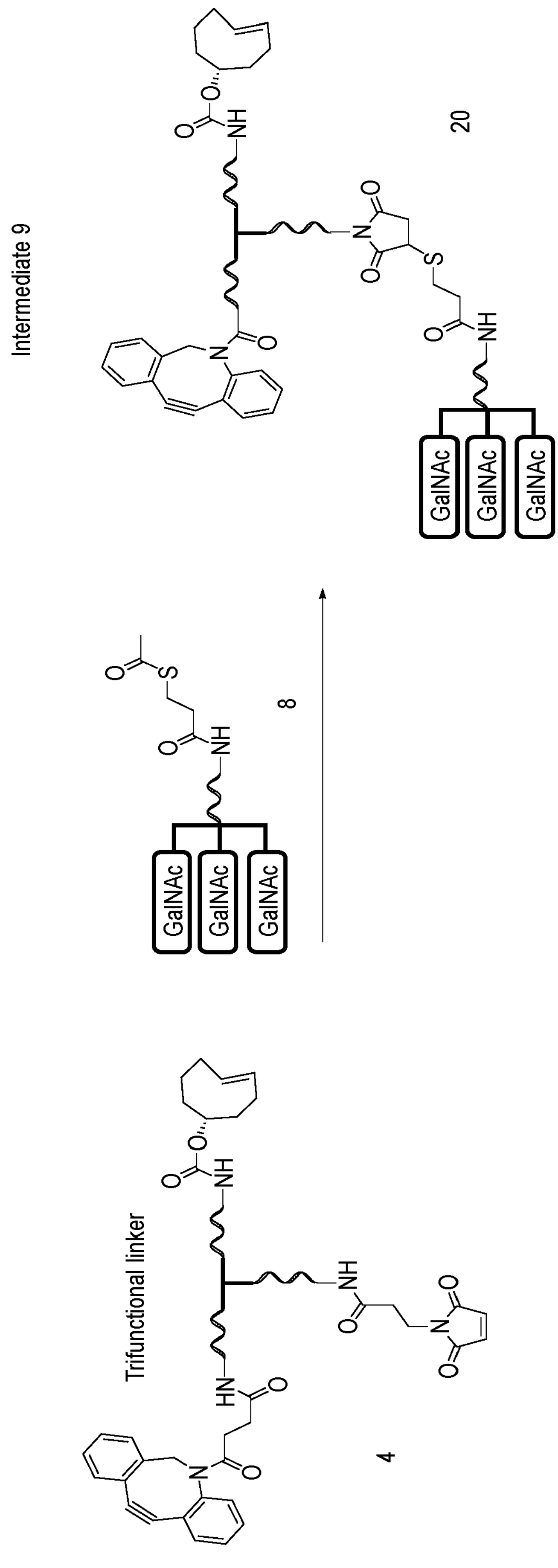

Trifunctional linker

4

8

Intermediate 9

20

# Fig. 36
# D

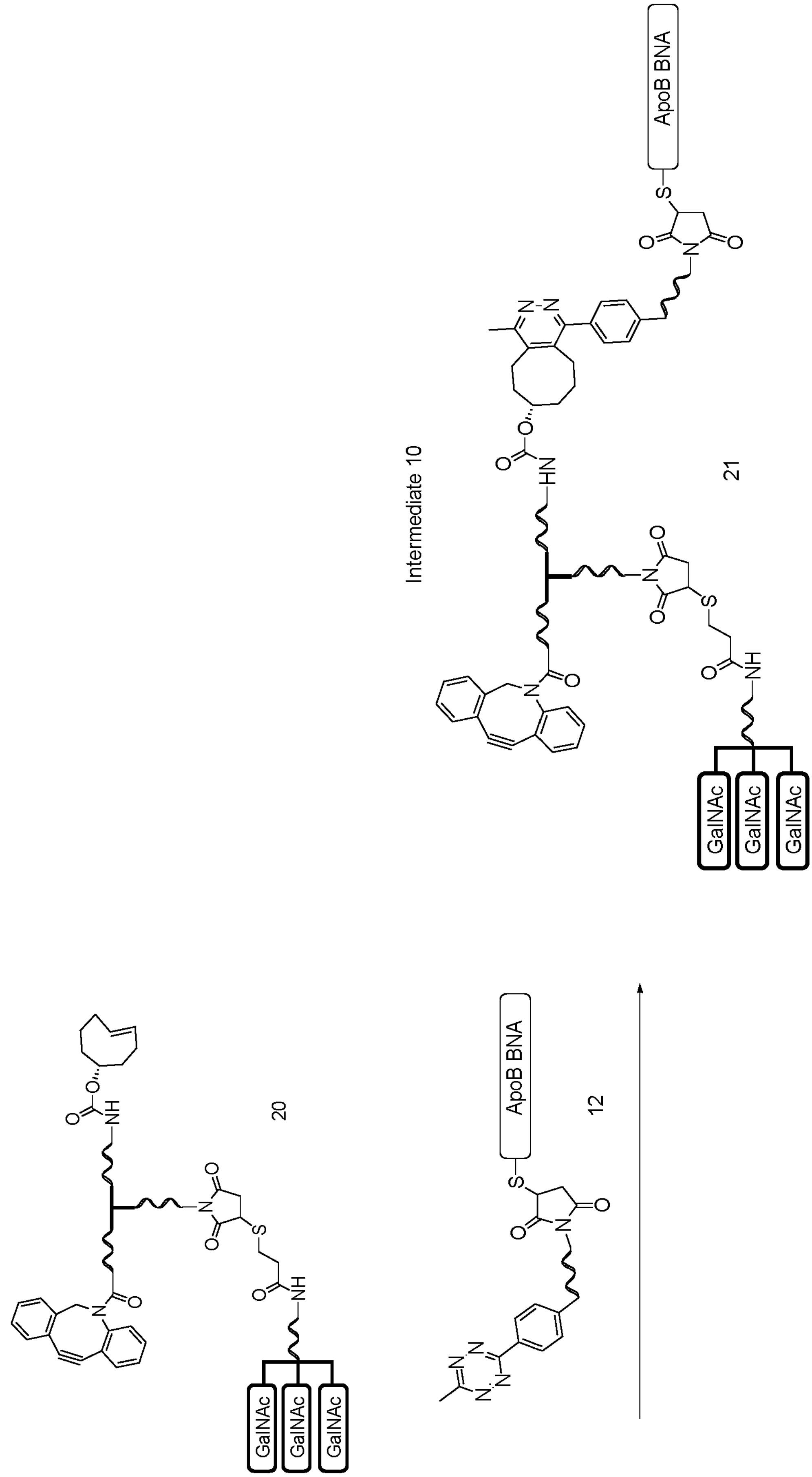

20

12

Intermediate 10

21

**Fig. 36**
**E**

TFL-(dendron(L-hydrazone-SO1861)4)-(L-BNA)-(trivalent GalNAc)

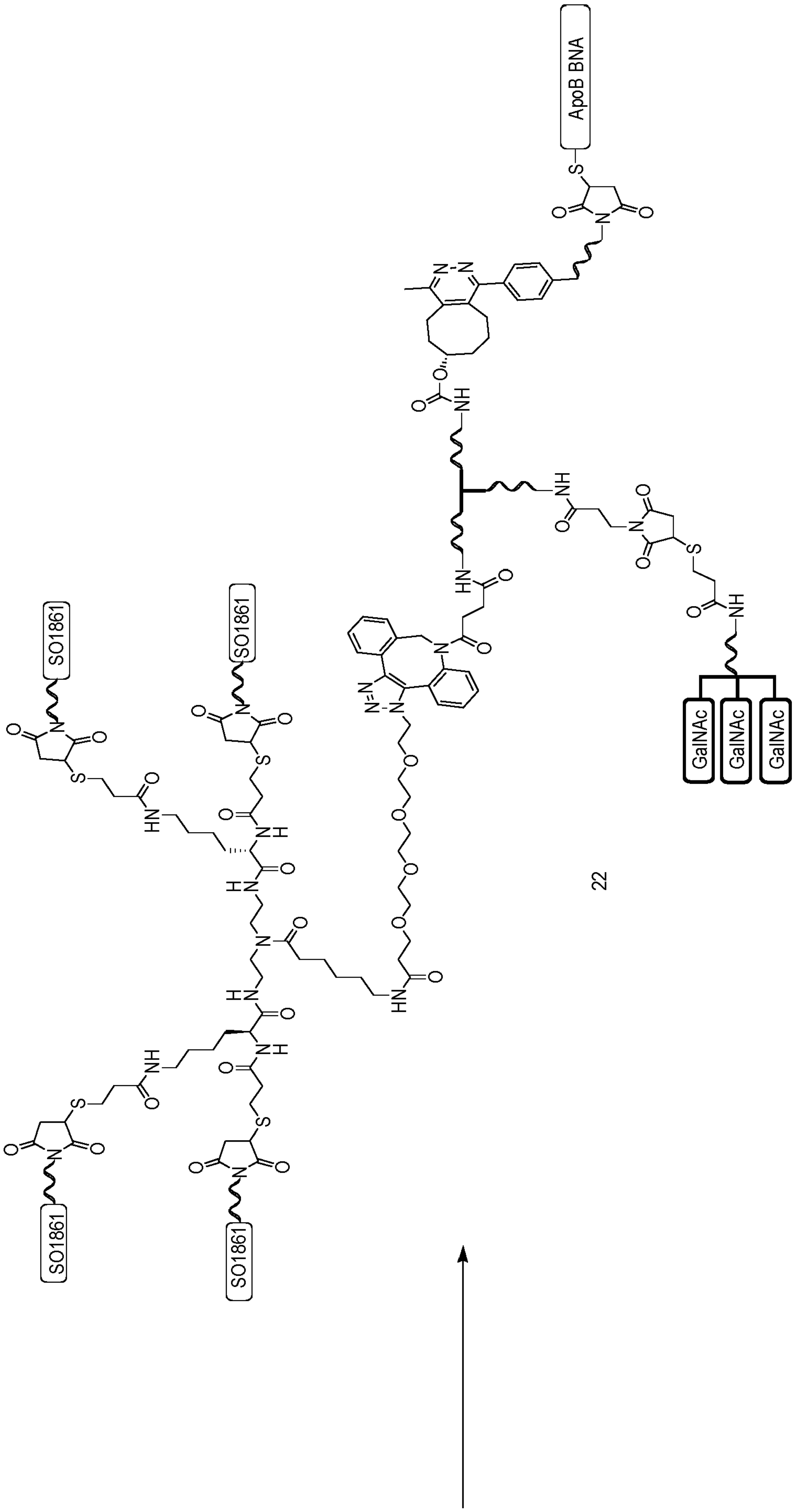


22

**Fig. 36**
**E (continued)**

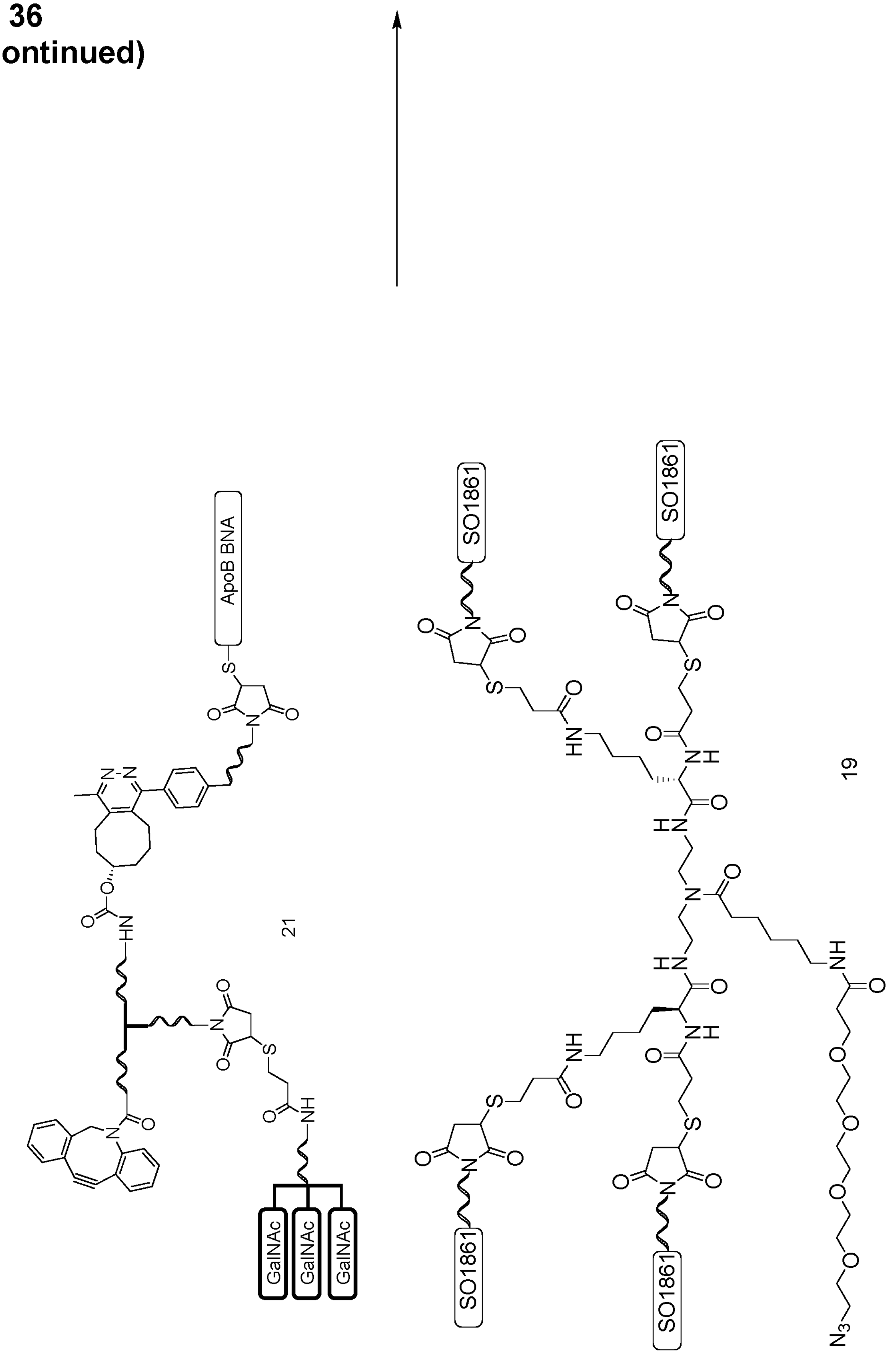

21

19

**Fig. 37**
**A**

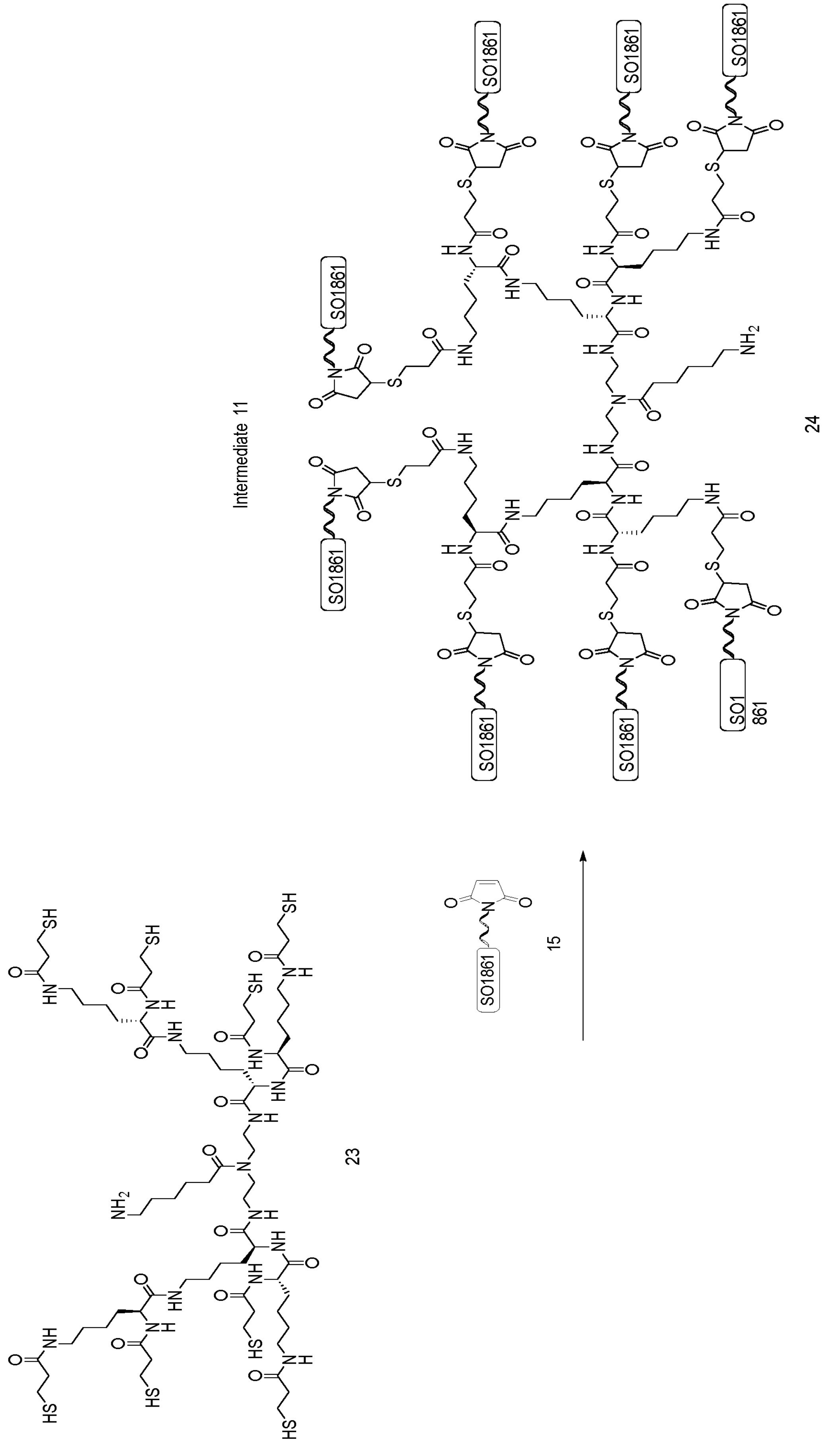

23

15

Intermediate 11

24

**Fig. 37 B**

Intermediate 12

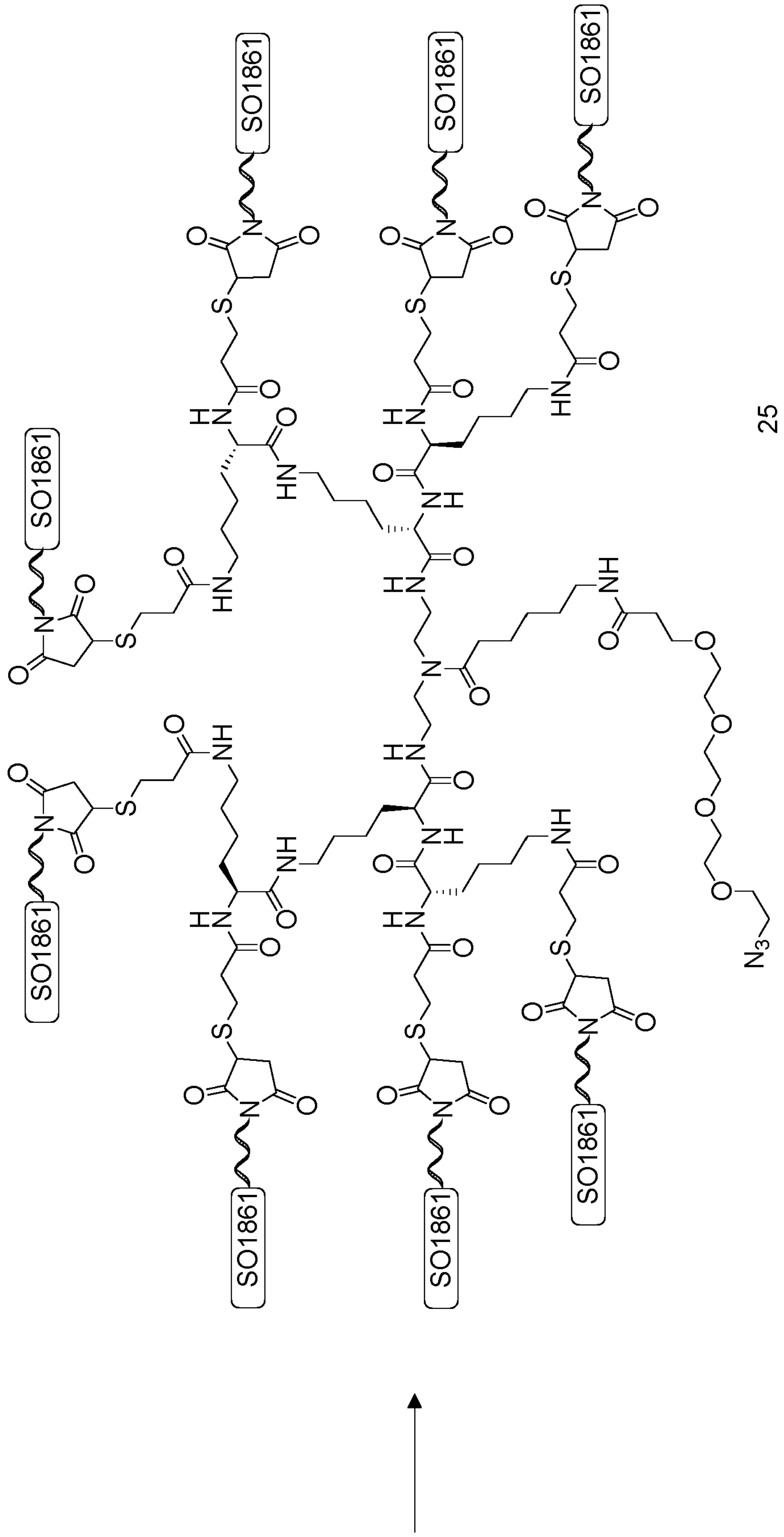

25

# Fig. 37
# B (continued)

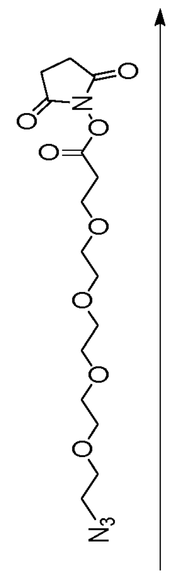

18

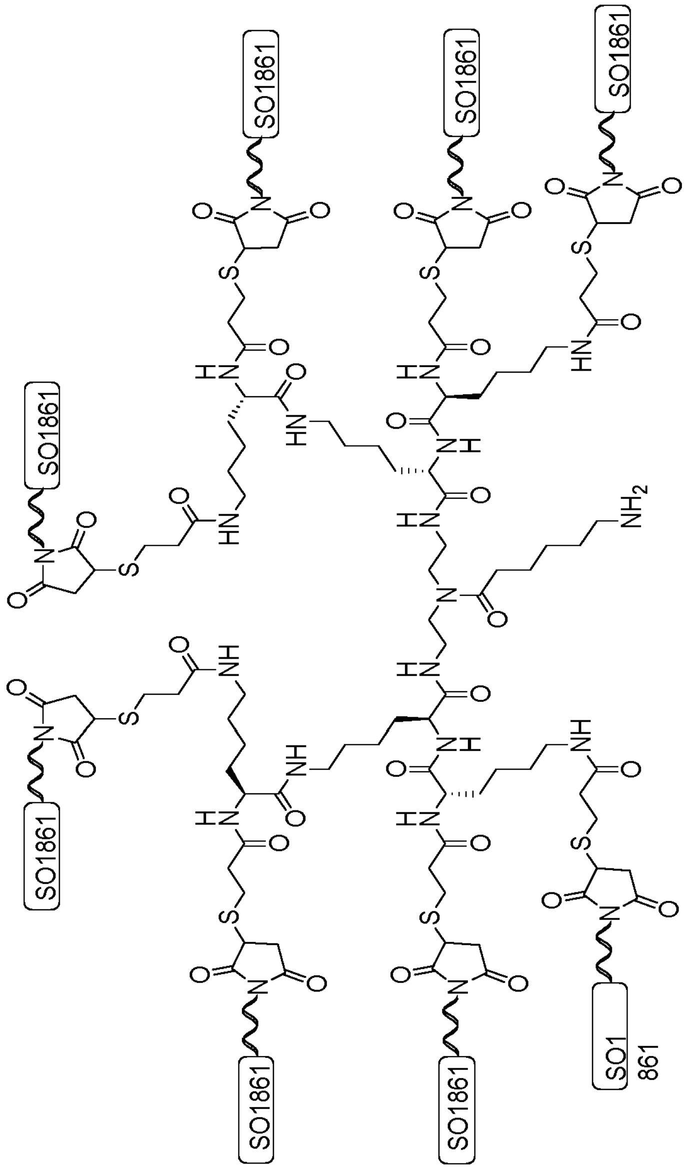

24

**Fig. 37 C**

TFL-(dendron(L-hydrazone-SO1861)8)-(L-BNA)-(trivalent GalNAc)

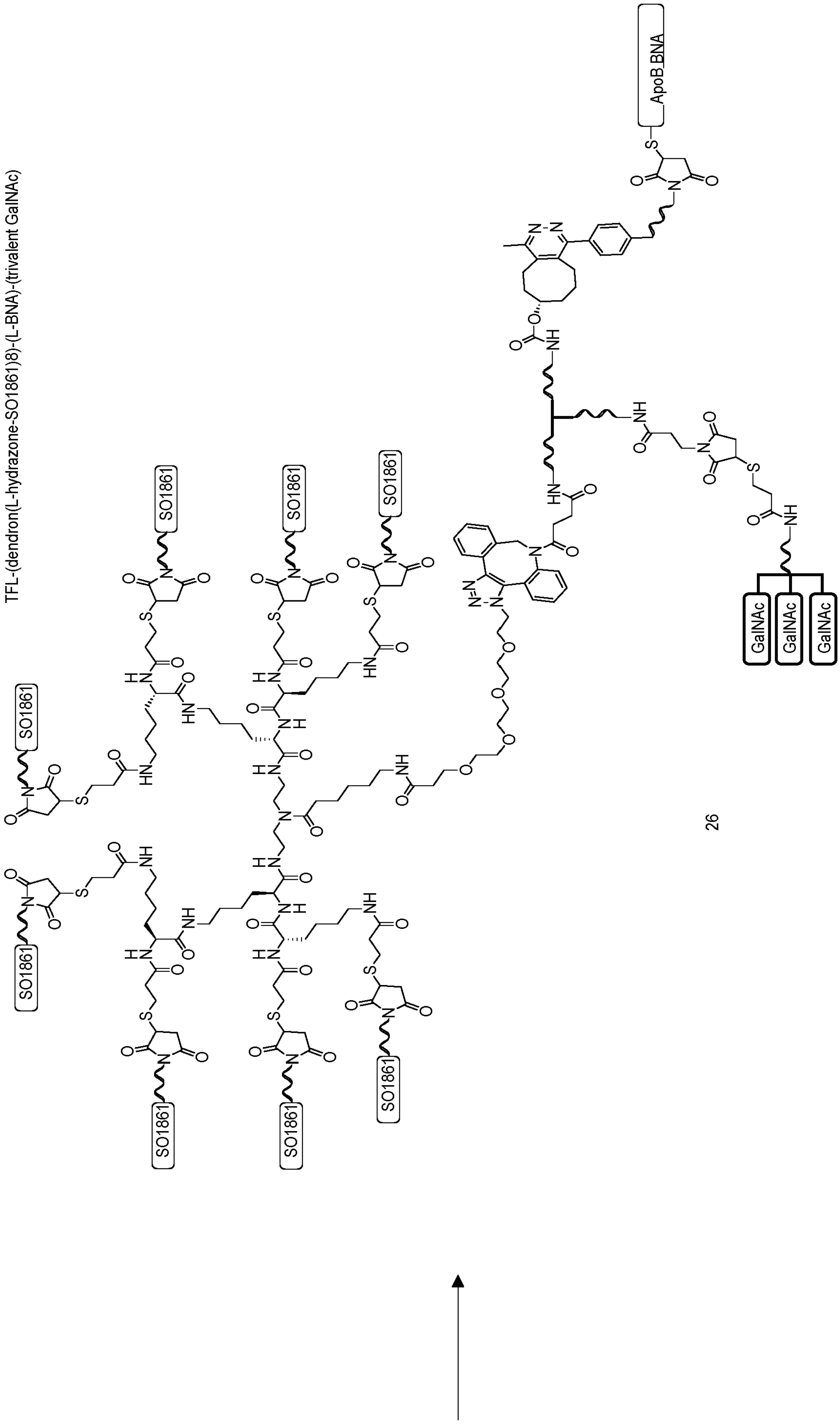

26

# Fig. 37
# C (continued)

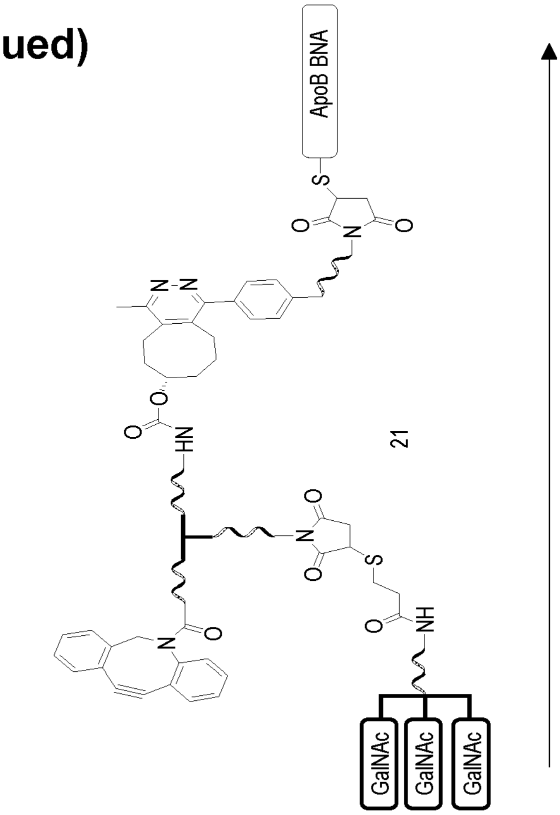

21

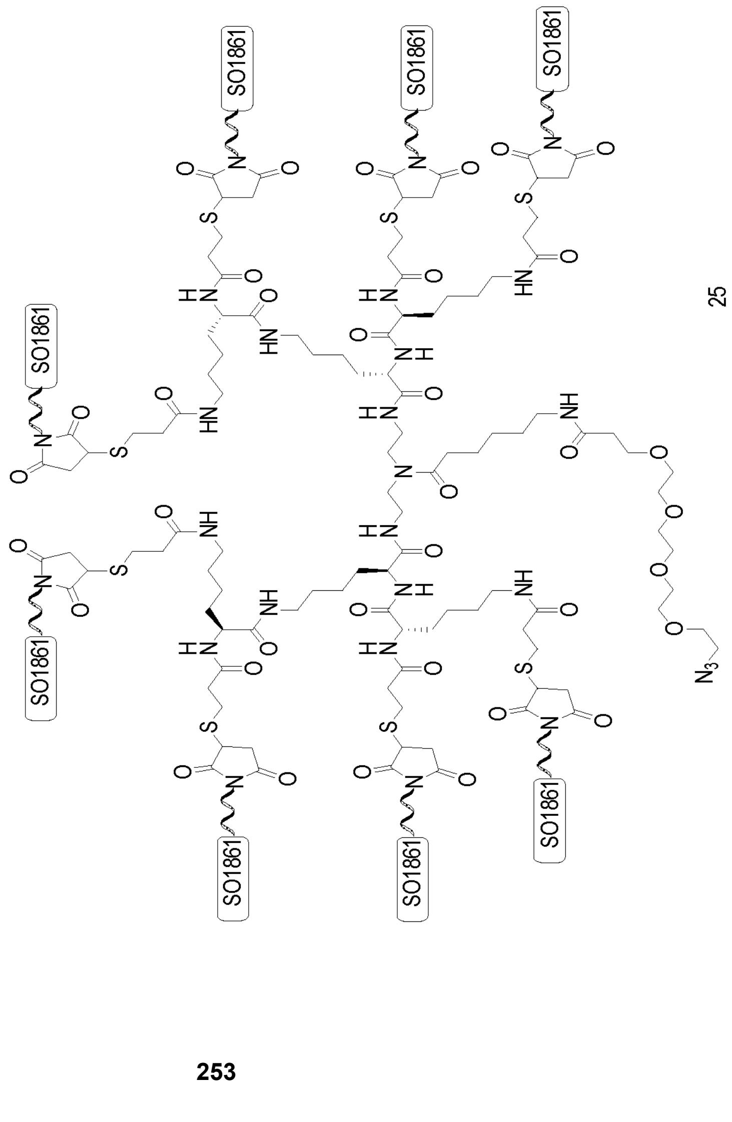

25

# Fig. 38
# A

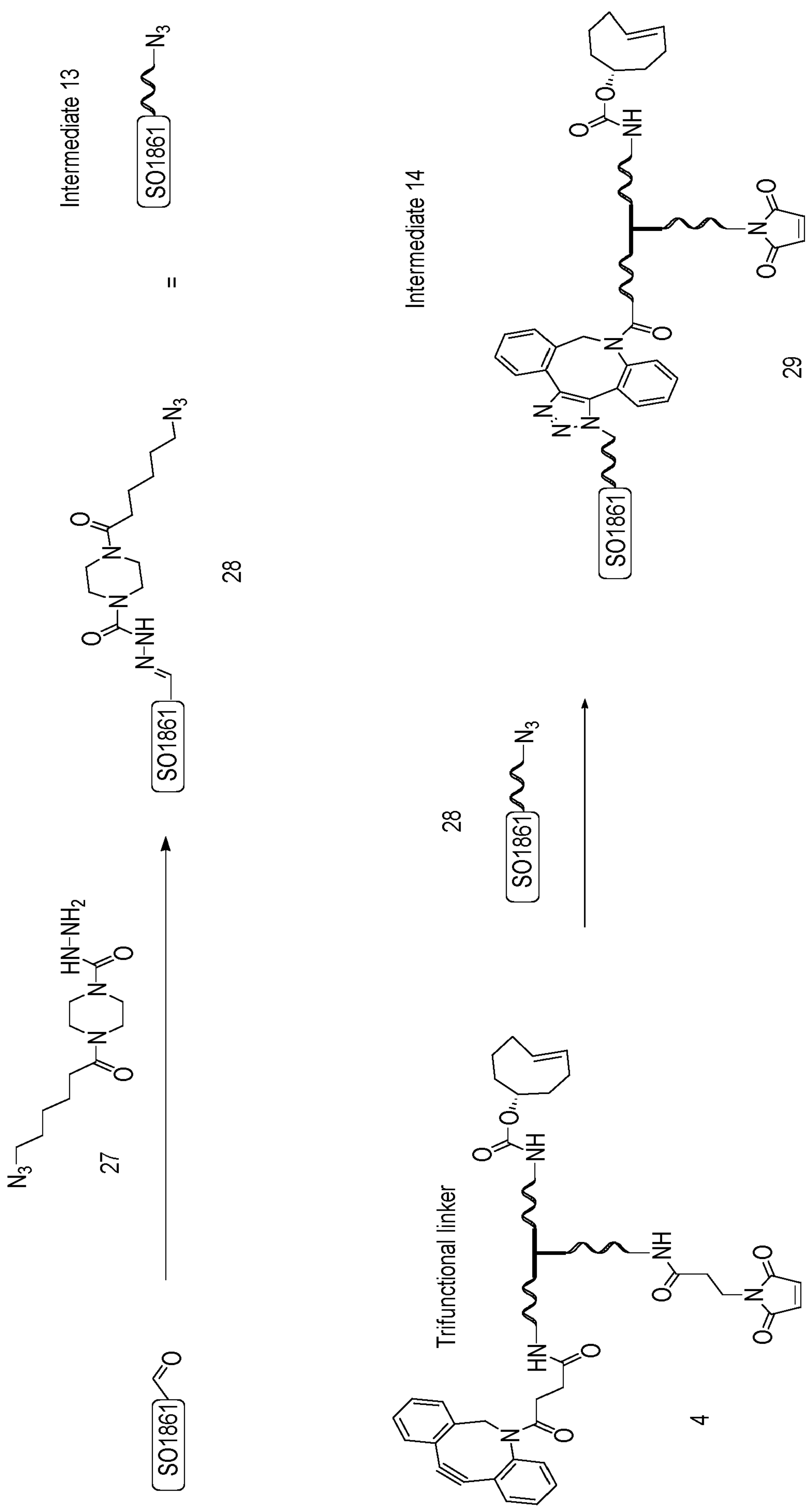

Intermediate 13

=

27

28

Intermediate 14

Trifunctional linker

4

28

29

**Fig. 38**
**B**

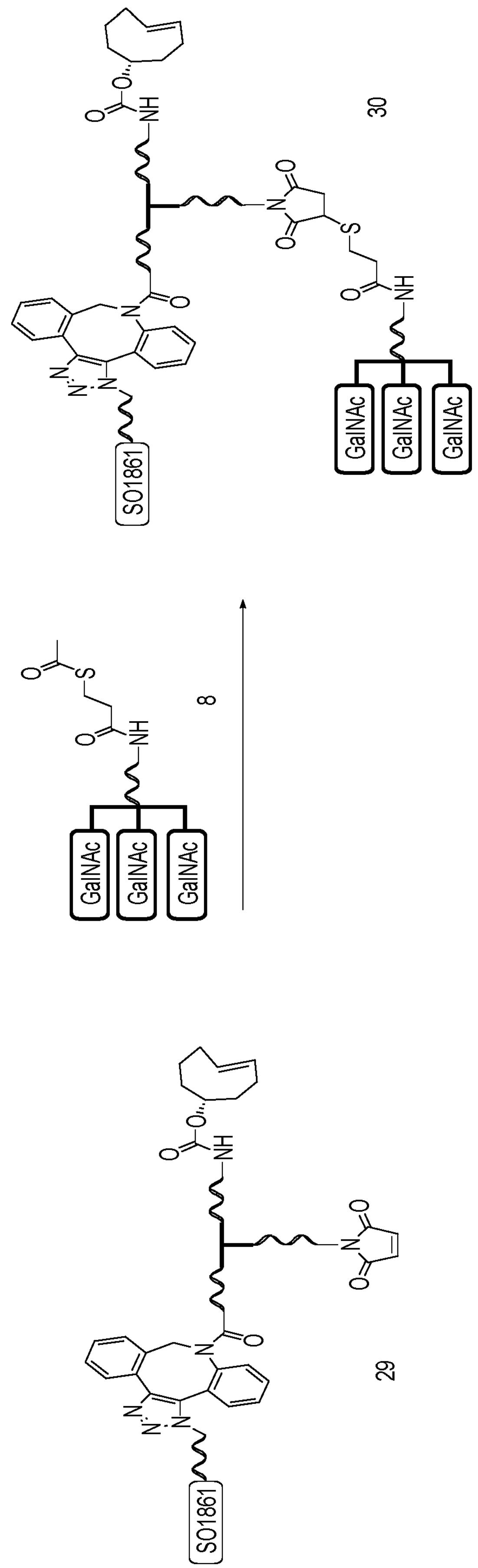

29

8

Intermediate 15

30

**Fig. 38**
**C**

TFL-(L-semicarbazone-SO1861)-(L-BNA)-(trivalent GalNAc)

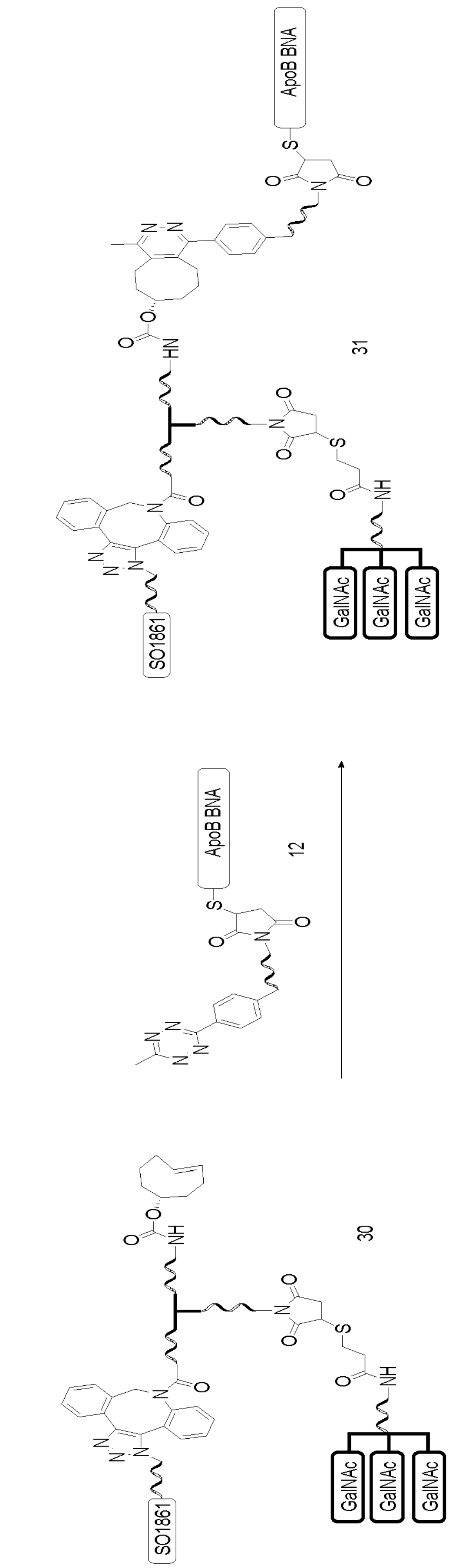

30

12

31

**Fig. 39**
**A**

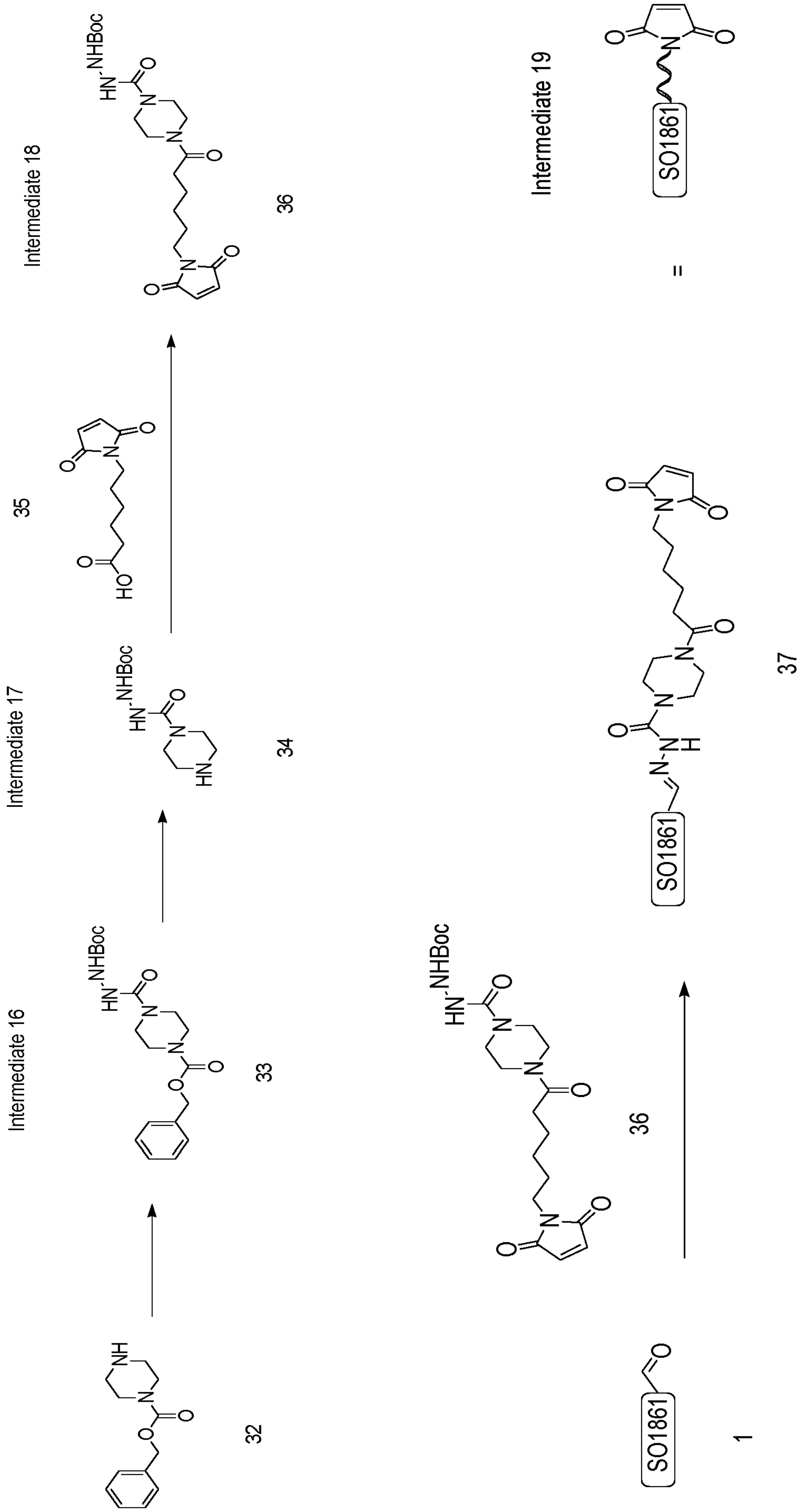

32

Intermediate 16

33

Intermediate 17

34

35

Intermediate 18

36

1

36

37

=

Intermediate 19

**Fig. 39**
**B**

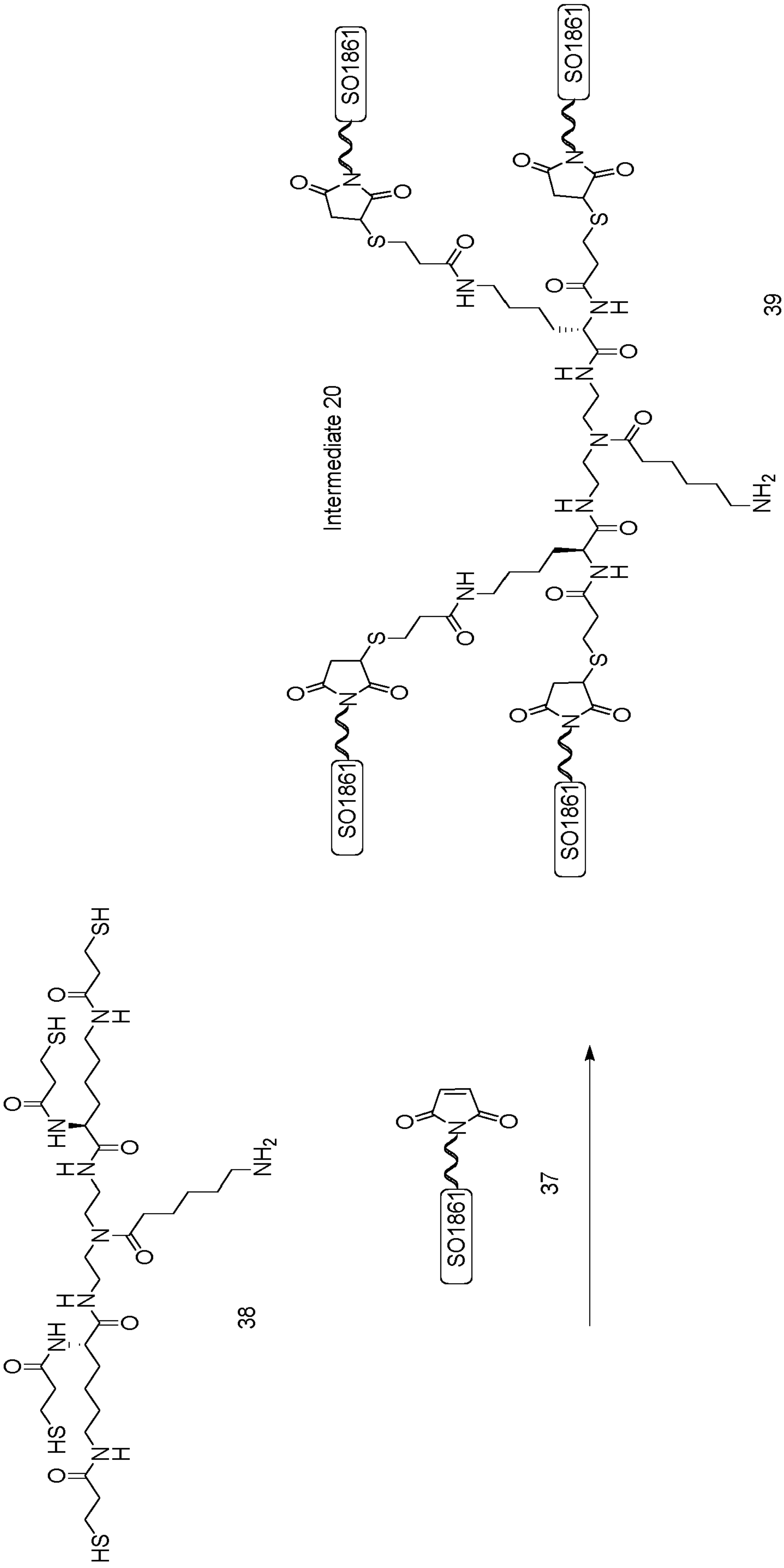

38

37

Intermediate 20

39

**Fig. 39**
**C**

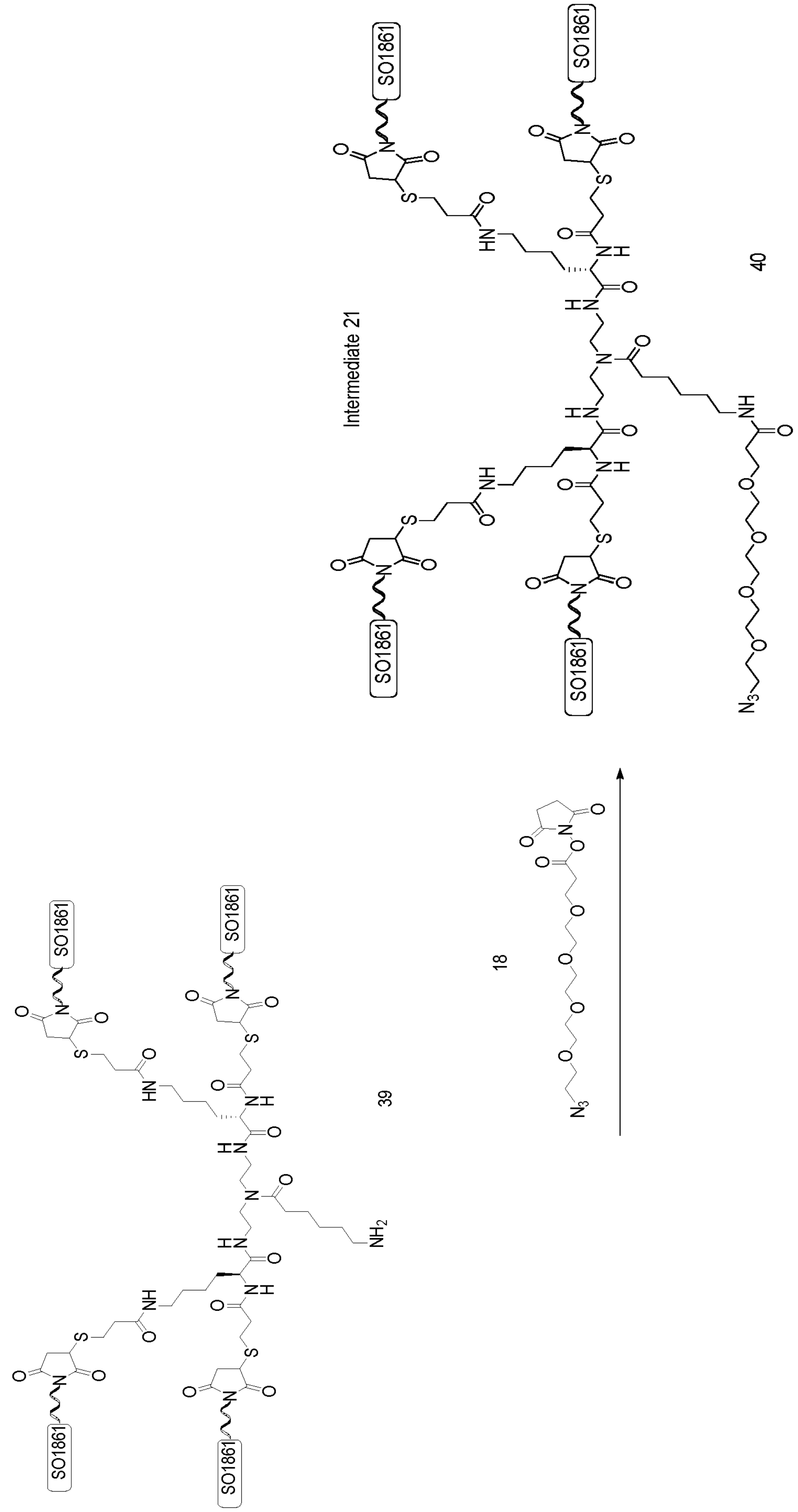

39

18

Intermediate 21

40

**Fig. 39**
**D**

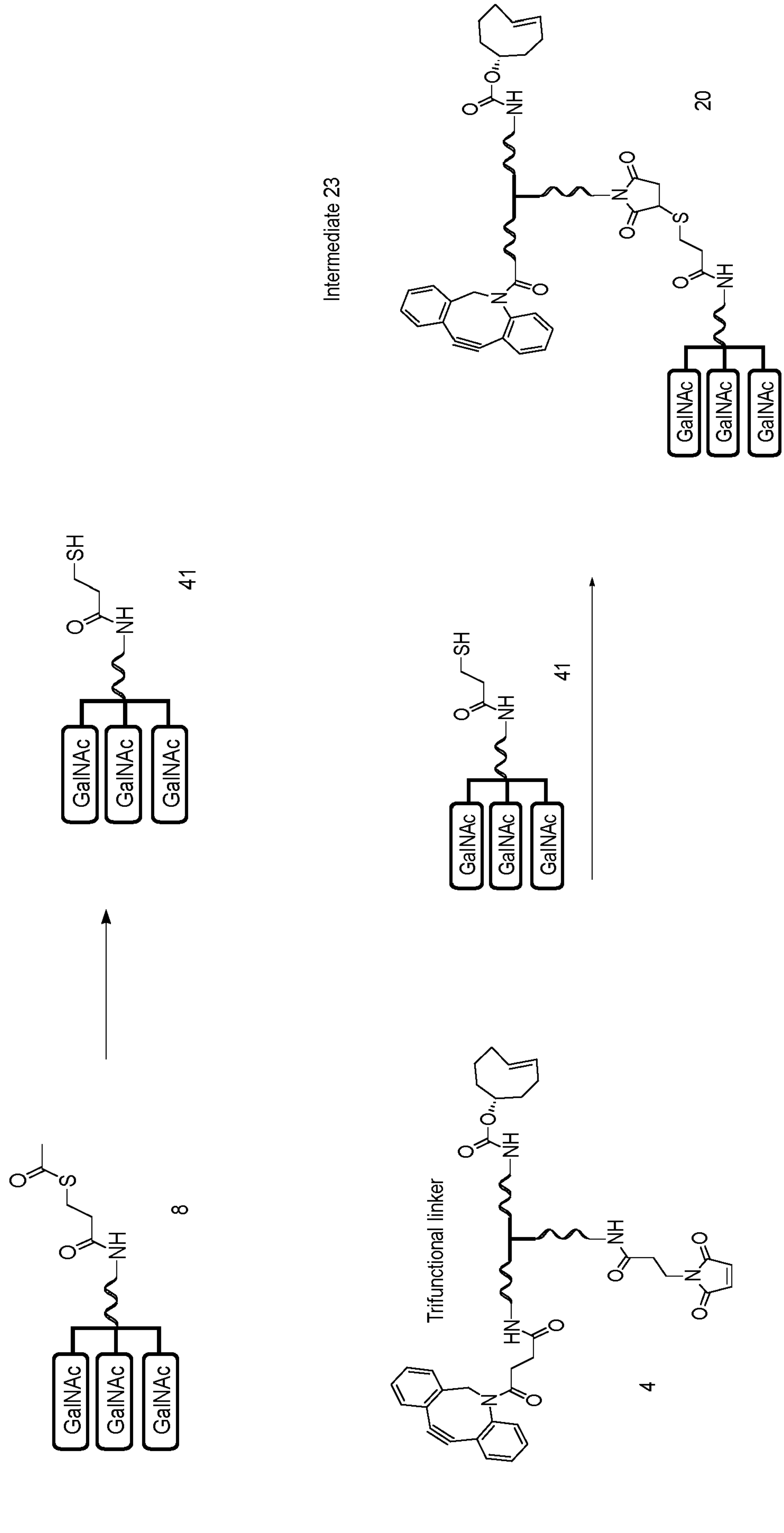

Intermediate 3

8

Intermediate 22

41

Trifunctional linker

4

41

Intermediate 23

20

**Fig. 39**
**E**

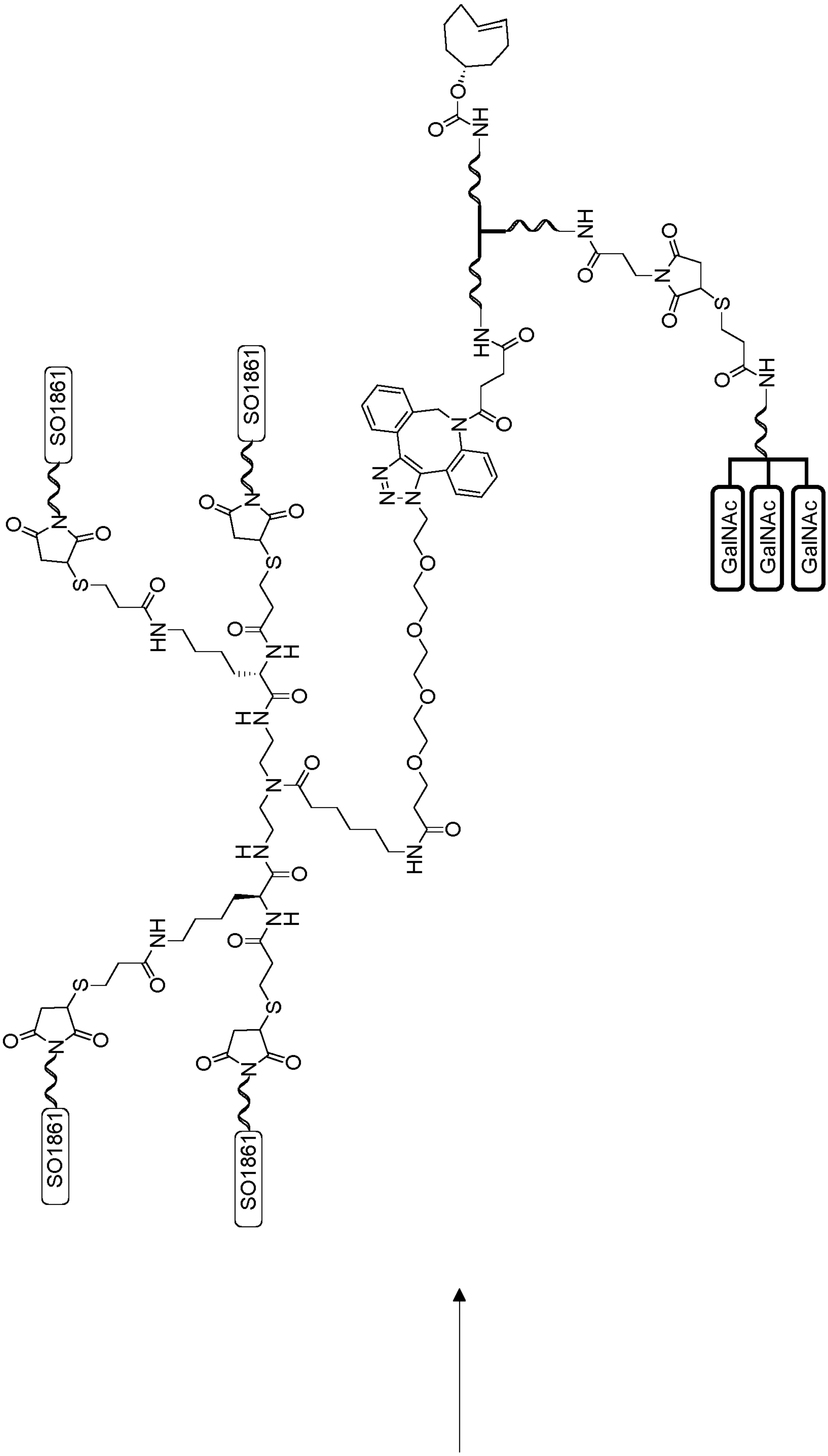


Intermediate 24

# Fig. 39
# E (continued)

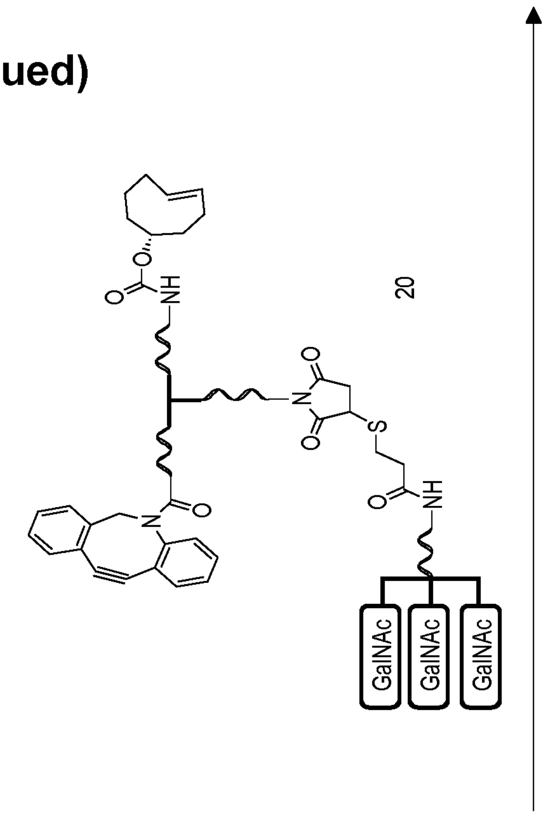

20

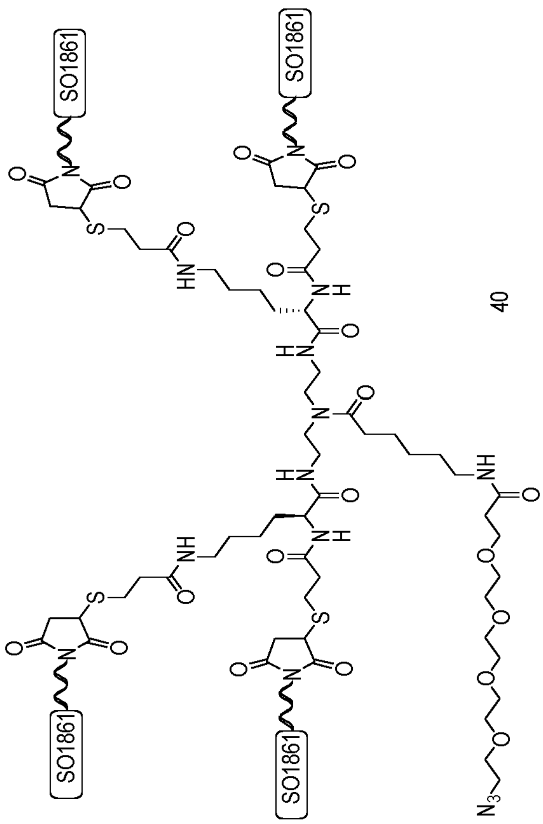

40

**Fig. 39**
**F**

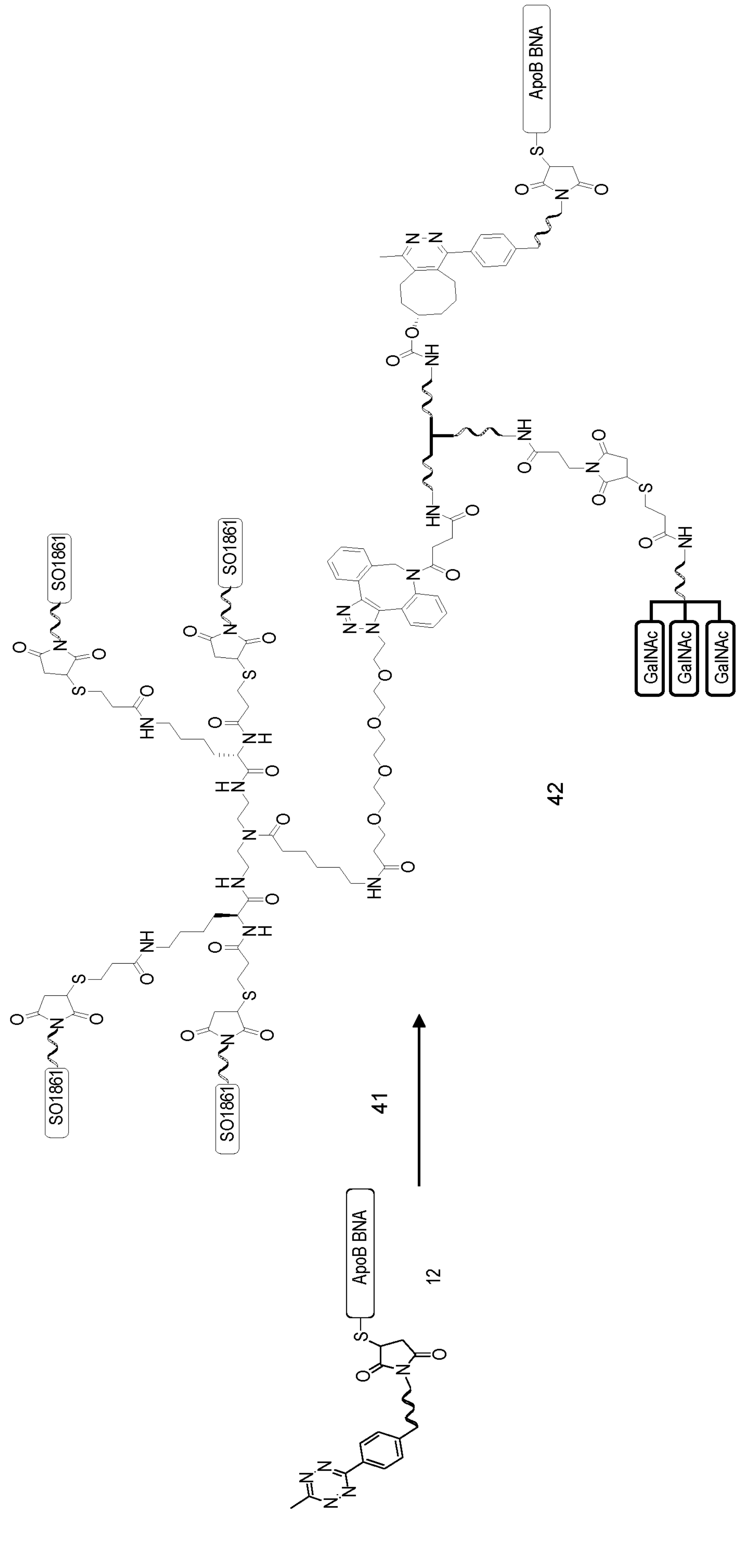

TFL-(dendron(L-semicarbazone-SO1861)4)-(L-BNA)-trivalent GalNAc)

12

41

42

**Fig. 40**
**A**

Intermediate 25

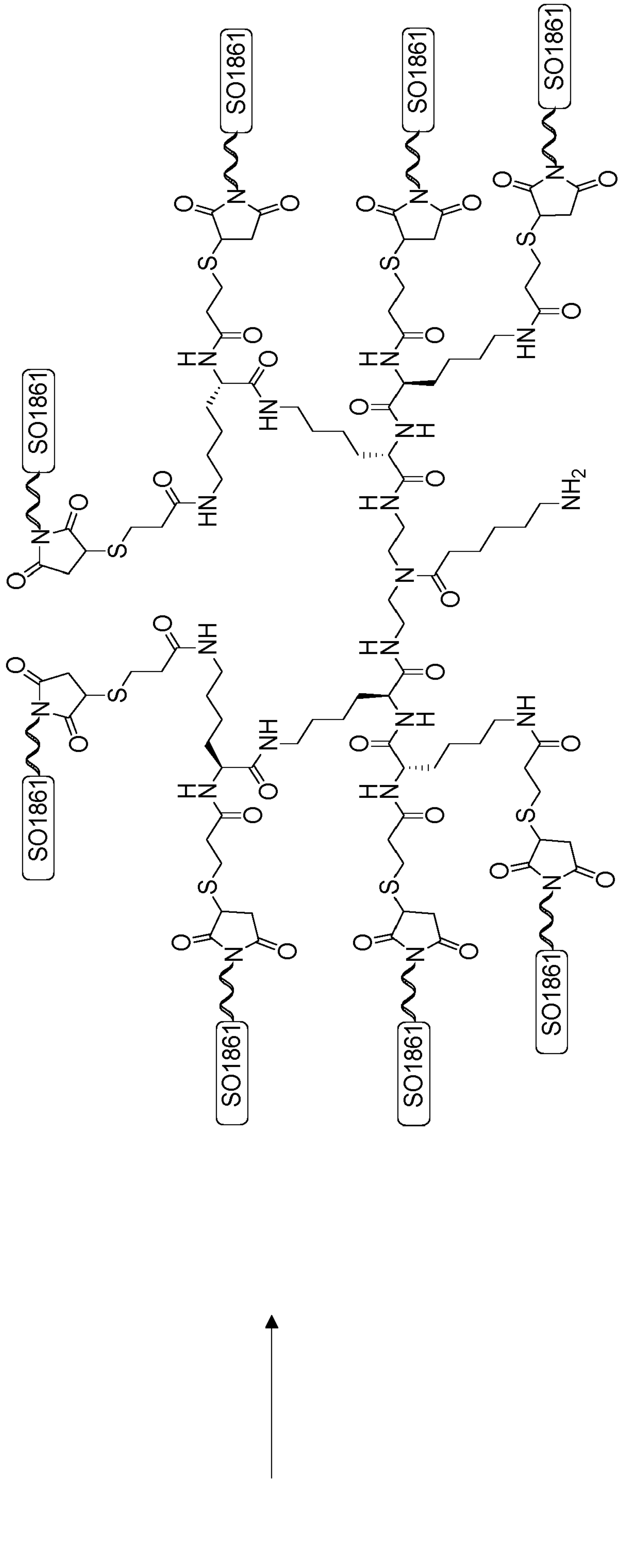

44

**Fig. 40**
**A (continued)**

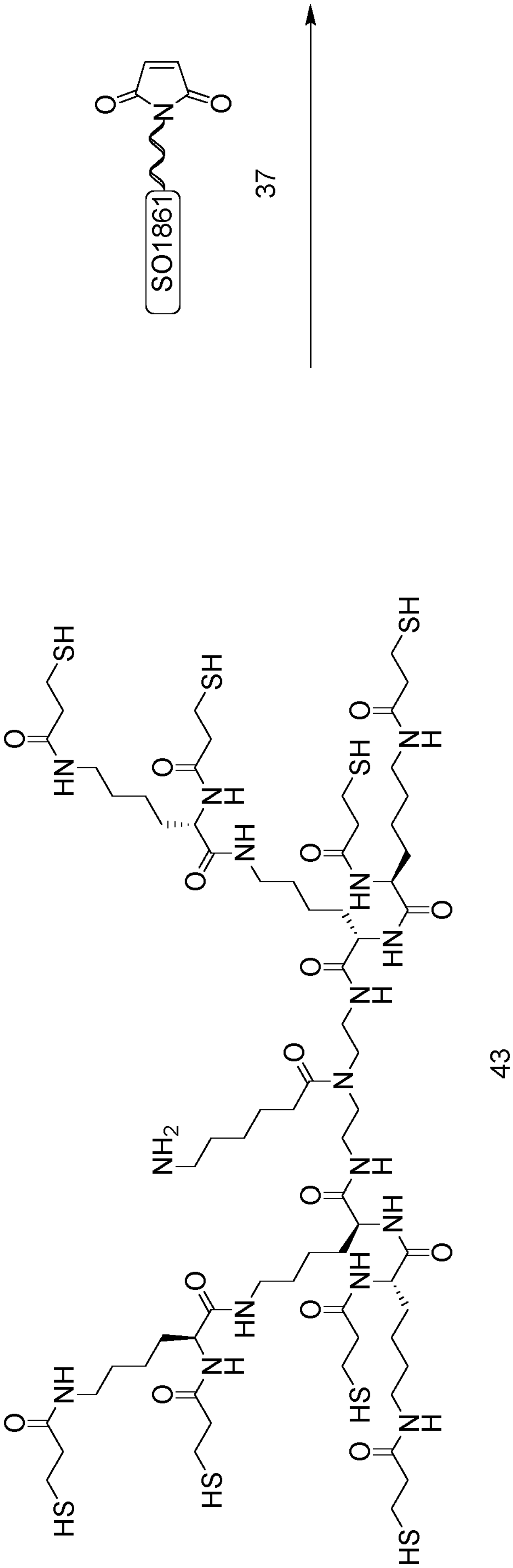

37

43

**Fig. 40**
**B**

Intermediate 26

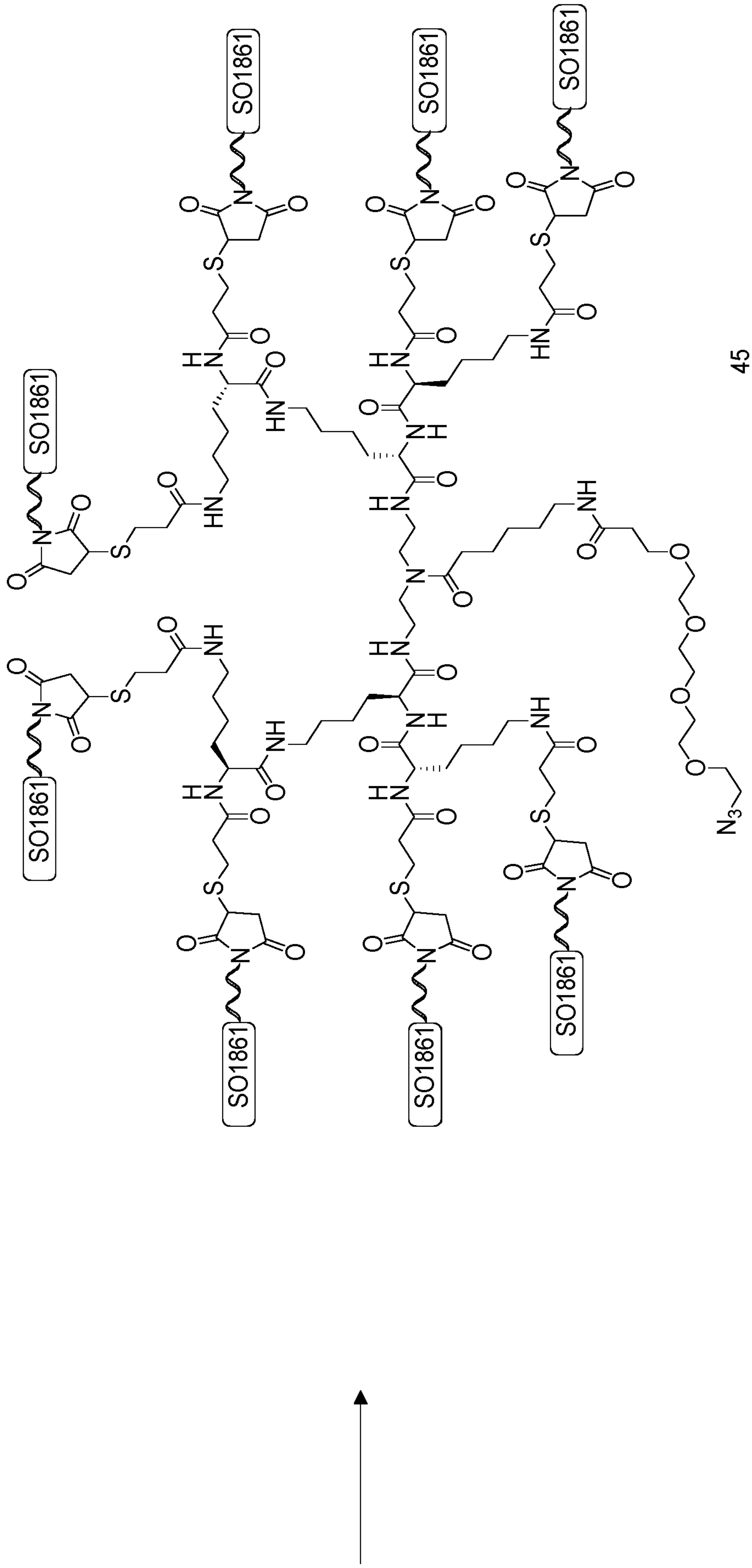

45

**Fig. 40**
**B (continued)**

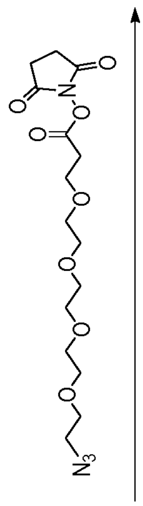

18

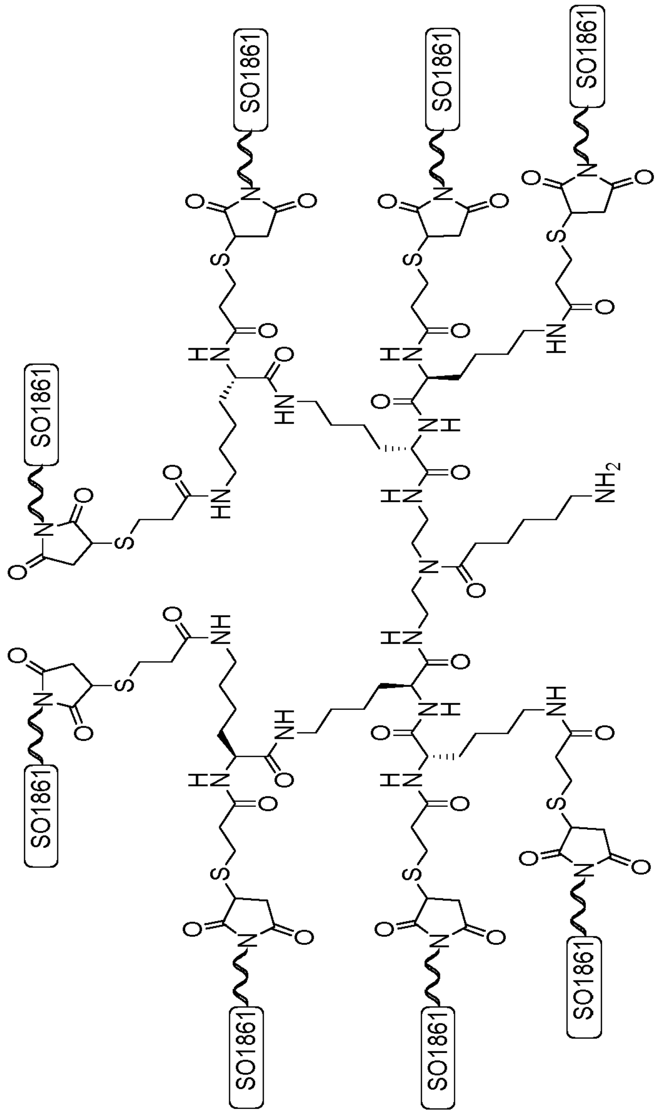

44

**Fig. 40**
**C**

Intermediate 27

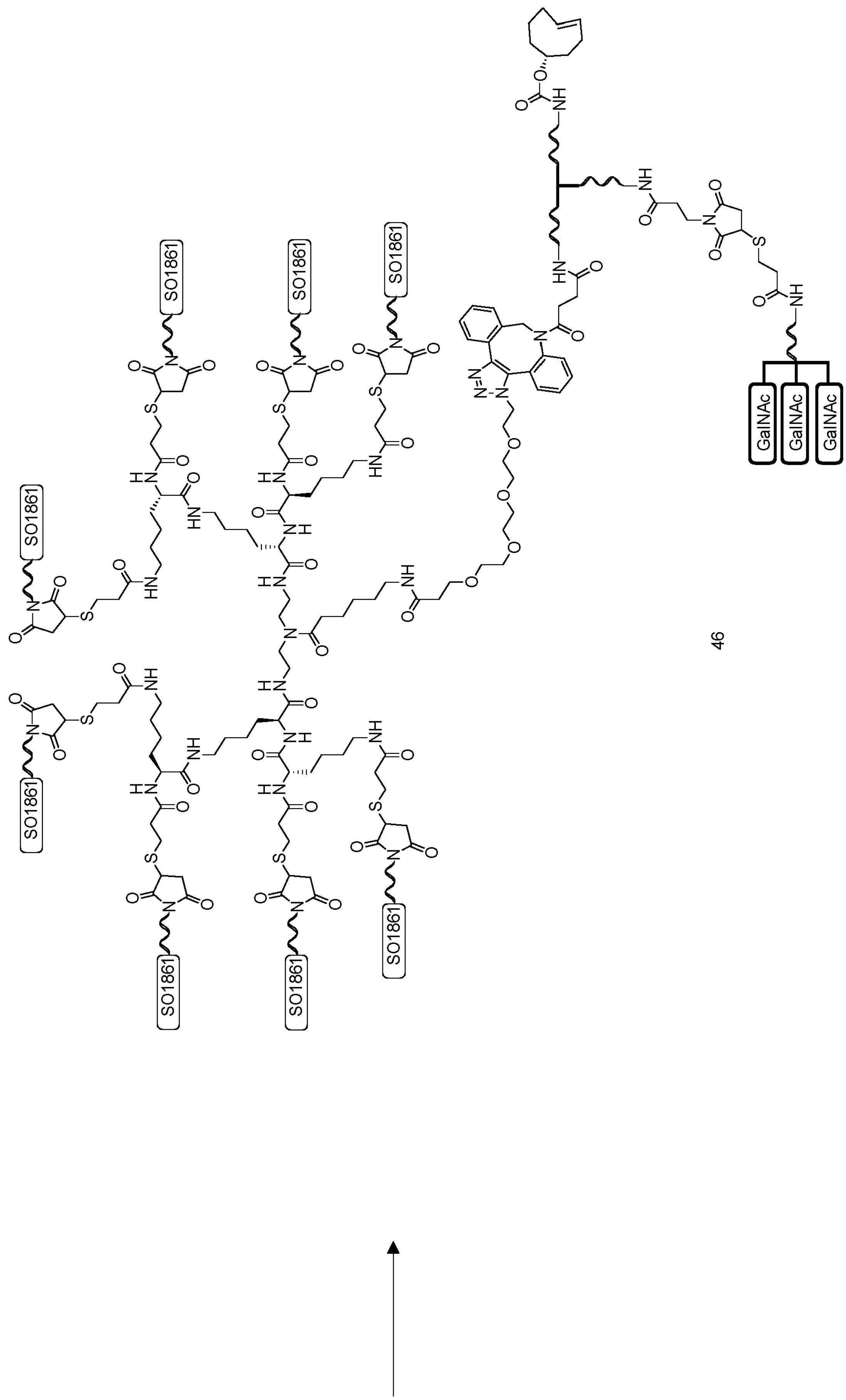

46

## Fig. 40
## C (continued)

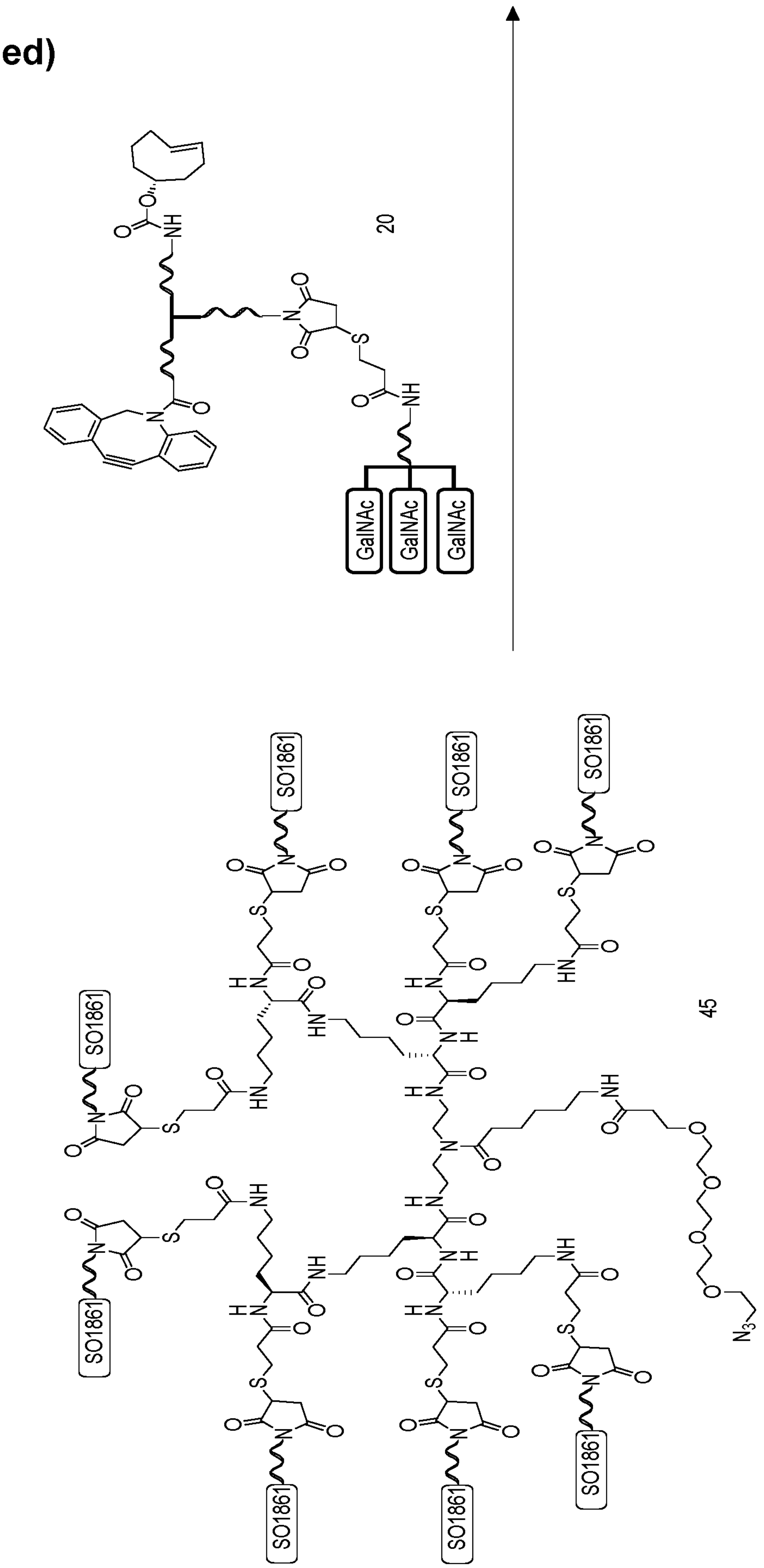

20

45

# Fig. 40
# D

TFL-(dendron(L-semicarbazone-SO1861)8)-(L-BNA)-trivalent GalNAc)

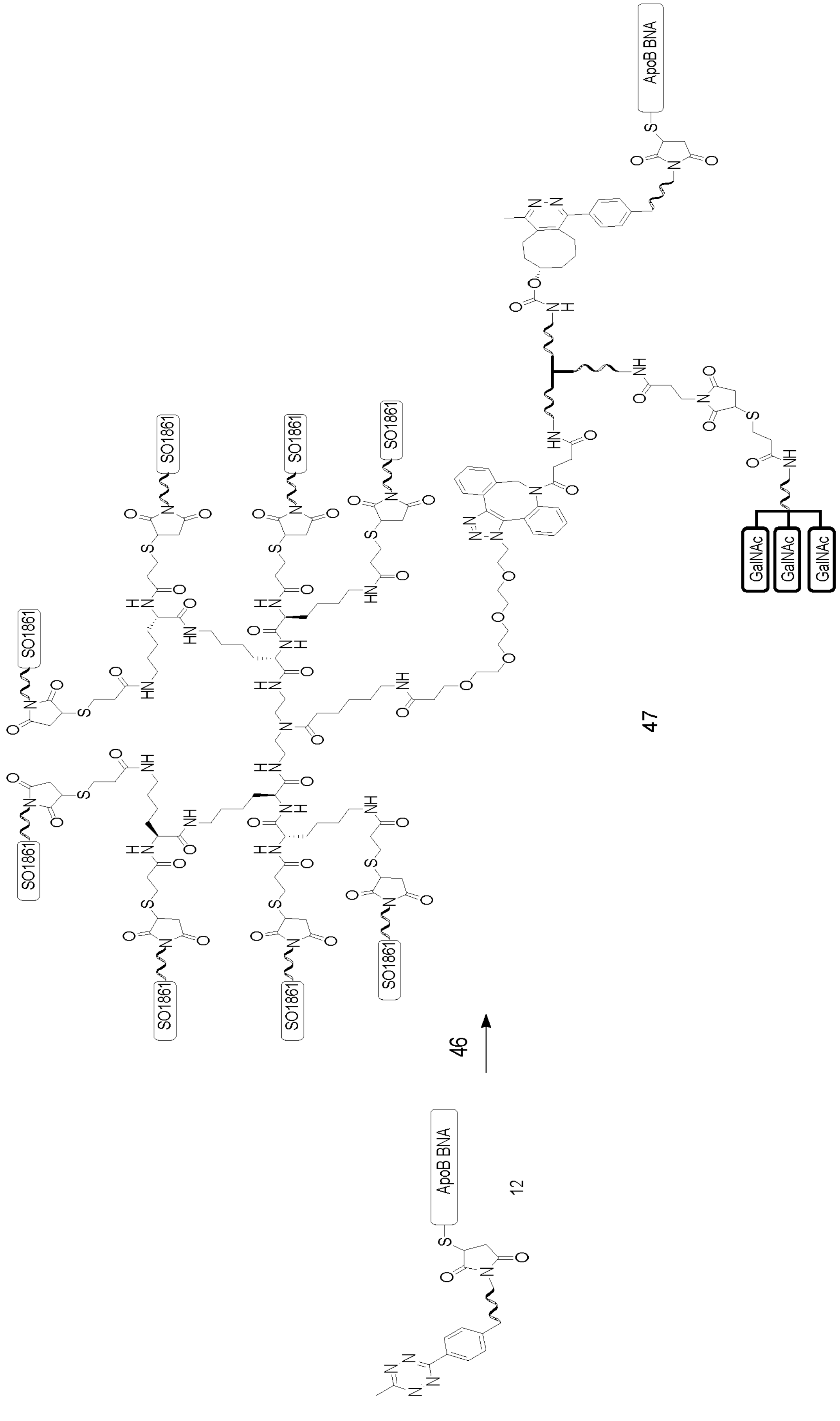

12

46

47

# Fig. 41

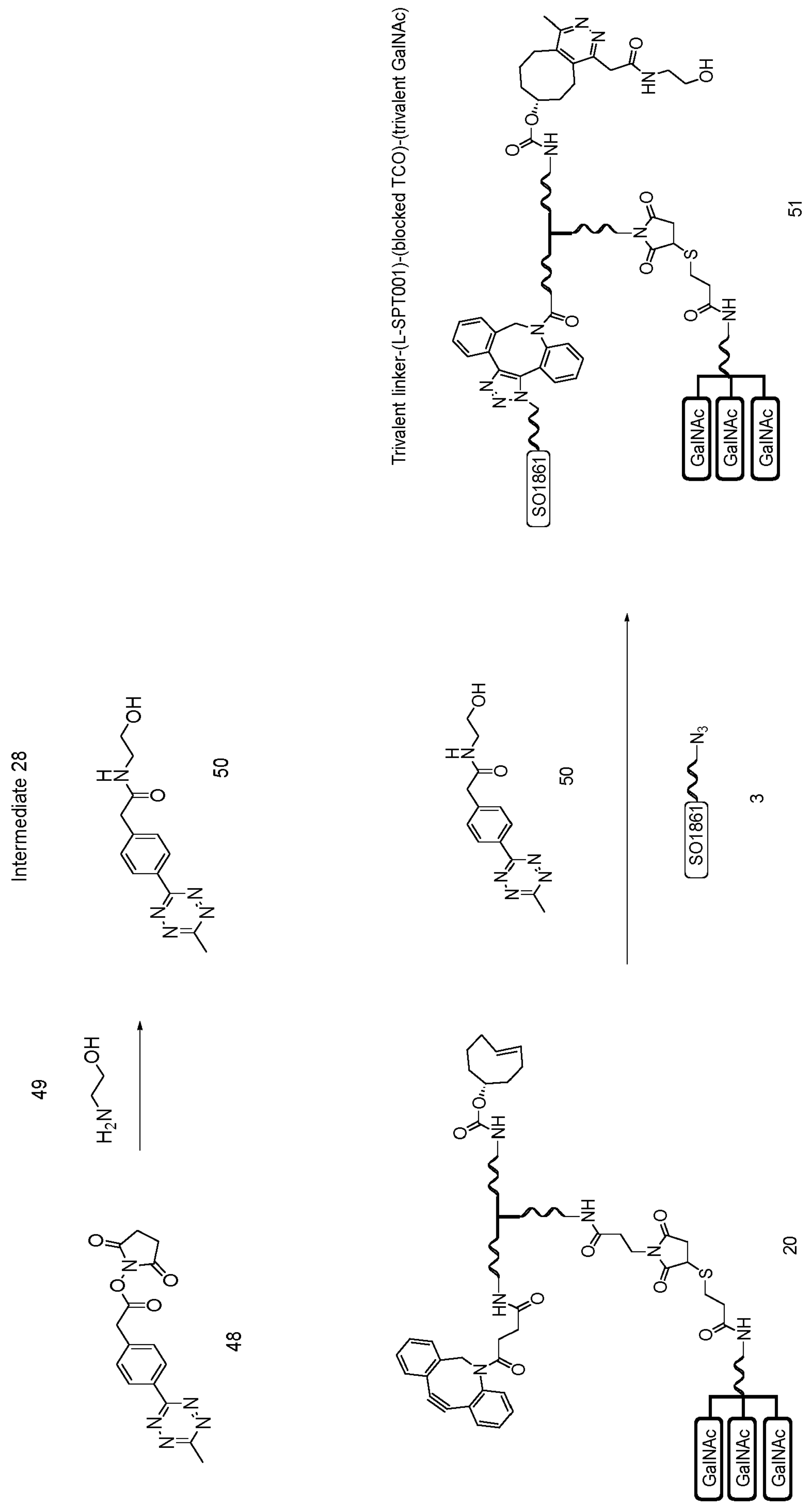


48

49

Intermediate 28

50

20

50

3

Trivalent linker-(L-SPT001)-(blocked TCO)-(trivalent GalNAc)

51

**Fig. 42A**

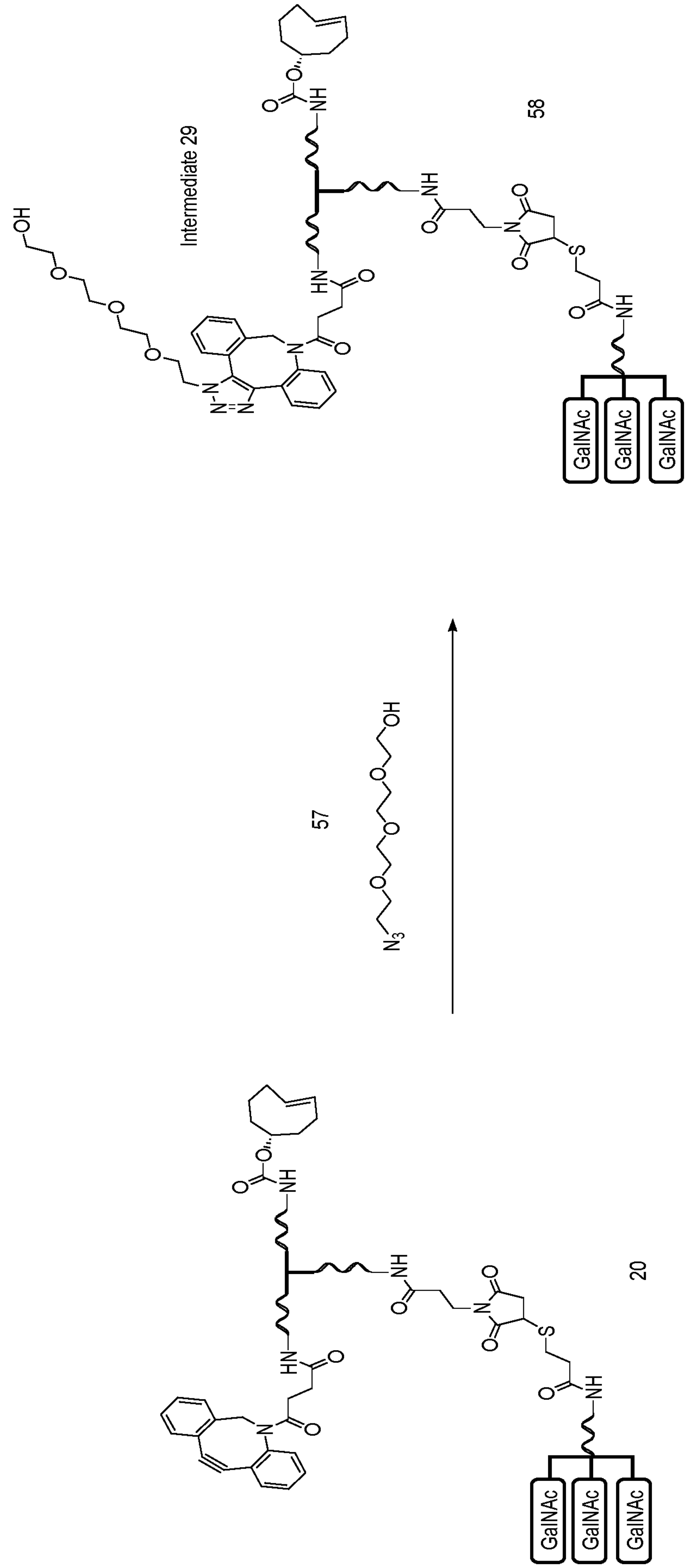

20

57

Intermediate 29

58

**Fig. 42B**

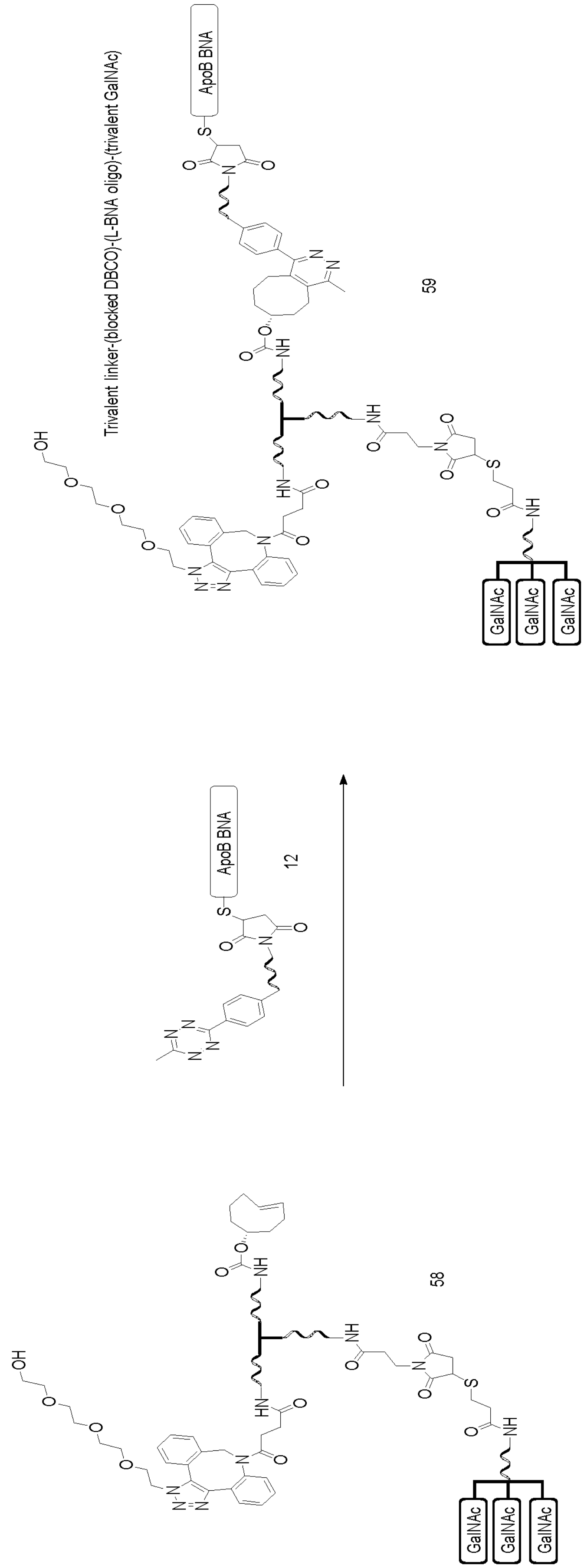

58

12

59

Trivalent linker-(blocked DBCO)-(L-BNA oligo)-(trivalent GalNAc)

# Fig. 43
## A

**Trifunctional linker**

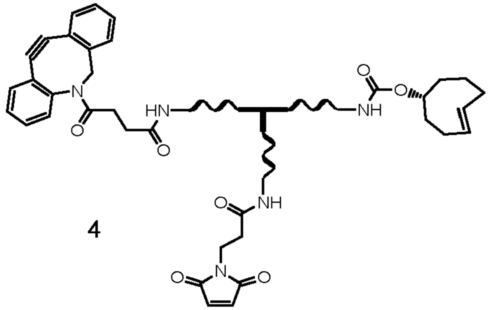

4

**Targeting ligand**

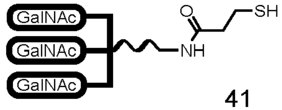

41

**Effector**

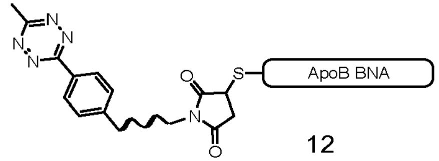

12

CONJUGATE C1

**Enhancers**

Hydrazone linker

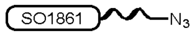

3

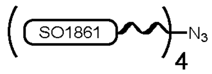

19

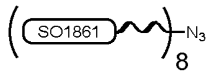

25

Semicarbazone linker

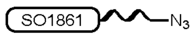

28

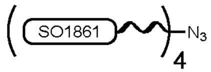

40

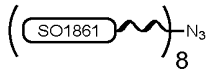

45

**Blocking agents for controls**

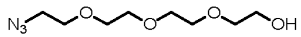

5

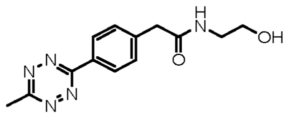

50

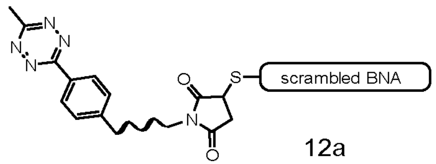

12a

# Fig. 43
# B

CONJUGATE C1 =

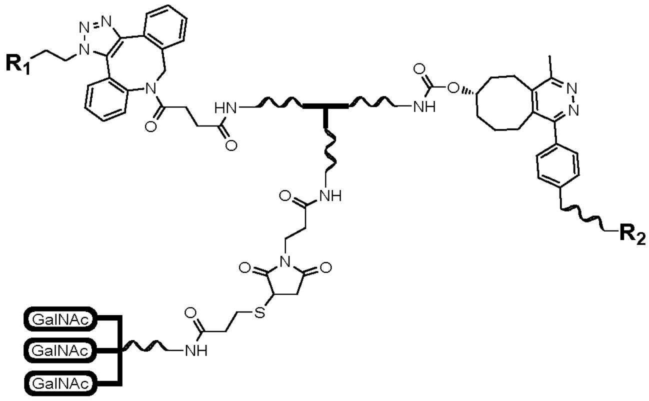

**Hydrazone linker**

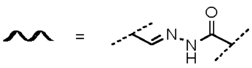

R1 = 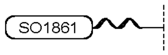 13, 13a, 51

R1 = 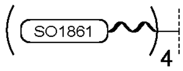 22, 22a, 52

R1 = 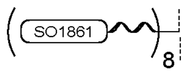 26, 26a, 53

**Semicarbazone linker**

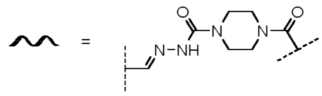

R1 = 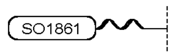 31, 31a, 54

R1 = 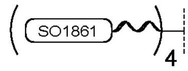 42, 42a, 55

R1 = 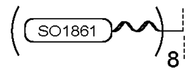 47, 47a, 56

R1 = 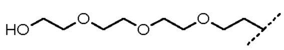 59, 59a

R2 = 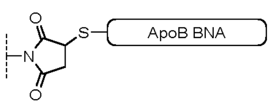 13, 22. 26, 31, 42, 47, 59

R2 = 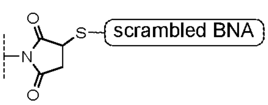 13a, 22a,, 26a, 31a, 42a, 47a. 59a

R2 = 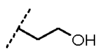 51, 52, 53, 54, 55, 56

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020126627 A


### Non-patent literature cited in the description


- **GOODING, MATT et al.** *Chemical biology & drug design*, 2012, vol. 80 (6), 787-809
- **SETTEN, RYAN L.** ; **JOHN J. ROSSI** ; **SI-PING HAN**. *Nature Reviews Drug Discovery*, 2019, vol. 18 (6), 421-446
- **HERMANSON, GREG T.** Bioconjugate techniques. Academic press, 2013
- **JIA et al.** Major Triterpenoid Saponins from Saponaria officinalis. *J. Nat. Prod.*, 19 September 1998, vol. 61 (11), 1368-1373, https://doi.org/10.1021/np980167u
- **J. CLOCHARD**. A new acetylated triterpene saponin from Agrostemma githago L. modulates gene delivery efficiently and shows a high cellular tolerance. *International Journal of Pharmaceutics*, 15 November 2020, vol. 589, 119822
- **MONIUSZKO-SZAJWAJ et al.** Highly Polar Triterpenoid Saponins from the Roots of Saponaria officinalis L.. *Helv. Chim. Acta*, 2016, vol. 99, 347-354
- **STEFAN BÖTTGER**. Untersuchungen zur synergistischen Zytotoxizität zwischen Saponinen und Ribosomen inaktivierenden Proteinen Typ I. *thesis*, 2013
- **SAMA et al.** Structure-Activity Relationship of Transfection-Modulating Saponins - A Pursuit for the Optimal Gene Trafficker. *Planta Med.*, 2019, vol. 85, 513-518
- **FUCHS et al.** Glycosylated Triterpenoids as Endosomal Escape Enhancers in Targeted Tumor Therapies. *Biomedicine*, 2017, vol. 5 (14)
- **SAMA et al.** Sapofectosid - Ensuring non-toxic and effective DNA and RNA delivery. *International Journal of Pharmaceutics*, 20 December 2017, vol. 534 (1-2), 195-205
- *Tetrahedron Letters*, 1963 (8), 477-482
- *J. Am. Chem Soc.*, 2014, vol. 136, 16958-16961
- **Y ZHANG** ; **Z QU** ; **S KIM** ; **V SHI** ; **B LIAO1** ; **P KRAFT** ; **R BANDARU** ; **Y WU** ; **LM GREENBERGER** ; **ID HORAK**. Down-modulation of cancer targets using locked nucleic acid (LNA)-based antisense oligonucleotides without transfection. *Gene Therapy*, 2011, vol. 18, 326-333